(19) Europäisches Patentamt
European Patent Office
Office européen des brevets

(11) EP 4 600 652 A2

(12) EUROPEAN PATENT APPLICATION

(43) Date of publication:
**13.08.2025 Bulletin 2025/33**

(21) Application number: **25166601.2**

(22) Date of filing: **12.08.2021**

(51) International Patent Classification (IPC):
***G01N 33/68*** *(2006.01)*

(52) Cooperative Patent Classification (CPC):
**G01N 33/6803; G01N 33/6809**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.08.2020 GB 202012749**
**21.07.2021 GB 202110514**

(62) Document number(s) of the earlier application(s) in accordance with Art. 76 EPC:
**21762758.7 / 4 196 797**

(71) Applicant: **Proteotype Diagnostics Ltd**
**Cambridge, England CB22 3AT (GB)**

(72) Inventor: **YATES, Emma Victoria**
**Cambridge, CB22 3AT (GB)**

(74) Representative: **Potter Clarkson**
**Chapel Quarter**
**Mount Street**
**Nottingham NG1 6HQ (GB)**

Remarks:
•This application was filed on 27-03-2025 as a divisional application to the application mentioned under INID code 62.
•Claims filed after the date of filing of the application/after the date of receipt of divisional application (Rule 68(4) EPC).

(54) METHODS OF IDENTIFYING THE PRESENCE AND/OR CONCENTRATION AND/OR AMOUNT OF PROTEINS OR PROTEOMES

(57) The present disclosure relates to methods of identifying the presence and/or concentration and/or amount of one or more proteins, peptides, oligopeptides, polypeptides, protein complexes, subproteomes, or proteomes of interest within a sample based on the measured label, amino acid concentration, or number of amino acids of two or more labelled amino acid types in the sample.

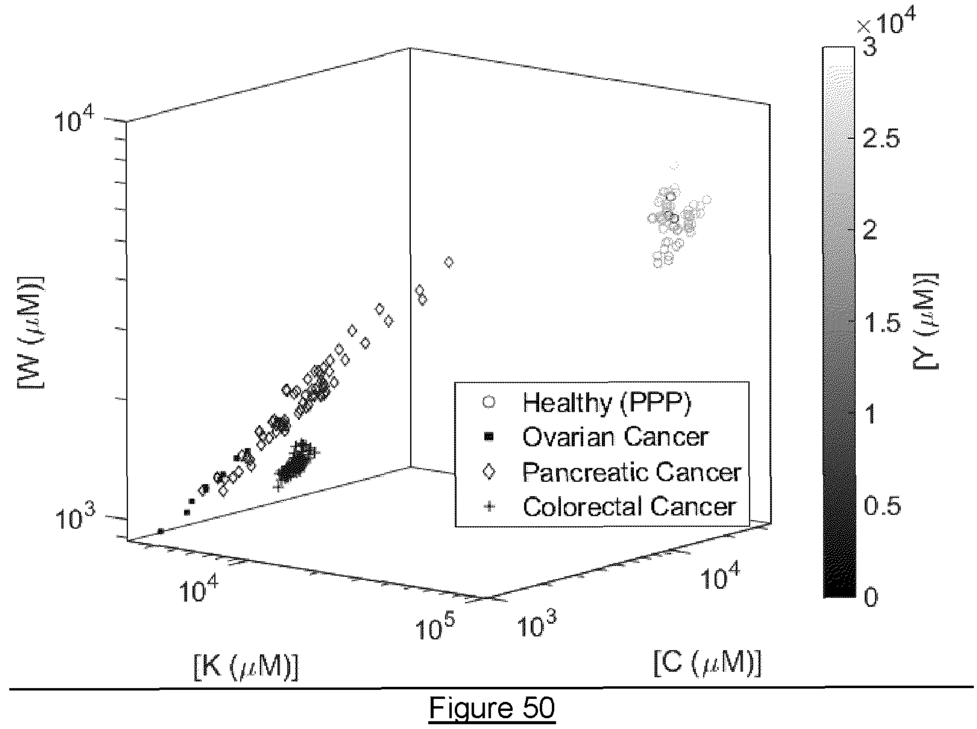


Figure 50



EP 4 600 652 A2

## Description

FIELD OF THE INVENTION

The present invention relates to methods of identifying the presence and/or concentration and/or amount of one or more proteins, peptides, oligopeptides, polypeptides, protein complexes, subproteomes, or proteomes of interest within a sample.

BACKGROUND OF THE INVENTION

Proteins are biological polymers that are comprised of sequences of amino acids. Proteomics is the large-scale study of proteins. It allows the identification of and quantification of proteins. Within the field of proteomics, there are multiple established methods to identify the presence or absence of a protein within a sample. Identification of the presence or absence of a subproteome or a proteome within a sample is challenging as this involves sequential identification of all of its proteins. Some proteomic methods allow the quantification of the concentration or amount of a protein within a sample.

The most common method for identifying the presence of proteins in a sample is mass spectrometry. Mass spectrometry measures the mass-to-charge ratio of ions present in a sample. The mass spectrum of a sample is a plot of the ion signal as a function of the mass-to-charge ratio. The spectra are used to determine the isotopic signature of a sample and the masses of particles, which are used to provide the chemical identity or structure of chemical compounds. However, mass spectrometry is labour intensive and is not inherently quantitative because different peptides are ionized and detected with different efficiencies. To combat this, approaches such as isotope-coded affinity tags (ICAT) are used, but this only permits a fraction of proteins identified to be quantified. Most quantitative mass spectrometry approaches permit determination of only relative changes in protein concentration or amount across samples, rather than absolute quantification of samples. Mass spectrometry proteomics is also limited in coverage, particularly for higher organisms. 'Top down' mass spectrometry proteomics which analyses whole proteins only permits protein identification for 10% of the proteins studied, and 'bottom up' mass spectrometry proteomics which analyses proteins which have been digested into fragments permits protein identification for 8-25% of the proteins studied. Due to the complexity of the mass spectra obtained, mixtures and complex samples must be separated into their components, for example by two-dimensional gel electrophoresis or high-performance liquid chromatography (HPLC), before they can be sequentially analysed with mass spectrometry.

An alternative approach to identify the presence of proteins is to use protein microarrays. Protein microarrays immobilize an array of proteins, or an array of probes, onto a support surface and are particularly suitable for multiplexed detection. Tagged probes or tagged proteins are added to the array and the binding interaction between the protein and the probe is detected. However, protein microarrays are labor intensive and suffer from a lack of reproducibility and accuracy. Detection requires a binding event near a surface and therefore, the binding event and thus the accuracy of detection can be affected by the surface. Furthermore, only the proteins which already have a corresponding probe, such as a specific antibody, can be identified by this method

Several methods have aimed to identify a protein via physical parameters characteristic of a protein, for example Zhang et al., "Top-down proteomics on a microfluidic platform" (2019), eprint 1910.11861 arXiv physics.bio-ph. In this microfluidic method, a protein's hydrodynamic radius ($R_H$) which is its size in solution is used with ratios of fluorescence signals from Trp/Lys and Tyr/Lys residues within proteins for protein identification. The lysine (Lys) residues are fluorescently labelled and the tryptophan (Trp) and tyrosine (Tyr) residues are unlabelled. Seven known proteins are measured four times, and a protein is identified when the values obtained the fourth time the protein is measured match the values obtained the other three times the protein was measured While it is shown that the values measured are characteristic of a known protein under a set of experimental conditions in that the measured proteins are distinguishable from each other based on these values, none of the values can be predicted for a protein of interest. $R_H$ cannot be predicted for an amino acid sequence, which has unknown and often partial intrinsic disorder. Those skilled in the art appreciate that intrinsic fluorescence from tryptophan and tyrosine residues depends in a complex manner on the local physical environment surrounding the tryptophan and tyrosine residues within a protein structure which is currently unpredictable from an amino acid sequence. Therefore, $R_H$, Trp and Tyr signals would all change with the solution conditions, for example different readings for the same protein would be obtained if the protein is placed in a different buffer or if it interacts with another biomolecule. The method does not allow protein quantification because none of the values used for protein identification provides information about protein amount or concentration. Due to the unpredictable nature of the results obtained, it is not possible to analyse a mixture of proteins or a proteome using this method.

Alternatively, the state-of-the-art includes newly developed protein sequencing methods such as Swaminathan, J et al. Nat Biotechnology 36, 1076-1082 (2018). Sparse fluorosequencing performs classical Edman degradation sequencing on single peptide fragment molecules that have been fluorescently labelled on specific amino acids prior to

their immobilization onto a surface and observes the pattern of fluorescence disappearance from the surface as the fluorescently labelled amino acids are sequentially cleaved from the peptide N-terminus. The pattern of fluorescence decreases reveals the positions of the labelled amino acids within the peptide being read and provides a sparse peptide sequence. These sparse peptide sequences can be predicted for a protein of interest based on the information-rich constraints of protease cleavage specificity, surface attachment chemistry, labelling chemistry, and the positions of the labelled amino acids within the predicted peptide fragments for the protein of interest. Practically, this labor and data intensive method is prone to error from a variety of sources and correct reads are observed approximately 40% of the time for a single purified peptide. Quantification was not evaluated for this method. The method relies on coupling to chromatographic separation methods like HPLC and/or mass spectrometry to first verify that all amino acids, such as all lysine and all cysteine amino acids, are quantitatively fluorescently labelled within each peptide fragment prior to carrying out the sequencing. Although peptide fragments within a two-component mixture were identified, this requires that the peptide fragments be spatially separated from one another via surface attachment at different positions on the surface, so that distinct fluorescence disappearance traces can be observed for each peptide. Like traditional Edman sequencing, this method is slow with one Edman cycle requiring 1 hour, not suitable for the analysis of N-terminally modified peptides, and not suitable for reading peptide fragments greater than 30 amino acids in length. It is admitted that due to relying on Edman sequencing, this method is more suitable for the identification of short peptides rather than long protein molecules. The average length of a protein molecule within the human proteome is 558 amino acids. It is not possible to analyze a mixture of proteins or a proteome using this method.

There is a recognized need for the development of simple and general alternatives to mass-spectrometry based protein identification that permit the identification of whole proteins as proteins of interest. There is a recognized need for an efficient method of characterizing complex mixtures of proteins, for example mixtures of proteins that are disease-associated. There is a great need for a rapid and general method of diagnosing any infection. Preferably these methods would enable protein quantification. Therefore, there is a need for a more efficient, cost-effective and general method for identifying the presence and/or concentration and/or amount of proteins, peptides, oligopeptides, polypeptides, protein complexes, subproteomes, or proteomes of interest within a sample.

## SUMMARY OF INVENTION

The invention is based on the discovery that labelling and measuring two or more amino acid types in a sample can identify the presence and/or concentration and/or amount of one or more proteins, peptides, oligopeptides, polypeptides, protein complexes, subproteomes, or proteomes of interest in a sample. This is based on the measured label, amino acid concentration, or number of amino acids of each labelled amino acid type in the sample.

It has been discovered that each protein, peptide, oligopeptide, polypeptide, protein complex, subproteome, or proteome has a unique signature based on the label values, amino acid concentrations, or number of amino acids of two or more amino acid types for the protein, peptide, oligopeptide, polypeptide, protein complex, subproteome, or proteome at each concentration.

The signature of the label values or amino acid concentrations of each of two or more amino acid types for a protein, peptide, oligopeptide, polypeptide, protein complex, subproteome, or proteome is unique for each protein, peptide, oligopeptide, polypeptide, protein complex, subproteome, or proteome at each concentration. The signature of the number of amino acids of each of two or more amino acid types for a protein, peptide, oligopeptide, polypeptide, protein complex, subproteome, or proteome is also unique for each protein, peptide, oligopeptide, polypeptide, protein complex, subproteome, or proteome.

Therefore, the signature of the sample can be compared to the signature of one or more proteins, peptides, oligopeptides, polypeptides, protein complexes, subproteomes, or proteomes of interest to identify the presence and/or concentration and/or amount of the one or more proteins, peptides, oligopeptides, polypeptides, protein complexes, subproteomes, or proteomes of interest in the sample.

The signature of the known label values or amino acid concentrations of two or more amino acid types in a protein, peptide, oligopeptide, polypeptide, protein complex, subproteome, or proteome of interest is a function of the concentration of the protein, peptide, oligopeptide, polypeptide, protein complex, subproteome, or proteome of interest, and is unique for each protein, peptide, oligopeptide, polypeptide, protein complex, subproteome, or proteome of interest at each concentration. Therefore, the values of the measured labels or amino acid concentrations of two or more amino acid types in the sample can be compared to the known label values or amino acid concentrations of the same two or more amino acid types that have been labelled in the sample for the protein, peptide, oligopeptide, polypeptide, protein complex, subproteome, or proteome of interest to provide a positive identification of the presence and/or concentration and/or amount of that protein, peptide, oligopeptide, polypeptide, protein complex, subproteome, or proteome of interest in the sample. The signature of the number of amino acids of two or more amino acid types in a protein, peptide, oligopeptide, polypeptide, protein complex, subproteome, or proteome of interest is unique for each protein, peptide, oligopeptide, polypeptide, protein complex, subproteome, or proteome of interest. Therefore, the number of amino acids of each of two

or more amino acid types in the sample can be compared to the number of amino acids of the same two or more amino acid types that have been labelled in the sample for the protein, peptide, oligopeptide, polypeptide, protein complex, subproteome, or proteome of interest to provide a positive identification of the presence in the sample.

In some embodiments, this comparison can be visualized using an n-dimensional space, where the number of dimensions is equal to the number of *n* amino acid types labelled and measured in the methods of the invention. For example, two labelled amino acid types are visualized in a 2-dimensional space, and three labelled amino acid types are visualized in a 3-dimensional space. This dimensional space increases as each additional amino acid type is labelled and measured in the sample. The amino acid concentrations or values of the label of the two or more amino acid types take on a line in *n*-dimensional space. The number of amino acids of each the two or more amino acid types take on a point in n-dimensional space. There are *n* dimensions for *n* amino acid types labelled in the sample.

It has been discovered that the label, amino acid concentration, or number of amino acids of only two or more amino acid types need to be measured in order to identify the presence and/or concentration and/or amount of a protein, peptide, oligopeptide, polypeptide, protein complex, subproteome, or proteome, of interest in the sample. Labelling and measuring two or more amino acid types is essential to the methods of the invention because when two or more amino acid types are labelled and measured, this provides the unique signature for each protein, peptide, oligopeptide, polypeptide, protein complex, subproteome, or proteome of interest. Two amino acid types are required to be labelled and measured because if only one amino acid type were labelled and measured, all proteins, peptides, oligopeptides, polypeptides, protein complexes, subproteomes, or proteomes of interest would have the same reference line. When the sample point is compared to *p* lines for *p* proteins, peptides, oligopeptides, polypeptides, protein complexes, subproteomes, or proteomes of interest, each a function of concentration, the presence and/or concentration and/or amount of a protein, peptide, oligopeptide, polypeptide, protein complex, subproteome, or proteome of interest within the sample is simultaneously determined. In this solution phase method, the amount of a protein contained within the sample is simply determined by multiplying the concentration of the protein, peptide, oligopeptide, polypeptide, protein complex, subproteome, or proteome identified within the sample by the volume of solution within the sample. It is not necessary or efficient to measure the label, amino acid concentration, or number of amino acids for every amino acid type in the sample.

Proteins, peptides, oligopeptides, polypeptides, protein complexes, subproteomes, and proteomes of interest all have unique signatures of the known values of the label, amino acid concentrations, or number of amino acids of two or more amino acid types. It is not necessary to know or suspect what category of molecules the sample contains (i.e. a protein, peptide, oligopeptide, polypeptide, protein complex, mixture, subproteome, or proteome) to determine the presence and/or concentration and/or amount of a member of that category of interest within the sample. For example, the two or more amino acid types labelled in the sample are tryptophan (W) and lysine (K), the measured label of tryptophan (W) is used to determine the concentration of tryptophan (W) in the sample, and the measured label of lysine (K) is used to determine the concentration of lysine (K) in the sample. The sample contains 10.9 μM W and 27.9 μM K. The sample is identified against the protein of interest hen egg white lysozyme and the proteome of interest HIV. Hen egg white lysozyme has 6 W and 6 K amino acids per protein sequence and HIV has 10.9 W amino acids and 27.9 K amino acids per protein sequence. The absence of hen egg white lysozyme in the sample is identified because there is no protein concentration of hen egg white lysozyme which would result in measuring the signature of the sample. However, the signature of the sample (10.9 μM W and 27.9 μM K) is the same as the signature of HIV (10.9 W and 27.9 K) at 1 μM protein concentration, and so the presence of 1 μM HIV in the sample is identified.

It is the label, amino acid concentration, or number of each labelled amino acid type in the sample compared to the known label values, amino acid concentrations, or number of amino acids, respectively, of the same amino acid types in the protein, peptide, oligopeptide, polypeptide, protein complex, subproteome, or proteome of interest that is important, and not the order of the amino acids in the sample compared to the order of the amino acids in the protein, peptide, oligopeptide, polypeptide, protein complex, subproteome, or proteome of interest. Other state-of-the-art methods for peptide and protein identification require determination of the order of the amino acids within peptide or protein sequences of the sample.

Two or more amino acid types in the sample are labelled. An amino acid type is defined by the R-group, i.e. side chain. The R-group is specific to each amino acid type. The R-group of one amino acid type is distinguishable from the R-group of every other amino acid type. For example, R-group for tryptophan (W) is an indole group. Every W amino acid has an indole group. Therefore, the W amino acid type is defined by the indole R-group. In another example, the R-group for lysine (K) is a ε-primary amino group. Every K amino acid has this ε-primary amino group. Therefore, the K amino acid type is defined by the ε-primary amino R-group. In another example, the R-group for tyrosine (Y) is a phenol group. Every Y amino acid has a phenol group. Therefore, the Y amino acid type is defined by the phenol R-group. The R-group of the amino acid type W is distinguishable to the R-group of the amino acid type K and the R-group of the amino acid type Y. Hence, the amino acid type W is distinguishable to the amino acid type K and the amino acid type Y because of the different R-groups between these amino acid types. All the amino acid types are distinguishable from each other by their specific R-group. In some embodiments, an amino acid type is labelled independently to the other amino acid types. In some embodiments, it is the R-group of each amino acid of an amino acid type that is labelled. In some embodiments, each R-group (i.e. each amino acid type) has a unique label and so each R-group (i.e. each amino acid type) is labelled independently to the other R-groups (i.e. other amino acid types). In some embodiments, two or more R-groups (i.e. two or more amino acid types) are labelled with the same label, but each labelled R-group (i.e. each labelled amino acid type) is detected differently to another labelled R-group (i.e. another labelled amino acid type). In some embodiments, each label is targeted to an amino acid type. In some embodiments, each label is specific for an amino acid type.

In some embodiments, the two or more amino acid types are selected from alanine (A), arginine (R), asparagine (N), aspartic acid (D), cysteine (C), glutamic acid (E), glutamine (Q), glycine (G), histidine (H), isoleucine (I), leucine (L), lysine (K), methionine (M), phenylalanine (F), proline (P), pyrrolysine (S), selenocysteine (O), threonine (T), tryptophan (W), tyrosine (Y) and valine (V) or synthetic amino acids. In some embodiments, an amino acid type comprises modified amino acids and/or unmodified amino acids. In some embodiments, an amino acid type comprises modified amino acids. In some embodiments, an amino acid type comprises unmodified amino acids. In some embodiments, an amino acid type comprises both modified and unmodified amino acids. In some embodiments, when both the modified and unmodified amino acids of an amino acid type are labelled, the modified amino acids are first converted into unmodified amino acids.

In some embodiments, proteins within the sample are fluorogenically labelled with molecules whose fluorescence "turns on" exclusively after reaction with the amino acid type of interest. Therefore, separation of labelled amino acids from unreacted dye is not required, because the unreacted dye is not fluorescent and does not provide a signal. In other state of the art methods for peptide or protein identification, separation of labelled amino acids from unreacted dye is required before peptide or protein identification can take place.

The label of each labelled amino acid type in the sample is measured. For example, if the two or more amino acid types labelled in the sample are tryptophan (W) and lysine (K), then the label of tryptophan (W) is measured, and the label of lysine (K) is measured.

In some embodiments, the measured label of each amino acid type is used to calculate the concentration of that labelled amino acid type and/or the number of amino acids of that labelled amino acid type in the sample. The measured label of each amino acid type can be linearly related to each of the concentration of the amino acid type, the number of amino acids of the amino acid type, and the concentration of the sample. For example, if the two or more amino acid types labelled in the sample are tryptophan (W) and lysine (K), then the label of tryptophan (W) is measured, and the label of lysine (K) is measured. The measured label of tryptophan (W) is used to calculate the amino acid concentration of tryptophan (W) and/or the number of tryptophan (W) amino acids in the sample and/or the concentration of the sample. The measured label of tryptophan is linearly related to each of the concentration of tryptophan amino acids, the number of tryptophan amino acids, and the concentration of the sample. The measured label of lysine (K) is used to calculate the amino acid concentration of lysine (K), and/or, the number of lysine (K) amino acids, and/or the concentration of the sample. The measured label of lysine is linearly related to each of the concentration of lysine, the number of lysine amino acids, and the protein concentration of the sample.

In some embodiments, a calibration curve or standard is used to convert the values of the measured label (e.g. signals) into amino acid concentrations for each of two or more amino acid types labelled in the sample. A calibration curve or standard shows how the response of an instrument changes with the known concentration of an analyte. A standard or calibration curve provides the values of the label for one or more known amino acid concentrations of each amino acid type. This conversion can be applied to the sample or the protein, peptide, oligopeptide, polypeptide, protein complex, subproteome, or proteome of interest. For example, the calibration curve reveals that for the amino acid type tryptophan (W), to determine the known value of the label for a protein, peptide, oligopeptide, polypeptide, protein complex, subproteome, or proteome of interest at an amino acid concentration of 10 μM W, this amino acid concentration is multiplied by $100\ {}^{AU}/_{uM}$ because that is the slope of the calibration curve. The calculation indicated by the calibration curve or standard is called a calibration function or a calibration factor. A calibration factor is used if the values are multiplied or divided by a scalar, and a calibration function is used if additional steps are performed. For example, $100\ {}^{AU}/_{uM}$ is a calibration factor. There is no requirement to calculate the calibration curve or standard each time a sample is measured, instead these curves or standards can be supplied to the user who only needs to measure the label (e.g. signal) of two or more labelled amino acid types in his sample and can be provided with the calibration function or factor for each amino acid type. In this embodiment, the positive identification of the presence and/or concentration and/or amount of the sample is based on the concentration of amino acids of each labelled amino acid type of the sample. The measured label of each labelled amino acid type in the sample can be linearly related to the concentration of that amino acid type in the sample, the number of amino acids per protein of that amino acid type in the sample, and/or the protein concentration of the sample.

In some embodiments, the number of amino acids of each labelled amino acid type in the sample is calculated by dividing the amino acid concentration of each labelled amino acid type by the molar protein concentration of the sample. Therefore, it is necessary to know the molar protein concentration of the sample in order to use the value of the number of amino acids in the sample. In this embodiment, the positive identification of the presence of a protein, peptide, oligopeptide, polypeptide, protein complex, subproteome, or proteome of interest within the sample can be based on

the number of amino acids of each labelled amino acid type in the sample.

If the amino acid concentrations or known label values of *n* amino acids for the protein, peptide, oligopeptide, polypeptide, protein complex, subproteome, or proteome of interest are plotted as a function of its concentration, this provides a line in n-dimensional space, from which the concentration of the protein, peptide, oligopeptide, polypeptide, protein complex, subproteome, or proteome of interest in the sample can be determined using the equation of the line. In some embodiments, the line originates at the origin. In alternative embodiments, the line comprises the amino acid concentrations or known label values corresponding to concentrations within a known concentration range. The amino acid concentrations or measured label for the labelled amino acid types in the sample take on a point in n-dimensional space. The point of the sample can be compared to the line in the n-dimensional space to identify the presence and/or concentration and/or amount of the protein, peptide, oligopeptide, polypeptide, protein complex, subproteome, or proteome of interest in the sample.

For example, if there are 4 proteins of interest; protein-A, protein-B, protein-C, and protein-D represented in 2-dimensional space, where dimension one and two are the label values for cysteine (C) and tryptophan (W) respectively. The cysteine (C) and tryptophan (W) amino acid types are labelled in the sample and measured. Figure 1 plots the measured label values of the cysteine (C) and tryptophan (W) amino acid types labelled in the sample as a point in 2-dimensional space, against the known label values of cysteine (C) and tryptophan (W) represented as a line in 2-dimensional space for each of the four proteins of interest respectively. The known label values of the cysteine (C) and tryptophan (W) amino acid types are plotted as a function of protein concentration for proteins of interest; protein-A, protein-B, protein-C and protein-D. The known label values take on a distinct line in 2-dimensional space for each of the four proteins of interest.

In some embodiments, this line is a reference line. In Figure 1, each point on the reference line of each of the four proteins of interest corresponds to a concentration of the respective protein of interest. As the protein concentration of a protein of interest increases, the known label values of each amino acid type provided by its reference line move further from its origin. The points corresponding to a concentration of 1 μM of each protein of interest are shown with shaded circles. The value of the label of each of the cysteine (C) and tryptophan (W) amino acid types in the sample is measured, and this point is shown with an open square. In some embodiments, the shortest distance between the sample point and each reference line is calculated.

In some embodiments, the sample point lies on the reference line for a protein, peptide, oligopeptide, polypeptide, protein complex, subproteome, or proteome of interest. The presence of the protein, peptide, oligopeptide, polypeptide, protein complex, subproteome, or proteome of interest within the sample is identified, and the concentration of the protein, peptide, oligopeptide, polypeptide, protein complex, subproteome, or proteome of interest is the concentration for which the measured value of the label or amino acid concentration of each of the two or more amino acid types labelled in the sample is equivalent to the known value of the label or amino acid concentration of each of the same two or more amino acid types as were labelled in the sample.

In other embodiments, the sample point is not on the reference line, and the distance between the sample point and the reference line is calculated. In some embodiments, this distance is the length of a vector or line segment to the reference line, connecting the sample point and the reference line. The sample point is closest to a single point on the reference line for the protein, peptide, oligopeptide, polypeptide, protein complex, subproteome, or proteome of interest, corresponding to the amino acid concentrations or known values of the label of *n* amino acid types for a single concentration of the protein, peptide, oligopeptide, polypeptide, protein complex, subproteome, or proteome of interest.

In some embodiments, the presence of the protein, peptide, oligopeptide, polypeptide, protein complex, subproteome, or proteome of interest is identified in the sample if the distance between the sample point and this closest point on the reference line for the protein, peptide, oligopeptide, polypeptide, protein complex, subproteome, or proteome of interest is less than or equal to an error margin. In some embodiments, the error margin is a distance threshold. If the presence of the protein, peptide, oligopeptide, polypeptide, protein complex, subproteome, or proteome of interest is identified within the sample, then it is present at the protein concentration of the point on the reference line to which the sample point was closest.

In Figure 1, the shortest distance between the sample point and the four reference lines corresponding to the four proteins of interest was the distance between the sample point and the reference line of protein-B. The presence of protein of interest protein-B in the sample is identified. Each point on the reference line for protein of interest protein-B shows the value of the label of the cysteine (C) and tryptophan (W) amino acid types for a distinct protein concentration of protein of interest protein-B. The sample is identified as the protein concentration of the point on the reference line of protein-B which provided the smallest distance. Here, the protein concentration of the sample is 0.5 μM. Therefore, a positive identification of protein of interest protein-B in the sample can be made, and the concentration of protein of interest protein-B at 0.5 μM within the sample is simultaneously determined.

In some embodiments, if molar protein concentration of the sample is known and so the value of the number of amino acids of two or more amino acid types in the sample is available, then the number of amino acids of the same corresponding two amino acid types are plotted in n-dimensional space, providing a point for each protein, peptide,

oligopeptide, polypeptide, protein complex, subproteome, or proteome of interest. There is only one point for each protein, peptide, oligopeptide, polypeptide, protein complex, subproteome, or proteome of interest. Therefore, the point of the sample can be compared to the point for each protein, peptide, oligopeptide, polypeptide, protein complex, subproteome, or proteome of interest and the presence of a protein, peptide, oligopeptide, polypeptide, protein complex, subproteome, or proteome of interest is identified in the sample if the point of the sample is the same as the point for the protein, peptide, oligopeptide, polypeptide, protein complex, subproteome, or proteome of interest. In some embodiments, the distance between the sample point and the point for each protein, peptide, oligopeptide, polypeptide, protein complex, subproteome, or proteome of interest can be calculated, and the presence of a protein, peptide, oligopeptide, polypeptide, protein complex, subproteome, or proteome of interest is identified in the sample if the distance between the sample point and the point for the protein, peptide, oligopeptide, polypeptide, protein complex, subproteome, or proteome of interest is less than or equal to an error margin.

In some embodiments, if the measured label and/or concentration and/or number of amino acids of each labelled amino acid type in the sample is equivalent to, or within an error margin to the known label values and/or concentrations and/or number of amino acids of the same amino acid types as were labelled in the sample in the protein, peptide, oligopeptide, polypeptide, protein complex, subproteome, or proteome of interest, then a positive identification of the presence and/or concentration and/or amount of the protein, peptide, oligopeptide, polypeptide, protein complex, subproteome, or proteome of interest in the sample can be made. For example, if the amino acid concentration of tryptophan (W) amino acids and the amino acid concentration of lysine (K) amino acids in the sample is equivalent to, or within an error margin to the amino acid concentration of tryptophan (W) amino acids and the amino acid concentration of lysine (K) amino acids for a protein, peptide, oligopeptide, polypeptide, protein complex, subproteome, or proteome of interest, then a positive identification of the presence and/or concentration and/or amount of that protein, peptide, oligopeptide, polypeptide, protein complex, subproteome, or proteome of interest in the sample can be made.

In some embodiments, the minimum distance between the measured value of the label, amino acid concentration, or number of amino acids of two or more amino acid types labelled in the sample and the known values of the label, amino acid concentrations, or number of amino acids of two or more amino acid types provided for a protein, peptide, oligopeptide, polypeptide, protein complex, subproteome, or proteome of interest is calculated, and this distance is compared to the error margin.

In some embodiments, the known label values, amino acid concentrations and/or number of amino acids of two or more amino acid types provided for each of the one or more proteins, peptides, oligopeptides, polypeptides, protein complexes, subproteomes, or proteomes of interest is a reference. In some embodiments, the reference is obtained from a database. Alternatively, the reference can be calculated.

The unit of each labelled amino acid type (i.e measured label, amino acid concentration and/or number of amino acids) in the sample must be compared to the same unit of the same amino acid types (i.e known label values, amino acid concentrations and/or number of amino acids) in the protein, peptide, oligopeptide, polypeptide, protein complex, subproteome, or proteome of interest (e.g. reference). For example, if the number of amino acids of W and Y are determined in the sample, then this must be compared to the number of amino acids of W and Y in the protein, peptide, oligopeptide, polypeptide, protein complex, subproteome, or proteome of interest (e.g. reference) so that the unit (number of amino acids) of the sample is compared to the same unit (number of amino acids) of the protein, peptide, oligopeptide, polypeptide, protein complex, subproteome, or proteome of interest. If the amino acid concentration of W and Y are determined in the sample, then this must be compared to the amino acid concentration of W and Y in the protein, peptide, oligopeptide, polypeptide, protein complex, subproteome, or proteome of interest (e.g. reference) so that the unit (amino acid concentration) of the sample is compared to the same unit (amino acid concentration) of the protein, peptide, oligopeptide, polypeptide, protein complex, subproteome, or proteome of interest.. If the measured label of W and Y in the sample is not used to determine the amino acid concentration or the number of amino acids of W and Y in the sample, then the measured label of W and Y in the sample must be compared to the known label value of W and Y for the protein, peptide, oligopeptide, polypeptide, protein complex, subproteome, or proteome of interest ( e.g. reference) so that the unit (measuring the label) of the sample is compared to the same unit (the known label value) of the protein, peptide, oligopeptide, polypeptide, protein complex, subproteome, or proteome of interest. For example, the measured fluorescence intensity of W and Y in the sample is compared to the known fluorescence intensity of W and Y in the protein, peptide, oligopeptide, polypeptide, protein complex, subproteome, or proteome of interest (e.g. reference).

In some embodiments, if the units measured for the sample (i.e measured label, amino acid concentration and/or number of amino acids) are different to the units (i.e known label value, amino acid concentration and/or number of amino acids) of the protein, peptide, oligopeptide, polypeptide, protein complex, subproteome, or proteome of interest, then the unit of the protein, peptide, oligopeptide, polypeptide, protein complex, subproteome, or proteome of interest is converted into the same unit that has been measured for the sample. In some embodiments, the number of amino acids of a particular amino acid type of the one or more proteins, peptides, oligopeptides, polypeptides, protein complexes, subproteomes, or proteomes of interest is multiplied by the concentration of the protein, peptide, oligopeptide, polypeptide, protein complex, subproteome, or proteome to provide the amino acid concentration of each amino acid type in the one or more proteins,

peptides, oligopeptides, polypeptides, protein complexes, subproteomes, or proteomes of interest. For example, if the amino acid concentration of W and Y has been measured in the sample, then the number of W and Y amino acids in one or more proteins, peptides, oligopeptides, polypeptides, protein complexes, subproteomes, or proteomes of interest is converted into the corresponding amino acid concentration of W and Y in each protein, peptide, oligopeptide, polypeptide, protein complex, subproteome, or proteome of interest. This allows the unit of the sample to be compared to the same unit of the protein, peptide, oligopeptide, polypeptide, protein complex, subproteome, or proteome of interest, i.e. the measured amino acid concentration of W and Y in the sample to be compared to the amino acid concentration of W and Y in the protein, peptide, oligopeptide, polypeptide, protein complex, subproteome, or proteome of interest.

In some embodiments, the known label value, amino acid concentration and/or number of amino acids of the corresponding amino acid types in the one or more proteins, peptides, oligopeptides, polypeptides, protein complexes, subproteomes, or proteomes of interest is calculated from the amino acid sequence or sequences and/or any experimental information about post-translation modifications of each protein, peptide, oligopeptide, polypeptide, protein complex, subproteome, or proteome of interest. In some embodiments, the amino acid sequence and/or any experimental information about post-translation modifications of each protein, peptide, oligopeptide, polypeptide, protein complex, subproteome, or proteome of interest is used to calculate the number of amino acids of each amino acid type that was labelled in the sample in the one or more proteins, peptides, oligopeptides, polypeptides, protein complexes, subproteomes, or proteomes of interest. For example, if the two or more amino acid types labelled in the sample are tryptophan (W) and lysine (K), then the number of tryptophan (W) amino acids and the number of lysine (K) amino acids in a protein, peptide, oligopeptide, polypeptide, protein complex, subproteome, or proteome of interest is calculated from the protein sequence or protein sequences of that protein, peptide, oligopeptide, polypeptide, protein complex, subproteome, or proteome of interest. For example, if the two or more amino acid types labelled in the sample are tryptophan (W) and lysine (K) and the protein of interest in the sample is bovine serum albumin, then the number of tryptophan (W) and lysine (K) amino acids in the amino acid sequence of bovine serum albumin is calculated from the amino acid sequence of bovine serum albumin as 2W and 59K. As another example, if it is known via the methods disclosed herein that a protein of interest has 3 post-translational modifications on lysine (K) amino acids that make these lysine amino acids unreactive to the label, then -3 is added to the number of lysine amino acids of this protein of interest.

In some embodiments, the amino acid sequence or sequences of the one or more proteins, peptides, oligopeptides, polypeptides, protein complexes, subproteomes, or proteomes of interest is known (e.g. obtained from a database). In some embodiments, the amino acid sequence of the one or more proteins, peptides, oligopeptides, polypeptides, protein complexes, subproteomes, or proteomes of interest is determined using standard techniques of the art (e.g. Edman degradation or mass spectrometry).

In some embodiments, the number of amino acids of two or more labelled amino acid types of the one or more proteins, peptides, oligopeptides, polypeptides, protein complexes, subproteomes, or proteomes of interest is determined using the methods disclosed herein, i.e. labelling two or more amino acid types, measuring the label and using the measured label to determine the number of amino acids of each amino acid type in the one or more proteins, peptides, oligopeptides, polypeptides, protein complexes, subproteomes, or proteomes of interest, or the concentration of amino acids of each amino acid type in a sample containing each protein, peptide, oligopeptide, polypeptide, protein complex, subproteome, or proteome of interest. In this way, the presence and/or concentration and/or amount of proteins, peptides, oligopeptides, polypeptides, protein complexes, subproteomes, or proteomes of interest whose amino acid sequences are not known or not fully known can be determined.

In some embodiments, it is the number of each of the two or more amino acid types in the protein, peptide, oligopeptide, polypeptide, protein complex, subproteome, or proteome of interest, and not the order of each of the two or more amino acid types in the protein sequence or the relative composition of each of two or more amino acid types in the protein sequence, that is used to calculate the corresponding amino acid concentration and/or known label value of these amino acid types at one or more concentrations of the protein, peptide, oligopeptide, polypeptide, protein complex, subproteome, or proteome of interest.

It has been discovered that the unique signature of the known values of the labels or amino acid concentrations for each protein, peptide, oligopeptide, polypeptide, protein complex, subproteome, or proteome of interest can be provided with a vector function, or a set of parametric equations, depending on the common parameter of the concentration of each protein, peptide, oligopeptide, polypeptide, protein complex, subproteome, or proteome of interest. In some embodiments, this vector function or set of parametric equations describes and is used to calculate the reference line disclosed herein, such that the reference line can be quantitatively compared to a sample point in order to identify the presence and/or concentration and/or amount of one or more proteins, peptides, oligopeptides, polypeptides, protein complexes, subproteomes, or proteomes of interest within a sample. A set of parametric equations describes a group of quantities as functions of a common independent variable, called a parameter. The set of parametric equations can alternatively be represented as an equivalent vector function which can simplify later calculations. Comparing the values of the label or amino acid concentrations of two or more labelled amino acid types measured in the sample to the known values of the label or amino acid concentrations of the same two or more amino acid types provided as a function of

(unknown) concentration of each protein, peptide, oligopeptide, polypeptide, protein complex, subproteome, or proteome of interest allows identification of one or more proteins, peptides, oligopeptides, polypeptides, protein complexes, subproteomes, or proteomes of interest within the sample, and simultaneous identification of the concentration and/or amount of that protein, peptide, oligopeptide, polypeptide, protein complex, subproteome, or proteome of interest within the sample. Optionally, this can be achieved by creating a vector function, or set of parametric equations, describing any protein, peptide, oligopeptide, polypeptide, protein complex, subproteome, or proteome of interest.

In some embodiments, the set of parametric equations provides the signature of amino acid concentrations that would be measured for two or more amino acid types in the protein, peptide, oligopeptide, polypeptide, or protein complex of interest. The number of parametric equations describing the protein, peptide, oligopeptide, polypeptide, or protein complex of interest is the number of two or more amino acid types labelled and measured in the sample. The parametric equations describe the amino acid concentrations of each of two or more amino acid types labelled and measured in the sample of the protein, peptide, oligopeptide, polypeptide, or protein complex of interest as a function of concentration, *t*. Set of parametric equations 1 is:

$$p_i(t) = [a_1 t, a_2 t, \cdots a_n t], \forall t \geq 0$$

wherein $p_i$ are the amino acid concentrations provided for protein, peptide, oligopeptide, polypeptide, or protein complex of interest i as a function of its concentration *t*, $a_1$ is the number of amino acids of amino acid type 1 in the protein, peptide, polypeptide, oligopeptide, or protein complex of interest, $a_2$ is the number of amino acids of amino acid type 2 in the protein, peptide, polypeptide, oligopeptide, or protein complex of interest, $a_n$ is the number of amino acids of amino acid type n in the protein, peptide, polypeptide, oligopeptide, or protein complex of interest, *t* is the total molar concentration of the protein, peptide, polypeptide, oligopeptide, or protein complex of interest, and there are n parametric equations in the set for the n amino acid types labelled and measured in the sample. In some embodiments, *t* is defined for all values of *t* greater than or equal to 0, $\forall t \geq 0$. In other embodiments, *t* is provided between a lower ($c_1$) and upper ($c_2$) limit of a concentration range ($\forall t \in c_1 \leq t \geq c_2$).

Set of parametric equations 1 can alternatively be collectively described as a vector function, describing the same reference line or reference curve. The representations are interchangeable. In this representation, vector function 1 is:

$$p_i(t) = \langle 0, 0, \cdots 0 \rangle + \langle a_1 t, a_2 t, \cdots a_n t \rangle, \forall t \geq 0$$

where $p_i$ are the amino acid concentrations provided for protein, peptide, oligopeptide, polypeptide, or protein complex of interest i as a function of concentration t, $\langle 0, 0, \cdots 0 \rangle$ is the origin, $a_1$ is the number of amino acids of amino acid type 1 in the protein, peptide, polypeptide, oligopeptide, or protein complex of interest, $a_2$ is the number of amino acids of amino acid type 2 in the protein, peptide, polypeptide, oligopeptide, or protein complex of interest, $a_n$ is the number of amino acids of amino acid type n in the protein, peptide, polypeptide, oligopeptide, or protein complex of interest, *t* is the total molar concentration of the protein, peptide, polypeptide, oligopeptide, or protein complex of interest which is defined for all values of *t* greater than or equal to 0 ($\forall t \geq 0$). In alternative embodiments, *t* is provided between a lower ($c_1$) and upper ($c_2$) limit of a concentration range ($\forall t \in c_1 \leq t \geq c_2$), and the vector begins at the amino concentrations of the lower bound of the concentration range, $\langle a_1 c_1, a_2 c_1, \cdots a_n c_1 \rangle$.

For example, there are 2 proteins of interest and 1 protein complex of interest. The first protein of interest is BSA. The K ($a_1$), C ($a_2$), and W ($a_3$) amino acid types are labelled and measured in the sample. $a_1 = 59$, $a_2 = 35$, and $a_3 = 2$ because there are 59, 35, and 2 amino acids of the K, C, and W amino acid types within the protein sequence of BSA, respectively. The vector function providing the amino acid concentrations as a function of protein concentration of BSA is

$$p_{BSA}(t) = \langle 0, 0, 0 \rangle + \langle 59t, 35t, 2t \rangle, \forall t \geq 0$$

Hen egg white lysozyme (LYZ) is the second protein of interest. $a_1 = 6$, $a_2 = 8$, and $a_3 = 6$ because there are 6, 8, and 6 amino acids of the K, C, and W amino acid types within the protein sequence of LYZ, respectively. The vector function providing the amino acid concentrations as a function of protein concentration of LYZ is

$$p_{LYZ}(t) = \langle 0, 0, 0 \rangle + \langle 6t, 8t, 6t \rangle, \forall t \geq 0$$

Transthyretin is the protein complex of interest. $a_1 = 32$, $a_2 = 4$, and $a_3 = 8$ because there are 32, 4, and 8 amino acids of the K, C, and W amino acid types within all of the protein sequences comprising the protein complex of interest (the number of amino acids of each of the 4 subunits of the protein complex are summed). The vector function providing the

amino acid concentrations as a function of concentration of transthyretin (TTR) is

$$p_{TTR}(t) = \langle 0,0,0 \rangle + \langle 32t, 4t, 8t \rangle, \forall t \geq 0$$

The vector equation for BSA provides a reference line for BSA in n dimensional space (3-dimensional space, because 3 types of amino acids are labelled and measured in the experiment), the vector equation for LYZ provides a reference line for LYZ in n dimensional space, and the vector equation for TTR provides a reference line for TTR in n dimensional space. These vector equations and corresponding reference lines are plotted in Figure 2, along with a sample point. To identify the presence and/or concentration and/or amount of one of these proteins or protein complexes of interest within the sample, the distance between the sample point and each of the reference lines provided for BSA, LYZ, and TTR are calculated and compared.

Previously, methods for the identification of a whole proteome or subproteome within a sample have not been available. It has been required to identify a proteome or subproteome within a sample via separation of the proteins, peptides, oligopeptides, polypeptides, and protein complexes comprising the proteome or subproteome within the sample followed by sequential identification of each protein, peptide, oligopeptide, polypeptide, and protein complex within the proteome or subproteome.

It has been discovered that it is not necessary to separate a proteome, subproteome, or other mixture of proteins within a sample in order to identify the proteome, subproteome, or other mixture and determine the concentration or amount of the proteome, subproteome, or other mixture. It has been discovered that it is not necessary to identify every protein within a proteome, subproteome or other mixture in order to identify and determine the concentration or amount of the proteome, subproteome, or other mixture. Instead, only a single measurement of the amino acid concentration, value of the label, or number of amino acids of two or more amino acid types of a proteome, subproteome, or other mixture contained within the sample has to be made.

It has been discovered that a proteome or subproteome within a sample can be alternatively thought of as an average protein sequence whose numbers of amino acids are a weighted mean of the numbers of amino acids of each protein, peptide, oligopeptide, polypeptide, or protein complex sequence within the proteome or subproteome, and whose concentration within the sample is the total molar protein concentration of all proteins, peptides, oligopeptides, polypeptides, or protein complexes which comprise the proteome or subproteome. An unseparated proteome or subproteome within a sample can be identified and quantified in this manner, because it has been discovered that these signatures are unique for each proteome and subproteome. The order of amino acids within this average protein sequence is not calculated, and the number of amino acids of two or more amino acid types within every such average protein sequence is unique for all proteomes and subproteomes. For example, the number of amino acids of two or more amino acid types within every average protein sequence is unique for all known bacterial proteomes and all known viral proteomes (Figure 3). This is demonstrated for the 7581 known bacterial reference proteomes and the 9377 known viral reference proteomes. A reference proteome is a complete proteome. Therefore, all known bacterial proteomes and all known viral proteomes have a distinct signature which can easily be detected within a sample using the methods of the invention without separating proteins, peptides, oligopeptides, polypeptides, or protein complexes which comprise a proteome from one another. This is a counterintuitive result, because while it would be expected that the number of amino acids of two or more amino acid types of proteins, oligopeptides, polypeptides, and protein complexes within a proteome would vary according to a distribution, it would be expected that the mean of the distribution for each proteome would cluster around single values dictated by biological function. Also, the mean number of amino acids of two or more amino acid types across proteomes does not follow the trend $x = y = z$, suggesting that this variability cannot be accounted for by differences in the mean length of protein, oligopeptide, polypeptide, and protein complex sequences across proteomes.

Current methods of diagnosing infection, such as SARS-CoV-2 infection, rely on the reverse transcription polymerase chain reaction (RT-PCR) for (generally qualitative) determination of SARS-CoV-2 RNA within patient samples. However, these tests have a 30% false negative rate, which has significant consequences for patient care, infection control, and modeling.

In addition to providing a new approach for the rapid diagnosis of any infection, the methods of the invention can be applied to the identification of the presence and/or concentration and/or amount of a disease-associated subproteome of interest within a patient sample. For example, the subproteomic signature of type 1 diabetes mellitus can be identified and quantified in saliva. In some embodiments, the subproteomic signature of human ovarian cancer, human pancreatic cancer, human prostate cancer or human colorectal cancer can be identified and quantified in blood plasma samples. In some embodiments, the subproteomic signature of human bladder cancer, human prostate cancer or human renal cancer can be identified and quantified in urine samples.

In some embodiments, when one or more subproteomes or proteomes are of interest, then the number of amino acids of a particular amino acid type is the weighted mean number of amino acids of a particular amino acid type across all of the proteins in the subproteome or proteome of interest. For example, if the two or more amino acid types labelled in the

sample are tryptophan (W) and lysine (K), and the proteome of interest in the sample is the SARS-CoV-2 proteome, then the weighted mean number of tryptophan (W) and the weighted mean number of lysine (K) amino acids in the average amino acid sequence of all of the proteins of the SARS-CoV-2 proteome is calculated from the amino acid sequences of the SARS-CoV-2 proteome as 11.3 W and 60.6 K.

It has been discovered that any proteome or subproteome of interest can be described by a set of parametric equations. In some embodiments, the parametric equations provide a signature of amino acid concentrations that would be measured for two or more amino acid types in the proteome or subproteome. The set of parametric equations depending on the common parameter of concentration is set of parametric equations 2 and takes the form:

$$p_i(t) = [w_1 t, w_2 t, \cdots w_n t], \forall t \geq 0$$

where $p_i$ are the amino acid concentrations provided for proteome or subproteome of interest i as a function of proteome/subproteome concentration t (wherein the proteome/subproteome concentration is the total molar concentration of all proteins, peptides, oligopeptides, polypeptides, and protein complexes comprising proteome or subproteome of interest $p_i$), $w_1$ is the weighted mean number of amino acids of amino acid type 1 in the proteome or subproteome of interest, $w_2$ is the weighted mean number of amino acids of amino acid type 2 in the proteome or subproteome of interest, $w_n$ is the weighted mean number of amino acids of amino acid type n in the proteome or subproteome of interest, t is the proteome or subproteome concentration (wherein the proteome or subproteome concentration is the total molar concentration of all proteins, peptides, oligopeptides, polypeptides, and protein complexes comprising proteome or subproteome of interest, $p_i$). In some embodiments, the proteome or subproteome concentration *t* is defined for all values of *t* greater than or equal to 0. There are n parametric equations in the set for the n amino acid types labelled and measured in the sample.

The unique signature of amino acid concentrations provided for a proteome or subproteome of interest can be equivalently described using vector function 2:

$$p_i(t) = \langle 0, 0, \cdots 0 \rangle + \langle w_1 t, w_2 t, \cdots w_n t \rangle, \forall t \geq 0$$

where $p_i$ are the amino acid concentrations provided for proteome or subproteome of interest i as a function of the concentration, *t,* of the proteome or subproteome, $\langle 0, 0, \cdots 0 \rangle$ is the origin, $w_1$ is the weighted mean number of amino acids of amino acid type 1 in the proteome or subproteome of interest, $w_2$ is the weighted mean number of amino acids of amino acid type 2 in the proteome or subproteome of interest, $w_n$ is the number of amino acids of amino acid type n in the proteome or subproteome of interest, and t is the proteome or subproteome concentration (wherein the proteome or subproteome concentration is the total molar concentration of all proteins, peptides, oligopeptides, polypeptides, and protein complexes comprising proteome or subproteome of interest, $p_i$). In some embodiments, the proteome or subproteome concentration *t* is defined for all values of *t* greater than or equal to 0.

In some embodiments, the mean number of amino acids of each of the same two or more amino acid types as were labelled and measured in the sample in the proteome or subproteome of interest is the weighted mean number of amino acids of each of the same two or more amino acid types as were labelled and measured in the sample. In some embodiments, the weights of the weighted mean are provided by the proportion of that protein sequence within the total number of protein sequences in the proteome or subproteome of interest. For example, the weighted mean number of tryptophan (W) amino acids per proteome is equal to a linear combination of the number of tryptophan amino acids per protein sequence multiplied by the proportion of that protein sequence within all protein sequences comprising the proteome or subproteome of interest, and the weighted number of lysine (K) amino acids per proteome is equal to a linear combination of the number of tryptophan amino acids per protein sequence multiplied by the proportion of that protein sequence within all protein sequences comprising the proteome or subproteome of interest.

The amino acid concentrations measured for two or more labelled amino acid types in the sample are compared to the amino acid concentrations of the same two or more amino acid types provided for one or more proteomes or subproteomes of interest. This allows identification of the sample as one of the proteomes or subproteomes of interest as well as determination of the concentration or amount of the proteome or subproteome of interest present within the sample.

In some embodiments, the concentration of each of two or more amino acid types is the concentration of that labelled amino acid type of each protein, peptide, oligopeptide, polypeptide, or protein complex of interest. In some embodiments, the concentration of each of the two or more amino acid types of each proteome or subproteome of interest is the total concentration of that labelled amino acid type across the proteins in the proteome or subproteome of interest. This is because the concentration of the amino acid type is equal to the mean number of amino acids per sequence in the proteome multiplied by the total protein concentration of the proteome.

Frequently, the molar protein concentration of an unknown sample is not known, because if standard methods in the art are used to determine the absorption ($A_{280}$) or mass protein concentration of the sample, this cannot be converted to

the molar protein concentration of the sample unless the molecular weight of the sample is known, and the molecular weight of the sample is unknown because the identity of the sample is unknown.

In some embodiments, the molar protein concentration of the sample is known. Let the known molar protein concentration of the sample be the constant SC. Therefore, if protein, peptide, oligopeptide, polypeptide, protein complex, subproteome, or proteome of interest $p_i$ is present within the sample, then it is present at the molar protein concentration of the sample, so $t = SC$. The result of this special case is considered using the example of set of parametric equations 1:

$$p_i(t) = [a_1 t, a_2 t, \cdots a_n t], \forall t \geq 0,$$

which simplifies to a point in n dimensional space

$$p_i = (a_1 SC, a_2 SC, \ldots, a_n SC)$$

This is no longer a set of parametric equations because it is not a function of a common parameter (independent variable), because the variable *t* was replaced with the constant *SC*. In this embodiment, the amino acid concentrations for protein of interest $p_i$ instead provide a point in *n* dimensional space.

In some embodiments, the amino acid concentrations of each of two or more amino acid types of the one or more proteins, peptides, oligopeptides, polypeptides, protein complexes, subproteomes, or proteomes of interest are used to determine the corresponding label values of each of the same two or more amino acid types for the one or more proteins, peptides, oligopeptides, polypeptides, protein complexes, subproteomes, or proteomes of interest with a set of parametric equations.

This is achieved by incorporating into the parametric equations describing the amino acid concentrations of any protein, peptide, oligopeptide, polypeptide, protein complex, subproteome, or proteome of interest a calibration function or calibration factor which converts between the measured label of each amino acid type and the amino acid concentration of each amino acid type. In some embodiments, the parametric equations describe the unique signature of the label values (e.g. signals of the label) for the protein, peptide, oligopeptide, polypeptide, or protein complex of interest as a function of its concentration, t, via set of parametric equations 3:

$$p_i(t) = [a_1 f_1 t + b_1, a_2 f_2 t + b_2, \cdots a_n f_n t + b_n], \forall t \geq 0$$

Where $p_i$ are the known values of the label provided for protein, peptide, oligopeptide, polypeptide, or protein complex of interest i as a function of its concentration *t*, $a_1$ is the number of amino acids of amino acid type 1 in the protein, peptide, polypeptide, oligopeptide, or protein complex of interest, $a_2$ is the number of amino acids of amino acid type 2 in the protein, peptide, polypeptide, oligopeptide, or protein complex of interest, $a_n$ is the number of amino acids of amino acid type n in the protein, peptide, polypeptide, oligopeptide, or protein complex of interest, $b_1$ is the background value for amino acid type 1 which is 0 if the measured values of the label in the sample are background-corrected, $b_2$ is the background value for amino acid type 2 which is 0 if the measured values of the label in the sample are background-corrected, $b_n$ is the background value for amino acid type n which is 0 if the measured values of the label in the sample are background-corrected, $f_1$ is the calibration function or calibration factor for amino acid type 1, $f_2$ is the calibration function or calibration factor for amino acid type 2, $f_n$ is the calibration function or calibration factor for amino acid type n, and t is the molar concentration of the protein, peptide, polypeptide, oligopeptide, or protein complex of interest. There are n parametric equations in the set for the n amino acid types labelled and measured in the sample. In some embodiments, *t* is defined for all values of *t* greater than or equal to 0, $\forall t \geq 0$. In other embodiments, t is provided between a lower ($c_1$) and upper ($c_2$) limit of a concentration range ($\forall t \in c_1 \leq t \geq c_2$).

The equations constituting set of parametric equations 3 can equivalently be collectively described as vector function 3:

$$p_i(t) = \langle b_1, b_2, \cdots b_n \rangle + \langle a_1 f_1 t, a_2 f_2 t, \cdots a_n f_n t \rangle, \forall t \geq 0$$

Where $p_i$ are the known values of the label provided for protein, peptide, oligopeptide, polypeptide, or protein complex of interest *i* as a function of its concentration *t*, $b_1$ is the background value for amino acid type 1 which is 0 if the measured values of the label in the sample are background-corrected, $b_2$ is the background value for amino acid type 2 which is 0 if the measured values of the label in the sample are background-corrected, $b_n$ is the background value for amino acid type *n*, which is 0 if the measured values of the label in the sample are background-corrected, $a_1$ is the number of

amino acids of acid type 1 in the protein, peptide, polypeptide, oligopeptide, or protein complex of interest, $a_2$ is the number of amino acids of amino acid type 2 in the protein, peptide, polypeptide, oligopeptide, or protein complex of interest, $a_n$ is the number of amino acids of amino acid type n in the protein, peptide, polypeptide, oligopeptide, or protein complex of interest, $f_1$ is the calibration function or calibration factor for amino acid type 1, $f_2$ is the calibration function or calibration factor for amino acid type 2, $f_n$ is the calibration function or calibration factor for amino acid type n, and t is the molar protein concentration of the protein, peptide, polypeptide, oligopeptide, or protein complex of interest. In some embodiments, $t$ is defined for all values of $t$ greater than or equal to 0 ($\forall t \geq 0$). In alternative embodiments, t is provided between a lower ($c_1$) and upper ($c_2$) limit of a concentration range ( $\forall t \in c_1 \leq t \geq c_2$), and the vector begins at the values of the label of the lower bound of the concentration range,

$$\langle a_1 f_1 c_1, a_2 f_2 c_1, \cdots a_n f_n c_1 \rangle.$$

In other embodiments, the parametric equations describing the unique signature of the label values (e.g. signal of the label) for a proteome or subproteome of interest at any concentration, *t,* is set of parametric equations 4:

$$p_i(t) = [w_1 f_1 t + b_1, w_2 f_2 t + b_2, \cdots w_n f_n t + b_n], \forall t \geq 0$$

where $p_i$ are the known values of the label provided for proteome or subproteome of interest i as a function of its concentration *t*, $w_1$ is the weighted mean number of amino acids of amino acid type 1 in the proteome or subproteome of interest, $w_2$ is the weighted mean number of amino acids of amino acid type 2 in the proteome or subproteome of interest, $w_n$ is the weighted mean number of amino acids of amino acid type n in the proteome or subproteome of interest, $b_1$ is the background value for amino acid type 1 which is 0 if the measured values of the label in the sample are background-corrected, $b_2$ is the background value for amino acid type 2 which is 0 if the measured values of the label in the sample are background-corrected, $b_n$ is the background value for amino acid type n which is 0 if the measured values of the label in the sample are background-corrected, $f_1$ is the calibration function or calibration factor for amino acid type 1, $f_2$ is the calibration function or calibration factor for amino acid type 2, $f_n$ is the calibration function or calibration factor for amino acid type n, and t is the molar concentration of the proteome or subproteome of interest. There are n parametric equations in the set for the n amino acid types labelled and measured in the sample.

In some embodiments, $t$ is defined for all values of $t$ greater than or equal to 0 ($\forall t \geq 0$).

The set of parametric equations in this embodiment can alternatively be collectively described using vector function 4:

$$p_i(t) = \langle b_1, b_2, \cdots b_n \rangle + \langle w_1 f_1 t, w_2 f_2 t, \cdots w_n f_n t \rangle, \forall t \geq 0$$

where $p_i$ are the known values of the label provided for proteome or subproteome of interest i as a function of its concentration *t*, $b_1$ is the background value for amino acid type 1 which is 0 if the measured values of the label in the sample are background-corrected, $b_2$ is the background value for amino acid type 2 which is 0 if the measured values of the label in the sample are background-corrected, $b_n$ is the background value for amino acid type n which is 0 if the measured values of the label in the sample are background-corrected, $w_1$ is the weighted mean number of amino acids of amino acid type 1 in the proteome or subproteome of interest, $w_2$ is the weighted mean number of amino acids of amino acid type 2 in the proteome or subproteome of interest, $w_n$ is the weighted mean number of amino acids of amino acid type n in the proteome or subproteome of interest, $f_1$ is the calibration function or calibration factor for amino acid type 1, $f_2$ is the calibration function or calibration factor for amino acid type 2, $f_n$ is the calibration function or calibration factor for amino acid type *n,* and $t$ is the molar concentration of the proteome or subproteome of interest. In some embodiments, $t$ is defined for all values of $t$ greater than or equal to 0.

Therefore, it has been discovered that a set of parametric equations or a vector function can be constructed for any protein, peptide, oligopeptide, polypeptide, protein complex, subproteome, or proteome of interest based on the amino acid sequence or sequences of the protein, peptide, oligopeptide, polypeptide, protein complex, subproteome, or proteome of interest alone, describing the unique signatures of the label values (e.g. signals) or amino acid concentrations of two or more amino acid types of the protein, peptide, oligopeptide, polypeptide, protein complex, subproteome, or proteome of interest as a function of concentration of the protein, peptide, oligopeptide, polypeptide, protein complex, subproteome, or proteome of interest. For example, if only the label of W and Y has been measured in the sample, and it has not been converted into the amino acid concentration or number of W and Y amino acid types in the sample, then the number of W and Y amino acids in the protein, peptide, oligopeptide, polypeptide, protein complex, subproteome, or proteome of interest is converted into the corresponding known label value of W and Y as a function of the unknown

concentration of the protein, peptide, oligopeptide, polypeptide, protein complex, subproteome, or proteome of interest. This allows the measured label of W and Y in the sample to be compared to the known label value of W and Y in the protein, peptide, oligopeptide, polypeptide, protein complex, subproteome, or proteome of interest, and determination of the presence and/or concentration and/or amount of the protein, peptide, oligopeptide, polypeptide, protein complex, subproteome, or proteome of interest in the sample. In some embodiments, no calculations are required on the signals measured for the sample.

It was discovered that the vector form of the reference line or reference curve for each protein, peptide, oligopeptide, polypeptide, protein complex, subproteome, or proteome of interest allows direct calculation of the concentration of the protein, peptide, oligopeptide, polypeptide, protein complex, subproteome, or proteome of interest which provides the known values of the label or amino acid concentrations of the two or more amino acid types closest (i.e. the distance between the sample point and the reference line is minimized) to the corresponding two or more amino acid types labelled and measured in the sample.

This is achieved by finding the dot product of the direction of the reference line with the vector between the sample point and any point on the reference line, setting the dot product equal to 0, and solving for the concentration of the reference line which provides a perpendicular line between the sample point and the reference line. A dot product is a scalar value that represents the angular relationship between two vectors A and B i.e. $A \cdot B = |A| * |B| * \cos\theta$ where the values $|A|$ and $|B|$ represent the lengths of vectors A and B respectively, and $\theta$ is the angle between the two vectors. If A and B are perpendicular (i.e. at 90 degrees to each other) then the dot product will be zero, because cos 90° will be zero. This distance between the sample point and the reference line is calculated, and if this distance is less than or equal to an error margin, then the protein, peptide, oligopeptide, polypeptide, protein complex, proteome, or subproteome of interest is identified as being present at the protein concentration on the reference line which provided the minimum distance.

In some embodiments, if the sample point is less than or equal to an error margin or distance threshold from more than one protein, peptide, oligpeptide, polypeptide, protein complex, subproteome, or proteome of interest, then a mixture of proteins, peptides, oligopeptides, polypeptides, protein complexes, subproteomes, or proteomes of interest is identified in the sample. If a component within the mixture comprises a larger proportion of the mixture, then its signature will have a greater effect on the signature of the sample than will the signature of a component which comprises a smaller proportion within the mixture. The proportion of components within the mixture is also available using the methods of the invention. The proportion of each protein, peptide, oligopeptide, polypeptide, protein complex, subproteome, or proteome within the mixture is calculated by comparing the distances between the sample and each protein, peptide, oligopeptide, polypeptide, protein complex, subproteome, or proteome identified as being present in the sample, where a smaller distance indicates a larger proportion of the component within the mixture. In some embodiments, the distances calculated from the sample point to the reference line for each identified component of the mixture are compared. It was discovered that the proportion of each component within the mixture is determined from the inverse of the normalized distances for each identified component of the mixture. The maximum distance for all identified components is calculated, and this is divided by the distance for each identified component. In some embodiments, the proportion of an identified component within the mixture is calculated by dividing its inverse normalized distance by the sum of the inverse normalized distances from all components within the mixture.

The methods of the present invention do not require the order (i.e. position) of the amino acids within an amino acid sequence to be determined in order to identify the presence and/or concentration and/or amount of one or more proteins, peptides, oligopeptides, polypeptides, protein complexes, subproteomes, or proteomes of interest in the sample. The methods of the present invention do not require the sequence of amino acids within proteins in the sample to be determined in order to identify the presence and/or concentration and/or amount of one or more proteins, peptides, oligopeptides, polypeptides, protein complexes, subproteomes, or proteomes of interest in the sample.

The methods of the invention can provide a reference for a protein, peptide, oligopeptide, polypeptide, protein complex, subproteome or proteome of interest which is described algebraically using the formulas disclosed herein. There is a variable, which is protein concentration, in the reference. The reference provides the amino acid concentrations or fluorescence intensities which would be measured for any concentration of protein, peptide, oligopeptide, polypeptide, protein complex, subproteome or proteome of interest. This feature makes it possible to quantify the protein, peptide, oligopeptide, polypeptide, protein complex, subproteome or proteome of interest when it is identified. Hence, the methods disclosed herein provide a quantitative technique.

**Clauses**

Representative features are set out in the following clauses, which stand alone or may be combined, in any combination, with one or more features disclosed in the text and/or drawings of the specification.

1a. A method of identifying the presence and/or concentration and/or amount of one or more proteins, peptides, oligopeptides, polypeptides, protein complexes, subproteomes, or proteomes of interest within a sample, the method

comprising:

a) Labelling two or more amino acid types within the sample, wherein an amino acid type is defined by the R-group of the amino acid;
b) measuring the label of each labelled amino acid type in the sample;
c) optionally calculating the amino acid concentration of each labelled amino acid type from the measured label;
d) optionally calculating the number of amino acids of each labelled amino acid type; and
e) identifying the presence and/or concentration and/or amount of one or more proteins, peptides, oligopeptides, polypeptides, protein complexes, subproteomes, or proteomes of interest in the sample by comparing the measured label and/or amino acid concentration of each labelled amino acid type in the sample to the known label values and/or amino acid concentrations of the same two or more amino acid types that have been labelled in the sample of each of the one or more proteins, peptides, oligopeptides, polypeptides, protein complexes, subproteomes, or proteomes of interest at one or more concentrations, or comparing the number of amino acids of each labelled amino acid type in the sample to the known number of amino acids of the same two or more amino acid types that have been labelled in the sample in the one or more proteins, peptides, oligopeptides, polypeptides, protein complexes, subproteomes, or proteomes of interest.

1b. A method of diagnosing a bacterial and/or viral and/or parasitic disease in a sample, the method comprising:

a) Labelling two or more amino acid types within the sample, wherein an amino acid type is defined by the R-group of the amino acid;
b) measuring the label of each labelled amino acid type in the sample;
c) optionally calculating the amino acid concentration of each labelled amino acid type from the measured label;
d) optionally calculating the number of amino acids of each labelled amino acid type; and
e) identifying a bacterial and/or viral and/or parasitic disease in the sample by identifying the presence and/or concentration and/or amount of one or more bacterial, viral and/or parasitic proteins, peptides, oligopeptides, polypeptides, protein complexes, subproteomes, or proteomes of interest in the sample by comparing the measured label and/or amino acid concentration of each labelled amino acid type in the sample to the known label values and/or amino acid concentrations of the same two or more amino acid types that have been labelled in the sample of each of the one or more bacterial, viral and/or parasitic proteins, peptides, oligopeptides, polypeptides, protein complexes, subproteomes, or proteomes of interest at one or more concentrations, or comparing the number of amino acids of each labelled amino acid type in the sample to the known number of amino acids of the same two or more amino acid types that have been labelled in the sample in the one or more viral, bacterial and/or parasitic proteins, peptides, oligopeptides, polypeptides, protein complexes, subproteomes, or proteomes of interest.

1c. A method of identifying one or more bacterial proteins, peptides, oligopeptides, polypeptides, protein complexes, subproteomes, or proteomes in a sample, the method comprising:

a) Labelling two or more amino acid types within the sample, wherein an amino acid type is defined by the R-group of the amino acid;
b) measuring the label of each labelled amino acid type in the sample;
c) optionally calculating the amino acid concentration of each labelled amino acid type from the measured label;
d) optionally calculating the number of amino acids of each labelled amino acid type; and
e) identifying the presence and/or concentration and/or amount of one or more bacterial proteins, peptides, oligopeptides, polypeptides, protein complexes, subproteomes, or proteomes in the sample by comparing the measured label, amino acid concentration or number of amino acids of each labelled amino acid type in the sample to the known label values or amino acid concentrations of the same two or more amino acid types in one or more bacterial proteins, peptides, oligopeptides, polypeptides, protein complexes, subproteomes, or proteomes of interest at one or more concentrations, or number of amino acids of the same two or more amino acid types in the one or more bacterial proteins, peptides, oligopeptides, polypeptides, protein complexes, subproteomes, or proteomes of interest.

1d. A method of identifying one or more viral proteins, peptides, oligopeptides, polypeptides, protein complexes, subproteomes, or proteomes in a sample, the method comprising:

a) Labelling two or more amino acid types within the sample, wherein an amino acid type is defined by the R-group of the amino acid;

b) measuring the label of each labelled amino acid type in the sample;
c) optionally calculating the amino acid concentration of each labelled amino acid type from the measured label;
d) optionally calculating the number of amino acids of each labelled amino acid type; and
e) identifying the presence and/or concentration and/or amount of one or more viral proteins, peptides, oligopeptides, polypeptides, protein complexes, subproteomes, or proteomes of interest in the sample by comparing the measured label and/or amino acid concentration of each labelled amino acid type in the sample to the known label values and/or amino acid concentrations of the same two or more amino acid types that have been labelled in the sample of each of the one or more viral proteins, peptides, oligopeptides, polypeptides, protein complexes, subproteomes, or proteomes of interest at one or more concentrations, or comparing the number of amino acids of each labelled amino acid type in the sample to the known number of amino acids of the same two or more amino acid types that have been labelled in the sample in the one or more viral proteins, peptides, oligopeptides, polypeptides, protein complexes, subproteomes, or proteomes of interest.

1e. A method of identifying one or more parasitic proteins, peptides, oligopeptides, polypeptides, protein complexes, subproteomes, or proteomes in a sample, the method comprising:

a) Labelling two or more amino acid types within the sample, wherein an amino acid type is defined by the R-group of the amino acid;
b) measuring the label of each labelled amino acid type in the sample;
c) optionally calculating the amino acid concentration of each labelled amino acid type from the measured label;
d) optionally calculating the number of amino acids of each labelled amino acid type; and
e) identifying the presence and/or concentration and/or amount of one or more parasitic proteins, peptides, oligopeptides, polypeptides, protein complexes, subproteomes, or proteomes of interest in the sample by comparing the measured label and/or amino acid concentration of each labelled amino acid type in the sample to the known label values and/or amino acid concentrations of the same two or more amino acid types that have been labelled in the sample of each of the one or more parasitic proteins, peptides, oligopeptides, polypeptides, protein complexes, subproteomes, or proteomes of interest at one or more concentrations, or comparing the number of amino acids of each labelled amino acid type in the sample to the known number of amino acids of the same two or more amino acid types that have been labelled in the sample in the one or more parasitic proteins, peptides, oligopeptides, polypeptides, protein complexes, subproteomes, or proteomes of interest.

1f. A method of identifying one or more human proteins, peptides, oligopeptides, polypeptides, protein complexes, subproteomes, or proteomes in a sample, the method comprising:

a) Labelling two or more amino acid types within the sample, wherein an amino acid type is defined by the R-group of the amino acid;
b) measuring the label of each labelled amino acid type in the sample;
c) optionally calculating the amino acid concentration of each labelled amino acid type from the measured label;
d) optionally calculating the number of amino acids of each labelled amino acid type; and
e) identifying the presence and/or concentration and/or amount of one or more human proteins, peptides, oligopeptides, polypeptides, protein complexes, subproteomes, or proteomes of interest in the sample by comparing the measured label and/or amino acid concentration of each labelled amino acid type in the sample to the known label values and/or amino acid concentrations of the same two or more amino acid types that have been labelled in the sample of each of the one or more human proteins, peptides, oligopeptides, polypeptides, protein complexes, subproteomes, or proteomes of interest at one or more concentrations, or comparing the number of amino acids of each labelled amino acid type in the sample to the known number of amino acids of the same two or more amino acid types that have been labelled in the sample in the one or more human proteins, peptides, oligopeptides, polypeptides, protein complexes, subproteomes, or proteomes of interest.

1g. A method of detecting an infection or identifying a host response to an infection, the method comprising:

a) Labelling two or more amino acid types within the sample, wherein an amino acid type is defined by the R-group of the amino acid;
b) measuring the label of each labelled amino acid type in the sample;
c) optionally calculating the amino acid concentration of each labelled amino acid type from the measured label;
d) optionally calculating the number of amino acids of each labelled amino acid type; and
e) identifying the presence and/or concentration and/or amount of one or more proteins, peptides, oligopeptides, polypeptides, protein complexes, subproteomes, or proteomes of interest relating to an infection, or host

response to an infection in the sample by comparing the measured label and/or amino acid concentration of each labelled amino acid type in the sample to the known label values and/or amino acid concentrations of the same two or more amino acid types that have been labelled in the sample of each of the one or more proteins, peptides, oligopeptides, polypeptides, protein complexes, subproteomes, or proteomes of interest relating to an infection, or host response to an infection at one or more concentrations, or comparing the number of amino acids of each labelled amino acid type in the sample to the known number of amino acids of the same two or more amino acid types that have been labelled in the sample in the one or more proteins, peptides, oligopeptides, polypeptides, protein complexes, subproteomes, or proteomes of interest relating to an infection, or host response to an infection.

1h. A method of detecting cancer, the method comprising:

a) Labelling two or more amino acid types within the sample, wherein an amino acid type is defined by the R-group of the amino acid;
b) measuring the label of each labelled amino acid type in the sample;
c) optionally calculating the amino acid concentration of each labelled amino acid type from the measured label;
d) optionally calculating the number of amino acids of each labelled amino acid type; and
e) identifying the presence and/or concentration and/or amount of one or more proteins, peptides, oligopeptides, polypeptides, protein complexes, subproteomes, or proteomes of related to cancer in the sample by comparing the measured label and/or amino acid concentration of each labelled amino acid type in the sample to the known label values and/or amino acid concentrations of the same two or more amino acid types that have been labelled in the sample of each of the one or more proteins, peptides, oligopeptides, polypeptides, protein complexes, subproteomes, or proteomes of interest related to cancer at one or more concentrations, or comparing the number of amino acids of each labelled amino acid type in the sample to the known number of amino acids of the same two or more amino acid types that have been labelled in the sample in the one or more proteins, peptides, oligopeptides, polypeptides, protein complexes, subproteomes, or proteomes of interest related to cancer.

2. The method of any one of clauses 1a-1h, wherein the sample is a bodily fluid sample.

3. The method of clause 2, wherein the bodily fluid sample is a whole blood sample, blood serum sample, blood plasma sample, salvia sample, sputum sample, faeces sample, urine sample, semen sample, nasal swab sample, nasopharyngeal aspirate sample, throat swab, lower respiratory sample, cerebrospinal (CSF) sample, breast milk sample, sexual health sample or a tissue sample or fluid produced by a lesion.

4. The method of clause 3, wherein the sexual health sample is a urethral swab, cervix swab, vaginal swab or rectal swab.
4a. The method of clause 2, wherein the sample is a blood sample or a urine sample.

5. The method of clause 3, wherein the lower respiratory sample is a lower respiratory mucus aspirate sample.

6. The method of clause 3, wherein the tissue sample is a biopsy of a tissue.

7. The method of clause 6, wherein the tissue is a solid state tumour.

8. The method of clause 6, wherein the tissue is a sarcoma, lymphoma, carcinoma and melanoma.

9. The method of any one of clauses 1a-1h, wherein the sample is a veterinary sample.

10. The method of clause 9, wherein the veterinary sample is a feline sample, canine sample, bovine sample, porcine sample, equine sample, asinine sample, ovine sample, caprine sample, piscine sample, cancrine sample, corraline sample, homarine sample, ostracine sample, reptilian sample, avian sample, galline sample, meleagrine sample, anatine sample, anserine sample, cervine sample, leporine sample, lapine sample, noctilionine sample, murine sample, pulicine sample, ancarine sample, aedine sample, cercopithecine sample, or pholidota sample.

11. The method of any one of clauses 1a-1h, wherein the sample is a soil sample, an environmental sample, a crop sample, a food sample, a drink sample or a laboratory sample.

12. The method of clause 11, wherein the environmental sample is a water sample such as a drinking water sample or

wastewater sample; or sample suspected of biological warfare; or an astrobiological sample.

13. The method of clause 11, wherein the food sample is a functional food sample

14. The method of clause 13, wherein the functional food sample is an infant formula sample or sports nutrition sample.

15. The method of clause 11, wherein the food sample is a dietary supplement sample.

16. The method of clause 11, wherein the food sample is a fermented food sample.

17. The method of clause 11, wherein the food sample is a dairy sample, egg sample, gelatin sample, soy sample, wheat sample, vegetable sample, beans sample, nuts sample or a brewed soybean product sample.

18. The method of any clause 11, wherein the food sample is suspected of containing an allergen or bacteria or virus or parasite.

19. The method of clause 18, wherein the food sample is a meat sample, and the meat sample is suspected of containing Escherichia Coli, Salmonela, Staphylococcus Aureus, Listeria Monocytogenes, Yersinia Enterocolitica, Salmonella Enteritidis, Campylobacter Jejuni, Clostridium perfringens, Clostridium perfringens, Norovirus, Toxoplasma gondii, tapeworm, roundworm, or anisakis.

20. The method of clause 18, wherein the allergen is peanuts, gluten, lactose, shellfish, fish, sesame seeds, pollen, caseins, lipocalins, c-type lysozymes, protease inhibitors, tropomyosins, parvalbumins, cat dander or dog dander.

21. The method of clause 11, wherein the drink sample is a milk sample, water sample, fruit juice sample, kefir sample, or kombucha sample.

22. The method of clauses 1a-1h, wherein the sample is a vaccine.

23. The method of clause 22, wherein the sample is an influenza vaccine, SARS-CoV-2 vaccine, 6-in-1 vaccine, Pneumococcal vaccine, MenB vaccine, Hib/MenC vaccine, MMR vaccine, 4-in-1 preschool booster vaccine, HPV vaccine, 3-in-1 teenage booster vaccine, tetanus vaccine, shingles vaccine, BCG (TB) vaccine, Hepatitis B vaccine, or Chickenpox vaccine.

24. The method of any one of clauses 1-23, wherein the one or more proteins or peptides of interest are selected from the group consisting of: $\alpha$-synuclein, lysozyme, bovine serum albumin, ovalbumin, $\beta$-Lactoglobulin, insulin, glucagon, amyloid $\beta$, angiotensin-converting enzyme 2, angiotensin-converting enzyme, bradykinin, chordin-like protein 1, tumor necrosis factor $\beta$, osteomodulin precursor, a matrix metalloproteinase, pleiotrophin, secretogranin-3, human growth hormone, insulin-like growth factor 1, leptin, telomerase, thyroid-stimulating hormone, and any combination thereof.

25. The method of any one of clauses 1-23, wherein the one or more proteomes of interest is one or more human proteomes.

26. The method of clause 25, wherein the one or more human proteomes are selected from the group consisting of: the human plasma proteome, the human eye proteome, retina, heart, skeletal muscle, smooth muscle, adrenal gland, parathyroid gland, thyroid gland, pituitary gland, lung, bone marrow, lymphoid tissue, liver, gallbladder, testis, epididymis, prostate, seminal vesicle, ductus deferens, adipose tissue, brain, salivary gland, esophagus, tongue, stomach, intestine, pancreas, kidney, urinary bladder, breast, vagina, cervix, endometrium, fallopian tube, ovary, placenta, skin or blood proteome, human metabolic proteome, human secretory proteome, stem cell proteome, erythrocyte proteome, neutrophil proteome, eosinophil proteome, basophil proteome, monocyte proteome, lymphocyte proteome, neuron proteome, neuroglial proteome, skeletal muscle proteome, cardiac muscle proteome, smooth muscle proteome, chrondocyte proteome, osteoblast proteome, osteoclast proteome, osteocyte proteome, bone lining cell proteome, keratinocyte proteome, melanocyte proteome, merkel cell proteone, Langerhans cell proteome, endothelial cell proteome, epithelial cell proteome, white adipocyte proteome, brown adipocyte proteome, upper respiratory cell proteome, spermatozoa proteome, or ova proteome, and any combination thereof.

27. The method of any one of clause 1-23, wherein the one or more proteomes of interest is one or more human cancer

subproteomes and/or proteomes.

28. The method of clause 27, wherein the one or more human cancer proteomes and/or subproteomes are selected from the group consisting of: the human pancreatic cancer proteome, human glioma proteome, human head and neck proteome, human thyroid gland proteome, human lung proteome, human liver proteome, human testis proteome, human prostate proteome, human stomach proteome, human colon/rectum proteome, human breast proteome, human endometrium proteome, human ovary proteome, human cervix proteome, human pancreas proteome, human kidney proteome, human urinary and bladder proteome, human melanoma proteome, the human type I diabetes subproteome, the human type II diabetes subproteome, Alzheimer's disease subproteome, human Parkinson's disease subproteome, human Lewy body dementia subproteome, human dementia subproteome, human metabolic syndrome subproteome, human obesity subproteome, human cardiovascular disease subproteome, human down syndrome subproteome, human aging subproteome, human cytokine subproteome, human immune subproteome, human subproteome in response to a bacterial infection, human subproteome in response to a viral infection, human subproteome in response to a coronavirus infection, human subproteome in response to a SARS-CoV-2 infection, human subproteome in response to SARS-CoV-2 infection including IFNs, IL-6, IL1RA, CCL2, CCL8 CXCL2, CXCL8, CXCL9, AND CXCL16 and any combination thereof.

28a. The method of clause 28, wherein the one or more cancer proteome is selected from the group consisting of human ovarian cancer proteome, human pancreatic cancer proteome, human colorectal cancer proteome, human bladder cancer proteome, human prostate cancer proteome, human renal cancer proteome.

28b. The method of clause 27, wherein 1, 2, 3, 4, 5, 6, 7, 8, 9, 10, 11, 12, 13, 14, 15, 16, 17, 18, 19, 20, 21, 22, 23, 24, 25 human cancer proteomes are detected in the sample, selected from the group consisting of: pancreatic cancer, colorectal cancer, human glioma, head and neck cancer, thyroid gland cancer, lung cancer, liver cancer, testisticular cancer, prostate cancer, stomach cancer, colon/rectal cancer, breast cancer, endometrial cancer, ovarian cancer, cervical cancer, kidney cancer, renal cancer, lymphoma, bladder cancer, human melanoma, brain cancer, endometrial cancer, leukemia, urothelial cancer and any combinations thereof.

28c. The method of clause 27, 28a, or 28b, wherein the method determines the amount of one or more cancer proteomes in the sample and the amount of cancer refers to the stage or grade of the cancer in the sample.

28d. The method of clause 28c, wherein the stage is stage I, stage II, stage III or stage IV, or, the TNM staging system, such as T1, T2, T3, T4, N0, N1, N2, N3, M0 or M1.

28e. The method of clause 28c, wherein the grade is grade I, II or III.

28f. The method of clause 27, 28a, or 28b, wherein the presence of cancer refers to the location of the cancer in the patient.

29. The method of any one of clauses 1-23, wherein the one or more proteomes of interest is one or more viral proteomes.

30. The method of clause 29, wherein the one or more viral proteomes are selected from the group consisting of: human papilloma virus (HPV) proteome, human immunodeficiency virus (HIV) proteome, Orthomyxoviridae proteome, Epstein Barr proteome, Ebolavirus proteome, Rabies lyssavirus proteome, Coronovirus proteome, Novovirus proteome, Hepatitis A proteome, Hepatitis B proteome, Hepatitis C proteome, Hepatitis E proteome, Hepatitis delta proteome, Herpesvirus proteome, Papillomavirus proteome, rhinovirus proteome, Measles virus proteome, Mumps virus proteome, Poliovirus proteome, rabies proteome, rotavirus proteome, west nile virus proteome, yellow fever virus proteome, Zika virus proteome, Caudovirales proteome, Nimaviridae proteome, Riboviria proteome, Inoviridae proteome, Fuselloviridae proteome, Herpesvirales proteome, Asfarviridae proteome, Bicaudaviridae proteome, tuberculosis proteome, bovine tuberculosis proteome, and any combination thereof.

31. The method of clause 30, wherein the Orthomyxoviridae proteome is an influenza proteome.

32. The method of clause 30, wherein the influenza proteome is the Influenza A proteome, the Influenza A subtype H1N1 proteome, Influenza B proteome, Influenza C proteome and/or Influenza D proteome, or any combination thereof.

33. The method of clause 30, wherein the coronavirus proteome is SARS-CoV-2 proteome, the SARS-CoV proteome, and/or the MERS-CoV proteome .

34. The method of clause 33, wherein the coronavirus proteome is the SARS-CoV-2 proteome and any mutations thereof.

35. The method of any one of clauses 1-34, wherein the one or more proteomes of interest is one or more bacterial proteome.

36. The method of clause 35, wherein the one or more bacterial proteomes are selected from the group consisting of: Escherichia coli (E. coli) proteome, Pseudomonas aeruginosa (P. aeruginosa) proteome, Salmonella proteome, Staphylococcus aureus proteome, Acinetobacter baumannii proteome, Bacteroides fragilis proteome, Burkholderia cepacia proteome, Clostridium difficile proteome, Clostridium sordellii proteome, Enterobacteriaceae proteome, Enterococcus faecalis proteome, Klebsiella pneumoniae proteome, Methicillin-resistant Staphylococcus aureus proteome, Morganella morganii proteome, Mycobacterium proteome and any combination thereof.

37. The method of clause 36, wherein the Mycobacterium proteome is the Mycobacterium tuberculosis proteome.

38. The method of any one of clauses 1-23, wherein the one or more proteomes of interest is one or more parasitic proteomes.

39. The method of clause 38, wherein the one or more parasitic proteomes are selected from the group consisting of Plasmodium proteome, Toxoplasma gondii proteome,Trichomonas vaginalis proteome, Giardia duodenalis proteome, Cryptosporidiu proteome or any combination thereof.

40. The method of clause 39, wherein the Plasmodium proteome is the Plasmodium falciparum proteome, Plasmodium knowlesi proteome, Plasmodium malariae proteome, Plasmodium ovale proteome and/or Plasmodium vivax proteome.

41. The method of clauses 1a, 1b, 1e-1h, wherein the one or more subproteomes of interest is the host response to a parasitic proteome.

42. The method of clause 1a, 1g or 1h, wherein the one or more proteomes of interest is an archaeal proteome.

43. The method of any one of clauses 1-41, wherein the one or more proteomes of interest are a mixture of one or more bacterial proteomes, one or more viral proteomes and/or one or more parasitic proteomes and any combination thereof.

44. The method of any one of clauses 1-23, wherein the one or more proteomes of interest is a pathogenic proteome.

45. The method of clause 44, wherein the pathogenic proteome is a bacterial proteome and/or a viral proteome.

46. The method of any one of clauses 1-23, wherein the one or more proteins of interest is a prion.

47. The method of clause 46, wherein the prion causes Creutzfeldt-Jakob disease (CJD).

48. The method of any one of clauses 1-23, wherein the one or more proteomes of interest is a proteome within any bacterial family of interest.

49. The method of any one of clauses 1-23, wherein the one or more subproteomes of interest is the host response to a bacterial proteome.

50. The method of any one of clauses 1-23, wherein the presence of a bacterial proteome and the host-response subproteome are detected in the sample.

51. The method of clause 29, wherein the one or more viral proteomes of interest is a veterinary viral proteome.

52. The method of clause 51, wherein the veterinary viral proteome is a Rhabdoviruse proteome, Foot-and-mouth

disease virus proteome, Pestiviruses proteome, Arteriviruses proteome, Coronavirus proteome, Toroviruse proteome, Influenza proteome, Bluetongue virus, or Circoviruses proteome and any combination thereof.

53. The method of clause 52, wherein the Influenza proteome is an Avian influenza proteome or a Swine influenza proteome.

54. The method of clause 52, wherein the Circovirus proteome is a Herpesvirus proteome, African swine fever virus protoeme, Retrovirus proteome, Flavivirus proteome, Paramyxovirus proteome, or Parlovirus proteome.

55. The method of any one of clauses 1-54, wherein the two or more amino acid types are selected from the group consisting of: alanine (A), arginine (R), asparagine (N), aspartic acid (D), cysteine (C), glutamic acid (E), glutamine (Q), glycine (G), histidine (H), isoleucine (I), leucine (L), lysine (K), methionine (M), phenylalanine (F), proline (P), pyrrolysine (O), selenocysteine (U), serine (S), threonine (T), tryptophan (W), tyrosine (Y) and valine (V), or synthetic amino acids, the N-terminus, and the C-terminus, and any combination thereof.

56. The method of clause 55, wherein the two or more amino acid types are selected from the group consisting of: alanine (A), arginine (R), asparagine (N), aspartic acid (D), cysteine (C), glutamic acid (E), glutamine (Q), glycine (G), histidine (H), isoleucine (I), leucine (L), lysine (K), methionine (M), phenylalanine (F), proline (P), pyrrolysine (O), selenocysteine (U), serine (S), threonine (T), tryptophan (W), tyrosine (Y) and valine (V), and any combination thereof.

57. The method of clause 55, wherein the two or more amino acid types are selected from the group consisting of: arginine (R), asparagine (N), aspartic acid (D), cysteine (C), glutamic acid (E), glutamine (Q), glycine (G), histidine (H), lysine (K), methionine (M), phenylalanine (F), proline (P), pyrrolysine (O), selenocysteine (U), serine (S), threonine (T), tryptophan (W), tyrosine (Y) and any combination thereof.

58. The method of clause 55, wherein the two or more amino acid types are selected from the group consisting of: alanine (A), arginine (R), asparagine (N), aspartic acid (D), cysteine (C), glutamic acid (E), glutamine (Q), histidine (H), isoleucine (I), leucine (L), lysine (K), methionine (M), phenylalanine (F), proline (P), pyrrolysine (O), selenocysteine (U), serine (S), threonine (T), tryptophan (W), tyrosine (Y) and valine (V), and any combination thereof.

59. The method of clause 55, wherein the two or more amino acid types labelled within the sample are selected from the group consisting of: tryptophan (W), cysteine (C), tyrosine (Y), lysine (K), arginine (R), histidine (H), proline (P), aspartic acid (D), glutamic acid (E), asparagine (B), glutamine (Q), serine (S) or threonine (T) and any combination thereof.

60. The method of clause 55, wherein the two or more amino acid types labelled within the sample are selected from the group consisting of: lysine and tryptophan; cysteine (C) and tryptophan (W); lysine (K) and cysteine (C); lysine (K) and tyrosine (Y); cysteine (C) and tyrosine (Y); tryptophan (W) and tyrosine (Y); leucine (L) and serine (S); leucine (L) and lysine (K); glutamic acid (E) and leucine (L); glycine (G) and leucine (L); alanine (A) and leucine (L); aspartic acid (D) and leucine (L); leucine (L) and serine (S); leucine (L) and proline (P); leucine (L) and valine (V); lysine (K) and serine (S); glutamic acid (E) and leucine (L); alanine (A) and arginine (R); alanine (A) and glutamic acid (E); alanine (A) and glycine (G); or alanine (A) and isoleucine (I).

61. The method of clause 55, wherein the two or more amino acid types labelled within the sample are selected from the group consisting of: tryptophan (W), cysteine (C), tyrosine (Y), lysine (K), arginine (R), histidine (H), proline (P), aspartic acid (D), glutamic acid (E), asparagine (B) and/or glutamine (Q) and any combination thereof.

62. The method of clause 55, wherein the two or more amino acid types labelled within the sample are selected from the group consisting of: tryptophan (W), cysteine (C), tyrosine (Y) and/or lysine (K) and any combination thereof.

63. The method of clause 55, wherein the two or more amino acids are selected from: cysteine (C), arginine (R), histidine (H) and/or aspartic acid (D) and any combination thereof.

64. The method of clause 55, wherein the two or more amino acid types are selected from: cysteine (C), arginine (R), histidine (H) and/or glutamic acid (E) and any combination thereof.

65. The method of clause 55, wherein the two or more amino acid types are selected from: cysteine (C), arginine (R), histidine (H) and/or glutamine (Q) and any combination thereof.

66. The method of clause 55, wherein the two or more amino acid types are selected from: cysteine (C), arginine (R), tryptophan (W) and/or aspartic acid (D) and any combination thereof.

67. The method of clause 55, wherein the two or more amino acid types are selected from: Lysine (K), Arginine (R), Histidine (H) and/or Aspartic acid (D) and any combination thereof.

68. The method of clause 55, wherein the two or more amino acid types are selected from: Lysine (K), Tryptophan (W), Arginine (R) and/or Glutamic acid (E) and any combination thereof.

69. The method of clause 55, wherein the two or more amino acid types are selected from: Tyrosine (Y), Lysine (K), Cysteine (C) and/or Aspartic acid (D) and any combination thereof.

70. The method of clause 55, wherein the two or more amino acid types are selected from: Tyrosine (Y), Lysine (K), Cysteine (C) and/or Glutamic Acid (E) and any combination thereof.

71. The method of clause 55, wherein the two or more amino acid types are selected from: Proline (P), Cysteine (C), Arginine (R), and/or Glutamic Acid (E) and any combination thereof.

72. The method of clause 55, wherein the two or more amino acid types are selected from: Proline (P), Cysteine (C), Arginine (R) and/or Aspartic acid (D) and any combination thereof.

73. The method of clause 55, wherein the two or more amino acid types are selected from: Cysteine (C), Asparagine (B), Arginine (R) and/or Aspartic acid (D) and any combination thereof.

74. The method of clause 55, wherein the two or more amino acid types are selected from: Cysteine (C), Asparagine (B), Arginine (R) and/or Glutamic Acid (E) and any combination thereof.

75. The method of clause 55, wherein the two or more amino acid types are selected from: Lysine (K), Asparagine (B), Tryptophan (W) and/or Cysteine (C) and any combination thereof.

76. The method of clause 55, wherein the two or more amino acid types are selected from: Arginine (R), Histidine (H), Proline (P) and/or Aspartic acid (D) and any combination thereof.

77. The method of clause 55, wherein the two or more amino acid types are selected from: Arginine (R), Lysine (K), Cysteine (C) and/or Aspartic acid (D) and any combination thereof.

78. The method of clause 55, wherein the two or more amino acid types are selected from: Arginine (R), Lysine (K), Cysteine (C) and/or Glutamic Acid (E) and any combination thereof.

79. The method of clause 55, wherein the two or more amino acid types are selected from: Arginine (R), Lysine (K), Cysteine (C) and/or Tryptophan (W) and any combination thereof.

80. The method of clause 55, wherein the two or more amino acid types are selected from: Arginine (R), Lysine (K), Cysteine (C) and/or Tyrosine (Y) and any combination thereof.

81. The method of clause 55, wherein the two or more amino acid types are selected from: Arginine (R), Lysine (K), Histidine (H) and/or Tryptophan (W) and any combination thereof.

82. The method of clause 55, wherein the two or more amino acid types are selected from: Arginine (R), Lysine (K), Histidine (H) and/or Cysteine (C) and any combination thereof.

83. The method of clause 55, wherein the two or more amino acid types are selected from: Arginine (R), Lysine (K), Histidine (H) and/or Tyrosine (Y) and any combination thereof.

84. The method of clause 55, wherein the two or more amino acid types are selected from: Arginine (R), Cysteine (C), Tryptophan (W) and/or Tyrosine (Y) and any combination thereof.

85. The method of clause 55, wherein the two or more amino acid types are selected from: Arginine (R), Cysteine (C),

Tryptophan (W) and/or Proline (P) and any combination thereof.

86. The method of clause 55, wherein the two or more amino acid types are selected from: Tryptophan (W), Cysteine (C) and/or Lysine (K) and any combination thereof.

87. The method of clause 55, wherein the two or more amino acid types are selected from: Lysine (K), Tryptophan (W) and/or Tyrosine (Y) and any combination thereof.

88. The method of clause 55, wherein the two or more amino acid types are selected from: Tryptophan (W), Tyrosine (Y) and/or Cysteine (C) and any combination thereof.

89. The method of clause 55, wherein the two or more amino acid types are selected from: Tryptophan (W), Tyrosine (Y) and/or Lysine (K) and any combination thereof.

90. The method of clause 55, wherein the two or more amino acid types are selected from: Cysteine (C), Tryptophan (W) and/or Tyrosine (Y) and any combination thereof.

91. The method of clauses 1a-1h, wherein 2, 3, 4, 5, 6, 7, 8, 9, 10, 11, 12, 13, 14, 15, 16, 17, 18, 19 or 20, 21, 22, 23, 24, 25, 26, 27, 28, 29, 30, 31, 32, 33, 34, 35, 36, 37, 38, 39 or 40 amino acid types are labelled within a sample.

92. The method of clause 91, wherein 2 amino acid types are labelled.

93. The method of clause 92, wherein the 2 amino acid types labelled are selected from the group consisting of: Alanine (A), Arginine (R), Asparagine (N), Aspartic acid (D), Cysteine (C), Glutamic Acid (E), Glutamine (Q), Glycine (G), Histidine (H), Isoleucine (I), Leucine (L), Lysine (K), Methionine (M), Phenylalanine (F), Proline (P), Pyrrolysine (O), Selenocysteine (U), Serine (S), Threonine (T), Tryptophan (W), Tyrosine (Y) and Valine (V) and any combination thereof.

94. The method of clause 92, wherein the 2 amino acid types are Leucine (L) and Serine (S).

95. The method of clause 92, wherein the 2 amino acid types are Leucine (L) and Lysine (K).

96. The method of clause 92, wherein the 2 amino acid types are Leucine (L) and Glutamic acid (E).

97. The method of clause 92, wherein the 2 acid types are Glycine (G) and Leucine (L).

98. The method of clause 92, wherein the 2 amino acid types are Alanine (A) and Leucine (L).

99. The method of clause 92, wherein the 2 amino acid types are Aspartic acid (D) and Leucine (L).

100. The method of clause 92, wherein the 2 amino acid types are Leucine (L) and Proline (P).

101.The method of clause 92, wherein the 2 amino acid types are Leucine (L) and Valine (V).

102.The method of clause 92, wherein the 2 amino acid types are Lysine (K) and Serine (S).

103.The method of clause 92, wherein the 2 amino acid types are Glutamic acid (E) and Leucine (L).

104.The method of clause 92, wherein the 2 amino acids types are Alanine (A) and Arginine (R).

105.The method of clause 92, wherein the 2 amino acids are Alanine (A) and Glutamic acid (E).

106. The method of clause 92, wherein the 2 amino acids are Alanine (A) and Glycine (G).

107.The method of clause 91, wherein 3 amino acids types are labelled.

108. The method of clause 107, wherein the 3 amino acid types labelled are selected from the group consisting of: Alanine (A), Arginine (R), Asparagine (N), Aspartic acid (D), Cysteine (C), Glutamic Acid (E), Glutamine (Q), Glycine

(G), Histidine (H), Isoleucine (I), Leucine (L), Lysine (K), Methionine (M), Phenylalanine (F), Proline (P), Pyrrolysine (O), Selenocysteine (U), Serine (S), Threonine (T), Tryptophan (W), Tyrosine (Y) and Valine (V) and any combination thereof.

109.The method of clause 107, wherein the 3 amino acid types labelled are Tryptophan (W), Cysteine (C), and Tyrosine (Y).

110.The method of clause 107, wherein the 3 amino acid types labelled are Cysteine (C), Tyrosine (Y) and Lysine (K).

111.The method of clause 107, wherein the 3 amino acid types are Tryptophan (W), Cysteine (C) and Lysine (K).

112.The method of clause 107, wherein the 3 amino acid types are Lysine (K), Tryptophan (W) and Tyrosine (Y).

113.The method of clause 107, wherein the 3 amino acid types are Tryptophan (W), Tyrosine (Y) and Cysteine (C).

114.The method of clause 107, wherein the 3 amino acid types are Tryptophan (W), Tyrosine (Y) and Lysine (K).

115.The method of clause 107, wherein the 3 amino acid types labelled are: Cysteine (C), Tryptophan (W) and Tyrosine (Y).

116.The method of clause 107, wherein the 3 amino acid types labelled are: Asparagine (R), Glutamic Acid (E) and Glycine (G).

117.The method of clause 107, wherein the 3 amino acid types labelled are: Alanine (A), Leucine (L) and Serine (S).

118.The method of clause 107, wherein the 3 amino acid types labelled are: Asparagine (A), Glutamic Acid (E) and Leucine (L).

119.The method of clause 107, wherein the 3 amino acid types labelled are: Alanine (A), Aspartic Acid (D) and Leucine (L).

120.The method of clause 107, wherein the 3 amino acid types labelled are: Alanine (A), Leucine (L) and Proline (P).

121.The method of clause 107, wherein the 3 amino acid types labelled are: Alanine (A), Glutamic Acid (E) and Leucine (L).

122.The method of clause 107, wherein the 3 amino acid types labelled are: Leucine (L), Serine (S) and Valine (S).

123.The method of clause 107, wherein the 3 amino acid types labelled are: Glutamic Acid (E), Isoleucine (I) and Proline (P).

124.The method of clause 107, wherein the 3 amino acid types labelled are: Glutamic Acid (E), Glycine (G) and Valine (V).

125.The method of clause 107, wherein the 3 amino acid types labelled are: Arginine (R), Serine (S) and Valine (V).

126.The method of clause 107, wherein the 3 amino acid types labelled are: Alanine (A), Leucine (L) and Lysine (K).

127.The method of clause 107, wherein the 3 amino acid types labelled are: Alanine (A), Arginine (R) and Leucine (L).

128.The method of clause 107, wherein the 3 amino acid types labelled are: Alanine (A), Leucine (L) and Valine (V).

129.The method of clause 91, wherein 4 amino acid types are labelled.

130.The method of clause 129, wherein the 4 amino acid types labelled are selected from the group consisting of: alanine (A), arginine (R), asparagine (N), aspartic acid (D), cysteine (C), glutamic Acid (E), glutamine (Q), glycine (G), histidine (H), isoleucine (I), leucine (L), lysine (K), methionine (M), phenylalanine (F), proline (P), pyrrolysine (O), selenocysteine (U), serine (S), threonine (T), tryptophan (W), tyrosine (Y) and valine (V), and any combination thereof.

131. The method of clause 129, wherein the 4 amino acid types labelled are tryptophan (W), tyrosine (Y), lysine (K) and cysteine (C).

132. The method of clause 129, wherein the 4 amino acid types labelled are cysteine (C), arginine (R), Histidine (H) and aspartic acid (D).

133. The method of clause 129, wherein the 4 amino acid types labelled are Cysteine (C), Arginine (R), Histidine (H) and Glutamic Acid (E).

134. The method of clause 129, wherein the 4 amino acid types labelled are Cysteine (C), Arginine (R), Histidine (H) and Glutamine (Q).

135. The method of clause 129, wherein the 4 amino acid types labelled are Cysteine (C), Arginine (R), Tryptophan (W) and Aspartic acid (D).

136. The method of clause 129, wherein the 4 amino acid types labelled are Lysine (K), Arginine (R), Histidine (H) and Aspartic acid (D).

137. The method of clause 129, wherein the 4 amino acid types labelled are Lysine (K), Tryptophan (W), Arginine (R) and Glutamic Acid (E).

138. The method of clause 129, wherein the 4 amino acid types labelled are Tyrosine (Y), Lysine (K), Cysteine (C) and Aspartic acid (D).

139. The method of clause 129, wherein the 4 amino acid types labelled are Tyrosine (Y), Lysine (K), Cysteine (C) and Glutamic Acid (E).

140. The method of clause 129, wherein the 4 amino acid types labelled are Proline (P), Cysteine (C), Arginine (R), and Glutamic Acid (E).

**141. The method** of clause 129, wherein the 4 amino acid types labelled are Proline (P), Cysteine (C), Arginine (R) and Aspartic acid (D).

142. The method of clause 129, wherein the 4 amino acid types labelled are Cysteine (C), Asparagine (B), Arginine (R) and Aspartic acid (D).

143. The method of clause 129, wherein the 4 amino acid types labelled are Cysteine (C), Asparagine (B), Arginine (R) and Glutamic Acid (E).

144. The method of clause 129, wherein the 4 amino acid types labelled are Lysine (K), Asparagine (B), Tryptophan (W) and Cysteine (C).

145. The method of clause 129, wherein the 4 amino acid types labelled are Arginine (R), Histidine (H), Proline (P) and Aspartic acid (D).

146. The method of clause 129, wherein the 4 amino acid types labelled are Arginine (R), Lysine (K), Cysteine (C) and Aspartic acid (D).

147. The method of clause 129, wherein the 4 amino acid types labelled are Arginine (R), Lysine (K), Cysteine (C) and Glutamic Acid (E).

148. The method of clause 129, wherein the 4 amino acid types labelled are Arginine (R), Lysine (K), Cysteine (C) and Tryptophan (W).

149. The method of clause 129, wherein the 4 amino acid types labelled are Arginine (R), Lysine (K), Cysteine (C) and Tyrosine (Y).

150. The method of clause 129, wherein the 4 amino acid types labelled are Arginine (R), Lysine (K), Histidine (H) and

Tryptophan (W).

151.The method of clause 129, wherein the 4 amino acid types labelled are Arginine (R), Lysine (K), Histidine (H) and Cysteine (C).

152.The method of clause 129, wherein the 4 amino acid types labelled are Arginine (R), Lysine (K), Histidine (H) and Tyrosine (Y).

153.The method of clause 129, wherein the 4 amino acid types labelled are Arginine (R), Cysteine (C), Tryptophan (W) and Tyrosine (Y).

154.The method of clause 129, wherein the 4 amino acid types labelled are Arginine (R), Cysteine (C), Tryptophan (W) and Proline (P).

155.The method of clause 129, wherein the 4 amino acid types labelled are Glutamine (Q), Leucine (L), Lysine (K) and Valine (V).

156.**The method** of clause 129, wherein the 4 amino acid types labelled are Arginine (R), Isoleucine (I), Leucine (L) and Serine (S).

157.The method of clause 129, wherein the 4 amino acid types labelled are Alanine (A), Asparagine (N), Glutamic Acid (E), and Serine (S).

158.The method of clause 91, wherein 5 amino acid types are labelled.

159.The method of clause 158, wherein the 5 amino acid types labelled are selected from the group consisting of: Alanine (A), Arginine (R), Asparagine (N), Aspartic acid (D), Cysteine (C), Glutamic Acid (E), Glutamine (Q), Glycine (G), Histidine (H), Isoleucine (I), Leucine (L), Lysine (K), Methionine (M), Phenylalanine (F), Proline (P), Pyrrolysine (O), Selenocysteine (U), Serine (S), Threonine (T), Tryptophan (W), Tyrosine (Y) and Valine (V), and any combination thereof.

160.The method of clause 158, wherein the 5 amino acid types labelled are Arginine (R), Glutamic Acid (E), Lysine (K), Serine, and Glutamine (Q).

161.The method of clause 158, wherein the 5 amino acid types labelled are Arginine (R), Aspartic Acid (D), Lysine (K), Serine, and Glutamine (Q).

162.The method of clause 158, wherein the 5 amino acid types labelled are Arginine (R), Glycine (G), Lysine (K), Serine, and Glutamine (Q).

163.The method of clause 158, wherein the 5 amino acid types labelled are Alanine (A), Aspartic Acid (D), Glycine (G), Serine, and Arginine (R).

164.The method of clause 158, wherein the 5 amino acid types labelled are Pyrrolysine (O), Aspartic Acid (D), Glycine (G), Serine, and Arginine (R).

165.The method of clause 158, wherein the 5 amino acid types labelled are Pyrrolysine (O), Aspartic Acid (D), Selenocysteine (U), Serine, and Arginine (R).

166.The method of clause 158, wherein the 5 amino acid types labelled are Pyrrolysine (O), Aspartic Acid (D), Selenocysteine (U), Lysine, and Arginine (R).

167.The method of any one of the preceding clauses, wherein each of the two or more labelled amino acid types comprises modified amino acids and/or unmodified amino acids of an amino acid type.

168.The method of clause 167, wherein the modified amino acids of an amino acid type are post translationally modified amino acids of the amino acid type.

169.The method of clause 167 or 168, wherein 4 amino acid types are labelled and the 4 amino acid types are Cysteine (C), Tyrosine (Y) and Lysine (K) and Tryptophan (W), wherein both unmodified Cysteine (CR) amino acids, and the modified and unmodified Cysteine amino acids are labelled.

170.The method of clause 167 or 168, wherein the modified amino acids of Cysteine are disulphide bonded cysteine ($C_D$) amino acids .

171.The method of clause 167 or 168, wherein the modified amino acids of arginine are N-glycosylated Arginine (Rg) amino acids.

172.The method of clause 167 or 168, wherein the modified amino acids of asparagine are N-Glycosylated Asparagine (Ng) amino acids.

173.The method of clause 167 or 168, wherein the modified amino acids of lysine are N6-(pyridoxal phosphate)Lysine (Kp) amino acids.

174.The method of clause 167 or 168, wherein the modified amino acids of proline are 4-hydroxyproline (Ph) amino acids.

175.The method of clause 167 or 168, wherein the modified amino acids of serine are Phosphoserine (Sp) amino acids.

176.The method of clause 167 or 168, wherein the modified amino acids of threonine are Phosphothreonine (Tp) amino acids.

177.The method of clause 167 or 168, wherein the modified amino acids of Alanine are N-acetylated Alanine (An) amino acids.

178.The method of clause 167 or 168, wherein the modified amino acids of Arginine are methylated Arginine (Rm) amino acids.

179.The method of clause 167 or 168, wherein the modified amino acids of Arginine are deiminated Arginine (Ri) amino acids.

180.The method of clause 167 or 168, wherein the modified amino acids of Asparagine are deamidated Asparagine (Qa) amino acids.

181.The method of clause 167 or 168, wherein the modified amino acids of an amino acid type are amino acids that have been post-translationally modified via phosphorylation, methylation, acetylation, amidation, deamidation, deamidation, formation of pyrrolidone carboxylic acid, isomerization, hydroxylation, sulfation, flavin-binding, cysteine oxidation, cyclization, nitrosylation, acylation, formylation, alkylation, arginylation, amide bond formation, butyrylation, gamma-carboxylation, glycosylation, O-linked glycosylation, malonylation, hydroxylation, iodination, isopeptide bond formation, nucleotide addition, N-acetylation, N-myristoylation, phosphorylation, adenylylation, uridylylation, propionylation, pyroglutamate formation, S-glutathionylation, oxidation, sulfenylation, sulfonylation, succinylation, sulfation, SUMOylation, myristoylation, palmitoylation, isoprenylation, prenylation, ubiquitination, and glipyation and any combination thereof.

182.The method of clause 167 or 168, wherein both the modified and the unmodified amino acids of an amino acid type are labelled.

183.The method of clause 182, wherein both the modified and the unmodified amino acids of the amino acid type cysteine (C) are labelled.

184.The method of clause 182, wherein both the modified and unmodified amino acids of the amino acid type Tryptophan (W) are labelled.

185.The method of clause 182, wherein both the modified and unmodified amino acids of the amino acid type Tyrosine (Y) are labelled.

186.The method of clause 182, wherein both the modified and unmodified amino acids of the amino acid type Glycine (G) are labelled.

187.The method of clause 182, wherein both the modified and unmodified amino acids of the amino acid type Histidine (H) are labelled.

188.The method of clause 182, wherein both the modified and unmodified amino acids of the amino acid type Methionine (M) are labelled.

189.The method of clauses 1a-1h, wherein at least one amino acid type is a synthetic amino acid type selected from: amino acid types which contain the functional groups azide, alkyne, alkene, cyclooctyne, diene, acyl, iodo, boronic acid, diazirine, cyclooctene, epoxide, cyclopropane, sulfonic acid, sulfinic acid, biotin, oxime, nitrone, norbornene, tetrazene, tetrazole, quadricyclane, electron poor pi systems, electron rich pi systems, halogen, NHS ester, maleimide, hydrazine, hydrazone, and/or diazo and any combination thereof.

190.The method of any one of the preceding clauses, wherein all or a proportion of the amino acids of each amino acid type are labelled.

191.The method of clause 190, wherein all amino acids of each amino acid type are labelled.

192.The method of clause 190, wherein all of at least a first amino acid type are labelled, and a proportion of at least a second amino acid type are labelled.

193.The method of clause 190, wherein three amino acid types are labelled in the sample, wherein all of the amino acids of a first amino acid type are labelled, and a proportion of the amino acids of a second and third amino acid type are labelled.

194.The method of clause 190, wherein three amino acid types are labelled in the sample, wherein all of the amino acids of a first and second amino acid type are labelled, and a proportion of the amino acids of third amino acid type are labelled.

195.The method of clause 190, wherein four amino acid types are labelled in the sample, wherein all of the amino acids of a first amino acid type are labelled, and a proportion of the amino acids of a second, third and fourth amino acid type are labelled.

196.The method of clause 190, wherein four amino acid types are labelled in the sample, wherein all of the amino acids of a first and second amino acid type are labelled, and a proportion of the amino acids of a third and fourth amino acid type are labelled.

197.The method of clause 190, wherein four amino acid types are labelled in the sample, wherein all of the amino acids of a first, second and third amino acid type are labelled, and a proportion of the amino acids of a fourth amino acid type are labelled.

198.The method of clause 190, wherein five amino acid types are labelled in the sample, wherein all of the amino acids of a first amino acid type are labelled, and a proportion of the amino acids of a second, third, fourth and fifth amino acid type are labelled.

199.The method of clause 190, wherein five amino acid types are labelled in the sample, wherein all of the amino acids of a first, second, third and fourth amino acid type are labelled, and a proportion of the amino acids of a fifth amino acid type are labelled.

200.The method of clause 190, wherein five amino acid types are labelled in the sample, wherein all of the amino acids of a first and second amino acid type are labelled, and a proportion of the amino acids of a third, fourth and fifth amino acid type are labelled.

201.The method of clause 190, when dependent on clause 167 or 168, wherein five amino acid types are labelled in the sample, wherein all of the amino acids of a first, second and third amino acid type are labelled, and a proportion of the amino acids of a fourth and fifth amino acid type, wherein the unmodified amino acids of the first, second and third

amino acid type are labelled and the modified amino acids of the fourth and fifth amino acid type are labelled.

202. The method of clause 190, when dependent on clause 167 or 168, wherein all of at least a first amino acid type are labelled, and a proportion of at least a second amino acid type are labelled, wherein the unmodified amino acids of the first amino acid type are labelled and the modified amino acids of the second amino acid type are labelled.

203. The method of clause 190, when dependent on clause 167 or 168, wherein three amino acid types are labelled in the sample, wherein all of the amino acids of a first amino acid type are labelled, and a proportion of the amino acids of a second and third amino acid type are labelled, wherein the unmodified amino acids of a first amino acid type are labelled and the modified amino acids of the second and third amino acid type are labelled.

204. The method of clause 190, when dependent on clause 167 or 168, wherein three amino acid types are labelled in the sample, wherein all of the amino acids of a first and second amino acid type are labelled, and a proportion of the amino acids of third amino acid type are labelled, wherein the unmodified amino acids of the first and second amino acid type are labelled and the modified amino acids of the third amino acid type are labelled.

205. The method of clause 190, when dependent on clause 167 or 168, wherein four amino acid types are labelled in the sample, wherein all of the amino acids of a first amino acid type are labelled, and a proportion of the amino acids of a second, third and fourth amino acid type are labelled, wherein the unmodified amino acids of the first amino acid type are labelled and the modified amino acids of the second, third and fourth amino acid type are labelled.

206. The method of clause 190, when dependent on clause 167 or 168, wherein four amino acid types are labelled in the sample, wherein all of the amino acids of a first and second amino acid type are labelled, and a proportion of the amino acids of a third and fourth amino acid type are labelled, wherein the unmodified amino acids of a first and second amino acid type are labelled and the modified amino acids of the third and fourth amino acid type are labelled.

207. The method of clause 190, when dependent on clause 167 or 168, wherein four amino acid types are labelled in the sample, wherein all of the amino acids of a first, second and third amino acid type are labelled, and a proportion of the amino acids of a fourth amino acid type are labelled, wherein the unmodified amino acids of the first, second and third amino acid type are labelled and the modified amino acids of the fourth amino acid type are labelled.

208. The method of clause 190, when dependent on clause 167 or 168, wherein five amino acid types are labelled in the sample, wherein all of the amino acids of a first amino acid type are labelled, and a proportion of the amino acids of a second, third, fourth and fifth amino acid type are labelled, wherein the unmodified amino acids of a first amino acid type are labelled and the modified amino acids of the second, third, fourth and fifth amino acid type are labelled.

209. The method of clause 190, when dependent on clause 167 or 168, wherein five amino acid types are labelled in the sample, wherein all of the amino acids of a first, second, third and fourth amino acid type are labelled, and a proportion of the amino acids of a fifth amino acid type are labelled, wherein the unmodified amino acids of the first, second, third and fourth amino acid type are labelled and the modified amino acids of the fifth amino acid type are labelled.

210. The method of clause 190, when dependent on clause 167 or 168, wherein five amino acid types are labelled in the sample, wherein all of the amino acids of a first and second amino acid type are labelled, and a proportion of the amino acids of a third, fourth and fifth amino acid type are labelled, wherein the unmodified amino acids of the first and second amino acid type are labelled and the modified amino acids of the third, fourth and fifth amino acid type are labelled.

211. The method of clause 190, when dependent on clause 167 or 168, wherein five amino acid types are labelled in the sample, wherein all of the amino acids of a first, second and third amino acid type are labelled, and a proportion of the amino acids of a fourth and fifth amino acid type are labelled, wherein the unmodified amino acids of the first, second and third amino acid type are labelled and the modified amino acids of the fourth and fifth amino acid type are labelled.

212. The method of clause 190, when dependent on clause 167 or 168, wherein five amino acid types are labelled in the sample, wherein all of the amino acids of a first, second and third amino acid type are labelled, and a proportion of the amino acids of a fourth and fifth amino acid type are labelled, wherein the unmodified amino acids of the first, second and third amino acid type are labelled and the modified amino acids of the fourth and fifth amino acid type are

labelled.

213.The method of clause 190, when dependent on clause 167 or 168, wherein five amino acid types are labelled in the sample, wherein a proportion of the amino acids of a first, second and third amino acid type are labelled, and all of the amino acids of a fourth and fifth amino acid type are labelled, wherein the modified amino acids of the first, second and third amino acid type are labelled and the unmodified amino acids of the fourth and fifth amino acid type are labelled.

214.The method of clause 190, when dependent on clause 167 or 168, wherein three amino acid types are labelled in the sample, wherein all of the amino acids of a first amino acid type are labelled, and a proportion of the amino acids of a second and third amino acid type are labelled, wherein the modified amino acids of the first amino acid type are labelled and unmodified amino acids of the second and third amino acid type are labelled.

215.The method of clause 190, when dependent on clause 167 or 168, wherein three amino acid types are labelled in the sample, wherein all of the amino acids of a first and second amino acid type are labelled, and a proportion of the amino acids of third amino acid type are labelled, wherein the modified amino acids of the first and second amino acid type are labelled and the unmodified amino acids of the third amino acid type are labelled.

216.The method of clause 190, when dependent on clause 167 or 168, wherein four amino acid types are labelled in the sample, wherein all of the amino acids of a first amino acid type are labelled, and a proportion of the amino acids of a second, third and fourth amino acid type are labelled, wherein the modified amino acids of the first and second amino acid types are labelled and the unmodified amino acids of the third and fourth amino acid type are labelled.

217.The method of clause 190, when dependent on clause 167 or 168, wherein four amino acid types are labelled in the sample, wherein all of the amino acids of a first and second amino acid type are labelled, and a proportion of the amino acids of a third and fourth amino acid type are labelled, wherein the modified amino acids of the first and second amino acid type are labelled and the unmodified amino acids of the third and fourth amino acid type are labelled.

218.The method of clause 190, when dependent on clause 167 or 168, wherein four amino acid types are labelled in the sample, wherein all of the amino acids of a first, second and third amino acid type are labelled, and a proportion of the amino acids of a fourth amino acid type are labelled, wherein the modified amino acids of the first, second and third amino acid type are labelled and the unmodified amino acids of the fourth amino acid type are labelled.

219.The method of clause 190, when dependent on clause 167 or 168, wherein five amino acid types are labelled in the sample, wherein all of the amino acids of a first amino acid type are labelled, and a proportion of the amino acids of a second, third, fourth and fifth amino acid type are labelled, wherein the modified amino acids of the first amino acid type are labelled and the unmodified amino acids of the second, third, fourth and fifth amino acid type are labelled.

220.The method of clause 190, when dependent on clause 167 or 168, wherein five amino acid types are labelled in the sample, wherein all of the amino acids of a first, second, third and fourth amino acid type are labelled, and a proportion of the amino acids of a fifth amino acid type are labelled, wherein the modified amino acids of the first, second, third and fourth amino acid types are labelled and the unmodified amino acids of the fifth amino acid type is labelled.

221.The method of clause 190, when dependent on clause 167 or 168, wherein five amino acid types are labelled in the sample, wherein all of the amino acids of a first and second amino acid type are labelled, and a proportion of the amino acids of a third, fourth and fifth amino acid type are labelled, wherein the modified amino acids of the first and second amino acid type are labelled and the unmodified amino acids of the third, fourth and fifth amino acid type are labelled.

222.The method of clause 190, when dependent on clause 167 or 168, wherein five amino acid types are labelled in the sample, wherein all of the amino acids of a first, second and third amino acid type are labelled, and a proportion of the amino acids of a fourth and fifth amino acid type are labelled, wherein the modified amino acids of the first, second and third amino acid type are labelled and the unmodified amino acids of the fourth and fifth amino acid type are labelled.

223.The method of clause 190, when dependent on clause 167 or 168, wherein all of the modified amino acids of at least a first amino acid type are labelled, and a proportion of the unmodified amino acids of at least a second amino acid

type are labelled.

224.The method of clauses 1a-1h, wherein step e) comprises identifying the presence and/or concentration and/or amount of one or more proteins, peptides, oligopeptides, polypeptides, protein complexes, subproteomes, or proteomes of interest in the sample by comparing the measured label and/or amino acid concentration of each labelled amino acid type in the sample to the known label values and/or amino acid concentrations of the same two or more amino acid types that have been labelled in the sample of each of the one or more proteins, peptides, oligopeptides, polypeptides, protein complexes, subproteomes, or proteomes of interest at one or more protein concentrations, or comparing the number of amino acids of each labelled amino acid type in the sample to the known number of amino acids of the same two or more amino acid types that have been labelled in the sample in the one or more proteins, peptides, oligopeptides, polypeptides, protein complexes, subproteomes, or proteomes of interest.

225.The method of clauses 1a-1h or clause 224, wherein information indicating the known label values, and/or amino acid concentrations, and/or number of amino acids of the same two or more amino acid types as the amino acid types that have been labelled in the sample as identifying the presence and/or concentration of each protein, peptide, oligopeptide, polypeptide, protein complex, subproteome, or proteome of interest is a reference.

226.The method of clause 225, wherein the reference provides the known values of the label or amino acid concentrations of the same two or more amino acid types as the amino acid types that have been labelled in the sample of each protein, peptide, oligopeptide, polypeptide, protein complex, subproteome, or proteome of interest at one or more protein concentrations, or, wherein the reference provides the number of amino acids of the same two or more amino acid types as the amino acid types that have been labelled in the sample of each protein, peptide, oligopeptide, polypeptide, protein complex, subproteome, or proteome of interest.

227.The method of clause 226, wherein the reference provides the known values of the label or amino acid concentrations of the same two or more amino acid types as the amino acid types that have been labelled in the sample of each protein, peptide, oligopeptide, polypeptide, protein complex, subproteome, or proteome of interest at as a function of protein concentration, or, wherein the wherein the reference provides the number of amino acids of the same two or more amino acid types as the amino acid types that have been labelled in the sample of each protein, peptide, oligopeptide, polypeptide, protein complex, subproteome, or proteome of interest.

228.The method of any one of clauses 225-227, wherein the reference provides a reference line or a reference curve for each protein, peptide, oligopeptide, polypeptide, protein complex, subproteome, or proteome of interest.

229.The method of clause 228, wherein the reference line or reference curve for each protein, peptide, oligopeptide, polypeptide, protein complex, subproteome, or proteome of interest is comprised of continuous points which each provide the known label values or amino acid concentrations for the protein, peptide, oligopeptide, polypeptide, protein complex, subproteome, or proteome of interest at each concentration.

230.The method of clause 229, wherein the reference line or reference curve for each protein, peptide, oligopeptide, polypeptide, protein complex, subproteome, or proteome of interest is comprised of continuous points which each provide the known label values or amino acid concentrations for the protein, peptide, oligopeptide, polypeptide, protein complex, subproteome, or proteome of interest at each protein concentration.

231.The method of clause 229 or 230, wherein the reference line or reference curve is described parametrically, using the common parameter of concentration or protein concentration.

232.The method of clause 229 or 230, wherein the reference line or reference curve is described in vector format, using the common independent variable of concentration or protein concentration.

233.The method of clause 232, wherein the reference line or reference curve is a vector.

234.The method of clauses 228-233, wherein the measured values of the label, amino acid concentrations, or number of amino acids of two or more amino acid types labelled in the sample provide a point on the reference line or reference curve.

235.The method of clause 234, wherein the shortest distance between the sample point and the reference or reference vector is calculated.

236.The method of clause 235, wherein the shortest distance between the sample point and the reference vector is the perpendicular distance between the sample point and the reference vector.

237.The method of clause 223, 234 or 235, wherein the vector from the sample point to the reference line is determined.

238.The method of clause 233, 234, 235 or 237, wherein the dot product (·) between the vector from the sample point to the reference line and the direction of the reference line is determined, and the perpendicular distance from the sample point to the reference vector is the distance between the sample point and the specific point on the reference vector for which the dot product (·) is equal to 0.

239.The method of clause 237, wherein the equation is solved to provide the concentration, or the protein concentration, which identifies the specific point on the reference line for which the vector between the sample point and the reference line is perpendicular.

240.The method of clause 239, wherein the specific point on the reference line which provides the perpendicular distance is calculated by inputting the identified value of concentration or protein concentration into the vector function of the reference line.

241.The method of clause 236 and 240, wherein the distance between the sample point and this point on the reference line which provides the perpendicular distance is calculated.

242.The method of clauses 1a-1h and 241, wherein this perpendicular distance is compared to an error margin.

243.The method of clause 242, wherein the presence and/or concentration and/or amount of the protein, peptide, oligopeptide, polypeptide, protein complex, subproteome, or proteome of interest is identified if the perpendicular distance between the sample point and its reference line is less than or equal to an error margin, and wherein the concentration or protein concentration of the protein, peptide, oligopeptide, polypeptide, protein complex, subproteome, or proteome of interest is the concentration or protein concentration which provided this perpendicular distance.

244.The method of any one of clauses 190 or 192-223, wherein a proportion of the amino acids of an amino acid type are labelled, and wherein the proportion is about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, or about 79%, about 80%, about 81%, about 82%, about 83%, about 84%, about 85%, about 86%, about 87%, about 89%, about 90%, about 91%, about 92%, about 93%, about 94%, about 95%, about 96%, about 97%, about 98% or about 99% of the amino acids of an amino acid type are labelled

245.The method of clause 167 or 168, wherein the modified amino acids of an amino acid type are labelled differently to the unmodified amino acids of an amino acid type.

246.The method of clause 182, wherein the unmodified amino acids of an amino acid type are labelled differently to the total of the modified and unmodified amino acids.

247.The method of clauses 245 or 246, wherein the modified amino acids of an amino acid type are labelled by first converting them to unmodified amino acids of that amino acid type.

248.The method of clause 245 or 246, wherein the modified amino acids of an amino acid type are labelled by first converting them to unmodified amino acids of an amino acid type by a chemical transformation.

249.The method of clause 245 or 246, wherein the modified amino acids of an amino acid type are labelled by first converting them to unmodified amino acids of an amino acid type by a chemical reaction.

250.The method of clause 245 or 246, wherein the modified amino acids of an amino acid type are labelled by first converting them to unmodified amino acids of an amino acid type by a reduction step.

251.The method of clause 245 or 246, wherein the modified amino acids of an amino acid type are labelled by first

converting them to unmodified amino acids of an amino acid type by a PTM cleavage step.

252.The method of clause 245 or 246, wherein the modified amino acids of an amino acid type are labelled by first converting them to unmodified amino acids of an amino acid type by a hydrolysis step.

253.The method of clause 245 or 246, wherein the modified amino acids of an amino acid type are labelled by first converting them to unmodified amino acids of an amino acid type using an enzyme.

254.The method of clause 253, wherein the enzyme is removed from the sample prior to the labelling step.

255.The method of clause 246, wherein the unmodified amino acids of cysteine ($C_R$) amino acids are labelled differently to when both of the modified and unmodified amino acids of cysteine are labelled.

256.The method of any one of the preceding clauses, wherein the labelling of each of the amino acid types is specific for that amino acid type.

257.The method of any one of the preceding clauses, wherein the R-group of the amino acids of an amino acid type is labelled.

258.The method of clause 257, wherein the R-group of the modified and/or unmodified amino acids of an amino acid type are labelled.

259.The method of clause 258, wherein the R-group labelled for unmodified A amino acids is methyl.

260.The method of clause 258, wherein the R-group labelled for unmodified R amino acids is an aliphatic guanidino group.

261.The method of clause 260, wherein the aliphatic guanidino group is a partial primary amine character and/or an equal primary amine character.

262.The method of clause 258, wherein the R-group labelled for modified R amino acids (Rg) is carbohydrate glycoside bonded to guanidino amine. 263.The method of clause 258, wherein the R-group labelled for modified R amino acids (Rm) is methylated guanidino amine.

264.The method of clause 258, wherein the R-group labelled for modified R amino acids (Rc) is citrulline.

265.The method of clause 258, wherein the R-group labelled for modified A amino acids (Aa) is N-acetylated alanine at the N-terminus

266.The method of clause 258, wherein the R-group labelled for unmodified N amino acids is β-carboxamide.

267.The method of clause 258, wherein the R-group labelled for modified N amino acids (Ng) is carbohydrate glycoside bonded to β-carboxamide amine.

268.The method of clause 258, wherein the R-group labelled for modified N amino acids (Nd) is a carboxylic acid (aspartic acid, D, or isoaspartic acid, isoD)

269.The method of clause 258, wherein the R-group labelled for modified D amino acids (Di) is a carboxylic acid (isoaspartic acid)

270. The method of clause 258, wherein the R-group labelled for modified and unmodified C amino acids is reduced thiols.

271.The method of clause 258, wherein the R-group labelled for unmodified C amino acids ($C_R$) is reduced thiols.
**272.The method** of clause 258, wherein the R-group labelled for modified C amino acids ($C_D$) is oxidized thiols.

273.The method of clause 258, wherein the R-group labelled for modified C amino acids (Cfe) is sulfenic acid.

274.The method of clause 258, wherein the R-group labelled for modified C amino acids (Cfu) is sulfonic acid.

275.The method of clause 258, wherein the R-group labelled for modified C amino acids (Cp) is palmitoylated thiol.

276.The method of clause 258, wherein the R-group labelled for modified C amino acids (Cn) is N-acetylated cysteine at the N-terminus.

277.The method of clause 258, wherein the R-group labelled for modified C amino acids (Cno) is S-nitrosothiol.

278.The method of clause 258, wherein the R-group labelled for modified E amino acids (Ep) is pyroglutamate.

279.The method of clause 258, wherein the R-group labelled for modified E amino acids (Ep) is pyroglutamate at the N-terminus.

280.The method of clause 258, wherein the R-group labelled for modified E amino acids (Ec) is γ-dicarboxyic acid.

281.The method of clause 258, wherein the R-group labelled for modified E amino acids (Ec) is γ-dicarboxyic acid.

282.The method of clause 258, wherein the R-group labelled for modified Q amino acids (Qp) is pyroglutamate at the N-terminus.

283.The method of clause 258, wherein the R-group labelled for modified Q amino acids (Qe) is a γ-carboxylic acid.

284.The method of clause 258, wherein the R-group labelled for modified Q amino acids (Qip) is an isopeptide bond with a K amino acid.

285.The method of clause 258, wherein the R-group labelled for modified G amino acids (Gm) is N-Myristoyl at the N-terminus.

286.The method of clause 258, wherein the R-group labelled for modified G amino acids is N-acetyl at the N-terminus.

287.The method of clause 258, wherein the R-group labelled for modified H amino acids (Hp) is phosphoimidazole.

288.The method of clause 258, wherein the R-group labelled for modified K amino acids (Ka) is ε-secondary amino group with an acetyl substituent.

289.The method of clause 258, wherein the R-group labelled for modified K amino acids (Ku) is an ε-secondary amino group with a Ubiquitin substituent.

290.The method of clause 258, wherein the R-group labelled for modified K amino acids (Ks) is an ε-secondary amino group SUMOyl substituent.

291.The method of clause 258, wherein the R-group labelled for modified K amino acids (Km) is an ε-secondary amino group with a methyl substituent.

292.The method of clause 258, wherein the R-group labelled for modified K amino acids (Ki) is an ε-secondary amino group with an isopeptide bond to glutamine.

293.The method of clause 258, wherein the R-group labelled for modified K amino acids (Kh) is an ε-secondary amino group with a hydroxyl substituent.

294.The method of clause 258, wherein the R-group labelled for modified M amino acids (Ma) is N-acetyl at the N-terminus.

295.The method of clause 258, wherein the R-group labelled for modified M amino acids (Mu) is thioester-linked ubiquitin.

296.The method of clause 258, wherein the R-group labelled for modified M amino acids (Msx) is sulfoxide.

297.The method of clause 258, wherein the R-group labelled for modified M amino acids (Mso) is sulfone.

298.The method of clause 258, wherein the R-group labelled for modified P amino acids (Ph) is hydroxypyrrolidine.

299.The method of clause 258, wherein the R-group labelled for modified S amino acids (Sp) is hydroxymethyl phosphate.

300.The method of clause 258, wherein the R-group labelled for modified S amino acids (Sg) is hydroxymethyl glycoside.

301.The method of clause 258, wherein the R-group labelled for modified S amino acids (Sn) is N-acetyl at the N-terminus.

302.The method of clause 258, wherein the R-group labelled for modified T amino acids (Tp) is hydroxy phosphate.

303.The method of clause 258, wherein the R-group labelled for modified T amino acids (Tg) is hydroxy glycoside.

304.The method of clause 258, wherein the R-group labelled for modified T amino acids (Tn) is N-acetyl at the N-terminus.

305.The method of clause 258, wherein the R-group labelled for modified W amino acids (Wmo) is indoleol (a mono hydroxyl indole).

306.The method of clause 258, wherein the R-group labelled for modified W amino acids (Wdo) is indolediol (a di hydroxyl indole).

307.The method of clause 258, wherein the R-group labelled for modified W amino acids (Wk) is Kynurenine.

308.The method of clause 258, wherein the R-group labelled for modified Y amino acids (Ys) is phenyl sulfate.

309.The method of clause 258, wherein the R-group labelled for modified Y amino acids (Yp) is phenyl phosphate.

310.The method of clause 258, wherein the R-group labelled for modified V amino acids (Vn) is N-acetyl at the N terminus.

311.The method of clause 258, wherein the R-group labelled for unmodified E amino acids is $\gamma$-carboxylic acid.

312.The method of clause 258, wherein the R-group labelled for unmodified Q amino acids is $\gamma$-carboxamide.

313.The method of clause 258, wherein the R-group labelled for unmodified G amino acids the alpha carbon on which hydrogen is a substituent.

314.The method of clause 258, wherein the R-group labelled for unmodified H amino acids is Imidazole.

315.The method of clause 258, wherein the R-group labelled for unmodified I amino acids is secondary butyl.

316.The method of clause 258, wherein the R-group labelled for unmodified L amino acids is isobutyl.

317.The method of clause 258, wherein the R-group labelled for unmodified K amino acids is $\varepsilon$-primary amino group.

318.The method of clause 258, wherein the R-group labelled for modified K amino acids is Pyridoxyal phosphate aldimine.

319.The method of clause 258, wherein the R-group labelled for unmodified M amino acids is S-methyl thioether.

320.The method of clause 258, wherein the R-group labelled for unmodified F amino acids is Benzyl.

321.The method of clause 258, wherein the R-group labelled for unmodified P amino acids is pyrrolidine.

322.The method of clause 258, wherein the R-group labelled for modified P amino acids (Ph4) is 4-hydroxypyrrolidine.

323.The method of clause 258, wherein the R-group labelled for S unmodified amino acids is hydroxymethyl.

324.The method of clause 258, wherein the R-group labelled for modified S amino acids (Sp) is Phospho methyl ester.

325.The method of clause 258, wherein the R-group labelled for unmodified T amino acids is hydroxyl.

326.The method of clause 258, wherein the R-group labelled for modified T amino acids (Tp) is Phosphoester.

327.The method of clause 258, wherein the R-group labelled for unmodified W amino acids is indole.

328.The method of clause 258, wherein the R-group labelled for unmodified Y amino acids is phenol.

329.The method of clause 258, wherein the R-group labelled for modified Y amino acids (Yp) is Phosphophenol.

330.The method of clause 258, wherein the R-group labelled for unmodified V amino acids is Isopropyl.

331.The method of clause 258, wherein the R-group for pyrrolysine (O) is pyrrol (N,2,3-trimethyl-3,4-dihydro-2H-pyrrole-2-carboxamide).

332.The method of clause 258, wherein the R-group for selenocysteine (U) is ethylselenol.

333.The method of clause 258, wherein the R-group for modified and unmodified W amino acids is an indole group, wherein the R-group for mono-oxidized (modified) W amino acids is a hydroxy indole group, and the R-group for dioxidzed (modified) W amino acids is an dihydroxy indole group.

334.The method of clause 258, wherein the R-group for unmodified K amino acids is an ε-primary amino group, wherein the R-group for acetylated (modified) K is an acetylated ε-secondary amino group, the R-group for ubiquitinated (modified) K is an ubiquitinated ε-secondary amino group, the R-group for SUMOlyated (modified) K is an SUMOlyated ε-secondary amino group, and the R-group for methylated (modified) K is a methylated (alkylated) ε-secondary amino group

335.The method of clause 258, wherein the R-group for modified and unmodified Y amino acids is a phenol group, wherein the R-group for sulfated (modified) Y amino acids is a phenol sulfate group, and the R-group for phosphorylated (modified) Y amino acids is a phosphophenol group.

336.The method of any one of clauses 257-335, wherein the labelling of the R-group of each amino acid type is specific for that amino acid type.

337.The method of any one of clauses 257-335, wherein the labelling of the R-group of each unmodified amino acid type is specific for that unmodified amino acid type.

338.The method of any one of clauses 257-335, wherein the labelling of the R-group of each modified amino acid type is specific for that amino acid type.

339.The method of any one of clauses 257-335, wherein the labelling of R-groups of modified amino acid types with the same substituent is specific to the substituent of the R-group

340.The method of clause 336, wherein labelling of R-groups containing a phosphate is specific for R-groups containing a phosphate, allowing detection of all phosphorylated amino acid types.

341.The method of clause 336, wherein labelling of R-groups containing a glycoside is specific for R-groups containing a glycoside and comprises Selective conversion to azide with TT/n-Bu4NN3 or Ph3P:2,3-dichloro-5,6-dicyanobenzoquinone (DDQ):n-Bu4NN3 followed by reaction with FI-DIBO

342.The method of clause 336, wherein labelling of R-groups containing a fatty acid is specific for R-groups containing a fatty acid comprises labelling with Dipolar 3-methoxychromones, allowing detection of all lipidated amino acid types.

343.The method of clause 336, wherein labelling of R-groups containing a phosphate comprises activation with carbonyldiimidazole to provide a leaving group, followed by reaction with a cysteine BODIPY dye, and is specific for R-groups containing a phosphate, allowing detection of all amino acid types modified with a phosphate.

344.The method of any of the preceding clauses, wherein any peptides, oligopeptides, polypeptides, proteins, protein complexes, or peptides, oligopeptides, polypeptides, proteins, or protein complexes within subproteomes or proteomes are denatured during or prior to the labeling reaction of the amino acid types in the sample.

345.The method of clause 344, wherein peptides, oligopeptides, polypeptides, proteins, protein complexes, or peptides, oligopeptides, polypeptides, proteins, or protein complexes within subproteomes or proteomes are denatured during or prior to the labeling reaction of the amino acid types in the sample using an organic solvent

346.The method of clause 344, wherein peptides, oligopeptides, polypeptides, proteins, protein complexes, or peptides, oligopeptides, polypeptides, proteins, or protein complexes within subproteomes or proteomes are denatured during or prior to the labeling reaction of the amino acid types in the sample using a surfactant.

347.The method of clause 344, wherein peptides, oligopeptides, polypeptides, proteins, protein complexes, or peptides, oligopeptides, polypeptides, proteins, or protein complexes within subproteomes or proteomes are denatured during or prior to the labeling reaction of the amino acid types in the sample using reducing agent.

348.The method of clause 344, wherein peptides, oligopeptides, polypeptides, proteins, protein complexes, or peptides, oligopeptides, polypeptides, proteins, or protein complexes within subproteomes or proteomes are denatured during or prior to the labeling reaction of the amino acid types in the sample using high or low pH conditions.

349. The method of clause 344, wherein peptides, oligopeptides, polypeptides, proteins, protein complexes, or peptides, oligopeptides, polypeptides, proteins, or protein complexes within subproteomes or proteomes are denatured during or prior to the labeling reaction of the amino acid types in the sample using any combination of an organic solvent, surfactant, reducing agent, or high or low pH conditions.

350.The method of any one of clauses 1a-1h or 2-256, wherein two or more amino acid types are labelled with the same label and the label is independently identified for each amino acid type.

351.The method of clause 350, wherein the parameters for detecting the label are distinct.

352.The method of clause 350, wherein the labelling reactions are distinct.

353.The method of clause 350, wherein one amino acid type is converted into a reactive form under different conditions from another amino acid type, before reaction with the label.

354.The method of clause 353, wherein different catalysts are used during the labeling reactions.

355.The method of clause 353, wherein different wavelengths of light are used to catalyze the labelling reactions.

356.The method of clause 353, wherein a different chemical reaction is performed on an amino acid type to install a reactive group prior to reaction with the label.

357.The method of clause 349, wherein different reaction times are used. In embodiments when one amino acid type reacts more rapidly with the label than another amino acid type.

358.The method of clause 350 or 351, wherein the measured label for one amino acid type is deconvoluted from the label for a second amino acid type.

359.The method of clause 358, wherein the measured label for one amino acid type is deconvoluted from the label for a second amino acid type using a deconvolution standard which contains only amino acids of one of the labelled amino acid types.

360.The method of clause 358 or 359, wherein the amino acid types tryptophan (W) and tyrosine (Y) are labelled with the same label and the measured label for W amino acids is deconvoluted from the label for Y amino acids.

361.The method of clause 358 or 359, wherein the amino acid types tryptophan (W) and tyrosine (Y) are labelled with the same label and the measured label for W amino acids is detected separately from the measured label for W and Y amino acids, using separate excitation wavelengths.

362.The method of clause 358 or 359, wherein the amino acid types tryptophan (W) and tyrosine (Y) are labelled with the same label and the measured label for W amino acids at the excitation wavelength at which both W and Y amino acids are labelled is calculated using a deconvolution standard containing only W amino acids, and this is subtracted from the total value of the label for both W and Y amino acids to reveal the value of the label exclusively for Y amino acids.

363.The method of any one of the preceding clauses, wherein two or more amino acid types are labelled within the whole sample.

364.The method of any one of clauses 1-363, wherein the sample is separated into multiple fractions and different labelling reactions are performed in each fraction which label specifically two or more of the amino acid types.

365.The method of clause 364, wherein the fractions have equal volume.

366.The method of clause 364 or 365, wherein 4 amino acid types are being labelled and the sample is separated into two fractions before labelling, wherein two amino acid types are labelled in one fraction and the two other amino acid types are labelled in a second fraction.

367.The method of clause 366, wherein the 4 amino acid types W, K, Y and C are being labelled and the sample is separated into two fractions before labelling, wherein W and K amino acids are labelled in one fraction and Y and C are labelled in a second fraction.

368.The method of clause 366, wherein the 4 amino acid types W, K, Y and C are being labelled and the sample is separated into three fractions before labelling, wherein W and Y amino acids are labelled in one fraction and C and K amino acids are labelled in separate fractions.

369.The method of clause 364 or 365, wherein 4 amino acid types are being labelled and the sample is separated into 4 fractions before labelling, wherein one amino acid type is labelled in each fraction.

370.The method of clause 369, wherein the amino acid types W, K, Y and C are being labelled and the sample is separated into 4 fractions before labelling, wherein W is labelled in the first fraction, K is labelled in the second fraction, C is labelled in the third fraction, Y is labelled in the fourth fraction.

371.The method of clause 364 or 365, wherein the number of fractions is equal to the number of amino acid types labelled in the sample.

372.The method of clause 364 or 365, wherein each fraction contains all amino acid types, because the amino acid types are contained on intact protein or peptide chains which have not been digested or hydrolyzed.

373.The method of clause 364 or 365, wherein the number of fractions is not equal to the number of amino acid types labelled in the sample, and more than one amino acid type is labelled per fraction.

374.The method of clause 364 or 365, wherein two or more amino acid types have the same label and they are labelled in different fractions.

375.The method of any of the preceding clauses, wherein the labeling reactions are performed in bulk and not in a microfluidic device.

376.The method of any one of the preceding clauses, wherein the label of the sample and/or the known label value of the proteins, peptides, oligopeptides, polypeptides, protein complexes, subproteomes, proteomes of interest provides a signal.

377.The method of any one of the preceding clauses, wherein the label of the sample is a fluorophore.

378.The method of any one of the preceding clauses, wherein the label of the sample is a reactive derivative of a fluorophore.

379.The method of clause 377 or 378, wherein the label of the sample is a fluorescent label.

380.The method of clause 379, wherein the fluorescent label is a fluorescent probe.

381.The method of clause 380, wherein the fluorescent label is a fluorescent tag.

382.The method of clause 380, wherein the fluorescent label is a fluorescent protein.

383.The method of clause 380, wherein the fluorescent label is a fluorescent dye.

384.The method of clause 380, wherein the fluorescent label includes a reactive group which is specific for an amino acid type.

385.The method of clause 380, wherein the fluorescent label includes a reactive group that targets an amino acid type.

386.The method of clause 380, wherein the fluorescent label includes a reactive group which is specific for the R-group of an amino acid type.

387.The method of clause 380, wherein the fluorescent label includes a reactive group that targets the R-group an amino acid type.

388.The method of clause 380, wherein the fluorescent label includes a reactive group that is specific for the N or C terminus of the protein.

389.The method of clause 380, wherein the fluorescent label includes a reactive group that targets the N or C terminus of the protein.

390.The method of clause 380, wherein the fluorescent label includes a quantum dot.

391.The method of any of the proceeding clauses, wherein the label of the sample includes a nanoparticle.

392.The method of clauses 379-390, wherein the fluorescent label includes a fluorophore.

393.The method of clause 392, wherein the fluorophore is selected from the group consisting of: Hydroxycoumarin, Aminocoumarin, Methoxycoumarin, Cascade Blue, Pacific Blue, Pacific Orange, Lucifer yellow, NBD, R-Phycoerythrin (PE), PE-Cy5 conjugates, PE-Cy7 conjugates, Red 613, PerCP, TruRed, FluorX, BODIPY-FL, G-Dye100, G-Dye200, G-Dye300, G-Dye400, Cy2, Cy3, Cy3B, Cy3.5, Cy5, Cy5.5, Cy7, TRITC, X-Rhodamine, Lissamine Rhodamine B, Texas Red, Allophycocyanin (APC), APC-Cy7 conjugates, DAPI, Hoechst 33258, SYTOX Blue, Chromomycin A3, Mithramycin, YOYO-1, ATTO 390, ATTO 425, ATTO 465, ATTO 488, ATTO 495, ATTO 514, ATTO 520, ATTO 532, ATTO Rho6G, ATTO 550, ATTO 565, ATTO Rho3B, ATTO Rho11, ATTO Rho12, ATTO Thio12, ATTO Rho101, ATTO 590, ATTO Rho13, ATTO 594, ATTO 610, ATTO Rho14, ATTO 633, ATTO 647, ATTO 647N, ATTO 655, ATTO Oxa12, ATTO 665, ATTO Oxa12, ATTO 665, ATTO 680, ATTO 700, ATTO 725, ATTO 740, Brilliant Violet 421, Brilliant Violet 510, Brilliant Violet 570, Brilliant Violet 605, Brilliant Violet 650, Brilliant Violet 711, Brilliant Violet 750, Brilliant Violet 785, TM-BDP, KFL-1, KFL-2, KFL-3, KFL-4, Super Bright 436, Super Bright 600, Super Bright 645, Super Bright 702, Super Bright 780, Alexa Flour 350, Alexa Flour 405, Alexa Flour 488, Alexa Flour 532, Alexa Flour 546, Alexa Flour 555, Alexa Flour 568, Alexa Flour 594, Alexa Flour 647, Alexa Flour 680, Alexa Flour 850, Coumarin, Pacific Green, Oregon Green, Flourescein (FITC), PE-Cyanine7, PerCP-Cyanine5.5, Tetramethylrhodamine (TRITC), eFlour 450, eFlour506, eFlour660, PE-eFlour 610, PerCP-eFlour 710, APC-eFlour 780, Super Bright 436, Super Bright 600, Super Bright 645, Super Bright 702, Super Bright 780, DAPI, SYTOX Green, SYTO 9, TO-PRO-3, Qdot 525, Qdot 565, Qdot 605, Qdot 655, Qdot 705, Qdot 800, R-Phycoerythrin (R-PE), CFP, GFP (emGFP), RFP (tagRFP), VioBlue, VioGreen, VioBright 515, Vio 515, VioBright FITC, PE, PE-Vio 615, PerCP, PerCP-Vio 700, PE-Vio 770, APC, APC-Vio 770, 1,8-Naphthalimides , Acridine Orange, SYTOX Green, TOTO-1, TO-PRO-1, TO-PRO: Cyanine Monomer, Thiazole Orange, CyTRAK Orange, Propidium Iodide (PI), LDS 751, 7-AAD, SYTOX Orange, TOTO-3, TO-PRO-3, DRAQ5, DRAQ7, Indo-1, Fluo-3, Fluo-4, DCFH, DHR or SNARF.

394.The method of any one of clauses 379-390, wherein the fluorescent label includes a fluorescent protein.

395. The method of clause 394, wherein the fluorescent protein is GFP (Y66H mutation), GFP (Y66F mutation), EBFP, EBFP2, Azurite, GFPuv, T-Sapphire, Cerulean, mCFP, mTurquoise2, ECFP, CyPet, GFP (Y66W mutation), mKeima-Red, TagCFP, AmCyan1, mTFP1, GFP (S65A mutation), Midoriishi Cyan, Wild Type GFP, GFP (S65C mutation), TurboGFP, TagGFP, GFP (S65L mutation), Emerald, GFP (S65T mutation), EGFP, Azami Green, ZsGreen1, TagYFP, EYFP, Topaz, Venus, mCitrine, YPet, TurboYFP, ZsYellow1, Kusabira Orange, mOrange, Allophycocyanin (APC), mKO, TurboRFP, tdTomato, TagRFP, DsRed monomer, DsRed2 ("RFP"), mStrawberry, TurboFP602, AsRed2, mRFP1, J-Red, R-phycoerythrin (RPE), B-phycoerythrin (BPE), mCherry, HcRed1, Katusha, P3, Peridinin Chlorophyll (PerCP), mKate (TagFP635), TurboFP635, mPlum or mRaspberry.

396. The method of any of the preceding clauses, wherein the label includes a reactive group that is specific for an amino acid type.

397.The method of clause 396, wherein the label includes a reactive group that is specific for the R-group of an amino acid type.

398.The method of clauses 377-391 or 392-395, wherein the fluorescent label includes a reactive group that is specific for chemical modifications made to an amino acid type prior to or during the labeling step.

399. The method of clause 398, wherein the fluorescent label includes a reactive group that is specific for chemical modifications made to the R-group an amino acid type prior to or during the labeling step.

400. The method of clause 398, wherein the fluorescent label includes a reactive group that is specific for chemical modifications made to protein backbone adjacent to the R-group an amino acid type prior to or during the labeling step.

401.The method of clauses 396-400, wherein the reactive group is selected from the group consisting of: NHS-ester, maleimide, alkyne, azide, bromide, chloride, fluoride, iodide, aryl bromide, aryl chloride, aryl fluoride, aryl iodide, diene, dienophile, olefin, tetrazine, cyclooctyne, biotin, streptavidin, isothiocyanate, active ester, sulfonyl chloride, dialdehyde, iodoacetamide, ethylenediamine, aminoacridone, hydrazide, carboxyl, or alkoxyamine

402.The method of clause 379, wherein the fluorescent label is a fluorescent dye.

403.The method of clause 402, wherein the fluorescent dye is a fluorogenic dye, non-flourogenic dye, molecule which becomes fluorescent upon reaction with an amino acid type, and/or molecule which shifts the fluorescence of an intrinsically fluorescent amino acid type into the visible region of the spectrum

404.The method of clause 402, wherein the fluorescent dye is a fluorogenic dye, non-flourogenic dye, a molecule which becomes fluorescent upon reaction with the R-group of an amino acid type, or a molecule which shifts the fluorescence of the R-group of an intrinsically fluorescent amino acid type into the visible region of the spectrum.

405.The method of clause 403, wherein the fluorogenic dye, molecule which becomes fluorescent upon reaction with an amino type, or molecule which shifts the fluorescence of an intrinsically fluorescent amino acid type into the visible region of the spectrum is selected from the group consisting of: 4-Fluoro-7-sulfamoylbenzofurazan (ABD-F), 2,2,2-Trichloroethanol (TCE) and/or ortho-phthalaldehyde (OPA), or a mixture thereof.

406.The method of clause 403, wherein fluorogenic dye, molecule which becomes fluorescent upon reaction with an amino type, or molecule which shifts the fluorescence of an intrinsically fluorescent amino acid type into the visible region of the spectrum is a halo compound.

407.The method of clause 406, wherein the halo compound is selected from the group consisting of trichloroacetic acid, chloroform, triflouroethanol, triflouroacetic acid, flouroform, tribromoethanol, tribromoacetic acid, bromoform, triiodoethanol, triiodoacetic acid or iodoform.

408.The method of clause 407, wherein the amino acid types tryptophan (W) and/or tyrosine (Y) are labelled with trichloroacetic acid, chloroform, triflouroethanol, triflouroacetic acid, flouroform, tribromoethanol, tribromoacetic acid, bromoform, triiodoethanol, triiodoacetic acid or iodoform.

409.The method of any one of the preceding clauses, wherein the R-group of each amino acid type is labelled.

410.The method of clause 409, wherein the R-group of an amino acid type labelled is the R-group of the modified and/or unmodified amino acids of an amino acid type.

411.The method of clause 410, wherein the R-group for unmodified A amino acids is labelled via Palladium catalysed $C(sp^3)$-$H_3$ bond activation, $Pd(OAc)_2$ with 1-ethynyl-4-iodobenzene, to install an alkyne followed by Cu(I) catalyzed azide-alkyne cycloaddition (CuAAC) "click-chemistry" with 3-azido-2H-chromen-2-one.

412.The method of clause 410, wherein the R-group for unmodified R amino acids is labelled with Dopachrome.

413.The method of clause 410, wherein the R-group for unmodified N amino acids is labelled with 4-amino-3-formylphenyl nitrate.

414. The method of clause 410, wherein the R-group for unmodified D amino acids is labelled with 4-(diethylamino)-2-(pyridin-2-ylmethoxy)benzaldehyde appended BODIPY based probe.

415. The method of clause 410, wherein the R-group for modified and unmodified C amino acids is labelled with 4-aminosulfonyl-7-fluoro-2,1,3-benzoxadiazole (ABD-F) after reduction of the oxidized thiols with tris(2-carboxyethyl) phosphine (TCEP).

416.The method of clause 410, wherein the R-group for unmodified C amino acids ($C_R$) is labelled with 4-aminosulfonyl-7-fluoro-2,1,3-benzoxadiazole (ABD-F) or o-maleimide BODIPY or ethyl (Z)-2-(6-(ethyl((3-(trifluoromethyl)phenyl)selanyl)amino)-3-(ethylimino)-2,7-dimethyl-3H-xanthen-9-yl)benzoate.

417.The method of clause 410, wherein the R-group for unmodified E amino acids is labelled with 4-(diethylamino)-2-(pyridin-2-ylmethoxy)benzaldehyde appended BODIPY based probe.

418.The method of clause 410, wherein the R-group for unmodified Q amino acids is labelled with 4-amino-3-formylphenyl nitrate.

419.The method of clause 410, wherein the R-group for unmodified G amino acids is labelled via C-H bond functionalization alpha to the carbonyl via reaction with H-alkynyl-Phe in the presence of CuBr (1 $\mu$M) and 10 $\mu$M of tBuOOH in DCM, followed by CuAAc with 3-azido-7-methoxy-2H-chromen-2-oneKetone .

420.The method of clause 410, wherein the R-group labelled for unmodified H amino acids is labelled with 2-butyl-6-(4-((6-(((2-ethoxyethyl)amino)methyl)pyridin-2-yl)methyl)piperazin-1-yl)-1H-benzo[de]isoquinoline-1,3(2H)-dione-$Cu^{2+}$.

421.The method of clause 410, wherein the R-group for unmodified I amino acids is labelled with a Blue light meditated Hoffman-Loffler-Freytag reaction for $\delta$-C-H functionalization of isoleucine, followed by reaction with acetic hypobromous anhydride catalyzed by blue LED to install a Br group, followed by $SN_2$ reaction with $KN_3$ to install an azide group, then CuAAc with 4-((7-ethynyl-2-oxo-2H-chromen-4-yl)methoxy)-4-oxobutanoic acid.

422. The method of clause 410, wherein the R-group for unmodified L amino acids is labelled with a Blue light meditated Hoffman-Loffler-Freytag reaction for $\delta$-C-H functionalization of isoeleucine, followed by reaction with acetic hypobromous anhydride catalyzed by blue LED to install a Br group, followed by SN2 reaction with KN3 to install azide group, then CuAAc with 4-((7-ethynyl-2-oxo-2H-chromen-4-yl)methoxy)-4-oxobutanoic acid.

423.The method of clause 410, wherein the R-group for unmodified K amino acids is labelled with ortho-phthalaldehyde (OPA) in the presence of $\beta$-mercaptoethanol (BME)

424. The method of clause 410, wherein the R-group for unmodified M amino acids is labelled with a reaction with an alkyne bearing methionine-selective iodonium salt, followed by click chemistry with a CalFlour dye.

425.The method of clause 410, wherein the R-group for unmodified F amino acids is labelled via Palladium catalysed alkynylation reaction with (bromoethynyl)triisopropylsilane 1 $\mu$M $Pd(OAc)_2$ with 20 $\mu$M of $K_2CO_3$ as a base, and 1 $\mu$M PivOH as an additive, followed by CuAAc with 3-azido-7-hydroxy-2H-chromen-2-one.

426.The method of clause 410, wherein the R-group for unmodified P amino acids is labelled with an amphiphilic dipolar Schiff base $Zn^{II}$ complexe.

427.The method of clause 410, wherein the R-group for S unmodified amino acids is labelled via selective conversion to azide with TT/n-$Bu_4NN_3$ or $Ph_3P$:2,3-dichloro-5,6- dicyanobenzoquinone (DDQ):n-$Bu_4NN_3$ followed by reaction with FI-DIBO.

428.The method of clause 410, wherein the R-group for unmodified T amino acids is labelled via selective conversion to azide with TT/n-Bu4NN3 or Ph3P:2,3-dichloro-5,6- dicyanobenzoquinone (DDQ):n-$Bu_4NN_3$ followed by reaction with FI-DIBO.

429.The method of clause 410, wherein the R-group for unmodified W amino acids is labelled with trichloroethanol (TCE), trichloroacetic acid (TCA), chloroform, trifluoroethanol (TFE), triflouroacetic acid (TFA), flouroform, tribromoethanol, tribromoacetic acid (TBA), bromoform, triiodoethanol (TIE), or triiodoacetic acid (TIA), iodoform, or, with 2-(2-(2-methoxyethoxy)ethoxy)ethyl (E)-2-diazo-4-phenylbut-3-enoate in the presence of $Rh_2(OAc)_4$ and tBuHNOH.

430.The method of clause 410, wherein the R-group for modified W amino acids is labelled with trichloroethanol (TCE).

431.The method of clause 410, wherein the R-group for unmodified Y amino acids is labelled with trichloroethanol (TCE), or, installation of an aryl group ortho to the tyrosine hydroxyl groups using $[RhCl(PPh_3)_3]$, $R_2P(OAr)$,Ar-Br,$CsCO_3$.

432.The method of clause 410, wherein the R-group for unmodified V amino acids is labelled via installation of quaternary azide group on the valine side chain using a $[Ru(bpy)_3]Cl_2$ catalyst and 1-azido-1l3-benzo[d][1,2] iodaoxol-3(1H)-one catalysed by visible light, followed by a fluorogenic CuAAC reaction with 4-((7-ethynyl-2-oxo-2H-chromen-4-yl)methoxy)-4-oxobutanoic acid.

433.The method of clause 410, wherein the R-group for unmodified O amino acids is labelled via a Diels Alder reaction with an azaphthalimide.

434.The method of clause 410, wherein the R-group for unmodified U amino acids is labelled with ABD-F, at pH 7

435.The method of clause 410, wherein the R-group for modified S amino acids is labelled with BO-IMI.

436.The method of clause 410, wherein the R-group for modified T amino acids of threonine is labelled with BO-IMI.

437.The method of clause 410, wherein the R-group for modified Y amino acid is labelled with BO-IMI.

438.The method of clause 410, wherein the modified R amino acids are labelled with o-maleimide bodipy.

439. The method of clause 410, wherein the modified N amino acids are labelled with a boronic acid tosyl probe with an alkyne substituent, which is subsequently reacted with a CalFlour dye.

440.The method of clause 410, wherein the modified K amino acids are labelled with 9-fluorenylmethyl chloroformate.

441.The method of clause 379, wherein the fluorescent label is a fluorescent protein or conjugated antibody.

442. The method of clause 441, wherein the fluorescent protein is selected from the group consisting of: smURFP, GFP, EGFP, Cerulean, mTurquoise, TagBFP, mCherry, mOrange, Citrine, Dronpa, dsRed, eqFP611, Dendra, EosFP, IrisFP, TagRFPs, FbFPs.

443.The method of clause 441, wherein the conjugated antibody is a post-translationally modified monoclonal antibody.

444.The method of clause 443, wherein the post-translationally modified monoclonal antibody detects phosphoserine, phosphotheronine, phosphotyrosine, phosphorylation, lysine methylation, arginine methylation, lysine acetylation, arginine acetylation, amidation, formation of pyrrolidone carboxylic acid, isomerization, proline hydroxylation,

lysine hydroxylation, sulfation, flavin-binding, cysteine oxidation, nitrosylation, lysine acylation, cysteine acylation, N-terminal acylation, lysine formylation, lysine alkylation, cysteine alkylation, arginylation, amide bond formation, butyrylation, gamma-carboxylation, arginine glycosylation, asparagine glycosylation, cysteine glycosylation, hydroxylysine glycosylation, serine glycosylation, threonine glycosylation, tyrosine glycosylation, tryptophan glycosylation, malonylation, proline hydroxylation, lysine hydroxylation, tyrosine iodination, nucleotide addition, phosphorylation, adenylylation, uridylylation, propionylation, pyroglutamate formation, S-glutathionylation, cysteine sulfenylation, cysteine sulfonylation, lysine succinylation, tyrosine sulfation, myristoylation, palmitoylation, isoprenylation, prenylation or glipyation.

445. The method of clauses 1-376, wherein the label is a tandem mass tag.

446.The method of clause 445, wherein the tandem mass tag is selected from the group consisting of TMTzero, TMTduplex, TMTsimplex, TMT 10-plex, TMTpro and TMTpro Zero.

447.The method of clauses 1-376, wherein the label is an isotopic label.

448.The method of clause 447, wherein the isotopic label is a non-radioactive isotope.

449.The method of clause 449, wherein the non-radioactive isotopic label is selected from: 2H, 13C, and/or 15N.

450.The method of clause 350, wherein the signal detected is a chemiluminescent signal or a biochemiluminescent signal.

451.The method of clause 450, wherein the chemiluminescent label is N-(4-aminobutyl)-N-ethyl-isoluminol (ABEI) macrocyclic lactone

452.The method of any one of clauses 377-451, wherein a combination of fluorescent labels, isotopic labels, tandem mass tags, and/or chemiluminescent labels are used to label two or more amino acid types.

453.The method of clause 358, wherein the measured label for the amino acid types Serine and Threonine are deconvoluted from each other.

454.The method of clause 358, wherein the measured label for the amino acid types Asparagine and Glutamine are deconvoluted from each other.

455. The method of clause 358, wherein the measured label for the amino acid types Glutamic Acid and Aspartic Acid are deconvoluted from each other.

456.The method of clause 358, wherein the measured label for the amino acid types Leucine and Isoleucine are deconvoluted from each other.

457.The method of any one of the preceding clauses, wherein the sample is denatured prior to labelling, or during the labelling reaction.

458.The method of clause 376, wherein the signal of the label is measured.

459. The method of any of the preceding clauses, wherein the measured label is background corrected.
459a. The method of clause 459, wherein autofluorescence of the sample is removed.

460.The method of clauses 447-449, wherein the isotopic label is measured through NMR and/or mass spectrometry.

461.The method of clauses 445-446, wherein the tandem mass tag is measured through mass spectrometry.

462.The method of clauses 379-395 or 398-442, wherein the fluorescent label is measured through fluorescence microscopy

463.The method of clauses 379-395 or 398-442, wherein the fluorescent label is measured through a fluorimeter.

464.The method of clause 379-395 or 398-442, wherein the fluorescent label is measured through a fluorescence plate reader.

465. The method of clause 379-395 or 398-442, wherein the fluorescent label is measured via an instrument that performs and/or reads several fluorescence reactions in parallel or in series.

466. The method of clause 462, wherein the amino acid type Y is labelled with a fluorescent label and the fluorescent label is measured at an excitation wavelength of from about 250nm to about 380 nm and an emission wavelength of from about 370nm to about 500nm.

467.The method of clause 462, wherein the amino acid type W is labelled with a fluorescent label and the fluorescent label is measured at an excitation wavelength of from about 270nm to about 380nm and an emission wavelength of from about 430nm to about 600nm.

468.The method of clause 462, wherein the amino acid type K is labelled with a fluorescent label and the fluorescent label is measured at an excitation wavelength of from about 320nm to about 415nm and an emission wavelength of from about 400 nm to about 500nm.

469. The method of clause 462, wherein the amino acid type C is labelled with a fluorescent label and the fluorescent label is measured at an excitation wavelength of from about 330nm to about 400nm and an emission wavelength of from about 430nm to about 580nm.

470.The method of clause 462, wherein, from the excitation and emission wavelength ranges provided, the excitation wavelength is separated from the emission wavelength by from about 10nm to about 20nm for each fluorescent label of each amino acid type being labelled in the sample.

471.The method of any one of the preceding clauses, wherein the amino acid concentration of each labelled amino acid type is calculated from the measured label and the amino acid concentration is calculated from the measured label using a calibration curve or standard which converts between the measured label of the sample and the amino acid concentration of that amino acid type in the sample.

472.The method of clause 471, wherein the calibration curve or standard is calculated from the measured label of one or more known amino acid concentrations of one or more proteins or amino acids.

473.The method of clause 471, wherein the amino acid concentration of each labelled amino acid type is calculated from the measured label and the amino acid concentration is calculated from the measured label using a calibration curve which converts between the measured label of the sample and the amino acid concentration of that amino acid type in the sample.

474.The method of clause 471, wherein the amino acid concentration of each labelled amino acid type is calculated from the measured label and the amino acid concentration is calculated from the measured label using a standard which converts between the measured label of the sample and the amino acid concentration of that amino acid type in the sample.

475. The method of clause 473, wherein the calibration curve is calculated from the measured label of more than one known amino acid concentrations of one or more proteins or amino acids.

476. The method of clause 474, wherein the standard is calculated from the measured label of one known amino acid concentration of one protein or amino acid.

477.The method of clause 471, 474 or 476, wherein more than one standard produces a calibration curve.

478.The method of any one of clauses 471, 472, 473 or 476, wherein the calibration curve is nonlinear.

478a. The method of clause 478, wherein the non-linear fit is a polynomial fit.

478b. The method of clause 478, wherein the non-linear fit is a power law fit.

478c. The method of clause 478, wherein the non-linear fit is a exponential fit.

478d. The method of clause 478, wherein the non-linear fit is a a sigmoidal fit.

479.The method of any one of clauses 471, 472, 473 or 476, wherein the calibration curve is linear.

480.The method of any one of clauses 471, 472, 473 or 475, wherein a best fit to convert between the measured label and the amino acid concentration is calculated for the calibration curve.

481.The method of clause 480, wherein the best fit to convert between the measured label and the amino acid concentration is calculated forthe calibration curve is a linear fit.

482.The method of clause 481, wherein the best fit line is calculated using linear regression.

483. The method of clause 471, 472, 473 or 475, wherein a best fit is calculated using nonlinear regression.

484. The method of clause 481, wherein the label is a fluorescent label and the best fit line to the calibration curve is calculated using equation 5:

$$Label\ Value_n = m_n \times A.A.Concentration_n + b_n$$

where *Label Value*$_n$ is the value of the label of amino acid type n in AU, $m_n$ is the slope of the best fit line in AU / amino acid concentration for amino acid type *n, A.A. Concentration*$_n$ is the amino acid concentration of amino acid type n, and $b_n$ is the value of the label when the amino acid concentration of amino acid type n is zero. The output of the fit is $m_n$ and $b_n$

485.The method of clause 483, wherein the amino acid concentration of each labelled amino acid type of the sample is determined using the inverse of the calibration curve, which is equation 6:

$$A.A.Concentration_n = \frac{Label\ Value_n - b_n}{m_n}$$

where *A.A. Concentration*$_n$ is the amino acid concentration of amino acid type n, *Label Value*$_n$ is the measured value of the label of amino acid type n in AU, $b_n$ is the value of the label when the amino acid concentration of amino acid type n is zero, and $m_n$ is the slope of the calculated best fit line in AU / amino acid concentration for amino acid type n

486.The method of clause 481, wherein the label is a fluorescent label and the label is background corrected and the best fit line of the calibration curve is calculated using equation 7:

$$Label\ Value_n = m_n \times A.A.Concentration_n$$

where *Label Value*$_n$ is the value of the label of amino acid type n in AU, $m_n$ is the slope of the best fit line in AU / amino acid concentration for amino acid type n, and *A.A. Concentration*$_n$ is the amino acid concentration of amino acid type n. The output of the fit is $m_n$.

487.The method of clause 485, wherein the amino acid concentration of each labelled amino acid type of the sample is determined using the inverse of the calibration function, which is equation 8:

$$Amino\ Acid\ Concentration_n = \frac{Label\ Value_n}{m_n}$$

where *A.A. Concentration*$_n$ is the amino acid concentration of amino acid type n, *Label Value*$_n$ is the measured value of the label of amino acid type n in AU, and $m_n$ is the slope of the calculated best fit line in AU / amino acid concentration

488. The method of any one of clauses 484 or 486, wherein the slope of the best fit line, $m_n$, for amino acid type n is a calibration factor for amino acid type n, $f_n$, which can be used when converting from amino acid concentration to known label value for the proteins, peptides, oligopeptides, polypeptides, protein complexes, subproteomes, or proteomes of

interest.

489. The method of any one of clauses 485 or 487, wherein the inverse of the calibration factor for amino acid type *n*, $f_n^{-1}$, is the inverse of the slope of the best fit line, $1/m_n$, which can be used when converting known label value to amino acid concentration for the proteins, peptides, oligopeptides, polypeptides, protein complexes, subproteomes, or proteomes of interest.

490.The method of clause 488 or 489, wherein the calibration factor for each labelled amino acid type in the sample is determined using data from one amino acid concentration of one standard.

491.The method of clause 490, wherein the standard is a protein or an amino acid.

492. The method of clause 490, wherein the inverse of the calibration factor for amino acid type n is determined by:

$$f_n^{-1} = \frac{Amino\ acid\ concentration\ of\ amino\ acid\ type\ n\ of\ the\ standard}{Signal\ of\ the\ label\ of\ standard}$$

493.The method of any one of the preceding clauses, wherein the number of amino acids of each labelled amino acid type in the sample is calculated, and the number of amino acids of each labelled amino acid type in the sample is calculated by dividing the amino acid concentration by the total molar protein concentration of the sample.

494. The method of clause 493, wherein the sample is being identified for the presence of a proteome, subproteome or complex mixture of interest and the number of amino acids of each labelled amino acid type is the mean number of amino acids of each labelled amino acid type in all the proteins across the proteome, subproteome or complex mixture of interest.

495. The method of clause 494, wherein the mean number of amino acids is the weighted mean number of amino acids of each labelled amino acid in all the proteins across the proteome, subproteome or mixture of interest, weighted by the proportion of each protein across the proteome, subproteome or mixture of proteins.
496. The method of clause 494 or 495, wherein the weighted mean number of amino acids of each amino acid type is determined using equation 11:

$$w_n = \sum_{i=1}^{c} \left( a_{n,i} \times \frac{q_i}{q} \right)$$

where $w_n$ is the weighted mean number of amino acids of amino acid type n in the proteome or subproteome of interest, c is the number of proteins in the proteome or subproteome of interest, $a_{n,i}$ is the number of amino acids of amino acid type n in protein i in the proteome or subproteome of interest, $q_i$ is a measure of the quantity of protein i in the proteome or subproteome of interest, and $q$ is an equivalent measure of the total quantity of all proteins (proteins i through c) in the proteome or subproteome of interest.

497.The method of clause 496, wherein $q_i/q$ gives the proportion of protein *i* within the proteome or subproteome of interest.

498.The method of clauses 496 or 497, wherein $q_i$ is the expression level of protein of interest *i* within the proteome or subproteome of interest.

499.The method of clause 498, wherein the expression level of interest i within the proteome or subproteome of interest is determined from publicly available data, including mass spectrometry or immunoassay.

500. The method of clause 499, wherein the publicly available data is a public database such as the Human Protein Atlas, Human Peptide Atlas, and/or ProteomeXchange.

501.The method of any one of clauses 496 or 497, wherein q is the total predicted expression level of all proteins (proteins i through c) contained within the proteome or subproteome of interest each assessed using publicly available

protein expression data.

502. The method of clause 501, wherein q is total protein concentration of the proteome or subproteome of interest.

503. The method of clause 502, wherein q is total protein concentration of the proteome or subproteome of interest calculated using standard methods in the art.

504. The method of clauses 501 and 502, wherein $q_i$ and $q$ are determined using mRNA expression data.

505.The method of clause 504, wherein $q_i$ is determined using mRNA expression data and a gene specific RNA-to-protein (RTP) conversion factor.

506.The method of clause 496, wherein $q_i$ and q can are calculated from a known structural model.

506a. The method of clause 496, wherein $\frac{q_i}{q}$ is provided by

$$\frac{q_i}{q} = \frac{int_m}{\sum int_m} = MSIF_m$$

wherein $int_m$ is the molar intensity of an individual protein within a sample calculated from a mass spectrometry database, $\Sigma\, int_m$ is the sum of the molar intensities of all individual proteins within a sample calculated from a mass spectrometry database, and $MSIF_m$ is the mass spectrometry molar intensity fraction.

506b. The method of clause 506a, wherein $q_i = int_m$
wherein $int_m$ is the molar intensity of an individual protein within a sample calculated from a mass spectrometry database.

506c. The method of clause 506a or 506b, wherein $q = \Sigma\, int_m$
wherein $\Sigma\, int_m$ is the sum of the molar intensities of all individual proteins within a sample calculated from a mass spectrometry database.

506d. The method of clause 506b, wherein

$$int_m = \frac{int}{m_r}$$

wherein int is the intensity or abundance of an individual protein within a sample provided by a mass spectrometry database, and $m_r$ is the molecular weight of an individual protein within a sample provided by a mass spectrometry database or by a database providing the molecular weight and amino acid sequences of proteins.

506e. The method of clause 506d, wherein int is a normalized intensity, raw intensity, normalized abundance, or raw abundance.

506f. The method of any one of clauses 506a-506e, wherein int was calculated using label free quantification (LFQ).

506g. The method of any one of clauses 506a-506d, wherein the mass spectrometry database is the Proteome Xchange database.

506h. The method of any one of clauses 506a -506g, wherein the database providing the molecular weight and amino acid sequences of proteins is the UniProt database.

506i. The method of any one of clauses 506a-506c, wherein

$$mean\left(\sum int_m\right) = a \sum molar\ protein\ concentration$$

wherein Σ *molar protein concentration* is the sum of the molar protein concentration for all proteins in a proteome, subproteome, or sample type of interest, provided by a database of molar concentration values and wherein $mean(\Sigma\ int_m)$ is the mean of the $\Sigma\ int_m$ values for all samples within the database.

506j. The method of clause 506i, wherein the molar concentration values are calculated from the Human Peptide Atlas database.

506k. The method of clause 506i, wherein the molar protein concentration values for each protein in the database was calculated using an immunoassay based technology such as an ELISA assay, or where the mass protein concentration values for each protein in the database was calculated using an immunoassay based technology such as an ELISA asay, and transformed to molar protein concentration values using a database of molecular weights for each protein such as accessed from the UniProt database.

506l. The method of clause 506i, wherein the molar protein concentration values for each protein in the database was calculated using an aptamer based technology such as the Somascan assay, or where the mass protein concentration values for each protein in the database was calculated using an immunoassay based technology such as an ELISA asay, and transformed to molar protein concentration values using a database of molecular weights for each protein such as accessed from the UniProt database.

506m. The method of any one of clauses 506i-506l, wherein *a* is calculated for an given set of samples by calculating

$$a = \frac{mean(\sum int_m)}{\sum molar\ protein\ concentration}$$

506n. The method of clause 506m, wherein $mean(\Sigma\ int_m)$ is the mean of the $\Sigma\ int_m$ values for all samples in the database.

506o. The method of clause 496, wherein $\frac{q_i}{q}$ is provided by

$$\frac{q_i}{q} = \frac{int}{\sum int} = MSIF_{mass}$$

wherein int is the intensity of an individual protein within a sample provided by a mass spectrometry database, Σ int is the sum of the intensities of all individual proteins within a sample calculated from a mass spectrometry database, and $MSIF_{mass}$ is the mass spectrometry mass intensity fraction.

506p. The method of clause 506o, wherein $q_i = int$
wherein int is the intensity of an individual protein within a sample provided by a mass spectrometry database.

506q. The method of clause 506o or 506p, wherein $q = \Sigma\ int$
wherein Σ int is the sum of the intensities of all individual proteins within a sample calculated from a mass spectrometry database.

506r. The method of any one of clauses 506o-506q, wherein int is a normalized intensity, raw intensity, normalized abundance, or raw abundance.

506s. The method of amy one of clauses 506o-506r, wherein int was calculated using label free quantification (LFQ).

506t. The method of clause 506o and 506p, wherein the mass spectrometry database is the Proteome Xchange database.

506u. The method of clauses 506n, wherein

$$mean\left(\sum int\right) = a\sum mass\ protein\ concentration$$

wherein Σ *mass protein concentration* is the sum of the mass protein concentration for all proteins in a proteome, subproteome, or sample type of interest, provided by a database of mass concentration values and wherein *mean* (Σ *int)* is the mean of the Σ int values for all samples within the database.

506v. The method of clause 506u, wherein the mass protein concentration values are calculated from the Human Peptide Atlas database.

506w. The method of any one of clauses 506t-506v, wherein the mass protein concentration values for each protein in the database was calculated using an immunoassay based technology such as an ELISA assay.

506x. The method of any one of clause 506t-506v, wherein the mass protein concentration values for each protein in the database was calculated using an aptamer based technology such as the Somascan assay.

506y. The method of any one of clause 506u-506x wherein *a* is calculated for an given set of samples by calculating

$$a = \frac{mean(\sum int)}{\sum mass\ protein\ concentration}$$

506z. The method of clause 506y, wherein *mean*(Σ *int*) is the mean of the Σ int values for all samples in the database.

507.The method of clause 496, wherein proteome of interest is a virus, and $q_i$ is the number of protein i within the structure of the virus and q is the number of all proteins (proteins *i* through *c*) within the structure of the virus.

508. The method of clause 507, wherein the number of coronavirus spike proteins is calculated from a model of the coronavirus viral capsid.

509.The method of clause 495, wherein the weighted mean number of amino acids of each amino acid type is determined with equation 12:

$$w_n = \sum_{i=1}^{c}\left(a_{i,n} \times \frac{1}{c}\right)$$

where $w_n$ is the weighted mean number of amino acids of amino acid type n in the proteome or subproteome of interest, *c* is the number of proteins in the proteome or subproteome of interest, and $a_{i,n}$ is the number of amino acids of amino acid type *n* in protein *i* in the proteome, or subproteome of interest.

510.The method of clause 509 wherein all proteins within the proteome or subproteome of interest are taken as having equivalent expression or proportion within the proteome or subproteome of interest, so the weights for each protein of interest within the proteome or subproteome of interest are equal.

511. The method of clause 496 or 509, wherein a linear combination is taken for all proteins i through c in the proteome or subproteome of interest.

512. The method of clause 494, wherein the weighted mean number of amino acids of each amino acid type is determined using equation 6:

$$w_n = \sum_{i=1}^{c}\left(a_{i,n} \times \frac{q_i}{q}\right)$$

where $w_n$ is the weighted mean number of amino acids of amino acid type n in the complex mixture of proteins of interest, *c* is the number of proteins in the complex protein mixture of proteins of interest, $a_{i,n}$ is the number of amino

acids of amino acid type n in protein i in the complex mixture of proteins of interest, $q_i$ is a measure of the quantity of protein *i* in the complex mixture of proteins of interest, and q is an equivalent measure of the total quantity of all proteins (proteins *i* through *c*) in the complex mixture of proteins of interest.

513. The method of clause 512, wherein any of the methods used in 492-502 are used to calculate $q_i/q$ .

514.The method of clauses 495, wherein the weighted mean number of amino acids of each amino acid type is determined with equation 12:

$$w_n = \sum_{i=1}^{c}\left(a_{i,n} \times \frac{1}{c}\right)$$

where $w_n$ is the weighted mean number of amino acids of amino acid type *n* in the complex mixture of proteins of interest, c is the number of proteins in the complex mixture of proteins of interest, and $a_{i,n}$ is the number of amino acids of amino acid type n in the complex mixture of proteins of interest.

515. The method of clause 494, 509 and 514, wherein a complex mixture of proteins is a mixture with more than 5, 6, 7, 8, 9, or 10 proteins.

516. The method of clause 494, 509 and 514, wherein the fraction, proportion, or composition of each protein across the proteome, subproteome or mixture of proteomes is determined by comparing the fraction of that protein's expression level to the expression level of all proteins within the mixture of proteins or proteome.

517. The method of any one of the preceding clauses, wherein the known label values, or amino acid concentrations of the same two or more amino acid types in the one or more proteins, peptides, oligopeptides, polypeptides, protein complexes, subproteomes, proteomes or mixture of proteins, peptides, polypeptides, oligopeptides, subproteomes, or proteomes of interest at one or more protein concentrations is calculated from the amino acid sequence of the one or more proteins, peptides, oligopeptides, polypeptides, protein complexes, subproteomes, proteomes or mixture of proteins, peptides, polypeptides, oligopeptides, subproteomes, or proteomes of interest.

518. The method of clause 517, wherein the amino acid sequence of the one or more proteins, peptides, oligopeptides, polypeptides, protein complexes, subproteomes, proteomes or mixture of proteins, peptides, polypeptides, oligopeptides, subproteomes, or proteomes of interest provides the number of amino acids of each amino acid type.

519. The method of clause 518, wherein the number of amino acids includes the number of unmodified amino acids of an amino acid type within an amino acid sequence, wherein the number of unmodified amino acids of an amino acid type is the number of occurrences of that amino acid type within the amino acid sequence minus the number of post-translational modifications of that amino acid type.

520. The method of any one of the preceding clauses, wherein when calculating the number of amino acids of each of two or more amino acid types within an amino acid sequence of or contained within a protein, peptide, oligopeptide, protein complex, subproteome, or proteome of interest, the number of amino acids of each amino acid type in a protein of interest is adjusted by considering post-translational modifications (PTMs) that affect the amino acid type in a manner which makes it chemically unreactive with the label used for amino acid labelling.

521. The method of clause 520, wherein when calculating the number of amino acids of each of two or more amino acid types within an amino acid sequence of or contained within a protein, peptide, oligopeptide, protein complex, subproteome, or proteome of interest, the number of amino acids of each amino acid type in a protein of interest is adjusted by considering post-translational modifications (PTMs) that affect the R-group which defines the amino acid type in a manner which makes it chemically unreactive with the label used for amino acid labelling.

522.The method of clause 519-521, wherein the information about post-translational modifications can be obtained based on the results of experiments, or obtained using predictions.

523.The method of clause 519-522, wherein the rules provided in Table 4 are applied.

524. The method of clauses 523, wherein if -1 is added to the number of the amino acid type within an amino acid sequence, then unmodified amino acids of the amino acid type are labelled within the sample using the labeling chemistries disclosed herein.

525. The method of clauses 523, wherein if 0 is added to the number of the amino acid type within an amino acid sequence, then all (both unmodified and modified amino acids) of the amino acid type are labelled within the sample using the labeling chemistries disclosed herein.

526. The method of any of the preceding clauses, wherein the rules of clauses 523 are not applied if modified amino acids of an amino acid type are converted to unmodified amino acids of an amino acid type within the sample prior to or during the labeling reaction.

527. The method of clause 495, wherein the weighted mean number of amino acids of each of two or more amino acid types for a proteome or subproteome of interest is calculated using publicly available proteome wide PTM statistics.

528. The method of clause 495, wherein the numbers of unmodified or modified amino acids are calculated for a proteome or subproteome of interest by using publicly available proteome-wide post-translational modification statistics.

529. The method of clause 528, wherein the proteome-wide post-translational modification statistics are filtered to provide post-translational modification frequencies specific to prokaryotes, eukaryotes, and mammals including humans.

530. The method of clause 529, wherein viruses are treated as not undergoing post-translational modifications because they do not contain genes coding for enzymes which carry out post-translational modifications.

531. The method of clause 530, wherein viruses are treated as undergoing post-translational modifications or a subset of post-translational modifications that proteins within their host undergoes because viruses hijack the protein translational machinery of their host cells.

532. The method of clauses 528-531, wherein to predict the number of unmodified amino acids of an amino acid type, or to predict the number of modified amino acids of an amino acid type, then the frequency of modification of that amino acid type is determined by summing all of the post-translational modifications affecting that amino acid type and dividing by the total number of amino acids in that amino acid type in the Swiss Prot database, wherein the post-translational modifications affecting an amino acid type are provided in clause 523..

533. The method of clause 532, wherein a modification factor for each amino acid type is provided which can differ by class of organism.

534. The method of any one of the preceding claims, wherein the presence and/or concentration of one or more proteins, peptides, oligopeptides, polypeptides, protein complexes, subproteomes, or proteomes of interest is identified from information indicating the known label values and/or amino acid concentrations, and/or number of amino acids of the same two or more amino acid types as the amino acid types that have been labelled in the sample in one or more proteins, peptides, oligopeptides, polypeptides, protein complexes, subproteomes, or proteomes of interest.

535. The method of clause 534, wherein the information relating the known label values, amino acid concentrations, or number of amino acids of two or more amino acid types to the identity and/or protein concentration of one or more proteins, peptides, oligopeptides, polypeptides, protein complexes, subproteomes, or proteomes of interest is obtained from a database.

536. The method of clause 535, wherein the information relating the known label values, amino acid concentrations, or number of amino acids of two or more amino acid types to the identity and/or protein concentration of one or more proteins, peptides, oligopeptides, polypeptides, protein complexes, subproteomes, or proteomes of interest obtained from a database includes the protein sequence or sequences of each protein, peptide, oligopeptide, polypeptide, protein complex, subproteome, or proteome of interest.

537. The method of clause 534, 535 or 536, wherein the information relating the known label values, amino acid

concentrations, or number of amino acids of two or more amino acid types to the identity and/or protein concentration of one or more proteins, peptides, oligopeptides, polypeptides, protein complexes, subproteomes, or proteomes of interest obtained from a database includes information about post-translational modifications of the protein sequence or sequences of each protein, peptide, oligopeptide, polypeptide, protein complex, subproteome, or proteome of interest.

538. The method of any one of clauses 534-537, wherein the information relating the known label values, amino acid concentrations, or number of amino acids of two or more amino acid types to the identity and/or protein concentration of one or more proteins, peptides, oligopeptides, polypeptides, protein complexes, subproteomes, or proteomes of interest obtained from a database includes the identifier of each protein, peptide, oligopeptide, polypeptide, protein complex, subproteome, or proteome of interest.

539. The method of any one of clauses 534-538, wherein the information relating the known label values, amino acid concentrations, or number of amino acids of two or more amino acid types to the identity and/or protein concentration of one or more proteins, peptides, oligopeptides, polypeptides, protein complexes, subproteomes, or proteomes of interest obtained from a database includes the name of each protein, peptide, oligopeptide, polypeptide, protein complex, subproteome, or proteome of interest.

540. The method of any one of clauses 534-539, wherein the information relating the known label values, amino acid concentrations, or number of amino acids of two or more amino acid types to the identity and/or protein concentration of one or more proteins, peptides, oligopeptides, polypeptides, protein complexes, subproteomes, or proteomes of interest obtained from a database includes the lineage of each protein, peptide, oligopeptide, polypeptide, protein complex, subproteome, or proteome of interest.

541. The method of any one of clauses 534-540, wherein the information relating the known label values, amino acid concentrations, or number of amino acids of two or more amino acid types to the identity and/or protein concentration of one or more proteins, peptides, oligopeptides, polypeptides, protein complexes, subproteomes, or proteomes of interest obtained from a database includes the taxon of each protein, peptide, oligopeptide, polypeptide, protein complex, subproteome, or proteome of interest.

542. The method of any one of clauses 534-541, wherein the information relating the known label values, amino acid concentrations, or number of amino acids of two or more amino acid types to the identity and/or protein concentration of one or more proteins, peptides, oligopeptides, polypeptides, protein complexes, subproteomes, or proteomes of interest obtained from a database includes the known protein concentration range of each protein, peptide, oligopeptide, polypeptide, protein complex, subproteome, or proteome of interest within sample types of interest.

543. The method of any one of clauses 534-542, wherein the information relating the known label values, amino acid concentrations, or number of amino acids of two or more amino acid types to the identity and/or protein concentration of one or more proteins, peptides, oligopeptides, polypeptides, protein complexes, subproteomes, or proteomes of interest obtained from a database includes the known protein concentration range of each protein, peptide, oligopeptide, polypeptide, protein complex, subproteome, or proteome of interest within tissue types of interest.

544. The method of any one of clauses 534-543, wherein the information relating the known label values, amino acid concentrations, or number of amino acids of two or more amino acid types to the identity and/or protein concentration of one or more proteins, peptides, oligopeptides, polypeptides, protein complexes, subproteomes, or proteomes of interest obtained from a database includes the known protein expression data of each protein, peptide, oligopeptide, polypeptide, protein complex, subproteome, or proteome of interest within sample types of interest.

545.The method of any one of clauses 534-544, wherein information relating the known label values, amino acid concentrations, or number of amino acids of two or more amino acid types to the identity and/or protein concentration of each protein, peptide, oligopeptide, polypeptide, protein complex, subproteome, or proteome of interest is a reference.
545a. The method of clause 545, wherein information relating the known label values, amino acid concentrations, or number of amino acids of two or more amino acid types to to the identity and/or protein concentration of each proteome or subproteome of interest is provided as a single reference.

546. The method of clauses 1a-1h wherein the known label values and/or amino acid concentrations of the same two or more amino acid types as the amino acid types that have been labelled in the sample of each of the one or more

proteins, peptides, oligopeptides, polypeptides, protein complexes, subproteomes, or proteomes of interest at one or more protein concentrations, and/or number of amino acids of the same two or more amino acid types as the amino acid types that have been labelled in the sample in the one or more proteins, peptides, oligopeptides, polypeptides, protein complexes, subproteomes, or proteomes of interest are determined from the amino acid sequence of the one or more proteins, peptides, oligopeptides, polypeptides, protein complexes, subproteomes, or proteomes of interest.

547. The method of clause 546, wherein the amino acid sequence of the one or more proteins, peptides, oligopeptides, polypeptides, protein complexes, subproteomes, proteomes or mixture of proteins, peptides, polypeptides, oligopeptides, subproteomes, or proteomes of interest is determined using protein sequencing.

548. The method of any one of the preceding clauses, wherein the known label values, amino acid concentrations or number of amino acids of the same two or more amino acid types in the one or more proteins, peptides, oligopeptides, polypeptides, protein complexes, subproteomes, or proteomes of interest is obtained from a database.

549. The method of any one of the preceding clauses, wherein the known label values, amino acid concentrations or number of amino acids of the same two or more amino acid types in the one or more proteins, peptides, oligopeptides, polypeptides, protein complexes, subproteomes, or proteomes of interest is a reference.

550. The method of any one of the preceding clauses, wherein each reference provides the known label values or amino acid concentrations of the same two or more amino acid types of each protein, peptide, oligopeptide, polypeptide, protein complex, subproteome, or proteome of interest as a set of parametric equations or a vector-valued function depending on the common parameter of protein concentration, or, wherein each reference provides the number of amino acids of the same two or more amino acid types of each protein, peptide, oligopeptide, polypeptide, protein complex, subproteome, or proteome of interest.

551. The method of any one of the preceding clauses, wherein the known label values, or amino acid concentrations of the same two or more amino acid types in one or more proteins, peptides, oligopeptides, polypeptides, protein complexes, subproteomes, or proteomes of interest at one or more protein concentrations is a function of the protein concentration of the protein, peptide, oligopeptide, polypeptide, protein complex, subproteome, or proteome of interest.

552. The method of clause 551, wherein the known label values, or amino acid concentrations of the same two or more amino acid types in one or more proteins, peptides, oligopeptides, polypeptides, protein complexes, subproteomes, or proteomes of interest at one or more protein concentrations is a function of the total molar protein concentration of the protein, peptide, oligopeptide, polypeptide, protein complex, subproteome, or proteome of interest.

553. The method of clause 551, wherein the known label values, or amino acid concentrations of the same two or more amino acid types in one or more proteins, peptides, oligopeptides, polypeptides, protein complexes, subproteomes, or proteomes of interest at one or more protein concentrations is a function of the peptide, oligopeptide, polypeptide, protein, or protein complex concentration, or of the total protein concentration within the subproteome or proteome of interest.

554. The method of clause 551, wherein the known label values, or amino acid concentrations of the same two or more amino acid types in one or more proteins, peptides, oligopeptides, polypeptides, protein complexes, subproteomes, or proteomes of interest at one or more protein concentrations is a function of the total molar peptide, oligopeptide, polypeptide, protein, or protein complex concentration, or of the total molar protein concentration within the subproteome or proteome of interest.

555. The method of clause 551, wherein the known label values or amino acid concentrations of the same two or more amino acid types of each protein, peptide, oligopeptide, polypeptide, protein complex, subproteome, or proteome of interest are provided as a vector-valued function depending on the common parameter of protein concentration

556. The method of clause 551, wherein the known label values or amino acid concentrations of the same two or more amino acid types of each protein, peptide, oligopeptide, polypeptide, protein complex, subproteome, or proteome of interest are provided as a vector function depending on the common parameter of protein concentration.

557. The method of clause 551, wherein the known label values or amino acid concentrations of the same two or more amino acid types of each protein, peptide, oligopeptide, polypeptide, protein complex, subproteome, or proteome of

interest are provided as a vector function depending on the common parameter of total molar protein, peptide, oligopeptide, polypeptide, protein complex, or total molar protein concentration within the subproteome or proteome of interest.

558. The method of clauses 555-557, wherein the direction of the vector providing the amino acid concentrations of the same two or more amino acid types of each protein, peptide, oligopeptide, polypeptide, protein complex, subproteome, or proteome of interest is the number or weighted mean number of amino acids of each amino acid type within each protein, peptide, oligopeptide, polypeptide, protein complex, subproteome, or proteome of interest.

559. The method of clauses 558, wherein the direction of the vector providing the amino acid concentrations of the same two or more amino acid types of each protein, peptide, oligopeptide, polypeptide, protein complex, subproteome, or proteome of interest is the number or weighted mean number of amino acids of each amino acid type within each protein, peptide, oligopeptide, polypeptide, protein complex.

560. The method of clauses 555-559, wherein the vector begins at the origin if the values of the label of all amino acid types are background corrected in the sample, or at the point (n-tuple) providing the background value for each of the *n* amino acid types labelled and measured in the sample if the values of the label of each/any amino acid type are not background corrected in the sample.

561. The method of clauses 555-559, wherein the vector is bounded by lower and upper limits of protein concentration available from known or calculated protein expression data.

562. The method of clause 557, wherein the vector function depending on the common parameter of protein concentration is vector function 1 and takes the form:

$$p_i(t) = \langle 0, 0, \cdots 0\rangle + \langle a_1 t, a_2 t, \cdots a_n t\rangle, \forall t \geq 0$$

Where $p_i$ are the amino acid concentrations provided for protein, peptide, oligopeptide, polypeptide, or protein complex of interest *i* as a function of protein concentration *t*, $\langle 0, 0, \cdots 0\rangle$ is the origin, $a_1$ is the number of amino acid type 1 in the protein, peptide, polypeptide, oligopeptide, or protein complex of interest, $a_2$ is the number of amino acid type 2 in the protein, peptide, polypeptide, oligopeptide, or protein complex of interest, $a_n$ is the number of amino acid type *n* in the protein, peptide, polypeptide, oligopeptide, or protein complex of interest, *t* is the total molar protein concentration of the protein, peptide, polypeptide, oligopeptide, or protein complex of interest which is defined for all values of *t* greater than or equal to 0.

563. The method of clause 557, wherein the vector function depending on the common parameter of protein concentration takes the form:

$$p_i(t) = (0, 0, \cdots 0) + (a_1 t, a_2 t, \cdots a_n t), \forall t \geq 0$$

Where $p_i$ are the amino acid concentrations provided for protein, peptide, oligopeptide, polypeptide, or protein complex of interest *i* as a function of protein concentration *t*, $(0, 0, \cdots 0)$ is the origin, $a_1$ is the number of amino acid type 1 in the protein, peptide, polypeptide, oligopeptide, or protein complex of interest, $a_2$ is the number of amino acid type 2 in the protein, peptide, polypeptide, oligopeptide, or protein complex of interest, $a_n$ is the number of amino acid type *n* in the protein, peptide, polypeptide, oligopeptide, or protein complex of interest, *t* is the total molar protein concentration of the protein, peptide, polypeptide, oligopeptide, or protein complex of interest which is defined for all values of *t* greater than or equal to 0.

564. The method of clause 557, wherein the vector function depending on the common parameter of protein concentration takes the form:

$$p_i(t) = \langle a_1 c_1, a_2 c_1, \cdots a_n c_1\rangle + \langle a_1 t, a_2 t, \cdots a_n t\rangle, \forall t \in c_1 \leq t \geq c_2$$

Where $p_i$ are the amino acid concentrations provided for protein, peptide, oligopeptide, polypeptide, or protein complex of interest *i* as a function of protein concentration *t*, $a_1$ is the number of amino acid type 1 in the protein, peptide, polypeptide, oligopeptide, or protein complex of interest, $a_2$ is the number of amino acid type 2 in the protein, peptide, polypeptide, oligopeptide, or protein complex of interest, $a_n$ is the number of amino acid type *n* in the protein,

peptide, polypeptide, oligopeptide, or protein complex of interest, $c_1$ is the lower limit of the protein concentration range, $c_2$ is the upper limit of the protein concentration range, t is the total molar protein concentration of the protein, peptide, polypeptide, oligopeptide, or protein complex of interest which is defined for all values of *t* between $c_1$ and $c_2$.

565. The method of clause 557, wherein the vector function depending on the common parameter of protein concentration is vector function 2 and takes the form:

$$p_i(t) = \langle 0, 0, \cdots 0 \rangle + \langle w_1 t, w_2 t, \cdots w_n t \rangle, \forall t \geq 0$$

Where $p_i$ are the amino acid concentrations provided for proteome or subproteome of interest *i* as a function of protein concentration *t*, $\langle 0, 0, \cdots 0 \rangle$ is the origin, $w_1$ is the weighted mean number of amino acid type 1 in the proteome or subproteome of interest, $w_2$ is the weighted mean number of amino acid type 2 in the proteome or subproteome of interest, $w_n$ is the number of amino acid type *n* in the proteome or subproteome of interest, t is the total molar or mass protein concentration of the proteome or subproteome of interest which is defined for all values of *t* greater than or equal to 0.

566. The method of clause 557, wherein the vector function depending on the common parameter of protein concentration takes the form:

$$p_i(t) = \langle b_1, b_2, \cdots b_n \rangle + \langle a_1 f_1 t, a_2 f_2 t, \cdots a_n f_n t \rangle, \forall t \in c_1 \leq t \geq c_2$$

Where $p_i$ are the known values of the label provided for protein, peptide, oligopeptide, polypeptide, or protein complex of interest *i* as a function of protein concentration *t*, $b_1$ is the background value for amino acid type 1 which is 0 if measured values of the label in the sample are background-corrected, $b_2$ is the background value for amino acid type 2 which is 0 if measured values of the label in the sample are background-corrected, $b_n$ is the background value for amino acid type n which is 0 if measured values of the label in the sample are background-corrected, $a_1$ is the number of amino acid type 1 in the protein, peptide, polypeptide, oligopeptide, or protein complex of interest, $a_2$ is the number of amino acid type 2 in the protein, peptide, polypeptide, oligopeptide, or protein complex of interest, $a_n$ is the number of amino acid type *n* in the protein, peptide, polypeptide, oligopeptide, or protein complex of interest, $f_1$ is the calibration function or calibration factor for amino acid type 1, $f_2$ is the calibration function or calibration factor for amino acid type 2, $f_n$ is the calibration function or calibration factor for amino acid type *n*, $c_1$ is the lower limit of the protein concentration range, $c_2$ is the upper limit of the protein concentration range, *t* is the total molar protein concentration of the protein, peptide, polypeptide, oligopeptide, or protein complex of interest which is defined for all values of *t* between $c_1$ and $c_2$.

567. The method of clause 557, wherein the vector function depending on the common parameter of protein concentration is vector function 3 and takes the form:

$$p_i(t) = \langle b_1, b_2, \cdots b_n \rangle + \langle a_1 f_1 t, a_2 f_2 t, \cdots a_n f_n t \rangle, \forall t \geq 0$$

Where $p_i$ are the known values of the label provided for protein, peptide, oligopeptide, polypeptide, or protein complex of interest *i* as a function of protein concentration *t*, $b_1$ is the background value for amino acid type 1 which is 0 if measured values of the label in the sample are background-corrected, $b_2$ is the background value for amino acid type 2 which is 0 if measured values of the label in the sample are background-corrected, $b_n$ is the background value for amino acid type n which is 0 if measured values of the label in the sample are background-corrected, $a_1$ is the number of amino acid type 1 in the protein, peptide, polypeptide, oligopeptide, or protein complex of interest, $a_2$ is the number of amino acid type 2 in the protein, peptide, polypeptide, oligopeptide, or protein complex of interest, $a_n$ is the number of amino acid type n in the protein, peptide, polypeptide, oligopeptide, or protein complex of interest, $f_1$ is the calibration function or calibration factor for amino acid type 1, $f_2$ is the calibration function or calibration factor for amino acid type 2, $f_n$ is the calibration function or calibration factor for amino acid type *n*, *t* is the total molar protein concentration of the protein, peptide, polypeptide, oligopeptide, or protein complex of interest which is defined for all values of *t* greater than or equal to 0.

568.The method of clause 557, wherein the vector function depending on the common parameter of protein concentration takes the form:

$$p_i(t) = \langle b_1, b_2, \cdots b_n \rangle + \langle w_1 f_1 t, w_2 f_2 t, \cdots w_n f_n t \rangle, \forall t \in c_1 \leq t \geq c_2$$

Where $p_i$ are the known values of the label provided for proteome or subproteome of interest *i* as a function of protein concentration *t*, $b_1$ is the background value for amino acid type 1 which is 0 if measured values of the label in the sample are background-corrected, $b_2$ is the background value for amino acid type 2 which is 0 if measured values of the label in the sample are background-corrected, $b_n$ is the background value for amino acid type *n* which is 0 if measured values of the label in the sample are background-corrected, $w_1$ is the weighted mean number of amino acid type 1 in the proteome or subproteome of interest, $w_2$ is the weighted mean number of amino acid type 2 in the proteome or subproteome of interest, $w_n$ is the weighted mean number of amino acid type n in the proteome or subproteome of interest, $f_1$ is the calibration function or calibration factor for amino acid type 1, $f_2$ is the calibration function or calibration factor for amino acid type 2, $f_n$ is the calibration function or calibration factor for amino acid type *n*, $c_1$ is the lower limit of the protein concentration range, $c_2$ is the upper limit of the protein concentration range, *t* is the total molar protein concentration of the proteome or subproteome of interest which is defined for all values of *t* between $c_1$ and $c_2$.

569. The method of clause 557, wherein the vector function depending on the common parameter of protein concentration is vector function 4 and takes the form:

$$p_i(t) = \langle b_1, b_2, \cdots b_n\rangle + \langle w_1 f_1 t, w_2 f_2 t, \cdots w_n f_n t\rangle, \forall t \geq 0$$

Where $p_i$ are the known values of the label provided for proteome or subproteome of interest *i* as a function of protein concentration *t*, $b_1$ is the background value for amino acid type 1 which is 0 if measured values of the label in the sample are background-corrected, $b_2$ is the background value for amino acid type 2 which is 0 if measured values of the label in the sample are background-corrected, $b_n$ is the background value for amino acid type *n* which is 0 if measured values of the label in the sample are background-corrected, $w_1$ is the weighted mean number of amino acid type 1 in the proteome or subproteome of interest, $w_2$ is the weighted mean number of amino acid type 2 in the proteome or subproteome of interest, $w_n$ is the weighted mean number of amino acid type n in the proteome or subproteome of interest, $f_1$ is the calibration function or calibration factor for amino acid type 1, $f_2$ is the calibration function or calibration factor for amino acid type 2, $f_n$ is the calibration function or calibration factor for amino acid type *n*, and *t* is the total molar protein concentration of the proteome or subproteome of interest which is defined for all values of t greater than or equal to 0.

570. The method of clause 557, wherein the vector function depending on the common parameter of protein concentration takes the form:

$$p_i(t) = \langle a_1, a_2, \cdots a_n\rangle + \langle 0t, 0t, \cdots 0t\rangle, \forall t \geq 0$$

Where $p_i$ are number of amino acids for protein, peptide, oligopeptide, polypeptide, or protein complex of interest *i*, $a_1$ is the number of amino acid type 1 in the protein, peptide, oligopeptide, polypeptide, or protein complex of interest, $a_2$ is the number of amino acid type 1 in the protein, peptide, oligopeptide, polypeptide, or protein complex of interest, $a_n$ is the number of amino acids of amino acid type *n* in the protein, peptide, oligopeptide, polypeptide, or protein complex of interest, and *t* is the total molar protein concentration of the proteome or subproteome of interest which is defined for all values of *t* greater than or equal to 0.

571. The method of clause 557, wherein the vector function depending on the common parameter of protein concentration takes the form:

$$p_i(t) = \langle w_1, w_2, \cdots w_n\rangle + \langle 0t, 0t, \cdots 0t\rangle, \forall t \geq 0$$

Where $p_i$ are the weighted mean number of amino acids for proteome or subproteome of interest *i*, $w_1$ is the weighted mean number of amino acid type 1 in the proteome or subproteome of interest, $w_2$ is the weighted mean number of amino acid type 2 in the proteome or subproteome of interest, $w_n$ is the weighted mean number of amino acid type *n* in the proteome or subproteome of interest, and *t* is the total molar protein concentration of the proteome or subproteome of interest which is defined for all values of *t* greater than or equal to 0.

572.The method of clause 546, wherein the known label values, or amino acid concentrations of the same two or more amino acid types in the one or more proteins, peptides, oligopeptides, polypeptides, protein complexes, subproteomes, or proteomes of interest at one or more protein concentrations is calculated from the amino acid sequence or sequences of the one or more proteins, peptides, oligopeptides, polypeptides, protein complexes, subproteomes, or proteomes of interest using a set of parametric equations.

573. The method of clause 572, wherein the set of parametric equations is bounded by lower and upper limits of protein concentration available from known or calculated protein expression data.

574. The method of any of clauses 572 or 573, wherein the set of parametric equations depending on the common parameter of protein concentration is set of parametric equations 1 and takes the form:

$$p_i(t) = [a_1 t, a_2 t, \cdots a_n t], \forall t \geq 0$$

Where $p_i$ are the amino acid concentrations provided for protein, peptide, oligopeptide, polypeptide, or protein complex of interest *i* as a function of protein concentration *t*, $\langle 0, 0, \cdots 0 \rangle$ is the origin, $a_1$ is the number of amino acid type 1 in the protein, peptide, polypeptide, oligopeptide, or protein complex of interest, $a_2$ is the number of amino acid type 2 in the protein, peptide, polypeptide, oligopeptide, or protein complex of interest, $a_n$ is the number of amino acid type n in the protein, peptide, polypeptide, oligopeptide, or protein complex of interest, *t* is the total molar protein concentration of the protein, peptide, polypeptide, oligopeptide, or protein complex of interest which is defined for all values of *t* greater than or equal to 0, and there are *n* parametric equations in the set for the *n* amino acid types labelled an measured in the sample.

575. The method of any of clauses 572 or 573, wherein the set of parametric equations depending on the common parameter of protein concentration takes the form:

$$p_i(t) = [a_1 t, a_2 t, \cdots a_n t], \forall t \in c_1 \leq t \geq c_2$$

Where $p_i$ are the amino acid concentrations provided for protein, peptide, oligopeptide, polypeptide, or protein complex of interest *i* as a function of protein concentration *t*, $a_1$ is the number of amino acid type 1 in the protein, peptide, polypeptide, oligopeptide, or protein complex of interest, $a_2$ is the number of amino acid type 2 in the protein, peptide, polypeptide, oligopeptide, or protein complex of interest, $a_n$ is the number of amino acid type n in the protein, peptide, polypeptide, oligopeptide, or protein complex of interest, $c_1$ is the lower limit of the protein concentration range, $c_2$ is the upper limit of the protein concentration range, *t* is the total molar protein concentration of the protein, peptide, polypeptide, oligopeptide, or protein complex of interest which is defined for all values of *t* between $c_1$ and $c_2$, and there are n parametric equations in the set for the *n* amino acid types labelled an measured in the sample.

576.The method of any of clauses 572 or 573, wherein the set of parametric equations depending on the common parameter of protein concentration is set of parametric equations 2 and takes the form:

$$p_i(t) = [w_1 t, w_2 t, \cdots w_n t], \forall t \geq 0$$

Where $p_i$ are the amino acid concentrations provided for proteome or subproteome of interest *i* as a function of protein concentration *t*, $w_1$ is the weighted mean number of amino acid type 1 in the proteome or subproteome of interest, $w_2$ is the weighted mean number of amino acid type 2 in the proteome or subproteome of interest, $w_n$ is the number of amino acid type n in the proteome or subproteome of interest, *t* is the total molar protein concentration of the proteome or subproteome of interest which is defined for all values of *t* greater than or equal to 0, and there are n parametric equations in the set for the n amino acid types labelled an measured in the sample.

577.The method of any of clauses 572 or 573, wherein the set of parametric equations depending on the common parameter of protein concentration takes the form:

$$p_i(t) = [a_1 f_1 t + b_1, a_2 f_2 t + b_2, \cdots a_n f_n t + b_n], \forall t \in c_1 \leq t \geq c_2$$

Where $p_i$ are the known values of the label provided for protein, peptide, oligopeptide, polypeptide, or protein complex of interest *i* as a function of protein concentration *t*, $a_1$ is the number of amino acid type 1 in the protein, peptide, polypeptide, oligopeptide, or protein complex of interest, $a_2$ is the number of amino acid type 2 in the protein, peptide, polypeptide, oligopeptide, or protein complex of interest, $a_n$ is the number of amino acid type n in the protein, peptide, polypeptide, oligopeptide, or protein complex of interest, $b_1$ is the background value for amino acid type 1 which is 0 if measured values of the label in the sample are background-corrected, $b_2$ is the background value for amino acid type 2 which is 0 if measured values of the label in the sample are background-corrected, $b_n$ is the background value for amino acid type n which is 0 if measured values of the label in the sample are background-corrected, $f_1$ is the calibration function or calibration factor for amino acid type 1, $f_2$ is the calibration function or calibration factor for amino

acid type 2, $f_n$ is the calibration function or calibration factor for amino acid type $n$, $c_1$ is the lower limit of the protein concentration range, $c_2$ is the upper limit of the protein concentration range, $t$ is the total molar protein concentration of the protein, peptide, polypeptide, oligopeptide, or protein complex of interest which is defined for all values of $t$ between $c_1$ and $c_2$, and there are $n$ parametric equations in the set for the $n$ amino acid types labelled an measured in the sample.

578.The method of any of clauses 572 or 573, wherein the set of parametric equations depending on the common parameter of protein concentration is set of parametric equations 3 and takes the form:

$$p_i(t) = [a_1 f_1 t + b_1, a_2 f_2 t + b_2, \cdots a_n f_n t + b_n], \forall t \geq 0$$

Where $p_i$ are the known values of the label provided for protein, peptide, oligopeptide, polypeptide, or protein complex of interest $i$ as a function of protein concentration $t$, $a_1$ is the number of amino acid type 1 in the protein, peptide, polypeptide, oligopeptide, or protein complex of interest, $a_2$ is the number of amino acid type 2 in the protein, peptide, polypeptide, oligopeptide, or protein complex of interest, $a_n$ is the number of amino acid type $n$ in the protein, peptide, polypeptide, oligopeptide, or protein complex of interest, $b_1$ is the background value for amino acid type 1 which is 0 if measured values of the label in the sample are background-corrected, $b_2$ is the background value for amino acid type 2 which is 0 if measured values of the label in the sample are background-corrected, $b_n$ is the background value for amino acid type n which is 0 if measured values of the label in the sample are background-corrected, $f_1$ is the calibration function or calibration factor for amino acid type 1, $f_2$ is the calibration function or calibration factor for amino acid type 2, $f_n$ is the calibration function or calibration factor for amino acid type $n$, $t$ is the total molar protein concentration of the protein, peptide, polypeptide, oligopeptide, or protein complex of interest which is defined for all values of t greater than or equal to 0, and there are n parametric equations in the set for the n amino acid types labelled an measured in the sample.

579. The method of any of clauses 572 or 573, wherein the set of parametric equations depending on the common parameter of protein concentration takes the form:

$$p_i(t) = [w_1 f_1 t + b_1, w_2 f_2 t + b_2, \cdots w_n f_n t + b_n], \forall t \in c_1 \leq t \geq c_2$$

Where $p_i$ are the known values of the label provided for proteome or subproteome of interest $i$ as a function of protein concentration $t$, $w_1$ is the weighted mean number of amino acid type 1 in the proteome or subproteome of interest, $w_2$ is the weighted mean number of amino acid type 2 in the proteome or subproteome of interest, $w_n$ is the weighted mean number of amino acid type $n$ in the proteome or subproteome of interest, $b_1$ is the background value for amino acid type 1 which is 0 if measured values of the label in the sample are background-corrected, $b_2$ is the background value for amino acid type 2 which is 0 if measured values of the label in the sample are background-corrected, $b_n$ is the background value for amino acid type n which is 0 if measured values of the label in the sample are background-corrected, $f_1$ is the calibration function or calibration factor for amino acid type 1, $f_2$ is the calibration function or calibration factor for amino acid type 2, $f_n$ is the calibration function or calibration factor for amino acid type $n$, $c_1$ is the lower limit of the protein concentration range, $c_2$ is the upper limit of the protein concentration range, $t$ is the total molar protein concentration of the proteome or subproteome of interest which is defined for all values of $t$ between $c_1$ and $c_2$, and there are n parametric equations in the set for the n amino acid types labelled an measured in the sample.

580. The method of any of clause 550, wherein the vector function depending on the common parameter of protein concentration takes the form:

$$p_i(t) = [0t + a_1, 0t + a_2, \cdots 0t + a_n], \forall t \geq 0$$

Where $p_i$ are number of amino acids for protein, peptide, oligopeptide, polypeptide, or protein complex of interest $i$, $a_1$ is the number of amino acid type 1 in the protein, peptide, oligopeptide, polypeptide, or protein complex of interest, $a_2$ is the number of amino acid type 1 in the protein, peptide, oligopeptide, polypeptide, or protein complex of interest, $a_n$ is the number of amino acids of amino acid type n in the protein, peptide, oligopeptide, polypeptide, or protein complex of interest, and t is the total molar protein concentration of the proteome or subproteome of interest which is defined for all values of $t$ greater than or equal to 0, and there are $n$ parametric equations in the set for the $n$ amino acid types labelled an measured in the sample.

581.The method of any of clauses 550 wherein the vector function depending on the common parameter of protein

concentration takes the form:

$$p_i(t) = [0t + w_1, 0t + w_2, \cdots 0t + w_n], \forall t \geq 0$$

Where $p_i$ are the weighted mean number of amino acids for proteome or subproteome of interest *i*, $w_1$ is the weighted mean number of amino acid type 1 in the proteome or subproteome of interest, $w_2$ is the weighted mean number of amino acid type 2 in the proteome or subproteome of interest, $w_n$ is the weighted mean number of amino acid type *n* in the proteome or subproteome of interest, and t is the total molar protein concentration of the proteome or subproteome of interest which is defined for all values of t greater than or equal to 0, and there are n parametric equations in the set for the *n* amino acid types labelled an measured in the sample.

582. The method of clause 572, wherein the known label values, or amino acid concentrations of the same two or more amino acid types in one or more proteins, peptides, oligopeptides, polypeptides or protein complexes is calculated from the amino acid sequence of the one or more proteins, peptides, oligopeptides, polypeptides or protein complexes using set of parametric equations 1 or 3, or vector function 1 or 3.

583. The method of clause 572, wherein the known label values, or amino acid concentrations of the same two or more amino acid types in one or more proteomes, or subproteomes of interest is calculated from the amino acid sequences of the one or more proteomes or subproteomes of interest using set of parametric equations 2 or 4, or vector function 2 or 4.

584. The method of clause 549, wherein the reference is obtained from a database.

585. The method of any one of the preceding clauses, wherein step e) comprises identifying the presence and/or concentration and/or amount of the one or more proteins, peptides, oligopeptides, polypeptides, protein complexes, subproteomes, or proteomes of interest in the sample when the measured label, amino acid concentration or number of amino acids of each labelled amino acid type in the sample is the same as, or less than or equal to an error margin to the known label values, amino acid concentrations or number of amino acids of the same two or more amino acid types that have been labelled in the sample in the one or more proteins, peptides, oligopeptides, polypeptides, protein complexes, subproteomes, or proteomes of interest.

586. The method of clause 585, wherein the error margin includes a user-specified tolerance value, or is an order statistic of the minimum distances between the measured labels, amino acid concentrations or number of the labelled amino acid types of the sample and the known label values, amino acid concentrations or number of amino acids of the same amino acid types of the proteins, peptides, oligopeptides, polypeptides, protein complexes, subproteomes, or proteomes of interest where the *k*th order statistic is the kth smallest value.

587. The method of clause 585, wherein the error margin is a distance threshold between the measured labels, amino acid concentrations or number of the labelled amino acid types of the sample and the known label values, amino acid concentrations or number of amino acids of the same amino acid types in the proteins, peptides, oligopeptides, polypeptides, protein complexes, subproteomes, or proteomes of interest.

588. The method of clause 587, wherein the distance between the measured labels, amino acid concentrations or number of the labelled amino acid types of the sample and the known label values, amino acid concentrations or number of amino acids of the same amino acid types in each protein, peptide, oligopeptide, polypeptide, protein complexe, subproteome, or proteome of interest is a Euclidian distance measurement.

589. The method of clause 585, wherein the error margin is the minimum distance between measured label, amino acid concentration or number of the labelled amino acid type of the sample and the known label value, amino acid concentration or number of amino acids of the same amino acid types in the proteins, peptides, oligopeptides, polypeptides, protein complexes, subproteomes or proteomes of interest.

590. The method of clause 588, wherein a Euclidian distance measurement is calculated using Equation 17:

$$D = \sqrt{\sum_{i=1}^{n}(S_i - Q_i)^2}$$

where $S_i$ is the value (value of the label, amino acid concentration, or number of amino acids) measured for the sample for amino acid type $i$ = 1:$n$ , and $Q_i$ is the corresponding value provided for the protein, peptide, oligopeptide, polypeptide, protein complex, subproteome, or proteome of interest for amino acid type $i$ = 1: $n$.

591. The method of clause 585, wherein the error margin includes a user-specific tolerance value multiplied by the values of the label, amino acid concentration, or number of amino acids of two or more amino acid types measured for the sample.

592. The method of clause 585, wherein the error margin includes a user-specific tolerance value multiplied by the square root of the sum of the values of the label, amino acid concentration, or number of amino acids of two or more amino acid types measured for the sample squared.

593. The method of clause 585, wherein the error margin is provided from a user-inputted tolerance value, that is multiplied by the square root of the sample values squared, reflecting the distance calculation. This is provided by equation 8:

$$\varepsilon = \varphi\sqrt{S_1^2 + S_2^2 + \cdots + S_n^2}$$

wherein $\varepsilon$ is the error margin, $\varphi$ is a user-inputted tolerance value, $S_1$ is the value (value of the label, amino acid concentration, or number of amino acids) measured for the sample for amino acid type 1, $S_2$ is the value (value of the label, amino acid concentration, or number of amino acids) measured for the sample for amino acid type 1, and $S_n$ is the value (value of the label, amino acid concentration, or number of amino acids) measured for the sample for amino acid type (n).

594. The method of clause 593, wherein the user-specified tolerance value, $\varphi$, is 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, or 0.10.

595. The method of clause 585, wherein if it is suspected that the sample contains k proteins, peptides, oligopeptides, polypeptides, protein complexes, subproteomes, or proteomes of interest, the error margin is the $k^{th}$ order statistic of the distances calculated for all of the proteins, peptides, oligopeptides, polypeptides, protein complexes, subproteomes, or proteomes of interest.

596. The method of clause 585, wherein if it is suspected that the sample contains *k* proteins, peptides, oligopeptides, polypeptides, protein complexes, subproteomes, or proteomes of interest, the distances are sorted, and the error margin is *k* smallest distance.

597. The method of any one of the preceding clauses, wherein step e) comprises comparing the measured label of each labelled amino acid type in the sample to the known label values of the same two or more amino acid types of the one or more proteins, peptides, oligopeptides, polypeptides, protein complexes, subproteomes, or proteomes of interest at one or more protein concentrations, wherein the known label value of the two or more amino acid types of the one or more proteins, peptides, oligopeptides, polypeptides, protein complexes, subproteomes, or proteomes of interest at one or more protein concentrations is calculated from the amino acid sequence or sequences and/or experimental information about post-translation modifications of the one or more proteins, peptides, oligopeptides, polypeptides, protein complexes, subproteomes, or proteomes of interest using a vector function or a set of parametric equations.

598. The method of any one of the preceding clauses, wherein step e) comprises comparing the amino acid concentration of each labelled amino acid type in the sample to the amino acid concentrations of the same two or more amino acid types of the one or more proteins, peptides, oligopeptides, polypeptides, protein complexes, subproteomes, or proteomes of interest at one or more protein concentrations, wherein the amino acid concentrations of the two or more amino acid types of the one or more proteins, peptides, oligopeptides, polypeptides, protein complexes, subproteomes, or proteomes of interest at one or more protein concentrations is calculated from the amino acid sequence or sequences and/or experimental information about post-translation modifications of the one or more proteins, peptides, oligopeptides, polypeptides, protein complexes, subproteomes, or proteomes of interest using a vector function or a set of parametric equations.

599. The method of any one of the preceding clauses, wherein step e) comprises comparing the number of amino

acids of each labelled amino acid type in the sample to the number of amino acids of the same two or more amino acid types of the one or more proteins, peptides, oligopeptides, polypeptides, protein complexes, subproteomes, or proteomes of interest, wherein the number of amino acids of the two or more amino acid types of the one or more proteins, peptides, oligopeptides, polypeptides, protein complexes, subproteomes, or proteomes of interest is calculated from the amino acid sequence or amino acid sequences and/or experimental information about post-translation modifications of the one or more proteins, peptides, oligopeptides, polypeptides, protein complexes, subproteomes, or proteomes of interest.

600. The method of any one of the preceding clauses, wherein step e) comprising comparing the measured label, amino acid concentration and/or number of amino acids of each labelled amino acid type in the sample to the known label values or amino acid concentrations of the same two or more amino acid types in the one or more proteins, peptides, oligopeptides, polypeptides, protein complexes, subproteomes, or proteomes of interest at one or more protein concentrations, or number of amino acids of the same two or more amino acid types in the one or more proteins, peptides, oligopeptides, polypeptides, protein complexes, subproteomes, or proteomes of interest using an *n*-dimensional space.

601. The method of any one of the preceding claims, wherein step e) comprises comparing the measured label, amino acid concentration and/or number of amino acids of each labelled amino acid type in the sample to the known label values or amino acid concentrations of the same two or more amino acid types in the one or more proteins, peptides, oligopeptides, polypeptides, protein complexes, subproteomes or proteomes of interest as a function of protein concentration, or number of amino acids of the same two or more amino acid types in the one or more proteins, peptides, oligopeptides, polypeptides, protein complexes, subproteomes or proteomes of interest using an n-dimensional space, wherein the known label values or amino acid concentrations of the same two or more amino acid types in the one or more proteins, peptides, oligopeptides, polypeptides, protein complexes, subproteomes, or proteomes of interest as a function of protein concentration provide a line or a curve in n-dimensional space which can optionally be bounded by known protein expression levels in biological samples, and the number of amino acids of the same two or more amino acid types in the one or more proteins, peptides, oligopeptides, polypeptides, protein complexes, subproteomes, or proteomes of interest provide a point in *n*-dimensional space

602. The method of clause 601, wherein 2 amino acids are labelled in the sample, and the *n*-dimensional space is a 2-dimensional space.

603. The method of clause 601, wherein 3 amino acids are labelled in the sample, and the n-dimensional space is a 3-dimensional space.

604. The method of clause 601, wherein 4 amino acids are labelled in the sample, and the n-dimensional space is a 4-dimensional space.

605. The method of clause 601, wherein 5 amino acids are labelled in the sample, and the n-dimensional space is a 5-dimensional space.

606. The method of clause 601, wherein 6 amino acids are labelled in the sample, and the n-dimensional space is a 6-dimensional space.

607. The method of clause 601, wherein 7 amino acids are labelled in the sample, and the n-dimensional space is a 7-dimensional space.

608. The method of clause 601, wherein 8 amino acids are labelled in the sample, and the n-dimensional space is a 8-dimensional space.

609. The method of clause 601, wherein 9 amino acids are labelled in the sample, and the n-dimensional space is a 9-dimensional space.

610. The method of clause 601, wherein 10 amino acids are labelled in the sample, and the n-dimensional space is a 10-dimensional space.

611. The method of clause 601, wherein 11 amino acids are labelled in the sample, and the n-dimensional space is a 11-dimensional space.

612. The method of clause 601, wherein 12 amino acids are labelled in the sample, and the n-dimensional space is a 12-dimensional space.

613. The method of clause 601, wherein 13 amino acids are labelled in the sample, and the n-dimensional space is a 13-dimensional space.

614. The method of clause 601, wherein 14 amino acids are labelled in the sample, and the n-dimensional space is a 14-dimensional space.

615. The method of clause 601, wherein 15 amino acids are labelled in the sample, and the n-dimensional space is a 15-dimensional space.

616. The method of clause 601, wherein 16 amino acids are labelled in the sample, and the n-dimensional space is a 16-dimensional space.

617. The method of clause 601, wherein 17 amino acids are labelled in the sample, and the n-dimensional space is a 17-dimensional space.

618. The method of clause 601, wherein 18 amino acids are labelled in the sample, and the n-dimensional space is a 18-dimensional space.

619. The method of clause 601, wherein 19 amino acids are labelled in the sample, and the n-dimensional space is a 19-dimensional space.

620. The method of clause 601, wherein 20 amino acids are labelled in the sample, and the n-dimensional space is a 20-dimensional space.

621. The method of clause 601, wherein 21 amino acids are labelled in the sample, and the n-dimensional space is a 21-dimensional space.

622. The method of clause 601, wherein 22 amino acids are labelled in the sample, and the n-dimensional space is a 22-dimensional space.

623. The method of clause 601, wherein 23 amino acids are labelled in the sample, and the n-dimensional space is a 23-dimensional space.

624. The method of clause 601, wherein 24 amino acids are labelled in the sample, and the n-dimensional space is a 24-dimensional space.

625. The method of clause 601, wherein 25 amino acids are labelled in the sample, and the n-dimensional space is a 25-dimensional space.

626. The method of clause 601, wherein 26 amino acids are labelled in the sample, and the n-dimensional space is a 26-dimensional space.

627. The method of clause 601, wherein 27 amino acids are labelled in the sample, and the n-dimensional space is a 27-dimensional space.

628. The method of clause 601, wherein 28 amino acids are labelled in the sample, and the n-dimensional space is a 28-dimensional space.

629. The method of clause 601, wherein 29 amino acids are labelled in the sample, and the n-dimensional space is a 29-dimensional space.

630. The method of clause 601, wherein 30 amino acids are labelled in the sample, and the n-dimensional space is a 30-dimensional space.

631. The method of clause 601, wherein 31 amino acids are labelled in the sample, and the n-dimensional space is a

31-dimensional space.

632. The method of clause 601, wherein 32 amino acids are labelled in the sample, and the n-dimensional space is a 32-dimensional space.

633. The method of clause 601, wherein 33 amino acids are labelled in the sample, and the n-dimensional space is a 33-dimensional space.

634. The method of clause 601, wherein 34 amino acids are labelled in the sample, and the n-dimensional space is a 34-dimensional space.

635. The method of clause 601, wherein 35 amino acids are labelled in the sample, and the n-dimensional space is a 35-dimensional space.

636. The method of clause 601, wherein 36 amino acids are labelled in the sample, and the n-dimensional space is a 36-dimensional space.

637. The method of clause 601, wherein 37 amino acids are labelled in the sample, and the n-dimensional space is a 37-dimensional space.

638. The method of clause 601, wherein 38 amino acids are labelled in the sample, and the n-dimensional space is a 38-dimensional space.

639. The method of clause 601, wherein 39 amino acids are labelled in the sample, and the n-dimensional space is a 39-dimensional space.

640. The method of clause 601, wherein 40 amino acids are labelled in the sample, and the n-dimensional space is a 40-dimensional space.

641. The method of clause 585, wherein one or more proteins, peptides, oligopeptides, polypeptides, protein complexes, subproteomes, or proteomes of interest is identified in the sample if there exists a single value of protein concentration for which the value of the label or amino acid concentration of two or more amino acid types measured in the sample is equal to, or less than or equal to an error margin to, the known values of the label or amino acid concentrations of two or more amino acid types provided by the reference functions for one or more proteins, peptides, oligopeptides, polypeptides, protein complexes, subproteomes, or proteomes of interest

642. The method of clause 585, wherein the protein concentration of a protein, peptide, oligopeptide, polypeptide, protein complex, subproteome, or proteome of interest identified in the sample is the protein concentration for which the value of the label or amino acid concentration of two or more amino acid types measured in the sample was equal to, or less than or equal to an error margin to, the known values of the label or amino acid concentrations of two or more amino acid types provided by the reference functions for the protein, peptide, oligopeptide, polypeptide, protein complex, subproteome, or proteome of interest identified in the sample.

643. The method of clauses 642, wherein the protein amount of the protein, peptide, oligopeptide, polypeptide, protein complex, subproteome, or proteome of interest identified in the sample is the protein concentration of the protein, peptide, oligopeptide, polypeptide, protein complex, subproteome, or proteome of interest identified in the sample multiplied by the volume of the sample.

644. The method of any of the preceding clauses, wherein the presence of a protein, peptide, oligopeptide, polypeptide, protein complex, subproteome, or proteome of interest identified in the sample if there exists a single value of protein concentration for which the amino acid concentrations of two or more amino acid types measured for the sample are equal to the amino acid concentrations of the same two or more corresponding amino acid types provided for the protein, peptide, oligopeptide, polypeptide, protein complex, subproteome, or proteome of interest by its reference vector function or set of parametric equations.

645. The method of clause 228, wherein the sample point is on the reference line if a single solution for protein concentration, *t*, exists, and this solution for *t* is the protein concentration of the protein, peptide, oligopeptide, polypeptide, protein complex, subproteome, or proteome of interest identified in sample.

646. The method of clause 585,wherein one or more proteins, peptides, oligopeptides, polypeptides, protein complexes, subproteomes, or proteomes of interest is identified in the sample if there exists a single value of protein concentration for which the value of the label or amino acid concentration of two or more amino acid types measured in the sample is less than or equal to an error margin to the known values of the label or amino acid concentrations of two or more amino acid types provided by the reference functions for one or more proteins, peptides, oligopeptides, polypeptides, protein complexes, subproteomes, or proteomes of interest.

647. The method of clause 234, wherein the distance between the value of the label or amino acid concentration of two or more amino acid types measured in the sample and the known values of the label or amino acid concentrations of two or more amino acid types provided by the reference functions for one or more proteins, peptides, oligopeptides, polypeptides, protein complexes, subproteomes, or proteomes of interest is calculated.

648. The method of clause 647, wherein the minimum distance between the value of the label or amino acid concentration of two or more amino acid types measured in the sample and the known values of the label or amino acid concentrations of two or more amino acid types provided by the reference functions for one or more proteins, peptides, oligopeptides, polypeptides, protein complexes, subproteomes, or proteomes of interest is calculated by finding a point on the reference line for which the distance between the sample point and the reference line is perpendicular.

649. The method of clause 648, wherein the point on the reference line for which the distance between the sample point and the reference line is perpendicular is found by providing a general vector equation for the vector between the sample point and the reference line, taking the dot product of this vector with the direction vector of the reference line, setting the dot product equal to 0, and solving for protein concentration, t, which is the protein concentration of the reference line which yields the point to which the distance from the sample point is perpendicular.

650. The method of clause 649, wherein the amino acid concentration or value of the label for each amino acid type at this protein concentration on the reference line is calculated, and the distance between this point an the sample point is calculated and compared to the error margin.

651. The method of clause 650, wherein if the distance is less than or equal to the error margin, the protein, peptide, oligopeptide, polypeptide, protein complex, subproteome, or proteome of interest is contained in the sample at the protein concentration for which the distance was perpendicular.

652. The method of clause 647, wherein if more than one protein, peptide, oligopeptide, polypeptide, protein complex, subproteome, or proteome of interest is identified in the sample, then a mixture of proteins, peptides, oligopeptides, polypeptides, protein complexes, subproteomes, or proteomes of interest is identified in the sample, and the relative composition of each component within the mixture is inversely related to the distance between the values measured for the sample and the values provided for each identified component of the mixture.

653. The method of clause 652, wherein the relative composition of each component within the mixture is determined by inverse normalizing the distance between the sample and each component with the maximum distance between the sample and any component.

654. The method of clause 653, wherein the inverse normalized distance of each component is divided by the sum of the inverse normalized distance of all components to provide the relative composition of each component within the mixture.

655. The method of clause 652, wherein the relative composition of each component within the mixture is multiplied by the protein concentration at which each protein, peptide, oligpeptide, polypeptide, protein complex, subproteome, or proteome of interest was identified, to provide the concentration of each protein, peptide, oligpeptide, polypeptide, protein complex, subproteome, or proteome of interest within the mixture.

656. The method of any one of the preceding clauses, wherein the known label values, amino acid concentrations or number of amino acids of the same two or more amino acid types in a subproteome or proteome of interest is a weighted mean based on the known label value, amino acid concentrations or number of amino acids of each amino acid type based on all amino acid sequences contained within the proteome or subproteome of interest.

657. The method of any one of the preceding clauses, wherein the number of amino acids of the same two or more amino acid types in a subproteome or proteome of interest is a weighted mean of the numbers of amino acids of each

amino acid type in all amino acid sequences contained within the proteome or subproteome of interest

658. The method of claim 656, wherein the known label values or amino acid concentrations of the same two or more amino acid types in a subproteome or proteome of interest are calculated using a weighted mean number of amino acids of each amino acid type of all amino acid sequences contained within the proteome or subproteome of interest.

659. The method of any one of the preceding clauses, wherein step e) comprises removing any proteins, peptides, oligopeptides, polypeptides, protein complexes, subproteomes, or proteomes of interest from the sample for which the measured labels, amino acid concentrations or numbers of each amino acid type refers to duplicate proteins, peptides, oligopeptides, polypeptides, protein complexes, subproteomes, proteomes of interest.

660. The method of any one of the preceding clauses, wherein step e) comprises identifying the presence and/or concentration and/or amount of the one or more proteins, peptides, oligopeptides, polypeptides, protein complexes, subproteomes, or proteomes of interest in the sample when the measured label, amino acid concentration or number of amino acids of each labelled amino acid type in the sample is the same as, or less than or equal to an error margin to the known label values, amino acid concentrations or number of amino acids of the same two or more amino acid types in the one or more proteins, peptides, oligopeptides, polypeptides, protein complexes, subproteomes, or proteomes of interest, and when the identified concentration is within the protein concentration bounds ($c_1$, $c_2$) based on known concentration levels of the one or more proteins, peptides, oligopeptides, polypeptides, protein complexes, subproteomes, or proteomes of interest in a sample type of interest.

661. The method of clauses 233-238, wherein only the direction of the vector corresponding to the protein, peptide, oligopeptide, polypeptide, protein complex, proteome, or subproteome of interest is considered when finding the minimum distance between the sample point and any point on the vector corresponding to the protein, peptide, oligopeptide, polypeptide, protein complex, proteome, or subproteome of interest, via the dot product between the vector between the sample point and any point on the vector corresponding to the protein, peptide, oligopeptide, polypeptide, protein complex, proteome, or subproteome of interest and the direction of the vector corresponding to the protein, peptide, oligopeptide, polypeptide, protein complex, proteome, or subproteome of interest.

662. The method of clause 564, 566, 568, wherein the vector corresponding to the protein, peptide, oligopeptide, polypeptide, protein complex, proteome, or subproteome of interest is treated as unbounded, or bounded only at the origin, when calculating the dot product.

663. The method of clauses 1a-1h, wherein three amino acid types are labelled in the sample and the measured labels, amino acid concentration or number of amino acids of each of the three labelled amino acid type in the sample are compared to the known label value, amino acid concentration or number of amino acids of the same three amino acid types of no more than 200 proteins, peptides, oligopeptides, polypeptides, protein complexes, subproteomes, or proteomes of interest.

664. The method of any one of clauses 1a-1h, wherein three amino acid types are labelled in the sample and the measured labels, amino acid concentration or number of amino acids of each of the three labelled amino acid type in the sample are compared to the known label value, amino acid concentration or number of amino acids of the same three amino acid types of no more than 9000 proteomes or subproteomes of interest.

665. The method of any one of the preceding clauses, wherein the proteome or subproteome of interest have less than 4000 proteins.

666. The method of any one of the preceding clauses, wherein the sample is not sequenced to identify the proteins, peptides, oligopeptides, polypeptides, protein complexes, subproteomes, proteomes of interest via the order of the amino acids within the sample.

667. The method of any one of the preceding clauses, wherein the sample does not need to be separated into individual protein components in order to identify the presence and/or concentration and/or amount of a proteome, subproteome, or mixture of proteins, peptides, polypeptide, oligopeptides, subproteomes or proteomes of interest in the sample.

668. The method of any one of clauses 1-350, step a) comprises isolating the protein component from the sample prior to labelling.

669. The method of clause 668, wherein the protein component is isolated using centrifugation, filtration, electrophoresis, or chromatography.

670. The method of clause 669, wherein the chromatography isolation involves HPLC.

671. The method of any one of the preceding clauses, wherein the method is carried out in bulk.

672. The method of any one of the preceding clauses, wherein steps d) and e) are carried out in a classifier.

673. The method of any one of clauses 472 or 475, wherein the same proportion of amino acids of an amino acid type are labelled in the sample as are labelled in any/all proteins and/or amino acids used to create a calibration curve.

674. The method of any one of clauses 472 or 476, wherein the same proportion of amino acids of an amino acid type are labelled in the sample as are labelled in any protein used as a standard.

675. The method of any one of clauses 472 or 476, wherein the same proportion of amino acids of an amino acid type are labelled in the sample as are labelled in any amino acid used as a standard

676. The method of any one of clauses 472 and 475, wherein +/- 5% of the same proportion of amino acids of an amino acid type are labelled in the sample as are labelled in any/all proteins and/or amino acids used to create a calibration curve

677. The method of any one of clauses 472 and 476, wherein +/- 5% of the same proportion of amino acids of an amino acid type are labelled in the sample as are labelled in any protein used as a standard.

678. The method of any one of clauses 472 and 476, wherein +/-5% of the same proportion of amino acids of an amino acid type are labelled in the sample as are labelled in any amino acid used as a standard

679. The method of any one of clauses 472 and 475, wherein +/- 10% of the same proportion of amino acids of an amino acid type are labelled in the sample as are labelled in any/all proteins and/or amino acids used to create a calibration curve

680. The method of any one of clauses 472 and 476 , wherein +/- 10% of the same proportion of amino acids of an amino acid type are labelled in the sample as are labelled in any protein used as a standard

681. The method of any one of clauses 472 and 476, wherein +/-10% of the same proportion of amino acids of an amino acid type are labelled in the sample as are labelled in any amino acid used as a standard

682. The method of clause 225 or 545, wherein the amino acid concentration of the two or more amino acid types in the sample is compared to the amino acid concentration of the same two or more amino acid types in one or more proteins, peptides, oligopeptides, polypeptides, protein complexes, subproteomes or proteomes of interest, wherein the amino acid concentration of the same two or more amino acid types in one or more proteins, peptides, oligopeptides, polypeptides, protein complexes, subproteomes or proteomes of interest is an experimental reference.

683. The method of clause 682, wherein the same proportion of amino acids of an amino acid type are labelled in the sample as are labelled in any/all proteins and/or amino acids used to create a calibration curve and any experimental reference

684. The method of clause 682, wherein the same proportion of amino acids of an amino acid type are labelled in the sample as are labelled in any protein used as a standard and any experimental reference.

685. The method of clause 682, wherein the same proportion of amino acids of an amino acid type are labelled in the sample and any experimental reference as are labelled in any amino acid used as a standard.

686. The method of clause 682, wherein +/- 5% of the same proportion of amino acids of an amino acid type are labelled in the sample and any experimental reference as are labelled in any/all proteins and/or amino acids used to create a calibration curve.

687. The method of any one of clause 682, wherein +/- 5% of the same proportion of amino acids of an amino acid type are labelled in the sample and any experimental reference as are labelled in any protein used as a standard.

688. The method of clause 682, wherein +/-5% of the same proportion of amino acids of an amino acid type are labelled in the sample and any experimental reference as are labelled in any amino acid used as a standard.

689. The method of clause 682, wherein +/- 10% of the same proportion of amino acids of an amino acid type are labelled in the sample and any experimental reference as are labelled in any/all proteins and/or amino acids used to create a calibration curve.

690. The method of clause 682, wherein +/- 10% of the same proportion of amino acids of an amino acid type are labelled in the sample and any experimental reference as are labelled in any protein used as a standard.

691. The method of clause 682, wherein +/-10% of the same proportion of amino acids of an amino acid type are labelled in the sample and any experimental reference as are labelled in any amino acid used as a standard.

692. The method of clause 682, wherein the same proportion of amino acids of an amino acid type are labelled in the sample and any experimental reference.

693. The method of clause 682, wherein +/- 5% of the same proportion of amino acids of an amino acid type are labelled in the sample and any experimental reference.

694. The method of clause 682, wherein +/- 5% of the same proportion of amino acids of an amino acid type are labelled in the sample and any experimental reference.

695. The method of clause 682, wherein +/-5% of the same proportion of amino acids of an amino acid type are labelled in the sample and any experimental reference.

696. The method of clause 682, wherein +/- 10% of the same proportion of amino acids of an amino acid type are labelled in the sample and any experimental reference.

697. The method of clause 682, wherein +/- 10% of the same proportion of amino acids of an amino acid type are labelled in the sample and any experimental reference.

698. The method of clause 682, wherein +/-10% of the same proportion of amino acids of an amino acid type are labelled in the sample and any experimental reference.

699. The method of clause 1c, wherein the bacterial proteome is salmonella and/or E Coli.

700. The method of clause 1d wherein the viral proteome of interest is the SARS-CoV-2 proteome.

701. The method of clause 1d, wherein the viral proteome of interest is a zoonotic virus proteome.

702. The method of clause 1d, wherein the viral proteome of interest is the HIV proteome.

703. The method of clause 1f, wherein the human proteins, peptides, oligopeptides, polypeptides, protein complexes, subproteomes, or proteomes of interest are used for early detection of cancer.
704. The method of clause 1g, wherein the infection is a zoonotic infection.

## BRIEF DESCRIPTION OF THE DRAWINGS

Embodiments of the invention are described below with reference to the accompanying drawings, in which:

**Figure 1** shows a schematic drawing illustrating how the unique signatures calculated for Protein-A of interest, Protein-B of interest, Protein-C of interest, and Protein-D of interest vary as a function of the protein concentration of each protein of interest. Reference vectors are provided for each protein of interest, and each point on the reference vector corresponds to a unique protein concentration of the protein of interest (e.g. 1 μM, filled circle). The shortest distance from the Sample point (open square) to each reference line is calculated, identifying the presence of Protein-B of interest in the Sample; the concentration of Protein-B of interest in the sample is the protein concentration of Protein-B of interest which provided the shortest distance (e.g. 0.5 μM).

**Figure 2** shows reference lines in n-dimensional space. Set of parametric equations 1 provides the following reference lines for BSA, LYZ, and TTR. The sample point is shown with an open circle. The methods of the invention include determining the presence and/or concentration and/or amount of the proteins/protein complexes of interest in the sample based on a comparison of the distance between the sample point and each reference line.

**Figure** 3 shows the unique signatures for pathogenic proteomes. (a) All 7581 bacterial reference proteomes analysed have a unique signature of known label values, amino acid concentrations, or mean number of amino acids across all proteins in the bacterial reference proteome. (b) Zoomed image showing a wide distribution of the mean number of the number of amino acids of two or more amino acid types within every average protein sequence. (c) All 9377 viral reference proteomes analysed have a unique signature of known label values, amino acid concentrations, or mean number of amino acids across all proteins in the viral reference proteome. (d) All 16958 bacterial and viral reference proteomes analysed have a unique signature of known label values, amino acid concentrations, or mean number of amino acids across all proteins in the bacterial or viral reference proteome. This enables the identification of a whole proteome in a sample without separation.

**Figure 4** shows analysis of the probability distribution of leading digits in a set of numbers according to Benford's law shows that amino acid types in the human plasma proteome follow the expected distribution.

**Figure 5** shows analysis of the probability distribution of leading digits in a set of numbers according to Benford's law shows that mean numbers of amino acids across proteins, peptides, oligopeptides, polypeptides, and protein subunits in viral proteomes deviate from the expected distribution, suggesting increased variability in this dataset relative to human proteomes.

**Figure 6** shows analysis of the probability distribution of leading digits in a set of numbers according to Benford's law shows that mean numbers of amino acids across proteins, peptides, oligopeptides, polypeptides, and protein subunits in bacterial proteomes deviate from the expected distribution, suggesting increased variability in this dataset relative to human proteomes.

**Figure 7** shows identifying the order of amino acids within a protein sequence within the human proteome is inefficient compared to identifying only the number of amino acids within a protein sequence. Identifying the order of two types of amino acids within a protein sequence adds no additional information to identifying the order of one type of amino acid within a protein sequence.

**Figure 8** shows demonstration of the effect of constraining the reference line to known protein concentration ranges within the human plasma proteome. (a) Reference lines for all 3263 proteins, peptides, oligopeptides, polypeptides, and protein complexes within the human plasma proteome. (b) Bounded reference lines for all 3263 proteins, peptides, oligopeptides, polypeptides, and protein complexes within the human plasma proteome, wherein the reference lines are bounded by the known concentration ranges of these proteins, peptides, oligopeptides, polypeptides, and protein complexes within the human plasma proteome.

**Figure 9** shows the occurrences of references referring to more than one protein of interest was quantified across the human plasma proteome for various combinations of amino acid types (C and W, K and W, K and Y, K and S, K and P, L and S, L and K, E and L, G and L, C K and W, C K and Y, L K and S, E G and K, E G and S, R E P and T, and Q L K and V - with and without protein concentration information, accessible via the methods of the invention, compared to known protein concentration bounds.

**Figure 10** shows when two amino acid types are labelled and compared, without application of any bounds or constraints on the protein concentration or other classification, all references are distinguishable and map uniquely to proteins of interest within most of the clinically relevant proteomes and subproteomes considered (SARS-CoV-2, HIV, Epstein-Barr, Glioma) and do not correspond to multiple proteins of interest within the clinically relevant proteomes and subproteomes.

**Figure 11** shows comparing the information content provided by all combinations of two amino acid types to the uniqueness of references for protein sequences within the (a) human plasma proteome and (b) human salivary proteome.

**Figure 12** shows that all reference bacterial proteomes (7581 reference proteomes) have a mean number of amino acids within two amino acid types across proteins in their proteomes that is distinct from all other mean numbers of amino acids within two amino acid types across proteins all other proteomes.

**Figure 13** shows that for the labelling of only two amino acid types within a proteome of interest, bacterial and viral proteomes cluster together according to their lineage. Here the labeling of K and W amino acid types is provided, showing clustering within the orders: Corynebacteriaceae, Legionellales, Bacillales, Streptomycetaceae, and Mycoplasmataceae.

**Figure 14** describes the treatment for an unknown mixture of proteins. The identity of the mixture is unknown, and the protein concentration of the mixture is unknown.

**Figure 15** shows that hydrodynamic radius cannot be predict based on protein sequences alone, because state-of-the art scaling methods still require knowledge of whether a protein is folded or unfolded, and do not account for partial intrinsic disorder.

**Figure 16** schematics showing the reaction of (a) Tryptophan (W), (b) Tyrosine (Y), (c) Reduced Cysteine ($C_R$), (d) Cysteine (C), and (e) Lysine (K) amino acid types with fluorogenic dyes, or molecules which becomes fluorescent upon reaction with the indicated amino acid type.

**Figure 17** shows comparison of Patient samples to (a) C and K, (b) C and W, and (C) K and W SARS-CoV-2 and Influenza A reference lines.

**Figure 18** shows a calibration curve for conversion of background-corrected fluorescence intensity from the K amino acid type K F.I. in arbitrary units (AU) to amino acid concentration of the K amino acid type [K] in μM. Nonlinear regression revealed a provided a polynomial fit for the calibration curve with $R^2$ = 0.9987.

**Figure 19** shows a calibration curve for conversion of background-corrected fluorescence intensity from the C amino acid type C F.I. in arbitrary units (AU) to amino acid concentration of the C amino acid type [C] in μM. Nonlinear regression revealed a provided a polynomial fit for the calibration curve with $R^2$ = 0.9886.

**Figure 20** shows a calibration curve for conversion of background-corrected fluorescence intensity from the W amino acid type W F.I. in arbitrary units (AU) to amino acid concentration of the W amino acid type [W] in μM. Nonlinear regression revealed a provided a polynomial fit for the calibration curve with $R^2$ = 0.9886.

**Figure 21** shows that when the mean measured amino acid concentrations across the three technical replicates of each experimentally measured patient PPP sample are plotted in N-dimensional space (4-dimensional space), the data takes on a line in N-dimensional space as predcted by the concepts of the invention. This conceptual line was illustrated by drawing a line through the data set. To calculate the actual position and equation of the reference line defining the PPP proteome of interest, the K, C, W, and Y components of the vector function defining the PPP proteome of interest were calculated experimentally in the following figures.

**Figure 22** shows how the coefficient (direction) of the K component of the experimental reference line was calculated for the PPP and PRP proteomes of interest. The measured amino acid molar concentrations in μM of the amino acid type K are plotted against the measured total protein concentrations in μg/mL for each proteome of interest and a linear regression was performed. The linear regression was constrained to pass through the origin.

**Figure 23** shows how the coefficient (direction) of the C component of the experimental reference line was calculated for the PPP and PRP proteomes of interest. The measured amino acid molar concentrations in μM of the amino acid type C are plotted against the measured total protein concentrations in μg/mL for each proteome of interest and a linear regression was performed. The linear regression was constrained to pass through the origin.

**Figure 24** shows how the coefficient (direction) of the W component of the experimental reference line was calculated for the PPP and PRP proteomes of interest. The measured amino acid molar concentrations in μM of the amino acid type W are plotted against the measured total protein concentrations in μg/mL for each proteome of interest and a linear regression was performed. The linear regression was constrained to pass through the origin.

**Figure 25** shows how the coefficient (direction) of the Y component of the experimental reference line was calculated for the PPP and PRP proteomes of interest. The measured amino acid molar concentrations in μM of the amino acid type Y are plotted against the measured total protein concentrations in μg/mL for each proteome of interest and a linear regression was performed. The linear regression was constrained to pass through the origin.

**Figure 26** shows how the coefficient (direction) of the K component of the experimental reference line was calculated for the PPP_50 and PRP_50 subproteomes of interest. The measured amino acid molar concentrations in μM of the amino acid type K are plotted against the measured total protein concentrations in μg/mL for each subproteome of interest and a linear regression was performed. The linear regression was constrained to pass through the origin.

**Figure 27** shows how the coefficient (direction) of the C component of the experimental reference line was calculated for the PPP_50 and PRP_50 subproteomes of interest. The measured amino acid molar concentrations in μM of the amino acid type C are plotted against the measured total protein concentrations in μg/mL for each subproteome of interest and a linear regression was performed. The linear regression was constrained to pass through the origin.

**Figure 28** shows how the coefficient (direction) of the W component of the experimental reference line was calculated for the PPP_50 and PRP_50 subproteomes of interest. The measured amino acid molar concentrations in μM of the amino acid type W are plotted against the measured total protein concentrations in μg/mL for each subproteome of interest and a linear regression was performed. The linear regression was constrained to pass through the origin. In the subsequent 8 figures, how the coefficient (direction) of the components of the experimental reference lines based on the common parameter of molar, rather than mass, protein concentration is explained.

**Figure 29** shows how the coefficient (direction) of the K component of the experimental reference line was calculated for the PPP and PRP proteomes of interest. The measured amino acid molar concentrations in μM of the amino acid type K are plotted against the measured total protein molar concentrations in μM for each proteome of interest and a linear regression was performed. The linear regression was constrained to pass through the origin.

**Figure 30** shows how the coefficient (direction) of the C component of the experimental reference line was calculated for the PPP and PRP proteomes of interest. The measured amino acid molar concentrations in μM of the amino acid

type C are plotted against the measured total protein molar concentrations in μM for each proteome of interest and a linear regression was performed. The linear regression was constrained to pass through the origin.

**Figure 31** shows how the coefficient (direction) of the W component of the experimental reference line was calculated for the PPP and PRP proteomes of interest. The measured amino acid molar concentrations in μM of the amino acid type W are plotted against the measured total protein molar concentrations in μM for each proteome of interest and a linear regression was performed. The linear regression was constrained to pass through the origin.

**Figure 32** shows how the coefficient (direction) of the Y component of the experimental reference line was calculated for the PPP and PRP proteomes of interest. The measured amino acid molar concentrations in μM of the amino acid type Y are plotted against the measured total protein molar concentrations in μM for each proteome of interest and a linear regression was performed. The linear regression was constrained to pass through the origin.

**Figure 33** shows how the coefficient (direction) of the K component of the experimental reference line was calculated for the PPP_50 and PRP_50 subproteomes of interest. The measured amino acid molar concentrations in μM of the amino acid type K are plotted against the measured total protein molar concentrations in μM for each subproteome of interest and a linear regression was performed. The linear regression was constrained to pass through the origin.

**Figure 34** shows how the coefficient (direction) of the C component of the experimental reference line was calculated for the PPP_50 and PRP_50 subproteomes of interest. The measured amino acid molar concentrations in μM of the amino acid type C are plotted against the measured total protein molar concentrations in μM for each subproteome of interest and a linear regression was performed. The linear regression was constrained to pass through the origin.

**Figure 35** shows how the coefficient (direction) of the W component of the experimental reference line was calculated for the PPP_50 and PRP_50 subproteomes of interest. The measured amino acid molar concentrations in μM of the amino acid type W are plotted against the measured total protein molar concentrations in μM for each subproteome of interest and a linear regression was performed. The linear regression was constrained to pass through the origin.

**Figure 36** shows the mean measured amino acid concentrations across the three technical replicates of each patient sample (stars) and the theoretical reference line (solid line). The close agreement between the experimentally measured dataset and predicted reference line illustrate the robustness of the approach disclosed herein whereby any proteome or subproteome of interest can be described algebraically by a single reference which is a vector function of a common parameter of total protein concentration.

**Figure 37** shows the amino acid concentrations in μM of the amino acid type K versus amino acid concentrations in μM of the amino acid type C, for both PPP and PRP proteomes of interest. This dataset was partitioned into a training set and a testing set, the training set was used to train a classifier to identify the proteome of interest of a patient sample based on its measured concentratinos of the K and C amino acid types.

**Figure 38** shows the predictions of the trained classifier explained in Figure 37. There are no incorrect predictions shown because 100% of its predictinos were correct.

**Figure 39** shows a 100% percentage of accuracy (true versus predicted class using a Fine K-Nearest Neighbor, KNN classifier of PPP vs PRP proteome identification using only the amino acid concentrations calculated from the measured values of the label of two labeled amino acid types: K and C.

**Figure 40** shows that the high classification sensitivities and specificites are robust to the type of classifier used. For example, an 100% percentage of accuracy (true versus predicted class using a Bagged Decision Tree classifier) of PPP vs PRP proteome identification using just the two amino acid types K and C is shown. Additionally no optimization or hyperparameter tuning was required to achieve this level (100% accuracy) of classifier performance based on the amino acid concentrations calculated from the measured values of the label of two labeled amino acid types: K and C.

**Figure 41** shows a 100% Positive Predictive Value (true versus predicted class using a Bagged Decision Tree classifier) of PPP vs PRP proteome identification using just the two amino acid types K and C.

**Figure 42** shows the K coefficient of the experimental reference line for the PPP and PRP proteomes of interest for every individual male and female patient plotted as a function of patient age. There was there was no impact of patient gender or age on the coefficient of the experimental proteomic reference lines calculated for each patient. This confirms that the proteomic signatures measured using the methods of the present invention describe any patient population and are specifically not affected by gender or age. This result confirms that the methods of the invention are robust to individual patient variations and that healthy patients exhibt a single identifying proteomic signature.

**Figure 43** shows the C coefficient of the experimental reference line for the PPP and PRP proteomes of interest for every individual male and female patient plotted as a function of patient age. There was there was no impact of patient gender or age on the coefficient of the experimental proteomic reference lines calculated for each patient. This confirms that the proteomic signatures measured using the methods of the present invention describe any patient population and are specifically not affected by gender or age. This result confirms that the methods of the invention are robust to individual patient variations and that healthy patients exhibt a single identifying proteomic signature.

**Figure 44** shows the W coefficient of the experimental reference line for the PPP and PRP proteomes of interest for every individual male and female patient plotted as a function of patient age. There was there was no impact of patient gender or age on the coefficient of the experimental proteomic reference lines calculated for each patient. This

confirms that the proteomic signatures measured using the methods of the present invention describe any patient population and are specifically not affected by gender or age. This result confirms that the methods of the invention are robust to individual patient variations and that healthy patients exhibit a single identifying proteomic signature.

**Figure 45** shows the Y coefficient of the experimental reference line for the PPP and PRP proteomes of interest for every individual male and female patient plotted as a function of patient age. There was there was no impact of patient gender or age on the coefficient of the experimental proteomic reference lines calculated for each patient. This confirms that the proteomic signatures measured using the methods of the present invention describe any patient population and are specifically not affected by gender or age. This result confirms that the methods of the invention are robust to individual patient variations and that healthy patients exhibit a single identifying proteomic signature.

**Figure 46** shows the K coefficient of the experimental reference line for the PPP_50 and PRP_50 subproteomes of interest for every individual male and female patient plotted as a function of patient age. There was there was no impact of patient gender or age on the coefficient of the experimental subproteomic reference lines calculated for each patient. This confirms that the subproteomic signatures measured using the methods of the present invention describe any patient population and are specifically not affected by gender or age. This result confirms that the methods of the invention are robust to individual patient variations and that healthy patients exhibit a single identifying subproteomic signature.

**Figure 47** shows the C coefficient of the experimental reference line for the PPP_50 and PRP_50 subproteomes of interest for every individual male and female patient plotted as a function of patient age. There was there was no impact of patient gender or age on the coefficient of the experimental subproteomic reference lines calculated for each patient. This confirms that the subproteomic signatures measured using the methods of the present invention describe any patient population and are specifically not affected by gender or age. This result confirms that the methods of the invention are robust to individual patient variations and that healthy patients exhibit a single identifying subproteomic signature.

**Figure 48** shows the W coefficient of the experimental reference line for the PPP_50 and PRP_50 subproteomes of interest for every individual male and female patient plotted as a function of patient age. There was there was no impact of patient gender or age on the coefficient of the experimental subproteomic reference lines calculated for each patient. This confirms that the subproteomic signatures measured using the methods of the present invention describe any patient population and are specifically not affected by gender or age. This result confirms that the methods of the invention are robust to individual patient variations and that healthy patients exhibit a single identifying subproteomic signature.

**Figure 49** shows $w_k$, $w_C$, $w_W$, and $w_Y$ values calculated from healthy patient mass spectrometry data compared to $w_k$, $w_C$, $w_W$, and $w_Y$ values calculated from healthy patient Human Peptide Atlas immunoassay data with both sets of $w_k$, $w_C$, $w_W$, and $w_Y$ values calculated using the methods of the present invention. The agreement of these values illustrates that equation 11 robustly performs on abundance data generated from both mass spectrometry and immunoassay, providing a means to build a congruent/unified set of references (vector functions), even though different experimental techniques were employed to generate the underlying data. The provides a framework to build upon existing sources of data.

**Figure 50** shows the amino acid concentrations in μM for the amino acid types C, W, Y and K in a plotted in an N-dimensional space for ovarian cancer plasma samples, pancreatic cancer plasma samples, colorectal cancer plasma samples and healthy patient plasma (PPP) samples, and that each of these data sets is observed to take on the form of a reference line (which is the function of the common parameter of total protein concentration) as taught herein.

**Figure 51** shows amino acid concentration in μM of amino acid type K plotted as a function of total molar protein concentration in μM for the ovarian cancer plasma proteome and calculation of the K coefficient (direction) of the ovarian cancer reference line.

**Figure 52** shows amino acid concentration in μM of amino acid type C plotted as a function of total molar protein concentration in μM for the ovarian cancer plasma proteome and calculation of the C coefficient (direction) of the ovarian cancer reference line.

**Figure 53** shows amino acid concentration in μM of amino acid type W plotted as a function of total molar protein concentration in μM for the ovarian cancer plasma proteome and calculation of the W coefficient (direction) of the ovarian cancer reference line.

**Figure 54** shows amino acid concentration in μM of amino acid type Y plotted as a function of total molar protein concentration in μM for the ovarian cancer plasma proteome and calculation of the Y coefficient (direction) of the ovarian cancer reference line.

**Figure 55** shows amino acid concentration in μM of amino acid type K plotted as a function of total molar protein concentration in μM for the pancreatic cancer plasma proteome and calculation of the K coefficient (direction) of the pancreatic cancer reference line.

**Figure 56** shows amino acid concentration in μM of amino acid type C plotted as a function of total molar protein concentration in μM for the pancreatic cancer plasma proteome and calculation of the C coefficient (direction) of the

pancreatic cancer reference line.

**Figure 57** shows amino acid concentration in μM of amino acid type W plotted as a function of total molar protein concentration in μM for the pancreatic cancer plasma proteome and calculation of the W coefficient (direction) of the pancreatic cancer reference line.

**Figure 58** shows amino acid concentration in μM of amino acid type Y plotted as a function of total molar protein concentration in μM for the pancreatic cancer plasma proteome and calculation of the Y coefficient (direction) of the pancreatic cancer reference line.

**Figure 59** shows amino acid concentration in μM of amino acid type K plotted as a function of total molar protein concentration in μM for the colorectal cancer plasma proteome and calculation of the K coefficient (direction) of the colorectal cancer reference line.

**Figure 60** shows amino acid concentration in μM of amino acid type C plotted as a function of total molar protein concentration in μM for the colorectal cancer plasma proteome and calculation of the C coefficient (direction) of the colorectal cancer reference line.

**Figure 61** shows amino acid concentration in μM of amino acid type W plotted as a function of total molar protein concentration in μM for the colorectal cancer plasma proteome and calculation of the W coefficient (direction) of the colorectal cancer reference line.

**Figure 62** shows amino acid concentration in μM of amino acid type Y plotted as a function of total molar protein concentration in μM for the colorectal cancer plasma proteome and calculation of the Y coefficient (direction) of the colorectal cancer reference line.

**Figure 63** shows that when the vector function approach described herein as one possible way of determining the presence and/or concentration and/or amount of a proteome or subproteome of interest in a patient sample is carried out, very high sensitivities and specificites are obtained for determination of the presence and absence of colorectal cancer, ovarian cancer, and pancreatic cancer in patient blood plasma. Specifically, as is outlined in the provided confusion matrix, 100% accuracy is achieved for the identification of colorectal cancer and pancreatic cancer from blood plasma, 90% accuracy for the identification of ovarian cancer from blood plasma, and 95% specificity for the correct identification of cancer negative, healthy samples as cancer-negative, healthy samples.

**Figure 64** shows that the concentration and/or amount of a proteome of interest which are determined as part of the (quantitative) vector function approach described herein are highly accurate, following the line y = x for all proteomes of interest, and allowing for the determination of the concentration and/or amount of a proteome of interest within a patient sample with very low error (only 2% error).

**Figure 65** shows that the proteomes of interest can also be identified in blood plasma using a machine learning classifier. A linear support vector machine (SVM) classifier was trained on the molar (μM) amino acid concentrations of the K, C, W, and Y amino acid types of patient plasma samples with 25% of the data held out. A 100% positive predictive value and 0% false discovery rate was obtained for each cancer proteome of interest (all cancer patient samples) as well as the healthy proteome of interest (all healthy patient samples).

**Figure 66** shows that the proteomes of interest can also be identified in blood plasma using a machine learning classifier trained on amino acid concentrations of only three labeled amino acid types. A linear support vector machine (SVM) classifier was trained on the molar (μM) amino acid concentrations of the K, C, and W amino acid types of patient plasma samples with 25% of the data held out. A 100% positive predictive value and 0% false discovery rate was obtained for each cancer proteome of interest (all cancer patient samples) as well as the healthy proteome of interest (all healthy patient samples).

**Figure 67** shows that the proteomes of interest can also be identified in blood plasma using a machine learning classifier trained on amino acid concentrations of only two labeled amino acid types. A linear support vector machine (SVM) classifier was trained on the molar (μM) amino acid concentrations of only the K and C amino acid types of patient plasma samples with 25% of the data held out. A 100% positive predictive value and 0% false discovery rate was obtained for each cancer proteome of interest (all cancer patient samples) as well as the healthy proteome of interest (all healthy patient samples).

**Figure 68** shows a confusion matrix indicating 78% accuracy for detecting stage III colorectal cancer using the methods of the invention based on the amount of the K, C, W, and Y amino acid types.

**Figure 69** shows a confusion matrix indicating 100% positive predictive value for detecting the location of colorectal cancer using the methods of the invention based on the amount of the K, C, W, and Y amino acid types.

**Figure 70** shows the molar concentration of amino acids in μM of the K, C, W, and Y amino acid types within bladder cancer samples, prostate cancer samples and renal cancer samples measured in urine.

**Figure 71** shows a positive predictive value false discovery confusion matrix, indicating 100% positive predictive identification and 0% false discovery for the identification of bladder cancer, prostate cancer, and renal cancer from urine samples using the methods of the invention. All included types of cancer (bladder cancer, prostate cancer, and renal cancer) can be correctly identified from urine samples with a true positive rate of 100% and a false negative rate of 0%.

DETAILED DESCRIPTION OF THE INVENTION

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

The invention is based on the discovery that it is only necessary to measure the label and/or amino acid concentration or number of amino acids of two or more amino acid types in a sample in order to identify the presence and/or concentration and/or amount of one or more proteins, peptides, oligopeptides, polypeptides, protein complexes, subproteomes, or proteomes of interest within a sample. It is only necessary to label and measure two or more amino acid types within a sample in order to identify and quantify proteins, peptides, oligopeptides, polypeptides, protein complexes, subproteomes or proteomes, without the need to sequence the sample. This is because each protein, peptide, oligopeptide, polypeptide, protein complex, subproteome, or proteome has a unique signature based on the measured label, amino acid concentration and/or number of amino acids of two or more amino acid types in the protein, peptide, oligopeptide, polypeptide, protein complex, subproteome, or proteome. The measured label and amino acid concentration signature of two or more amino acid types in a protein, peptide, oligopeptide, polypeptide, protein complex, subproteome, or proteome is unique based on the concentration of that protein, peptide, oligopeptide, polypeptide, protein complex, subproteome, or proteome. It is not necessary to identify the order of the amino acids in the sample, for example, by sequencing the sample for the identification of one or more proteins, peptides, oligopeptides, polypeptides, protein complexes, subproteomes, or proteomes of interest in a sample. Identifying the order of amino acids within a protein sequence in a sample adds no additional information when two or more amino acid types are labelled and measured in a sample (Figure 7).

The methods of the invention described herein can be used to identify the presence and/or concentration and/or amount of one or more proteins, peptides, oligopeptides, polypeptides, protein complexes, subproteomes, or proteomes of interest in a sample. This is because each protein, peptide, oligopeptide, polypeptide, protein complex, subproteome, or proteome of interest has a unique signature based on the known label values, amino acid concentrations or number of amino acids of two or more amino acid types in each protein, peptide, oligopeptide, polypeptide, protein complex, subproteome, or proteome of interest. Therefore, the signature of the sample can be compared to the signature of a protein, peptide, oligopeptide, polypeptide, protein complex, subproteome, or proteome of interest in order to identify the presence and/or concentration and/or amount of a protein, peptide, oligopeptide, polypeptide, protein complex, subproteome, or proteome of interest in a sample. For example, the SARS-CoV-2 proteome has a unique signature based on the known label values and/or amino acid concentrations and/or number of amino acids of each amino acid type and the concentration of the SARS-CoV-2 proteome compared to the known label values and/or amino acid concentrations and/or number of amino acids of each amino acid type and the concentration of the Influenza A proteome. Therefore, the measured label, amino acid concentration and/or number of amino acids of two or more amino acid types in the sample can be determined, and compared to the known label values and/or amino acid concentrations or number of amino acids of the same two or more amino acid types in the SARS-CoV-2 proteome and/or the HIV proteome to identify the presence and/or concentration and/or amount of the SARS-CoV-2 proteome and/or the HIV proteome in the sample.

Previously, strategies for identifying a whole proteome or subproteome of interest within a sample at one time were not available. Strategies for the identification of a pathogen, such as SARS-CoV-2, have relied on the reverse transcription polymerase chain reaction (RT-PCR) for the qualitative detection of nucleic acid from SARS-CoV-2. Current state-of-the art tests have an approximately 30% false negative rate, with significant consequences for infection control. Quantitative information about viral load is not routinely available. As a general alternative, the methods of the present invention are used to identify a whole proteome or subproteome of interest within the sample at one time, for example, for the identification of the presence and/or concentration and/or amount of the SARS-CoV-2 proteome of interest within patient samples.

The methods of the invention described herein can be used to identify the presence and/or concentration and/or amount of a subproteome or proteome of interest in a sample because each subproteome or proteome of interest has a unique signature based on the known values of the label, amino acid concentrations and/or number of amino acids of two or more amino acid types in each protein, peptide, oligopeptide, polypeptide, and protein complex across the subproteome or proteome of interest. Therefore, the signature of the sample can be compared to the signature of one or more subproteomes or proteomes of interest to identify the presence and/or concentration and/or amount of a subproteome or proteome of interest in a sample. For example, the human plasma proteome has a unique signature based on the mean known label values, amino acid concentrations and/or number of amino acids of each amino acid type compared to the mean known label values, amino acid concentrations and/or number of amino acids of each amino acid type in the human eye proteome. Therefore, the measured label, amino acid concentration and/or number of amino acids of two or more amino acid types in the sample can be determined, and compared to the mean known label values, amino acid concentrations and/or number of amino acids of the same two or more amino acid types in a proteome of interest to

identify the presence and/or concentration and/or amount of that proteome in the sample.

The methods of the invention can be used to identify the presence of a viral proteome in a sample. Each viral proteome has a unique signature based on the mean known label values, amino acid concentrations and/or number of amino acids of two or more amino acid types. Therefore, the mean measured label, amino acid concentration and/or number of amino acids of two or more amino acid types in the sample can be compared to the mean known label values, amino acid concentrations and/or number of amino acids of the same two or more amino acid types of a viral proteome to identify the presence and/or concentration and/or amount of the viral proteome in the sample. In addition, the methods of the invention can be used to identify the viral load of the viral proteome within the sample. For example, each viral proteome has a unique signature based on the mean number of amino acids of two or more amino acid types in each protein across the viral proteome multiplied by the total protein concentration of the viral proteome. Therefore, the amino acid concentration of two or more amino acid types in the sample can be compared to the mean amino acid concentration of the same two or more amino acid types of a viral proteome at one or more protein concentrations to identify the concentration of the viral proteome within the sample.

Previously, when using solution phase strategies to identify the presence and/or concentration and/or amount of a mixture of proteins, peptides, polypeptides, oligopeptides, subproteomes or proteomes in a sample, it has been necessary to first separate the mixture into its individual protein components. For example, the proteins within a mixture are separated based on size by gel electrophoresis, or based on adsorption of compounds to the adsorbent using a chromatography column, before individual proteins are sequenced. For example, if a mixture contains two proteins; bovine serum albumin and lysozyme, it was previously necessary to separate the mixture into the individual protein components of bovine serum albumin and lysozyme. In contrast, it has been discovered that the methods of the invention described herein can be used to identify the presence and/or concentration and/or amount of a mixture of proteins, peptides, polypeptides, oligopeptides, subproteomes or proteomes in a sample, based on the average signature from the whole mixture, without the need to separate the mixture into the individual components. For example, a mixture that contains bovine serum albumin and lysozyme can be identified without the need to separate the mixture into its individual protein components of bovine serum albumin and lysozyme. This is because it has been discovered that a mixture of proteins, peptides, polypeptides, oligopeptides, subproteomes or proteomes has a unique signature based on the mean number of amino acids of two or more amino acid types across the proteins, peptides, polypeptides, oligopeptides, subproteomes or proteomes in the mixture. For example, a mixture that contains bovine serum albumin and lysozyme has a unique signature based on the mean measured label, amino acid concentration and/or number of amino acids of two or more amino acid types in bovine serum albumin and lysozyme compared to another mixture that contains bovine serum albumin and alpha synuclein, which has a different unique signature based on the mean measured label, amino acid concentration and/or number of amino acids of the same two or more amino acid types in a bovine serum albumin and alpha synuclein mixture. It is not necessary to know the proportion of the components within a mixture in order to identify the presence and/or concentration and/or amount of a mixture within the sample. Instead, the presence of a mixture is identified in the sample when more than one protein, peptide, oligopeptide, polypeptide, protein complex, subproteome, or proteome of interest is identified in the sample. The signature of the sample is influenced by the signature of each of the components within the mixture. If protein of interest A is identified within the mixture and comprises a higher proportion of the mixture than protein of interest B which has also been identified in the mixture, then the distance between the sample point and the reference line or point for protein of interest A is smaller than the distance between the sample point and the reference line or point for protein of interest B. It was discovered that, conversely, the distances between the sample point and protein of interest A and B can be calculated and compared to determine the proportion of protein of interest A and B in the mixture. Therefore, the signature of the sample can be compared to the signatures of more than one more than one protein, peptide, oligopeptide, polypeptide, protein complex, subproteome, or proteome of interest identified as being present in the sample in order to identify the presence and/or concentration and/or amount of such a mixture in the sample.

The methods of the invention can also be used to identify co-infection of two or more proteomes in a sample, i.e. a mixture of proteomes in a sample. This is because a mixture of proteomes has a unique signature based on the mean known label values, amino acid concentrations and/or number of amino acids of two or more amino acid types in each protein across the mixture of proteomes. Therefore, the measured label value, amino acid concentration and/or number of amino acids of two or more amino acid types in the sample can be determined and compared to the mean known label values, amino acid concentrations and/or number of amino acids of the same two or more amino acid types in more than one proteome of interest. If the presence of more than one proteome of interest is identified within the sample, then a mixture of proteomes of interest is identified within the sample, and the proportion of each proteome within the mixture can be determined as explained above for a mixture of proteins. If proteome of interest A is identified within the mixture and comprises a higher proportion of the mixture than proteome of interest B which has also been identified in the mixture, then the distance between the sample point and the reference line or point for proteome of interest A is smaller than the distance between the sample point and the reference line or point for proteome of interest B. Conversely the distances between the sample point and proteome of interest A and B can be calculated and compared to determine the proportion of proteome of interest A and B in the mixture. Therefore, the signature of the sample can be compared to the signatures of more than one

more than one proteome of interest identified as being present in the sample in order to identify the presence and/or concentration and/or amount of such a mixture in the sample. For example, a patient may have a viral and a secondary bacterial infection, or two viral infections. In this case, the bacteria and virus proteomes and the two viral proteomes do not need to be separated from one another before the method of the invention is carried out. This can equally apply to any combination of proteomes, such as bacteria, fungi, protozoa, plant, animal including human, and any combination thereof. The methods of the invention described herein are simple, effortless, and highly efficient and avoid the inherent disadvantages of methods that require known sequencing techniques and/or separation techniques.

The methods disclosed herein can be applied to any protein, peptide, oligopeptide, polypeptide, protein complex, subproteome, proteome, or mixture of proteins, peptides, polypeptides, oligopeptides, subproteomes and/or proteomes. The methods of the invention simply require the labelling of amino acids of two or more amino acid types and the measuring of these labels. An amino acid type is defined by the R-group specific to each amino acid. The R-group of each type of amino acid is unique. An amino acid type can include modified and/or unmodified amino acids of the 22 proteinogenic amino acids and/or non-proteinogenic or synthetic amino acids.

The only requirement for the method is that the signature of the two or more amino acid types of the one or more proteins, peptides, oligopeptides, polypeptides, protein complexes, subproteomes, or proteomes of interest (e.g reference), or the amino acid sequence and/or any experimental information about post-translational modifications is available. It is not necessary to determine the sequence of amino acids within the sample in order to identify the presence and/or concentration and/or amount of a protein in the sample. In some embodiments, the signature of two or more amino acid types of the one or more proteins, peptides, oligopeptides, polypeptides, protein complexes, subproteomes, or proteomes of interest is known (e.g. from a database). In some embodiments, the signature of two or more amino acid types of the one or more proteins, peptides, oligopeptides, polypeptides, protein complexes, subproteomes, or proteomes of interest is determined from the amino acid sequence or sequences of each of the one or more proteins, peptides, oligopeptides, polypeptides, protein complexes, subproteomes, or proteomes, of interest as part of the method of the invention. In some embodiments, if the signature of two or more amino acid types of the one or more proteins, peptides, oligopeptides, polypeptides, protein complexes, subproteomes, or proteomes, of interest is not known, the amino acid sequence of the one or more proteins, peptides, oligopeptides, polypeptides, protein complexes, subproteomes, or proteomes of interest can be used to determine the signature. Alternatively, the signature of two or more amino acid types of the one or more proteins, peptides, oligopeptides, polypeptides, protein complexes, subproteomes, or proteomes of interest is determined using the methods disclosed herein (e.g. labelling two or more amino acid types, measuring the value of the label, measuring the total protein concentration of the sample via standard methods, and converting the measured label to the number of amino acids of each labelled amino acid type).

If the one or more of the proteins, peptides, oligopeptides, polypeptides, protein complexes, subproteomes, or proteomes of interest has one or more amino acid types that include modified amino acids of the amino acid type, then the signature of the modified amino acids of that amino acid type in the one or more proteins, peptides, oligopeptides, polypeptides, protein complexes, subproteomes, or proteomes of interest can be determined. In some embodiments, this is determined from experimental post-translational modification information for the one or more proteins, peptides, oligopeptides, polypeptides, protein complexes, subproteomes, or proteomes of interest. For example, if the amino acid type C has been labelled in the sample and includes the modified cysteine amino acids cysteine disulphide ($C_D$), then the signature of the number of amino acids of the amino acid type cysteine can include the post-translational modification information for the modified amino acids oxidized cysteine Co. This signature of the sample can be compared to the signature of the known label values, amino acid concentrations, or number of amino acids of modified amino acids (such as oxidized cysteine $C_D$) in the one or more proteins, peptides, oligopeptides, polypeptides, protein complexes, subproteomes, or proteomes of interest.

## Definitions

Unless defined otherwise, all technical and scientific terms used herein have the same meaning as is commonly understood by one of ordinary skill in the art. All patents, applications, published applications and other publications referenced herein, are incorporated by reference in their entirety unless stated otherwise. In the event that there is a plurality of definitions for a term herein, those in this section prevail unless stated otherwise.

As used herein, the term "presence" refers to the positive identification of one or more proteins, peptides, oligopeptides, polypeptides, protein complexes, subproteomes, or proteomes of interest in a sample.

As used herein, the term "concentration" refers to the abundance of an entity per unit of volume. An entity can be a molecule, a complex, a monomer within a polymer such as an amino acid contained within a protein chain, or an atom. Mass concentration refers to the mass of an entity per unit of volume. Number concentration refers to the number of molecules of an entity per unit of volume. Molar concentration refers to the number of moles of an entity per unit of volume. The number of moles of entities is the total number of entities contained within the sample divided by the Avogadro constant NA, which is 6.02214076×1023 mol-1. Unless otherwise stated, the term "concentration" refers to the molar

concentration of an entity. Reference is frequently made to the "protein, peptide, oligopeptide, polypeptide, or protein complex of interest as a function of concentration, *t*" or equivalent. This means that *t* is the concentration of the protein of interest, or, *t* is the concentration of the peptide of interest, or, *t* is the concentration of the oligopeptide of interest, or, *t* is the concentration of the polypeptide of interest, or, *t* is the concentration of the protein complex of interest. As used herein, in some embodiments, the concentration of a protein complex of interest refers to the concentration of the complex, not the monomer concentration of subunits within the complex. For example, if protein complex of interest $\alpha$ has two subunits, A and B, such that protein complex of interest $\alpha$ can be described with the complex stoichiometry A:B, then the concentration of protein complex $\alpha$ is the concentration of the complex A:B, not the concentration of subunit A plus the concentration of subunit B. Reference is also frequently made to the "subproteome or proteome of interest as a function of concentration, *t*" or equivalent. The concentration of a subproteome of interest is the total concentration of all proteins, peptides, oligopeptides, polypeptides, and protein complexes which comprise the subproteome of interest. This means that t is the total concentration of all proteins, peptides, oligopeptides, polypeptides, and protein complexes which comprise the subproteome of interest. The concentration of a proteome of interest is the total concentration of all proteins, peptides, oligopeptides, polypeptides, and protein complexes which comprise the proteome of interest. This means that t is the total concentration of all proteins, peptides, oligopeptides, polypeptides, and protein complexes which comprise the proteome of interest. Once the molar concentration of a protein, peptide, oligopeptide, polypeptide, protein complex, subproteome, or proteome of interest present in the sample has been identified, the mass concentration of the protein, peptide, oligopeptide, polypeptide, protein complex, subproteome, or proteome of interest is the molar concentration of the protein, peptide, oligopeptide, polypeptide, protein complex, subproteome, or proteome of interest multiplied by the molecular weight of the (now identified, such that it's amino acid sequence or sequences are available) protein, peptide, oligopeptide, polypeptide, protein complex, subproteome, or proteome of interest. In some embodiments, the molecular weight of a protein complex is the combined molecular weight of its subunits. The molecular weight of a subproteome or proteome of interest is the mean of the molecular weights of the proteins, peptides, oligopeptides, polypeptides, and/or protein complexes which comprise the proteome or subproteome of interest. In some embodiments, the concentration of a proteome is a measure of the viral load, bacterial load and/or parasitic load of a proteome, or mixture of proteomes in the sample. In some embodiments, the proteome is a viral proteome, and the method provides the total molar protein concentration of the viral proteome within the sample. This is equivalent to the traditional viral load measurement in copies/mL. Alternatively, the method provides the viral load measurement in copies/mL using standard techniques known in the art. In some embodiments, the proteome is a bacterial proteome, and the method provides the total bacterial concentration of the bacterial proteome within the sample. This is equivalent to the bacterial load measurement in colony forming units (CFU). Alternatively, the method provides the bacterial load measurement in CFU using standard techniques known in the art. In some embodiments, the proteome is a parasitic proteome and the method provides the total parasitic concentration of the parasitic proteome within the sample. This is equivalent to the parasitic load measurement in number of parasites per host sample. Alternatively, the method provides the parasitic load measurement in number of parasites per host sample using standard techniques known in the art. Although we have described certain embodiments, in relation to molar concentration, these embodiments are equally applicable to mass concentration, as has been described in the examples.

As used herein, the term "concentration of the protein, peptide, oligopeptide, polypeptide, protein complex, subproteome, or proteome of interest" is an abbreviation to refer to the protein concentration of the protein of interest, or, peptide concentration of the peptide of interest, or, oligopeptide concentration of the oligopeptide of interest, or, polypeptide concentration of the polypeptide of interest, or, protein complex concentration of the protein complex of interest, or, subproteome concentration of the subproteome of interest, or, proteome concentration of the proteome of interest.

As used herein, the term "amount" refers to the number of moles of entities within a sample. An entity can be a molecule, a complex, a monomer within a polymer such as an amino acid contained within a protein chain, or an atom. The number of moles of entities is the total number of entities contained within the sample divided by the Avogadro constant $N_A$, which is $6.02214076 \times 10^{23}$ $mol^{-1}$. Unless otherwise stated, the amount refers to the number of moles of molecules within a sample. The amount refers to the number of moles of molecules of the one or more proteins, peptides, oligopeptides, polypeptides, protein complexes, subproteomes or proteomes of interest in the sample. In some embodiments, the amount of a protein complex containing multiple protein subunits considers the entire protein complex as one molecule. A proteome or subproteome of interest has many different types of molecules. The amount of a subproteome or proteome of interest refers to the total number of moles of proteins, peptides, oligopeptides, polypeptides, and protein complexes that comprise the proteome or subproteome of interest within the sample. In some embodiments, the molar concentration of the sample is multiplied by the volume of the sample to provide the amount of the sample.

As used herein, the term "relative concentration" refers to fold changes in the concentration of molecules between samples. For example, a first sample that has been diluted from a second sample has a lower relative concentration than the second sample.

As used herein, the term "amino acid concentration" refers to the molar or mass concentration of amino acids

within an amino acid type. Amino acid concentration refers to the amount or mass of amino acids within an amino acid type per unit of volume. Unless otherwise stated, the term amino acid concentration refers to the molar concentration of amino acids within an amino acid type. The molar concentration of amino acids within an amino type may be different than the concentration of molecules, because more than one amino acid of an amino acid type, or zero amino acids of an amino acid type, can be contained within a molecule. The amino acid concentration of amino acids within an amino acid type is equal to the total molar concentration of molecules multiplied by the number of amino acids of the amino acid type per molecule. For example, if the molecules are proteins, then the amino acid concentration of an amino acid type can be (and is usually) different than the protein concentration. The amino acid concentration of an amino acid type within a sample is calculated from the measured value of the label of that amino acid type within the sample, using a calibration curve or standard providing the value of the label for one or more proteins or amino acids of known amino acid concentration. Importantly, the amino acid concentration of two or more amino acid types of a sample does not refer to the concentration of the sample. The amino acid concentrations of two or more amino acid types of a protein, peptide, oligopeptide, polypeptide, protein complex, subproteome, or proteome of interest does not refer to the concentration of the protein, peptide, oligopeptide, polypeptide, protein complex, subproteome, or proteome of interest.

As used herein, the term "protein" refers to a biomolecule or macromolecule comprised of one or more linear polypeptide chains of amino acids. A protein is a polymer of amino acids. The term "protein" includes, but is not limited to, molecules which contain from about 50 to about 3000 amino acids. The term "protein" refers to one or more polypeptide chains arranged in a way which is often biologically functional. A protein can have a 3-dimensional structure which is folded, 3-dimensional structure which is intrinsically disordered or a 3-dimensional structure which is partially folded and partially disordered. A protein also refers to a biomolecule or macromolecule comprised of one or more linear polypeptide chains of amino acids that also includes other components. For example, a protein also includes glycoproteins (in which chains of sugar molecules are covalently attached to protein molecules), or a nucleoprotein in which a protein is associated with or bonded to a nucleic acid.

As used herein, the term "peptide" refers to short chains of amino acids linked by peptide (amide) bonds. The term "peptide" includes, but is not limited to, molecules that contain from about 2 to about 50 amino acids. In a preferred aspect, the term "peptide" refers to molecules that contain greater than 10 amino acids.

As used herein, the term "oligopeptide" refers to a class within peptides that includes, but is not limited to, molecules that contain from about 2 to about 20 amino acids. The term "oligopeptide" includes, but is not limited to, dipeptides that contain 2 amino acids, tripeptides that contain 3 amino acids, tetrapeptides that contain 4 amino acids, and pentapeptides that contain 5 amino acids.

As used herein the term "polypeptide" is a single linear chain of many amino acids, held together by peptide bonds.

As used herein, the term "protein complex" refers to a structurally associated group of two or more subunits containing at least one protein subunit. A protein complex often contains two or more proteins. It can also contain one or more proteins and one or more nucleic acids (ribonucleoproteins). Protein complexes are a form of stable protein-protein interactions in which the protein subunits usually cooperate to perform a biological function. An example of a protein complex is a ribosome. Because the protein subunits within protein complexes are stably structurally associated with one another and cooperate to form a biological function, the number of amino acids of each of two or more amino acid types within each subunit of the protein complex are summed to determine the number of amino acids of each of two or more amino acid types for the protein complex.

As used herein, the term "protein-protein interaction" refers to an interaction between protein molecules, usually involving specific physical contacts. Protein-protein interactions can be stable or transient. In the methods of the invention, protein-protein interactions which do not comprise protein complexes, such as transient protein interactions, are treated as protein mixtures.

As used herein, the term "subproteome" is a collection of proteins that are part of a proteome and share a common characteristic, such as being disease-associated. For example, a subproteome within the human plasma proteome is the heart disease subproteome. A disease-associated subproteome can include all or some of the proteins within a proteome. A subproteome can also describe proteins within a proteome that share a common physical characteristic, such as, but not limited to being low molecular weight, size, charge and/or density. In some embodiments, low molecular weight characteristics refers to proteins of less than 10kDa, less than 30 kDa, less than 50kDa, less than 100kDa, 10-30 kDa, 30-50 kDa, 10-50kDA , 30-50kDA, 10-100kDa, 50-100kDa or 30-100 kDa. In preferred embodiments, low molecular weight refers to proteins of less than less than 10kDa, less than 30 kDa, less than 50kDa, less than 100kDa, or proteins of 10 kDa, 30 kDa, 50 kDa or 100 kDa. In preferred embodiments, low molecular weight refers to proteins of less than 50kDa or proteins of 50kDa. In some embodiments, charge characteristics refers to chromatography including ion-exchange chromatography that can be used to select proteins that bind to oppositely charged resins. In some embodiments, density characterisitcs refers to sedimentation coefficient which is related to protein size and shape.

As used herein, the term "proteome" refers to all of the proteins expressed by an organism. The term "proteome" also refers to all the proteins expressed by an organism within a particular tissue type, for example, the human plasma

proteome. The term "proteome" also refers to all the proteins expressed within a particular cell type, for example, glioblastoma cells. The term "proteome" also refers to changes in the proteins expressed by an organism, tissue type, or cell type at a given time or under a given set of conditions, for example when treated with a drug. The term "proteome" includes, but is not limited to, viral proteomes, bacterial proteomes, archaea proteomes, parasitic proteomes, yeast proteomes, plant proteomes, animal proteomes, mammalian proteomes, and the human proteome. The term "proteome" includes, but is not limited to, viral proteomes with less than 50 proteins, bacterial proteomes with less than 7000 proteins, the human plasma proteome with less than 5000 proteins, the human urine proteome with less than 5000 proteins, the human salivary proteome with less than 5000 proteins, and the human proteome with approximately 22000 proteins.

As used herein, the term "mixture" refers to two or more proteins, peptides, polypeptides or oligopeptides, subproteomes and/or proteomes in a sample. For example, a mixture of peptides is a combination of two or more peptides, a mixture of polypeptides is a combination of two or more polypeptides, and a mixture of proteins is a combination of two or more proteins. The mixture does not have to be comprised of the same components. For example, a mixture can also be a mixture of proteins and peptides, a mixture of peptides and polypeptides, a mixture of proteins and polypeptides etc.

As used herein, the "sample" refers to any sample that may contain one or more proteins, peptides, oligopeptides, polypeptides, protein complexes, subproteomes, or proteomes of interest. The term "sample" also includes any sample that does not contain any proteins and thus no value (e.g. signal of the label) is obtained when the label is measured.

As used herein, the term "amino acid type" refers to organic compounds that comprise one amine (-NH) and one carboxyl (-CO) group, one alpha carbon, and one R group (side chain) specific to each amino acid type, or that comprise one amine (-$NH_2$) and one carboxyl (-COOH) group, one alpha carbon, and one R group (side chain) specific to each amino acid type, or that comprise one amine (-$NH_2$) and one carboxyl (-CO) group, one alpha carbon, and one along R group (side chain) specific to each amino acid type, or that comprise one amine (-NH) and one carboxyl (-COOH) group, one alpha carbon, and one R group (side chain) specific to each amino acid type, or, describing the amino acid type proline, amino acid type also refers to organic compounds that comprise one imine (-NH) and one carboxyl (-COOH) group, one alpha carbon, and one R group (side chain) specific to each amino acid type, or that comprise one imine (-NH) and one carboxyl (-CO) group, one alpha carbon, and one R group (side chain) specific to each amino acid type. Amino acid type includes both free amino acids and amino acids within protein sequences. Amino acids within protein sequences can alternatively be called amino acid residues or residues. The amino acid type is defined by the R-group (side chain) specific to each amino acid type. The term amino acid type refers to a proteinogenic amino acid selected from: alanine (A), arginine (R), asparagine (N), aspartic acid (D), cysteine (C), glutamic acid (E), glutamine (Q), glycine (G), histidine (H), isoleucine (I), leucine (L), lysine (K), methionine (M), phenylalanine (F), pyrrolysine (O), proline (P), selenocysteine (U), serine (S), threonine (T), tryptophan (W), tyrosine (Y) and valine (V), or, a non-proteinogenic synthetic amino acid, including, but not limited to non-proteinogenic synthetic amino acids that contains the functional groups azide, alkyne, alkene, diene, acyl, iodo and/or boronic acid. The term "amino acid type" encompasses modified amino acids, unmodified amino acids and/or a combination of both modified and unmodified amino acids of an amino acid type. In some embodiments, the term "amino acid type" refers to modified amino acids of an amino acid type. In some embodiments, the term "amino acid type" refers to unmodified amino acids of an amino acid type. In some embodiments, the term "amino acid type" refers to a combination of modified and unmodified amino acids of an amino acid type. In some embodiments, the term "amino acid type" refers to both the unmodified amino acids of an amino acid type and the combination of the modified and the unmodified amino acids of an amino acid type.

As used herein, the term "R-group" refers to the side chain present in each amino acid of each amino acid type. The R-group is a substituent; an atom, or group of atoms which replaces one or more hydrogen atoms on the alpha carbon of the amino acid. The R-group of each amino acid type is unique for that amino acid type. The R-groups of each amino acid type encompassed by the invention are defined in Table 2. An amino acid type is defined by the R-group present on the unmodified (as translated) amino acid type. If subsequent modifications are made to the R-group, the amino acid type does not change. For example, the cysteine (C) amino acid type is defined by the thiol R-group. This is the R-group of unmodified amino acids of the cysteine amino acid type (reduced cysteine, $C_R$). A subset of cysteine amino acids within the cysteine amino acid type can be post-translationally modified to form cysteine disulphide ($C_D$), and this same subset of cysteine amino acids can be reduced to form reduced cysteine ($C_R$). The amino acid type remains cysteine (C) during these transformations. This is the case regardless of whether a post-translational modification or other modification is reversible or irreversible.

As used herein, a "modified amino acid" refers to amino acids of an amino acid type that have been chemically modified after being incorporated into a protein. In some embodiments, an enzyme carries out this chemical modification. In some embodiments, the modified amino acids have undergone post-translational modification. Examples of such post-translational modification of amino acids include, but are not limited to, methylation, deamination, deamidation, N-linked glycosylation, isomerization, disulfide-bond formation, oxidation to sulfenic, sulfinic or sulfonic acid, palmitoylation, N-acetylation (N-terminus), S-nitrosylation, cyclization to pyroglutamic acid (N-terminus), gamma-carboxylation, isopeptide bond formation, N-Myristoylation (N-terminus), phosphorylation, acetylation, ubiquitination, SUMOylation, methylation,

hydroxylation, oxidation to sulfoxide or sulfone, hydroxylation, O-linked glycosylation, mono- or di-oxidation, formation of Kynurenine, and/or sulfation. For example, the amino acids of the amino acid type cysteine (C) can be modified during post-translational modification to form cysteine disulphide ($C_D$) amino acids that contains disulphide bonds and an oxidized thiol R-group.

As used herein, an "unmodified amino acid" refers to amino acids of an amino acid type that have not been chemically modified after being incorporated into a protein. For example, the unmodified amino acids of the amino acid type cysteine (C) are reduced cysteine ($C_R$); reduced cysteine ($C_R$) is not disulphide bonded and has not undergone any other post-translational modification, and contains a reduced thiol.

As used herein, the term "two or more amino acid types" refers to at least two amino acid types. The term "two or more amino acid types" encompasses, but is not limited to, 2, 3, 4, 5, 6, 7, 8, 9, 10, 11, 12, 13, 14, 15, 16, 17, 18, 19, 20, 21,22, 23, 24, 25, 26, 27, 28, 29, 30, 31, 32, 33, 34, 35, 36, 37, 38, 39 or 40 amino acid types. In some embodiments, 2 amino acid types are labelled. In some embodiments, 3 amino acid types are labelled. In some embodiments, 4 amino acid types are labelled. In some embodiments, 5 amino acid types are labelled. In some embodiments, 6 amino acid types are labelled. In some embodiments, 7 amino acid types are labelled. In some embodiments, 8 amino acid types are labelled. In some embodiments, 9 amino acid types are labelled. In some embodiments, 10 amino acid types are labelled. In some embodiments, 11 amino acid types are labelled. In some embodiments, 12 amino acid types are labelled. In some embodiments, 13 amino acid types are labelled. In some embodiments, 14 amino acid types are labelled. In some embodiments, 15 amino acid types are labelled. In some embodiments, 16 amino acid types are labelled. In some embodiments, 17 amino acid types are labelled. In some embodiments, 18 amino acid types are labelled. In some embodiments, 19 amino acid types are labelled. In some embodiments, 20 amino acid types are labelled. In some embodiments, 21 amino acid types are labelled. In some embodiments, 22 amino acid types are labelled. In some embodiments, 23 amino acid types are labelled. In some embodiments, 24 amino acid types are labelled. In some embodiments, 25 amino acid types are labelled. In some embodiments, 26 amino acid types are labelled. In some embodiments, 27 amino acid types are labelled. In some embodiments, 28 amino acid types are labelled. In some embodiments, 29 amino acid types are labelled. In some embodiments, 30 amino acid types are labelled. In some embodiments, 31 amino acid types are labelled. In some embodiments, 32 amino acid types are labelled. In some embodiments, 33 amino acid types are labelled. In some embodiments, 34 amino acid types are labelled. In some embodiments, 35 amino acid types are labelled. In some embodiments, 36 amino acid types are labelled. In some embodiments, 37 amino acid types are labelled. In some embodiments, 38 amino acid types are labelled. In some embodiments, 39 amino acid types are labelled. In some embodiments, 40 amino acid types are labelled.

As used herein, the term "label" or "labelled" refers to a tag, identifier, or probe that is added, inserted, attached, bound, or bonded to the amino acids within an amino acid type to aid the detection and/or identification of the amino acid type within the sample. For example, a label can include a fluorophore, an isotope, or a tandem mass tag. In some embodiments, the label provides a signal. In some embodiments, the label is a fluorescent label. In some embodiments, the label is a fluorogenic dye, or a molecule which becomes fluorescent upon reaction with an amino acid type. In some embodiments, the label is covalently bonded to the amino acids within an amino acid type. In some embodiments, the label is covalently bonded to the R-group of amino acids within an amino acid type.

As used herein, the term "signal" refers to an occurrence that conveys information. In some embodiments, a signal is a time-varying occurrence that conveys information. The signal of a label can be read at a single point in time, or the signal of a label can be read as a function of time. In some embodiments, the label is a fluorescent label and the signal of the label is fluorescence intensity.

As used herein, the term "luminescence" refers to spontaneous emission of light by a substance not resulting from heat. In some embodiments, label is a luminescent label and the signal of the label is a luminescent signal. There are several types of luminescence, including but not limited to photoluminescence (which includes fluorescence), chemiluminescence (which includes bioluminescence), electroluminescence, radioluminescence, and thermoluminescence. Photoluminescence is the result of absorption of photons. There are several types of photoluminescence, including fluorescence which is photoluminescence as a result of singlet-singlet electronic relaxation with a typical lifetime of nanoseconds. Phosphorescence is another type of photoluminescence which is the result of triplet-singlet electronic relaxation with a typical lifetime of milliseconds to hours. Chemiluminescence is the emission of light as a result of a chemical reaction. Bioluminescence is a form of chemiluminescence which is the result of biochemical reactions in a living organism. Electrochemiluminescence is the result of an electrochemical reaction. Electroluminescence is a result of an electric current passed through a substance. Cathodoluminescence is the result of a luminescent material being struck by electrons. Sonoluminescence is the result of imploding bubbles in a liquid when excited by sound. Radioluminescence is the result of bombardment by ionizing radiation. Thermoluminescence is the re-emission of absorbed energy when a substance is heated. Cryoluminescence is the emission of light when an object is cooled.

As used herein, the term "calibration curve" or the term "standard" refers to a general analytical chemistry method for determining the concentration of a substance in an unknown sample by comparing the unknown sample to a set of standard samples, or one standard sample, of known concentration. If the unknown sample is compared to a set of

standard samples, "calibration curve" is used. If the unknown sample is compared to a single standard sample, the term "standard" is used. A calibration curve or standard is used to convert between known amino acid concentration and measured label (e.g. signal of the label) of each of two or more amino acid types for the protein of interest, or, to convert between measured label (e.g. signal of the label) of the same two or more amino acid types and the amino acid concentration of each amino acid type in the sample. A calibration curve for an amino acid type refers to data (signal of the label) collected for several known amino acid concentrations of the amino acid type, and a standard refers to data (signal of the label) collected for one known amino acid concentration of the amino acid type. A calibration function or (scalar) calibration factor is calculated from the calibration curve or standard

As used herein, the term "proportion" refers to any number of amino acids of an amino acid type that is less than all of the amino acids of an amino acid type in the sample, i.e. less than 100% of the amino acids of an amino acid type in the sample. The term "proportion" also refers to any number of amino acids of an amino acid type that is less than all of the subset of the amino acids of the amino acid type that react with the label (e.g. unmodified amino acids of an amino acid type), for example according to the rules provided in Table 4. The term "proportion" includes, but is not limited to, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 89%, about 90%, about 91%, about 92%, about 93%, about 94%, about 95%, about 96%, about 97%, about 98% or about 99% of amino acids of each amino acid type being labelled in the sample. In some embodiments, a proportion is about 50% of the amino acids of a particular amino acid type present in the sample. In some embodiments, a proportion is about 60% of the amino acids of a particular amino acid type present in the sample. In some embodiments, a proportion is about 70% of the amino acids of a particular amino acid type present in the sample. In some embodiments, a proportion is about 80% of the amino acids of a particular amino acid type present in the sample. In some embodiments, a proportion is about 90% of the amino acids of a particular amino acid type present in the sample.

As used herein, the term "measuring" refers to the detection and quantification. In some embodiments, measuring includes measuring a signal.

As used herein, the term "number of amino acids" refers to the number of amino acids of a certain amino acid type per molecule. To determine the number of amino acids of each labelled type in a sample, the amino acid concentration of an amino acid type in a sample is divided by the molar protein concentration of the sample. To determine the number of amino acids of an amino acid type in a protein of interest, or reference, the number of amino acids of an amino acid type is calculated from the protein sequence of the protein of interest, or, has been previously determined and for example is accessible via a database. Alternatively, the number of amino acids of an amino acid type in a protein of interest can be determined by labelling an amino acid type in the protein of interest at a known protein concentration, measuring the label, converting the measuring label to the amino acid concentration using the methods disclosed herein and dividing the amino acid concentration of the amino acid type by the molar protein concentration of the protein of interest. For example, if lysine is the amino acid type being labelled and there are 54 lysine's per protein molecule in the sample, then the number of amino acids of the amino acid type of lysine is 54. The number of amino acids of an amino acid type does not refer to the total number of amino acids of an amino acid type in a solution containing the sample. For example, if there are 10000 protein molecules in the sample, and each protein molecule contains 54 lysine amino acids, then the number of amino acids of the lysine amino acid type is 54, not 540000.

As used herein, the term "background correct" or "background corrected" refers to the measured label of each labelled amino acid type which has been corrected to exclude any signal from the free label in solution not added, inserted, attached, bound, bonded or covalently bonded to amino acids of the amino acid type of interest, non-specific labelling, or other sources of signal that would otherwise contribute to the total label being measured, such as cellular autofluorescence. This is achieved by standard means in the art.

As used herein, the term "bulk" refers to studies performed without constraining the sample within channels that have dimensions of in general hundreds of micrometers or less. Classically, bulk studies do not involve manipulation of small amounts (picoliters to nanoliters) of fluids, and fluids mix turbulently in addition to diffusively. Bulk studies include the automated manipulation of fluids, for example by pumps or robots. Bulk studies can involve analysing samples in plates, which have sample reservoirs to perform many reactions and/or measurements in parallel, and can involve using a plate reader or similar instrument. Generally, bulk studies do not seek to detect single protein molecules.

As used herein, the term "solution phase" refers to studies performed and measured in solution. Solution phase excludes methods which require measurement on a surface, such as transforming internal reflection fluorescence (TIRF) microscopy. Solution phase excludes methods that require proteins within a sample to be passed through synthetic or natural pores within a surface. For example, solution phase excludes methods incorporating nanopores, small channels within surfaces, and excludes methods incorporating biological nanopores, transmembrane proteins embedded within lipid membranes.

As used herein, the term "deconvolute" refers to a process in which a signal deriving from multiple components is

analyzed or transformed to reveal the portions from each component. In some embodiments, if a time-resolved signal derives from two components and there are two separated peaks, then a signal can be deconvoluted kinetically such that analysis of one peak provides information about one component and analysis of the other peak provides information about the other component. For example, kinetic deconvolution can be used if the label is a fluorescent label and two or more amino acid types are labelled with the same fluorescent label under the same conditions, but the labelling reactions proceed at different rates, such that measuring the signal of the label at a certain time provides information about exclusively one amino acid type, and measuring the signal of the label at another time provides information about exclusively another amino acid type. Alternatively, if a signal derives from two components and one component is known, the signal can be transformed to remove the known component and only reveal information about the unknown component.

As used herein, the term "deconvolution standard" refers to a protein of known amino acid concentration of the two or more amino acid types labelled and measured in the sample which is used to deconvolute the signals obtained when two amino acid types are labelled with the same label under the same conditions. A deconvolution standard can be measured at different excitation and emission wavelengths, to deconvolute the contribution of each labelled amino acid type at each wavelength and enable separation of the signals of each labelled amino acid type in the sample. A deconvolution standard is not a "calibration curve or standard" discussed above.

As used herein, the term "protein sequencing" refers to determining the sequence of amino acids in a protein, peptide, oligopeptide, or polypeptide. Protein sequencing involves consecutively reading and identifying single amino acids along an amino acid sequence, starting at one terminus of the amino acid chain, and moving, one amino acid at a time, along the amino acid chain. Protein sequencing determines the positions of amino acids within a protein. For example, Edman degradation is a common method of protein sequencing.

As used herein, the term "n-dimensional space" refers to a mathematical space in which *n* is the minimum number of coordinates needed to specify any point within it. Within an n-dimensional space, there are n-dimensions of information. The dimensions of information are the number of amino acid types being labelled. For example, 3 dimensions of information refers to 3 amino acid types being labelled and requires a 3-dimensional space. In some embodiments, an n-dimensional space is used to plot the values of the label, amino acid concentrations, or number of amino acids for *n* amino acid types. In an n-dimensional space, there are n coordinates necessary to specify any vector.

As used herein, the term "reference" is a standard or control value against which the value of the sample is compared. The reference can include information indicating the known label values, and/or amino acid concentrations, and/or number of amino acids of the same two or more amino acid types as the amino acid types that have been labelled in the sample for each protein, peptide, oligopeptide, polypeptide, protein complex, subproteome, or proteome of interest. The reference can include the known label values (e.g. signal, e.g. fluorescence intensity) or the amino acid concentrations of two or more amino acid types of the protein, peptide, oligopeptide, polypeptide, protein complex subproteome or proteome of interest at one or more protein concentrations, or, the number of amino acids of two or more amino acid types in the amino acid sequence or sequences of the protein, peptide, oligopeptide, polypeptide, protein complex, subproteome or proteome of interest. The two or more amino acid types are the same two or more amino acid types that have been labelled in the sample. The reference for each protein, peptide, oligopeptide, polypeptide, protein complex, subproteome, or proteome of interest is used to identify the presence and/or concentration and/or amount of one or more proteins, peptides, oligopeptides, polypeptides, protein complexes, subproteomes, or proteomes of interest within a sample. In some embodiments, the reference is the weighted mean of the known label values, amino acid concentration or number of amino acids of two or more amino acid types across all of the amino acid sequences of a proteome or subproteome, weighted by the proportion of each protein across the proteome, subproteome or mixture of proteins. In some embodiments, the reference is stored in, and accessed/obtained from, a database. In some embodiments, the reference is experimentally determined. In some embodiments, the reference is calculated from the amino acid sequence or sequences of the one or more proteins, peptides, oligopeptides, polypeptides, protein complexes, subproteomes or proteomes of interest. In some embodiments, creating the reference includes accessing the publicly available amino acid sequences of a variety of proteins and removing the portions of the sequence that are biologically cleaved in the mature proteins. In some embodiments, creating the reference includes determining the number of amino acids of the same two or more amino acid types as have been labelled in the sample within the amino acid sequence or amino acid sequences of the one or more proteins, peptides, oligopeptides, polypeptides, protein complexes, subproteomes, or proteomes of interest, having optionally applied the rules outlined in Table 4 to remove from the number of amino acids of an amino acid type post-translationally modified amino acids that would not react with the label for the amino acid type. In some embodiments, the reference is determined using the methods disclosed herein, i.e. labelling two or more amino acid types, measuring the label and using the measured label to determine the number of amino acids of each amino acid type in the one or more proteins, peptides, oligopeptides, polypeptides, protein complexes, subproteomes, or proteomes of interest or the concentration of amino acids of each amino acid type in a sample containing each protein, peptide, oligopeptide, polypeptide, protein complex, subproteome, or proteome of interest. In some embodiments, the reference provides the known label values and/or amino acid concentrations of the same two or more amino acid types as the amino acid types

that have been labelled in the sample of each protein, peptide, oligopeptide, polypeptide, protein complex, subproteome, or proteome of interest as a set of parametric equations or a vector function depending on the common parameter of concentration. In other embodiments, the reference provides the number of amino acids of the same two or more amino acid types as the amino acid types that have been labelled in the sample of each protein, peptide, oligopeptide, polypeptide, protein complex, subproteome, or proteome of interest. In some embodiments, the reference includes concentration ranges for the protein, peptide, oligopeptide, polypeptide, protein complex, subproteome or proteome of interest, that are known or that are determined using the methods of the invention. In some embodiments, these known concentration ranges are used as bounds on the function or functions which comprise the reference. In some embodiments, the reference includes additional information, such as information incorporating observed experimental error rates. In some embodiments, the reference includes information derived from Benford's law which provides the frequency distribution of leading digits within many datasets observed in nature.

As used herein the term "single reference" refers to a reference provided for a proteome and/or subproteome of interest uniquely identifying the proteome and/or subproteome of interest on the basis of its average composition. Although many individual proteins may be contained in a proteome and/or subproteome of interest, it is not necessary to provide the known label values, amino acid concentrations and/or number of amino acids as a reference for each protein contained within the proteome and/or subproteome of interest in order to identify the proteome and/or subproteome of interest. For example, if a proteome of interest contains 700 proteins, it is not necessary to provide a the known label values, amino acid concentrations and/or number of amino acids as a reference for all 700 proteins contained within the proteome and/or subproteome of interest. Instead, the single reference provided for the proteome and/or subproteome of interest provides the average signature of the proteome and/or subproteome of interest, permitting its identification. For example, the single reference for the colorectal cancer proteome of interest in blood plasma permits the identification of the colorectal cancer proteome of interest from blood plasma via only labeling and measuring two or more amino acid types within the blood plasma solution and comparing the measured values of the label or amino acid concentrations calculated from the measured values of the label to the values provided by the single reference. There is no need to measure individual proteins and/or biomarkers within the colorectal cancer proteome and/or subproteome of interest in order to detect the presence and/or concentration and/or amount of the colorectal cancer proteome and/or subproteome of interest. A proteome and/or subproteome of interest is identified and it's concentration/amount determined without any requirement to measure a single protein within it. The single reference for a proteome and/or subproteome of interest can be calculated theoretically or experimentally using the methods of the invention and is an algebraic function of the total protein concentration of the proteome and/or subproteome of interest, which can for example be described by one of the vector functions or sets of parametric equations described herein.

As used herein, the term "reduced cysteine" ($C_R$) refers to unmodified amino acids of the amino acid type cysteine (C), which have a reduced thiol R-group. Reduced cysteine is unmodified because it is not disulphide bonded during post-translational modification and has not undergone any other post-translational modification of the thiol R-group such as oxidation to sulfenic, sulfinic or sulfonic acid, palmitoylation, or S-nitrosylation. The term "reduced cysteine" ($C_R$) is equivalent to the term "free cysteine" known in the art.

As used herein, the term "cysteine disulphide" ($C_D$) refers to modified amino acids of the amino acid type cysteine (C), in which a thiol R-group has undergone an oxidative coupling reaction with another thiol R-group resulting in the formation of a disulphide bond. Cysteine disulphide ($C_D$) has an oxidized thiol R-group. Cysteine disulphide ($C_D$) is a type of reversible post-translational modification of the amino acid type cysteine (C). The number of cysteine disulphides refers to the number of cysteine amino acids engaged in disulphide bonds, not the number of disulphide bonds which is ½ the number of cysteine disulphides engaged in disulphide bonds because one disulphide bond comprises two cysteine amino acids.

As used herein, the term "cysteine" (C) refers to unmodified amino acids of cysteine ($C_R$), modified amino acids of cysteine ($C_D$) and/or the combination of unmodified and modified amino acids of cysteine. In order to label both modified and unmodified cysteine amino acids , first the cysteine disulphide ($C_D$) amino acids are chemically reduced to reduced cysteine ($C_R$), such that they are available for reaction with the label.

As used herein, the term "classifier" refers to an algorithm that implements classification. Classification is the identification of a category to which a new observation belongs, on the basis of a training set of data that contains observations whose category membership is known. The term "classifier" encompasses a machine learning classifier that uses supervised learning to learn a function that maps an input to an output based on example input-output pairs, including using both lazy learning (instance-based learning) and eager learning. For example, a classifier describes a k-nearest neighbor classifier (lazy learning) and/or a support vector machine classifier (eager learning). The classifier can be used in the comparison step of the methods described herein.

As used herein, the term "duplicate" refers to rare a case in which more than one protein, peptide, oligopeptide, polypeptide, protein complex, subproteome, or proteome of interest have the same reference, or where the references for more than one protein, peptide, oligopeptide, polypeptide, protein complex, subproteome, or proteome of interest are indistinguishable based on a comparison of values of the label of two or more amino acid types, amino acid concentration,

or number of amino acids of two or more amino acid types. This occurs because the number of amino acids of two or more amino acid types in one protein of interest is the same as, or a multiple of, the number of amino acids of the same two or more amino acid types in another protein of interest. A reference can have 1, 2, 3, 4, 5, 6, 7, 8, 9, 10, 11, 12, 13, 14, 15, 16, 17, 18, 19 or 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 duplicates, but more than 1 duplicate is rare. If two proteins of interest have the same reference, and this reference is identified within the sample, then the sample is identified as containing either of these two proteins of interest. There are a number of approaches to eliminate this effect and uniquely identify the protein of interest present within the sample, such as comparing the protein concentration at which the reference has been identified in the sample to the known biologically relevant concentration ranges of both proteins of interest. The sample is identified as containing the protein of interest which is within its biologically relevant concentration range.

For the sake of clarity and explanation, the methods are described in the context of a protein or proteome of interest. However, unless otherwise specified or made clear from the context, the methods of the invention should be understood to be generally, additionally, or alternatively, applicable to one or more proteins, peptides, oligopeptides, polypeptides, protein complexes, subproteomes, or proteomes of interest.

## Sample

The samples utilised in the present methods have been obtained from the subject using standard methodology. Preferably, the sample is a bodily fluid sample, tissue sample, soil sample, water sample, environmental sample, crop sample, food sample, drink sample or laboratory sample.

Bodily fluid samples encompassed by the invention include, but are not limited to: whole blood samples, blood serum samples, blood plasma samples, salvia samples, sputum samples, faeces samples, urine samples, semen samples, nasal swab samples, nasopharyngeal aspirate samples, throat swab, or lower respiratory samples, such as a lower respiratory mucus aspirate sample, cerebrospinal (CSF) sample, sexual health sample, such as a urethral swab, cervix swab, vaginal swab or rectal swab. Alternatively, the sample can contain any other bodily fluid known in the art. In some embodiments, the bodily fluid sample is any type of fluid produced by a lesion. In some embodiments, the sample is a blood plasma sample. In some embodiments, the sample is a platelet poor plasma (PPP) sample. In some embodiments, the sample is a platelet rich plasma (PPP) sample. In some embodiments, the sample is a platelet sample. In some embodiments, the sample is a blood plasma exosome sample. In some embodiments, the sample is a blood cell sample. In some embodiments, the blood cell sample is a lymphocyte sample or a myeloid cell sample. In some embodiments, the sample is a urine sample.

Alternatively, the sample may be a tissue sample. Preferably, the tissue sample is a biopsy of any tissue type of interest. For example, the tissue sample can be a biopsy of a solid tumor. This includes, for example, sarcomas, lymphomas, carcinomas and melanomas.

Alternatively, the sample may be an environmental sample. Preferably, the environmental sample is a water sample, such as a drinking water sample or wastewater sample. In some embodiments, the sample is a sample suspected of biological warfare.

Alternatively, the sample may be a food sample, for example in the food industry. For example, the methods of the invention may be used to test a food sample for bacterial growth and composition, for example in cheese making, testing for flour and bread quality in bread making such as via assessing the strength of gluten, quantifying the amount of a fermentation agent (for example, identifying and quantifying the amount of bacteria in kombucha to ensure it is safe to consume), testing yoghurt, or testing a sourdough mother culture. Preferably, the food sample is suspected of containing an allergen.

In some embodiments, the sample can be suspected of containing an allergen. Preferably, the allergen is peanuts, gluten, lactose, pollen or dust mites, dust, caseins, lipocalins, c-type lysozymes, protease inhibitors, tropomyosins, parvalbumins, cat dander, dog dander.

Alternatively, the sample may be a drink sample such as a milk sample, a water sample or a fruit juice sample. For example, the methods of the invention could be used in the agriculture industry to measure a chemical signature of the hormone component of milk, or to assess unpasteurized milk or fruit juices for bacterial contamination.

In some embodiments, the sample is a bodily fluid sample (e.g. whole blood samples, blood serum samples, blood plasma samples, salvia samples, sputum samples, faeces samples, urine samples, semen samples, nasal swab samples, nasopharyngeal aspirate samples, throat swab, or lower respiratory samples, such as a lower respiratory mucus aspirate sample, cerebrospinal (CSF) sample, sexual health sample, such as a urethral swab, cervix swab, vaginal swab or rectal swab, or any type of fluid produced by a lesion), a tissue sample, a soil sample, an environmental sample (e.g. water sample such as a drinking water sample or wastewater sample; or sample suspected of biological warfare), a food sample (e.g. suspected of containing an allergen such as peanuts, gluten, lactose or pollen, caseins, lipocalins, c-type lysozymes, protease inhibitors, tropomyosins, parvalbumins, cat dander and/or dog dander, or a functional foods sample) or drink sample (e.g. milk, water, fruit juice).

In some embodiments, the proteins are isolated from the sample using standard techniques in the art such as centrifugation, filtration, extraction, precipitation and differentiation solubilization, ultracentrifugation, size exclusion chromatography, separation based on charge or hydrophobicity (examples include hydrophobic interaction chromatography, ion exchange chromatography, and/or free-flow electrophoresis), and/or affinity chromatography such as immunoaffinity chromatography or high-performance liquid chromatography (HPLC). The proteins within the sample can also be concentrated once isolated. This can involve, but is not limited to, lyophilization or ultrafiltration. For example, if the sample is a saliva sample, and the presence of viruses or bacteria are being detected in the sample, the viral and bacterial proteins in the sample are separated from the human protein in the sample by centrifugation. After centrifugation, the pellet corresponds to the viruses and bacteria present in the sample, without the human proteins present within the supernatant. In another example, if the sample is a solid tissue sample, and the presence of viruses or bacteria are being detected in the sample, the viral and bacterial proteins in the sample are separated from the human protein in the sample by freezing the tissue sample, crushing the sample and extracting the protein from the tissue into a buffer. An example of these technique, which is standard in the art for extracting proteins from tissue samples is provided by January Ericsson, C. Protein Extraction from Solid Tissue. 2011. Methods in molecular biology (Clifton, N.J.) 675:307-12. DOI: 10.1007/978-1-59745-423-0_17.

The sample may be suspected of containing the presence of one or more proteins, peptides, oligopeptides, polypeptides, protein complexes, or proteomes of interest. In some embodiments, the proteins, peptides, oligopeptides, polypeptides, protein complexes, or proteomes of interest are isolated from other proteins in the sample.

**Protein of interest**

Although a "protein of interest" is referred to throughout this application, the term "protein of interest" is provided as an example and can be substituted with peptide of interest, oligopeptide of interest, polypeptide of interest, proteome complex of interest, subproteome of interest, or proteome of interest, or combination thereof, whose presence and/or concentration and/or amount within the sample is being tested. In this general sense of the term, "protein of interest" is suspected of being in the sample, and the hypothesis of the protein of interest being within the sample is tested via the methods of the invention.

In some embodiments, the proteome of interest is a viral proteome, bacterial proteome, fungal proteome or parasitic proteome that is suspected of causing a viral infection, bacterial infection, fungal infection or parasitic infection, respectively. For example, in some embodiments, the subject is suspected of having malaria and the proteomes of interest include *P. falciparum, P. malariae, P. ovale, P. vivax* and *P. knowlesi* proteomes. These parasites are the known causative agents of malaria. A sample, such as a blood sample is obtained from a subject suspected of having malaria, and the parasitic proteomes are separated from the blood using filtration. The parasitic proteins isolated from the blood sample are tested for the presence of any one of *P. falciparum, P. malariae, P. ovale, P. vivax* and *P. knowlesi* proteomes in order to confirm the diagnosis of Malaria and identify the particular parasite causing Malaria in the subject's sample.

In some embodiments, the proteome of interest is a viral proteome. For example, in some embodiments, a subject is showing symptoms of a dry cough, tiredness, muscle aches and fever and so the subject is suspected of having influenza or coronavirus. A sample, such as a blood sample, nasal swab, nasopharyngeal aspirate or lower respiratory mucus aspirate sample is obtained from the subject and the sample is tested for the presence of Influenza proteomes, for example the Influenza A H1N1 proteome, and/or Coronavirus proteomes, for example the SARS-CoV-2 (Covid-19) proteome to identify the virus causing the symptoms in the subject and thus identify the infection that the subject has.

In some embodiments, the proteome of interest is the human proteome. In some embodiments, the proteome of interest is the human plasma proteome. In some embodiments, the albumin fraction of the human plasma proteome is removed prior to the remaining steps of the method. In some embodiments, the albumin and globulin fraction of the human plasma proteome is removed prior to the remaining steps of the method. In alternative embodiments, the albumin fraction of the human plasma proteome is not removed prior to the remaining steps of the method. In some embodiments, the albumin and globulin fraction of the human plasma proteome is not removed prior to the remaining steps of the method. In some embodiments, the albumin and globulin fraction of the human plasma proteome is removed prior to the remaining steps of the method using a centrifugal filtration step that removes high molecular weight proteins such as albumin and globulin prior to the remaining steps of the method. In some embodiments, the proteome of interest is one or more of the following human proteomes of specific glands/tissues: human eye proteome, retina, heart, skeletal muscle, smooth muscle, adrenal gland, parathyroid gland, thyroid gland, pituitary gland, lung, bone marrow, lymphoid tissue, liver, gallbladder, testis, epididymis, prostate, seminal vesicle, ductus deferens, adipose tissue, brain, salivary gland, esophagus, tongue, stomach, intestine, pancreas, kidney, urinary bladder, breast, vagina, cervix, endometrium, fallopian tube, ovary, placenta, skin, blood, or any combination thereof. The proteome of interest can also include the human metabolic proteome and/or the human secretory proteome.

In some embodiments, the proteome of interest can be a subproteome. For example, one or more human cancer subproteomes, selected from: the human pancreatic cancer subproteome, human glioma subproteome, human head and

neck cancer subproteome, human thyroid gland cancer subproteome, human lung cancer subproteome, human liver cancer subproteome, human testisticular cancer subproteome, human prostate cancer subproteome, human stomach cancer subproteome, human colon/rectal cancer subproteome, human breast cancer subproteome, human endometrial cancer subproteome, human ovarian cancer subproteome, human cervical cancer subproteome, human kidney cancer subproteome, human urinary and bladder cancer subproteome, human melanoma subproteome and any combinations thereof. The following subproteomes can also be of interest: the human type I diabetes mellites subproteome, the human type II mellites diabetes subproteome, Alzheimer's disease subproteome, human Parkinson's disease subproteome, human dementia subproteome, human cardiovascular disease subproteome, human down syndrome subproteome, human aging subproteome or any combination thereof.

In some embodiments, a disease-associated sub proteome includes those proteins of an organism affected by the disease state of that organism. In some embodiments, the subproteome of interest is the human pancreatic cancer subproteome of the human blood plasma proteome. In some embodiments, the subproteome of interest is human pancreatic cancer subproteome of the human platelet poor plasma (PPP) proteome. In some embodiments, the subproteome of interest is human pancreatic cancer subproteome of the human platelet rich plasma (PRP) proteome. In some embodiments, the subproteome of interest is the human pancreatic cancer subproteome of the human blood plasma proteome. In some embodiments, the subproteome of interest is human pancreatic cancer subproteome of the human platelet poor plasma (PPP) proteome. In some embodiments, the subproteome of interest is human pancreatic cancer subproteome of the human platelet rich plasma (PRP) proteome. In some embodiments, the subproteome of interest is human prostate cancer subproteome. In some embodiments, the subproteome of interest is human colorectal cancer subproteome. In some embodiments, the subproteome of interest is human pancreatic cancer subproteome.

In some embodiments, the proteome of interest is a viral proteome. In some embodiments, the viral proteome is selected from: human papilloma virus (HPV) proteome, human immunodeficiency virus (HIV) proteome, Orthomyxoviridae proteome, Epstein Barr proteome, Ebolavirus proteome, Rabies lyssavirus proteome, Coronovirus proteome, Novovirus proteome, Hepatitis A proteome, Hepatitis B proteome, Hepatitis C proteome, Hepatitis E proteome, Hepatitis delta proteome, Herpesvirus proteome, Papillomavirus proteome, rhinovirus proteome, Measles virus proteome, Mumps virus proteome, Poliovirus proteome, rabies proteome, rotavirus proteome, west nile virus proteome, yellow fever virus proteome, Zika virus proteome, Caudovirales proteome, Nimaviridae proteome, Riboviria proteome, Inoviridae proteome, Fuselloviridae proteome, Herpesvirales proteome, Asfarviridae proteome, Bicaudaviridae proteome, tuberculosis proteome, bovine tuberculosis proteome, and any combination thereof.

In some embodiments, the Orthomyxoviridae proteome is an influenza proteome. The influenza proteome includes, but is not limited to: the Influenza A proteome, the Influenza A subtype H1N1 proteome, Influenza B proteome, Influenza C proteome or Influenza D proteome, or any combination thereof. In some embodiments, the Coronovirus proteome is the SARS-CoV-2 (Covid-19) proteome, the SARS-CoV proteome, or the MERS-CoV proteome. In some embodiments, the viral proteome of interest is a zoonotic virus proteome.

In some embodiments, the proteome of interest is a bacterial proteome. In some embodiments, the bacterial proteome includes, but is not limited to, the *Escherichia coli* (E. coli) proteome, *Pseudomonas aeruginosa* (P. aeruginosa) proteome, *Salmonella* proteome, *Staphylococcus aureus* proteome, *Acinetobacter baumannii* proteome, *Bacteroides fragilis* proteome, *Burkholderia cepacia* proteome, *Clostridium difficile* proteome, *Clostridium sordellii* proteome, *Enterobacteriaceae* proteome, *Enterococcus faecalis* proteome, *Klebsiella pneumoniae* proteome, *Methicillin-resistant Staphylococcus aureus* proteome, *Morganella morganii* proteome, *Mycobacterium* proteome or any combination thereof. In some embodiments, the Mycobacterium proteome is the *Mycobacterium tuberculosis* proteome.

In some embodiments, the proteome of interest is a parasitic proteome. In some embodiments, the parasitic proteome is selected from: a *Plasmodium* proteome, *Toxoplasma gondii* proteome, *Trichomonas vaginalis* proteome, Giardia duodenalis proteome, *Cryptosporidiu proteome* or any combination thereof. In some embodiments, the *Plasmodium* proteome is the *Plasmodium falciparum* proteome, *Plasmodium knowlesi* proteome, *Plasmodium malariae* proteome, *Plasmodium ovale* proteome or *Plasmodium vivax* proteome.

In some embodiments, the protein of interest is an allergen. Preferably, the allergen is peanuts, gluten, lactose, pollen, caseins, lipocalins, c-type lysozymes, protease inhibitors, tropomyosins, parvalbumins, cat dander and/or dog dander.

In some embodiments, the coumpound of interest is one or more proteins or peptides (e.g. alpha synuclein, lysozyme, bovine serum albumin, ovalbumin, β-Lactoglobulin, insulin, glucagon, amyloid beta, angiotensin-converting enzyme 2, angiotensin-converting enzyme, bradykinin, chordin-like protein 1, tumor necrosis factor beta, osteomodulin precursor, a matrix metalloproteinase protein, pleiotrophin, secretogranin-3, human growth hormone, insulin-like growth factor 1, leptin, telomerase, thyroid-stimulating hormone), human proteome (e.g. human plasma proteome, human eye proteome, retina, heart, skeletal muscle, smooth muscle, adrenal gland, parathyroid gland, thyroid gland, pituitary gland, lung, bone marrow, lymphoid tissue, liver, gallbladder, testis, epididymis, prostate, seminal vesicle, ductus deferens, adipose tissue, brain, salivary gland, esophagus, tongue, stomach, intestine, pancreas, kidney, urinary bladder, breast, vagina, cervix, endometrium, fallopian tube, ovary, placenta, skin, blood, human metabolic proteome, human secretory

proteome), human subproteome (e.g. human cancer subproteome, selected from: the human pancreatic cancer proteome, human glioma subproteome, human head and neck cancer subproteome, human thyroid gland cancer subproteome, human lung cancer subproteome, human liver cancer subproteome, human testisticular cancer subproteome, human prostate cancer subproteome, human stomach cancer subproteome, human colon/rectal cancer subproteome, human breast cancer subproteome, human endometrial cancer subproteome, human ovarian cancer subproteome, human cervical cancer subproteome, human kidney cancer subproteome, human urinary and bladder cancer subproteome, human melanoma subproteome), (or e.g. the human type I diabetes subproteome, the human type II diabetes subproteome, Alzheimer's disease subproteome, human Parkinson's disease subproteome, human dementia subproteome, human cardiovascular disease subproteome, human down syndrome subproteome, human aging subproteome), viral proteome (e.g. human papilloma virus (HPV) proteome, human immunodeficiency virus (HIV) proteome, Orthomyxoviridae proteome, such as influenza proteome, such as Influenza A proteome, the Influenza A subtype H1N1 proteome, Influenza B proteome, Influenza C proteome or Influenza D proteome, Epstein Barr proteome, *Ebolavirus* proteome, *Rabies lyssavirus* proteome, Coronovirus proteome, such as SARS-CoV-2 (Covid-19) proteome, the SARS-CoV proteome, or the MERS-CoV, Novovirus proteome, Hepatitis A proteome, Hepatitis B proteome, Hepatitis C proteome, Hepatitis E proteome, Hepatitis delta proteome, Herpesvirus proteome, Papillomavirus proteome, rhinovirus proteome, Measles virus proteome, Mumps virus proteome, Poliovirus proteome, rabies proteome, rotavirus proteome, west nile virus proteome, yellow fever virus proteome, Zika virus proteome, Caudovirales proteome, Nimaviridae proteome, Riboviria proteome, Inoviridae proteome, Fuselloviridae proteome, Herpesvirales proteome, Asfarviridae proteome, Bicaudaviridae proteome, tuberculosis proteome, bovine tuberculosis proteome), zoonotic virus proteome, bacterial proteome (e.g. *Escherichia coli* (E. coli) proteome, *Pseudomonas aeruginosa* (P. aeruginosa) proteome, *Salmonella* proteome, *Staphylococcus aureus* proteome, *Acinetobacter baumannii* proteome, *Bacteroides fragilis* proteome, *Burkholderia cepacia* proteome, *Clostridium difficile* proteome, *Clostridium sordellii* proteome, *Enterobacteriaceae* proteome, *Enterococcus faecalis* proteome, *Klebsiella pneumoniae* proteome, *Methicillin-resistant Staphylococcus aureus* proteome, *Morganella morganii* proteome, *Mycobacterium* proteome, such as the *Mycobacterium tuberculosis* proteome), parasitic proteome (e.g. Plasmodium proteome, Toxoplasma gondii proteome, Trichomonas vaginalis proteome, Giardia duodenalis proteome, Cryptosporidiu proteome or any combination thereof. In some embodiments, the Plasmodium proteome is the Plasmodium falciparum proteome, Plasmodium knowlesi proteome, Plasmodium malariae proteome, Plasmodium ovale proteome or Plasmodium vivax proteome) and any combination thereof.

**Amino acid types**

In the methods described, two or more amino acid types are labelled.

All amino acids have a common structure: a carboxylic acid, an amine, and an alpha carbon which has an R-group side chain. The carboxylic acid, amine, and alpha carbon are common to all amino acid types. Within chains of amino acids (peptides, oligopeptides, polypeptides, proteins), peptide bonds, which are a type of amide bonds, link adjacent amino acids. These adjacent amino acids have undergone a condensation reaction in which the non-side chain carboxylic acid group of one amino acid reacted with the non-side chain amine group of the other. One adjacent amino acid has lost a hydrogen and oxygen from its carboxyl group (COOH) and the other has lost a hydrogen from its amine group ($NH_2$), producing a molecule of water ($H_2O$) and two amino acids joined by a peptide bond (-CO-NH-). Amino acids joined in this way can also be called residues or amino acid residues. All amino acids participate in the peptide backbone, which describes the repetitive covalent linkages from one amino acid to the next which incorporates the amine nitrogen, alpha carbon, and carboxyl carbon of each amino acid linked via a peptide bond to the same atoms of the next amino acid in a repeating pattern. Every alpha carbon has a variable side chain, called an R-group, which does not participate in the peptide backbone. An amino acid type is defined by the R-group, i.e. side chain. The R-group is specific to each amino acid type. The R-group of one amino acid type is distinguishable from the R-group of every other amino acid type. For example, the R-group for lysine (K) is a ε-primary amino group. Every K amino acid has this ε-primary amino group when translated. Therefore, the K amino acid type is defined by the ε-amino R-group. In another example, the R-group for tryptophan (W) is an indole group. Every W amino acid has an indole group. Therefore, the W amino acid type is defined by the indole R-group. Hence, the amino acid type K is distinguishable to the amino acid type W because of the different R-groups between these amino acid types. If the R-group of an amino acid type is subsequently modified after translation, such as post-translationally modified, the amino acid type does not change.

The two or more amino acid types encompassed by the invention include modified and/or unmodified amino acids of each amino acid type. This includes modified and/or unmodified amino acids of the 22 proteinogenic amino acid types and/or non-proteinogenic or synthetic amino acids.

The two or more amino acid types encompassed by the invention include the 22 proteinogenic amino acids selected from: alanine (A), arginine (R), asparagine (N), aspartic acid (D), cysteine (C), glutamic acid (E), glutamine (Q), glycine (G), histidine (H), isoleucine (I), leucine (L), lysine (K), methionine (M), phenylalanine (F), proline (P), pyrrolysine (O), selenocysteine (U), serine (S), threonine (T), tryptophan (W), tyrosine (Y) and valine (V), and any combination thereof.

In some embodiments, the two or more amino acid types are selected from: cysteine (C), tyrosine (Y), lysine (K), arginine (R), histidine (H), proline (P), aspartic acid (D), glutamic acid (E), asparagine (B), glutamine (Q), serine (S) and/or threonine (T) and any combination thereof. In some embodiments, the two or more amino acid types are selected from: tryptophan (W), cysteine (C), tyrosine (Y), lysine (K), arginine (R), histidine (H), proline (P), aspartic acid (D), glutamic acid (E), asparagine (B) and/or glutamine (Q) and any combination thereof. In some embodiments, the two or more amino acid types are selected from: tryptophan (W), cysteine (C), tyrosine (Y) and/or lysine (K) and any combination thereof. In some embodiments, the two or more amino acids are selected from: cysteine (C), arginine (R), histidine (H) and/or aspartic acid (D) and any combination thereof. In some embodiments, the two or more amino acid types are selected from: cysteine (C), arginine (R), histidine (H) and/or glutamic acid (E) and any combination thereof. In some embodiments, the two or more amino acid types are selected from: cysteine (C), arginine (R), histidine (H) and/or glutamine (Q) or the modified types thereof and any combination thereof. In some embodiments, the two or more amino acid types are selected from: cysteine (C), arginine (R), tryptophan (W) and/or aspartic acid (D) or the modified version thereof and any combination thereof. In some embodiments, the two or more amino acid types are selected from: lysine (K), Arginine (R), histidine (H) and/or aspartic acid (D) and any combination thereof. In some embodiments, the two or more amino acid types are selected from: lysine (K), tryptophan (W), arginine (R) and/or glutamic acid (E) and any combination thereof. In some embodiments, the two or more amino acid types are selected from: tyrosine (Y), lysine (K), cysteine (C) and/or aspartic acid (D) and any combination thereof. In some embodiments, the two or more amino acid types are selected from: tyrosine (Y), lysine (K), cysteine (C) and/or glutamic acid (E) and any combination thereof. In some embodiments, the two or more amino acid types are selected from: proline (P), cysteine (C), arginine (R), and/or glutamic acid (E) and any combination thereof. In some embodiments, the two or more amino acid types are selected from: proline (P), cysteine (C), arginine (R) and/or aspartic acid (D) and any combination thereof. In some embodiments, the two or more amino acid types are selected from: cysteine (C), asparagine (B), arginine (R) and/or aspartic acid (D) and any combination thereof. In some embodiments, the two or more amino acid types are selected from: cysteine (C), asparagine (B), arginine (R) and/or glutamic acid (E) and any combination thereof. In some embodiments, the two or more amino acid types are selected from: lysine (K), asparagine (B), tryptophan (W) and/or cysteine (C) and any combination thereof. In some embodiments, the two or more amino acid types are selected from: arginine (R), histidine (H), proline (P) and/or aspartic acid (D) and any combination thereof. In some embodiments, the two or more amino acid types are selected from: arginine (R), lysine (K), cysteine (C) and/or aspartic acid (D) and any combination thereof. In some embodiments, the two or more amino acid types are selected from: arginine (R), lysine (K), cysteine (C) and/or glutamic acid (E) and any combination thereof. In some embodiments, the two or more amino acid types are selected from: arginine (R), lysine (K), cysteine (C) and/or tryptophan (W) and any combination thereof. In some embodiments, the two or more amino acid types are selected from: arginine (R), lysine (K), cysteine (C) and/or tyrosine (Y) and any combination thereof. In some embodiments, the two or more amino acid types are selected from: arginine (R), lysine (K), histidine (H) and/or tryptophan (W) and any combination thereof. In some embodiments, the two or more amino acid types are selected from: arginine (R), lysine (K), histidine (H) and/or cysteine (C) and any combination thereof. In some embodiments, the two or more amino acid types are selected from: arginine (R), lysine (K), histidine (H) and/or tyrosine (Y) and any combination thereof. In some embodiments, the two or more amino acid types are selected from: arginine (R), cysteine (C), tryptophan (W) and/or tyrosine (Y) and any combination thereof. In some embodiments, the two or more amino acid types are selected from: arginine (R), cysteine (C), tryptophan (W) and/or proline (P) and any combination thereof. In some embodiments, the two or more amino acid types are selected from: tryptophan (W), cysteine (C) and/or lysine (K) and any combination thereof. In some embodiments, the two or more amino acid types are selected from: lysine (K), tryptophan (W) and/or tyrosine (Y) and any combination thereof. In some embodiments, the two or more amino acid types are selected from: tryptophan (W), tyrosine (Y) and/or cysteine (C) and any combination thereof. In some embodiments, the two or more amino acid types are selected from: tryptophan (W), tyrosine (Y) and/or lysine (K) and any combination thereof. In some embodiments, the two or more amino acid types are selected from: cysteine (C), tryptophan (W) and/or tyrosine (Y) and any combination thereof. In some embodiments, the two amino acid types are leucine (L) and serine (S). In some embodiments, the two amino acid types are leucine (L) and lysine (K). In some embodiments, the two amino acid types are leucine (L) and glutamic acid (E). In some embodiments, the two acid types are glycine (G) and leucine (L). In some embodiments, the two amino acid types are alanine (A) and leucine (L). In some embodiments, the two amino acid types are aspartic acid (D) and leucine (L). In some embodiments, the two amino acid types are leucine (L) and proline (P). In some embodiments, the two amino acid types are leucine (L) and valine (V). In some embodiments, the two amino acid types are lysine (K) and serine (S). In some embodiments, the two amino acid types are glutamic acid (E) and Leucine (L). In some embodiments, the two amino acids types are alanine (A) and arginine (R). In some embodiments, the two amino acids are alanine (A) and glutamic acid (E). In some embodiments, the two amino acids are alanine (A) and glycine (G). In some embodiments, 3 amino acid types are labelled and the 3 amino acid types labelled are tryptophan (W), cysteine (C), and tyrosine (Y). In some embodiments, 3 amino acid types are labelled and the 3 amino acid types labelled are cysteine (C), tyrosine (Y) and lysine (K). In some embodiments, 3 amino acid types are labelled and the 3 amino acid types are tryptophan (W), cysteine (C) and lysine (K). In some embodiments, 3 amino acid types are labelled and the 3 amino acid types are lysine (K), tryptophan (W) and tyrosine

(Y). In some embodiments, 3 amino acid types are labelled and the 3 amino acid types are tryptophan (W), tyrosine (Y) and cysteine (C). In some embodiments, 3 amino acid types are labelled and the 3 amino acid types are tryptophan (W), tyrosine (Y) and lysine (K). In some embodiments, 3 amino acid types are labelled, and the 3 amino acid types labelled are: cysteine (C), tryptophan (W) and tyrosine (Y). In some embodiments, 3 amino acid types are labelled, and the 3 amino acid types labelled are: asparagine (R), glutamic acid (E) and Glycine (G). In some embodiments, 3 amino acid types are labelled, and the 3 amino acid types labelled are: alanine (A), leucine (L) and serine (S). In some embodiments, 3 amino acid types are labelled, and the 3 amino acid types labelled are: asparagine (A), glutamic acid (E) and leucine (L). In some embodiments, 3 amino acid types are labelled, and the 3 amino acid types labelled are: alanine (A), aspartic acid (D) and leucine (L). In some embodiments, 3 amino acid types are labelled, and the 3 amino acid types labelled are: alanine (A), leucine (L) and proline (P). In some embodiments, 3 amino acid types are labelled, and the 3 amino acid types labelled are: alanine (A), glutamic acid (E) and leucine (L). In some embodiments, 3 amino acid types are labelled, and the 3 amino acid types labelled are: leucine (L), serine (S) and valine (S). In some embodiments, 3 amino acid types are labelled, and the 3 amino acid types labelled are: glutamic acid (E), Isoleucine (I) and proline (P). In some embodiments, 3 amino acid types are labelled, and the 3 amino acid types labelled are: glutamic acid (E), Glycine (G) and valine (V). In some embodiments, 3 amino acid types are labelled, and the 3 amino acid types labelled are: Arginine (R), serine (S) and valine (V). In some embodiments, 3 amino acid types are labelled, and the 3 amino acid types labelled are: alanine (A), leucine (L) and lysine (K). In some embodiments, 3 amino acid types are labelled, and the 3 amino acid types labelled are: alanine (A), Arginine (R) and leucine (L). In some embodiments, 3 amino acid types are labelled, and the 3 amino acid types labelled are: alanine (A), leucine (L) and valine (V). In some embodiments, 4 amino acid types are labelled and the 4 amino acid types labelled are selected from the group consisting of: alanine (A), arginine (R), asparagine (N), aspartic acid (D), cysteine (C), glutamic Acid (E), glutamine (Q), glycine (G), histidine (H), isoleucine (I), leucine (L), lysine (K), methionine (M), phenylalanine (F), proline (P), pyrrolysine (O), selenocysteine (U), serine (S), threonine (T), tryptophan (W), tyrosine (Y) and valine (V), and any combination thereof. In some embodiments, 4 amino acid types are labelled, and the 4 amino acid types labelled are tryptophan (W), tyrosine (Y), lysine (K) and cysteine (C). In some embodiments, 4 amino acid types are labelled, and the 4 amino acid types labelled are cysteine (C), arginine (R), Histidine (H) and aspartic acid (D). In some embodiments, 4 amino acid types are labelled, and the 4 amino acid types labelled are cysteine (C), arginine (R), histidine (H) and glutamic acid (E). In some embodiments, 4 amino acid types are labelled, and the 4 amino acid types labelled are cysteine (C), arginine (R), histidine (H) and Glutamine (Q). In some embodiments, 4 amino acid types are labelled, and the 4 amino acid types labelled are cysteine (C), arginine (R), tryptophan (W) and aspartic acid (D). In some embodiments, 4 amino acid types are labelled, and the 4 amino acid types labelled are lysine (K), arginine (R), histidine (H) and aspartic acid (D). In some embodiments, 4 amino acid types are labelled, and the 4 amino acid types labelled are lysine (K), tryptophan (W), arginine (R) and glutamic acid (E). In some embodiments, 4 amino acid types are labelled, and the 4 amino acid types labelled are tyrosine (Y), lysine (K), cysteine (C) and aspartic acid (D). In some embodiments, 4 amino acid types are labelled, and the 4 amino acid types labelled are tyrosine (Y), lysine (K), cysteine (C) and glutamic acid (E). In some embodiments, 4 amino acid types are labelled, and the 4 amino acid types labelled are proline (P), cysteine (C), arginine (R), and glutamic acid (E). In some embodiments, 4 amino acid types are labelled, and the 4 amino acid types labelled are proline (P), cysteine (C), arginine (R) and aspartic acid (D). In some embodiments, 4 amino acid types are labelled, and the 4 amino acid types labelled are cysteine (C), asparagine (B), arginine (R) and aspartic acid (D). In some embodiments, 4 amino acid types are labelled, and the 4 amino acid types labelled are cysteine (C), asparagine (B), arginine (R) and glutamic acid (E). In some embodiments, 4 amino acid types are labelled, and the 4 amino acid types labelled are lysine (K), asparagine (B), tryptophan (W) and cysteine (C). In some embodiments, 4 amino acid types are labelled, and the 4 amino acid types labelled are arginine (R), histidine (H), proline (P) and aspartic acid (D). In some embodiments, 4 amino acid types are labelled, and the 4 amino acid types labelled are arginine (R), lysine (K), cysteine (C) and aspartic acid (D). In some embodiments, 4 amino acid types are labelled, and the 4 amino acid types labelled are arginine (R), lysine (K), cysteine (C) and glutamic acid (E). In some embodiments, 4 amino acid types are labelled, and the 4 amino acid types labelled are arginine (R), lysine (K), cysteine (C) and tryptophan (W). In some embodiments, 4 amino acid types are labelled, and the 4 amino acid types labelled are arginine (R), lysine (K), cysteine (C) and tyrosine (Y). In some embodiments, 4 amino acid types are labelled, and the 4 amino acid types labelled are arginine (R), lysine (K), histidine (H) and tryptophan (W). In some embodiments, 4 amino acid types are labelled, and the 4 amino acid types labelled are arginine (R), lysine (K), histidine (H) and cysteine (C). In some embodiments, 4 amino acid types are labelled, and the 4 amino acid types labelled are arginine (R), lysine (K), histidine (H) and tyrosine (Y). In some embodiments, 4 amino acid types are labelled, and the 4 amino acid types labelled are arginine (R), cysteine (C), tryptophan (W) and tyrosine (Y). In some embodiments, 4 amino acid types are labelled, and the 4 amino acid types labelled are arginine (R), cysteine (C), tryptophan (W) and proline (P). In some embodiments, 4 amino acid types are labelled, and the 4 amino acid types labelled are Glutamine (Q), leucine (L), lysine (K) and valine (V). In some embodiments, 4 amino acid types are labelled, and the 4 amino acid types labelled are arginine (R), Isoleucine (I), leucine (L) and serine (S). In some embodiments, 4 amino acid types are labelled, and the 4 amino acid types labelled are alanine (A), asparagine (N), glutamic acid (E), and serine (S). In some embodiments, 5 amino acid types are labelled and the 5 amino acid types labelled are arginine (R), glutamic acid (E), lysine (K), serine, and Glutamine (Q) In

some embodiments, 5 amino acid types are labelled and the 5 amino acid types labelled are **arginine** (R), aspartic acid (D), lysine (K), serine, and Glutamine (Q) In some embodiments, 5 amino acid types are labelled and the 5 amino acid types labelled are arginine (R), glycine (G), lysine (K), serine, and Glutamine (Q) In some embodiments, 5 amino acid types are labelled and the 5 amino acid types labelled are alanine (A), aspartic acid (D), glycine (G), serine, and arginine (R) In some embodiments, 5 amino acid types are labelled and the 5 amino acid types labelled are pyrrolysine (O), aspartic acid (D), glycine (G), serine, and arginine (R) In some embodiments, 5 amino acid types are labelled and the 5 amino acid types labelled are pyrrolysine (O), aspartic acid (D), Selenocysteine (U), serine, and arginine (R). In some embodiments, 5 amino acid types are labelled and the 5 amino acid types labelled are pyrrolysine (O), aspartic acid (D), selenocysteine (U), lysine, and arginine (R).

The amino acid types encompass L (levo) isomers and/or D (dextro) isomers of each amino acid type.

In some embodiments, the two or more labelled amino acid types comprise modified amino acids and/or unmodified amino acids of an amino acid type. In some embodiments, an amino acid type comprises the unmodified amino acids of an amino acid type. In some embodiments, the unmodified amino acids of an amino acid type have not undergone post-translational modification. In some embodiments, an amino acid type comprises the modified amino acids of an amino acid type. In some embodiments, the modified amino acids of an amino acid type have undergone post-translational modification. In some embodiments, an amino acid type comprises the modified and unmodified amino acids of an amino acid type. For example, the amino acid type cysteine (C) can comprise unmodified cysteine amino acids ($C_R$), modified cysteine amino acids such as cysteine disulfide ($C_D$) and/or a combination of both the unmodified and cysteine disulphide amino acids of cysteine. In some embodiments, a modification of amino acids, such as a post-translational modification, occurs on or including an amino acid R-group. In some embodiments, after a modification, the modified R-groups are not available for a labelling reaction. In some embodiments, the unmodified amino acid type are the amino acids within the amino acid type whose R-groups have not been modified and are therefore available for labelling without any prior chemical modifications. In some embodiments, the modified amino acids are the amino acids within the amino acid type whose R-groups have been modified and are not available for labelling without any prior chemical modifications.

In some embodiments, the amino acid type alanine (A) refers to unmodified alanine amino acids, modified alanine amino acids and/or a combination of modified and unmodified alanine amino acids. In some embodiments, the amino acid type arginine (R) refers to unmodified arginine amino acids, modified arginine amino acids and/or a combination of modified and unmodified arginine amino acids. In some embodiments, the amino acid type , asparagine (N) refers to unmodified asparagine amino acids, modified asparagine amino acids and/or a combination of modified and unmodified asparagine amino acids. In some embodiments, the amino acid type aspartic acid (D) refers to unmodified aspartic acid amino acids, modified aspartic acid amino acids and/or a combination of modified and unmodified aspartic acid amino acids. In some embodiments, the amino acid type cysteine (C) refers to unmodified cysteine amino acids, modified cysteine amino acids and/or a combination of modified and unmodified cysteine amino acids. In some embodiments, the amino acid type glutamic acid (E) refers to unmodified glutamic acid amino acids, modified glutamic acid amino acids and/or a combination of modified and unmodified glutamic acid amino acids. In some embodiments, the amino acid type glutamine (Q) refers to unmodified glutamine amino acids, modified glutamine amino acids and/or a combination of modified and unmodified glutamine amino acids. In some embodiments, the amino acid type glycine (G) refers to unmodified glycine amino acids, modified glycine amino acids and/or a combination of modified and unmodified glycine amino acids. In some embodiments, the amino acid type histidine (H) refers to unmodified histidine amino acids, modified histidine amino acids and/or a combination of modified and unmodified histidine amino acids. In some embodiments, the amino acid type isoleucine (I) refers to unmodified isoleucine amino acids, modified isoleucine amino acids and/or a combination of modified and unmodified isoleucine amino acids. In some embodiments, the amino acid type leucine (L) refers to unmodified leucine amino acids, modified leucine amino acids and/or a combination of modified and unmodified leucine amino acids. In some embodiments, the amino acid type lysine (K) refers to unmodified lysine amino acids, modified lysine amino acids and/or a combination of modified and unmodified lysine amino acids. In some embodiments, the amino acid type methionine (M) refers to unmodified methionine amino acids, modified methionine amino acids and/or a combination of modified and unmodified methionine amino acids. In some embodiments, the amino acid type phenylalanine (F) refers to unmodified phenylalanine amino acids, modified phenylalanine amino acids and/or a combination of modified and unmodified phenylalanine amino acids. In some embodiments, the amino acid type pyrrolysine (O) refers to unmodified pyrrolysine amino acids, modified pyrrolysine amino acids and/or a combination of modified and unmodified pyrrolysine amino acids. In some embodiments, the amino acid type proline (P) refers to unmodified proline amino acids, modified proline amino acids and/or a combination of modified and unmodified proline amino acids. In some embodiments, the amino acid type selenocysteine (U) refers to unmodified selenocysteine amino acids, modified selenocysteine amino acids and/or a combination of modified and unmodified selenocysteine amino acids. In some embodiments, the amino acid type serine (S) refers to unmodified serine amino acids, modified serine amino acids and/or a combination of modified and unmodified serine amino acids. IN some embodiments, the amino acid type threonine (T) refers to unmodified threonine amino acids, modified threonine amino acids and/or a combination of modified and unmodified threonine amino acids. In some embodiments, the amino acid type tryptophan (W) refers to unmodified

tryptophan amino acids, modified tryptophan amino acids and/or a combination of modified and unmodified tryptophan amino acids. In some embodiments, the amino acid type tyrosine (Y) refers to unmodified tyrosine amino acids, modified tyrosine amino acids and/or a combination of modified and unmodified tyrosine amino acids. In some embodiments, the amino acid type valine (V) refers to unmodified valine amino acids, modified valine amino acids and/or a combination of modified and unmodified valine amino acids.

In preferred embodiments, the reactivity of the R-groups with the specific dyes disclosed in Table 3 defines whether, if an amino acid within an amino acid type has undergone a post-translational modification, the labelling reaction will label amino acid within that amino acid type that have not undergone the post-translational modification (unmodified amino acids), or will also label amino acids within that amino acid type that have undergone the post-translational modification (modified amino acids). For example, the skilled person will appreciate that if the labelling reaction involves attack of a nucleophilic R-group, such as lysine primary amine, on an electrophilic dye, the labelling reaction will not proceed if lysine has been post-translationally modified such that it no longer has a nucleophilic primary amine. As another example, the skilled person will appreciate that if the labelling reaction involves radical reaction with the tryptophan indole R-group and trichloroethanol (TCE), this reaction is not inhibited if the tryptophan indole R-group has been mono-oxidized to include a hydroxyl group. Applying these principles, whether a label discussed herein will label unmodified amino acids or almost modified amino acids that have undergone a post-translational modification available for the amino acid type is provided in the following Table (Table 1). If the labelling reaction will only label amino acids within the indicated amino acid type that are unmodified with the indicated post-translational modification, "unmodified" is shown in the labelling column. If the labeling reaction will also label amino acids within the indicated amino acid type that are modified with the indicated post-translational modification, "unmodified + modified) is shown in the labeling column.

**Table 1: Labelling modified and/or unmodified amino acids of each amino acid type**

| Amino Acid Type | R-group | PTM | Labeling |
|---|---|---|---|
| Alanine, A | methyl | N-acetylation | unmodified + modified |
| Arginine, R | aliphatic guanidino group | methylation | unmodified |
| | | deimination to citrulline | unmodified |
| Asparagine, N | β-carboxamide | deamidation to D or iso(D) | unmodified |
| | | N-linked glycosylation | unmodified |
| Asparatic acid, D | β-carboxylic acid | isomerization to isoaspartic acid | unmodified + modified |
| Cysteine, C | thiol | disulfide-bond formation | unmodified |
| | | oxidation to sulfenic, sulfinic or sulfonic acid | unmodified |
| | | palmitoylation | unmodified |
| | | N-acetylation (N-terminus) | unmodified + modified |
| | | S-nitrosylation | unmodified |
| Glutamic acid, E | γ-carboxylic acid | cyclization to Pyroglutamic acid (N-terminus) | unmodified |
| | | gamma-carboxylation | unmodified |
| Glutamine, Q | γ-carboxamide | cyclization to Pyroglutamic acid (N-terminus) | unmodified |
| | | deamidation to Glutamic acid | unmodified |
| | | isopeptide bond formation to a lysine | unmodified |
| Glycine, G | hydrogen | N-Myristoylation (N-terminus) | unmodified + modified |
| | | N-acetylation (N-terminus) | unmodified + modified |
| Histidine, H | imidazole | Phosphorylation | unmodified |

(continued)

| Amino Acid Type | R-group | PTM | Labeling |
|---|---|---|---|
| Lysine, K | ε-primary amino group | acetylation | unmodified |
| | | Ubiquitination | unmodified |
| | | SUMOylation | unmodified |
| | | methylation | unmodified |
| | | isopeptide bond formation to a glutamine | unmodified |
| | | hydroxylation | unmodified |
| Methionine, M | S-methyl thioether | N-acetylation (N-terminus) | unmodified + modified |
| | | N-linked Ubiquitination | unmodified |
| | | oxidation to sulfoxide or sulfone | unmodified |
| Proline, P | pyrrolidine | hydroxylation | unmodified |
| Serine, S | hydroxymethyl | Phosphorylation | unmodified |
| | | O-linked glycosylation | unmodified |
| | | N-acetylation (N-terminus) | unmodified + modified |
| Threonine, T | hydroxyl | Phosphorylation | unmodified |
| | | O-linked glycosylation | unmodified |
| | | N-acetylation (N-terminus) | unmodified + modified |
| Tryptophan, W | indole | mono- or di-oxidation | unmodified + modified |
| | | formation of Kynurenine | unmodified |
| Tyrosine, Y | phenol | sulfation | unmodified + modified |
| | | phosphorylation | unmodified + modified |
| Valine, V | isopropyl | N-acetylation (N-terminus) | unmodified + modified |

In some embodiments, if the user wishes to, the user can select whether to label only unmodified, and/or unmodified + modified versions of an amino acid type by transforming the modified amino acids of an amino acid type (e.g. by a chemical modification) into the unmodified amino acids to enable detection of both the modified and unmodified amino acids of an amino acid type. For example, when a combination of both the modified and unmodified amino acids of cysteine are being labelled , the modified amino acids ($C_D$) are first reduced to become unmodified cysteine amino acids ($C_R$) and all of the unmodified amino acids (which includes the newly reduced modified amino acids) are then labelled. The amino acids of the amino acid type cysteine (C) can undergo reversible post-translational modification (PTM). Specifically, the oxidation of cysteine amino acids into a disulphide bond during PTM is reversible. As another example, glycosylated (modified) serine, threonine, or asparagine residues can be converted to unmodified serine, thereonine, or asparagine residues by raising the pH of the sample solution, for example to pH 10.5. Glycosylation of serine, threonine, and asparagine residues is also reversible.

Cysteine disulphide ($C_D$) are modified cysteine amino acids. Unmodified cysteine amino acids are reduced cysteine ($C_R$). In some embodiments, the term cysteine (C) refers to the unmodified amino acids, i.e. reduced cysteine ($C_R$). In some embodiments, the term cysteine (C) refers to the modified amino acids, i.e. cysteine disulphide ($C_D$). In some embodiments, the term cysteine refers to both the unmodified amino acids ($C_R$) and the modified amino acids ($C_D$). In some embodiments, both the unmodified amino acids ($C_R$) and the modified amino acids ($C_D$) can both be labelled separately as part of the methods of the invention. The modified amino acids can be an amino acid type and/or the unmodified amino acids can be an amino acid type. The combination of the modified amino acids and the unmodified amino acids can also be an amino acid type.

In some embodiments, the term cysteine (C) refers to the combination unmodified cysteine amino acids, i.e. reduced cysteine ($C_R$) and modified cysteine amino acids, i.e. cysteine disulphide ($C_D$), when the modified cysteine amino acids, i.e. cysteine disulphide ($C_D$) has been reduced, and all of the unmodified amino acids (which includes the newly reduced modified amino acids) are then labelled.In some embodiments, the term cysteine refers to both unmodified amino

acids ($C_R$) being labelled, and the combination of modified and unmodified amino acids when the modified amino acids have been reduced..

Unmodified amino acids of cysteine, i.e. reduced cysteine ($C_R$) and/or modified amino acids of cysteine, i.e. cysteine disulphide ($C_D$) and/or the combination of modified and unmodified amino acids of cysteine are a subset of the amino acid type cysteine (C). The unmodified amino acids of cysteine, i.e. reduced cysteine ($C_R$) and the combination of modified and unmodified cysteine, once the modified cysteine has been reduced can both be labelled and provide different measurements of the label. For example, both the unmodified amino acids $C_R$ and the combination of $C_R$ and $C_D$ can both be labelled with a fluorogenic dye and provide a different fluorescence intensity. Therefore, the invention encompasses reduced cysteine ($C_R$), cysteine disulphide ($C_D$) and/or the combination of modified and unmodified cysteine amino acids of the amino acid type cysteine (C). Any reference to the amino acid type cysteine (C) encompasses reduced cysteine ($C_R$), cysteine disulphide ($C_D$) and/or the combination of modified and unmodified cysteine amino acids. Preferably, any reference to the amino acid type cysteine (C) encompasses reduced cysteine ($C_R$) and/or the combination of modified and unmodified cysteine ($C_T$). Preferably, reduced cysteine ($C_R$) and/or the combination of modified and unmodified cysteine are labelled in the sample.

Any other amino acid types with a distinct R-group which can be labelled can equally be used as part of the invention. For example, the two or more amino acid types encompassed by the invention also includes synthetic amino acid types. Synthetic amino acid types are non-proteinogenic amino acids that occur naturally, or are chemically synthesized. Synthetic amino acid types encompassed by the invention include amino acid types which contain the functional groups azide, alkyne, alkene, cyclooctyne, diene, acyl, iodo, boronic acid, diazirine, cyclooctene, epoxide, cyclopropane, biotin, dienophile, sulfonic acid, sulfinic acid, biotin, oxime, nitrone, norbornene, tetrazene, tetrazole, quadricyclane, electron poor pi systems, electron rich pi systems, halogen, NHS ester, maleimide, and/or diazo and any combination thereof. These functional groups are incorporated in place of the natural functional groups. In addition, synthetic amino acid types encompassed by the invention also include amino acid types with synthetic substituents appended or attached to the natural functional groups of an amino acid type. For example, the invention encompasses a tryptophan amino acid which has been synthetically modified to contain a norbornene on its indole ring. In some embodiments, when the synthetic substituents are appended or attached to the natural functional groups of an amino acid type, this incorporation has taken place prior to the labelling reactions disclosed herein.

## Labelling two or more amino acid types

The amino acids of two or more amino acid types are labelled in the sample.

In some embodiments, the labelling reactions are specific for each amino acid type. All amino acids within every amino acid type are contained within intact protein molecules. This allows reaction with exclusively the amino acid types of interest within intact protein chains, without requiring hydrolysis of the protein chain into individual amino acids or proteolytic digestion of the protein chain into fragments containing only one or a fraction of amino acid types contained within the intact protein chain. This is similar to how an antibody reacts only with a protein of interest, even though other proteins not of interest are also present within the solution. Because of the complementary chemical reactivity of the labels and the amino acid types, the labels react exclusively with the amino acid type of interest. In some embodiments, each label reacts with only one amino acid type. In some embodiments, each label reacts with one or two amino acid types. In some embodiments, each label reacts with one, two or three amino acid types. For example, the label o-maleimide-BODIPY is specific for the cysteine (C) amino acid type because only the thiol which defines the cysteine (C) R-group can react with the maleimide moiety. This is because thiols are "soft" nucleophiles and react preferentially with "soft" electrophiles such as maleimide.

In some embodiments, each amino acid type has a distinct label for identification. For example, if 5 amino acid types are labelled, then there are 5 different labels. If 2 amino acid types are labelled, then there are 2 different labels. For example, the amino acids of the amino acid type K are labelled with a first label, and the amino acids of the amino acid type W are labelled with a second label, which is distinct from the first label.

In some embodiments, 2 amino acid types are labelled. In some embodiments, 3 amino acid types are labelled. In some embodiments, 4 amino acid types are labelled. In some embodiments, 5 amino acid types are labelled. In some embodiments, 6 amino acid types are labelled. In some embodiments, 7 amino acid types are labelled. In some embodiments, 8 amino acid types are labelled. In some embodiments, 9 amino acid types are labelled. In some embodiments, 10 amino acid types are labelled. In some embodiments, 11 amino acid types are labelled. In some embodiments, 12 amino acid types are labelled. In some embodiments, 13 amino acid types are labelled. In some embodiments, 14 amino acid types are labelled. In some embodiments, 15 amino acid types are labelled. In some embodiments, 16 amino acid types are labelled. In some embodiments, 17 amino acid types are labelled. In some embodiments, 18 amino acid types are labelled. In some embodiments, 19 amino acid types are labelled. In some embodiments, 20 amino acid types are labelled. In some embodiments, 21 amino acid types are labelled. In some embodiments, 22 amino acid types are labelled. In some embodiments, 23 amino acid types are labelled. In some

embodiments, 24 amino acid types are labelled. In some embodiments, 25 amino acid types are labelled. In some embodiments, 26 amino acid types are labelled. In some embodiments, 27 amino acid types are labelled. In some embodiments, 28 amino acid types are labelled. In some embodiments, 29 amino acid types are labelled. In some embodiments, 30 amino acid types are labelled. In some embodiments, 31 amino acid types are labelled. In some embodiments, 32 amino acid types are labelled. In some embodiments, 33 amino acid types are labelled. In some embodiments, 34 amino acid types are labelled. In some embodiments, 35 amino acid types are labelled. In some embodiments, 36 amino acid types are labelled. In some embodiments, 37 amino acid types are labelled. In some embodiments, 38 amino acid types are labelled. In some embodiments, 39 amino acid types are labelled. In some embodiments, 40 amino acid types are labelled. In some embodiments, 2, 3, 4 or 5 amino acid types are labelled. In some embodiments, 4 or 5 amino acid types are labelled. In some embodiments, 3 or 4 amino acid types are labelled. In some embodiments, 2 amino acid types are labelled.

In some embodiments, the 2 amino acid types labelled are selected from: tryptophan (W), cysteine (C), tyrosine (Y) or lysine (K). In some embodiments, the two amino acid types are leucine (L) and serine (S). In some embodiments, the two amino acid types are leucine (L) and lysine (K). In some embodiments, the two amino acid types are leucine (L) and glutamic acid (E). In some embodiments, the two acid types are glycine (G) and leucine (L). In some embodiments, the two amino acid types are alanine (A) and leucine (L). In some embodiments, the two amino acid types are aspartic acid (D) and leucine (L). In some embodiments, the two amino acid types are leucine (L) and proline (P). In some embodiments, the two amino acid types are leucine (L) and valine (V). In some embodiments, the two amino acid types are lysine (K) and serine (S). In some embodiments, the two amino acid types are glutamic acid (E) and leucine (L). In some embodiments, the two amino acids types are alanine (A) and arginine (R). In some embodiments, the two amino acids are alanine (A) and glutamic acid (E). In some embodiments, the two amino acids are alanine (A) and glycine (G). In some embodiments, the 3 amino acid types labelled are selected from: tryptophan (W), cysteine (C), tyrosine (Y) or lysine (K). In some embodiments, the 3 amino acid types labelled are: tryptophan (W), cysteine (C) and lysine (K). In some embodiments, the 3 amino acid types labelled are: lysine (K), tryptophan (W) and tyrosine (Y). In some embodiments, the 3 amino acid types labelled are: tryptophan (W), tyrosine (Y) and cysteine (C). In some embodiments, the 3 amino acid types labelled are: tryptophan (W), tyrosine (Y) and lysine (K). In some embodiments, the 3 amino acid types labelled are: cysteine (C), tryptophan (W) and tyrosine (Y). In some embodiments, the 3 amino acid types labelled are: asparagine (R), glutamic acid (E) and glycine (G). In some embodiments, the 3 amino acid types labelled are: alanine (A), leucine (L) and serine (S). In some embodiments, the 3 amino acid types labelled are: asparagine (A), glutamic acid (E) and leucine (L). In some embodiments, the 3 amino acid types labelled are: 3 amino acid types labelled are: alanine (A), aspartic acid (D) and leucine (L). In some embodiments, the 3 amino acid types labelled are: the 3 amino acid types labelled are: alanine (A), leucine (L) and proline (P). In some embodiments, the 3 amino acid types labelled are: alanine (A), glutamic acid (E) and leucine (L). In some embodiments, the 3 amino acid types labelled are: leucine (L), serine (S) and valine (S). In some embodiments, the 3 amino acid types labelled are: glutamic acid (E), isoleucine (I) and proline (P). In some embodiments, the 3 amino acid types labelled are: glutamic acid (E), glycine (G) and valine (V). In some embodiments, the 3 amino acid types labelled are: arginine (R), serine (S) and valine (V). In some embodiments, the 3 amino acid types labelled are: alanine (A), leucine (L) and lysine (K). In some embodiments, the 3 amino acid types labelled are: alanine (A), arginine (R) and leucine (L). In some embodiments, the 3 amino acid types labelled are: alanine (A), leucine (L) and valine (V).

In some embodiments, the 4 amino acid types labelled are: tryptophan (W), tyrosine (Y) and lysine (K) and cysteine (C), wherein the combination of modified and unmodified amino acids of cysteine are labelled. In some embodiments, the 4 amino acid types labelled are: tryptophan (W), cysteine (C), tyrosine (Y) and lysine (K), wherein reduced cysteine ($C_R$) is labelled. In some embodiments, the 4 amino acid types labelled are: tryptophan (W), cysteine (C), tyrosine (Y) and lysine (K). In some embodiments, the 4 amino acid types labelled are: cysteine (C), arginine (R), histidine (H) and aspartic acid (D). In some embodiments, the 4 amino acid types labelled are: cysteine (C), arginine (R), histidine (H) and glutamic acid (E). In some embodiments, the 4 amino acid types labelled are: cysteine (C), arginine (R), histidine (H) and Glutamine (Q). In some embodiments, the 4 amino acid types labelled are: cysteine (C), arginine (R), tryptophan (W) and aspartic acid (D). In some embodiments, the 4 amino acid types labelled are: lysine (K), arginine (R), histidine (H) and aspartic acid (D). In some embodiments, the 4 amino acid types labelled are: lysine (K), tryptophan (W), arginine (R) and glutamic acid (E). In some embodiments, the 4 amino acid types labelled are: tyrosine (Y), lysine (K), cysteine (C) and aspartic acid (D). In some embodiments, the 4 amino acid types labelled are: tyrosine (Y), lysine (K), cysteine (C) and glutamic acid (E). In some embodiments, the 4 amino acid types labelled are: proline (P), cysteine (C), arginine (R), and glutamic acid (E). In some embodiments, the 4 amino acid types labelled are: proline (P), cysteine (C), arginine (R) and aspartic acid (D). In some embodiments, the 4 amino acid types labelled are: cysteine (C), asparagine (B), arginine (R) and aspartic acid (D). In some embodiments, the 4 amino acid types labelled are: cysteine (C), asparagine (B), arginine (R) and glutamic acid (E). In some embodiments, the 4 amino acid types labelled are: lysine (K), asparagine (B), tryptophan (W) and cysteine (C). In some embodiments, the 4 amino acid types labelled are: arginine (R), histidine (H), proline (P) and aspartic acid (D). In some embodiments, the 4 amino acid types labelled are: arginine (R), lysine (K), cysteine (C) and aspartic acid (D). In some embodiments, the 4 amino acid types labelled are: arginine (R), lysine (K), cysteine (C) and

glutamic acid (E). In some embodiments, the 4 amino acid types labelled are: arginine (R), lysine (K), cysteine (C) and tryptophan (W). In some embodiments, the 4 amino acid types labelled are: arginine (R), lysine (K), cysteine (C) and tyrosine (Y). In some embodiments, the 4 amino acid types labelled are: arginine (R), lysine (K), histidine (H) and tryptophan (W). In some embodiments, the 4 amino acid types labelled are: arginine (R), lysine (K), histidine (H) and cysteine (C). In some embodiments, the 4 amino acid types labelled are: arginine (R), lysine (K), histidine (H) and tyrosine (Y). In some embodiments, the 4 amino acid types labelled are: arginine (R), cysteine (C), tryptophan (W) and tyrosine (Y). In some embodiments, the 4 amino acid types labelled are: arginine (R), cysteine (C), tryptophan (W) and proline (P). In **some embodiments,** the 4 amino acid types labelled are: Glutamine (Q), leucine (L), lysine (K) and valine (V). In some embodiments, the 4 amino acid types labelled are: arginine (R), isoleucine (I), leucine (L) and serine (S). In some embodiments, the 4 amino acid types labelled are: alanine (A), asparagine (N), glutamic acid (E), and serine (S).

Each amino acid type refers to the modified and/or unmodified amino acids of that amino acid type. Preferably, the amino acid cysteine (C) refers to the unmodified amino acids ($C_R$) and/or the combination of the unmodified and the modified (cysteine disulphide) amino acids, once the modified amino acids have been reduced.

In some embodiments, the 5 amino acids types labelled are: tryptophan (W), cysteine (C), tyrosine (Y) and lysine (K), wherein both reduced cysteine ($C_R$), and the combination of modified ($C_D$) and unmodified ($C_R$) amino acids of cysteine are labelled. In some embodiments, the 5 amino acids types labelled are: arginine (R), glutamic acid (E), lysine (K), serine, and glutamine (Q). In some embodiments, the 5 amino acids types labelled are: arginine (R), aspartic acid (D), lysine (K), serine, and glutamine (Q). In some embodiments, the 5 amino acids types labelled are: arginine (R), glycine (G), lysine (K), serine, and glutamine (Q). In some embodiments, the 5 amino acids types labelled are: alanine (A), aspartic acid (D), glycine (G), serine, and arginine (R). In some embodiments, the 5 amino acids types labelled are: pyrrolysine (O), aspartic acid (D), glycine (G), serine, and arginine (R). In some embodiments, the 5 amino acids types labelled are: pyrrolysine (O), aspartic acid (D), selenocysteine (U), serine, and arginine (R). In some embodiments, the 5 amino acids types labelled are: pyrrolysine (O), aspartic acid (D), selenocysteine (U), lysine, and arginine (R).

In some embodiments, the two or more amino or more acid types can be labelled with the same label and the label is independently identified for each amino acid type. For example, the amino acids of the amino acid type W are labelled with the same label as the amino acids of the amino acid type Y and the label of the amino acid type W is independently identified to the label of the amino acid type Y. In some embodiments, when two amino acid types are labelled with the same label, the parameters for detecting the label are distinct. For example, the label for one amino acid type is deconvoluted from the label for a second amino acid type. For example, the amino acid types of tryptophan (W) and tyrosine (Y) can both be labelled with the same fluorescent label, but the fluorescence intensity of the tryptophan (W) label is deconvoluted from the fluorescence intensity of the tyrosine (Y) label. In some embodiments, the amino acid types of tryptophan (W) and tyrosine (Y) are both labelled with the same fluorogenic dye, but the excitation and emission wavelengths for measuring the signal from the fluorogenic dye for tryptophan (W) are different than the excitation and emission wavelength parameters for measuring the signal from the fluorogenic dye for tyrosine (Y). In some embodiments, the amino acid types of tryptophan (W) and tyrosine (Y) are both labelled with the same fluorogenic dye, but the excitation and emission wavelengths for measuring the signal from the fluorogenic dye for tryptophan (W) are different from the excitation and emission wavelengths for measuring the signal from the fluorogenic dye for tyrosine (Y) and tryptophan (W). In some embodiments, the tyrosine (Y) signal is measured from the total tryptophan (W) and tyrosine (Y) signal minus the tryptophan signal (W).

In some embodiments, the two or more amino acid types can be labelled (e.g. reacted) with the same label but the labelling (e.g. reactions) are performed under different conditions. In some embodiments, a multi-step labelling process allows the same label to react specifically with only one amino acid type. For example, methionine (M) and phenylalanine (F) amino acid types can be reacted with the same label, a dye bearing an azide reactive group. The labelling reaction involves Copper(I)-catalyzed azide-alkyne cycloaddition (CuAAC), also known as "click chemistry". The first step of the labelling reaction for the methionine (M) or phenylalanine (F) amino acid types is installation of an alkyne group onto the methionine (M) or phenylalanine (F) R-group that subsequently reacts with the azide on the dye during the second step of the labelling reaction. This first step is performed under conditions specific for reaction with only the methionine (M) amino acid type or only the phenylalanine (F) amino acid type. In this way, the same label (e.g. dye) can react specifically with more than one amino acid type, such that only the desired amino acid type is labelled under the conditions of the reaction.

In some embodiments, all of the two or more amino acid types which are labelled are labelled within the whole sample. In some embodiments, the sample is not separated into multiple individual fractions prior to the labelling reaction. For example, a urine sample is provided and the amino acid types W, Y and K are all labelled in the urine sample, without separating the sample into multiple individual fractions, and labelling W, Y and K separately in separate fractions. For example, a single protein molecule will have all of the two or more amino acid types labelled within the molecule. In some embodiments, all of the amino acid types being labelled are labelled in one fraction. In this embodiment, the label of each amino acid type is selected to be specific to one amino acid type so that it does not cross react with the other amino acid type. In some embodiments, the selection of the label is governed by the chemistry of the amino acid type to be modified. For example, when lysine and tryptophan are labelled in the same fraction, the labelling chemistries do not interfere with

one another, and the signal of the dye linked to tryptophan is separable from the signal of the dye linked to lysine, i.e. different excitation and emission wavelengths in the case of fluorescence intensity.

In some embodiments, the sample is separated into multiple fractions prior to the labelling reaction. Because the amino acids of each amino acid type are contained within intact protein molecules which are not hydrolysed or digested, one protein molecule contains many amino acid types, and therefore one fraction contains many amino acid types. When the sample is separated into multiple fractions, different labelling reactions are performed in each fraction which label specifically the amino acid type of interest. In some embodiments, each fraction contains an equal volume. In this embodiment, each fraction is labelled. For example, the sample is separated into two fractions before labelling and 4 amino acid types are being labelled; wherein two amino acid types are labelled in one fraction and two alternative amino acid types are labelled in the second fraction. For example, the 4 amino acid types in the sample being labelled are W, K, Y and C, wherein C is the combination of $C_D$ and $C_R$. The sample is separated into two fractions before labelling; in the first fraction, the amino acid types (W) and lysine (K) are labelled with using labels specific for the (W) and (K) amino acid types, and in the second fraction the amino acid types cysteine (C) and tyrosine (Y) are labelled using labels specific for the (C) and (Y) amino acid types. In another example, the sample is separated into four fractions before labelling. 4 amino acid types are being labelled; with one amino acid type being labelled in each fraction. For example, the 4 amino acid types in the sample being labelled are W, K, Y and C. The sample is separated into four fractions before labelling; the amino acid type tryptophan (W) is labelled in the first fraction, the amino acid type lysine (K) is labelled in the second fraction, the amino acid type cysteine (C) is labelled in the third fraction, and the amino acid type tyrosine (Y) is labelled in the fourth fraction. In some embodiments, the number of fractions is equal to the number of amino acid types being labelled, and one amino acid type is labelled per fraction. In some embodiments, the number of fractions is less than the number of amino acid types being labelled, and more than one amino acid type is labelled per fraction. The presence and/or concentration and/or amount of one or more proteins, peptides, oligopeptides, polypeptides, protein complexes, subproteomes, or proteomes of interest, or, a mixture of proteins, peptides, polypeptides, oligopeptides, subproteomes or proteomes of interest within a sample is determined, for each fraction, based on the measured label of each fraction.

In some embodiments, if two amino acid types have the same label, they are labelled and measured in different fractions. For example, in some embodiments, the amino acid type W and Y are labelled and measured in different fractions. In some embodiments, if the label of a first amino acid type is predicted to cross react with the label of a second amino acid type, then the first and second amino acid types are separated into separate fractions. The first fraction is reacted with a label that is specific for the first amino acid type within the sample, and the second fraction is reacted with a label that is specific for the second amino acid type within the sample. This avoids cross-reaction of the label.

In some embodiments, the two or more amino acid types to be labelled are separated into a fraction with a fluorogenic dye which does not cross-react with another fluorogenic dye or amino acid type in the sample.

In some embodiments, all of the amino acids, i.e. every amino acid, of two or more amino acid types in the sample are labelled. In some embodiments, every amino acid (i.e. all amino acids) of each of two or more amino acid types in the sample is labelled. For example, if the amino acid type tryptophan was being labelled, then every tryptophan amino acid present in the sample is labelled. In some embodiments, every amino acid (i.e. all amino acids) of each of two or more amino acid types in the sample is labelled. For example, if the two or more amino acid types to be labelled are tryptophan (W) and lysine (K), then every, i.e. all, tryptophan (W) amino acids in the sample are labelled and every, i.e. all, lysine (K) amino acids in the sample are labelled. In another example, if the two or more amino acid types to be labelled are tryptophan (W), lysine (K) and tyrosine (Y), then every, i.e. all, tryptophan (W) amino acids in the sample are labelled, every, i.e. all, lysine (K) amino acids in the sample are labelled and every, i.e. all, tyrosine (Y) amino acids in the sample are labelled.

In some embodiments, a proportion of the amino acids (i.e. not all amino acids) of two or more amino acid types in the sample are labelled. In some embodiments, a proportion of amino acids (i.e. not all amino acids) of each of two or more amino acid types in the sample are labelled. For example, if the amino acid type tryptophan was being labelled, then a proportion of the tryptophan amino acids present in the sample are labelled. For example, if the two or more amino acid types to be labelled are tryptophan (W) and lysine (K), then a proportion of tryptophan (W) amino acids in the sample is labelled and a proportion of lysine (K) amino acids in the sample is labelled. If the two or more amino acid types to be labelled are tryptophan (W), lysine (K) and tyrosine (Y), then a proportion of tryptophan (W) amino acids in the sample is labelled, a proportion of lysine (K) amino acids in the sample is labelled and a proportion of tyrosine (Y) amino acids in the sample is labelled. Preferably, about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78% or 79%, 80%, 81%, 82%, 83%, 84%, 85%, 86%, 87%, 89%, 90%, 91%, 92%, 93%, 94%, 95%, 96%, 97%, 98% or 99% of amino acids of each of two or more amino acid types are labelled within the sample. In some embodiments, the proportion of the amino acid of an amino acid type labelled within the sample is determined using mass spectrometry. In some embodiments, a proportion of the amino acids (i.e. not all amino acids) of two or more amino acid types in the proteome or subproteome contained within the sample are labelled.

In some embodiments, every (i.e. all) of the amino acids of one amino acid type are labelled and a proportion of

the amino acids of another amino acid type are labelled. For example, if the two or more amino acid types to be labelled are tryptophan (W) and lysine (K), then all of the tryptophan (W) amino acids in the sample and 90% of the lysine (K) amino acids in the sample are labelled. Alternatively, 90% of the tryptophan (W) amino acids in the sample and all of the lysine (K) amino acids in are labelled.

In some embodiments, the R-group of amino acids within two or more amino acid types is labelled within the sample. The R-group of each amino acid type is unique for each amino acid type. For example, the R-group of tryptophan (W) is distinct to the R-group of lysine (K). The R-group specific to each amino acid type is provided in Table 2. Two or more amino acid types in the sample are labelled. In some embodiments, every amino acid (i.e. all the amino acids) of an amino acid type selected to be labelled is labelled. In some embodiments, the R-group of every amino acid (i.e. all the amino acids) of an amino acid type are labelled. In some embodiments, a proportion (i.e. not every amino acid) of an amino acid type is labelled. In some embodiments, the R-group of a proportion of the amino acids (i.e. not all of the amino acids) of an amino acid type is labelled. In some embodiments, every amino acid (i.e. all the amino acids) of an amino acid type are labelled, and a proportion (i.e. not all of the amino acids) of a second amino acid type are labelled. In some embodiments, the R-group of every amino acid (i.e. all the amino acids) of a first amino acid type are labelled and the R-group of a proportion of the amino acids (i.e. not all of the amino acids) of a second amino acid type are labelled.

Preferably, the R-groups for each of two or more of the amino acid types selected from: W, C, Y or K are labelled. Preferably, the R-group labelled for C is the R-group of reduced cysteine ($C_R$). Preferably, the R group being labelled for C is the R-group of the combination of $C_D$ and $C_R$, after $C_D$ has been reduced. Preferably, both the R-groups for ($C_R$) and the combination of $C_D$ and $C_R$, after $C_D$ has been reduced, within the amino acid type cysteine (C) are labelled within the sample.

In a preferred embodiment, two or more amino acid types are labelled, and the R-groups of each of the amino acid types are labelled in the sample (i.e. two or more types of R-groups are labelled). The two or more amino acid types of R-groups corresponds to the two or more amino acid types. For example, when tryptophan and lysine are the two amino acid types being labelled, the R-group for tryptophan and the R-group for lysine are labelled in the sample. In some embodiments, the R-groups of each of 2, 3, 4, 5, 6, 7, 8, 9, 10, 11, 12, 13, 14, 15, 16, 17, 18, 19, 20, 21 or 22 amino acid types are labelled. In some embodiments, the 3 amino acid R-groups being labelled are the R-groups for each of the 3 amino acid types selected from: C, W and Y, wherein C is the unmodified C amino acids ($C_R$) and the combination of $C_D$ and $C_R$, after $C_D$ has been reduced. In some embodiments, the 3 amino acid R-groups being labelled are the R-groups for each of the 3 amino acid types selected from: C W and K, wherein C is the unmodified C amino acids ($C_R$) and the combination of $C_D$ and $C_R$, after $C_D$ has been reduced. In some embodiments, the 3 amino acid R-groups being labelled are the R-groups for each of the 3 amino acid types selected from: C, K and Y, wherein C is the unmodified C amino acids ($C_R$) and the combination of $C_D$ and $C_R$, after $C_D$ has been reduced. In some embodiments, the 4 amino acid R-groups being labelled are the R-groups for each of the 4 or more amino acid types selected from: C, W, K and Y, wherein C is the combination of $C_D$ and $C_R$, after $C_D$ has been reduced. In some embodiments, the 4 amino acid R-groups being labelled are the R-groups for each of the 4 amino acid types selected from: $C_R$, K, W and Y. In some embodiments, the 4 amino acid R-groups being labelled are the R-groups for each of the 4 amino acid types selected from: C K, W and Y, , wherein C is the unmodified C amino acids ($C_R$) and the combination of $C_D$ and $C_R$, after $C_D$ has been reduced.

In some embodiments, one amino acid R-group is labelled for each amino acid type. For example, the indole R-group on each tryptophan amino acid is labelled for the amino acid type tryptophan. In another example, the ε-amino R-group on each lysine amino acid is labelled for the amino acid type lysine. The R-group for each amino acid type is outlined in Table 2.

**Table 2: R-group for each amino acid type**

| Amino acid type | Modified or unmodified amino acids of the amino acid type | R-group labelled |
|---|---|---|
| Alanine (A) | Unmodified | Methyl |
| arginine (R) | Unmodified | aliphatic guanidino group; partial primary amine character or equal primary amine character |
| N-Glycosylated arginine (GR) | Modified | Carbohydrate glycoside bonded to guanidino amine |
| asparagine (N) | Unmodified | β-carboxamide |
| N-Glycosylated asparagine (GN) | Modified | Carbohydrate glycoside bonded to β-carboxamide amine |
| Aspartic acid (D) | Unmodified | β-carboxylic acid |

(continued)

| Amino acid type | Modified or unmodified amino acids of the amino acid type | R-group labelled |
|---|---|---|
| Cysteine (C) | Modified ($C_D$) | Oxidised (disulphide bonded) thiol |
| Cysteine (C) | Unmodified ($C_R$) | Reduced thiol |
| Cysteine (C) | Unmodified and modified, after modified have been reduced | Reduced thiol |
| glutamic acid (E) | Unmodified | γ-carboxylic acid |
| Glutamine (Q) | Unmodified | γ-carboxamide |
| glycine (G) | Unmodified | Hydrogen |
| histidine (H) | Unmodified | Imidazole |
| Isoleucine (I) | Unmodified | sec-butyl |
| Leucine (L) | Unmodified | Isobutyl |
| Lysine (K) | Unmodified | ε-primary amino group |
| N6-(pyridoxal phosphate) lysine | Modified | Pyridoxyal phosphate aldimine |
| Methionine (M) | Unmodified | S-methyl thioether |
| Phenylalanine (F) | Unmodified | Benzyl |
| Proline (P) | Unmodified | Pyrrolidine |
| 4-hydroxyproline (HP) | Modified | 4-hydroxypyrrolidine |
| serine (S) | Unmodified | Hydroxymethyl |
| Phosphoserine (PS) | Modified | Phospho methyl ester |
| Threonine (T) | Unmodified | Hydroxyl |
| Phosphothreonine (PT) | Modified | Phosphoester |
| Tryptophan (W) | Unmodified | Indole |
| Tyrosine (Y) | Unmodified | Phenol |
| Phosphothrosine (PY) | Modified | Phosphophenol |
| valine (V) | Unmodified | Isopropyl |

In a preferred embodiment, the two or more amino acid types within the sample are labelled fluorescently, isotopically, or using mass tags. Alternatively, the two or more amino acid types within the sample are labelled with nucleotides. In some embodiments, the R-group of each amino acid type is labelled fluorescently, isotopically, or using mass tags. In some embodiments, the R-group of each amino acid is labelled with nucleotides.

In some embodiments, one amino acid is labelled with one type of label and another amino acid type is labelled with another type of label. For example, one amino acid type is labelled with a fluorescent label and a second amino acid type is labelled with a tandem mass tag.

In some embodiments, the label is a fluorescent label. In some embodiments, the fluorescent label is a fluorescent dye, fluorescent tag, fluorescent probe, or fluorescent protein. In some embodiments, the fluorescent label includes a fluorophore. In some embodiments, the fluorophore is selected from the group consisting of: Hydroxycoumarin, Aminocoumarin, Methoxycoumarin, Cascade Blue, Pacific Blue, Pacific Orange, Lucifer yellow, NBD, R-Phycoerythrin (PE), PE-Cy5 conjugates, PE-Cy7 conjugates, Red 613, PerCP, TruRed, FluorX, BODIPY-FL, G-Dye100, G-Dye200, G-Dye300, G-Dye400, Cy2, Cy3, Cy3B, Cy3.5, Cy5, Cy5.5, Cy7, TRITC, X-Rhodamine, Lissamine Rhodamine B, Texas Red, Allophycocyanin (APC), APC-Cy7 conjugates, DAPI, Hoechst 33258, SYTOX Blue, Chromomycin A3, Mithramycin, YOYO-1, ATTO 390, ATTO 425, ATTO 465, ATTO 488, ATTO 495, ATTO 514, ATTO 520, ATTO 532, ATTO Rho6G, ATTO 550, ATTO 565, ATTO Rho3B, ATTO Rho11, ATTO Rho12, ATTO Thio12, ATTO Rho101, ATTO 590, ATTO Rho13, ATTO 594, ATTO 610, ATTO Rho14, ATTO 633, ATTO 647, ATTO 647N, ATTO 655, ATTO Oxa12, ATTO 665, ATTO Oxa12, ATTO 665, ATTO 680, ATTO 700, ATTO 725, ATTO 740, Brilliant Violet 421, Brilliant Violet 510, Brilliant Violet 570, Brilliant Violet 605, Brilliant Violet 650, Brilliant Violet 711, Brilliant Violet 750, Brilliant Violet 785, TM-BDP, KFL-1, KFL-2, KFL-3,

KFL-4, Super Bright 436, Super Bright 600, Super Bright 645, Super Bright 702, Super Bright 780, Alexa Flour 350, Alexa Flour 405, Alexa Flour 488, Alexa Flour 532, Alexa Flour 546, Alexa Flour 555, Alexa Flour 568, Alexa Flour 594, Alexa Flour 647, Alexa Flour 680, Alexa Flour 850, Coumarin, Pacific Green, Oregon Green, Flourescein (FITC), PE-Cyanine7, PerCP-Cyanine5.5, Tetramethylrhodamine (TRITC), eFlour 450, eFlour506, eFlour660, PE-eFlour 610, PerCP-eFlour 710, APC-eFlour 780, Super Bright 436, Super Bright 600, Super Bright 645, Super Bright 702, Super Bright 780, DAPI, SYTOX Green, SYTO 9, TO-PRO-3, Qdot 525, Qdot 565, Qdot 605, Qdot 655, Qdot 705, Qdot 800, R-Phycoerythrin (R-PE), , VioBlue, VioGreen, VioBright 515, Vio 515, VioBright FITC, PE, PE-Vio 615, PerCP, PerCP-Vio 700, PE-Vio 770, APC, APC-Vio 770, 1,8-Naphthalimides, Thiazole Orange, CyTRAK Orange, LDS 751, 7-AAD, SYTOX Orange, TOTO-3, TO-PRO-3, DRAQ5, DRAQ7, Indo-1, Fluo-3, Fluo-4, DCFH, DHR, SNARF, CFP, GFP (emGFP), RFP (tagRFP), GFP (Y66H mutation), GFP (Y66F mutation), EBFP, EBFP2, Azurite, GFPuv, T-Sapphire, mCerulean, mCerulean3 mCFP, mTurquoise2, ECFP, CyPet, GFP (Y66W mutation), mKeima-Red, TagCFP, AmCyan1, mTFP1, GFP (S65A mutation), Midoriishi Cyan, Wild Type GFP, GFP (S65C mutation), TurboGFP, TagGFP, GFP (S65L mutation), Emerald, GFP (S65T mutation), EGFP, Azami Green, ZsGreen1, TagYFP, EYFP, Topaz, Venus, mCitrine, YPet, TurboYFP, ZsYellow1, Kusabira Orange, mOrange, Allophycocyanin (APC), mKO, TurboRFP, tdTomato, TagRFP, DsRed monomer, DsRed2 ("RFP"), mStrawberry, TurboFP602, AsRed2, mRFP1, J-Red, R-phycoerythrin (RPE), B-phycoerythrin (BPE), mCherry, HcRed1, Katusha, P3, Peridinin Chlorophyll (PerCP), mKate (TagFP635), TurboFP635, mPlum or mRaspberry.

In some embodiments, the fluorescent tag or fluorescent label is not a fluorogenic dye. In some embodiments, the fluorescent tag or fluorescent label also includes a reactive group that is specific for the R-group which defines an amino acid type. In this way, the fluorescent label targets a particular amino acid type. In some embodiments, labelling an amino acid type of interest is covalently labelling an amino acid type of interest. In some embodiments, the reactive group permits selective covalent labelling of the R-group of the amino acid type of interest. In some embodiments, the reactive group is selected from the group consisting of: NHS-ester, maleimide, alkyne, azide, bromide, chloride, fluoride, iodide, aryl bromide, aryl chloride, aryl fluoride, aryl iodide, diene, dienophile, olefin, tetrazine, cyclooctyne, biotin, streptavidin, isothiocyanate, active ester, sulfonyl chloride, dialdehyde, iodoacetamide, ethylenediamine, aminoacridone, hydrazide, carboxyl, or alkoxyamine. For example, it is appreciated by those skilled in the art that the electrophilic maleimide group selectively targets nucleophilic cysteine thiol residues. Therefore, any of the fluorophores listed above can be selected and coupled with a maleimide reactive group, to selectively label cysteine thiol resides. For example, cysteine thiol residues can be labelled with a fluorescent label comprising Super Bright 436 and a maleimide reactive group. As another example, it is appreciated by those skilled in the art that the labile NHS ester group selective targets the lysine primary amine R-group, and can undergo a covalent SN2 reaction with the lysine primary amine R-group. Therefore, the lysine residues can be labelled with the NHS-ester form of Cy5. These methods of labeling are appreciated by the skilled person and the indicated reactive forms of the fluorophores disclosed are commercially available.

In some embodiments, the fluorescent label is a fluorogenic dye which targets an amino acid type or a molecule which becomes fluorescent exclusively upon reaction with an amino acid type. Preferably, the fluorogenic dye becomes fluorescent exclusively after covalently reacting with specific amino acid types within the protein. In this case, there is no need to couple a fluorophore with a reactive group, because in the case of a fluorogenic dye or molecule which becomes fluorescent exclusively on reaction with an amino acid type, the selectivity for an amino acid type is already built into the chemical structure of the fluorogenic dye or molecule which becomes fluorescent exclusively upon reaction with an amino acid type. In some embodiments, the fluorogenic dye which targets an amino acid type or a molecule which becomes fluorescent exclusively upon reaction with an amino acid type is selected from the group consisting of: 4-Fluoro-7-sulfamoylbenzofurazan (ABD-F), 2,2,2-Trichloroethanol (TCE) and/or ortho-phthalaldehyde (OPA), or a mixture thereof. Preferably, the fluorogenic dye is selected for each amino acid type, or R-group in Table 2 and Table 3. However, this list is non-exhaustive and any other fluorogenic dye or molecule which becomes fluorescent upon reaction with an amino acid type known within the art can also be used. Those skilled in the art will appreciate that labelling with high quantum yield fluorogenic or non-fluorogenic labels can permit identification of very low concentrations of protein within the sample, such as at the single molecule level. This corresponds to protein concentrations between 1 pM and 1 nM.

In some embodiments, amino acid type is reacted with a molecule which becomes fluorescent after reaction with the amino acid type, or which shifts the fluorescence of an already fluorescent amino acid type into the visible spectrum. For example, in some embodiments, the molecule which becomes fluroescent after reaction with an amino acid type is a halo compound. In some embodiments, the halo compounds are trichloroacetic acid, chloroform, triflouroethanol, triflouroacetic acid, flouroform, tribromoethanol, tribromoacetic acid, bromoform, triiodoethanol, triiodoacetic acid or iodoform. In some embodiments, the amino acid types tryptophan (W) and/or tyrosine (Y) are labelled with Trichloroethanol trichloroethanol (TCE ), trichloroacetic acid (TCA), chloroform, trifluoroethanol (TFE), triflouroacetic acid (TFA), flouroform, tribromoethanol, tribromoacetic acid (TBA), bromoform, triiodoethanol (TIE), or triiodoacetic acid (TIA), iodoform, or, with 2-(2-(2-methoxyethoxy)ethoxy)ethyl (E)-2-diazo-4-phenylbut-3-enoate in the presence of Rh2(OAc) 4, tBuHNOH. In some embodiments, the amino acid type Y is labelled with trichloroethanol (TCE), or, installation of an aryl group ortho to the tyrosine hydroxyl groups using [RhCl(PPh3)3], R2P(OAr),Ar-Br,CsCO3.

The skilled person would readily understand how amino acids can be labelled.

In some embodiments, the label is selected based on a specific interaction with an amino acid type. For example, the label is a fluorogenic dye and is selected based on a specific interaction with an amino acid type where the dye only becomes fluorescent (i.e. its signal only becomes detectable) after it has reacted with the specific amino acid type. In some embodiments, the selection of the label is governed by the chemistry of the amino acid type to be modified. In some embodiments, for specific reaction with an amino acid type, there is a reactive group on an amino acid type and a reactive group on a label that react exclusively with one another. This is determined by the specific chemical reactivity of the R-group on an amino acid type and the reactive group on a label. For example, ABD-F contains a halogen at a labile position on an aromatic system and is susceptible to electrophilic aromatic substitution. There are several nucleophilic amino acid types (e.g. cysteine, lysine, histidine), but the cysteine amino acid type (C) is the strongest nucleophile because it is the most polarizable. Because the electron cloud is more polarizable, the activation energy for nucleophilic attack is reduced. Therefore, ABD-F reacts preferentially with cysteine (C) residues and does not react with other amino acid types, such as lysine, or histidine amino acid types, which would require a higher activation energy.

In some embodiments, the labelling reaction is a fluorogenic reaction. This means that fluorescence is generated exclusively after reaction with the amino acid type, such that there is not a need to purify the unreacted label from the sample.

In some embodiments, a fluorogenic reaction involves removing a group from a fluorophore that quenches reaction. For example, it is known that maleimide quenches flourophores when it is directly conjugated to fluorophores due to maleimide's low energy $n\pi^*$ state provides a non-radiative pathway for decay of the flourophore's excited state, and can also quench flourophores when it is joined to the fluorophore by a spacer group because photoinduced electron transfer (PET) to the C=C double bond can occur. For example, when maleimide is attached in the ortho position to the fluorescent dye BODIPY, maleimide quenches the fluorescence of the dye BODIPY. However, when o-maleimide BODIPY reacts with the thiol R-group of the cysteine (C) amino acid type, the C=C double bond becomes saturated and no longer quenches fluorescence, so the BODIPY label becomes emissive. Other quenching groups known in the art include azido, alkyne, phosphine, sydnone, tetrazine, or oxime and these can become unquenched after a fluorogenic click reaction, including copper-catalyzed/strain-promoted alkyne-azide cycloaddition (CuAAC/SPAAC), Staudinger ligation, copper-catalyzed/-strain-promoted sydnone-alkyne cycloaddition (CuSAC/SPSAC), inverse electron demand Diels-Alder reaction (iEDDA), or 1,3- dipolar cycloaddition.

In some embodiments, a fluorogenic reaction involves generating a fluorophore. An example of this type of fluorogenic reaction is the reaction of the lysine (K) amino acid type with ortho-pthalaldehyde. A second ring is formed, extending the electronic conjugation, and this larger delocalized pi system becomes fluorescent in the visible region of the spectrum. In some embodiments, a fluorogenic reaction involves changing the fluorescence properties of an existing fluorescent substrate. For example, the amino acid tryptophan which is intrinsically fluorescent undergoes a light-catalyzed radical reaction with trichloroethanol (TCE), that installs an alpha hydroxy ketone on the tryptophan indole ring, extending the conjugation and shifting the intrinsic fluorescence of tryptophan 100 nm to the red end of the spectrum.

To illustrate further how amino acid types can be specifically and fluorogenically labelled, a table of fluorescent dyes and reaction approaches is presented below for use with the invention, from which an appropriate label and reaction strategy can be chosen for each reaction type.

**Table 3: Fluorogenic labelling for each R-group of each amino acid type**

| **Amino acid type** | **R-group labelled** | **Fluorogenic Labelling** |
|---|---|---|
| Alanine (A) | Methyl | Palladium catalysed $C(sp_3)-H_3$ bond activation, $Pd(OAc)_2$ with 1-ethynyl-4-iodobenzene, to install alkyne followed by Cu(I) catalyzed azide-alkyne cycloaddition<br>(CuAAC) "click-chemistry" with 3-azido-2H-chromen-2-one.<br>$\lambda$ex = 365 nm, $\lambda$em = 478 nm |
| Arginine (R) | aliphatic guanidino group; partial primary amine character and/or an equal primary amine character | Dopachrome, pH 10.5, 20 mM dopachrome<br>$\lambda$ex = 380 nm, $\lambda$em = 480 nm |
| Asparagine (N) | $\beta$-carboxamide | 4-amino-3-formylphenyl nitrate<br>$\lambda$ex = 350 nm, $\lambda$em = 450 nm |

(continued)

| Amino acid type | R-group labelled | Fluorogenic Labelling |
|---|---|---|
| Aspartic acid (D) | β-carboxylic acid | 4-(diethylamino)-2-(pyridin-2-ylmethoxy)benzaldehyde appended BODIPY based probe<br>λex = 500 nm, λem = 510 nm |
| Cysteine (C) | Reduced thiol | 4-aminosulfonyl-7-fluoro-2,1,3-benzoxadiazole (ABD-F) at pH 10.5<br>OR<br>o-maleimide BODIPY<br>λex = 500 nm, λem = 510 nm<br>OR<br>ethyl (Z)-2-(6-(ethyl((3-(trifluoromethyl)phenyl)selanyl)amino)-3-(ethylimino)-2,7-dimethyl-3H-xanthen-9-yl)benzoate |
| Cysteine (C) | Reduced thiol | 4-aminosulfonyl-7-fluoro-2,1,3-benzoxadiazole (ABD-F) at pH 10.5<br>OR<br>o-maleimide BODIPY<br>λex = 500 nm, λem = 510 nm<br>OR<br>ethyl (Z)-2-(6-(ethyl((3-(trifluoromethyl)phenyl)selanyl)amino)-3-(ethylimino)-2,7-dimethyl-3H-xanthen-9-yl)benzoate after tris(2-carboxyethyl)phosphine (TCEP) |
| Glutamic acid (E) | γ-carboxylic acid | 4-(diethylamino)-2-(pyridin-2-ylmethoxy)benzaldehyde appended BODIPY based probe<br>λex = 500 nm, λem = 510 nm |
| Glutamine (Q) | γ-carboxamide | 4-amino-3-formylphenyl nitrate<br>λex = 500 nm, λem = 600 nm |
| Glycine (G) | hydrogen | C-H bond functionalization alpha to carbonyl via reaction with H-alkynyl-Phe in the presence of CuBr (10 mol-%) and 1 equivalent (eq.) of tBuOOH in DCM, followed by CuAAc with 3-azido-7-methoxy-2H-chromen-2-one<br>λex = 365 nm, λem = 420-449 nm |
| histidine Histidine (H) | Imidazole | 2-butyl-6-(4-((6-(((2-ethoxyethyl)amino)methyl)pyridin-2-yl)methyl)piperazin-1-yl)-1H-benzo[de]isoquinoline-1,3(2H)-dione-$Cu^{2+}$ |
| Isoleucine (I) | sec-butyl | Blue light meditated Hoffman-Loffler-Freytag reaction for δ-C-H functionalization of isoeleucine: Reaction with acetic hypobromous anhydride catalyzed by blue LED to install a Br group, followed by SN2 reaction with $KN_3$ to install azide group, then CuAAc with 4-((7-ethynyl-2-oxo-2H-chromen-4-yl) methoxy)-4-oxobutanoic acid |
| Leucine (L) | Isobutyl | Blue light meditated Hoffman-Loffler-Freytag reaction for δ-C-H functionalization of isoeleucine: Reaction with acetic hypobromous anhydride catalyzed by blue LED to install a Br group, followed by SN2 reaction with $KN_3$ to install azide group, then CuAAc with 4-((7-ethynyl-2-oxo-2H-chromen-4-yl) methoxy)-4-oxobutanoic acid |
| Lysine (K) | ε-amino group | Ortho-phthalaldehyde (OPA) in the presence of β-mercaptoethanol (BME) |
| Methionine (M) | S-methyl thioether | Reaction with alkyne bearing methionine-selective iodonium salt, followed by click chemistry with CalFlour dye<br>λex = 488 nm, λem = 520 nm |
| Phenylalanine (F) | Benzyl | Palladium catalysed alkynylation reaction with (bromoethynyl)triisopropylsilane (10 mol % Pd(OAc)2 with 2 equivalents of K2CO3 as a base, and 0.2 equiv of PivOH as an additive), followed by CuAAc with 3-azido-7-hydroxy-2H-chromen-2-one<br>λex = 365 nm, λem = 490-499 nm |

(continued)

| Amino acid type | R-group labelled | Fluorogenic Labelling |
|---|---|---|
| Proline (P) | pyrrolidine | amphiphilic dipolar Schiff base $Zn^{II}$ complexes |
| Sserine (S) | Hydroxymethyl | Selective conversion to azide with TT/n-Bu4NN3 or Ph3P:2,3-dichloro-5,6-dicyanobenzoquinone (DDQ):n-Bu4NN3 followed by reaction with FI-DIBO |
| Threonine (T) | Hydroxyl | Selective conversion to azide with TT/n-Bu4NN3 or Ph3P:2,3-dichloro-5,6-dicyanobenzoquinone (DDQ):n-Bu4NN3 followed by reaction with FI-DIBO |
| Tryptophan (W) | Indole | Trichloroethanol (TCE) |
| Tyrosine (Y) | Phenol | Trichloroethanol (TCE)<br>OR<br>Installation of aryl groups ortho to the tyrosine hydroxyl groups using $[RhCl(PPh_3)_3]$ (5 mol-%), $R_2P(OAr)$ (20 mol-%)<br>Ar-Br (1.5 eq.), $CsCO_3$ (2 eq.)<br>Ar = aryl, R = t-butyl, Ar |
| Valine (V) | Isopropyl | Installation of quaternary azide group on valine side chain using [Ru(bpy)3] Cl2 (0.1 mol-%) catalyst and 2 eq. of 1-azido-1I3-benzo[d][1,2]iodaxox-ol-3(1H)-one catalysed by visible light, followed by fluorogenic CuAAC reaction with 4-((7-ethynyl-2-oxo-2H-chromen-4-yl)methoxy)-4-oxobutanoic acid |
| Pyrrolysine (O) | pyrrol (N,2,3-trimethyl-3,4-dihydro-2H-pyrrole-2-carboxamide) | Diels Alder reaction with an azaphthalimide |
| Selenocysteine (U) | ethylselenol | ABD-F, at pH 7 |

Strategies for the labelling of aliphatic amino acids exploit the state-of-the-art area of C-H bond functionalization (DOI: 10.1002/ejoc.201800896). In alternative embodiments, a protease with cleavage specificity for an aliphatic (A, I, L, F or V) amino acid at the P1 or P1' position can be used to cut the protein sequence whenever the amino acid type of interest occurs. That generates a new protein N-terminus wherever the protein sequence has been cut. This can easily be modelled as the cleavage specificity for proteases is known. The protein N-terminus can react using a fluorogenic dye specific for the N-terminus such as an NHS-ester. In this way, a fluorogenic dye specific for the N-terminus reacts exclusively when the N-terminus is adjacent to the amino acid type of interest, hence, the concentration of an aliphatic, e.g. valine (V), amino acid type in the sample is measured based on the concentration of N-termini generated when the protease cleaves at the V position (and the signal of the label reports on the amino acid concentration of the V amino acid type). For example, human neutrophil elastase cleaves at valine amino acids. The number of V amino acids for the protein of interest is adjusted to add the number of N-termini already present within the protein of interest (based on the number of protein chains), and this is used as input to set of parametric equations 1. In some embodiments, the protease also cleaves, generating signal due to its own valine amino acids, but this is incorporated into the background fluorescence intensity measurement.

Preferably, the R-groups labelled for the amino acid types are the R-groups for the amino acid types; these include labelling of R-groups containing a glycoside specific for R-groups containing a glycoside and comprises Selective conversion to azide with TT/n-Bu4NN3 or Ph3P:2,3-dichloro-5,6- dicyanobenzoquinone (DDQ):n-Bu4NN3 followed by reaction with FI-DIBO.

Labelling of R-groups containing a fatty acid is specific for R-groups containing a fatty acid comprises labelling with Dipolar 3-methoxychromones, allowing detection of all lipidated amino acid types. Labelling of R-groups containing a phosphate comprises activation with carbonyldiimidazole to provide a leaving group, followed by reaction with a cysteine BODIPY dye, and is specific for R-groups containing a phosphate, allowing detection of all amino acid types modified with a phosphate.

In some embodiments, the modified amino acids of an amino acid type are labelled differently to the unmodified amino acids of the amino acid type. For example, for the amino acid type cysteine, the labelling reaction for the unmodified

amino acids (e.g. R-group of) $C_R$ is different to the labelling reaction for the combination of the modified and unmodified amino acids (e.g. R-group of, once the modified amino acids have been reduced). In the $C_R$ labelling reaction, only reduced cysteine amino acids are available for modification. The disulphide bonded cysteine amino acids are not available for modification. To label both modified and unmodified amino acids of cysteine, both the reduced cysteine amino acids and disulphide bonded cysteine amino acids need to be labelled. One way to achieve this is to reduce the disulphide bonded cysteine amino acids to reduced cysteine amino acids before being labelled. Preferably, the disulphide bonded cysteine amino acids are reduced with TCEP before labelling all of the reduced cysteine, that includes the oxidsied cysteine which has newly been reduced i.e. the combination of $C_D$ and $C_R$, after $C_D$ has been reduced, with ABD-F. This reduction of the oxidized cysteine amino acids allows both the disulphide bonded and the reduced cysteine amino acids in the sample to be labelled, i.e. the combination of $C_D$ and $C_R$. By fractionating the sample into multiple fractions, it is possible to measure $C_D$, $C_R$ and/or the combination of $C_D$ and $C_{RT}$ amino acids within the sample. In some embodiments, the sample is separated into two fractions. In one fraction, $C_R$ is labelled. In the second fraction, the combination of $C_D$ and $C_R$ is labelled. The number of $C_D$ labelled is equal to the number of the combination of $C_D$ and $C_R$ labelled per protein minus the number of $C_R$ labelled.

The skilled person would appreciate that although all or a proportion of amino acids within a peptide are exposed to the solvent and available for a labelling reaction, this may not be the case for a folded protein. In some embodiments, a sample is denatured prior to or during the labelling reaction. Methods for denaturing a protein are known in the art. In some embodiments, this is achieved via adding a miscible organic solvent such as dimethyl sulfoxide, methanol, acetonitrile, ethanol, or isopropanol. In some embodiments, this is achieved via changing the buffer conditions to low or high pH such as pH 2, pH 3, pH 4, pH5, pH 7.5, pH 8.5, pH 9, pH 10, or pH 10.5. In some embodiments, this is achieved by heating the solution to 40 °C, 50 °C, 60 °C, 70 °C, 80 °C, 90 °C, or 100 °C. In some embodiments, this is achieved by reducing protein disulphide bonds with TCEP, β-mercapto ethanol, DTBA, or DTT. In some embodiments, this is achieved by adding a denaturing agent such as urea, guanidinium chloride, or guanidinium thiocyanate. In some embodiments, this is achieved by adding a surfactant such as sodium dodecyl sulfate (SDS), dodecyltrimethylammonium bromide (DTAB), cetyltrimethylammonium bromide (CTAB), phosphatidylcholine, Triton X-100, Triton X-114, CHAPS, NP-40, sodium 1-undecanesulfonate (SUS) sodium dodecylbenzenesulfonate (SDBS), sodium deoxycholate (DOC), sodium stearate, 4-(5-dodecyl)benzenesulfonate, dioctyl sodium sulfosuccinate, alkyl ether phosphates, benzalkaonium chloride (BAC), and perfluorooctanesulfonate (PFOS). In some embodiments, denaturing proteins contained within a sample is achieved during the labeling reaction. In some embodiments, the labelling reactions are performed in the presence of the additives listed herein. In some embodiments, denaturing polypeptides contained within a sample is achieved by reducing polypeptide disulphide bonds and adding a surfactant. In some embodiments, denaturing proteins contained within a sample is achieved by reducing protein disulphide bonds and adding a surfactant. In some embodiments, denaturing polypeptides contained within a sample is achieved by reducing polypeptide disulphide bonds, adding a surfactant and changing the buffer conditions to high or low pH. In some embodiments, denaturing proteins contained within a sample is achieved by reducing protein disulphide bonds, adding a surfactant and changing the buffer conditions to high or low pH. In some embodiments, denaturing proteins contained within a sample is achieved by reducing protein disulphide bonds with TCEP and adding the surfactant SDS. In some embodiments, denaturing proteins, peptides, oligopeptides, polypeptides, and/or protein complexes which comprise a subproteome or a proteome contained within a sample is achieved by reducing protein, peptide, oligopeptide, polypeptide, and/or protein complex disulphide bonds and adding a surfactant. In some embodiments, denaturing proteins, peptides, oligopeptides, polypeptides, and/or protein complexes which comprise a subproteome or a proteome contained within a sample is achieved by reducing by reducing protein, peptide, oligopeptide, polypeptide, and/or protein complex disulphide bonds, adding a surfactant and changing the buffer conditions to high or low pH. In some embodiments, denaturing proteins, peptides, oligopeptides, polypeptides, and/or protein complexes which comprise a subproteome or a proteome contained within a sample is achieved by reducing protein, peptide, oligopeptide, polypeptide, and/or protein complex disulphide bonds, adding a surfactant and changing the buffer conditions to high or low pH. In some embodiments, denaturing proteins, peptides, oligopeptides, polypeptides, and/or protein complexes which comprise a subproteome or a proteome contained within a sample is achieved by reducing protein, peptide, oligopeptide, polypeptide, and/or protein complex disulphide bonds with TCEP and adding the surfactant SDS. For example, the labelling reactions are performed at pH 10 in the presence of 4% w/v SDS and 18 mM β-mercaptoethanol. As another example, the labelling reactions are performed at pH 10.5 in the presence of 4% SDS and 10 mM TCEP. In some embodiments, there are multiple steps to the labelling reaction. In some embodiments, the first step makes the R-group of an unmodified or modified amino acid type reactive for labelling, and proceeds under a set of conditions appropriate for that reaction. Then the second step of the labelling reacts the now reactive R-group under the denaturing conditions described for the labelling of all amino acids of the amino acid type. For example, labelling of the the combination of $C_D$ and $C_R$ amino acid type first involves reduction of the $C_D$ amino acid subtype with 10 mM TCEP to expose reactive thiols, and proceeds at pH 7 within 45 minutes. Then, the exposed thiols are reacted with ABD-F at pH 10.5 in the presence of 4% SDS. As another example, the first step of labelling phenylalanine is making the R-group reactive for labelling, and involves a palladium catalysed alkynylation reaction with (bromoethynyl)triisopropylsilane in the presence of

10 $\mu$M $Pd(OAc)_2$ with 10 mM $K_2CO_3$ as a base, and 1 mM PivOH as an additive in water. This installs an alkyne group onto the phenylalanine ring, which is specifically reactive for azide groups. The next step of the labelling reaction involves CuAAc with 3-azido-7-hydroxy-2H-chromen-2-one in 75% H2O/25% BuOH in the presence of 5 $\mu$M CuSO4 and 25 $\mu$M Na ascorbate.

In some embodiments, the amino acids of (e.g. R-group), the combination of $C_D$ and $C_R$ are fluorogenically labelled with ABD-F after reduction with TCEP, and denaturation with sodium dodecyl sulfate (SDS) in a buffer. In some embodiments, the TCEP buffer is HEPES buffer and the ABD-F / SDS buffer is sodium carbonate buffer. In some embodiments, the amino acids of the combination of $C_D$ and $C_R$ are fluorogenically labelled with 2-10 mM ABD-F, 2-10% SDS and 50-500 mM sodium carbonate buffer. In some embodiments, the amino acids of the combination of $C_D$ and are fluorogenically labelled with 5 mM ABD-F, 4% (SDS) in 80 mM sodium carbonate buffer. In some embodiments, the timing of the TCEP reaction is from about 20 to about 40 minutes, preferably 30 minutes. In some embodiments, the timing of the ABD-F reaction is from about 5 to about 55 minutes, preferably about 45 minutes.

In some embodiments, the amino acids, (e.g. R-group) of reduced cysteine ($C_R$) are fluorogenically labelled with ABD-F after denaturation with SDS in a buffer. In some embodiments, the buffer is a sodium carbonate buffer. In some embodiments, the amino acids of $C_R$ are fluorogenically labelled with 2-10 mM ABD-F, 2-10% SDS and 50-500 mM sodium carbonate buffer. In some embodiments, the amino acids of $C_R$ are fluorogenically labelled with 5mM ABD-F, 4% SDS in 80 mM sodium carbonate buffer.

In some embodiments, the unmodified amino acids of the amino acid type, (e.g. R-group) of lysine (K) are fluorogenically labelled with OPA, $\beta$-mercaptoethanol (BME) and SDS in a buffer. In some embodiments, the buffer is a sodium carbonate buffer. In some embodiments, the amino acids are fluorogenically labelled with 10-20 mg OPA + 5-10 mL carbonate buffer + 10-20 $\mu$L BME + 1-5 mL 20% SDS. In some embodiments, the amino acids are fluorogenically labelled with 12 mM ortho-phthalaldehyde (OPA), 18 mM beta-mercaptoethanol (BME), 4% SDS in 200 mM sodium carbonate buffer. The dye molecule, OPA, is a dialdehyde. The lysine primary amine attacks one aldehyde and water is lost. This results in the formation of an imine, specifically a Schiff base. The thiol nucleophile presented by BME attacks this Schiff base, such that the amine is again available for a ring-closing attack on the other pendant aldehyde. BME participates in the reaction to create the fluorophore, however, other thiols can be used instead of BME. Water is lost, and conjugation is extended into the newly formed ring, resulting in generation of fluorescence into the visible region of the spectrum.

In some embodiments, the combination of the modified and unmodified amino acids of the amino acid type (e.g. R-group) of tryptophan (W) and the combination of the modified and unmodified amino acid type (e.g. R-group) of tyrosine (Y) are fluorogenically labelled with TCE, after reduction with TCEP, and denaturation with sodium dodecyl sulfate (SDS) in a buffer. TCEP reduces the disulphide bonds within the protein such that, together with SDS denaturation, all tryptophan (W) and tyrosine (Y) amino acids are available for reaction. In some embodiments, the buffer is HEPES buffer. In some embodiments, the excited state of W amino acids, Y amino acids, or W and Y amino acids undergoes a radical reaction with TCE. In some embodiments, the reaction is photo-catalyzed with UV light of a wavelength absorbed by W amino acids, Y amino acids, or W and Y amino acids. In some embodiments, the amino acid type of W is fluorogenically labelled with 0.01-5 M TCE, 2-50 mM TCEP and 2-20% SDS in 1-10 mM HEPES catalyzed by UV light with wavelengths of 260-310 nm. In some embodiments, the amino acid type of W is fluorogenically labelled in 0.2 M TCE, 10 mM TCEP and 4% SDS in 5 mM HEPES catalyzed by UV light with wavelengths of 295-305 nm. In some embodiments, the amino acid type of Y is fluorogenically labelled with 0.01-5 M TCE, 2-50 mM TCEP and 2-20% SDS in 1-10 mM HEPES catalyzed by UV light with wavelengths of 260-310 nm. In some embodiments, the amino acid type of Y is fluorogenically labelled in 0.2 M TCE, 10 mM TCEP and 4% SDS in 5 mM HEPES catalyzed by UV light with wavelengths of 285-295 nm.

In some embodiments, when labelling the amino acid type (e.g. R-group) cysteine (C), wherein both the modified and the unmodified amino acids of cysteine are labelled, the oxidized (disulphide bonded) cysteines are reduced via a reducing agent (TCEP), before the cysteine thiol contained within the cysteine R-group acts as a nucleophile in an electrophilic addition/elimination reaction on the dye ABD-F. This results in loss of a fluorine quenching group, such that the fluorescence of the dye is no longer quenched. When labelling the amino acid type lysine (K), the lysine primary amine contained within the R-groups attacks one of the aldehydes contained within OPA. This forms an imine which is attacked by a thiol (BME) added to the reaction, releasing a primary amine for attack on the remaining pendant aldehyde. This closes a second ring and extends the aromatic conjugation bringing the fluorescence into the visible region.

The labelling reaction for the amino acid types tryptophan and tyrosine is a photo-catalyzed radical reaction. Tryptophan can be labelled with 2,2,2-trichloroethanol (TCE), 2,2,2-trichloroacetate (TCA) or chloroform, as well as other di/tri halogenated compounds. Radicals from the tryptophan R-group and TCE combine, and a hydrogen atom is lost resulting in the addition of a dihalo compound to the indole ring. This is unstable and attacked by a water molecule, then hydrochloric acid is lost, resulting in the addition of an alpha hydroxy ketone to the indole ring which shifts the intrinsic fluorescence of tryptophan about 100 nm to the right and into the visible region. The labelling reaction for tyrosine reaction is also a photo-catalyzed radical reaction with TCE which shifts the intrinsic fluorescence of tyrosine about 100 nm to the right and into the visible region. The phenol R-group of tyrosine combines with TCE resulting in the addition of an alpha

hydroxy ketone to the ring via the same mechanism as in the tryptophan labelling reaction.

It was discovered that labelling an already intrinsically fluorescent amino acid type can enable quantitative detection not possible for the unlabelled amino acid type. For example, tryptophan and tyrosine amino acids are intrinsically fluorescent, so labelling intrinsically fluorescent amino acids with a fluorescent label is counterintuitive. It is appreciated by those skilled in the art that intrinsic fluorescence for tryptophan and tyrosine amino acid types is highly dependent on the local environment surrounding these residues within the 3D-protein structure, therefore intrinsic fluorescence from the tryptophan and tyrosine amino acid types cannot reveal the content of tryptophan and tyrosine residues within protein sequences. However, when these amino acid types are labelled, the environmental sensitivity of their fluorescence disappears, and the fluorescence from the labelled tryptophan or tyrosine does reveal the number or concentration of tryptophan or tyrosine amino acid types within the sample. For example, a disappearance of environmental sensitivity of the fluorescence is observed for tryptophan and tyrosine amino acid types labelled with trichloroethanol (TCE). This unexpected result could be due to a change in the electronic properties of tryptophan or tyrosine whose indole or phenol ring conjugation has been extended via the addition of an alpha hydroxy ketone, making the fluorophore less sensitive to the polarity of the local environment.

Alternatively, the fluorescent label includes a fluorescent protein or conjugated antibody. Preferably, the fluorescent protein is selected from the group consisting of: CFP, GFP (emGFP), RFP (tagRFP), GFP (Y66H mutation), GFP (Y66F mutation), EBFP, EBFP2, Azurite, GFPuv, T-Sapphire, mCerulean, mCerulean3 mCFP, mTurquoise2, ECFP, CyPet, GFP (Y66W mutation), mKeima-Red, TagCFP, AmCyan1, mTFP1, GFP (S65A mutation), Midoriishi Cyan, Wild Type GFP, GFP (S65C mutation), TurboGFP, TagGFP, GFP (S65L mutation), Emerald, GFP (S65T mutation), EGFP, Azami Green, ZsGreen1, TagYFP, EYFP, Topaz, Venus, mCitrine, YPet, TurboYFP, ZsYellow1, Kusabira Orange, mOrange, Allophycocyanin (APC), mKO, TurboRFP, tdTomato, TagRFP, DsRed monomer, DsRed2 ("RFP"), mStrawberry, TurboFP602, AsRed2, mRFP1, J-Red, R-phycoerythrin (RPE), B-phycoerythrin (BPE), mCherry, HcRed1, Katusha, P3, Peridinin Chlorophyll (PerCP), mKate (TagFP635), TurboFP635, mPlum or mRaspberry. In some embodiments, conjugated antibodies specific for post-translational modifications can be used within the methods of the invention. In some embodiments, the conjugated antibody is labelled with one of the fluorescent labels or fluorophores provided herein. Preferably, the conjugated antibody is a monoclonal antibody derived traditionally or synthetically, and is selected from the group including IgG, IgM, IgA, IgE or nanobodies. Preferably, the antibody is labelled with one or a fluorogenic dye, fluorescent label, or fluorophore per antibody. In some embodiments, the conjugated antibody is selective for a post-translational modification including: N-acetylation, methylation, deimination to citrulline, deamidation to aspartic acid or isoaspartic acid, N-linked glycosylation, isomerization to isoaspartic acid, disulfide-bond formation, oxidation to sulfenic, sulfinic or sulfonic acid, palmitoylation, N, acetylation (N-terminus), S-nitrosylation, cyclization to Pyroglutamic acid (N-terminus), gamma-carboxylation, deamidation to glutamic acid, isopeptide bond formation, N-Myristoylation (N-terminus), Phosphorylation, Ubiquitination, SUMOylation, isopeptide bond formation to a glutamine, hydroxylation, N-linked Ubiquitination, oxidation to sulfoxide or sulfone, Hydroxylation, O-linked glycosylation, mono- or di-oxidation, formation of Kynurenine, or sulfation.

Alternatively, the label is a tandem mass tag (TMT). Preferably, the tandem mass tags are TMTzero, TMTduplex, TMTsimplex, TMT 10-plex, TMTpro and TMTpro Zero. Alternatively, the label is a stable isotope label (i.e. isotopic labelling). In some embodiments, the stable isotope label is a non-radioactive isotope. In some embodiments, the non-radioactive isotope label is 2H, 13C, and/or 15N. In some embodiments, the labelling strategies are used in combination. For example, each amino acid type can be labelled with chemically (e.g. with a fluorogenic dye) and then labelled with an antibody. For example, two or more amino acid types are labelled chemically, and then a post-translational modification specific antibody is used, for example, to detect phosphorylation of amino acids of a different amino acid type.

It would be appreciated by the person skilled in the art, that the labelling reactions encompassed as part of the invention, can be performed without, or following a separation step to isolate the protein component of the sample, or a particular protein of interest in the sample. For example, a separation step such as extraction, precipitation and differentiation solubilization, centrifugation, ultracentrifugation, sonication, size exclusion chromatography, separation based on charge or hydrophobicity (examples include hydrophobic interaction chromatography, ion exchange chromatography, free-flow electrophoresis, capillary electrophoresis), affinity chromatography such as immunoaffinity chromatography and high-performance liquid chromatography (HPLC), or other methods known within the technical field can be used.

In some embodiments, the proteins within the sample are concentrated once isolated. This can involve, but is not limited to, lyophilization or ultrafiltration. In some embodiments, one or more proteins, peptides, oligopeptides, polypeptides, protein complexes, subproteomes, or proteomes within a sample are concentrated once isolated.

In some embodiments, the label is a fluorescent label and two or more amino acid types are labelled with the same fluorescent label under the same conditions, but the labelling reactions proceed at different rates. Therefore, measuring the time-resolved signal of the label at a certain time reveals the signal of the label from exclusively one labelled amino acid type, while measuring the time-resolved signal of the label at another time reveals the signal of the label from exclusively another labelled amino acid type, or from both labelled amino acid types such that the signal of the label at the

first time can be subtracted from the signal of the label at the second time to reveal the signal of the label from exclusively the second amino acid type. This is kinetic deconvolution. Preferably, the detection of the label for each amino acid type is deconvoluted from the other amino acid type to enable the label for each individual amino acid type to be detected. For example, both W and Y amino acid types are labelled with the fluorogenic label TCE, and the labelling reactions take place under the same conditions, 0.2 M TCE, 10 mM TCEP and 4% SDS in 5 mM HEPES and photocatalyzed by UV light with a wavelength of 280 nm, but the labelling reactions proceed at different rates. In this example, W labelling occurs before Y labelling. Therefore, W and Y are de-convoluted by stopping the labelling reaction when only W residues are labelled and then performing the reaction allowing sufficient time for both W and Y to be labelled, so that the fluorescence of each of Y and W can be measured in the sample. In some embodiments, the fluorescence of Y in the sample is equal to the fluorescence of W and Y in the sample minus the fluorescence of W in the sample. In a preferred embodiment, the amino acid types W and Y are deconvoluted from each other. In a preferred embodiment, the amino acid types serine and Threonine are deconvoluted from each other. In a preferred embodiment, the amino acid types asparagine and Glutamine are deconvoluted from each other. In a preferred embodiment, the amino acid types glutamic acid and Aspartic Acid are deconvoluted from each other. In a preferred embodiment, the amino acid types Leucine and Isoleucine are deconvoluted from each other.

Deconvolution can be achieved at the labelling stage. In some embodiments, deconvolution is achieved when forming the fluorescent dye. In this embodiment, the conditions when forming the label are changed such that the two or more amino acid types that are being labelled with the same fluorescence label react differently with the label compared to each other. In some embodiments, deconvolution of two amino acid types labelled with the same label can be achieved by choosing conditions in which one amino acid type will react with the label and the other amino acid type will not react with the label. Preferably, the labelling reaction of one amino acid type is catalysed and the labelling reaction of the other amino acid type is not catalysed. For example, for the light-catalysed modification reaction of W and Y amino acid types, the label is formed at different photo-catalysis wavelengths such that either only W amino acids or both W and Y amino acids absorb the light required to catalyse the reaction at the photo-catalysis wavelength. In some embodiments, the W amino acid type is selectively labelled.It is well known in the art that the absorbance spectra of W amino acids and Y amino acids are different, as described in https://www.biotek.com/resources/application-notes/peptide-and-aminoacid-quantification-using-uv-fluorescence-in-synergy-ht-multi-mode-microplate-reader/. The W amino acid type absorbs at wavelengths greater than 295 nm whereas the Y amino acid types does not absorb at wavelengths greater than 295 nm. Therefore, reaction of the W amino acid type can be catalysed without catalysing reaction of the Y amino acid type by using a photo-catalysis wavelength at which the W amino acid type absorbs and the Y amino acid type does not absorb, such as 300 nm. Alternatively, labelling of both W and Y amino acid types can be achieved by catalysing the reaction with a wavelength of light at which both the W and Y amino acid types absorb, such as 280 nm. Therefore, it is possible to separate the labels by using light at about 300nm so that the wavelength of the excitation light is too large from the amino acid type Y to absorb in that region so that only the W amino acid type is labelled. In some embodiments, a spectrum of light can be used, for example a 30 nm bandwidth within a specified region. In some embodiments, a 10 nm bandwidth within a specific region can be used. In other embodiments a single wavelength of light can be used, for example via a laser or LED.

In some embodiments, the sample contains, or is suspected to contain, a subproteome, or proteome. In such embodiments, two or more amino acid types (e.g. R-groups) in the sample are labelled without the need to separate the sample into its individual protein, peptide, oligopeptide, polypeptide, or protein complex components. It will be appreciated by those skilled in the art that separation of a complex mixture, such as a proteome or a subproteome, into its individual components can require significant time and labour. It will be appreciated by those skilled in the art that a requirement for sequentially analysing each individual protein within a proteome or subproteome significantly increases the time required for analysis, as if a proteome contains 1000 proteins this will require sequential analysis of 1000 samples, The ability of the present invention to analyse a proteome, subproteome or mixture sample without separating the sample into its individual protein components allows a high throughput of the method, because separation of the subproteome, proteome or mixture is not required.

**Measuring the label**

The label of each of the labelled amino acid type in the sample is measured. In some embodiments, the label provides a signal, and the signal of the label is measured.

The measured label of each amino acid type is linearly related to the concentration of that amino acid type within the sample, to the number of amino acids of each amino acid type in the sample, and to the protein concentration of the sample. In a preferred embodiment, the label is a fluorogenic dye, and the measured fluorescence intensity of each amino acid type is linearly related to the concentration of that amino acid type within the sample, to the number of amino acids of each amino acid type in the sample, and to the protein concentration of the sample. In a preferred embodiment, the label is a non-fluorogenic dye and the measured signal of the non-fluorogenic dye of each amino acid type is linearly related to the concentration of that amino acid type in the sample, to the number of amino acids of each amino acid type in the sample,

and to the protein concentration of the sample. Preferably, the signal of the non-fluorogenic dye is purified to remove the unreacted dye. This can be achieved by a chromatography column. In some embodiments, the label is a nucleotide sequence, which can be amplified using PCR, and the measured nucleotide sequence of each amino acid type is linearly related to the concentration of that amino acid type within the sample, to the number of amino acids of each amino acid type in the sample, and to the protein concentration of the sample. In a preferred embodiment, the label is a mass tag or isotopic label, and the measured tag or isotopic label is linearly related to the concentration of that amino acid type within the sample, to the number of amino acids of each amino acid type in the sample, and to the protein concentration of the sample.

In some embodiments, the two or more amino acid types in the sample are each labelled with a different isotopic label, and each isotopic label of the at least two labelled amino acid types is detected through nuclear magnetic resonance (NMR) and mass spectrometry.

In some embodiments, the two or more amino acid types in the sample are each labelled with a different tandem mass tag within a tandem mass tag system and the tandem mass tag of each of the at least two labelled amino acid types is detected through mass spectrometry. In some embodiments, the TMTduplex, TMTsixplex, TMT10plex, TMT11plex or TMT16plex tandem mass tag systems are used. In some embodiments, the protein reactive groups within the tandem mass tags are specific for each of two or more amino acid types.

Preferably, the label is fluorescent, chemiluminescence, or bioluminescent. In some embodiments, the spectral properties of the label are measured. In some embodiments, the spectral properties of the label are measured upon illumination of the label or a chemical reaction of the label. Upon illumination of the label, light can be reflected, transmitted, absorbed, or emitted. This reflection, transmission, absorption, or emission of light of the label can be measured. Preferably, the label is fluorescence and the emission of light of the label is measured in response to irradiation with light. In some embodiments, the excitation spectrum and emission spectrum of one fluorescently labelled amino acid type is distinguishable from the excitation spectrum and emission spectrum of the second fluorescently labelled amino acid type. This is preferred if the two or more amino acid types are being labelled in one single fraction. The fluorescence label provides each labelled amino acid type in the sample with a unique signature of fluorescence.

In some embodiments, the label is fluorescence. The two or more amino acid types in the sample are each labelled with fluorescence, such as a fluorogenic dye, and the fluorescence intensity of each of the at least two labelled amino acid types is determined. In some embodiments, the fluorescence intensity of the fluorogenic labels of each of the at least two labelled amino acid types is detected using fluorescence microscopy. In some embodiments, the fluorescence intensity of the fluorogenic label of each of the at least two labelled amino acid types is detected using a fluorescence plate reader. In some embodiments, the fluorescence intensity of the non-fluorogenic dye is detected. Preferably, the non-fluorogenic dye is purified from the unreacted dye before detection. In some embodiments, the fluorescence intensity of the non-fluorogenic label of each of the at least two labelled amino acid types is detected using fluorescence microscopy. In some embodiments, the fluorescence intensity of the non-fluorogenic label of each of the at least two labelled amino acid types is detected using a fluorescence plate reader.

In some embodiments, the fluorescence of the fluorescent label of the amino acid type Y is measured at an excitation wavelength of from about 250nm to about 400nm and an emission wavelength of from about 370nm to about 600nm. Preferably, the fluorescence of the amino acid type Y is measured at an excitation wavelength of from about 270nm to about 330nm and an emission wavelength of from about 375nm to about 500nm. In some embodiments, the label for the amino acid type Y is TCE, after reduction of any disulphide bonds contained within the protein with TCEP, and denaturation with sodium dodecyl sulfate (SDS) in a buffer and the fluorescence is measured at an excitation wavelength of from about 270nm to about 330nm and an emission wavelength of from about 375nm to about 500nm.

In some embodiments, the fluorescence of the fluorescent label of amino acid type W is measured at an excitation wavelength of from about 250nm to about 400nm and an emission wavelength of from about 370nm to about 600nm. Preferably, the fluorescence of the amino acid type W is measured at an excitation wavelength of from about 270nm to about 320nm or from about 350nm to about 370nm and an emission wavelength of from about 440nm to about 550nm. In some embodiments, the fluorescent label of the amino acid type W is TCE, after reduction of any disulphide bonds contained within the protein with TCEP, and denaturation with sodium dodecyl sulfate (SDS) in a buffer and the fluorescence is measured at an excitation wavelength of from about 250nm to about 400nm and an emission wavelength of from about 370nm to about 600nm. In some embodiments, the fluorescent label of the amino acid type W is TCE, after reduction of any disulphide bonds contained within the protein with TCEP, and denaturation with sodium dodecyl sulfate (SDS) in a buffer and the fluorescence is measured at an excitation wavelength of from about 270nm to about 320nm or from about 350nm to about 370nm and an emission wavelength of from about 440nm to about 550nm.

In some embodiments, the fluorescence of the amino acid type K is measured at an excitation wavelength of from about 320nm to about 400nm and an emission wavelength of from about 415nm to about 500nm. Preferably, the fluorescence of the amino acid type K is measured at an excitation wavelength of from about 330nm to about 390nm and an emission wavelength of from about 415nm to about 480nm. In some embodiments, the fluorescence of the amino acid type K is measured from about 2 to about 25 seconds after the labelling reaction is initiated. Preferably, the fluorescence of the amino acid type K is measured within 4 seconds after the labelling reaction is initiated. In some embodiments, the amino

acid type K is labelled with OPA, β-mercaptoethanol (BME) and SDS in a buffer and the fluorescence is measured at an excitation wavelength of from about 320nm to about 400nm and an emission wavelength of from about 415nm to about 500nm. In some embodiments, the amino acid type K is labelled with OPA, β-mercaptoethanol (BME) and SDS in a buffer and the fluorescence is measured at an excitation wavelength of from about 330nm to about 390nm and an emission wavelength of from about 415nm to about 480nm. In some embodiments, the amino acid type K is labelled with OPA, β-mercaptoethanol (BME) and SDS in a buffer and the fluorescence is measured at an excitation wavelength of 350nm and an emission wavelength of 460 nm.

In some embodiments, the fluorescence of the amino acid type C is measured at an excitation wavelength of from about 330nm to about 400nm and an emission wavelength of from about 430nm to about 550nm. Preferably, the fluorescence of the amino acid C is measured at an excitation wavelength of from about 340nm to about 390nm and an emission wavelength of from about 470nm to about 530nm. These excitation and emissions wavelengths are used to measure the label for both reduced cysteine ($C_R$) and/or the combination of $C_D$ and $C_R$). In some embodiments, the fluorescence label for reduced cysteine ($C_R$) is ABD-F after denaturation with SDS in a buffer and the fluorescence is measured at an excitation wavelength of from about 330nm to about 400nm and an emission wavelength of from about 430nm to about 550nm. In some embodiments, the fluorescence label for reduced cysteine ($C_R$) is ABD-F after denaturation with SDS in a buffer and the fluorescence is measured at an excitation wavelength of from about 340nm to about 390nm and an emission wavelength of from about 470nm to about 530nm. In some embodiments, the fluorescence label for the combination of $C_D$ and $C_R$ is ABD-F, after reduction with TCEP, and denaturation with sodium dodecyl sulfate (SDS) in a buffer and the fluorescence is measured at an excitation wavelength of from about 330nm to about 400nm and an emission wavelength of from about 430nm to about 550nm. In some embodiments, the fluorescence label for the combination of $C_D$ and $C_R$ is ABD-F, after reduction with TCEP, and denaturation with sodium dodecyl sulfate (SDS) in a buffer and the fluorescence is measured at an excitation wavelength of from about 340nm to about 390nm and an emission wavelength of from about 470nm to about 530nm.

Preferably, for the fluorescent label of each amino acid type being measured, the excitation wavelength is separated from the emission wavelength by from about 10nm to about 20nm from one another to avoid any crosstalk. This ensures that the excitation light does not provide a false signal for the emission light. Preferably, for the fluorescent label of each amino acid type, the excitation wavelength is separated from the emission wavelength by from about 15nm to about 20nm from one another to avoid any crosstalk.

In some embodiments, the label is a fluorescent label and two or more amino acid types are labelled with the same fluorescent label under the same conditions (e.g. the label is the same, the concentration of the label is the same, the wavelength of light used to catalyse the reaction is the same). Preferably, the detection of the label for each amino acid type is deconvoluted from the other amino acid type to enable the label for each individual amino acid type to be detected. For example, both W and Y amino acid types are labelled with the fluorogenic label TCE. For example, both W and Y amino acid types are labelled with 0.2 M TCE, 10 mM TCEP and 4% SDS in 5 mM HEPES and photocatalyzed by UV light with a wavelength of 280 nm at which both the amino acid types W and the amino acid types Y absorb. Therefore, the fluorescence intensity of W and Y are de-convoluted so that the fluorescence of each of Y and W can be measured in the sample. In a preferred embodiment, the amino acid types W and Y are deconvoluted from each other. In a preferred embodiment, the amino acid types serine and Threonine are deconvoluted from each other. In a preferred embodiment, the amino acid types asparagine and Glutamine are deconvoluted from each other. In a preferred embodiment, the amino acid types glutamic acid and Aspartic Acid are deconvoluted from each other. In a preferred embodiment, the amino acid types Leucine and Isoleucine are deconvoluted from each other.

In some embodiments, deconvolution is achieved at the detection stage. Preferably, the deconvolution uses separate excitation wavelengths. In other embodiments, the deconvolution uses separate emission wavelengths. In other embodiments, the deconvolution uses separate excitation and separate emission wavelengths.

The separate photo-excitation wavelengths excite the newly formed dye and the fluorescence of the dye is measured. In this embodiment, deconvolution is achieved by using excitation and emission wavelength pairs where only one amino acid type will contribute to the fluorescence intensity. The separate photo-excitation wavelengths target each amino acid type. For example, proteins containing W and Y amino acids labelled with TCE have two excitation peaks. Exciting the sample at around 310 nm and measuring the fluorescence at around 450-480 nm results in detecting fluorescence from both W and Y amino acid types (wavelength pair 1). However, exciting the sample at around 355 nm and measuring the fluorescence at around 450-480 nm results in measuring fluorescence intensity from exclusively the W amino acid type (wavelength pair 2). This provides the measured label for one amino acid type, for example the W amino acid type via wavelength pair 2. Preferably, the measured label of the other amino acid type labelled in the sample and measured at the excitation-emission wavelength pair at which both amino acid types are detected is determined from the fluorescence intensity measured in the sample using a deconvolution standard.

A deconvolution standard only needs to be measured once, and the results can be stored or supplied to the user. There is no need to measure a deconvolution standard each time the amino acid types being deconvoluted at an excitation and emission wavelength pair are measured for a sample. There is no need to measure a deconvolution standard each

time a sample is measured.

In some embodiments, the deconvolution standard is chosen by accessing the publicly available amino acid sequences of a variety of proteins and removing the portions of the sequence that are biologically cleaved in the mature proteins. The number of amino acids within two or more of the corresponding amino acid types in these proteins is determined. For example, if W and Y amino acid types are being labelled in the sample, then the number of W and Y amino acids within these protein sequences are determined.

In some embodiments, a deconvolution standard comprises amino acids of only one type of amino acid that is labelled in a sample and is being deconvoluted at the wavelength pair at which both types of amino acids are detected. For example, if the amino acid types W and Y are being labelled in the sample, then the deconvolution standard contains W amino acids, but does not contain Y amino acids. In another example, the deconvolution standard contains Y amino acids but does not contain W amino acids. Preferably, the deconvolution standard contains only the type of labelled amino acid whose label value (e.g signal) for the sample is already known based on the excitation and emission wavelength pairs. The deconvolution standard is used to determine the contribution of exclusively one type of amino acid to the total label (e.g signal) measured at a wavelength pair at which both types of amino acids are detected.

In some embodiments, the deconvolution standard is used to deconvolute the amino acid types of tryptophan and tyrosine; leucine and isoleucine; aspartic acid and glutamic acid; serine and threonine; and/or asparagine and glutamine. For example, a selection of deconvolution standards is presented for deconvolution of the tryptophan and tyrosine; leucine and isoleucine; aspartic acid and glutamic acid; serine and threonine; and/or asparagine and glutamine amino acid types. These deconvolution standards were found by identifying proteins within the human plasma proteome for which the product of the number of the convoluted amino acid types is zero and the sum of the number of convoluted amino acid types is non-zero. In some embodiments, for deconvolution of the tryptophan and tyrosine amino acid types, the deconvolution standards are selected from the group comprising: alpha-synuclein parathyroid hormone, Age-related maculopathy susceptibility protein 2, 10 kDa heat shock protein mitochondrial, Small proline-rich protein 2F, Sperm protamine P1, Kunitz-type protease inhibitor 4, Statherin, Histatin-3, Elastin, Beta-defensin 133, Tumor suppressor ARF, Complexin-2, B melanoma antigen 5, and/or Selenoprotein W. In some embodiments, for deconvolution of the leucine and isoleucine amino acid types, the deconvolution standards are selected from the group comprising: Proline-rich protein 9, serine/arginine-rich splicing factor 3, Loricrin, Metallothionein-1M Apolipoprotein C-III, Beta-defensin 124, and Zinc finger protein 575. In some embodiments, for deconvolution of the aspartic acid and glutamic acid amino acid types, the deconvolution standards are selected from the group comprising: Humanin-like 9, Beta-defensin 136, Beta-defensin 4A, Putative zinc finger protein 726P1, T cell receptor delta diversity 1 Small proline-rich protein 2A, Small integral membrane protein 38, T cell receptor beta joining 1-3 , Putative uncharacterized protein PRO0628, Small proline-rich protein 2D, T cell receptor beta joining 2-5, Islet amyloid polypeptide, and/or Putative uncharacterized protein URB1-AS1. In some embodiments, for deconvolution of the serine and threonine amino acid types, the deconvolution standards are selected from the group comprising: Cytochrome c oxidase assembly factor 1 homolog , Basic salivary proline-rich protein 1, Protein BEX3, Histatin-1, Beta-defensin 134, Adropin, Dexamethasone-induced protein, Oculomedin, and/or Protein BEX5. In some embodiments, for deconvolution of the asparagine and glutamine amino acid types, the deconvolution standards are selected from the group comprising: Transthyretin, T-cell leukemia/lymphoma protein 1A, Testis development-related protein 1, Protein WFDC11, Ubiquitin-like protein FUBI, and/or Mitochondrial import receptor subunit TOM7 homolog.

The deconvolution standard which, of the labelled amino acid types being deconvoluted, only contains the amino acid type separately detected and does not contain the amino acid type not separately detected is fluorescently labelled and the fluorescence is detected at an excitation and emission wavelength pair at which both amino acid types are detected (wavelength pair 1). Fluorescence from the same fluorescently labelled deconvolution standard is then measured at an excitation and emission wavelength pair at which only one amino acid type is detected (wavelength pair 2). The protein concentration of the deconvolution standard does not need to be known. Preferably, the same solution of the deconvolution standard is measured at wavelength pair 1 and wavelength pair 2, so the relative and absolute protein concentration of the solution measured at wavelength pair 1 and wavelength pair 2 is the same. If the relative protein concentration of the solution measured at wavelength pair 1 and wavelength pair 2 is changed (e.g. the sample is diluted by a factor of 2 by adding an equal volume of the solution to an equal volume of buffer), then this dilution factor is noted, and the measured signal for a wavelength pair at which the deconvolution standard has been diluted is multiplied by the dilution factor to get the measured signal of the undiluted solution. After this optional dilution adjustment step, the measured signal for labelled deconvolution standard at wavelength pair 1 is divided by the measured signal for the labelled deconvolution standard at wavelength pair 2, resulting in a wavelength signal conversion. Then, the signal of the label for the sample at wavelength pair 2 at which only one amino acid type was detected is multiplied by the wavelength signal conversion, to reveal the signal at wavelength pair 1 deriving from the separately detected amino acid type. This signal is subtracted from the total signal at wavelength pair 1 to reveal the signal exclusively from the other amino acid type. In this way, a signal deriving from two amino acid types is split into two signals, each deriving exclusively from one amino acid type, so that the number of signals equals the number of amino acid types labelled and measured in the sample. For example, a deconvolution standard which only contains W amino acids and does not contain any Y amino acids is fluorescently

labelled and the fluorescence is detected at an excitation and emission wavelength pair at which both W and Y amino acid types are detected (wavelength pair 1; excitation: 310 nm, emission: 450 nm). Fluorescence from the same fluorescently labelled deconvolution standard solution is then measured at an excitation and emission wavelength pair at which only the W amino acid type is detected (wavelength pair 2; excitation: 355 nm, emission: 450 nm). The same fluorescently labelled deconvolution standard solution is measured at wavelength pair 1 and 2, so there has been no dilution. The measured signal for labelled deconvolution standard at wavelength pair 1 is divided by the measured signal for the labelled deconvolution standard at wavelength pair 2, resulting in a wavelength signal conversion. Then, the signal of the label for the sample at wavelength pair 2 at which only the W amino acid type was detected is multiplied by the wavelength signal conversion, to reveal the signal at wavelength pair 1 deriving from the W amino acid type. This signal is subtracted from the total signal at wavelength pair 1 to reveal the signal exclusively from the Y amino acid type.

Alternatively, two deconvolution standards of known protein concentration are used to deconvolute between two labelled types of amino acids detected at the same excitation and emission wavelength pair. Among the labelled amino acid types being deconvoluted, the first deconvolution standard has only the amino acid type whose signal is not known based on the excitation-emission wavelength pair in which only one amino acid type was detected (wavelength pair 2). The second deconvolution standard has both the amino acid type whose signal is known and the amino acid type whose signal is not known. The first deconvolution standard is measured at the excitation-emission wavelength pair at which both amino acid types are detected (wavelength pair 1). The second deconvolution standard is measured at the excitation-emission wavelength pair at which both amino acid types are detected (wavelength pair 1). The amino acid concentration of the detected amino acid type in the first deconvolution standard is known because the number of amino acids of this amino acid type is known and the protein concentration of the first deconvolution standard is known; these are multiplied to reveal the amino acid concentration of this amino acid type in the first deconvolution standard. The signal measured for the first deconvolution standard at wavelength pair 1 is divided by the amino acid concentration of this amino acid type for the first deconvolution standard at wavelength pair 1 to reveal the signal per amino acid concentration for the amino acid type being deconvoluted which is present in the first deconvolution standard.The amino acid concentrations of both amino acid types being deconvoluted in the second deconvolution standard are known because the numbers of both amino acid types in the second deconvolution standard are known and the protein concentration of the second deconvolution standard is known. The amino acid concentration of the amino acid type provided in the first deconvolution standard is multiplied by the signal per amino acid concentration for that amino acid type which was calculated using the first deconvolution standard. This provides the signal for that amino acid type within the second deconvolution standard . The signal for that amino acid type within the second deconvolution standard at wavelength pair 1 is subtracted from the total signal measured at wavelength pair 1, which reveals the signal for the other amino acid type at wavelength pair 1. This is the same amino acid type whose signal is separately detected at wavelength pair 2. The measured signal for this amino acid type of the second deconvolution standard at wavelength pair 1 is divided by the measured signal for this amino acid type of the second deconvolution standard at wavelength pair 2, resulting in a wavelength signal conversion. Then, the signal of the label for the sample at wavelength pair 2 at which only one amino acid type was detected is multiplied by the wavelength signal conversion, to reveal the signal at wavelength pair 1 deriving from the separately detected amino acid type. This signal is subtracted from the total signal at wavelength pair 1 to reveal the signal exclusively from the other amino acid type. In this way, a signal deriving from two amino acid types is split into two signals, each deriving exclusively from one amino acid type, so that the number of signals equals the number of amino acid types labelled and measured in the sample. For example, the first deconvolution standard only contains Y amino acids and does not contain any W amino acids. The first deconvolution standard has a known Y amino acid concentration. The second deconvolution standard contains both Y and W amino acids. The second deconvolution standard has known Y and known W amino acid concentrations. The first deconvolution standard is fluorescently labelled and the fluorescence is detected at an excitation and emission wavelength pair at which both W and Y amino acid types are detected (wavelength pair 1; excitation: 310 nm, emission: 450 nm). The second deconvolution standard is fluorescently labelled and the fluorescence is detected at an excitation and emission wavelength pair at which both W and Y amino acid types are detected (wavelength pair 1; excitation: 310 nm, emission: 450 nm). The fluorescence intensity for the first deconvolution standard at wavelength pair 1 is divided by the amino acid concentration of the Y amino acid type for the first deconvolution standard to reveal the fluorescence intensity per Y amino acid concentration. This fluorescence intensity per Y amino acid concentration is multiplied by the known Y amino acid concentration of the second deconvolution standard to reveal the fluorescence intensity from the Y amino acid type of the second deconvolution standard at wavelength pair 1. This is subtracted from the total fluorescence intensity measured for the second deconvolution standard at wavelength pair 1 to reveal the fluorescence intensity for the W amino acid type at wavelength pair 1. The fluorescence intensity for the W amino acid type of the second deconvolution standard at wavelength pair 1 is divided by the fluorescence intensity for the W amino acid type of the second deconvolution standard at wavelength pair 2 (wavelength pair 2; excitation: 355 nm, emission: 450 nm) to reveal the wavelength signal conversion. The fluorescence intensity for the W amino acid type measured for the sample at wavelength pair 2 is multiplied by the wavelength signal conversion to obtain the fluorescence intensity measured for the W amino acid type at wavelength pair 1. The fluorescence intensity measured for the W amino acid type of the sample at wavelength pair 1 is

subtracted from the fluorescence intensity measured for the W and Y amino acid types of the sample at wavelength pair 1 to reveal the fluorescence intensity measured for the Y amino acid type of the sample at wavelength pair 1. In this way, separate fluorescence intensities for the W and Y amino acid types are obtained.

Fluorescence from the same fluorescently labelled deconvolution standard solution is then measured at an excitation and emission wavelength pair at which only the W amino acid type is detected (wavelength pair 2; excitation: 355 nm, emission: 450 nm). The same fluorescently labelled deconvolution standard solution is measured at wavelength pair 1 and 2, so there has been no dilution. The measured signal for labelled deconvolution standard at wavelength pair 1 is divided by the measured signal for the labelled deconvolution standard at wavelength pair 2, resulting in a wavelength signal conversion. Then, the signal of the label for the sample at wavelength pair 2 at which only the W amino acid type was detected is multiplied by the wavelength signal conversion, to reveal the signal at wavelength pair 1 deriving from the W amino acid type. This signal is subtracted from the total signal at wavelength pair 1 to reveal the signal exclusively from the Y amino acid type.

Alternatively, the signals of the fluorescence intensity can be deconvoluted in time. For example, the kinetics of one labelling reaction may be faster than the kinetics of another labelling reaction. In some embodiments, the signal is monitored and the measurement is taken at the time point where one labelling reaction has reached completion and the other labelling reaction has not begun. In some embodiments, the fluorescence intensity is monitored and the measurement is taken at the time point where one labelling reaction has reached completion and the other labelling reaction has not begun.

In some embodiments, the measured label is background corrected. In a preferred embodiment, the measured label is fluorescence intensity, and the fluorescence intensity for each labelled amino acid type is background corrected. The fluorescent background is subtracted from the fluorescence intensity to produce a background corrected fluorescence value. Any background correction technique known in the art can be used. In some embodiments, to calculate the background fluorescence, the fluorescent dye solution is combined with an equal volume of buffer, rather than of protein. In some embodiments, the fluorescent dye solution is combined with an equal volume of buffer to the volume of protein-containing solution that was supplied during the labelling reaction. The fluorescence intensity detected from the dye and buffer solution is subtracted from the fluorescence intensity detected from the dye and protein solution to provide a background corrected fluorescence signature. Alternatively, a titration curve can be used to determine the low concentration limit of fluorescence, which can be identified as the background. The detection limit is identified as the first concentration of protein detectable over this limit.

The measured label of an amino acid type in the sample is related to the amino acid concentration of that amino acid type in the sample. In some embodiments, the measured label of an amino acid type in the sample is linearly related to the amino acid concentration of that amino acid type in the sample. In some embodiments, the measured label of an amino acid type in the sample is nonlinearly related to the amino acid concentration of that amino acid type in the sample. In some embodiments, examples of a nonlinear relationship include a power law, polynomial equation, or exponential equation. In some embodiments, the measured label of an amino acid type in the sample is related to the amino acid concentration of that amino acid type in the sample with a polynomial equation.

The measured label, amino acid concentration or number of amino acids of each labelled amino acid type provides a signature for that labelled amino acid type in the sample. The signature of each of the labelled amino acid types in the sample can be compared to the signature of the same amino acid types in a reference in order to identify the presence and/or amount of one or more proteins, peptides, oligopeptides, polypeptides, protein complexes, subproteomes, or proteomes of interest in the sample.

When all the amino acids of an amino acid type are labelled in the sample, and any amino acids or proteins used for the calibration curve or standard, the measured label (e.g. signal of the label) reveals the concentration of all (e.g. 100%) and/or the number of all (e.g. 100%) amino acids of that amino acid type in the sample. When the same proportion (e.g. 80%) of amino acids of an amino acid type are labelled in the sample and any amino acids or proteins used for the calibration curve or standard, the measured label (e.g. signal of the label) reveals the concentration of all (e.g. 100%) and/or the number of all (e.g. 100%) amino acids of that amino acid type in the sample. This is because when the same proportion of amino acids are labelled in any proteins measured, this proportion factors out of the conversion between measured label (e.g. signal of the label) and concentration or number of amino acids for the sample.

### Measuring the concentration of each labelled amino acid type

In some embodiments, the concentration of each labelled amino acid type in the sample is measured. In some embodiments, the concentration of each labelled amino acid type in the sample is calculated from the measured label (e.g. measured signal of the label) of the amino acid type. In some embodiments, the concentration of an amino acid type in the sample is calculated from the measured fluorescence intensity of that amino acid type in the sample. In some embodiments, there is a linear relationship between the concentration of each labelled amino acid type in the sample and the measured label. In alternative embodiments, there is a nonlinear relationship between the concentration of each labelled

amino acid type in the sample and the measured label. In some embodiments, examples of this nonlinear relationship include a polynomial relationship, power law relationship, or exponential relationship. In some embodiments, there is a polynomial relationship between the concentration of each labelled amino acid type in the sample and the measured label. In some embodiments, there is a power law relationship between the concentration of each labelled amino acid type in the sample and the measured label. In some embodiments, there is an exponential relationship between the concentration of each labelled amino acid type in the sample and the measured label. In some embodiments, when the two or more amino acid types are labelled with a fluorescent dye, there is a linear relationship between the concentration of each labelled amino acid type in the sample and the measured fluorescence intensity. In some embodiments, when the two or more amino acid types are labelled with a fluorogenic dye, there is a linear relationship between the concentration of each labelled amino acid type in the sample and the measured fluorescence intensity. Fluorescence intensity increases as the concentration of that amino acid type in the sample increases.

In some embodiments, the concentration of amino acids of each of two or more labelled amino acid types in the sample is determined from the measured label of the same two or more amino acid types in the sample using a calibration curve or standard.A calibration curve or standard is a general analytical chemistry method for determining the concentration of a substance in an unknown sample by comparing the unknown sample to a set of standard samples, or one standard sample, of known concentration. When using a calibration curve, the values of the label for more than one standard of known amino acid concentration of an amino acid type is plotted. The data is fit to a calibration curve, and the calibration curve provides the relationship between amino acid concentration of a labelled amino acid type and value of the label of the amino acid type. When a single standard of known amino acid concentration of an amino acid type is plotted, the whole calibration curve is not available, and the amino acid concentration of labelled amino acid type and value of the label of the amino acid type of a single protein standard provide the relationship between amino acid concentration of a labelled amino acid type and value of the label of the amino acid type. Because less information is available with a single protein standard, this can be used when the relationship between the value of the label and amino acid concentration of the amino acid type is linear and passes through the origin, and when the value of the label of the amino acid type has been background corrected.

In some embodiments the signal of the label is plotted as a function of the amino acid concentration for each amino acid concentration of each calibration protein to provide a calibration plot for each amino acid type. In some embodiments, the signal of the label is measured and plotted in arbitrary units (AU). Each calibration plot is fit to provide a calibration curve. In some embodiments, a calibration curve determines the relationship between fluorescence intensity, or background corrected fluorescence intensity, and the amino acid concentration for each labelled amino acid type in the sample. In some embodiments, the fluorescence intensity or background corrected fluorescence intensity is plotted in arbitrary units (AU). For example, a calibration curve determines the relationship between the fluorescence intensity measured for the amino acid type tryptophan (W) and the corresponding amino acid concentration of W.

In some embodiments, fitting the (linear) calibration plot to provide the calibration curve is performing linear least squares regression. An equation is calculated for the best fit line to calibrate between the signal of the label of an amino acid type and amino acid concentration. In some embodiments this is a linear equation. In some embodiments, this linear fit is constrained to pass through the origin.

In some embodiments, when the best fit line is calculated using linear regression, the equation of the best fit line for amino acid type n is equation 5:

$$Label\ Value_n = m_n \times A.A.Concentration_n + b_n$$

where $Label\ Value_n$ is the value of the label of amino acid type n in AU, $m_n$ is the slope of the best fit line in AU / amino acid concentration for amino acid type n, $A.A.\ Concentration_n$ is the amino acid concentration of amino acid type $n$, and $b_n$ is the value of the label when the amino acid concentration of amino acid type n is zero. The output of the fit is $m_n$ and $b_n$.

When signal of an amino acid type for the sample is measured, the calibration determined by the fit can be used to transform the signal measured for the amino acid type for the sample into the amino acid concentration of the amino acid type of the sample. In some embodiments, the output of the fit from equation 5 is used to convert the value of the label of amino acid type n in AU to the amino acid concentration of amino acid type n, using equation 6:

$$A.A.Concentration_n = \frac{Label\ Value_n - b_n}{m_n}$$

where $A.A.\ Concentration_n$ is the amino acid concentration of amino acid type $n$, $Label\ Value_n$ is the measured value of the label of amino acid type $n$ in AU, $b_n$ is the value of the label when the amino acid concentration of amino acid type n is zero, and $m_n$ is the slope of the calculated best fit line in AU / amino acid concentration for amino acid type n. In some embodiments, this is described as the inverse of the calibration function; it is the inverse of the calibration function because

the fit from equation 5 has been inverted.

In some embodiments, when the best fit line is calculated using linear regression and, the equation of the best fit line for amino acid type n is equation 7:

$$Label\ Value_n = m_n \times A.A.Concentration_n$$

where *Label Value$_n$* is the value of the label of amino acid type n in AU, $m_n$ is the slope of the best fit line in AU / amino acid concentration for amino acid type n, and *A. A. Concentration$_n$* is the amino acid concentration of amino acid type n. The output of the fit is $m_n$. In some embodiments, equation 7 is used if the linear fit is constrained to pass through the origin.

In some embodiments, the output of the fit from equation 7 is used to convert the value of the label of amino acid type n in AU to the amino acid concentration of amino acid type n, using equation 8:

$$A.A.Concentration_n = \frac{Label\ Value_n}{m_n}$$

where *A. A. Concentration$_n$* is the amino acid concentration of amino acid type n, *Label Value$_n$* is the measured value of the label of amino acid type n in AU, and $m_n$ is the slope of the calculated best fit line in AU / amino acid concentration for amino acid type n. In some embodiments, equation 8 is used if the linear fit is constrained to pass through the origin. In some embodiments, $m_n$ is the calibration factor, which is the slope of the line of the calibration curve, and $1/m_n$ is described as the inverse of the calibration factor for amino acid type *n*, this is the inverse of the calibration factor because the fit from equation 7 has been inverted. The inverse of the calibration factor is the inverse of the slope of the line of the calibration curve, and the measured label value of amino acid type *n* is multiplied by this to calculate the amino acid concentration for amino acid type n. For example, the signal of the label is plotted for 1 μM, 5 μM, 10 μM, 20 μM, 50 μM, and 100 μM amino acid concentrations of 5 calibration proteins. There is one plot for one amino acid type being calibrated, so there is one plot for the amino acid type tryptophan (W) and another plot for the amino acid type lysine (K) and the slope of the best fit line is calculated for these plots individually; the slopes are not related. The slope of the best fit line for the amino acid type tryptophan (W) is 10 AU/ μM, so the calibration factor is 0.1 μM / AU. The slope of the best fit line for the amino acid type lysine (K) is 50 AU / μM, so the calibration factor is 0.02 μM / AU.

There is no requirement to perform this step whenever the measured signal of the labels for the sample will be transformed into the labelled amino acid concentrations for the sample. In some embodiments, the calibration curve for each labelled amino acid type includes and extends beyond the linearity range for the labelling reaction for each amino acid type. In some embodiments, the data used to calculate the calibration curve contains equal spacing in amino acid concentration such that the linear least squares regression is unbiased. In some embodiments, the data used to calculate the calibration curve is normalized. In some embodiments, the logarithm is taken of the amino acid concentration and signal data prior to the fit to avoid biasing the fit to higher amino acid concentrations if a wide amino acid concentration range is surveyed.

Alternatively, the calibration factor is determined by dividing the signal of the label of a standard solution containing a known amino acid concentration of an amino acid or protein by the known amino acid concentration of the amino acid or protein. In some embodiments, the calibration factor for each amino acid type is determined using data from one amino acid concentration of one standard (calibration protein or amino acid). A calibration curve is not available in this embodiment because there is only one point used for the calibration, and a curve requires at least two points. Each standard has a known amino acid concentration of the amino acid type being calibrated, or a known protein concentration and number of amino acids of the amino acid type being calibrated which are multiplied to provide the amino acid concentration of the amino acid type being calibrated. All or a constant proportion of the amino acid type being calibrated is labelled, so the signal of the label measured for each calibration protein is proportional to the amino acid concentration of the amino acid type being calibrated for each calibration protein. The amino acid concentration for the amino acid type being calibrated is divided by the signal of the label measured for the amino acid type being calibrated to provide the amino acid concentration per signal of the label measured. For example, the signal of the label measured for 10 μM of the amino acid type tryptophan (W) is 100 AU. Therefore, the calibration factor for the amino acid type tryptophan (W) is 10 μM / 100 AU = 0.1 μM / AU. The signal measured for 10 μM of the amino acid type lysine (K) is 500 AU. Therefore, the calibration factor for the amino acid type lysine (K) is 10 μM / 500 AU = 0.02 μM / AU. This is performed for each of the amino acid types which will be labelled and measured in the experiments. For example, if two amino acid types will be labelled and measured in the experiments, there are two calibration factors and if three amino acid types will be labelled and measured in the experiments, there are three calibration factors.

In some embodiments, the calibration or calibration factor for each amino acid type is determined using data from one or more amino acid concentrations of a free amino acid. The free amino acid is not incorporated within a protein chain or a peptide. In some embodiments, more than one amino acid concentration of a free amino acid is used. In some

embodiments, one amino acid concentration of a free amino acid is used.

In some embodiments, one or more amino acid types is calibrated using a calibration amino acid, and one or more amino acid types is calibrated using one or more protein concentrations of one or more calibration proteins. In some embodiments, when the data determining the relationship between signal of the label and amino acid concentration for each amino acid type is determined, data from the free amino acid in solution can be included together with data from the amino acid incorporated within amino acid sequences.

This step only needs to be performed once and the results can be stored and/or supplied to the user; there is no requirement to perform this step whenever the measured signal of the labels for the sample will be transformed into the labelled amino acid concentrations for the sample.

In some embodiments, the concentration of amino acids of each of two or more labelled amino acid types in the sample is determined from the measured fluorescence intensity of the same two or more amino acid types in the sample using a calibration factor. Each type of amino acid labelled and measured has a different calibration. Each type of amino acid labelled and measured has a different calibration factor. The calibration factor converts between the measured label of the sample, which is often in arbitrary units (AU), and the amino acid concentration of that amino acid type in the sample. The calibration factor determines the relationship between the measured label and the amino acid concentration for each labelled amino acid type in the sample.

This is performed for each of the amino acid types which will be labelled and measured in the experiments. For example, if two amino acid types will be labelled and measured in the experiments, there are two calibration factors and if three amino acid types will be labelled and measured in the experiments, there are three calibration factors

To identify and quantify a sample, the user only needs to label and measure the label of two or more amino acid types within the sample. The calibration function or calibration factor for any amino acid type can be provided for several detection settings, for example, the calibration factor for fluorescence based detection can be provided according to the excitation wavelength, emission wavelength, and gain or photomultiplier (PMT) setting of the instrument.

This calibration factor or calibration function is independent of the amino acid sequence and is calculated by measuring the label at one or more amino acid concentrations of one or more calibration amino acids or calibration proteins of known and non-zero amino acid concentrations of each labelled amino acid type. In some embodiments, the one or more amino acid concentrations of the one or more calibration amino acids or proteins is measured at the same conditions (e.g. excitation and emission wavelength pair) at which the sample and any optional experimental reference is measured. In some embodiments, a different one or more calibration amino acids or calibration proteins is used for each amino acid type. In some embodiments, the calibration function is non-linear. In preferred embodiments, the calibration function is linear, providing a scalar calibration factor. If more than one amino acid concentration of one or more calibration amino acids or calibration proteins is used, then the calibration factor for each amino acid type is calculated by fitting the data describing the relationship between the known amino acid concentration of that amino acid type and label value (e.g measured signal of the label) of that amino acid type. If one amino acid concentration of one calibration amino acid or protein is used, then the calibration factor for each amino acid type is calculated by dividing the measured label for the amino acid type by the known amino acid concentration of the amino acid type, providing what label value (e.g. signal) would be measured for the label of each amino acid type for a known amino acid concentration of that amino acid type.

In some embodiments, the calibration function or calibration factor for each amino acid type is determined using data from several amino acid concentrations of one or more calibration proteins. Each calibration protein has a known amino acid concentration of the amino acid type being calibrated, or a known protein concentration and number of amino acids of the amino acid type being calibrated which are multiplied to provide the amino acid concentration of the amino acid type being calibrated. In some embodiments, all or the same proportion of the amino acid type being calibrated is labelled for each calibration protein, so the signal of the label measured for each calibration protein is proportional to the amino acid concentration of the amino acid type being calibrated for each calibration protein. For example, if the two or more amino acid types to be labelled are tryptophan (W) and lysine (K), then 90% of the tryptophan (W) amino acids in the sample and any amino acids or proteins used for calibration are labelled and 90% of the lysine (K) amino acids in the sample and any amino acids or proteins used for calibration are labelled. In some embodiments, the proportion of amino acids labelled does not need to be the same proportion for each amino acid type. For example, if the two or more amino acid types to be labelled are tryptophan (W) and lysine (K), then 90% of the tryptophan (W) amino acids in the sample and any amino acids or proteins used for calibration are labelled and 80% of the lysine (K) amino acids in the sample and any amino acids or proteins used for calibration are labelled. In another example, if the two or more amino acid types to be labelled are tryptophan (W), lysine (K) and tyrosine (Y), then 90% of tryptophan (W) amino acids in the sample and any amino acids or proteins used for calibration are labelled, 85% of lysine (K) amino acids in the sample and any amino acids or proteins used for calibration are labelled and 80% of tyrosine (Y) amino acids in the sample and any amino acids or proteins used for calibration are labelled.

In some embodiments, all, or the same proportion of amino acids within any experimentally measured proteins (the sample, any standards such as proteins or amino acids, and any experimental reference protein, peptide, oligopeptide, polypeptide, protein complex, subproteome, or proteome of interest, or calibration curve are the same. This ensures

that labeling of a proportion of amino acids within an amino acid type cancels out and is not observed in the results. When the same proportion (e.g. 80%) of amino acids of an amino acid type are labelled in the sample and any amino acids or proteins used for the calibration, the signal of the label reveals the concentration of all (e.g. 100%) and/or the number of all (e.g. 100%) amino acids of that amino acid type in the sample. This is because when the same proportion of amino acids are labelled, this proportion factors out of the conversion between signal of the label and concentration or number of amino acids for the sample or the calibration.

**Measuring the number of amino acids of each labelled amino acid type**

In some embodiments, when the molar protein concentration of the sample is known, the number of amino acids of each labelled amino acid type in the sample can be measured. The number of amino acids of an amino acid type is equal to the amino acid concentration of that amino acid type divided by the protein concentration. The number of amino acids of an amino acid type is equivalent to the change in amino acid concentration of the amino acid type with protein concentration.

The number of amino acids of a labelled amino acid type in the sample is calculated from the measured label of that labelled amino acid type in the sample. The measured label provides the amino acid concentration of that labelled amino acid type in the sample. Preferably, the amino acid types are labelled with a fluorogenic dye and the number of amino acids of a labelled amino acid type is calculated from the fluorescence intensity for that labelled amino acid type in the sample. The fluorescence intensity provides the amino acid concentration of that labelled amino acid type in the sample.

In some embodiments, the number of amino acids of a labelled amino acid type for the sample is calculated from Equation 9:

$$number\ of\ amino\ acids\ = \frac{molar\ amino\ acid\ concentration}{total\ molar\ protein\ concentration\ of\ the\ sample}$$

The measured label provides the amino acid concentration. One or more amino acid concentrations of one or more calibration amino acids or calibration proteins is used to convert the measured label of the sample into the molar amino acid concentration of a labelled amino acid type in the sample using a calibration curve or standard.The molar amino acid concentration of a labelled amino acid type in the sample is divided by the total molar protein concentration of the sample to provide the number of amino acids of an amino acid type for the sample. This calculation is carried out on each labelled amino acid type in the sample. For example, if the amino acid types W, K and Y were labelled in the sample, then equation 9 is carried out on each of amino acid types W, K and Y. For example, the number of W amino acids in the sample is calculated by dividing the molar amino acid concentration of W in the sample by the total molar protein concentration of the sample. The number of K amino acids in the sample is calculated by dividing the molar amino acid concentration of K in the sample by the total molar protein concentration of the sample. The number of Y amino acids in the sample is calculated by dividing the molar amino acid concentration of Y in the sample by the total protein concentration of the sample.

**The number** of amino acids of each labelled amino acid type in the sample provides a unique signature for the sample. For example, three amino acid types in the sample are labelled; W, K and C, wherein C is the combination of $C_D$ and $C_R$. The number of amino acids in each amino acid type is determined. It is determined that the sample has 3 amino acids of the amino acid type of W, 5 amino acids of the amino acid type of K and 7 amino acids of the amino acid type of C, in a protein molecule within the sample. The number of amino acids is provided per protein molecule in the sample because this is calculated by dividing the molar amino acid concentration of the amino acid type by the total molar protein concentration and therefore provides the number of amino acids per protein in the sample. Therefore, the signature for the sample is 3W, 5K and 7C. The signature of the sample can be compared to the signature of a reference to identify the presence of protein, peptide, oligopeptide, polypeptide, protein complex, subproteome, or proteome of interest in a sample.

In some embodiments, the total molar protein concentration of the sample is known, or is determined using standard techniques in the art. In some embodiments, the total molar protein concentration is known passively. For example, the total molar protein concentration has been determined, for example, via the $A_{280}$ signal, to determine the molar protein concentration.

In some embodiments, the total protein concentration is known actively, i.e. the protein concentration in mg/mL has been actively determined. For example, the mass protein concentration has been weighed out or measured, so the mass concentration of total protein in the sample is known. For example, 0.05mg/mL of protein has been weighed out and dissolved in 1 mL of buffer and therefore it is known that the total mass protein concentration is 0.05 mg/mL. As another example, methods known in the art are used to determine the mass protein concentration of the sample. It is not possible to calculate the number of amino acids of each of two or more amino acid types of the sample when the total mass protein concentration has been determined. If the total protein concentration were provided by mass, and the amino acid concentration were provided by mass, equation 9 does not allow calculation of the number of amino acids in the sample.

Instead of calculating the number of amino acids per protein in the sample, the result of dividing the amino acid mass concentration by the protein mass concentration would be the relative fraction of mass of the protein contributed by amino acids of the labelled amino acid type. Determining the number of amino acids of each of two or more amino acid types in the sample from this information would require knowledge of the exact protein molecular weight (MW), which depends on the protein sequence which is not available for the sample because the identity of the sample is unknown. MW also cannot be calculated from protein size such as hydrodynamic radius ($R_H$) of the sample unless the level of intrinsic disorder of the sample is known, and this is not available for the sample because the identity of the sample is unknown (Figure 15).

For the number of amino acids in equation 9 to be calculated for the sample, the amino acid concentration must be the molar amino acid concentration rather than the mass amino acid concentration; this is required for the units to cancel revealing a unitless number of amino acids. However, it was discovered that the methods of the invention can still be used when only the total mass protein concentration of the sample is known because the relevant transformations can be performed on the protein, peptide, oligopeptide, polypeptide, protein complex, subproteome, or proteome of interest, for which the protein identity is, by definition, known.

In some embodiments, the molar protein concentration of the sample is known actively if exclusively the N-terminus or C-terminus of the protein is labelled with a fluorogenic dye. For example, the N-terminus of the protein is site-specifically modified via a biomimetic transamination reaction with pyridoxal-5-phosphate (PLP), which oxidizes the N-terminus to a ketone (all amino acid types except glycine) or an aldehyde (the glycine amino acid type), which is then reacted with a fluorescent label bearing an alkoxyamine reactive group, forming a stable covalent oxime linkage, as described in (https://doi.orq/10.1002/9780470559277.ch100018). This provides one label per protein so the total protein concentration is equivalent to the total concentration of the label in the sample.

## Reference

In some embodiments, the measured label (e.g. signal of the label) of two amino acid types in the sample is compared to a reference of the known label values (e.g. signals of the labels, e.g. fluorescence intensity of the labels or intensity of the mass to charge ratios) of the same two or more amino acid types in a solution containing the protein, peptide, oligopeptide, polypeptide, protein complex, subproteome, or proteome of interest at one or more protein concentrations. In some embodiments, the amino acid concentration of two or more amino acid types in the sample is compared to a reference of the amino acid concentrations of the same two or more amino acid types in a solution containing the protein, peptide, oligopeptide, polypeptide, protein complex, subproteome, or proteome of interest at one or more concentrations. In some embodiments, when the reference is the amino acid concentration or the known label value (e.g signal of the label), the reference is a group of functions that provides a value for the reference as a function of the concentration of the protein, peptide, oligopeptide, polypeptide, protein complex, subproteome, or proteome of interest. In some embodiments, the reference provides the value that is measured for the sample (e.g. amino acid concentration, or signal of the label of each labelled amino acid type, such as fluorescence intensity or intensity of signal to mass ratio) as a function of the concentration, for any protein, peptide, oligopeptide, polypeptide, protein complex, subproteome, or proteome of interest. In some embodiments, these functions are linear, and provide a line in n-dimensional space (where *n* is the number of amino acid types being labelled in the sample). For example, if W and K amino acid types are being labelled in the sample, then the reference is the line of W and K in a 2-dimensional space. The values measured for the sample (e.g. amino acid concentration of *n* amino acid types, or signal of the label of *n* amino acid types) always provide a point in n-dimensional space. The presence of a protein, peptide, oligopeptide, polypeptide, protein complex, subproteome, or proteome of interest can be detected when the sample point is on the reference line, or within an error margin to the reference line. Whenever the presence of a protein, peptide, oligopeptide, polypeptide, protein complex, subproteome, or proteome of interest is detected, the concentration of the protein, peptide, oligopeptide, polypeptide, protein complex, subproteome, or proteome of interest can also determined by solving the reference functions for the concentration. In some embodiments, the functions which comprise the reference are generated using set of parametric equations 1, 2, 3, and 4, or vector function 1, 2, 3, or 4.

In some embodiments, the measured label of two or more labelled amino acid types in the sample is compared to a reference of the one or more known label values (e.g. signal of the labels, e.g. fluorescence intensity of the labels, mass, vibrational mode, or radioactive decay of the labels, or the M-F-N-R regions of the labels) of the same two or more amino acid types in a protein, peptide, oligopeptide, polypeptide, protein complex, subproteome, or proteome of interest. In some embodiments, the amino acid concentration of two or more labelled amino acid types in the sample is compared to a reference of the one or more amino acid concentrations of the same two or more amino acid types in a sample containing the protein, peptide, oligopeptide, polypeptide, protein complex, subproteome, or proteome of interest. In some embodiments, the number of amino acids of two or more labelled amino acid types in the sample is compared to a reference of the number of the same two or more amino acid types in a protein, peptide, oligopeptide, polypeptide, protein complex, subproteome, or proteome of interest.

The unit of the reference must be the same as the unit determined for the sample. For example, the amino acid

concentrations of an amino acid type in the sample is compared to the amino acid concentrations of the same amino acid type in the reference. If the unit of the reference is not the same, then the unit of the reference is converted into the same unit of the sample, or vice versa. In some embodiments, the unit of the reference is converted. In some embodiments, the unit of the sample is converted. For example, fluorescence intensity of an amino acid type in the sample cannot be compared to the amino acid concentration of the same amino acid type in the reference because fluorescence intensity and amino acid concentration are different units. Instead, the amino acid concentration of the amino acid type in the reference can be converted to the fluorescence intensity using set of parametric equations 3. Then, the fluorescence intensity of an amino acid type in the sample is compared to the fluorescence intensity of the same amino acid type in the reference. Alternatively, the unit of the sample can be converted into the same unit as the reference. For example, the fluorescence intensity of the amino acid type in the sample is converted to the amino acid concentration of the amino acid type using a calibration curve, or standard, and the amino acid concentration of the amino acid type in the sample is compared to the amino acid concentration of that same amino acid type in the reference. If the molar concentration of the sample is known, then the amino acid concentration of each labelled amino acid type in the sample can be used to calculate the number of amino acids of each labelled amino acid type in the sample using the methods disclosed herein. The number of amino acids of a labelled amino acid type in the sample is compared to the number of that same amino acid type in the reference.

In some embodiments, if the value (i.e. measured labels, amino acid concentrations and/or number of amino acids of two or more amino acid types) of the sample, is the same as, or within an error margin to the value (i.e. known label values, amino acid concentrations and/or number of amino acids of the same two or more amino acid types) of the reference for each amino acid type, this indicates that the protein, peptide, oligopeptide, polypeptide, protein complex, subproteome, or proteome of interest, is present in the sample at a specific concentration and/or amount. Conversely, in some embodiments, a difference in the value (i.e. measured labels, amino acid concentrations and/or number of amino acids of two or more amino acid types) of sample, outside of the error margin, compared to the value (i.e. known label values, amino acid concentrations and/or number of amino acids of the same two or more amino acid types) of the reference indicates that the reference protein is not present in the sample at any concentration and/or amount.

In some embodiments, the reference has been previously determined. For example, information relating the known label values, amino acid concentrations or number of amino acids of two or more amino acid types to the identity and/or concentration of one or more proteins, peptides, oligopeptides, polypeptides, protein complexes, subproteomes or proteomes of interest have been previously determined. For example, the fluorescence intensities of two or more amino acid types at one or more concentrations of a solution containing the protein, peptide, oligopeptide, polypeptide, protein complex, subproteome, or proteome of interest, or the amino acid concentrations of two or more amino acid types at one or more concentrations in a solution containing the protein, peptide, oligopeptide, polypeptide, protein complex, subproteome, or proteome of interest, or, the number of amino acids of two or more amino acid types in the amino acid sequence of the protein, peptide, oligopeptide, polypeptide, protein complex, subproteome, or proteome of interest has been previously determined. In some embodiments, the reference is stored in a medium that can be copied, accessed or transmitted. Information indicating the known label values and/or amino acid concentrations, and/or number of amino acids of the same two or more amino acid types as the amino acid types that have been labelled in the sample as identifying the presence and/or concentration of one or more proteins, peptides, oligopeptides, polypeptides, protein complexes, subproteomes, or proteomes of interest can be stored in a medium that can be copied, accessed or transmitted. The name of the reference (e.g. the name of the protein, peptide, oligopeptide, polypeptide, protein complex, subproteome, or proteome of interest) can also be stored in a medium that can be copied, accessed or transmitted. For example, the fluorescence intensity of two or more amino acid types at one or more concentrations of a solution containing the protein, peptide, oligopeptide, polypeptide, protein complex, subproteome, or proteome of interest, or the amino acid concentration of two or more amino acid types at one or more concentrations of a solution containing the protein, peptide, oligopeptide, polypeptide, protein complex, subproteome, or proteome of interest, or, the number of amino acids of two or more amino acid types in the amino acid sequence of the protein, peptide, oligopeptide, polypeptide, protein complex, subproteome, or proteome of interest is stored in a medium that can be copied, accessed or transmitted. In some embodiments, the reference may be sourced or derived from any suitable data source, including, for example databases, public databases of genomic information, published data, or data generated for a specific population of reference subject which may each have a common attribute (e.g., type of organism, disease status, pathogen, tissue type, cell type, prognostic value, age or response to a drug). For example, the amino acid concentrations or known label value (e.g. signal of the label, e.g. fluorescence intensity) of a solution containing a protein, peptide, oligopeptide, polypeptide, protein complex, subproteome, or proteome of interest at different concentrations, and/or, the number of amino acids of each amino acid type in the amino acid sequence of the protein, peptide, oligopeptide, polypeptide, protein complex, subproteome, or proteome of interest and/or the name or identifier of the protein, peptide, oligopeptide, polypeptide, protein complex, subproteome, or proteome of interest can be accessed from a library or database. In some embodiments, the reference provides the known label values and/or amino acid concentrations of the same two or more amino acid types as the amino acid types that have been labelled in the sample of each protein, peptide, oligopeptide, polypeptide, protein complex, subproteome, or proteome of interest as a set of parametric equations or a vector function depending on the

common parameter of concentration. In other embodiments, the reference provides the number of amino acids of the same two or more amino acid types as the amino acid types that have been labelled in the sample of each protein, peptide, oligopeptide, polypeptide, protein complex, subproteome, or proteome of interest.

In some embodiments, the reference provides the number of amino acids of the same two or more amino acid types as the amino acid types that have been labelled in the sample and the number of amino acids is determined using Power BI; a Microsoft analytic programme, Microsoft Excel or Python.

In some embodiments, the reference amino acid concentrations or known label values (e.g. signals) of two or more amino acid type in a solution containing the protein, peptide, oligopeptide, polypeptide, protein complex, subproteome, or proteome of interest at one or more concentrations can be calculated from the number or mean number of amino acids of each amino acid type and the concentration of the protein, peptide, oligopeptide, polypeptide, protein complex, subproteome, or proteome of interest in the amino acid sequence of the protein, peptide, oligopeptide, polypeptide, protein complex, subproteome, or proteome of interest and may include the calibration factors for each amino acid type of interest using set of parametric equations 1, 2, 3, or 4 or vector functions 1, 2, 3, or 4.

In some embodiments, the reference may be enhanced with information relating to the frequency distributions observed for the number of amino acids across various samples and/or subproteomes and/or proteomes, such as observations about the frequency distribution of the leading digits of the number of amino acids. Benford's law, the Newcomb-Benford law, the Law of First Digits, or the Significant-Digit-Law, provides information about the expected distribution of significant digits (leading numerals of a number) in a diverse set of naturally occurring datasets (especially ones with high orders of magnitude) and can be used to detect pattern or the lack thereof, enabling the detection of anomalies in number patterns. This law states that the expected distribution of leading significant digits is not uniformly distributed but instead follows a particular logarithmic distribution. In Figures 4, 5 and 6 below, P(d) denotes the expected probability under Benford's law of a leading digit d, where d is in {1,2,3,4,5,6,7,8,9}.

It was discovered that the leading digits of mean amino acid numbers for the Human Plasma proteome obey this law (Figure 4), however Viral proteomes deviate somewhat (Figure 5), with the greatest deviation observed for the distribution of leading digits of the mean number of amino acids for Bacterial Proteomes (Figure 6). The lack of agreement with Benford's law for Viral and Bacterial proteomes of interest confirms the information-richness of the signature of the mean number of amino acids, amino acid concentrations, and/or values of the label for two or more amino acid types within any Viral or Bacterial proteome of interest. In contrast, if a uniform distribution for the leading digits of the number of amino acids then P(d) would be 11.11% for all d in {1,2,3,4,5,6,7,8,9}. Additionally, knowing specific amino acids that conform to or deviate from Benford's law or the uniform distribution can be used to assign probabilities of certain signatures occurring within certain sample types.

In some embodiments, the reference is a calculated reference, which is calculated based on sequence data obtained from the publicly available amino acid sequence of the protein, peptide, oligopeptide, polypeptide, protein complex, subproteome, or proteome of interest. Alternatively, if the amino acid sequence or sequences of a protein, peptide, oligopeptide, polypeptide, protein complex, subproteome, or proteome of interest is not publicly available, then the amino acid sequence or sequences of the protein, peptide, oligopeptide, polypeptide, protein complex, subproteome, or proteome of interest can be determined using standard sequencing methods, for example Edman degradation. In some embodiments, the reference is an experimental reference. In some embodiments, when determining the experimental reference, the protein, peptide, oligopeptide, polypeptide, protein complex, subproteome, or proteome of interest is provided at a known molar concentration, two or more amino acid types are labelled as disclosed herein, the label is measured and the measured label is used to determine the number of amino acids of each labelled amino acid type in the protein, peptide, oligopeptide, polypeptide, protein complex, subproteome, or proteome of interest by dividing the amino acid concentration (determined from the measured label) by the known protein, peptide, oligopeptide, polypeptide, protein complex, subproteome, or proteome concentration. Alternatively, in some embodiments, when determining the experimental reference, the protein, peptide, oligopeptide, polypeptide, protein complex, subproteome, or proteome of interest in the experimental reference is provided at a known concentration such as a mass concentration determined via methods known in the art such as the Bradford assay, two or more amino acid types are labelled as disclosed herein, the label is measured and the measured label is used to determine the amino acid concentration of each labelled amino acid type in the protein, peptide, oligopeptide, polypeptide, protein complex, subproteome, or proteome of interest using a calibration curve or standard.Alternatively, in some embodiments, when determining the experimental reference, the concentration of the protein, peptide, oligopeptide, polypeptide, protein complex, subproteome, or proteome of interest in the experimental reference is not known and not determined. This permits identification of the presence of the protein, peptide, oligopeptide, polypeptide, protein complex, subproteome, or proteome of interest in the sample, and determination of the relative concentration and/or amount of the protein, peptide, oligopeptide, polypeptide, protein complex, subproteome, or proteome of interest in the sample. The relative concentration and/or amount of the protein, peptide, oligopeptide, polypeptide, protein complex, subproteome, or proteome of interest in the sample is provided relative to the concentration and/or amount of the protein, peptide, oligopeptide, polypeptide, protein complex, subproteome, or proteome of interest another sample.

In all embodiments, the experimental reference can be simultaneously determined with the testing of the sample, prior to the testing of the sample or after testing of the sample. Typically, as would be understood by the skilled person, the reference is determined or characterized under conditions comparable to those utilized to determine or characterize the sample.

Labelling all or a proportion of the amino acids of an amino acid type in the sample does not affect the calculated reference because all amino acids or the same proportion of amino acids are labelled in the sample and the one or more proteins used for the calibration curve or standard used to convert between fluorescence intensity and amino acid concentration (of the sample or the reference). If all of the amino acids of an amino acid type are labelled in the sample, then all of the amino acids of that amino acid type should also be labelled in the experimental reference. If a proportion of the amino acids of an amino acid type are labelled in the sample, then the same proportion of amino acids of that amino acid type should be labelled in the experimental reference. This is because when the same proportion of amino acids are labelled in any proteins measured experimentally, this proportion factors out of the conversion between the label value (e.g. signal of the label) and concentration or number of amino acids for the sample or the reference.

In some embodiments, both the identity and the protein quantity (concentration and amount) of the sample are unknown. In some embodiments, both the identity and the protein quantity (molar protein concentration and molar protein amount) of the sample are unknown. This is the most common situation encountered in diagnostic settings, because if a sample contains a protein whose identity is unknown, its molar protein concentration cannot be determined without knowing the identity of the protein because this requires knowledge of the protein's exact molecular weight which is determined from its amino acid sequence. When both the identity and the protein quantity (molar protein concentration and molar protein amount) of the sample are unknown, the measurements (amino acid concentrations of two or more labelled amino acid types, or measured label (e.g. signal of the label) of two or more labelled amino acid types) provided for the sample depend on the unknown protein concentration of the sample. Therefore, the reference for the protein, peptide, oligopeptide, polypeptide, protein complex, subproteome, or proteome of interest is a function of the unknown concentration of the sample. A parametric equation describes a group of quantities as functions of a common independent variable, called a parameter. Here, the unknown molar concentration of the sample is the parameter, called *t*, which must be greater than or equal to 0 because negative concentrations are not physically possible.

The general form of the reference is a line in n dimensional space, where n is the number of amino acid types labelled and measured in the sample. The reference can be described with a parametric equation, which specifies how each of the coordinates (amino acid concentration, or signal of the label) varies as a function of the concentration, *t*. The general parametric equation is:

$$n_i(t) = [c_1 t, c_2 t, \cdots, c_n t], \forall t \geq 0$$

wherein $n_i$ is the protein, proteome, peptide, oligopeptide, polypeptide, protein complex, or subproteome of interest, $c_1$ the coefficient for amino acid type 1, $c_2$ the coefficient for amino acid type 2, and $c_n$ the coefficient for amino acid type n labelled and measured in the sample, each provided according to (explained for the example of amino acid type n but also applying to amino acid type 1 and 2):

- when the measurement for the sample is provided in amino acid concentration and the reference line describes a protein, peptide, oligopeptide, polypeptide, or protein complex of interest, $c_n = a_n$, where $a_n$ is the number of amino acids of amino acid type nin the protein, peptide, oligopeptide, polypeptide, or protein complex of interest, with $a_n$ being an integer greater than or equal to 0 ($a_1 \in Z^+$)
- when the measurement for the sample is provided in amino acid concentration and the reference line describes a proteome or subproteome of interest, $c_n = w_n$, where $w_n$ is the weighted mean number of amino acids of amino acid type n in the proteome or subproteome of interest
- when the measurement for the sample is provided in measured label (e.g. signal of the label) and the reference line describes a protein, peptide, oligopeptide, polypeptide, or protein complex of interest is $c_n = a_n f_n$, where $a_n$ is the number of amino acids of amino acid type n in the protein, peptide, oligopeptide, polypeptide, or protein complex of interest, with $a_n$ being an integer greater than or equal to 0 ($a_j \in Z^+$), and where $f_n$ is a calibration factor or calibration function which scales between amino acid concentration and label value (e.g. signal of the label) for amino acid type n
- when the measurement for the sample is provided in measured label (e.g. signal of the label) and the reference line describes a proteome or subproteome of interest is $c_n = w_n f_n$ where $w_n$ is the number of amino acids of amino acid type n in the protein, peptide, oligopeptide, polypeptide, or protein complex of interest, where $w_n$ is the weighted mean number of amino acids of amino acid type n in the proteome or subproteome of interest, and where $f_n$ is a calibration factor or calibration function which converts between amino acid concentration and measured label (e.g. signal of the label) for amino acid type n.

Where *t* is the concentration of a solution containing the protein, peptide, oligopeptide, polypeptide, protein complex,

subproteome, or proteome which is the common independent variable (or parameter) in each of the n functions which collectively specify the reference line in each of the n dimensions, and where *t* is defined for all *t* greater than or equal to 0 ($\forall t \geq 0$)

The reference line can alternatively be described as a vector in n dimensional space (see discussion of hypothesis test 2, with the formal equations having been provided in the Summary section).

The approach for how the reference is used in the comparison step, outlined here in the context of a protein of interest and amino acid concentrations measured for the sample, is conserved across the embodiments of the invention, and specific details are provided in the following Comparison Step section.

In some embodiments, the reference is the amino acid concentration of two or more amino acid types for a protein of interest. The amino acid concentrations of two or more amino acid types for protein of interest $p_i$ as a function of the unknown molar protein concentration or the unknown mass protein concentration of the sample, *t*, is provided by set of parametric equations 1:

$$p_i(t) = [a_1t, a_2t, \ldots, a_nt], \; \forall t \geq 0$$

The amino acid concentrations of two or more amino acid types are labelled and measured in the sample. Amino acid type 1 is labelled and measured in the sample, amino acid type 2 is labelled and measured in the sample, and optionally amino acid type n is labelled and measured in the sample. In parametric equation 1, $a_1$ is the number of amino acids amino acid type 1 within protein of interest $p_i$ and is the coefficient of the function $a_1t$, $a_2$ is the number of amino acids of amino acid type 2 within protein of interest $p_i$ and is the coefficient of the function $a_2t$, and $a_j$ is the optional number amino acid type n within protein of interest $p_i$ and is the coefficient of the function $a_nt$. $\forall t \geq 0$ means that the functions are defined for all/any $t \geq 0$. The amino acid concentration of each amino acid type is the number of amino acids of each amino acid type multiplied by the unknown concentration, and is a function $a_1t$ is the amino acid concentration of the first amino acid type as a function of the unknown concentration of the protein of interest, *t*. $a_2t$ is the amino acid concentration of the second amino acid type as a function of the unknown concentration of the sample, *t*. $a_nt$ is the amino acid concentration of the optional $n^{th}$ amino acid type as a function of the unknown concentration of the sample, *t*. Therefore, when the amino acid concentrations of two or more amino acid types are labelled and measured in the sample, the reference for a solution containing protein of interest $p_i$ at any concentration, *t*, greater than or equal to zero is provided by the functions $[a_1t, a_2t, \ldots, a_nt]$.

Because there are n types of amino acids labelled and measured in the sample, n functions define the reference. For example, if there are 2 amino acid types labelled and measured in the sample, then the reference for a solution containing protein of interest $p_i$ at any concentration, *t,* with $t \geq 0$ is provided by the functions $[a_1t, a_2t]$. As another example, if there are 3 amino acid types labelled and measured in the sample, then the reference for a solution containing protein of interest $p_i$ at any concentration, *t,* with $t \geq 0$ is provided by the functions $[a_1t, a_2t, a_3t]$. As another example, if there are 4 amino acid types labelled and measured in the sample, then the reference for a solution containing protein of interest $n_i$ at any concentration, *t,* with $t \geq 0$ is provided by the functions $[a_1t, a_2t, a_3t, a_4t]$. As another example, if there are 5 amino acid types labelled and measured in the sample, then the reference for a solution containing protein of interest $n_i$ at any concentration, *t,* with $t \geq 0$ is provided by the functions $[a_1t, a_2t, a_3t, a_4t, a_5t]$.

To identify the presence and/or concentration and/or amount of protein of interest $n_i$ within the sample, the amino acid concentrations of each amino acid type measured for the sample are compared to the reference amino acid concentrations of these same two or more amino acid types for protein of interest $p_i$. The n amino acid concentrations measured for the n amino acid types labelled in the sample define a point in n dimensional space. The point has coordinates $(AAC_1, AAC_2, \ldots, AAC_j)$ where $AAC_1$ is the amino acid concentration measured for amino acid type 1 labelled in the sample, $AAC_2$ is the amino acid concentration measured for amino acid type 2 labelled in the sample, and $AAC_n$ is the amino acid concentration optionally measured for amino acid type n optionally labelled in the sample.

Protein, peptide, oligopeptide, polypeptide, protein complex, subproteome, or proteome of interest $p_i$ is present within the sample if the amino acid concentrations provided for a concentration of protein of interest $p_i$ are equal to, or within an error margin, of the amino acid concentrations measured for the sample. This is achieved by recognizing that protein of interest $p_i$ is present within the sample if the point providing the amino acid concentrations measured for the sample $(AAC_1, AAC_2, \ldots, AAC_j)$ is on the line, or within an error margin of the line, provided as the reference for protein of interest $p_i$. This recognition means that if protein of interest $p_i$ is present within the sample, then the concentration of protein of interest $n_i$ within the sample can be simultaneously determined.

This is carried out in the comparison step. In some embodiments, within the comparison step, the reference of the amino acid concentrations provided for protein of interest $p_i$, $[a_1t, a_2t, \ldots, a_nt]$, is compared to the amino acid concentrations measured for the sample, $(AAC_1, AAC_2, \ldots, AAC_n)$, by setting each amino acid concentration measured for the sample equal to the corresponding amino acid concentration provided by a function for the reference. This tests the hypothesis that protein of interest $p_i$ is present within the sample at any concentration. Test 1 is fulfilled if for all $t \geq 0$, there exists a value of *t* such that:

$$AAC_1 = a_1 t$$

$$AAC_2 = a_2 t$$

$$AAC_n = a_n t$$

where the number of equations is equal to the n number of amino acid types labelled and measured in the sample. If the n equations comprising test 1 can be solved for a single value of $t$, then protein of interest $p_i$ is present within the sample at concentration $t$, because the sample point is on the reference line.

In some embodiments, this can be stated formally as test 1 is fulfilled if

$$\forall t \geq 0, \exists t \ni AAC_k = a_k t \; \forall \; k = 1,2,\cdots,n$$

This says that test 1 is fulfilled if for all $t \geq 0$, there exists a value of $t$ such that $AAC_k = a_k t$ for all $k$ = 1, 2, ... , $n$.

For example, a sample of unknown protein identity and unknown concentration is obtained, the tryptophan (W) and lysine (K) amino acid types are labelled in the sample, and the amino acid concentrations of the W and K amino acid types in the sample are determined from the measured label as disclosed herein. Tryptophan (W) is amino acid type 1 and lysine (K) is amino acid type 2. The concentration of the amino acid tryptophan (W) in the sample, $S_{AAC,1}$, is 0.5 μM and the concentration of the amino acid lysine (K) in the sample, $S_{AAC,2}$, is 7 μM.

In this example, the protein of interest is the cytokine interleukin-6 (IL-6) which has been implicated in differential host response to SARS-CoV-2 infection. In some embodiments, the number of amino acids of an amino acid type is the total number of occurrences of amino acids of the amino acid type in the amino acid sequence of the protein of interest. The number of W amino acids in IL-6 is 1 and the number of K amino acids in IL-6 is 14. The W amino acid type is amino acid type 1 and the K amino acid type is amino acid type 2. Therefore, the reference for IL-6 at any protein concentration is provided by parametric equation 1 to be:

$$n_{IL-6}(t) = [t, 14t]$$

The presence of any protein concentration of the protein of interest IL-6 within the sample is evaluated by setting each amino acid concentration measured for the sample equal to the corresponding amino acid concentration function provided for the reference, according to test 1:

$$AAC_1 = a_1 t$$

$$AAC_2 = a_2 t$$

which is

$$0.5\ \mu M = t$$

$$7\ \mu M = 14t$$

and determining if a solution exists

$$7\mu M = 14(0.5\mu M)$$

$$\therefore t = 0.5\ \mu M$$

Meaning that when the amino acid concentration measured for amino acid type 1 in the sample is set equal to the amino acid concentration function provided for amino acid type 1 of the reference, and the amino acid concentration measured for amino acid type 2 in the sample is set equal to the amino acid concentration function provided for amino acid type 2 of the reference, there exists a solution to the equations. The solution to the equations is $t = 0.5\ \mu M$. Therefore, the protein of interest IL-6 is identified as being present in the sample, at a protein concentration of 0.5 μM.

As another example, a sample of unknown protein identity and unknown protein concentration is obtained, the tryptophan (W) and lysine (K) amino acid types are labelled in the sample, and the amino acid concentrations of the W and K amino acid types in the sample are measured from the signal of the label as described herein. Tryptophan (W) is amino acid type 1 and lysine (K) is amino acid type 2. The concentration of the amino acid tryptophan (W) in the sample, $S_{AAC,1}$, is 2.4 μM and the concentration of the amino acid lysine (K) in the sample, $S_{AAC,2}$, is 17.6 μM.

In this example, the protein of interest is cyclin-dependent-like kinase 5 (CDK5) which is kinase essential for neuronal development believed to be involved in apoptotic cell death in neurological diseases, which is secreted to blood plasma. The number of tryptophan (W) amino acids within the protein sequence is 3, and the number of lysine (K) amino acids within the protein sequence is 23. However, it is known from the public SwissProt database that 1 lysine amino acid is post-translationally modified (acetylated) and would therefore be not available for reaction with the lysine labelling dye chosen for this experiment. In some embodiments, the number of amino acids of an amino acid type within a protein of interest is the number of occurrences of that amino acid type within amino acid sequence or sequences of the protein of interest minus the number of post-translational modifications of that amino acid type that would prevent the amino acid type from reacting with the label. Therefore, the number of amino acids of the lysine (K) amino acid type within the protein of interest is 22 and the number of tryptophan (W) amino acids within the protein of interest is 3. Set of parametric equations 1 provides the following reference for the protein of interest (CDK5) at the unknown protein concentration of the sample:

$$n_{CDK5} = [3t, 22t]$$

The presence of any protein concentration of the protein of interest CDK5 within the sample is evaluated by setting each amino acid concentration measured for each labelled amino acid type in the sample equal to the corresponding amino acid concentration function provided for the reference, according to test 1:

$$AAC_1 = a_1 t$$

$$AAC_2 = a_2 t$$

which is

$$2.4\,\mu M - 3t$$

$$17.6\,\mu M = 22t$$

and determining whether a solution exists

$$2.4\,uM = 3t$$

$$t = \frac{2.4}{3}\mu M = 0.8\,\mu M$$

$$17.6\,\mu M = 22(0.8\,\mu M)$$

$$\therefore t = 0.8\,\mu M$$

Meaning that when the amino acid concentration measured for amino acid type 1 in the sample is set equal to the amino acid concentration function provided for amino acid type 1 of the reference, and the amino acid concentration measured for amino acid type 2 in the sample is set equal to the amino acid concentration function provided for amino acid type 2 of the reference, there exists a solution to the equations. The solution to the equations is $t = 0.8\ \mu M$. Therefore, the protein of interest CDK5 is identified as being present in the sample, at a protein concentration of 0.8 μM.

In some embodiments, parametric equation 1 provides the reference for multiple proteins of interest, and optionally, the results are stored in a reference database. In some embodiments, the number of amino acids of each amino acid type used within parametric equation 1 is also stored in a database, and parametric equation 1 operates on this database to provide the reference database.

For example, a protein is isolated from human blood plasma using HPLC and its molar protein concentration is unknown. The amino acid types C, K, and W are labelled in the sample. All (unmodified + modified) amino acids of the C amino acid type are labelled, unmodified amino acids of the K amino acid type (amino acids of the K amino acid type whose ε-amino group is a primary amine, not a secondary amine), and all (unmodified + modified) amino acids of the W amino acid type are labelled in the sample. The amino acid concentrations of 3.8 μM C, 159 μM K, and 0.9 μM W are measured in the sample from the signal of the label as described herein. The C amino acid type is $AAC_1$, the K amino acid type is $AAC_2$, and the W amino acid type is $AAC_3$. $AAC_1 = 3.8\ \mu M$, $AAC_2 = 15.9\ \mu M$, and $AAC_3 = 0.9\ \mu M$.

The reference is constructed for 5 proteins of interest found within human blood plasma. These include Affamin, Talin-1, L-selectin, C-reactive protein, and Lumican. In some embodiments, the reference is obtained from a reference database.

In some embodiments, the number of amino acids of the C, K, and W amino acid types in each protein of interest is determined by removing portions of the amino acid sequence of each protein of interest such as signal sequences that are cleaved in the mature protein, determining the number of occurrences of C, K, and W amino acids in the mature protein sequences of each protein, peptide, oligopeptide, polypeptide, protein complex, subproteome, or proteome of interest, and subtracting from the calculated occurrences of K amino acids the number of post-translational modifications (PTMs) that would result in conversion of the lysine ε-amino group from a primary amine to a secondary amine, specifically the number of acetylations, alkylations, and glycyl-lysine isopeptide formations.

This provides the following number of amino acids for the C, K, and W amino acid types, which can optionally be obtained from a database:

| | C | K | W |
|---|---|---|---|
| Affamin | 34 | 49 | 0 |
| Talin-1 | 38 | 159 | 9 |
| L-selectin | 22 | 24 | 11 |
| C-reactive protein | 2 | 13 | 6 |
| Lumican | 6 | 25 | 1 |

Parametric equation 1 is applied sequentially to each row of the database to produce the reference for each protein of interest as a function of any protein concentration, *t*:

| | | $a_1t$ | $a_2t$ | $a_3t$ |
|---|---|---|---|---|
| Affamin | $p_1$ | 34t | 49t | 0t |
| Talin-1 | $p_2$ | 38t | 159t | 9t |
| L-selectin | $p_3$ | 22t | 24t | 11t |
| C-reactive protein | $p_4$ | 2t | 13t | 6t |
| Lumican | $p_5$ | 6t | 25t | 1t |

The reference for each protein of interest, $p_1$, $p_2$, $p_3$, $p_4$, and $p_5$ can be visually shown as a line in 3-dimensional space. The reference for each protein of interest is a line in 3-dimensional space because 3 types of amino acids are labelled and measured in the sample, the amino acid type C is amino acid type $a_1$, the amino acid type K is the amino acid type $a_2$, and the amino acid type W is amino acid type $a_3$. Because parametric equation 1 defines the reference for all $t \geq 0$, all of the reference lines intersect at the origin (and any value of $a_1$, $a_2$, ... $a_n$ multiplied by 0 equals 0).

In the comparison step, test 1 is applied to each reference provided for each protein of interest. We have:

$$AAC_1 = a_1 t$$

$$AAC_2 = a_2 t$$

$$AAC_3 = a_3 t$$

For protein of interest $p_1$:

$$3.8\ \mu M = 34t; 15.9\ \mu M = 49t; 0.9\ \mu M = 0t$$

$$\frac{3.8}{34}\mu M = t;\ 15.9\ \mu M \neq 49\left(\frac{3.8}{34}\mu M\right); 0.9\ \mu M \neq 0\left(\frac{3.8}{34}\mu M\right)$$

$$\therefore\ \forall t \geq 0, \nexists\, t \in AAC_k = a_k t\ \forall\, k = 1,2,\cdots,n$$

Therefore, for all $t \geq 0$, there does not exist a single value of $t$ that satisfies all of the equations comprising test 1, so protein of interest $n_1$ is not present within the sample at any protein concentration.

For protein of interest $p_2$:

$$3.8\ \mu M = 38t; 15.9\ \mu M = 159t; 0.9\ \mu M = 9t$$

$$\frac{3.8}{38}\mu M = t;\ 15.9\ \mu M = 159\left(\frac{3.8}{38}\mu M\right); 0.9\ \mu M = 9\left(\frac{3.8}{38}\mu M\right)$$

$$\therefore\ \forall t \geq 0, \exists\, t \in AAC_k = a_k t\ \forall\, k = 1,2,\cdots,n$$

$$\therefore t = \frac{3.8}{38}\mu M = 0.1\ \mu M$$

For all $t \geq 0$, there exists a $t$ such that all of the equations comprising test 1 are solved for a single value of $t$, therefore protein of interest $p_2$ is present within the sample. The protein concentration of protein of interest $p_2$ is the value of $t$ that satisfied test 1, which was 0.1 $\mu M$. Therefore, the sample contains Talin-1 at 0.1 $\mu M$ protein concentration.

For protein of interest p:

$$3.8\ \mu M = 22t; 15.9\ \mu M = 24t; 0.9\ \mu M = 11t$$

$$\frac{3.8}{22}\mu M = t;\ 15.9\ \mu M \neq 24\left(\frac{3.8}{22}\mu M\right); 0.9\ \mu M \neq 11\left(\frac{3.8}{22}\mu M\right)$$

$$\therefore\ \forall t \geq 0, \nexists\, t \in AAC_k = a_k t\ \forall\, k = 1,2,\cdots,n$$

Therefore, for all $t \geq 0$, there does not exist a single value of $t$ that satisfies all of the equations comprising test 1, so protein of interest $p_3$ is not present within the sample at any protein concentration.

For protein of interest $p_4$:

$$3.8\ \mu M = 2t; 15.9\ \mu M = 13t; 0.9\ \mu M = 6t$$

$$\frac{3.8}{2}\mu M = t;\ 15.9\ \mu M \neq 13\left(\frac{3.8}{2}\mu M\right); 0.9\ \mu M \neq 6\left(\frac{3.8}{2}\mu M\right)$$

$$\therefore\ \forall t \geq 0, \nexists\, t \in AAC_k = a_k t\ \forall\, k = 1,2,\cdots,j$$

Therefore, for all $t \geq 0$, there does not exist a single value of $t$ that satisfies all of the equations comprising test 1, so

protein of interest $p_4$ is not present within the sample at any protein concentration.

For protein of interest $p_5$:

$$3.8\ \mu M = 6t;\ 15.9\ \mu M = 25t;\ 0.9\ \mu M = 1t$$

$$\frac{3.8}{6}\mu M = t;\ 15.9\ \mu M\ \neq 25\left(\frac{3.8}{6}\mu M\right);\ 0.9\ \mu M \neq 1\left(\frac{3.8}{6}\mu M\right)$$

$$\therefore\ \forall t \geq 0, \nexists\ t\ \in\ AAC_k =\ a_k t\ \forall\ k = 1,2,\cdots,j$$

Therefore, for all $t \geq 0$, there does not exist a single value of $t$ that satisfies all of the equations comprising test 1, so protein of interest $p_5$ is not present within the sample at any protein concentration.

In some embodiments, the steps outlined for test 1 are automated as an algorithm with a computer program, returning a logical result (1,0) reporting if (1=true, 0=false) protein of interest $p_i$ is present within the sample ($\forall t \geq 0$, $\exists t \in AAC_k = a_k t \forall\ k = 1,2, \cdots ,n$) or not present within the sample ($\forall t \geq 0, \nexists t \in AAC_k = a_k t \forall\ k = 1,2, \cdots , n$). If and only if protein of interest $n_i$ is present within the sample (1 is returned), then the value of $t$, $\forall t \geq 0, \exists t \in AAC_k = a_k t \forall\ k = 1,2, \cdots ,n$, is provided. This is the molar protein concentration of protein of interest $p_i$ within the sample. In some embodiments, this is achieved using python open source program.

In some embodiments, the presence of protein of interest $p_i$ is identified in the sample if the point measured for the sample, for example ($AAC_1$, $AAC_2$, $AAC_j$), is not on the line provided as a reference for protein of interest $p$, but is instead within an error margin, $\varepsilon$, of the line provided as a reference for protein of interest $p_i$. This reflects the fact that experimental measurements have neither infinite accuracy nor infinite precision, so the point measured for the sample will not always lie exactly on the reference line when protein of interest $p_i$ is contained within the sample.

Test 2 tests the hypothesis that protein of interest $p$ is present within the sample by testing whether the sample point is within an error margin, $\varepsilon$, of the reference line. In some embodiments, this is achieved by finding the shortest distance between the sample point and the reference line, and then determining whether this distance is less than the error margin. If this shortest distance between the sample point and the reference line is less than the error margin, then the presence of protein of interest $p_i$ within the sample is identified, and the concentration of protein of interest $p$ within the sample is provided by the exact point (concentration) on the reference line which gave the shortest distance.

In some embodiments, the shortest distance between a point and a line is the perpendicular distance between the point and the line. The reference line, in addition to being described parametrically for example by parametric equation 1, can also be described in vector format allowing calculation of the exact point (concentration) on the reference line that yields this perpendicular distance via the dot product. Then, the distance formula, for example the Euclidean distance formula, is used to find the distance between the sample point and this perpendicular distance point, and the distance is compared to the error margin, $\varepsilon$, to determine whether protein of interest $p_i$ is present within the sample. If protein of interest $p_i$ is present within the sample, then its concentration within the sample is the concentration (point) on the reference line to which the sample point was perpendicular.

The general approach for *Test* 2 is:

1. Let R be the reference line for protein, peptide, oligopeptide, polypeptide, protein complex, subproteome, or proteome of interest $p_i$, and let S be the sample point to find the shortest distance from
2. Find the equation of reference line R in vector format as a function of $t$ which is the concentration of protein, peptide, oligopeptide, polypeptide, protein complex, subproteome, or proteome of interest $p_i$
**3.** Find the general equation of a point P on the reference line R
4. Find the exact location of the point P on reference line R, called Q, such that the vector from S to P is perpendicular to R. This means find the point Q on reference line R such that the vector between S and Q gives the perpendicular. This is achieved by finding the dot product ($\cdot$) of the vector from S to P with the direction of R, setting this equal to 0, and solving for $t$ to provide the value of $t$ which, when substituted into the general equation for a point P on R, yields the perpendicular vector. If the reference is contained within the sample, then this solution for $t$ is its concentration.
5. Find the distance between Q and S using the distance formula, called D.
6. Evaluate whether D is less than the error margin, $\varepsilon$.
7. *If* $D > \varepsilon$, then the protein, peptide, oligopeptide, polypeptide, protein complex, subproteome, or proteome of interest $p_i$ is not present within the sample.
8. *If* $D \leq \varepsilon$, then the protein, peptide, oligopeptide, polypeptide, protein complex, subproteome, or proteome of interest $p_i$ is present within the sample at concentration $t$.

For example, the parametric form of the reference line (R) is

$$p_i(t) = [a_1 t, a_2 t, \ldots, a_n t],\ \forall t \geq 0$$

The vector form of the reference line is R = <0, 0, 0> + $t$ < $a_1$, $a_2$, ... , $a_n$ >
The general equation of a point (P) on the reference line is

$$P = \langle 0 + a_1 t, 0 + a_2 t, \ldots, 0 + a_{jn} t\rangle$$

The measured sample point (S) has coordinates S = ($AAC_1$, $AAC_2$, ... , $AAC_n$)
The vector from the measured sample point (S) to any point on the reference line (P) is P -$S$

$$P - S = \langle a_1 t - AAC_1,\ a_2 t - AAC_2, \ldots, a_{jn} t - AAC_{jn}\rangle.$$

For this vector to be perpendicular, the dot product (·) of this vector with the direction of the reference line $\langle a_1, a_2, \ldots, a_{jn}\rangle$ $a_2, \ldots, a_{jn}\rangle$ must be 0. Therefore, we set

$$\langle a_1 t - AAC_1,\ a_2 t - AAC_2, \ldots, a_{jn} t - AAC_{jn}\rangle \cdot \langle a_1, a_2, \ldots, a_{jn}\rangle = 0$$

$$a_1\langle a_1 t - AAC_1\rangle + a_2\langle a_2 t - AAC_2\rangle + \cdots + a_n\langle a_n t - AAC_n\rangle = 0$$

$$a_1{}^2 t - a_1 AAC_1 + a_2{}^2 t - a_2 AAC_2 + \cdots + a_n{}^2 t - a_n AAC_n = 0$$

$$a_1{}^2 t + a_2{}^2 t + \cdots + a_n{}^2 t = a_1 AAC_1 + a_2 AAC_2 + \cdots + a_n AAC_n$$

$$t(a_1{}^2 + a_2{}^2 + \cdots + a_n{}^2) = a_1 AAC_1 + a_2 AAC_2 + \cdots + a_n AAC_n$$

$$t = \frac{a_1 AAC_1 + a_2 AAC_2 + \cdots + a_n AAC_n}{a_1{}^2 + a_2{}^2 + \cdots + a_n{}^2}$$

This solution for $t$ is the concentration of the protein, peptide, oligopeptide, polypeptide, protein complex, subproteome, or proteome of interest $p_i$ for which the distance between the sample and the reference line for the protein, peptide, oligopeptide, polypeptide, protein complex, subproteome, or proteome of interest $p_i$ is the shortest (the perpendicular distance, which we identified). Therefore, if protein, peptide, oligopeptide, polypeptide, protein complex, subproteome, or proteome of interest $p_i$ is present within the sample, then protein, peptide, oligopeptide, polypeptide, protein complex, subproteome, or proteome of interest $p_i$ is present within the sample at concentration $t$.

To determine whether protein, peptide, oligopeptide, polypeptide, protein complex, subproteome, or proteome of interest $p_i$ is present within the sample, find the point on the reference line, $Q$, which gives the perpendicular distance. $Q = P(t)$.

$$P = \langle 0 + a_1 t, 0 + a_2 t, \ldots, 0 + a_{jn} t\rangle = \langle a_1 t, a_2 t, \cdots, a_{jn} t\rangle$$

$$Q = (a_1\left(\frac{a_1 AAC_1 + a_2 AAC_2 + \cdots + a_n AAC_n}{a_1{}^2 + a_2{}^2 + \cdots + a_n{}^2}\right),$$

$$a_2\left(\frac{a_1 AAC_1 + a_2 AAC_2 + \cdots + a_n AAC_n}{a_1{}^2 + a_2{}^2 + \cdots + a_n{}^2}\right), \cdots, a_n\left(\frac{a_1 AAC_1 + a_2 AAC_2 + \cdots + a_n AAC_n}{a_1{}^2 + a_2{}^2 + \cdots + a_n{}^2}\right))$$

$Q$ is a point, which is the set of amino acid concentrations for protein, peptide, oligopeptide, polypeptide, protein complex, subproteome, or proteome of interest $p_i$ which correspond to the solution for $t$. S is also a point. Find the distance, D, between S and $Q$ using the distance formula.

For example, the Euclidean distance formula between point S and point Q is

$$distance = \sqrt{\sum_{i=1}^{n}(S_i - Q_i)^2}$$

Therefore, we have

$$D = \sqrt{\left(AAC_1 - a_1\left(\frac{a_1AAC_1 + a_2AAC_2 + \cdots + a_nAAC_n}{a_1^2 + a_2^2 + \cdots + a_n^2}\right)\right)^2 + \left(AAC_2 - a_2\left(\frac{a_1AAC_1 + a_2AAC_2 + \cdots + a_nAAC_n}{a_1^2 + a_2^2 + \cdots + a_n^2}\right)\right)^2 + \cdots + \left(AAC_n - a_n\left(\frac{a_1AAC_1 + a_2AAC_2 + \cdots + a_nAAC_n}{a_1^2 + a_2^2 + \cdots + a_n^2}\right)\right)^2}$$

$\varepsilon$ is the error threshold, for example provided by the user.

*If* $D > \varepsilon$, the protein, peptide, oligopeptide, polypeptide, protein complex, subproteome, or proteome of interest $p_i$ is not present within the sample.

If $D \leq \varepsilon$, the protein, peptide, oligopeptide, polypeptide, protein complex, subproteome, or proteome of interest $p_i$ is present within the sample at the concentration

$$t = \frac{a_1AAC_1 + a_2AAC_2 + \cdots + a_nAAC_n}{a_1^2 + a_2^2 + \cdots + a_n^2}$$

For example, a protein is isolated from human blood plasma using HPLC and its molar protein concentration is unknown. The amino acid types C, K, and W are labelled in the sample. All (unmodified + modified) amino acids of the C amino acid type are labelled, i.e. $C_T$, unmodified amino acids of the K amino acid type (amino acids of the K amino acid type whose ε-amino group is a primary amine, not a secondary amine), and all (unmodified + modified) amino acids of the W amino acid type are labelled in the sample. The amino acid concentrations of 3.9 μM C, 16.1 μM K, and 1.0 μM W are measured in the sample from the signal of the label as described herein. The C amino acid type is $AAC_1$, the K amino acid type is $AAC_2$, and the W amino acid type is $AAC_3$. $AAC_1$ = 3.9 $\mu M$, $AAC_2$ = 16.1 $\mu M$, and $AAC_3$ - 1.0 $\mu M$.

In this example, the reference database has already been constructed for 5 proteins of interest found within human blood plasma. These include Affamin, Talin-1, L-selectin, C-reactive protein, and Lumican, as described above, using parametric equation 1 to produce the reference for any protein concentration *t* of each protein of interest.

| | | $a_1t$ | $a_2t$ | $a_3t$ |
|---|---|---|---|---|
| Affamin | $p_1$ | 34t | 49t | 0t |
| Talin-1 | $p_2$ | 38t | 159t | 9t |
| L-selectin | $p_3$ | 22t | 24t | 11t |
| C-reactive protein | $p_4$ | 2t | 13t | 6t |
| Lumican | $p_5$ | 6t | 25t | 1t |

The reference for each protein of interest, $p_1$, $p_2$,$p_3$, $p_4$, and $p_5$ is a line in 3-dimensional space. The reference for each protein of interest is a line in 3-dimensional space because 3 types of amino acids are labelled and measured in the sample, the amino acid type C is amino acid type $a_1$, the amino acid type K is the amino acid type $a_2$, and the amino acid type W is amino acid type $a_3$. Because set of parametric equations 1 define the reference for all $t \geq 0$, all of the reference lines intersect at the origin (and any value of $a_1$, $a_2$, ... $a_n$ multiplied by 0 equals 0).

In the comparison step, test 1 is applied to each reference provided for each protein of interest. We have:

$$AAC_1 = a_1t$$

$$AAC_2 = a_2 t$$

$$AAC_3 = a_3 t$$

For protein of interest $p_1$:

$$3.9\ \mu M = 34t; 16.1\ \mu M = 49t; 1.0\ \mu M = 0t$$

$$\frac{3.9}{34}\mu M = t;\ 16.1\ \mu M \neq 49\left(\frac{3.9}{34}\mu M\right); 1.0\ \mu M \neq 0\left(\frac{3.9}{34}\mu M\right)$$

Protein of interest $p_1$ has failed test 1.

For protein of interest $p_2$:

$$3.9\ \mu M - 38t; 16.1\mu M - 159t; 1.0\ \mu M - 9t$$

$$\frac{3.9}{38}\mu M = t;\ 16.1\ \mu M \neq 159\left(\frac{3.9}{38}\mu M\right); 1.0\ \mu M \neq 9\left(\frac{3.9}{38}\mu M\right)$$

Protein of interest $p_2$ has failed test 1.

For protein of interest $p_3$:

$$3.9\ \mu M - 22t; 16.1\ \mu M - 24t; 1.0\ \mu M - 11t$$

$$\frac{3.9}{22}\mu M - t;\ 16.1\ \mu M \neq 24\left(\frac{3.9}{22}\mu M\right); 1.0\ \mu M \neq 11\left(\frac{3.9}{22}\mu M\right)$$

Protein of interest $p_3$ has failed test 1.

For protein of interest $p_4$:

$$3.9\ \mu M - 2t; 16.1\ \mu M - 13t; 1.0\ \mu M - 6t$$

$$\frac{3.9}{2}\mu M = t;\ 16.1\ \mu M \neq 13\left(\frac{3.9}{2}\mu M\right); 1.0\ \mu M \neq 6\left(\frac{3.9}{2}\mu M\right)$$

Protein of interest $p_4$ has failed test 1.

For protein of interest $p_5$:

$$3.9\ \mu M - 6t; 16.1\ \mu M - 25t; 1.0\ \mu M - 1t$$

$$\frac{3.9}{6}\mu M = t;\ 16.1\ \mu M \neq 25\left(\frac{3.9}{6}\mu M\right); 1.0\ \mu M \neq 1\left(\frac{3.9}{6}\mu M\right)$$

Protein of interest $p_5$ has failed test 1.

Therefore, the sample point is not on the reference line for any of the proteins of interest. However, the presence and/or concentration and/or amount of a protein of interest is identified within the sample if there exists a single value of concentration for which the amino acid concentration of two or more amino acid types measured in the sample is less than or equal to an error margin to the amino acid concentrations of the same two or more amino acid types as the amino acid types that have been labelled in the sample of the one or more proteins of interest. In some embodiments, the error margin is provided by equation 10:

$$\varepsilon = \varphi\sqrt{S_1^2 + S_2^2 + \cdots + S_j^2}$$

wherein $\varepsilon$ is the error margin, $\varphi$ is a user-inputted tolerance value, $S_1$ is the value (value of the label, amino acid concentration, or number of amino acids) measured for the sample for amino acid type 1, $S_2$ is the value (value of the label, amino acid concentration, or number of amino acids) measured for the sample for amino acid type 1, and $S_n$ is the value (value of the label, amino acid concentration, or number of amino acids) measured for the sample for amino acid type n.

The user has provided a user-inputted tolerance value, $\varphi$, of 0.01. Therefore, equation 10 becomes,

$$\varepsilon = 0.01\sqrt{(3.9\ \mu M)^2 + (16.1\ \mu M)^2 + (1.0\ \mu M)^2} = 0.167\ uM.$$

In test 2, all of the proteins of interest are assessed to determine whether the shortest (perpendicular) distance between the measured sample point and the reference line for each protein of interest falls within the error margin.

This approach is illustrated specifically for the example of protein of interest $p_2$, and the results are subsequently displayed for each protein of interest.

The parametric form of the reference line (R) protein of interest $p_2$ is

$$p_2(t) = [38t, 159t, 9t],\ \forall t \geq 0$$

In vector form, the equation for this reference line is

$$R = \langle 0, 0, 0\rangle + t\langle 38, 159, 9\rangle$$

$$P = (38t, 159t, 9t)$$

The sample point is S = (3.9, 16.1, 1)

The vector from the measured sample point (S) to any point on the reference line (P) is P -*S*

$$P - S = \langle 38t - 3.9, \quad 159t - 16.1, \quad 9t - 1\rangle$$

For this vector to be perpendicular, the dot product (·) of this vector with the direction of the reference line ⟨38, 159, 9⟩ must be 0. Therefore, we set

$$\langle 38t - 3.9,\ 159t - 16.1,\ 9t - 1\rangle \cdot \langle 38, 159, 9\rangle = 0$$

$$1444t - 148.2 + 25281t - 2559.9 + 81t - 9 = 0$$

$$26806t = 2717.1$$

$$t = 0.1014$$

This solution for *t* is the protein concentration of the protein of interest $p_2$ for which the distance between the sample and the reference line is shortest. Therefore, if protein of interest $p_2$ is present within the sample, then protein of interest $p_2$ is present within the sample at protein concentration *t*.

To determine whether the reference is present within the sample, find the point on the reference line, Q, which gives the perpendicular distance. *Q* = *P*(*t*).

$$P = \langle a_1 t, a_2 t, \cdots, a_j t\rangle$$

$$Q = (38 \times 0.1014, 159 \times 0.1014, 9 \times 0.1014) = (3.8532, 16.1226, 0.9126)$$

$$S = (3.9, 16.1, 1)$$

$$D = \sqrt{(3.9 - 3.8532)^2 + (16.1 - 16.1226)^2 + (1 - .9126)^2}$$

$$D = 0.1017$$

$$\varepsilon = 0.167$$

$$D \leq \varepsilon$$

Therefore, protein of interest $n_2$ is present within the sample at a protein concentration of 0.1017 μM.

In some embodiments, the number of amino acids of each amino acid type ($a_1$, $a_2$ ,$a_j$, or, $w_1$, $w_2$, $w_j$) is calculated experimentally, rather than being determined from the protein sequence or protein sequences of the protein, peptide, oligopeptide, polypeptide, subproteome, or proteome of interest. In this embodiment, post-translational modifications which result in a modified amino acid of an amino acid type not being labelled with a given dye, and protein expression levels in the case of a proteome or subproteome of interest are automatically incorporated within the number of amino acids calculation.

It has been explained, for the case of a protein of interest, how the reference is provided by a parametric equation, and how the reference is used within the comparison step to evaluate the presence and/or concentration and/or amount of a protein of interest within the sample based on the amino acid concentrations measured for two or more labelled amino acid types of the sample. The reference is used in the comparison step in the same way in other embodiments, and this is described further in the "Comparison Step" section. Here, the remaining forms of the reference are described, which follow the same approach.

The methods of the invention are also used to determine the presence and/or concentration and/or amount of a proteome or subproteome of interest within a sample. The reference for a proteome or subproteome of interest is can also be a line in n dimensional space, with n being the number of amino acid types labelled and measured in the experiments. In some embodiments, construction of the reference line for a proteome of subproteome of interest is enabled by determination of the number of amino acids of a (hypothetical) average protein sequence of the proteome or subproteome of interest, that has a number of amino acids of each amino acid type that is the weighted mean number of amino acids of all protein sequences contained within the proteome or subproteome of interest; there is no concept of order of amino acids in this representative protein sequence, and the canonical constraint of having the number of amino acids of each amino acid type be a positive integer (e.g. $a_j \in Z^+$), for example as discussed by Creighton in 1980 (https://www.nature.com/articles/284487a0), is removed. The weighted mean number of amino acids of each amino acid type can be determined with equation 11:

$$w_n = \sum_{i=1}^{c} \left( a_{n,i} \times \frac{q_i}{q} \right)$$

Wherein $w_n$ is the weighted mean number of amino acids of amino acid type n in the proteome or subproteome of interest, $c$ is the number of proteins in the proteome or subproteome of interest, $a_{n,i}$ is the number of amino acids of amino acid type n in protein i in the proteome or subproteome of interest, $q_i$ is a measure of the quantity of protein i in the proteome or subproteome of interest, and q is an equivalent measure of the total quantity of all proteins (proteins i through c) in the proteome or subproteome of interest, such that ${q_i}/{q}$ gives the proportion of protein *i* within the proteome or subproteome of interest. A linear combination of the results is taken for proteins i through c of the proteome or subproteome of interest. $q_i$ and q can be calculated using a variety of methods known in the art. For example, $q_i$ can be the expression level of protein of interest *i* within the proteome or subproteome of interest, preferably determined by publicly available data from mass spectrometry, immunoassays or protein microarrays such as is presented in the Human Protein Atlas database or ProteomeXchange, and $q$ can be the total concentration of the proteome or subproteome of interest or the total predicted expression level of all proteins (proteins *i* through *c*) contained within the proteome or subproteome of interest each assessed using publicly available protein expression data. Alternatively, $q_i$ and $q$ can be determined by mRNA expression data. The mRNA expression level of a gene can be converted to the expression level of a protein using a gene-specific RNA-to-protein (RTP) conversion factor, for example as described in https://www.embopress.org/doi/full/10.15252/msb.20167144. mRNA expression levels re available from public databases, for example the Human Protein Atlas and Expression Atlas. $q$ can be the expression level of all proteins within the proteome or subproteome of interest (proteins

i through c) calculated in this way. Alternatively, $q_i$ and q can be calculated from a known structural model. For example, if the proteome of interest is a virus, then $q_i$ can be the number of protein i within the structure of the virus (for example, the number of coronavirus spike proteins can be calculated from a model of the coronavirus viral capsid), and q is the number of all proteins (proteins i through c) within the structure of the virus.

As disclosed herein, in preferred embodiments, $q_i$ can be the expression level of protein of interest i within the proteome or subproteome of interest, preferably determined by publicly available data from mass spectrometry databases, and q can be the total concentration of the proteome or subproteome of interest or the total predicted expression level of all proteins (proteins i through c) contained within the proteome or subproteome of interest each assessed using publicly available protein expression data. In these embodiments, the expression level of protein of interest i within the proteome or subproteome of interest ($q_i$), and the total concentration of the proteome or subproteome of interest or the total predicted expression level of all proteins (proteins i through c) contained within the proteome or subproteome of interest (q) can be assessed using publicly available protein expression data provided by mass spectrometry databases. To achieve this, protein quantification data provided in mass spectrometry databases is used. Label free quantification data in mass spectrometry databases is provided as intensitiy vales, int, which are proportional to the amount (mass) of protein present. These provided int values can be converted to be proportional to the molar amount of protein present by dividing the intensity for an individual protein by its molecular mass. In some embodiments, $q_i$ within equation 11 is provided by equation 13:

$$q_i = int_m = \frac{int}{m_r}$$

wherein $int_m$ is the molar intensity of an individual protein, int is the provided intensity of the individual protein, and $m_r$ is the molecular mass of the individual protein. As required by equation 11, $int_m$ is calculated for each protein of interest within the reference proteome or subproteome of interest. In some embodiments, this is calculated using PowerBl; a Microsoft® analytic programme.

Within equation 11, q is the sum of the $q_i$ values across all proteins of interest i within the proteome or subproteome of interest containing c proteins, meaning

$$q = \sum_{i=1}^{c} q_i$$

It follows that just as $int_m$ is one possible form of $q_i$ within equation 11, $\Sigma\ int_m$ is one possible form of q for the proteome of subproteome of interest. This relationship is formalized in equation 14

$$q = \sum int_m$$

Therefore,

$$\frac{int_m}{\sum int_m}$$

is one possible form of $^{q_i}/_{q}$ in equation 11 as disclosed herein.

For ease of reference, $\frac{int_m}{\Sigma\, int_m}$ is called mass spectrometry molar intensity fraction, which is abbreviated $MSIF_m$. This relationship is formalized in equation 15

$$MSIF_m = \frac{int_m}{\sum int_m}$$

Mass spectrometry molar intensity fraction, $MSIF_m$, is a relative quantity, giving the proportion of molar concentration each protein contributes to the proteome or subproteome of interest. For example, if a protein contributes 1% of the molar concentration of the total molar protein concentration of the proteome of interest, then its $MSIF_m$ value would be the unitless value of 0.01. However, $\Sigma\ int_m$ is not a relative quantity and it can be related to the molar protein concentration of the proteome of interest. To achieve this, $\Sigma\ int_m$ values accessible via mass spectrometry are related to

molar or mass concentration values accessible via immunoassay or peptide microarray experiments on the same proteome of interest. For example, the total molar concentration and total mass concentration of of proteins within the healthy patient subproteome of the human plasma proteome was calculated using publicly available data deposited in the Human Protein Atlas. The data accessed is available at https://www.proteinatlas.org/humanproteome/blood/proteins+detected+by+immunoassay. The human gene names provided in the indicated Human Protein Atlas database was mapped to UniProtKB identifiers using the UniProt database Retrieve/ID mapping tool available at https://www.uniprot.org/uploadlists/. The molecular weight of each protein was downloaded. Then, using Microsoft Excel, the mass concentration of each protein within the human plasma proteome was calculated by dividing the mass concentration of each protein by the molecular weight of each protein. The results were summed across all proteins present within the human plasma proteome, to provide a total molar protein concentration for the human plasma proteome of 1201.5 μM. $\Sigma$ $int_m$ values may vary depending on the specific mass spectrometry instrument used, but because the total molar protein concentration for the healthy subproteome of the human plasma proteome is known to be 1201.5 μM, a relationship can be established for each mass spectrometry dataset based on the $\Sigma$ $int_m$ values for the healthy subproteome of the human plasma proteome in the given mass spectrometry dataset. To do this transformation the conversion between the $\Sigma$ $int_m$ of the mass spectrometry dataset and the total molar protein concentration for the healthy subproteome of the human plasma proteome is established by calculating expression 15

$$\frac{1201.5\ uM}{mean(\sum int_m)}$$

Wherein $mean(\Sigma\ int_m)$ is the mean of the $\Sigma$ $int_m$ values for all patient samples of the healthy subproteome of the human plasma proteome.

Additionally, in some embodiments it is desirable to convert the mass spectrometry intensity vales to a mass concentration or amount of a proteome or subproteome of interest, rather than to a molar concentration of a proteome or subproteome of interest. In this case, rather than calculating the mass spectrometry molar intensity fraction, the mass spectrometry mass intensity fraction is calculated. Rather than calculating $\frac{int_m}{\Sigma int_m}$ for ${q_i}/{q}$ in equation 11, the provided mass spectrometry intensity values are not converted to molar intensity by dividing by the protein molecular weight. Instead, the intrinsic proportionality between the mass spectrometry intensity values and the mass of protein present is utilized in the calculation of the mass spectrometry mass intensity fraction, equation 16

$$MSIF_{mass} = \frac{int}{\sum int}$$

wherein int is the mass spectrometry intensity for a given protein within a proteome or subproteome of interest and $\Sigma$ *int* is the sum of the intensity values across all proteins within the proteome or subproteome of interest. Like the $MSIF_m$ values, the $MSIF_{mass}$ values are relative quantities that provide the fraction of each protein within the proteome or subproteome of interest. They can be related to the mass protein concentration of the proteome of interest or to the mass amount of the proteome or subproteome of interest. To achieve this, $\Sigma$ int values accessible via mass spectrometry are related to mass concentration values accessible via immunoassay or peptide microarray experiments on the same proteome of interest. The Human Peptide Atlas mass concentrations as described above were summed across all proteins present within the human plasma proteome, to provide a total mass protein concentration for the human plasma proteome of 77453 μg/mL. $\Sigma$ int values may vary depending on the specific mass spectrometry instrument used, but because the total mass protein concentration for the healthy subproteome of the human plasma proteome is known to be 77453 μg/mL, a relationship can be established for each mass spectrometry dataset based on the $\Sigma$ *int* values for the healthy subproteome of the human plasma proteome in the given mass spectrometry dataset. To do this transformation the conversion between the $\Sigma$ *int* of the mass spectrometry dataset and the total molar protein concentration for the healthy subproteome of the human plasma proteome is established by calculating expression 17:

$$\frac{77453\ \text{ug/mL}}{mean(\sum int_m)}$$

Wherein $mean(\Sigma\ int)$ is the mean of the $\Sigma$ *int* values for all patient samples of the healthy subproteome of the human plasma proteome.

In some embodiments, it may be desirable to calculate a molar or mass amount of protein present using the mass spectrometry total molar intensity values available in mass spectrometry databases. The total intensity values $\Sigma$ *int* are proportional to mass, so if the total protein concentration added to the mass spectrometer is known, for example because it

has been standardized, then it is known that the sum of the total intensity values Σ *int* is equal to this mass of total protein. Therefore, calculating the intensity of each protein divided by the sum of the intensities across all proteins in a sample $\frac{int}{\Sigma int}$ provides the fractional mass amount of the individual protein within the sample. This can then be multiplied by the provided total protein mass amount per sample to provide an mass amount in μg of each individual protein within each sample. Alternatively, if the molar amount is desired, then for each protein within each sample, the mass amount in μg can be divided by the protein molecular weight (which can be downloaded from Uniprot as disclosed herein) in g/mol, providing the molar amount in μmol.

In equation 11, $q_i$ is a measure of the quantity of protein i in the proteome or subproteome of interest, and q is an equivalent measure of the total quantity of all proteins in the proteome or subproteome of interest. Hence, the total molar or mass amount of all proteins within each sample also provides q in Equation 11. ${q_i}/{q}$ can be calculated by dividing the molar amount of each protein within each sample by the total molar amount of all proteins within each sample, and equation 11 can then be used to calculate the $w_K$, $w_W$, $w_Y$, and $w_c$ values.

Alternatively, the weighted mean number of amino acids of each amino acid type can be determined with equation 12:

$$w_n = \sum_{i=1}^{c}\left(a_{n,i} \times \frac{1}{c}\right)$$

Wherein $w_n$ is the weighted mean number of amino acids of amino acid type n in the proteome or subproteome of interest, c is the number of proteins in the proteome or subproteome of interest, and $a_{n,i}$ is the number of amino acids of amino acid type n in protein i in the proteome or subproteome of interest. A linear combination of the results is taken for proteins i through c of the proteome or subproteome of interest. In this embodiment, all proteins within the proteome or subproteome of interest are taken as having equivalent expression or proportion within the proteome or subproteome of interest, so the weights for each protein of interest within the proteome or subproteome of interest are equal.

Alternatively, the weighted mean number of amino acids of amino acid type 1 ($w_1$) is calculated by measuring the amino acid concentration of amino acid type 1 in the proteome or subproteome of interest, measuring the total concentration of the proteome or subproteome of interest with methods known in the art, and dividing the measured amino acid concentration of amino acid type 1 by the measured total concentration of the proteome or subproteome of interest as disclosed herein. The weighted mean number of amino acids of amino acid type 2 ($w_2$) is calculated by measuring the amino acid concentration of amino acid type 2 in the proteome or subproteome of interest, measuring the total concentration of the proteome or subproteome of interest with methods known in the art, and dividing the measured amino acid concentration of amino acid type 2 by the measured total concentration as disclosed herein. The weighted mean number of amino acids of amino acid type n ($w_n$) is calculated by measuring the amino acid concentration of amino acid type n in the proteome or subproteome of interest, measuring the total concentration of the proteome or subproteome of interest with methods known in the art, and dividing the measured amino acid concentration of amino acid type n by the measured total concentration as disclosed herein.

This weighted mean number of amino acids of each amino acid type is used to provide the reference line for a proteome or subproteome of interest. When the measurements provided for the sample are the amino acid concentrations measured for amino acid type 1, amino acid type 2, and amino acid type n as disclosed herein, the reference line is described by set of parametric equations 2:

$$p_i(t) = [w_1 t, w_2 t, \cdots, w_j t], \forall t \geq 0$$

wherein $p_i$ is the proteome or subproteome of interest, $w_1$ is the weighted mean number of amino acids of amino acid type 1 in the proteome or subproteome of interest, $w_2$ is the weighted mean number of amino acids of amino acid type 2 in the proteome or subproteome of interest, and $w_j$ is the weighted mean number of amino acids of amino acid type n in the proteome or subproteome of interest, *t* is the proteome/subproteome concentration (wherein the proteome/subproteome concentration is the total molar concentration of all proteins, peptides, oligopeptides, polypeptides, and protein complexes comprising proteome or subproteome of interest $p_i$),and is the common independent variable (or parameter) in each of the n functions which collectively specify the reference line in each of the n dimensions, and where *t* is defined for all *t* greater than or equal to 0 ($\forall t \geq 0$).

In some embodiments, no calculations are required to be performed on the sample; the label of the sample can be simply measured, and all calculations can instead be performed on the protein, peptide, oligopeptide, polypeptide, protein complex, subproteome, or proteome of interest. The direct output of the measurement of the sample is the measured label

(e.g signal of the label) of each of two or more amino acid types labelled and measured in the methods of the invention, and the reference can be constructed to provide the known label value (e.g. known signal of the label) as a function of the concentration of a solution containing the protein, peptide, oligopeptide, polypeptide, protein complex, subproteome, or proteome of interest. A calibration curve or standard transforms between amino acid concentration for each amino acid type and known label value (e.g. signal of the label) for each amino acid type. Preferably, the calibration curve is a linear function. The calibration curve or standard is determined by measuring one or more standard solutions of known amino acid concentrations, for example provided by one or more calibration amino acids or calibration proteins. In some embodiments, the amino acid concentrations are provided in concentration units, such as nM or μM, and the signal of the label is measured in arbitrary units, such as AU.

$f_n$ is the calibration function, derived from the calibration curve, or the calibration factor for amino acid type n and converts from known amino acid concentration to signal of the label. $f_n^{-1}$ is the inverse of the calibration function, derived from the calibration curve, or the calibration factor for amino acid type n and converts from measured signal of the label to amino acid concentration.

$$Amino\ Acid\ Concentration\ (e.g.\ \mu M) \xrightarrow{f_n} AU$$

$$AU \xrightarrow{f_n^{-1}} Amino\ Acid\ Concentration\ (e.g.\ uM)$$

In some embodiments, the calibration curve is linear, meaning that the value of the label of amino acid type n is linearly related to the amino acid concentration of amino acid type n, and the calibration function is a calibration factor. In alternative embodiments, the calibration curve is nonlinear, and the calibration function cannot be reduced to a calibration factor because additional transformations are required (for example, the calibration function can describe a power law relationship).

For example, the values of the label for known amino acid concentrations of the W amino acid type were measured and plotted as a function of amino acid concentration of the W amino acid type, and the line was fit to equation 7 (within the "Measuring the concentration of each labelled amino acid type" section). For the W amino acid types, this provides:

$$W\ Signal\ (AU) = 100\ \left({}^{AU}/_{\mu M}\right) \times W\ Concentration\ (\mu M)$$

The calibration factor is linear, so a calibration factor can be determined. The calibration factor for the W amino acid type, $f_W$, is the slope of the calibration curve, and is 100 $100\ \left({}^{AU}/_{\mu M}\right)$ in this example. The calibration factor for amino acid type n is called $f_n$. The calibration curve or standard also has an inverse, used in the "Measuring the concentration of each labelled amino acid type" section. The inverse of the calibration curve or standard transforms in the opposite direction, so between the measured signal of the label of an amino acid type and the amino acid concentration of the amino acid type. The inverse of the calibration factor for amino acid type n is called $f_n^{-1}$. For example, the inverse of the calibration factor above is $f_W^{-1}$:

$$\frac{uM}{100\ AU}$$

$f_j^{-1}$ is used when the calibration is performed on measurements taken on the sample, and $f_j$ is used when the calibration is performed when calculating the reference.

When the measurements of the sample are provided in terms of the measured label (e.g. signal of the label) of amino acid type 1, amino acid type 2, and amino acid type n, the parametric equation of the reference line protein, peptide, oligopeptide, polypeptide, or protein complex of interest is given by parametric equation 3:

$$p_i(t) = [a_1 f_1 t, a_2 f_2 t, \cdots, a_n f_n t], \forall t \geq 0$$

wherein $p_i$ is the protein, peptide, oligopeptide, polypeptide, or protein complex of interest, $a_1$ is the number of amino acids of amino acid type 1 in the protein, peptide, oligopeptide, polypeptide, or protein complex of interest, $f_1$ is the calibration factor or calibration function for amino acid type 1, $a_2$ is the number of amino acids of amino acid type 2 in the protein, peptide, oligopeptide, polypeptide, or protein complex of interest, $f_2$ is the calibration factor or calibration function for amino acid type 2, $a_n$ is the number of amino acids of amino acid type n in the protein, peptide, oligopeptide, polypeptide, or protein complex of interest, $f_n$ is the calibration factor or calibration function for amino acid type n, $t$ is the concentration of the protein, peptide, oligopeptide, polypeptide, or protein complex of interest, and is the common independent variable (or parameter) in each of the n functions which collectively specify the reference line in each of the n dimensions, and where $t$ is defined for all $t$ greater than or equal to 0 ($\forall t \geq 0$).

If desired, optional processing steps can be undertaken to ensure that the $a_1$, $a_2$ and $a_j$ values or the $w_1$, $w_2$, and $w_j$ values used in the creation of the reference, or reference database, reflect the functional forms of one or more proteins, peptides, oligopeptides, polypeptides, protein complexes, proteomes, or subproteomes of interest represented in the reference database. These optional processing steps are described below.

In some embodiments, processing steps can be undertaken to ensure that the values measured for the sample are compared with references reflecting the functional forms of the one or more proteins, peptides, oligopeptides, polypeptides, protein complexes, subproteomes, or proteomes of interest, or, to enable comparison with more than one form of the one or more proteins, peptides, oligopeptides, polypeptides, protein complexes, subproteomes, or proteomes of interest. For example, processing steps can be undertaken to ensure that the values measured for the sample are compared to references reflecting proteins of interest that have undergone post-translational modifications, or that both have and have not undergone post-translational modifications.

In some embodiments, the number of amino acids of the corresponding amino acid types in the one or more proteins, peptides, oligopeptides, polypeptides, protein complexes, subproteomes, or proteomes of interest is determined from the amino acid sequence of the one or more proteins, peptides, oligopeptides, polypeptides, protein complexes, subproteomes, or proteomes of interest. In some embodiments, the number of amino acids of each amino acid type in a protein, peptide, oligopeptide or polypeptide of interest refers to the frequency of occurrences of the amino acid type in the amino acid sequence, and can be determined, for example, by finding the number of occurrences of the character corresponding to an amino acid type within the publicly available FASTA sequence for the protein, peptide, oligopeptide or polypeptide of interest using a computer program. Post-translational modifications are not considered. For example, hen egg white lysozyme is the protein of interest. The amino acid sequence of one molecule of hen egg white lysozyme is below:

*K*VFGR**C**ELAAAM*K*RHGLDNYRGYSLGNWV**C**AA*K*FESNFNTQATNRNTDGSTDYGILQINSRWW**C**NDGRTPGSRN**C**N
IP**C**SALLSSDITASVN**C**A*KK*IVSDGNGMNAWVAWRNR**C***K*GTDVQAWIRG**C**RL

The amino acid type K appears 6 times in the protein sequence (italicised). The number of K per protein molecule is 6, which is $a_1$ in set of parametric equations 1. The amino acid type C appears 8 times in the protein sequence (bolded). The number of C per protein molecule is 8, which is $a_2$ in set of parametric equations 1. The amino acid type W appears 6 times in the protein sequence (underlined). The number of W per protein molecule is 6, which is $a_3$ in set of parametric equation 1 (because 3 amino acid types are labelled, $a_n = a_3$).

In some embodiments, post-translational modifications are considered when calculating the $a_1$, $a_2$ and $a_n$ values or the $w_1$, $w_2$ and $w_n$ values used in the creation of the reference, or reference database. In some embodiments, the number of amino acids of an amino acid type within a protein of interest is the number of occurrences of that amino acid type within amino acid sequence of the protein of interest minus the number of post-translational modifications of that amino acid type that would prevent the amino acid type from reacting with the label. Preferably, the number of amino acids of each amino acid type in a protein of interest is adjusted by considering post-translational modifications (PTMs) that affect the R-group which defines the amino acid type in a manner which makes it chemically unreactive with the label used for amino acid labelling. PTMs affecting a protein sequence are publicly available, for example in the Uni Prot or Swiss Prot database. If an amino acid type is modified in a manner dictated by a specific PTM, this can result in specific calculations being indicated to be performed during the calculation of the number of amino acids of that amino acid type within a protein sequence of interest, provided in a series of logical rules. The possible Rules are to do nothing (Rule = 0), subtract one (Rule = -1), or add one to the number of that amino acid type contained within the protein sequence (Rule = +1), or to add one to the number of another amino acid type contained within the protein sequence if it is also labelled in the methods of the invention (+1other, e.g. +1D if the D amino acid type is also labelled and measured in the methods of the invention). The Rules are dictated by the specific interaction of the amino acid R-group with the specific labels and classes of labels disclosed herein, and are provided in Table 4. The rules can be included in the reference database.

**Table 4: Rules for PTM of amino acids within an amino acid type**

| Amino Acid Type | R-group | PTM | Rule (-0, -1, +1, +1 other) | Eliminates Edman (Y, N) |
|---|---|---|---|---|
| Alanine, A | methyl | N-acetylation | 0 | Y |
| Arginine, R | aliphatic guanidino group | methylation | -1 | N |
| | | deimination to citrulline | -1 | N |
| Asparagine, N | β-carboxamide | deamidation to D or iso(D) | -1; +1D | N |
| | | N-linked glycosylation | -1 | N |
| Asparatic acid, D | β-carboxylic acid | isomerization to isoaspartic acid | 0 | N |
| Cysteine, C | thiol | disulfide-bond formation | -1 | N |
| | | oxidation to sulfenic, sulfinic or sulfonic acid | -1 | N |
| | | palmitoylation | -1 | N |
| | | N-acetylation (N-terminus) | 0 | Y |
| | | S-nitrosylation | -1 | N |
| Glutamic acid, E | γ-carboxylic acid | cyclization to Pyroglutamic acid (N-terminus) | -1 | N |
| | | gamma-carboxylation | 1 | N |
| Glutamine, Q | γ-carboxamide | cyclization to Pyroglutamic acid (N-terminus) | -1 | N |
| | | deamidation to Glutamic acid | -1, +1E | N |
| | | isopeptide bond formation to a lysine | -1 | N |
| Glycine, G | hydrogen | N-Myristoylation (N-terminus) | 0 | Y |
| | | N-acetylation (N-terminus) | 0 | Y |
| Histidine, H | imidazole | Phosphorylation | -1 | N |
| Lysine, K | ε-primary amino group | acetylation | -1 | N |
| | | Ubiquitination | -1 | N |
| | | SUMOylation | -1 | N |
| | | methylation | -1 | N |
| | | isopeptide bond formation to a glutamine | -1 | N |
| | | hydroxylation | 0 | N |
| Methionine, M | S-methyl thioether | N-acetylation (N-terminus) | 0 | Y |
| | | N-linked Ubiquitination | -1 | N |
| | | oxidation to sulfoxide or sulfone | -1 | N |
| Proline, P | pyrrolidine | hydroxylation | -1 | N |
| Serine, S | hydroxymethyl | Phosphorylation | -1 | N |
| | | O-linked glycosylation | -1 | N |
| | | N-acetylation (N-terminus) | 0 | Y |
| Threonine, T | hydroxyl | Phosphorylation | -1 | N |
| | | O-linked glycosylation | -1 | N |
| | | N-acetylation (N-terminus) | 0 | Y |
| Tryptophan, W | indole | mono- or di-oxidation | 0 | N |
| | | formation of Kynurenine | -1 | N |

(continued)

| Amino Acid Type | R-group | PTM | Rule (-0, -1, +1, +1 other) | Eliminates Edman (Y, N) |
|---|---|---|---|---|
| Tyrosine, Y | phenol | sulfation | 0 | N |
| | | phosphorylation | 0 | N |
| Valine, V | isopropyl | N-acetylation (N-terminus) | 0 | Y |

Many of the PTMs eliminate the possibility of sequencing a protein of interest using classical approaches such as Edman degradation or state of the art approaches such as fluorosequencing. If the PTM eliminates the possibility of sequencing a protein using Edman degradation and flourosequencing, a "Y" occurs in the Eliminates Edman column. However, samples containing proteins modified with all PTMs are able to be identified with the methods of the invention, particularly when the number of amino acids is calculated using the rules disclosed herein.

In some embodiments, a reference can be provided for a protein of interest which has undergone certain post-translational modifications, and another reference can be provided for a protein of interest which has not undergone certain post-translational modifications. In some embodiments, the methods of the invention are used to detect whether a protein of interest has, or has not, undergone specific PTMs, by providing a reference value for the protein of interest applying the PTM rules, and an additional reference not applying any rule, when determining the number of amino acids of each amino acid type from the protein sequence of the protein of interest. Because PTMs can be dynamic modulators of protein behaviour, this result can be indicative of disease.

In some embodiments, unmodified amino acid types are labelled and measured using the methods of the invention. When this is the case, there is a -1 in the Rule column of Table 4 (Rule = -1), and 1 is subtracted from the number of occurrences of amino acids of that amino acid type in the protein sequence of a protein of interest. For example, labelling the lysine (K) amino acid type with OPA labels and measures unmodified lysine amino acids, and labelling serine with Fl-DIBO labels and measures unmodified serine amino acids. In some embodiments, both modified and unmodified amino acids of an amino acid type are labelled and measured using the methods of the invention. When this is the case, there is a 0 in the Rule column of Table 4 (Rule = 0), and no value is subtracted from the number of occurrences of amino acids of that amino acid type in the protein sequence of a protein of interest when experimental or theoretical data shows that the indicated post-translational modification is observed. For example, labelling the tryptophan (W) amino acid type with TCE labels and measures both unmodified and modified tryptophan amino acids, because attachment of a substituent to the aromatic ring does not significantly affect the nucleophilicity of the aromatic ring to radical-mediated TCE chemistry. Labelling the tyrosine (Y) amino acid type with TCE labels and measures both unmodified and modified tyrosine amino acids because the attachment of a substituent to the aromatic ring does not significantly affect the nucleophilicity of the aromatic ring to radical mediated TCE chemistry. Summarising these rules for labelling all (unmodified + modified) or unmodified amino acids within amino acid types reported in Table 4, both unmodified and modified amino acids are labelled if the post-translational modification does not affect the portion of the R-group that is reacting with the label (e.g. dye) chosen for the labelling of the amino acid type, and only unmodified amino acids are labelled if the post-translational modification does affect the portion of the R-group that is reacting with the dye chosen for the labelling of the amino acid type.

In some embodiments, amino acids within an amino acid type can be converted between their modified and unmodified forms within the labelling reaction. Specifically, modified amino acids can be converted to unmodified amino acids. This enables labelling all amino acids of an amino acid type (unmodified + modified) within an amino acid type. This is achieved by, before reaction with the label, first converting the modified amino acids of the amino acid type to the unmodified amino acids of the amino acid type with a chemical reaction. For example, modified amino acids can be converted to unmodified cysteine amino acids via reduction with tris(2-carboxyethyl)phosphine (TCEP). As another example, glycosylated (modified) serine, threonine, or asparagine amino acids can be converted to unmodified serine, thereonine, or asparagine amino acids by raising the pH of the sample solution, for example to pH 10.5, as described in https://www.hindawi.com/journals/ijcc/2012/640923/. This cleaves the glycan residue from the amino acid R-group, such that the amino acid is no longer modified. An enzyme can alternatively be used to convert modified amino acids to unmodified amino acids. If the labelling methods incorporate a conversion step prior to reaction with the label, such that all (unmodified + modified) amino acids of that amino acid type are available for reaction with the label, then the PTM rules discussed are not applied when calculating the number of amino acids of that amino acid type in a protein sequence of the protein of interest. For example, when labelling all (unmodified + modified) amino acids of the cysteine amino acid type , the number of cysteine amino acids participating in disulphide bonds is not subtracted from the number of C amino acids displayed in the protein sequence because the modified cysteine amino acids have already been converted to unmodified amino acids via reduction.

In some embodiments, when TCEP is not used as part of the labelling of the cysteine amino acid type in the sample, resulting in only unmodified, reduced cysteine being labelled ($C_R$), cysteine amino acids participating in disulphide bonds are subtracted from the total number of cysteine amino acids, as explained in Table 4, in order to provide exclusively the reduced form of the cysteine amino acid type and generate the reference for the label value, amino acid concentration, or number of this amino acid type for one or more proteins, peptides, oligopeptides, polypeptides, protein complexes, subproteomes, or proteomes of interest as a function of the concentration of the one or more proteins, peptides, oligopeptides, polypeptides, protein complexes, subproteomes or proteomes of interest. Alternatively, a machine learning approach is used to calculate the number of amino acids of the $C_R$ amino acid type in the one or more proteins, peptides, oligopeptides, polypeptides, protein complexes, subproteomes, or proteomes of interest and the reference is generated accordingly. In some embodiments, the machine learning approach is the DIANNA machine learning approach. In some embodiments, the machine learning approach is the Dinosolve, GDAP or DBCP machine learning approach. The experimental information about protein disulphide bonds accessed via public databases such as Uniprot or via machine learning approaches like DiANNA is usually used to determine the number of modified cysteine amino acids, e.g. cysteine amino acids within a protein which are disulphide bonded. When the number of reduced cysteines (CR) is determined using the protein sequences and publicly available PTM information or machine learning approaches, the number of reduced cysteines within one or more proteins, peptides, oligopeptides, polypeptides, protein complexes, subproteomes, proteomes of interest, or, a mixture of proteins, peptides, polypeptides, oligopeptides, subproteomes or proteomes of interest is the total number of cysteines within one or more proteins, peptides, oligopeptides, polypeptides, protein complexes, subproteomes, proteomes of interest, or, a mixture of proteins, peptides, polypeptides, oligopeptides, subproteomes or proteomes of interest, minus the number of disulphide bonded cysteines within the one or more proteins, peptides, oligopeptides, polypeptides, protein complexes, subproteomes, or proteomes of interest.

In some embodiments, the $w_1$, $w_2$, and $w_j$ values for a proteome or subproteome of interest are calculated using publicly available proteome wide PTM statistics. The numbers of unmodified or modified amino acids can be calculated for a proteome or subproteome of interest by using publicly available proteome-wide post-translational modification statistics, for example as described by https://www.nature.com/articles/srep00090.pdf, or provided by publicly available online resources such as http://ares.tamu.edu/PTMCuration/. This avoids calculation of PTMs for every protein within a proteome or subproteome of interest. In some embodiments, this information is filtered to provide post-translational modification frequencies specific to prokaryotes, eukaryotes, and mammals including humans. In some embodiments, viruses are treated as not undergoing post-translational modifications because they do not contain genes coding for enzymes which carry out post-translational modifications. In some embodiments, viruses are treated as undergoing post-translational modifications or a subset of post-translational modifications that proteins within their host undergoes because viruses hijack the protein translational machinery of their host cells. For example, bacteriophages are treated as undergoing prokaryotic post-translational modifications and viruses affecting eukaryotes or mammals are treated as undergoing eukaryotic or mammal post-translational modifications. The total number of experimental, putative (predicted), or experimental and putative post-translational modifications observed within the Swiss Prot database is publicly available. To predict the number of unmodified amino acids of an amino acid type, or to predict the number of modified amino acids of an amino acid type, then the frequency of modification of that amino acid type is determined by summing all of the post-translational modifications affecting that amino acid type and dividing by the total number of amino acids in that amino acid type in the Swiss Prot database. The post-translational modifications affecting an amino acid type are provided, for example, in Table 4. This reveals a modification factor for each amino acid type which can differ by class of organism. For example, the modification factor ($M_F$) for all amino acids of the amino acid type lysine (K) in prokaryotes is determined by adding the reported frequencies of all post-translational modifications affecting the lysine amino acid type and then dividing by the total number of lysine amino acids within prokaryotic organisms in the Swiss Prot database. The fraction of unmodified lysine residues in prokaryotes is 1 - $M_F$. Therefore, when calculating the weighted mean number of lysine amino acids in a representative protein sequence corresponding to a bacterial proteome, this number of amino acids is multiplied by (1 - $M_F$) to calculate the number of unmodified lysine amino acids predicted for a bacterial proteome, or this number of amino acids is multiplied by $M_F$ to calculate the number of modified lysine amino acids predicted for a bacterial proteome.

In some embodiments, when determining the number of amino acids of each amino acid type in the sequence of the one or more proteins, peptides, oligopeptides, polypeptides, protein complexes, subproteomes, or proteomes of interest, the signal sequences or the regions which are biologically cleaved are removed from the sequence before the number of occurrences of amino acids of each amino acid type in the sequence is determined and/or the PTM rules are applied. This provides the number of amino acids of each amino acid type in the mature protein. However, this is not required if the one or more proteins, peptides, oligopeptides, polypeptides, protein complexes, or, a mixture of proteins, peptides, polypeptides, oligopeptides are not a mature protein.

In some embodiments, when there is a protein complex of interest, the number of amino acids of each amino acid type in each subunit of the protein complex is added to the number of amino acids of each corresponding amino acid type in

the one or more remaining subunits of the protein complex. For example, to calculate the number of amino acids of the W and K amino acid types for the 26S proteasome protein complex, the number of amino acids of the W amino acid types is summed across all subunits of the 26S proteasome and the number of amino acids of the K amino acid types is summed across all subunits of the 26S proteasome.

In some embodiments, determining the number of amino acids of each amino acid type in one or more proteins, peptides, oligopeptides, polypeptides, protein complexes, proteomes, or subproteomes of interest, or a mixture of proteins, peptides, oligopeptides, polypeptides, protein complexes, proteomes, or subproteomes of interest to provide $a_1$, $a_2$, and $a_j$ or $w_1$, $w_2$, and $w_j$ values to generate the reference does not involve examining protein sequence or protein sequences of the of each protein, peptide, oligopeptide, polypeptide, protein complex, proteome, or subproteome of interest. The number of amino acids of each amino acid type can be determined using the methods of the invention. This automatically detects the biologically relevant forms (e.g. signal sequences cleaved) of the proteins and constructs the reference based on amino acids within amino acid types which have not been modified with PTMs that would affect reaction with the label. For example, the number of unmodified serine (S) amino acids for a proteome of interest is determined by measuring the proteome of interest using the methods of the invention. In this embodiment, it is not necessary to know the, for example, glycosylation patterns of all proteins within the proteome of interest, because the proteome of interest can simply be measured and provided as an experimental reference, revealing the number of unmodified serine amino acids within the proteome of interest, as $w_1$, $w_2$, or $w_j$ within parametric equation 2 or parametric equation 4. This approach can be preferable for proteomes of interest which have undergone extensive post-translational modifications, and also automatically provides the weighted number of amino acids within the proteome of interest without providing the proportion of individual proteins within the proteome of interest (for example, with equation 10 or equation 11). In some embodiments, the number of amino acids of each amino acid type in the one or more proteins, peptides, oligopeptides, polypeptides, protein complexes, subproteomes, proteomes, or a mixture of proteins, peptides, polypeptides, oligopeptides, subproteomes or proteomes of interest can be determined as part of the method. For example, when determining the reference for the SARS-CoV-2 proteome of interest, as an alternative to calculating the $w_1$, $w_2$, or $w_j$ values for the SARS-CoV-2 proteome based on proteins comprising the SARS-CoV-2 proteome, the SARS-CoV-2 proteome can be experimentally measured by isolating SARS-CoV-2 viruses from SARS-CoV-2$^+$ patients nasal secretions, lysing the viruses, measuring the amino acid concentrations of the amino acid types (e.g. the W and K amino acid types) using the methods disclosed herein, measuring the total molar concentration of the sample in mg/mL using methods known in the art, converting the mass concentration to molar concentration based on calculating the combined molecular weight of all protein sequences for proteins contained within the SARS-CoV-2 proteome, and dividing the measured amino acid concentrations for the W and K amino acid types by this calculated molar concentration, to provide the $w_1$, $w_2$, or $w_j$ values for the SARS-CoV-2 proteome, providing set of parametric equations 2 experimentally for subsequent determination of the presence and/or concentration and/or amount of the SARS-CoV-2 proteome of interest within patient samples.

In alternative embodiments, the sequence or sequences of the one or more proteins, peptides, oligopeptides, polypeptides, protein complexes, subproteomes, proteomes of interest, or, a mixture of proteins, peptides, polypeptides, oligopeptides, subproteomes or proteomes of interest is/are determined as part of the method. In some embodiments, the sequence or sequences of the one or more proteins, peptides, oligopeptides, polypeptides, protein complexes, subproteomes, proteomes of interest, or, a mixture of proteins, peptides, polypeptides, oligopeptides, subproteomes or proteomes of interest is/are determined using Edman protein degradation or mass spectrometry. In all embodiments, it is not necessary to sequence the sample.

In some embodiments, the protein sequence or protein sequences of the one or more proteins, peptides, oligopeptides, polypeptides, protein complexes, subproteomes, proteomes of interest, or, a mixture of proteins, peptides, polypeptides, oligopeptides, subproteomes or proteomes of interest are known. In some embodiments, the protein sequence or protein sequences are known and provided in a database. In some embodiments, the sequence of the one or more proteins, peptides, oligopeptides, polypeptides, protein complexes, subproteomes, proteomes of interest, or, a mixture of proteins, peptides, polypeptides, oligopeptides, subproteomes or proteomes of interest is provided in a database. Preferably, the database is the UniProt database, UniProt Proteome database, Swiss Prot database, GenBank, Blast, NCBI Protein database or GenBank Sequence Read Archive (SRA).

In some embodiments, the sequences of specifically the proteins, peptides, oligopeptides, polypeptides, protein complexes, subproteomes, or proteomes of interest, or of the proteins comprising a subproteome or proteome of interest, are displayed and accessed in a database. This database is a subset of a larger database, like the UniProt database, that lists all known protein sequences. The protein sequences can be retrieved from the publicly available database using their identifiers, such as their UniProt KB identifiers, and their sequence information downloaded, for example in FASTA format. The name or identifier of each sequence can also be stored and accessed in this smaller database, or in a corresponding database with the same indexing as the protein sequence database. This ensures that, for example, if the user finds that the 10$^{th}$ protein of interest is present within the sample, because the 10$^{th}$ protein of interest within the reference database (e.g. row 10 of the reference database) satisfied test 2, that the user can conveniently access the name and/or identifier and/or full protein sequence of the 10$^{th}$ protein of interest by accessing the 10$^{th}$ entry (e.g. row) of a corresponding

database containing the names and/or identifiers and/or full protein sequences of each protein of interest (see the sample tables provided herein). In some embodiments, the database of protein sequence names and/or identifiers and/or full protein sequences is updated if optional preprocessing steps have been undertaken to, for example, combine (e.g. roll up) subunits of protein complexes that will be reflected in the reference database.

In some embodiments, the $a_1$, $a_2$, and $a_j$ or $w_1$, $w_2$, and $w_j$ values of the one or more proteins, peptides, oligopeptides, polypeptides, protein complexes, subproteomes, proteomes of interest, or, a mixture of proteins, peptides, polypeptides, oligopeptides, subproteomes or proteomes of interest has been previously determined and can be accessed in a database. In some embodiments, the number of amino acids of each amino acid type in the one or more proteins, peptides, oligopeptides, polypeptides, protein complexes, subproteomes, proteomes of interest, or, a mixture of proteins, peptides, polypeptides, oligopeptides, subproteomes or proteomes of interest, or of the proteins comprising the proteome or subproteome of interest, has previously been determined and can be accessed separately. It can be displayed and accessed in a database, for example with the same indexing as the corresponding name and/or identifier and/or full amino acid sequence database discussed above. Preferably, the number of amino acids of each amino acid type in the one or more proteins, peptides, oligopeptides, polypeptides, protein complexes, subproteomes, proteomes of interest, or, a mixture of proteins, peptides, polypeptides, oligopeptides, subproteomes or proteomes of interest is available in a database.

For example, the database contains the number of amino acids of the C, K, and W amino acid types for all proteins within the human plasma proteome. The proteins within the human plasma proteome are accessed, for example via the Protein Atlas, Peptide Atlas or Proteome Xchange databse, which provides a repository of publicly available protein identification and quantification data (https://www.nature.com/articles/nbt.2839, http://proteomecentral.proteomex change.org/cgi/GetDataset). Identifiers which are provided and publicly available, for example UniProt KB identifiers, are used to retrieve a protein sequence for each identifier. Once the protein sequences are retrieved, signal sequences which are not present in mature proteins are discarded, and amino acid sequences for subunits within a protein complex are combined into one entry. In some embodiments, the number of amino acids of the C, K, and W amino acids types are calculated by determining the number of occurrences of the C, K, and W amino acid types within the processed protein sequences after the rules provided in Table 4 are applied to avoid counting amino acids of an amino acid type that will not react with a label because they are post-translationally modified in a manner which makes them unreactive with the label. For example, if it is indicated that a lysine amino acid is post-translationally modified to form an isopeptide bond with glutamine, and another lysine amino acid is acetylated, then 2 is subtracted from the total number of occurrences of the K amino acid type within the protein sequence of this protein. Preferably, this step is automated with a computer program, that processes the protein sequences according to the logical Rules outlined in Table 4. The rule to subtract one from the number of amino acids of an amino acid type can be overridden if the modified amino acid type is converted to the unmodified amino acid type prior to or during the labeling reactions (if it is desired to label both unmodified plus modified amino acids of an amino acid type). For example, if both modified and unmodified cysteine amino acid types are labelled, TCEP is used to reduce disulphide bonded cysteines contained in the sample, so the logical rule for disulphide bonded cysteine is ignored and no values are subtracted from the number of occurrences of the C amino acid type within protein sequences. Frequently, the number of occurrences of both the unmodified and modified amino acids within protein sequences is equal to all occurrences of the C amino acid type within protein sequences, because post-translational modifications on cysteine amino acids other than disulphide bond formation are rare.

As another example, applying these steps produced the following number of amino acids for the C, K, and W amino acid types, which can be displayed in a database:

| | C | K | W |
|---|---|---|---|
| Affamin | 34 | 49 | 0 |
| Talin-1 | 38 | 159 | 9 |
| L-selectin | 22 | 24 | 11 |
| C-reactive protein | 2 | 13 | 6 |
| Lumican | 6 | 25 | 1 |

In this example, the user will measure amino acid concentrations of C, W and K in the sample. To create the reference database, parametric equation 1 is applied sequentially to each row of the database to produce the reference for each protein of interest as a function of any protein concentration, $t$:

| | | $a_1t$ | $a_2t$ | $a_3t$ |
|---|---|---|---|---|
| Affamin | $n_1$ | 34t | 49t | 0t |
| Talin-1 | $n_2$ | 38t | 159t | 9t |
| L-selectin | $n_3$ | 22t | 24t | 11t |
| C-reactive protein | $n_4$ | 2t | 13t | 6t |
| Lumican | $n_5$ | 6t | 25t | 1t |

The reference for each protein of interest, $p_1$, $p_2$, $p_3$, $p_4$, and $p_5$ is a line in 3-dimensional space. The reference for each protein of interest is a line in 3-dimensional space because 3 types of amino acids are labelled and measured in the sample, C is amino acid type $a_1$, the amino acid type K is the amino acid type $a_2$, and the amino acid type W is amino acid type $a_3$. Because set of parametric equations 1 defines the reference for all $t \geq 0$, all of the reference lines intersect at the origin (and any value of $a_1$, $a_2$, ... $a_j$ multiplied by 0 equals 0).

In other embodiments, if the user will measure the value of the label of the C, K, and W amino acid types, then the calibration factor, *fn*, for each amino acid type is incorporated into the reference database.

For example, the C amino acid type is amino acid type 1, and the calibration factor for the C amino acid type, $f_1$, determined from a linear calibration curve is

$$f_1 = 50\ \left({}^{AU}/_{\mu M}\right)$$

The K amino acid type is amino acid type 2, and the calibration factor for the K amino acid type, $f_2$, determined from a linear calibration curve is

$$f_2 = 75\ \left({}^{AU}/_{\mu M}\right)$$

The W amino acid type is amino acid type 3, and the calibration factor for the W amino acid type, $f_3$, determined from a linear calibration curve is

$$f_3 = 100\ \left({}^{AU}/_{\mu M}\right)$$

To create the reference database, parametric equation 3 is applied sequentially to each row of the database to produce the reference for each protein of interest as a function of protein concentration, *t*:

| | | $a_1f_1t$ | $a_2f_2t$ | $a_3f_3t$ |
|---|---|---|---|---|
| Affamin | $n_1$ | 1700t | 3675t | 0t |
| Talin-1 | $n_2$ | 1900t | 11925t | 900t |
| L-selectin | $n_3$ | 1100t | 1800t | 1100t |
| C-reactive protein | $n_4$ | 100t | 975t | 600t |
| Lumican | $n_5$ | 300t | 1875t | 100t |

When the measurements of the sample are provided in terms of the measured label (e.g. signal of the label) of amino acid type 1, amino acid type 2, and amino acid type n, the parametric equation of the reference line for a proteome or subproteome of interest is given by parametric equation 4:

$$p_i(t) = [w_1 f_1 t, w_2 f_2 t, \cdots, w_n f_n t], \forall t \geq 0$$

wherein $n_i$ proteome or subproteome of interest, $w_1$ is the weighted mean number of amino acids of amino acid type 1 in the

proteome or subproteome of interest, $f_1$ is the calibration factor or calibration function for amino acid type 1, $w_2$ is the weighted mean number of amino acids of amino acid type 2 in the proteome or subproteome of interest, $f_2$ is the calibration factor or calibration function for amino acid type 2, $w_n$ is the weighted mean number of amino acids of amino acid type n in the proteome or subproteome of interest, $f_n$ is the calibration factor or calibration function for amino acid type n, $t$ is the concentration of the proteome or subproteome of interest which is the common independent variable (or parameter) in each of the n functions which collectively specify the reference line in each of the $n$ dimensions, and where $t$ is defined for all $t$ greater than or equal to 0 ($\forall t \geq 0$).

*Mixtures*

When the methods of the invention are used to detect a mixture of proteins, proteomes, peptides, oligopeptides, polypeptides, protein complexes, or subproteomes, the reference is provided using the approach outlined in this section for a single protein, proteome, peptide, oligopeptide, polypeptide, protein complex, or subproteome. As outlined in the Comparison Step section, a mixture is detected because the presence of multiple pure proteins, proteomes, peptides, oligopeptides, polypeptides, protein complexes, or subproteomes are detected in the sample, and the methods of the invention are used to provide the proportion and concentration of each component within the mixture.

*Special* cases

There are two special cases of these embodiments, described in the form of the general set of parametric equations; In special case 1, the molar concentration of the sample is known, and this is a constant SC. Therefore, if protein of interest $n_i$ is present within the sample, then it is present at the molar concentration of the sample, so $t = SC$. The general set of parametric equations is $p_i(t) = [c_1t, c_2t, \ldots c_nt], \forall t \geq 0$.

In special case 1, the general set of parametric equations simplifies to a point in n dimensional space

$$p_i - (c_1SC, c_2SC, \ldots, c_nSC)$$

This is no longer a parametric equation because it is not a function of a common parameter (independent variable), because the variable $t$ was replaced with the constant SC. In special case 1, the reference for protein of interest $p_i$ is provided by:

$$p_i = \left(c_1SC, c_2SC, \ldots, c_jSC\right)$$

This describes a point in n dimensional space, where n is the number of amino acid types labelled and measured in the sample.

In special case 1, test 1 is fulfilled if there exists a protein of interest $p_i$ such that

$$S_1 = c_1SC$$

$$S_2 = c_2SC$$

$$S_n = c_nSC$$

where $S_1$ is the value (amino acid concentration or signal of the label) measured for amino acid type 1 in the sample, $S_2$ is the value (amino acid concentration or signal of the label) measured for amino acid type 2 in the sample, and $S_n$ is the value (amino acid concentration or signal of the label) measured for amino acid type n in the sample, In special case 1, if test 1 is fulfilled, then protein of interest $p_i$ is present within the sample.

In special case 1, test 2 is fulfilled if there exists a protein of interest $p_i$ such that the distance between the sample point ($S_1$, $S_2$, $S_j$) and the reference point for the protein of interest ($c_1SC$, $c_2SC$, $c_nSC$) is less than the error margin, $\varepsilon$. The distance between the sample point ($S_1$, $S_2$, $S_n$) and the reference point for the protein of interest ($c_1SC$, $c_2SC$, $c_nSC$) is calculated using a distance formula such as Euclidean distance. For example, the distance D is calculated using

$$D = \sum_{i=1}^{n}((S_1 - c_1SC)^2 + (S_2 - c_2SC)^2 + \cdots + (S_n - c_nSC)^2)$$

Exclusively within special case 1, calculating the number of amino acids within each labelled and measured amino acid type in the sample is available. This is available because the number of amino acids of each amino acid type of the sample is equal to the amino acid concentration of that amino acid type of the sample divided by the known molar concentration of the sample. The number of amino acids of amino acid type 1 in the sample is $N_1$, the number of amino acids of amino acid type 2 in the sample is $N_2$, and the number of amino acids of amino acid type n in the sample is $N_n$. The number or mean number of amino acids of amino acids of amino acid type 1 in the protein, peptide, polypeptide, protein complex, subproteome, or proteome of interest is $RN_1$, the number of amino acids of amino acid type 2 in the protein, peptide, polypeptide, protein complex, subproteome, or proteome of interest is $R_{N2}$, and the number of amino acids of amino acid type n in the protein, peptide, polypeptide, protein complex, subproteome, or proteome of interest is $RN_n$. In some embodiments, these numbers of amino acids are calculated from the protein sequence or protein sequences of the protein, peptide, polypeptide, protein complex, subproteome, or proteome of interest, removing portions of the sequence that are biologically cleaved in the mature protein, and subtracting the number of amino acids of each amino acid type that would not react with the label due to post-translational modification of the reactive portion of the amino acid R-group, such as conversion of a lysine primary amine to a secondary amine through alkylation, acetylation, or glycyl-lysyl isopeptide formation, and/or applying equation 11 or equation 12 to determine the weighted mean number of amino acids in a proteome or subproteome of interest.

When the number of amino acids of each amino acid type in the sample is calculated within special case 1, test 1 is fulfilled when

$$N_1 = RN_1$$

$$N_2 = RN_2$$

$$N_n = RN_n$$

Test 1 is fulfilled when the sample point is exactly equal to the reference point.

When the number of amino acids of each amino acid type in the sample is calculated within special case 1, test 2 is fulfilled when the number of amino acids of each amino acid type in the protein, peptide, polypeptide, protein complex, subproteome, or proteome of interest is less than or equal to an error margin, $\varepsilon$, of the number of amino acids of each amino acid type in the sample. This is evaluated by determining the distance, D, between the sample point and the reference point, for example the Euclidean distance.

The formula is:

$$D = \sum_{i=1}^{n}((N_1 - RN_1)^2 + (N_2 - RN_2)^2 + \cdots + (N_n - RN_n)^2)$$

In special case 2, the presence of protein, peptide, oligopeptide, polypeptide, subproteome, or proteome of interest $n_i$ within the sample is known, but the concentration of protein, peptide, oligopeptide, polypeptide, subproteome, or proteome of interest $n_i$ within the sample is unknown. As before, values for amino acid type 1, amino acid type 2, and amino acid type n have been measured for the sample as $S_1$, $S_2$, and $S_n$.

The general parametric equation remains $p_i(t) = [c_1t, c_2t,...c_nt], \forall t \geq 0$

However, the presence of protein of interest $p_i$ is not evaluated using test 1 because the presence of the of protein, peptide, oligopeptide, polypeptide, subproteome, or proteome of interest in the sample is already known. Instead, the known values are inputted into the equations comprising test 1, and the equations are solved for *t*,

$$S_1 = c_1t$$

$$S_2 = c_2t$$

$$S_n = c_nt$$

Because the presence of protein, peptide, oligopeptide, polypeptide, subproteome, or proteome of interest $n_i$ within the sample is known, $c_1$, $c_2$, and $c_n$ are known. $S_1$, $S_2$, and $S_n$ are known because they have been measured. Therefore, these values are inputted into the equations comprising test 1 which are simply solved for *t*, and the n values of *t* are averaged (i.e. mean) to provide measurement of the concentration of of protein, peptide, oligopeptide, polypeptide,

subproteome, or proteome of interest $p_i$ within the sample. Because labeling each of the n amino acid types involves orthogonal chemistry subject to different types of errors, averaging the concentrations obtained via these orthogonal approaches provides a highly accurate measurement of concentration; existing approaches only use one method of determining concentration such as measuring the absorbance at 280 nm (A280). The concentration determined in this way is multiplied by the volume of the sample to provide the amount of protein of interest $p_i$ within the sample.

In some embodiments, if an isolation step has been performed on the sample, the same isolation step should be performed on the theoretical reference. As an example, an isolation step on the sample can include filtration through a centrifugal filter device incorporating a centrigual molecular weight cutoff filter, where proteins with molecular weights less than the molecular weight of the cutoff filter pass through the filter into the filtrate and proteins with molecular weights greater than the cutoff filter do not pass through the filter and are thus absent from the filtrate, such as an Amicon 50 kDa centrifugal filter unit. This step can be used to deplete high abundance high molecular weight proteins from the sample, as disclosed herein. If this step has been performed on the sample, then proteins with molecular weights greater than 50 kDa should also be removed from the database used to calculate the theoretical reference. This simulates the situation of proteins with molecular weights greater than 50 kDa no longer being present within the sample after the filtration step. The filtration step reduces the number of amino acids of each amino acid type in an average protein sequence within the sample. The molecular weight of an average protein sequence within the sample decreases because proteins with molecular weight e.g. greater than 50 kDa, are no longer included in the sample, and molecular weight and protein length are linearily correlated.

## Comparison

The measured label, amino acid concentration, and/or number of amino acids of each labelled amino acid type of the sample, is compared to the known label values, amino acid concentrations, and/or number of the same amino acid types of the one or more proteins, peptides, oligopeptides, polypeptides, protein complexes, subproteomes, proteomes of interest, or, a mixture of proteins, peptides, polypeptides, oligopeptides, subprotomes or proteomes of interest. Preferably, the known label values, amino acid concentrations, and/or number of the same amino acid types of the one or more proteins, peptides, oligopeptides, polypeptides, protein complexes, subproteomes, or proteomes of interest, or, a mixture of proteins, peptides, polypeptides, oligopeptides, subprotomes or proteomes of interest is a reference. In some embodiments, if the measured label, amino acid concentration, and/or number of amino acids of each labelled amino acid type of the sample is identical to, or is within an error margin to the known label values and/or amino acid concentrations as a function of protein concentration, and/or numbers of the same amino acid types of the one or more proteins, peptides, oligopeptides, polypeptides, protein complexes, subproteomes, proteomes of interest, or, a mixture of proteins, peptides, polypeptides, oligopeptides, subprotomes or proteomes of interest, then a positive identification of the presence and/or concentration and/or amount of the one or more proteins, peptides, oligopeptides, polypeptides, protein complexes, subproteomes, proteomes of interest, or, a mixture of proteins, peptides, polypeptides or oligopeptides, subproteomes or proteomes of interest in the sample is made.

If the measured label, amino acid concentration, and/or number of amino acids of each labelled amino acid type of the sample is outside of an error margin to the known label values and/or amino acid concentrations as a function of protein concentration, and/or numbers of the same amino acid types of the one or more proteins, peptides, oligopeptides, polypeptides, protein complexes, subproteomes, proteomes of interest, or, a mixture of proteins, peptides, polypeptides, oligopeptides, subprotomes or proteomes of interest then the presence of one or more proteins, peptides, oligopeptides, polypeptides, protein complexes, subproteomes, proteomes of interest, or, a mixture of proteins, peptides, polypeptides or oligopeptides, subproteomes or proteomes of interest is not identified in the sample. If a protein of interest is not present in the sample, it cannot have a concentration in the sample. In some embodiments, if the presence of the protein of interest in the sample is already known, the protein concentration is determined by solving the reference functions for the protein concentration (t). In some embodiments, the amount of the protein of interest is identified by multiplying the volume of the sample by the concentration of the protein, peptide, oligopeptide, polypeptide, protein complex, subproteome, or proteome of interest identified in the sample.

As described in the Reference section, each protein, peptide, oligopeptide, polypeptide, protein complex, proteome, or subproteome of interest can be uniquely described by a reference. The reference for each protein, peptide, oligopeptide, polypeptide, protein complex, proteome, or subproteome of interest provides the values (measured labels e.g. signal of the label, of each amino acid type and/or, amino acid concentration of each amino acid type) for each protein, peptide, oligopeptide, polypeptide, protein complex, proteome, or subproteome of interest as a function of the concentration of the protein, peptide, oligopeptide, polypeptide, protein complex, proteome, or subproteome of interest, or the number of amino acids of each amino acid type for each protein, peptide, oligopeptide, polypeptide, protein complex, proteome, or subproteome of interest. The reference for each protein, peptide, oligopeptide, polypeptide, protein complex, proteome, or subproteome of interest may be a group of functions which predicts the values (measured labels, e.g. signal of the label, of each amino acid type, amino acid concentration of each amino acid type) for each protein, peptide,

oligopeptide, polypeptide, protein complex, proteome, or subproteome of interest as a function of the concentration of the protein, peptide, oligopeptide, polypeptide, protein complex, proteome, or subproteome of interest. Alternatively, if the molar protein concentration of the sample is known, the reference is the number of amino acids of each corresponding amino acid type of each protein, peptide, oligopeptide, polypeptide, protein complex, proteome, or subproteome of interest, or the amino acid concentration or value of the label of each corresponding amino acid type at the concentration of the sample of each protein, peptide, oligopeptide, polypeptide, protein complex, proteome, or subproteome of interest. The values measured for the sample (measured label, e.g. signal of the label, of each amino acid type, amino acid concentration of each amino acid type, and/or number of amino acids of each amino acid type) are compared to the values provided by the reference for one or more proteins, peptides, oligopeptides, polypeptides, protein complexes, proteomes, or subproteomes of interest. In some embodiments, if the values measured for the sample are identical to, or within an error margin of, the values provided by reference, then the presence and/or concentration and/or amount of one or more proteins, peptides, oligopeptides, polypeptides, protein complexes, proteomes, or subproteomes of interest is identified in the sample. In some embodiments, if the values measured for the sample are identical to, or within an error margin of, the values provided by reference, then the presence of one or more proteins, peptides, oligopeptides, polypeptides, protein complexes, proteomes, or subproteomes of interest is identified in the sample. In some embodiments, if the values measured for the sample are outside of an error margin to the values provided by the reference, the presence of one or more proteins, peptides, oligopeptides, polypeptides, protein complexes, proteomes, or subproteomes of interest is not identified in the sample.

In some embodiments, the measured labels of each of two or more amino acid types (e.g. fluorescence intensity of the amino acid types W and K) of the sample are compared to the known label value provided by the reference of the same amino acid types (e.g. fluorescence intensity of the amino acid types W and K) of a protein of interest. If the measured labels (e.g. fluorescence intensity of the amino acid types W and K ) of the sample are the same as, or, within an error margin to the known label values (e.g. fluorescence intensity of the amino acid types W and K) provided by the reference for the protein of interest as a function of protein concentration then a positive identification of the presence and/or concentration and/or amount of that protein of interest in the sample is made. If the measured labels (e.g. fluorescence intensity of the amino acid types W and K) of the sample are not the same as, or outside of the error margin to the known label values (e.g. fluorescence intensity of the amino acid types W and K) provided by the reference for the protein of interest as a function of protein concentration, then the presence of that protein of interest is identified as not being present in the sample. Because that protein of interest is not identified in the sample, there is no concentration or amount of that protein of interest within the sample.

In some embodiments, the amino acid concentrations of each of two or more amino acid types (e.g. amino acid concentrations of the amino acid types W and K) of the sample are compared to the amino acid concentrations of the same amino acid types (e.g. amino acid concentrations of the amino acid types W and K) provided by the reference for a protein of interest as a function of protein concentration. If the amino acid concentrations (e.g. amino acid concentrations of the amino acid types W and K ) of the sample are the same as, or, within an error margin to the amino acid concentrations (e.g. amino acid concentrations of the amino acid types W and K) provided by the reference for the protein of interest as a function of protein concentration then a positive identification of the presence and/or concentration and/or amount of that protein of interest in the sample is made. If the amino acid concentrations (e.g. fluorescence intensity of the amino acid types W and K) of the sample are not the same as, or outside of the error margin to the amino acid concentrations (e.g. fluorescence intensity of the amino acid types W and K) provided by the reference the protein of interest as a function of protein concentation, then that protein of interest is identified as not being present in the sample. Because that protein of interest is not identified in the sample, there is no concentration or amount of that protein of interest within the sample.

In some embodiments, the number of amino acids of each of two or more amino acid types (e.g. number of amino acids per protein of the amino acid types W and K) of the sample are compared to the reference number of amino acids of the same amino acid types (e.g. number of amino acids per protein of the amino acid types W and K) of a protein of interest. If the number of amino acids of each amino acid type (e.g. number of amino acids per protein of the amino acid types W and K) of the sample are the same as, or, within an error margin to the reference number of amino acids of each amino acid type (e.g. number of amino acids per protein of the amino acid types W and K) of the protein of interest then a positive identification of the presence of that protein of interest in the sample is made. If the number of amino acids of each amino acid type (e.g. number of amino acids per protein of the amino acid types W and K) of the sample are outside of the error margin to the reference number of amino acids (e.g. number of amino acids per protein of the amino acid types W and K) of the protein of interest, then that protein of interest is identified as not being present in the sample. Because that protein of interest is not identified in the sample, there is no concentration or amount of that protein of interest within the sample. Measuring the number of amino acids of each amino acid type in the sample is available exclusively when the molar protein concentration of the sample is known and falls under special case 1, so the comparison step specific to this embodiment is discussed there.

The general form of the reference is a line in n dimensional space, where n is the number of amino acid types labelled and measured in the sample. The reference can be described with a set of parametric equations, which specifies

how each of the coordinates (amino acid concentration, or measured label, e.g. signal of the label) varies as a function of the concentration, *t*, of the protein, peptide, oligopeptide, polypeptide, protein complex, subproteome, or proteome of interest. The specific parametric equations have been provided and explained in the Reference section. The general parametric equation is:

$$p_i(t) = [c_1 t, c_2 t, \cdots, c_n t], \forall t \geq 0$$

wherein $p_i$ is the protein, proteome, peptide, oligopeptide, polypeptide, protein complex, or subproteome of interest, $c_1$ the coefficient for amino acid type 1, $c_2$ the coefficient for amino acid type 2, and $c_n$ the coefficient for amino acid type n labelled and measured in the sample, each provided according to (explained for the example of amino acid type n but also applying to amino acid type 1 and 2):

- when the measurement for the sample is provided in amino acid concentration and the reference line describes a protein, peptide, oligopeptide, polypeptide, or protein complex of interest, $c_n = a_n$, where $a_n$ is the number of amino acids of amino acid type n in the protein, peptide, oligopeptide, polypeptide, or protein complex of interest, with $a_n$ being an integer greater than or equal to 0 ($a_n \in Z^+$)
- when the measurement for the sample is provided in amino acid concentration and the reference line describes a proteome or subproteome of interest, $c_n = w_n$, where $w_n$ is the weighted mean number of amino acids of amino acid type n in the proteome or subproteome of interest
- when the measurement for the sample is provided in measured label, e.g. signal of the label and the reference line describes a protein, peptide, oligopeptide, polypeptide, or protein complex of interest is $c_n = a_n f_n$, where $a_n$ is the number of amino acids of amino acid type n in the protein, peptide, oligopeptide, polypeptide, or protein complex of interest, with $a_n$ being an integer greater than or equal to 0 ($a_n \in Z^+$), and where $t_n$ is a calibration factor or calibration function which converts between amino acid concentration and measured label, e.g. signal of the label for amino acid type n
- when the measurement for the sample is provided in measured label , e.g. signal of the label and the reference line describes a proteome or subproteome of interest is $c_n = w_n f_n$ where $w_n$ is the number of amino acids of amino acid type n in the protein, peptide, oligopeptide, polypeptide, or protein complex of interest, where $w_n$ is the weighted mean number of amino acids of amino acid type n in the proteome or subproteome of interest, and where $f_n$ is a calibration factor or calibration function which converts between amino acid concentration and measured label, e.g. signal of the label, for amino acid type n.

Where *t* is the protein concentration of a solution containing the reference which is the common independent variable (or parameter) in each of the n functions which collectively specify the reference line in each of the n dimensions, and where *t* is defined for all *t* greater than or equal to 0 ($\forall t \geq 0$) The reference line can alternatively be described as a vector in n dimensional space, which will be explained later within the discussion of hypothesis test 2.

There are n amino acid types labelled and measured in the methods of the invention ($n \geq 2$). The values measured for the sample always provide a point in n dimensional space, because one value is provided for each of the n dimensions.

The point has coordinates ($S_1$, $S_2$, ... , $S_n$) where $S_1$ is the value measured for amino acid type 1 labelled in the sample, $S_2$ is the value measured for amino acid type 2 labelled in the sample, and $S_n$ is the value optionally measured for amino acid type n optionally labelled in the sample. The reference for a protein, peptide, oligopeptide, polypeptide, protein complex, proteome, or subproteome of interest can be a line in n dimensional space.

The general parametric equation, explained above, which provides the reference for protein, peptide, oligopeptide, polypeptide, protein complex, proteome, or subproteome of interest $p_i$ as a function of the protein concentration of the protein, peptide, oligopeptide, polypeptide, protein complex, proteome, or subproteome of interest is

$$p_i(t) = [c_1 t, c_2 t, \cdots, c_n t], \forall t \geq 0$$

Set of parametric equations 1, 2, 3, or 4 is used to generate each reference line for specific embodiments of the invention, as explained in the reference section.

Within the comparison step, the values measured for the sample point are compared to the reference. In some embodiments, the presence and/or concentration and/or amount of a protein, peptide, oligopeptide, polypeptide, protein complex, proteome, or subproteome of interest is identified in the sample if the values measured for the sample are equal to the values provided by the reference line. This means that the sample point is on the reference line. If the sample point is on the reference line, then the hypothesis that the protein, peptide, oligopeptide, polypeptide, protein complex, proteome, or subproteome of interest is present in the sample is true. In order to evaluate this hypothesis, test 1 is performed.

In some embodiments, the reference for each protein, peptide, oligopeptide, polypeptide, protein complex,

proteome, or subproteome of interest is a group of functions that provide the value for the protein, peptide, oligopeptide, polypeptide, protein complex, proteome, or subproteome of interest for each amino acid type labelled and measured in the sample as a function of the protein concentration of the protein, peptide, oligopeptide, polypeptide, protein complex, proteome, or subproteome of interest. Test 1 sets the values measured for the sample ($S_1$, $S_2$, ... $S_n$) equal to the corresponding function comprising the reference. If a single solution to the equations exists, then the sample point is on the reference line, the protein, peptide, oligopeptide, polypeptide, protein complex, proteome, or subproteome of interest is present in the sample, the protein concentration of the protein, peptide, oligopeptide, polypeptide, protein complex, proteome, or subproteome of interest is the single protein concentration that provided a solution to the equations, and the protein amount of the protein, peptide, oligopeptide, polypeptide, protein complex, proteome, or subproteome of interest is the protein concentration of the protein, peptide, oligopeptide, polypeptide, protein complex, proteome, or subproteome of interest multiplied by the volume of the sample.

The comparison can be performed informally or formally. Informally, it is generally possible to work out by eye whether the sample point is on the reference line, either by graphing the results or by comparing the values measured to the sample to the reference functions. For example, the amino acid types W and K are labelled and measured in the sample. The W amino acid type is amino acid type 1 and the K amino acid type is amino acid type 2. The reference for the protein of interest lysozyme (LYZ) is:

$$p_{LYZ}(t) = [6t, 6t], \forall t \geq 0$$

Amino acid concentrations of 60 $\mu M$ W and 60 $\mu M$ K are measured for the sample. When the amino acid concentrations measured for the sample are compared to the reference, it is apparent that the sample point is on the reference line, and that the reference has a protein concentration, *t*, of 10 uM (Fig. X).

Formally, test 1 involves setting each value measured for the sample ($S_1$, $S_2$, ... , $S_n$) equal to the corresponding function provided for the reference [$c_1t$, $c_2t$, $\cdots$, $c_nt$], and determining whether a solution for *t* exists. Test 1 is fulfilled if for all *t* ≥ 0, there exists a value of *t* such that:

$$S_1 = c_1 t$$

$$S_2 = c_2 t$$

$$S_n = c_n t$$

where the number of equations is equal to the n number of amino acid types labelled and measured in the sample. If the n equations comprising test 1 can be solved for a single value of *t*, then each protein, peptide, oligopeptide, polypeptide, protein complex, proteome, or subproteome of interest $p_i$ is present within the sample at protein concentration *t*, because the sample point is on the reference line.

In some embodiments, this can be stated formally as test 1 is fulfilled if

$$\forall t \geq 0, \exists t \ni S_k = c_k t \; \forall \, k = 1,2,\cdots,n$$

This says that test 1 is fulfilled if for all *t* ≥ 0, there exists a value of *t* such that $S_k = c_kt$ for all *k* = 1, 2, ... , *n*

Continuing with the example, each value measured for the sample is set equal to the corresponding function comprising the reference, and it is determined whether a single solution for *t* exists.

$$60 \, \mu M = 6t$$

$$60 \, \mu M = 6t$$

$$\frac{60 \, \mu M}{6} = t \, ; 60 \, \mu M = 6 \left( \frac{60 \, \mu M}{6} \right)$$

$$\therefore t - \frac{60 \, \mu M}{6} - 10 \, \mu M$$

If there exists a value of $t$ such that $S_k = c_k t$ for all $k = 1, 2, \ldots, n$, then this value of $t$ is the protein concentration of the sample. The protein amount of the sample is the protein concentration of the sample multiplied by the volume of the sample. In our example, the protein concentration of LYZ is 1.0 $\mu M$. The sample volume was 100 $\mu L$, so the protein amount of LYZ is 10 $\mu M \times 100\ \mu L = 1\ nmol$. The presence of LYZ has been identified in the sample, and the molecular weight of LYZ is 14.3 *kDa*. Therefore the mass of LYZ in the sample is

$$1\,nmol \times \frac{mol}{10^9\,nmol} \times \frac{14.3\,g}{mol} \times \frac{ng}{10^9\,g} = 14.3\ ng\ LYZ$$

In some embodiments, the presence and/or concentration and/or amount of a protein, peptide, oligopeptide, polypeptide, protein complex, proteome, or subproteome of interest can also be identified if the values measured for the sample are within an error margin, $\varepsilon$, of the values provided by the reference for a protein, peptide, oligopeptide, polypeptide, protein complex, proteome, or subproteome of interest. This reflects the fact that experimental measurements have neither infinite accuracy nor infinite precision, so the point measured for the sample will not always lie exactly on the reference line when the protein, peptide, oligopeptide, polypeptide, protein complex, proteome, or subproteome of interest $p_i$ is contained within the sample.

Test 2 considers experimental error, and tests the hypothesis that protein, peptide, oligopeptide, polypeptide, protein complex, proteome, or subproteome of interest $p_i$ is present within the sample by testing whether the sample point is within an error margin, $\varepsilon$, of the reference line. This is achieved by finding the shortest distance between the sample point and the reference line, and then determining whether this distance is less than the error margin. If this shortest distance between the sample point and the reference line is less than the error margin, then the presence of protein, peptide, oligopeptide, polypeptide, protein complex, proteome, or subproteome of interest $p_i$ within the sample is identified, and the protein concentration of protein, peptide, oligopeptide, polypeptide, protein complex, proteome, or subproteome of interest $p_i$ within the sample is provided by the exact point (protein concentration) on the reference line which gave the shortest distance.

The shortest distance between a point and a line is the perpendicular distance between the point and the line. The reference line, in addition to being described parametrically for example by the general parametric equation, can also be described in vector format allowing calculation of the exact point (protein concentration) on the reference line that yields this perpendicular distance via the dot product. Then, the distance formula, for example the Euclidean distance formula, is used to find the distance between the sample point and this perpendicular distance point, and the distance is compared to the error margin, $\varepsilon$, to determine whether protein, peptide, oligopeptide, polypeptide, protein complex, proteome, or subproteome of interest $p_i$ is present within the sample. If protein, peptide, oligopeptide, polypeptide, protein complex, proteome, or subproteome of interest $p_i$ is present within the sample, then its protein concentration within the sample is the protein concentration (point) on the reference line to which the sample point was perpendicular.

The shortest distance between a line and a point is always the perpendicular distance between a line and a point. This is because any other angle between the point and the line other than 90 degrees would form a hypotenuse, whose length will always be greater than the perpendicular distance.

The shortest distance, i.e. perpendicular distance, is calculated by finding the dot product of the direction of the reference line with the vector between the sample point and any point on the reference line, setting the dot product equal to 0, and solving for the concentration of the reference line which provides a perpendicular line between the sample point and the reference line. A dot product is a scalar value that represents the angular relationship between two vectors A and B i.e. $A \cdot B = |A| * |B| = \cos\theta$ where the values $|A|$ and $|B|$ represent the lengths of vectors A and B respectively, and $\theta$ is the angle between the two vectors. If A and B are perpendicular (i.e. at 90 degrees to each other) then the dot product will be zero, because cos 90° will be zero. This distance between the sample point and the reference line is calculated, and if this distance is less than or equal to an error margin, then the protein, peptide, oligopeptide, polypeptide, protein complex, proteome, or subproteome of interest is identified as being present at the protein concentration on the reference line which provided the perpendicular (minimum) distance. In some embodiments, other distance metrics are used in the methods of the invention, for example cityblock, chebychev, correlation, cosine, hamming, jaccard, mahalanobis, minkowski, seuclidean, or spearman.

The general approach for *Test* 2 is:

9. Let R be the reference line for protein, peptide, oligopeptide, polypeptide, protein complex, proteome, or subproteome of interest $p_i$, and let S be the sample point to find the shortest distance from
10. Find the equation of reference line R in vector format
11. Find the general equation of a point P on the reference line R
12. Find the exact location of the point P on reference line R, called Q, such that the vector from S to P is perpendicular to R. This means find the point Q on reference line R such that the vector between S and Q gives the perpendicular. This is achieved by finding the dot product (•) of the vector from S to P with the direction of R, setting this equal to 0, and solving for $t$ to provide the value of $t$ which, when substituted into the general equation for a point P on R, yields the perpendicular vector. If protein, peptide, oligopeptide, polypeptide, protein complex, proteome, or subproteome of

interest $p_i$ is contained within the sample, then this solution for *t* is its protein concentration.
13. Find the distance between Q and S using the distance formula, called D.
14. Evaluate whether D is less than the error margin, $\varepsilon$.
15. If $D > \varepsilon$, then protein, peptide, oligopeptide, polypeptide, protein complex, proteome, or subproteome of interest $p_i$ is not present within the sample.
16. If $D \leq \varepsilon$, then protein, peptide, oligopeptide, polypeptide, protein complex, proteome, or subproteome of interest $p_i$ is present within the sample at protein concentration *t*

To illustrate how *Test* 2 is carried out, this approach is applied to the general parametric equation discussed in this section.

For example, the general parametric form of the reference line (R) for protein, peptide, oligopeptide, polypeptide, protein complex, proteome, or subproteome of interest $p_i$ is

$$p_i(t) = [c_1t, c_2t, \ldots, c_nt], \forall t \geq 0$$

The vector form of the reference line is $R = \langle 0,0, \ldots ,0\rangle + t\langle c_1, c_2, \ldots , c_n\rangle$
The general equation of a point (P) on the reference line is

$$P = \langle 0 + c_1t, 0 + c_2t, \ldots ,0 + c_nt\rangle$$

Our measured sample point (S) has coordinates S = $(S_1, S_2, \ldots ,S_n)$
The vector from our measured sample point (S) to any point on the reference line (P) is P -*S*

$$P - S = \langle c_1t - S_1, \ c_2t - S_2, \ldots, c_nt - S_n\rangle.$$

For this vector to be perpendicular, the dot product (·) of this vector with the direction of the reference line $\langle c_1, c_2, \ldots , c_n\rangle$ must be 0. Therefore, we set

$$\langle c_1t \quad S_1, \ c_2t \quad S_2, \ldots, c_nt \quad S_n\rangle \cdot \langle c_1, c_2, \ldots, c_n\rangle = 0$$

$$c_1\langle c_1t - S_1\rangle + \ c_2\langle c_2t - S_2\rangle + \cdots + c_n\langle c_nt - S_n\rangle = 0$$

$$c_1{}^2t - \ c_1S_1 + \ c_2{}^2t - c_2S_2 + \cdots + c_n{}^2t - \ c_nS_n = 0$$

$$c_1{}^2t + \ c_2{}^2t + \cdots + c_n{}^2t = \ c_1S_1 + c_2S_2 + \cdots + c_nS_n$$

$$t(c_1{}^2 + c_2{}^2 + \cdots + c_n{}^2) = \ c_1S_1 + c_2S_2 + \cdots + c_nS_n$$

$$t = \frac{c_1S_1 + c_2S_2 + \cdots + c_nS_n}{c_1{}^2 + c_2{}^2 + \cdots + c_n{}^2}$$

This solution for *t* is the protein concentration of the protein, peptide, oligopeptide, polypeptide, protein complex, proteome, or subproteome of interest $p_i$ for which the distance between the sample and the reference line is shortest. Therefore, if protein, peptide, oligopeptide, polypeptide, protein complex, proteome, or subproteome of interest $p_i$ is present within the sample, then the protein, peptide, oligopeptide, polypeptide, protein complex, proteome, or subproteome of interest $p_i$ is present within the sample at protein concentration *t*.

To determine whether the protein, peptide, oligopeptide, polypeptide, protein complex, proteome, or subproteome of interest $p_i$ is present within the sample, find the point on the reference line, Q, which gives the perpendicular distance. $Q = P(t)$.

$$P = \langle 0 \mid c_1t, 0 \mid c_2t, \ldots ,0 \mid c_nt\rangle = \langle c_1t, c_2t, \cdots, c_nt\rangle$$

$$Q = \left( c_1 \left( \frac{c_1 S_1 + c_2 S_2 + \cdots + c_n S_n}{{c_1}^2 + {c_2}^2 + \cdots + {c_n}^2} \right), \quad c_2 \left( \frac{c_1 S_1 + c_2 S_2 + \cdots + c_n S_n}{{c_1}^2 + {c_2}^2 + \cdots + {c_n}^2} \right), \cdots, c_n \left( \frac{c_1 S_1 + c_2 S_2 + \cdots + c_n S_n}{{c_1}^2 + {c_2}^2 + \cdots + {c_n}^2} \right) \right)$$

*Q* is a point, which is the set of values for the reference which correspond to the solution for *t.* S is also a point. Find the distance, D, between S and *Q* using the distance formula.

For example, the Euclidean distance formula between point S and point Q is

$$distance = \sqrt{\sum_{i=1}^{n} (S_i - Q_i)^2}$$

Therefore, we have

$$D = \sqrt{\left( S_1 - c_1 \left( \frac{c_1 S_1 + c_2 S_2 + \cdots + c_n S_n}{{c_1}^2 + {c_2}^2 + \cdots + {c_n}^2} \right) \right)^2 + \left( S_2 - c_2 \left( \frac{c_1 S_1 + c_2 S_2 + \cdots + c_n S_n}{{c_1}^2 + {c_2}^2 + \cdots + {c_n}^2} \right) \right)^2 + \cdots + \left( S_n - c_n \left( \frac{c_1 S_1 + c_2 S_2 + \cdots + c_n S_n}{{c_1}^2 + {c_2}^2 + \cdots + {c_n}^2} \right) \right)^2}$$

$\varepsilon$ is the error margin, for example provided by the user.

*If* $D > \varepsilon$, then protein, peptide, oligopeptide, polypeptide, protein complex, proteome, or subproteome of interest $p_i$ is not present within the sample. Test 2 is negative.

If $D \leq \varepsilon$, then protein, peptide, oligopeptide, polypeptide, protein complex, proteome, or subproteome of interest $p_i$ is present within the sample at the protein concentration

$$t = \frac{c_1 S_1 + c_2 S_2 + \cdots + c_n S_n}{{c_1}^2 + {c_2}^2 + \cdots + {c_n}^2}$$

and Test 2 is positive. As before, the amount of the protein, peptide, oligopeptide, polypeptide, protein complex, proteome, or subproteome of interest $p_i$ is calculated by multiplying the concentration of the protein protein, peptide, oligopeptide, polypeptide, protein complex, proteome, or subproteome of interest $p_i$ by the volume of the sample.

Returning to the LYZ example, the amino acid types W and K are labelled and measured in the sample. The W amino acid type is amino acid type 1 and the K amino acid type is amino acid type 2. Instead of measuring amino acid concentrations of 60 $\mu M$ W and 60 $\mu M$ K for the sample, amino acid concentrations of 56 $\mu M$ W and 62 $\mu M$ K are measured for the sample. The user has supplied a distance threshold of 6 uM corresponding to approximately 10% of the values he measured for his sample to account for experimental error. In some embodiments, the units of the error margin (i.e. amino acid concentration, e.g. $\mu M$, or value of the label, e.g. A.U., or number of amino acids, e.g. a scalar) are the same units in which the values measured for the sample.

The reference for the protein of interest lysozyme (LYZ) is, provided by set of parametric equations 1 is:

$$p_{LYZ}(t) = [6t, 6t], \forall t \geq 0$$

As described, when amino acid concentrations are measured for the sample and the reference describes a protein, peptide, oligopeptide, polypeptide, or protein complex of interest, $c_1 = a_1$, $c_2 = a_2$, $\cdots$, $c_n = a_n$.

The vector form of the reference line is $R = (0,0, \ldots ,0) + t\langle a_1, a_2, \ldots , a_n \rangle$
For the protein of interest LYZ, this becomes $R = (0,0) + t\langle 6, 6 \rangle$
The general equation of a point (P) on the reference line is

$$P = \langle 0 + 6t, 0 + 6t \rangle$$

The measured sample point (S) has coordinates S = (56,62)
The vector from the measured sample point (S) to any point on the reference line (P) is P -*S*

$$P - S = \langle 6t - 56, \quad 6t - 62 \rangle$$

For this vector to be perpendicular, the dot product (·) of this vector with the direction of the reference line (6,6) must be 0. Therefore, we set

$$\langle 6t-56,\ 6t-62\rangle \cdot \langle 6,6\rangle = 0$$

$$36t-336+\ 36t-372=0$$

$$72t=708$$

$$t=\frac{708}{72}=9.833$$

This solution for *t* is the protein concentration of the protein of interest, LYZ, for which the distance between the sample and the reference line is shortest. Therefore, if the protein of interest LYZ is present within the sample, then LYZ is present within the sample at protein concentration *t.* The units of *t* are the same units in which the measured amino acid concentrations of the sample were provided. If the measured amino acid concentrations of the sample were provided in $\mu M$, the unit of *t* is $\mu M$. If the measured amino acid concentrations of the sample were provided in nM, the unit of *t* is *nM.* If the measured amino acid concentrations of the sample were provided in *pM,* the unit of *t* is pM.Here, the measured amino acid concentrations of the sample were provided in $\mu M$, so the unit of *t* is $\mu M$. If LYZ is present within the sample, it is present within the sample at an protein concentration of 9.833 $\mu M$.

To determine whether the protein of interest, LYZ, is present within the sample, find the point on the reference line, Q, which gives the perpendicular distance. *Q* = *P*(*t*).

$$P=(0+6t,0+6t)=(6t,6t)$$

$$Q=\left(6\left(\frac{708}{72}\right),\quad 6\left(\frac{708}{72}\right)\right)$$

*Q* is a point, which is the set of values for the reference which correspond to the solution for *t.* S is also a point. Find the distance, D, between S and *Q* using the distance formula.

For example, the Euclidean distance formula between point S and point Q is

$$distance=\sqrt{\sum_{i=1}^{n}(S_i-Q_i)^2}$$

Therefore, we have

$$D=\sqrt{\left(56-6\left(\frac{708}{72}\right)\right)^2+\left(62-6\left(\frac{708}{72}\right)\right)^2}$$

$$D=4.24$$

The user has specified an error margin, $\varepsilon$, of 6.

*If D* > $\varepsilon$, then the protein of interest LYZ is not present within the sample.
If *D* ≤ ε, then the protein of interest LYZ is present within the sample at the protein concentration *t,* 9.833 $\mu M$.
*D* ≤ ε. Therefore, LYZ is present within the sample at the protein concentration of 9.333 $\mu M$

The sample volume was 100 $\mu L$, so the protein amount of LYZ is 9.833 $\mu M \times$ 100 $\mu L$ - 0.9833 *nmol.* The presence of LYZ has been identified in the sample, and the molecular weight of LYZ is 14.3 *kDa.* Therefore, the mass of LYZ in the sample is 14.06 *ng.*

In preferred embodiments, the user's choice of error threshold is guided by the number of amino acid types

labelled and measured in the sample, and by the values measured in the sample. Due to the sum in the distance calculation $D = \sqrt{\sum_{i=1}^{n}(S_i - Q_i)^2)}$, the total distance between the sample point and the line will increase as the number of dimensions increases. Additionally, if the values measured for the sample are provided in terms of signal of the label, the values depend on the calibration factor or calibration function and can be considerably larger than the amino acid concentration values. An example calibration factor, *f,* providing the *W Signal (AU)* for a known *W Concentration* ($\mu M$) is

$$f_W = 100 \left( {AU}/_{\mu M} \right)$$

Therefore, in some embodiments, the error margin is provided from a user-inputted tolerance value, that is multiplied by the square root of the sample values squared, reflecting the distance calculation. This is provided by equation 10:

$$\varepsilon = \varphi \sqrt{S_1^2 + S_2^2 + \cdots + S_n^2}$$

wherein $\varepsilon$ is the error margin, $\varphi$ is a user-inputted tolerance value, $S_1$ is the value measured for the sample for amino acid type 1, $S_2$ is the value measured for the sample for amino acid type 1, and $S_n$ is the value measured for the sample for amino acid type n. In some embodiments, $\varphi$ is 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, or 0.10.

In alternative preferred embodiments, the user tests the presence of multiple proteins, peptides, oligopeptides, polypeptides, protein complexes, proteomes, or subproteomes of interest in the sample. For example, there is a reference database of multiple proteins, peptides, oligopeptides, polypeptides, protein complexes, proteomes, or subproteomes of interest as described in the Reference section. The user suspects the number of proteins, peptides, oligopeptides, polypeptides, protein complexes, proteomes, or subproteomes of interest present in the sample. The suspected number of proteins, peptides, oligopeptides, polypeptides, protein complexes, proteomes, or subproteomes of interest present in the sample is c.

For example, the user suspects that one protein of interest is present in the sample, so c = 1. As another example, the user suspects that two proteomes of interest are present in the sample, so c = 2. In this case, the user performs test 2 on each of the proteins, peptides, oligopeptides, polypeptides, protein complexes, proteomes, or subproteomes of interest, generating a distance, *D*, to each reference line for each protein, peptide, oligopeptide, polypeptide, protein complex, proteome or subproteome of interest. For example, if there are 50 proteins of interest, there are 50 *D* values for proteins of interest $p_{i=1:50}$. The index of each *D* value, i, describing protein of interest $p_i$ is also noted. For example, this generates a 50 x 2 matrix, with the first column populated with the *D* values for protein of interest $p_i$, ($D_{p_i}$), and the second column populated with the index of that *D* value, *i*. The matrix is then sorted by the *D* values, for example sorted by the first column in ascending order of the *D* values (with rows remaining intact, meaning the second column *i* value moves with the first column $D_{p_i}$ value). It is suspected that there are c proteins of interest, so the error threshold is defined as the $c^{th}$ largest value. This ensures that $D \leq \varepsilon$ for c proteins of interest, such that c proteins of interest are identified as present within the sample using test 2, and $D > \varepsilon$ for the remaining proteins of interest, such that more than c proteins of interest are identified as not being present within the sample using test 2.

This approach can be expressed formally as let $\beta$ be the set of D values calculated for all of the proteins, peptides, oligopeptides, polypeptides, protein complexes, proteomes, or subproteomes of interest in the reference database.

There are $\alpha$ *D* values in set $\beta$. The $c^{th}$ order statistic (in statistics) is the $c^{th}$ smallest ($c^{th}$ minimum) value of a set. The $c^{th}$ order statistic of $\beta$ is $\beta_c$. $\varepsilon = \beta_c$.

Therefore, $D \leq \varepsilon$ for c proteins, peptides, oligopeptides, polypeptides, protein complexes, proteomes, or subproteomes of interest in the reference database, and so c proteins, peptides, oligopeptides, polypeptides, protein complexes, proteomes, or subproteomes of interest are identified in the sample.

The approach when c > 1, or when more than one protein, peptide, oligopeptide, polypeptide, protein complex, proteome, or subproteome of interest is identified in the sample using test 2 and the error threshold, $\varepsilon$, is explained in the Mixture subsection later in this section.

In some embodiments, test 1 is performed. In some embodiments, test 2 is performed. In some embodiments, both test 1 and test 2 are performed. In some embodiments, test 1 is performed, and if the presence and/or concentration and/or amount of the protein, peptide, oligopeptide, polypeptide, protein complex, proteome, or subproteome of interest $p_i$ is not identified in the sample with test 1, then test 2 is performed. If test 2 is negative, because $D > \varepsilon$, then protein, peptide, oligopeptide, polypeptide, protein complex, proteome, or subproteome of interest $p_i$ is not present within the sample. If protein, peptide, oligopeptide, polypeptide, protein complex, proteome, or subproteome of interest $p_i$ is not present within

the sample, then by definition its concentration and/or amount within the sample is zero.

In some embodiments, the value (.e.g measured label, amino acid concentration and/or number of amino acids) of each labelled amino acid type of the sample is compared to the reference (i.e. known label value, amino acid concentration and/or number of amino acids) of the same two or more amino acid types for one or more proteins, peptides, oligopeptides, polypeptides, protein complexes, subproteomes, proteomes of interest, and more than one protein, peptide, oligopeptide, polypeptide, protein complex, subproteome, or proteome of interest is identified as being present within the sample because more than one protein, peptide, oligopeptide, polypeptide, protein complex, subproteome, or proteome of interest satisfies test 1 and/or test 2. The presence of more than one protein, peptide, oligopeptide, polypeptide, protein complex, subproteome, or proteome of interest is identified, so the presence of a mixture of proteins, peptides, oligopeptides, polypeptides, protein complexes, subproteomes, or proteomes of interest is identified. The concentration and/or amount of the identified mixture of proteins, peptides, oligopeptides, polypeptides, protein complexes, subproteomes, or proteomes is also identified using the perpendicular distances between the sample point and each mixture component as described in the Mixture section. This embodiment is preferred if the mixture is a simple mixture, e.g. a mixture of 5 or fewer proteins, peptides, oligopeptides, polypeptides, protein complexes, subproteomes, or proteomes of interest.

In alternative embodiments, the value (e.g. measured label, amino acid concentration and/or number of amino acids) of each labelled amino acid type of the sample is compared to the reference (.e.g known label value, amino acid concentration and/or number of amino acids) of the same two or more amino acid types for a mixture of proteins, peptides, oligopeptides, polypeptides, protein complexes, subproteomes, or proteomes of interest, and a mixture of proteins, peptides, oligopeptides, polypeptides, protein complexes, subproteomes, or proteomes of interest is identified as being present in the sample because its reference satisfies test 1 or test 2. In this embodiment, the protein concentration of the mixture of proteins, peptides, oligopeptides, polypeptides, protein complexes, subproteomes, or proteomes of interest, and a mixture of proteins, peptides, oligopeptides, polypeptides, protein complexes, subproteomes, or proteomes of interest is identified as the value of *t* that satisfies test 1 or test 2. The difference between these two embodiments is that in this embodiment, the weighted mean number of amino acids, provided by equation 11 or equation 12, is used to generate a weighted mean number of amino acids for the mixture, and this is used within set of parametric equations 2 or 4 to generate the reference for the mixture of proteins, peptides, oligopeptides, polypeptides, protein complexes, subproteomes, or proteomes of interest, and a mixture of proteins, peptides, oligopeptides, polypeptides, protein complexes, subproteomes, or proteomes of interest. This embodiment is preferred if the mixture is not a simple mixture, e.g. a mixture of more than 5 proteins, peptides, oligopeptides, polypeptides, protein complexes, subproteomes, or proteomes of interest.

In some embodiments, the total molar protein concentration of the sample is not known. Converting from a measured $A_{280}$ value to the molar protein concentration of the sample requires knowledge of a protein-specific extinction coefficient, which is determined by the protein sequence of the sample. Because the protein identity is not known, the protein sequence of the sample is not known, and the extinction coefficient of the sample is not known. Converting from a measured protein concentration of the sample measured by mass using techniques known in the art to the molar protein concentration of the sample requires knowledge of the exact molecular weight of the sample, which is not known because the protein sequence of the sample and identity of the sample are not known.

In some embodiments, when the total molar protein concentration of the sample is not known, the amino acid concentration of the amino acids of each labelled amino acid type in the sample is compared to the reference generated for the amino acid concentrations of the same (i.e. corresponding) amino acid types for the one or more proteins, peptides, oligopeptides, polypeptides, or protein complexes of interest using set of parametric equations 1. In some embodiments, when the total molar protein concentration of the sample is not known, the amino acid concentration of the amino acids of each labelled amino acid type in the sample is compared to reference generated for the amino acid concentrations of the same (i.e. corresponding) amino acid types for the one or proteomes or subproteomes of interest using set of parametric equations 2. This allows identification of a protein of unknown protein concentration, and simultaneous determination of its protein concentration. For example, the amino acid concentration of W and K amino acid types in the sample is compared to the reference provided for amino acid concentrations of W and K amino acid types for the protein of interest $n_i$. In some embodiments, a group of functions providing the amino acid concentrations of the corresponding amino acid type for the one or more proteins, peptides, oligopeptides, polypeptides, protein complexes, subproteomes, proteomes of interest, or, a mixture of proteins, peptides, polypeptides or oligopeptides, subproteomes or proteomes of interest as a function of protein concentration is provided as a reference. In some embodiments, a group of functions providing the amino acid concentrations of the corresponding amino acid type for the one or more proteins, peptides, oligopeptides, polypeptides, protein complexes, subproteomes, proteomes of interest, or, a mixture of proteins, peptides, polypeptides or oligopeptides, subproteomes or proteomes of interest as a function of concentration of each protein, peptide, oligopeptide, polypeptide, protein complex, subproteome, or proteome of interest is provided as a reference.

The amino acid concentration of the corresponding amino acid type in the one or more proteins, peptides, oligopeptides, polypeptides, protein complexes, subproteomes, proteomes of interest, or, a mixture of proteins, peptides, polypeptides or oligopeptides, subproteomes or proteomes of interest can have been measured or can be provided as a

function of protein concentration incorporating the number of amino acids of each amino acid type in the one or more proteins of interest using set of parametric equations 1 or 2. Set of parametric equations 1 or 2 have already been described in detail in the Reference section, and are reproduced here.

Set of parametric equations 1 is: $p_i(t) = [a_1t, a_2t, \ldots, a_nt], \forall t \geq 0$
Set of parametric equations 2 is: $p_i(t) = [w_1t, w_2t, \cdots, w_nt], \forall t \geq 0$

If the concentration of each labelled amino acid type in the sample is the same, or within an error margin , $\varepsilon$, to the concentration of the same amino acid types for the one or more proteins, peptides, oligopeptides, polypeptides, protein complexes, subproteomes, proteomes of interest, or, a mixture of proteins, peptides, polypeptides or oligopeptides, subproteomes or proteomes of interest provided as a function of protein concentration by set of parametric equations 1 or 2, then identification of the presence and/or concentration and/or amount of these one or more proteins, peptides, oligopeptides, polypeptides, protein complexes, proteomes of interest, or, a mixture of proteins, peptides, polypeptides, oligopeptides, subproteomes or proteomes of interest within the sample can be made. Test 1 assess whether the concentration of each labelled amino acid type in the sample is the same as the concentration of the same amino acid types for the one or more proteins, peptides, oligopeptides, polypeptides, protein complexes, subproteomes, proteomes of interest, or, a mixture of proteins, peptides, polypeptides or oligopeptides, subproteomes or proteomes of interest, and test 2 assesses whether the concentration of each labelled amino acid type in the sample is within an error margin, $\varepsilon$, to the concentration of the same amino acid types for the one or more proteins, peptides, oligopeptides, polypeptides, protein complexes, subproteomes, proteomes of interest, or, a mixture of proteins, peptides, polypeptides or oligopeptides, subproteomes or proteomes of interest. For example, the amino acid concentration of W and K in the sample is compared to the amino acid concentrations of W and K in a protein of interest provided as a function of protein concentration for the protein of interest. If the amino acid concentration of W and K in the sample is the same (test 1), or within an error margin (test 2) to the amino acid concentration of W and K in the protein of interest provided as a function of protein concentration of the protein of interest then the presence of that protein of interest in the sample is positively identified, and the concentration and/or amount of that protein of interest in the sample is the protein concentration of the protein of interest for which test 1 or test 2 was satisfied. If the amino acid concentration of W and K in the sample is outside the error margin to the amino acid concentration of W and K in the protein of interest provided as a function of protein concentration for the protein of interest, then the presence of that protein of interest in the sample is not present. Because the protein of interest is not present within the sample, by definition, the protein concentration and/or amount of that protein of interest within the sample is zero.

In some embodiments, when a proteome, or subproteome are of interest, the measured label of each labelled amino acid type, amino acid concentration of each labelled amino acid type and/or number of amino acids of each amino acid type of the sample is compared to the reference providing the known label value of the corresponding two or more amino acid types, mean amino acid concentration of the corresponding two or more amino acid types, or mean number of amino acids of the corresponding two or more amino acid types of proteome or subproteome of interest.

In some embodiments, the measured label of each labelled amino acid type, amino acid concentration of each labelled amino acid type, and/or number of amino acids of each labelled amino acid type of the sample is compared to the reference providing the label value, mean concentration of the corresponding two or more amino acid types, or mean number of amino acids of the corresponding two or more amino acid types in a mixture of proteins, peptides, polypeptides, oligopeptides, proteomes, or subproteomes of interest. This embodiment is used when the methods of the invention will not be used to determine the relative proportions of the components within the mixture.

In some embodiments, the measured label or amino acid concentration of the labelled amino acid types of the sample, are compared to the reference line providing the known label values or concentration of the same corresponding amino acid types in the one or more proteins, peptides, oligopeptides, polypeptides, protein complexes, subproteomes, proteomes of interest, or, a mixture of proteins, peptides, polypeptides, oligopeptides, subproteomes or proteomes of interest by calculating the distance between the measured label or concentration of the labelled amino acid types of the sample and the reference line providing the label value or amino acid concentration of the one or more proteins, peptides, oligopeptides, polypeptides, protein complexes, subproteomes, proteomes of interest, or, a mixture of proteins, peptides, polypeptides, oligopeptides, subproteomes or proteomes of interest as a function of the protein concentration of the one or more proteins, peptides, oligopeptides, polypeptides, protein complexes, subproteomes, proteomes of interest, or, a mixture of proteins, peptides, polypeptides, oligopeptides, subproteomes or proteomes of interest by calculating the distance between the sample point and the reference line for the one or more proteins, peptides, oligopeptides, polypeptides, protein complexes, subproteomes, proteomes of interest, or, a mixture of proteins, peptides, polypeptides, oligopeptides, subproteomes or proteomes of interest.

In some embodiments, the measured label of each labelled amino acid type in the sample is compared to the reference providing the known label value of the same (i.e. corresponding) amino acid type for the one or more proteins, peptides, oligopeptides, polypeptides, or protein complexes as a function of the protein concentration of each protein,

peptide, oligopeptide, polypeptide, or protein complex of interest, with the reference generated using set of parametric equations 3. Set of parametric equations 3 has already been described in detail in the reference section, and is reproduced here:

$$p_i(t) = [a_1 f_1 t, a_2 f_2 t, \cdots, a_n f_n t], \forall t \geq 0$$

In some embodiments, the measured label of each labelled amino acid type in the sample is compared to the reference providing the known label value of the same (i.e. corresponding) amino acid type for the one or more proteomes or subproteomes of interest as a function of the protein concentration of each proteome or subproteome of interest, with the reference generated using set of parametric equations 4. Set of parametric equations 4 has already been described in detail in the reference section, and is reproduced here:

$$p_i(t) = [w_1 f_1 t, w_2 f_2 t, \cdots, w_n f_n t], \forall t \geq 0$$

For example, the fluorescence intensity of the labelled amino acid types W and K in the sample is compared to the reference fluorescence intensity of W and K for a protein of interest as a function of the protein concentration of the protein of interest, with the reference provided using set of parametric equations 3.

In some embodiments, if the measured label of each labelled amino acid type in the sample is the same as, or within an error margin to the reference providing the known label value of the same (i.e. corresponding) amino acid types for the one or more proteins, peptides, oligopeptides, polypeptides, protein complexes, subproteomes, or proteomes of interest, or, a mixture of proteins, peptides, polypeptides, oligopeptides, subproteomes or proteomes of interest as a function of the protein concentration of the protein, peptide oligopeptide, polypeptide, protein complex, proteome, or subproteome of interest or of the mixture of proteins, peptides, oligopeptides, polypeptides, protein complexes, proteomes, or subproteomes of interest, then identification of the presence and/or concentration and/or amount of the one or more proteins, peptides, oligopeptides, polypeptides, protein complexes, subproteomes, proteomes of interest, or, a mixture of proteins, peptides, polypeptides, oligopeptides, subproteomes or proteomes of interest within the sample can be made. The protein concentration of the sample is the protein concentration of the reference for which the label values were the same, or within an error margin, to the label values of the same amino acid types measured in the sample. For example, the fluorescence intensity of the amino acid types W and C in the sample is compared to the fluorescence intensity of the amino acid types W and C provided by the reference for a protein of interest as a function of the protein concentration of the protein of interest. If the fluorescence intensity of the amino acid types W and C in the sample is the same, or within an error margin to the fluorescence intensity of the amino acid types W and C provided by the reference for the protein of interest as a function of the protein concentration of the protein of interest, then the presence and/or concentration and/or amount of that protein of interest in the sample is positively identified. The presence of the protein of interest is identified, and the protein concentration is the protein concentration of the protein of interest which provided, via the reference functions, the fluorescence intensity of the amino acid types W and C which were the same, or within an error margin, to the amino acid types W and C measured in the sample. If the fluorescence intensity of the amino acid types W and C in the sample is outside the error margin to the fluorescence intensity of the amino acid types W and C provided by the reference for the protein of interest as a function of the protein concentration of the protein of interest, then the presence and/or concentration and/or amount of that protein of interest in the sample is not present. The presence of the protein of interest is not present in the sample, and therefore there is no amount and/or concentration of that protein of interest within the sample.

The known label value (e.g. fluorescence intensity) of each corresponding amino acid type in the protein or proteome of interest can have been measured previously, be provided by the reference as a function of the protein concentration of the protein of interest with the reference generated using set of parametric equations 3 or 4, or can be actively measured as part of the method.

In some embodiments, the concentration of each labelled amino acid type in the sample can be used to determine the unknown total protein concentration of the sample when the presence of a protein, peptide, oligopeptide, polypeptide, protein complex, subproteome, or proteome of interest, or, a mixture of proteins, peptides, polypeptides, oligopeptides, subproteomes or proteomes of interest is identified; the protein concentration of the sample is the protein concentration of the protein, peptide, oligopeptide, polypeptide, protein complex, subproteome, or proteome of interest, or, a mixture of proteins, peptides, polypeptides, oligopeptides, subproteomes or proteomes of interest which satisfied test 1 or test 2

In some embodiments, the total protein concentration of the sample which has been determined by the methods of the invention disclosed herein provides the total viral protein concentration within the patient's sample. In some embodiments, the identity of the virus proteome has been simultaneously determined. The total protein concentration per single virus particle is constant and therefore the total concentration of the virus proteome within the patient's sample is a measure of the relative viral load of the virus within the patient's sample. Preferably, this is reported in molar concentration

units, such as μM. Generally, a viral load is described as the number of viral particles per mL of sample. The viral load calculated within the methods of the invention can be converted into these units by determining theoretically the total moles of protein within one virus particle. This can be used to convert the molar total protein concentration measurements to the standard measure of viral particles per mL of sample if desired. However, because this just involves dividing the value directly obtained via the methods of the invention by a scalar, both methods of calculating viral load give equivalent quantitative information which can be used in patient care and modelling.

In some embodiments, the measured label of the labelled amino acid types, amino acid concentration of the labelled amino acid types, and/or number of amino acids of each labelled amino acid type for the sample, are compared to the known label values of the same amino acid types, amino acid concentrations of the same amino acids types, or number of amino acids of the same amino acids types of the one or more proteins, peptides, oligopeptides, polypeptides, protein complexes, subproteomes, proteomes of interest, or, a mixture of proteins, peptides, polypeptides, oligopeptides, subproteomes, proteomes of interest using a n dimensional space. Chemical information provided by the reference, for example constructed with set of parametric equations 1, 2, 3, or 4, is projected into the n dimensional space for each of the n amino acid types labelled within a sample, wherein n is the number of amino acid types labelled, for ease of visualization. For example, when 2 amino acid types are labelled in the sample, there is a 2-dimensional space. When 3 amino acid types are labelled in the sample, there is 3-dimensional space. When 4 amino acid types are labelled in the sample, there is a 4-dimensional space. This provides a graphical representation of the behavior of the reference, for example provided by the parametric equations, however this step is not required for the methods of the invention to be carried out.

The n dimensional space contains the known label value or amino acid concentration of n amino acid types of the one or more proteins, peptides, oligopeptides, polypeptides, protein complexes, subproteomes, proteomes of interest, or, a mixture of proteins, peptides, polypeptides, oligopeptides, protein complexes, subproteomes or proteomes of interest as a function of the protein concentration of the protein, peptide, oligopeptide, polypeptide, protein complex, proteome, or subproteome, or the mixture of proteins, peptides, oligopeptides, polypeptides, protein complexes, proteomes, or subproteomes of interest. Alternatively, if the protein concentration of the sample is known, the n dimensional space provides the label value, amino acid concentration, or number of amino acids of the one or more proteins, peptides, oligopeptides, polypeptides, protein complexes, subproteomes, proteomes of interest, or, a mixture of proteins, peptides, polypeptides, oligopeptides, protein complexes, subproteomes or proteomes of interest at the known protein concentration of the sample. The amino acid types in the n dimensional space are the same amino acid types that have been labelled in the sample. For example, if 4 amino acid types are labelled in the sample; W, C, Y and K, then the n dimensional space contains the known label value, amino acid concentration or number of amino acids of W, C, Y and K of the one or more proteins, peptides, oligopeptides, polypeptides, protein complexes, subproteomes, proteomes of interest, or, a mixture of proteins, peptides, polypeptides, oligopeptides, subproteomes or proteomes of interest at the protein concentration of the sample if known, or provides the label value or amino acid concentration of the one or more proteins, peptides, oligopeptides, polypeptides, protein complexes, subproteomes, proteomes of interest, or, a mixture of proteins, peptides, polypeptides, oligopeptides, subproteomes or proteomes of interest as a function of the protein concentration of the protein, peptide, oligopeptide, polypeptide, protein complex, proteome, or subproteome, or the mixture of proteins, peptides, oligopeptides, polypeptides, protein complexes, proteomes, or subproteomes of interest if the protein concentration of the sample is unknown.

In some embodiments, the number of amino acids of each labelled amino acid type is used within the general parametric equation, set of parametric equations 1, 2, 3 or 4 to calculate the reference, which provides the amino acid concentrations of each amino acid type or signal of the label of each amino acid type labelled and measured in the sample for the protein, peptide, oligopeptide, polypeptide, proteome, or subproteome of interest as a function of the protein concentration of the protein, peptide, oligopeptide, polypeptide, proteome, or subproteome of interest. Within the general parametric equation, set of parametric equations 1, 2, 3 or 4, the protein concentration of the protein, peptide, oligopeptide, polypeptide, proteome, or subproteome of interest is the variable $t$. This provides a line in n dimensional space. Each parametric equation has a domain, $\forall t \geq 0$, which means that the parametric equation is defined for all values of protein concentration greater than or equal to zero. This is because it is impossible to have a protein concentration less than zero. Therefore, when multiple references are provided for multiple proteins, peptides, oligopeptides, polypeptides, protein complexes, proteomes, or subproteomes of interest, the reference lines all intersect at the origin. The slope of each reference line with respect to protein concentration along a single dimension of the n dimensional space is the number, weighted mean number, number multiplied by a calibration factor or calibration function, or weighted mean number multiplied by a calibration factor or calibration function of amino acids of that amino acid type within the protein, peptide, oligopeptide, polypeptide, proteome, or subproteome of interest as a function of the protein concentration of the protein, peptide, oligopeptide, polypeptide, proteome, or subproteome of interest.

The comparison step has been described in general, with specific examples provided. The comparison step is now described in more detail, illustrating how it is carried out in each embodiment based on the information available for the sample. The comparison step is described below when the information available for the sample is the measured label of

two or more amino acid types within the sample, and when the information for the sample is the concentration of two or more amino acid types in the sample, which has been optionally calculated from the measured label of two or more amino acid types within the sample. Each case is described with reference to a protein and proteome of interest. However, the method outlined for a protein of interest can also be applied to the identification of the presence and/or concentration and/or amount of a peptide, oligopeptide, polypeptide, or protein complex of interest. The method outlined for a proteome of interest can also be applied to the identification of the presence and/or concentration and/or amount of a subproteome of interest, or to a mixture of proteins, peptides, oligopeptides, polypeptides, protein complexes, proteomes, or subproteomes of interest when the relative proportions of each component within the mixture is known. This is followed by a separate discussion of using the methods of the invention to identify a mixture of proteins or proteomes when the relative proportions of each component of the mixture is not known, and this is determined as part of the method. The method outlined for a mixture of proteins or proteomes when the relative proportions of each component in the mixture is not known is also applicable to the identification of the presence and/or concentration and/or amount of a mixture of peptides, oligopeptides, polypeptides, protein complexes, or subproteomes of interest. Finally, two special cases are described in which some information is already known about the sample. In special case 1, the molar protein concentration of the sample is known, therefore calculating the number of amino acids of each of two labelled amino acid types of the sample is available. In special case 2, the presence of a protein, peptide, oligopeptide, polypeptide, protein complex, proteome, or subproteome of interest, or a mixture of proteins, peptides, oligopeptides, polypeptides, protein complexes, proteomes, or subproteomes of interest within the sample is known, and the methods of the invention are used to accurately determine the concentration and/or amount of the protein, peptide, oligopeptide, polypeptide, protein complex, proteome, or subproteome of interest, or a mixture of proteins, peptides, oligopeptides, polypeptides, protein complexes, proteomes, or subproteomes of interest.

*Signal of the label of two or more amino acid types, protein of interest*

When the label (e.g. signal of the label) of two or more amino acid types is measured in the sample, and this is not converted into the amino acid concentration of two or more amino acid types of the sample, set of parametric equations 3 is used when providing the reference for a protein of interest. Set of parametric equations 3 has been fully described in the Reference section, and is reproduced here:

$$p_i(t) = [a_1 f_1 t, a_2 f_2 t, \cdots, a_n f_n t], \forall t \geq 0$$

In this equation, calibration factors or calibration functions ($f_1$, $f_2$, and $f_n$) are used within the functions for each amino acid type ($a_1 f_1 t$, $a_2 f_2 t$, and $a_n f_n t$).

In some embodiments, if the calibration function, *f*, which converts between amino acid concentration and signal of the label for each amino acid type described in the Reference section is not linear, or is not linear over certain concentration ranges, for example high or low amino acid concentrations, then each of the one or more proteins, peptides, oligopeptides, polypeptides, protein complexes, subproteomes, proteomes of interest, or, a mixture of proteins, peptides, polypeptides, oligopeptides, subproteomes or proteomes of interest has a signal of the label reference curve, rather than a reference line. In this embodiment, the measured label of the labelled amino acid types of the sample, are compared to the reference curve providing the label values of the same corresponding amino acid types in the one or more proteins, peptides, oligopeptides, polypeptides, protein complexes, subproteomes, proteomes of interest, or, a mixture of proteins, peptides, polypeptides, oligopeptides, subproteomes or proteomes of interest by calculating the distance between the measured label of the labelled amino acid types of the sample and the reference curve providing the label value of the same corresponding amino acid types for the one or more proteins, peptides, oligopeptides, polypeptides, protein complexes, subproteomes, proteomes of interest, or, a mixture of proteins, peptides, polypeptides, oligopeptides, subproteomes or proteomes of interest as a function of the protein concentration of the one or more proteins, peptides, oligopeptides, polypeptides, protein complexes, subproteomes, proteomes of interest, or, a mixture of proteins, peptides, polypeptides, oligopeptides, subproteomes or proteomes of interest by calculating the distance between the sample point and the reference curve for the one or more proteins, peptides, oligopeptides, polypeptides, protein complexes, subproteomes, proteomes of interest, or, a mixture of proteins, peptides, polypeptides, oligopeptides, subproteomes or proteomes of interest. The presence of a protein, peptide, oligopeptide, polypeptide, protein complex, proteome, or subproteome of interest in the sample is identified in the sample point is on the reference curve for the protein, peptide, oligopeptide, polypeptide, protein complex, proteome, or subproteome of interest. This is assessed by setting the functions comprising the reference curve for each amino acid type equal to the corresponding signal of the label for each amino acid type labelled and measured in the sample and solving for *t,* as in test 1. If a single solution for *t* exists, then the presence of the protein, peptide, oligopeptide, polypeptide, protein complex, proteome, or subproteome of interest is identified in the sample, and the protein concentration of the protein, peptide, oligopeptide, polypeptide, protein complex, proteome, or subproteome of interest is the single solution for *t* which solves the equations. Alternatively, the minimum distance between

the sample point and the reference curve is determined (for example, as described in 10.1109/TPCG.2003.1206938), and test 2 assesses whether this minimum distance is less than the error threshold, $\varepsilon$. If the minimum distance between the sample point and the reference curve is less than the error threshold, then the protein, peptide, oligopeptide, polypeptide, protein complex, proteome, or subproteome of interest is present within the sample at the protein concentration of the point on the reference curve which provided the minimum distance.

Preferably, the calibration curve which converts between amino acid concentration and signal of the label for each amino acid type is linear. In this case, a calibration factor $f_n$ is the slope of the calibration curve for amino acid type n. For example, the calibration factor for the C amino acid type, $f_C$, is $f_C = 50\ \left({}^{AU}/_{\mu M}\right)$, and this is multiplied by the amino acid concentration of the C amino acid type in $\mu M$ to provide the signal of the label of the C amino acid type in AU.

The calibration factor for the K amino acid type, $f_K$ is $f_K = 75\ \left({}^{AU}/_{\mu M}\right)$, and this is multiplied by the amino acid concentration of the K amino acid type in $\mu M$ to provide the signal of the label of the C amino acid type in AU.

The calibration factor for the W amino acid type, $f_W$ is $f_W = 100\ \left({}^{AU}/_{\mu M}\right)$, and this is multiplied by the amino acid concentration of the W amino acid type in $\mu M$ to provide the signal of the label of the W amino acid type in AU.

In some embodiments, the calibration curve depends on the parameters chosen for the experiment, and on the concentration unit in which the amino acid concentration is reported. For example, based on the provided calibration curve for the C amino acid type was

$$C\ Signal\ (AU) = 50\ \left({}^{AU}/_{\mu M}\right) \times C\ Concentration\ (\mu M)$$

The calibration factor was the slope of the calibration curve, $f_C = 50\ \left({}^{AU}/_{\mu M}\right)$. ,If the amino acid concentration of the C amino acid type were instead reported in nM, then the calibration curve for the C amino acid type would be

$$C\ Signal\ (AU) = 50\ \left(\frac{AU}{uM}\right) \times \frac{uM}{1000\ nM} \times C\ Concentration\ (nM) =$$

$$C\ Signal\ (AU) = .05\left(\frac{AU}{nM}\right) \times\ C\ Concentration\ (nM)$$

and the calibration factor, $f_c$, which is the slope of the calibration curve, would become, $f_C = .05\left(\frac{AU}{nM}\right)$.

Preferably, the calibration curve or calibration factor of each amino acid type is provided in the same units (e.g. all amino acid types labelled and measured in the experiments have calibration factors in units of μM, or all amino acid types labelled and measured in the experiments having calibration factors in units of nM). The calibration factor for each amino acid type is used to create the reference for each protein of interest, as described in the Reference section. For example, the C, K, and W amino acid types are labelled in the sample. For the C amino acid type (amino acid type 1), the value of the label measured is 690. For the K amino acid type (amino acid type 2), the value of the label measured is 3938. For the W amino acid type, the value of the label measured is 242. A reference has been created for Lumican using set of parametric equations 3. The reference is:

$$p_{Lumican}(t) = [6 \times 50 \times t, 25 \times 75t, 1 \times 100 \times t], \forall t \geq 0$$

$$p_{Lumican}(t) = [300t, 1875t, 100t], \forall t \geq 0$$

To identify the presence and/or concentration and/or amount of a protein of interest in the sample, when the values available for the sample are the values of the label of two or more amino acid types, test 1 and/or test 2 is performed.

Test 1 involves setting each value measured for the sample ($V_1$, $V_2$, $V_n$) equal to the corresponding function provided for the reference [$a_1f_1t, a_2f_2t, ..., a_nf_nt$], and determining whether a solution for $t$ exists. Test 1 is fulfilled if for all $t \geq 0$, there exists a value of $t$ such that:

$$V_1 = a_1 f_1 t$$

$$V_2 = a_2 f_2 t$$

$$V_n = a_n f_n t$$

where the number of equations is equal to the n number of amino acid types labelled and measured in the sample. If the n equations comprising test 1 can be solved for a single value of *t*, then protein interest $p_i$ is present within the sample at protein concentration *t*, because the sample point is on the reference line. Here we have:

$$300t = 690; t = \frac{690}{300} = 2.3; 3938 \neq 1875t; 242 \neq 100t$$

Because Test 1 has failed, the sample point is not on the reference line.

The approach for *Test* 2 is:

1. Let R be the reference line for protein, peptide, oligopeptide, polypeptide, protein complex, proteome, or subproteome of interest $p_i$, and let S be the sample point to find the shortest distance from. S has coordinates $(V_1, V_2, \cdots, V_n)$.
2. Find the equation of reference line R in vector format. This is $R = \langle 0,0, \ldots ,0\rangle + t\langle a_1f_1, a_2f_2, \cdots, a_nf_n\rangle$.
3. Find the general equation of a point P on the reference line R. This is P = $(0 + a_1f_1t, 0 + a_2f_2t, \cdots,0 + a_nf_nt\rangle$
4. The vector from S to P is $P - S = (a_1f_1t - V_1, a_2f_2t - V_2, \cdots, a_nf_nt - V_n\rangle$. Find the exact location of the point P on reference line R, called Q, such that the vector from S to P is perpendicular to R. This means find the point Q on reference line R such that the vector between S and Q gives the perpendicular. This is achieved by finding the dot product (·) of the vector from S to P $\langle a_1f_1t - V_1, a_2f_2t - V_2, \cdots, a_nf_nt - V_n\rangle$ with the direction of R $(a_1f_1, a_2f_2, \cdots a_nf_n\rangle$, setting this equal to 0, and solving for *t* to provide the value of *t* which, when substituted into the general equation for a point P on R, yields the perpendicular vector. If protein, peptide, oligopeptide, polypeptide, protein complex, proteome, or subproteome of interest $p_i$ is contained within the sample, then this solution for *t* is its protein concentration.
5. Find the distance between Q and S using the distance formula, called D.
6. Evaluate whether D is less than the error margin, $\varepsilon$.
7. If $D > \varepsilon$, then protein, peptide, oligopeptide, polypeptide, protein complex, proteome, or subproteome of interest $p_i$ is not present within the sample.
8. If $D \leq \varepsilon$, then protein, peptide, oligopeptide, polypeptide, protein complex, proteome, or subproteome of interest $p_i$ is present within the sample at protein concentration *t.*

The vector form of the reference line is $R - \langle 0,0, \ldots ,0\rangle + t\langle a_1f_1, a_2f_2, \ldots , a_nf_n\rangle$

For the protein of interest Lumican, this becomes

$$R = \langle 0,0,0\rangle + t\langle 300, 1875, 100\rangle$$

The general equation of a point (*P*) on the reference line is

$$P = \langle 0 + 300t, 0 + 1875t, 0 + 100t\rangle$$

Our measured sample point (*S*) has coordinates $(V_1,V_2,V_3)$ which is (690,3938,242)
The vector from our measured sample point (*S*) to any point on the reference line (P) is *P -S*

$$P - S = \langle 300t - 690, \quad 1875t - 3938, 100t - 242\rangle$$

For this vector to be perpendicular, the dot product (·) of this vector with the direction of the reference line (300,1875,100) must be 0. Therefore, we set

$$\langle 300t - 690,\ 1875t - 3938, 100t - 242\rangle \cdot \langle 300,1875,100\rangle = 0$$

$$90000t - 207000 + 3515625t - 7383750 + 10000t - 24200 = 0$$

$$3615625t = 7614950$$

$$t = \frac{7614950}{3615625} = 2.106$$

This solution for *t* is the protein concentration of the protein of interest, Lumican, for which the distance between the sample and the reference line is shortest. Therefore, if the protein of interest Lumican is present within the sample, then LYZ is present within the sample at protein concentration *t.* The units of *t* are the same units used in the calibration factors. If the calibration factors had units of $\mu M$, the unit of *t* is $\mu M$. If the calibration factors had units of *nM,* the unit of *t* is *nM.* If the calibration factors had units of *pM,* the unit of *t* is *pM.* Here, the calibration factors had units of $\mu M$, so the unit of *t* is $\mu M$. If Lumican is present within the sample, it is present within the sample at an protein concentration of 2.106 $\mu M$.

To determine whether the protein of interest, Lumican, is present within the sample, find the point on the reference line, *Q,* which gives the perpendicular distance. *Q* = *P*(*t*). *P* = (0 + 300*t*, 0 + 1875t, 0 + *100t*⟩ = ⟨300*t*, 1875t, 100t)

$$Q = (300 \times 2.106, 1875 \times 2.106, 100 \times 2.106) = (631.837, 3948.980, 210.613)$$

*Q* is a point, which is the set of values for the reference which correspond to the solution for *t. S* is also a point. We find the distance, *D*, between *S* and *Q* using the distance formula.

For example, the Euclidean distance formula between point S and point Q is

$$distance = \sqrt{\sum_{i=1}^{n} (S_i - Q_i)^2}$$

Therefore, we have

$$D = \sqrt{(690 - 631.837)^2 + (3938 - 3948.980)^2 + (242 - 210.613)^2} = 66.997$$

The error threshold provided by equation 10 is

$$\varepsilon = \varphi\sqrt{S_1^2 + S_2^2 + \cdots + S_n^2}$$

When the value of the label of two or more amino acid types is measured for the sample, $S_1 = V_1$, $S_2 = V_2$, and $S_n = V_n$ and equation 10 becomes,

$$\varepsilon = \varphi\sqrt{V_1^2 + V_2^2 + \cdots + V_n^2}$$

The user has specified a tolerance value, $\varphi$, of 0.05

Therefore, $\varepsilon = 0.05\sqrt{690^2 + 3938^2 + 242^2} = 200.27$

If $D > \varepsilon$, then the protein of interest Lumican is not present within the sample.

If $D \leq \varepsilon$, then the protein of interest Lumican is present within the sample at the protein concentration *t,* 2.106 $\mu M$.

$D \leq \varepsilon$ because 66.997 ≤ 200.27. Therefore, Lumican is present within the sample at the protein concentration of 2.106 $\mu M$

The sample volume was 100 $\mu L$, so the protein amount of Lumican is 2.106 $\mu M \times 100$ $\mu L$ = 2.106 *nmol.* The presence of Lumican has been identified in the sample, and the molecular weight of Lumican is 36.66 *kDa.* Therefore, the mass of Lumican in the sample is 77.21 *ng.*

*Signal of Two or More Amino Acid Types, Proteome of Interest*

The same approach outlined for a protein of interest is applied for a proteome of interest. The difference is that the reference for the proteome of interest is provided using set of parametric equations 4, defined in the reference section,

rather than set of parametric equations 3. Set of parametric equations 4 is:

$$p_i(t) = [w_1 f_1 t, w_2 f_2 t, \cdots, w_n f_n t], \forall t \geq 0$$

wherein the weighted number of amino acids of each amino acid type in the proteome of interest is defined as explained in the Reference section, for example using equation 11 or equation 12. As explained above, test 1 or test 2 is applied to determine whether the sample contains the proteome of interest, and if the presence of the proteome of interest is identified in the sample, to simultaneously determine the protein concentration of the proteome of interest within the sample.

For example, a reference for the SARS-CoV-2 proteome has been provided using the described calibration factors for the W and K amino acid types within set of parametric equations 4 as

$$p_{SARS-CoV-2}(t) = [1130t, 4545t], \forall t \geq 0$$

wherein the W amino acid type is amino acid type 1 and the K amino acid type is amino acid type 2. Using set of parametric equations 4 with the same amino acid types and calibration functions, a reference for the HIV proteome has also been provided:

$$p_{HIV}(t) = [1090t, 2093t], \forall t \geq 0$$

A blood sample is taken and the viral fraction isolated. The signals of the label measured for the W and K amino acid types, respectively, are 327 AU and 837 AU, providing a point in n-dimensional space (327, 837).

Using the methods described herein, test 2 is performed. For the SARS-CoV-2 proteome of interest we have:

$$\langle 1130t - 327, 4545t - 837 \rangle \cdot \langle 1130, 4545 \rangle = 0$$

$$t = 0.191\, uM, Q = (215.8, 868.1), D = 115.5$$

For the HIV proteome of interest we have:

$$\langle 1090t - 327, 2093t - 837 \rangle \cdot \langle 1090, 2093 \rangle = 0$$

$$t = 0.378\, uM, Q = (413.6, 792.4), D = 97.41$$

The distance threshold has been set at $\varepsilon = 0.01\sqrt{327^2 + 837^2} = 8.98$

Because for both proteomes of interest, $D > \varepsilon$, both proteomes of interest are identified as not present within the patient's sample (the absence of each proteome of interest is identified within the patient's sample).

As another example, it has been reported that IL-6, IL1RN, and IL1RA remain elevated after SARS-CoV-2 infection, as was observed for MERS. A subproteome of interest, for example comprising IL-6, IL1RN, and IL1RA or experimentally determined based on nasal secretions or blood plasma for patients following SARS-CoV-2 infection can be identified and quantified using the fluorescence intensity of W and K amino acid types labelled and measured in the sample. Presence of, or elevation of, this subproteome of interest relative to controls who have not previously had SARS-CoV-2 infection can identify a subproteome signature of previous SARS-CoV-2 infection as an alternative to an antibody test.

*Amino Acid Concentration of Two or More Amino Acid Types, Protein of Interest*

As already described, the amino acid concentration of two or more amino acid types has been calculated from the value of the label of two or more amino acid types, using the inverse of the calibration curve, for each amino acid type. For example, the amino acid concentrations of the C, K, and W amino acid types were calculated using the following inverse calibration curves

$$C\ Concentration\ (\mu M) = \frac{C\ Signal\ (AU)}{50\ {}^{AU}/_{uM}}$$

$$K\ Concentration\ (\mu M) = \frac{K\ Signal\ (AU)}{75\ ^{AU}/_{uM}}$$

$$W\ Concentration\ (\mu M) = \frac{K\ Signal\ (AU)}{100\ ^{AU}/_{uM}}$$

Which provided as their slopes the following inverse calibration factors.

$$f_C^{-1}: \frac{1}{50\ ^{AU}/_{uM}}$$

$$f_K^{-1}: \frac{1}{75\ ^{AU}/_{uM}}$$

$$f_W^{-1}: \frac{1}{100\ ^{AU}/_{uM}}$$

The reference for protein of interest $p_i$ has been provided by set of parametric equations 1, $p_i(t) = [a_1 f_1 t, a_2 f_2 t, \cdots, a_n f_n t]$, $\forall t \geq 0$, as described in the reference section. Test 1 involves setting each value measured for the sample ($AAC_1$, $AAC_2$,$AAC_n$) equal to the corresponding function provided for the reference $[a_1 t, a_2 t, \cdots, a_n t]$, and determining whether a solution for $t$ exists. Test 1 is fulfilled if for all $t \geq 0$, there exists a value of $t$ such that:

$$AAC_1 = a_1 t$$

$$AAC_2 = a_2 t$$

$$AAC_n = a_n t$$

The approach for *Test* 2 is:

1. Let R be the reference line for protein, peptide, oligopeptide, polypeptide, protein complex, proteome, or subproteome of interest $p_i$, and let S be the sample point to find the shortest distance from. S has coordinates ($AAC_1$, $AAC_2$, ... $AAC_n$).
2. Find the equation of reference line R in vector format. This is $R = \langle 0,0, ... ,0\rangle + t\langle a_1, a_2, ... , a_n\rangle$.
3. Find the general equation of a point P on the reference line R. This is $P = \langle 0 + a_1 t, 0 + a_2 t, \cdots ,0 + a_n t\rangle$
4. The vector from S to P is $P - S = \langle a_1 t - AAC_1, a_2 t - AAC_2, \cdots , a_n t - AAC_n\rangle$. Find the exact location of the point P on reference line R, called Q, such that the vector from S to P is perpendicular to R. This means find the point Q on reference line R such that the vector between S and Q gives the perpendicular. This is achieved by finding the dot product ($\cdot$) of the vector from S to P $\langle a_1 t - AAC_1, a_2 t - AAC_2, \cdots , a_n t - AAC_n\rangle$ with the direction of R $\langle a_1, a_2, \cdots a_n\rangle$, setting this equal to 0, and solving for $t$ to provide the value of $t$ which, when substituted into the general equation for a point P on R, yields the perpendicular vector. If protein, peptide, oligopeptide, polypeptide, protein complex, proteome, or subproteome of interest $p_i$ is contained within the sample, then this solution for t is its protein concentration.
5. Find the distance between Q and S using the distance formula, called D.
6. Evaluate whether D is less than the error margin, $\varepsilon$.
7. If $D > \varepsilon$, then protein, peptide, oligopeptide, polypeptide, protein complex, proteome, or subproteome of interest $p_i$ is not present within the sample.
8. If $D \leq \varepsilon$, then protein, peptide, oligopeptide, polypeptide, protein complex, proteome, or subproteome of interest $p_i$ is present within the sample at protein concentration $t$.

The amino acid types C, K, and W are labelled in the sample. TCEP is added during the C labelling step, so all (unmodified + modified) C amino acids are labelled in the sample. Unmodified amino acids of the K amino acid type are labelled in the sample (amino acids of the K amino acid type which have not undergone any of the PTMs listed in Table 4).

All (unmodified + modified) W amino acids are labelled in the sample. Amino acid concentration of 37.4 μM C, 37.4 μM K, and 22.0 μM W are measured in the sample. $AAC_1 = 37.4\ \mu M, AAC_2 = 43.2\ \mu M$, and $AAC_3 = 22.0\ \mu M$.

A reference database has already been constructed as described in the Reference section, and using the Rules described in Table 4 to ensure that the reference database reflects labelling all (unmodified + modified) amino acids of the C amino acid type, unmodified (amino acids of the K amino acid type which have not undergone any of the PTMs listed in Table 4) of the K amino acid type, and all (unmodified + modified) amino acids of the W amino acid type. The results are:

| | | $a_1t$ | $a_2t$ | $a_3t$ |
|---|---|---|---|---|
| Affamin | $p_1$ | 34t | 49t | 0t |
| Talin-1 | $p_2$ | 38t | 159t | 9t |
| L-selectin | $p_3$ | 22t | 24t | 11t |
| C-reactive protein | $p_4$ | 2t | 13t | 6t |
| Lumican | $p_5$ | 6t | 25t | 1t |

$$S = (37.4, 43.2, 22)$$

Test 1 has already been described and is not applied in this example.

Applying Test 2, with $S$ = (37.4, 43.2, 22)
for $p_1$, Affamin, we have $R = \langle 0, 0, 0\rangle + t\langle 34, 49, 0\rangle$.
$P = \langle 34t, 49t, 0t\rangle$. $P - S = \langle 34t - 37.4, 49t - 43.2, 0t - 22\rangle$. The direction of R is (34,49,0).

$$\langle 34t - 37.4,\ 49t - 43.2, 0t - 22\rangle \cdot \langle 34,49,0\rangle = 0$$

$$1156t - 1271.6 + 2401t - 2116.8 + 0t - 0 = 0;\ 3557t = 3388.4; t = 0.953$$

$$Q = P(t).\ Q = \langle 34 \times 0.953, 49 \times 0.953, 0 \times 0.953\rangle = \langle 32.402, 46.697, 0\rangle$$

$$D_1 = \sqrt{(37.4 - 34.402)^2 + (43.2 - 46.697)^2 + (22 - 0)^2} = 22.477$$

Applying the described approach for $p_2$, Talin-1, we have

$$\langle 38t - 37.4,\ 159t - 43.2, 9t - 22\rangle \cdot \langle 38,159,9\rangle = 0$$

$$t = 0.3166;\ Q = P(t) = (12.031, 50.339, 2.849)$$

$$D_2 = 32.578$$

For $p_3$, L-selectin, we have $\langle 22t - 37.4, 24t - 43.2, 11t - 22\rangle \cdot \langle 22,24,11\rangle = 0$

$$t = 1.780; Q = (39.149, 42.708, 19.57); D_3 = 3.030$$

For $p_4$, C-reactive protein, $\langle 2t - 37.4, 13t - 43.2, 6t - 22) \cdot (2, 13, 6) = 0$

$$t = 3.677; Q = (7.353, 47.795, 22.059);\ D_4 = 30.396$$

For $p_5$, Lumican, $\langle 6t - 37.4, 25t - 43.2, 1t - 22\rangle \cdot \langle 6, 25, 1\rangle = 0$

$$t = 2.004; Q = (12.022, 50.091, 2.004);\ D_5 = 33.036$$

The user has not defined a tolerance value, but instead suspects that 1 protein of interest is present within the sample,

setting $c = 1$. The error margin is defined as the $c^{th}$ order statistic of $\beta$, $\varepsilon - \beta_c$.
$\beta$ is the set of $D$ values calculated for all of the proteins of interest, $p_{1-5}$.

$$\beta = [22.477, 32.578, 3.030, 30.396, 33.036].$$

The $1^{st}$ order statistic of $\beta$, $\beta_1 = 3.030$.

Therefore, $D \le \varepsilon$ for the protein of interest, $n_3$ for which $D = \beta_1$. $p_3$ is L-selectin. The protein concentration of L-selectin is the identified solution for t, $t = 3.030$ $\mu M$.

The reference for protein of interest $p_i$ has been provided by set of parametric equations 1, $p_i(t) = [a_1t, a_2t, \cdots, a_nt]$, $\forall t \ge 0$, as described in the reference section. Rather than defining the reference for all values of protein concentration greater than or equal to 0 ( $\forall t \ge 0$), the reference can alternatively be provided at specific values of protein concentration, if it is desired to compare the sample to a protein of interest only within its biologically relevant concentration range. For example, the protein concentration of proteins within blood plasma vary by over 10 orders of magnitude. The reference can be defined exclusively for values of protein concentration, *t,* over which protein of interest $p_i$ is found in blood plasma. For example, the protein concentration of Protein-S in human blood plasma has been determined to be 3.4 mg/L. The molecular weight of Protein-S is 70645 g/mol, and the molar protein concentration of Protein-S in blood plasma is 48.3 nM. If Protein-S is identified in blood plasma, then it should have a molar protein concentration of approximately 48.3 nM, for example from 10 nM to 70 nM. To achieve comparison with Protein-S exclusively within the biologically relevant concentration range, the reference for the C and W (both unmodified + modified) amino acid types of Protein-S can be provided as:

$$p_i(t) - [34t, 6t], \forall t - 0.010 \le t \le 0.070$$

Wherein the C amino acid type (unmodified+modified) is amino acid type 1 and the W amino acid type (unmodified+modified) is amino acid type 2, and the constraints on the values of $t$ are provided in $\mu M$. This constraint has an effect on the comparison step. During the comparison step, the protein concentration of the protein of interest is identified. When a constraint is applied on the reference, Test 1 and/or test 2 is performed as normal, but an additional step is performed. If a solution for the protein concentration, *t,* is found such that the amino acid concentrations for the protein of interest are equal to (test 1) or less than or equal to an error margin (test 2) to the amino acid concentrations measured for the sample, then test 3 is performed. In test 3, the identified solution for the protein concentration is compared to the provided range of protein concentration values. If the protein concentration solution is within the range of protein concentration values provided for the reference, then the presence of the protein of interest is identified in the sample at the protein concentration which provided such a solution. However, if the protein concentration solution is outside of the range of protein concentration values provided for the reference, then the presence of the protein of interest is not identified within the sample. Alternatively, this check of the calculated protein concentration against provided concentration ranges can be incorporated into test 2.

In some embodiments, when constraints are applied, set of parametric equations 1 becomes:

$$p_i(t) = [a_1t, a_2t, \cdots, a_nt], \forall t \in \varphi \le t \le \omega$$

wherein set of parametric equations 1 is defined for all $t$ contained within the set $\varphi \le t \le \omega$, with $\varphi$ the lower bound on the protein concentration $t$ and $\omega$ the upper bound on the protein concentration *t,* which can be provided using publicly available experimental data.

In Test 3, if $\varphi \le$ the solution for $t$ identified using test 1 or test 2 $\le \omega$, then protein of interest $n_i$ is present within the sample at protein concentration *t.* Although this approach is discussed in terms of amino acid concentrations and a protein of interest, this approach is applicable to all embodiments of the invention including a protein, peptide, oligopeptide, polypeptide, protein complex, subproteome, or proteome of interest, as well as values measured for the sample provided in the value of the label of two or more amino acid types or the amino acid concentrations of two or more amino acid types.

*Amino Acid Concentration of Two or More Amino Acid Types, Proteome of Interest*

The same approach is applied for the detection of a proteome of interest within a sample based on the amino acid concentration of two or more amino acid types in the sample. Set of parametric equations 2 has been defined in the Reference section and provides the amino acid concentrations of two or more amino acid types for a proteome or

subproteome of interest:

$$p_i(t) = [w_1 t, w_2 t, \cdots, w_n t], \forall t \geq 0$$

The tests described herein (test 2 or test 1) are carried out on the values provided by a group of reference functions from proteome of interest $p_i$ in order to determine the presence and/or concentration and/or amount of proteome of interest $p_i$ within the sample.

For example, reference for the HIV proteome has also been provided for its W (amino acid type 1) and K (amino acid type 2) amino acid types:

$$p_{HIV}(t) = [10.9t, 27.9t], \forall t \geq 0$$

A blood sample is taken and the viral fraction isolated. The signals of the label measured for the W and K amino acid types, respectively, are 8.7 *uM* and 19.5 *uM,* providing a point in n-dimensional space (8.7, 19.5).

Using the methods described herein, test 2 is performed. We have:

$$\langle 10.9t - 8.7, 27.9t - 19.5 \rangle \cdot \langle 10.9, 27.9 \rangle = 0$$

$$t = 0.712\ uM, Q = (7.761, 18.865), D = 1.134$$

The distance threshold has been set at $\varepsilon = 0.05\sqrt{8.7^2 + 27.9^2 =} 1.461$

Because $D \leq \varepsilon$, the HIV proteome of interest is identified as being present in the sample, at the solution for *t,* 1.134 $\mu M$.

*Special Case 1*

In special case 1, the total molar protein concentration of the sample is known, so it is possible to calculate the number of amino acids of two or more amino acid types for the sample, and provide as a reference the number of two or more amino acid types in a protein, peptide, oligopeptide, polypeptide, protein complex, subproteome, or proteome of interest. Alternatively, the amino acid concentration or value of the label of the same two or more amino acid types can be provided at the protein concentration of the sample. There is no need to calculate the minimum distance between the sample point and the reference line for each protein of interest, because the reference for each protein of interest is simply a point in *n* dimensional space, rather than a line. Therefore the distance between the sample point and the reference point for each protein of interest is calculated using the methods as described herein, the calculated distances are compared to an error margin, $\varepsilon$, which can be either the $c^{th}$ order statistic of the calculated distances if it is suspected that the mixture contains *c* proteins of interest, or provided based on the values (measured value of the label, amino acid concentration, or number of amino acids) measured for the sample such as using equation 10, $\varepsilon = \varphi\sqrt{S_1^2 + S_2^2 + \cdots + S_n^2}$. A protein of interest $p_i$ is identified within the mixture if $D_i \leq \varepsilon$

In special case 1, the molar protein concentration of the sample is known, and this is a constant *SC.* Therefore, if protein of interest $p_i$ is present within the sample, then it is present at the molar protein concentration of the sample, so $t = SC$. The general parametric equation is $p_i(t) = [c_1 t, c_2 t, \ldots c_n t]$, $\forall t \geq 0$.

In special case 1, the general parametric equation simplifies to a point in n dimensional space

$$p_i = (c_1 SC, c_2 SC, \ldots, c_n SC)$$

This is no longer a parametric equation because it is not a function of a common parameter (independent variable), because the variable t was replaced with the constant *SC.* In special case 1, the reference for protein of interest $p_i$ is provided by:

$$p_i = (c_1 SC, c_2 SC, \ldots, c_n SC)$$

This describes a point in *n* dimensional space, where *n* is the number of amino acid types labelled and measured in the sample.

In special case 1, test 1 is fulfilled if there exists a protein of interest $p_i$ such that

$$S_1 = c_1SC$$
$$S_2 = c_2SC$$
$$\cdots$$
$$S_n = c_nSC$$

where $S_1$ is the value (amino acid concentration or signal of the label) measured for amino acid type 1 in the sample, $S_2$ is the value (amino acid concentration or signal of the label) measured for amino acid type 2 in the sample, and $S_n$ is the value (amino acid concentration or signal of the label) measured for amino acid type n in the sample, In special case 1, if test 1 is fulfilled, then protein of interest $p_i$ is present within the sample. In special case 1, test 2 is fulfilled if there exists a protein of interest $p_i$ such that the distance between the sample point ($S_1$,$S_2$, ... , $S_n$) and the reference point for the protein of interest ($c_1SC$, $c_2SC$, ... , $c_nSC$) is less than the error margin, $\varepsilon$.

The distance between the sample point ($S_1$,$S_2$, ... , $S_n$) and the reference point for the protein of interest ($c_1SC$, $c_2SC$, ... , $c_nSC$) is calculated using a distance formula such as Euclidean distance. For example, the distance *D* is calculated using the values provided for $S_i$, ($S_1$,$S_2$, ... ,$S_n$) ,and $c_iSC$, ($c_1SC$, $c_2SC$, ... , $c_nSC$), using $D = \sqrt{\sum_{i=1}^{n}(S_i - c_iSC)^2}$ Exclusively within special case 1, calculating the number of amino acids within each labelled and measured amino acid type in the sample is available. This is available because the number of amino acids of each amino acid type of the sample is equal to the amino acid concentration of that amino acid type of the sample divided by the known molar protein concentration of the sample. The number of amino acids of amino acid type 1 in the sample is $N_1$, the number of amino acids of amino acid type 2 in the sample is $N_2$, and the number of amino acids of amino acid type n in the sample is $N_n$. The number of amino acids of amino acids of amino acid type 1 in the reference is $RN_1$, the number of amino acids of amino acid type 2 in the reference is $R_{N2}$, and the number of amino acids of amino acid type n in the reference is $RN_n$. In some embodiments, these numbers of amino acids are calculated from the protein sequence or protein sequences of the reference, removing portions of the sequence that are biologically cleaved in the mature protein, and subtracting the number of amino acids of each amino acid type that would not react with the label due to post-translational modification of the reactive portion of the amino acid R-group, such as conversion of a lysine primary amine to a secondary amine through alkylation, acetylation, or glycyl-lysyl isopeptide formation.

When the number of amino acids of each amino acid type in the sample is calculated within special case 1, test 1 is fulfilled when

$$N_1 = RN_1$$
$$N_2 = RN_2$$
$$\cdots$$
$$N_n = RN_n$$

Test 1 is fulfilled when the sample point is exactly equal to the reference point.

When the number of amino acids of each amino acid type in the sample is calculated within special case 1, test 2 is fulfilled when the number of amino acids of each amino acid type in the reference is within an error margin, $\varepsilon$, of the number of amino acids of each amino acid type in the sample. This is evaluated by determining the distance, *D*, between the sample point and the reference point, for example the Euclidean distance.

The formula is:

$D = \sqrt{\sum_{i=1}^{n}(N_i - RN_i)^2}$. The number of amino acids for each labelled amino acid type in the sample is calculated from the measured label and dividing the amino acid concentration by the total molar protein concentration of the sample to provide the number of each amino acid type per protein molecule in the sample as described herein.

In some embodiments, the reference is provided experimentally rather than being calculated, and the protein concentration of the reference is equal to the known protein concentration of the sample. The amino acid concentration of the sample can then be directly compared to the amino acid concentration of the reference protein or proteome of interest. In other embodiments, the reference is provided experimentally rather than being calculated, and the protein concentration of the reference is not equal to the known protein concentration of the sample. The experimental reference is provided at several known protein concentrations and the rate of change of the amino acid concentration for each amino acid type with respect to protein concentration is calculated. This is equivalent to the slope of a line in *n* dimensional space along each dimension of information, passing through the origin, and is also equivalent to $a_1$, $a_2$, and $a_n$ for each corresponding amino acid type of set of parametric equations 1 for a single protein of interest or of set of parametric equations 2 for a proteome of interest. This is used to predict the amino acid concentration that would be measured for the experimental reference protein or proteome at the known protein concentration of the sample, and the signal of the sample is compared

to that.

In another embodiment, the measured label of C and the measured label of W of the sample is measured. The number of C amino acids and the number of W amino acids for each of the proteins or proteomes of interest is transformed into the amino acid concentration of C and the amino acid concentration of W for each of the proteins or proteomes of interest at the known protein concentration of the sample, and the amino acid concentration of C and the amino acid concentration of W for each of the proteins or proteomes of interest is transformed into the known label value (e.g. signal, such as fluorescence intensity) for each of the proteins or proteomes of interest by multiplying the amino acid concentration of each amino acid type by the calibration factor for each amino acid type. This provides a point in the $n$-dimensional space, rather than a line because the protein concentration of the sample is known.

In some embodiments, the reference is provided experimentally rather than being calulcated and the protein concentration of the protein of interest is equal to the known protein concentration of the sample. The measured label (e.g. signal) of the sample can then be directly compared to the known label value (e.g. signal) of the protein of interest. In other embodiments, the reference is provided experimentally rather than being calculated, and the protein concentration of the protein of interest is not equal to the known protein concentration of the sample. The experimental reference is provided at several known protein concentrations of the protein of interest and the rate of change of the signal for each amino acid type with respect to protein concentration is calculated. This is equivalent to the slope of a line in n dimensional space along each dimension of information, passing through the origin, and provides $a_1f_1$, $a_2f_2$ and $a_nf_n$ of set of parametric equations 3 for a protein of interest and $w_1f_1$, $w_2f_2$ and $w_nf_n$ of set of parametric equations 4 for a proteome of interest. This is used to predict the signal that would be measured for the experimental reference protein or proteome of interest at the known protein concentration of the sample, and the signal of the sample is compared to that.

The transformations in both of these two additional embodiments allow you to compare like with like, such that a comparison between the unknown protein the sample and each reference protein can be made and optionally confirmed using a distance calculation.

*Special* Case 2

In special case 2, the presence of one or more proteins, peptides, oligopeptides, polypeptides, protein complexes, subproteomes, or proteome of interest within the sample is known, but the total molar protein concentration of the one or more proteins, peptides, oligopeptides, polypeptides, protein complexes, subproteomes, or proteomes of interest within the sample is unknown. Set of parametric equations 1, 2, 3, or 4 provides the reference function for the one or more proteins, peptides, oligopeptides, polypeptides, protein complexes, subproteomes, or proteomes of interest. If more than one protein, peptide, oligopeptide, polypeptide, protein complex, subproteome, or proteome of interest is known within the sample, then set of parametric equations 2 is used to provide the reference for the amino acid concentrations of the known more than one protein, peptide, oligopeptide, polypeptide, protein complex, subproteome, or proteome of interest is within the sample and set of parametric equations 4 is used to provide the reference for the value of the labels of the known more than one protein, peptide, oligopeptide, polypeptide, protein complex, subproteome, or proteome of interest is within the sample. In both cases, this can be provided using equation 6 or equation 7.

In special case 2, there is no test performed, because a hypothesis is not being tested. Therefore, test 1 or test 2 is not performed. Instead, the values measured for two or more amino acid types labelled in the sample are set equal to the corresponding values provided by the reference functions, each equation is solved for t, and the results are averaged (i.e mean).

In special case 2, the presence of protein, peptide, oliopeptide, polypeptide, subproteome, or proteome of interest $p_i$ within the sample is known, but the protein concentration of protein of interest $p_i$ within the sample is unknown. As before, values for amino acid type 1, amino acid type 2, and amino acid type n have been measured for the sample as $S_1$, $S_2$, and $S_n$.

The general parametric equation remains $p_i(t) = [c_1t, c_2t, ... c_nt]$, $\forall t \geq 0$

However, the presence of protein of interest $p_i$ is not evaluated using test 1. Instead, the known values are inputted into the equations comprising test 1, and the equations are solved for t,

$$S_1 = a_1t$$
$$S_2 = a_2t$$
$$\cdots$$
$$S_n = a_nt$$

Because the presence of protein of interest $p_i$ within the sample is known, $a_1$,$a_2$, and $a_n$ are known. $S_1$, $S_2$, and $S_n$ are known because they have been measured. Therefore, these values are inputted into the equations comprising test 1 which are simply solved for *t,* and the *n* values of *t* are averaged (i.e. mean) to provide measurement of the concentration of

protein of interest $p_1$ within the sample. Because labeling each of the n amino acid types involves orthogonal chemistry subject to different types of errors, averaging the protein concentrations obtained via these orthogonal approaches provides a highly accurate measurement of protein concentration; existing approaches only use one method of determining protein concentration such as measuring the absorbance at 280 nm (A280). The protein concentration determined in this way is multiplied by the volume of the sample to provide the amount of protein of interest $p_1$ within the sample.

The formula for calculating the protein concentration of the sample from the amino acid concentrations measured for 2 to n amino acid types (where $i = 1, 2, \cdots n$) is calculated according to equation 19:

$$Protein\ Concentration = \sum_{i=1}^{n}\left(\frac{A.A.Concentration\ of\ Amino\ Acid\ Type\ i}{\#\ of\ Amino\ Acids\ of\ Amino\ Acid\ Type\ i} \times \frac{1}{n}\right)$$

The methods of the invention can also be used to calculate the amount of protein because protein concentration is equal to the amount of protein divided by the volume of solution. The formula for calculating the protein amount of the sample from the amino acid concentrations measured for 2 to $n$ amino acid types (where i = 1, $2,\cdots n$) is calculated according to equation 20:

$$Protein\ Amount = \sum_{i=1}^{n}\left(\frac{A.A.Concentration\ of\ Amino\ Acid\ Type\ i}{\#\ of\ Amino\ Acids\ of\ Amino\ Acid\ Type\ i} \times \frac{Sample\ Volume}{n}\right)$$

wherein Sample Volume is equal to the volume of the solution in which amino acid type $n$ was labelled. In some embodiments, the protein amount calculated is the total number of moles of protein present. This can be converted to the total number of protein molecules present in the solution by multiplying by Avogadro's number, $6.022 \times 10^{23}$ molecules per mole. In some embodiments, the total mass of protein present is calculated from the protein amount by multiplying the moles of protein present by the protein molecular weight. In some embodiments, where the sample contains a proteome of interest rather than a protein of interest, the total weight of the proteome present can be calculated from the protein amount by multiplying the moles of protein present by the protein molecular weight calculated for a mean protein sequence reflecting the mean or weighted mean number of all amino acid residues (not exclusively the labelled amino acid residues) for all protein sequences within the proteome.

**Duplicates**

Rarely, a sample can be identified as a reference, but the reference can refer to more than one protein of interest. This occurs when the number of two or more amino acid types in protein sequences is the same, or when the number of two or more amino acid types in one protein sequence is a multiple of the number of two or more amino acid types in another protein sequence. If the number of two or more amino acid types within one protein of interest is the same as the number of the same two or more amino acid types within another protein of interest, then the reference lines for the proteins of interest will be the same. In these cases, a sample is identified as a reference, but the reference may refer to more than one protein of interest, for example the reference could refer to two proteins of interest.

The preferred approach for either of these cases is to eliminate a reference referring to more than one protein of interest by constraining the reference line to only protein concentrations of the protein of interest which are biologically relevant. Preferably, the issue of a reference referring to more than one protein of interest can be avoided if the reference for the protein of interest is defined exclusively within the biologically relevant concentration range for the protein of interest. It was discovered that this dramatically decreases the presence of a reference referring to more than one protein of interest, or two reference lines being indistinguishable, by constraining the reference lines in space. The effect is shown in Figure 8 for blood plasma, over which there is a 10 order of magnitude protein concentration range. The information richness of a 10 order of magnitude concentration range dramatically decreases the presence of identical or indistinguishable reference lines for a protein of interest within the human plasma proteome (3263 proteins and peptides).

This discovery was further quantified in Figure 9, where the occurrences of references referring to more than one protein of interest was quantified across the human plasma proteome for various combinations of amino acid types (C and W, K and W, K and Y, K and S, K and P, L and S, L and K, E and L, G and L, C K and W, C K and Y, L K and S, E G and K, E G and S, R E P and T, and Q L K and V. The percentage occurrences of references referring to more than one protein of interest varied considerably across the amino acid types considered, but it was discovered that when the protein concentration of the protein of interest, available as part of the methods of the invention, is compared to concentration bounds available for the protein of interest, one reference maps to one unique protein of interest (amino acid sequence / protein sequence) 100.00% of the time for every combination of two, three, and four amino acid types tested. In some

embodiments, comparing the value of the label and/or amino acid concentration of amino acids and/or number of amino acids within the two or more labelled amino acid types to the values of the label, amino acid concentrations, or number of amino acids provided for the same two or more amino acid types as were labelled in the sample, and comparing the identified protein concentration to the known protein concentration range for the protein of interest uniquely identifies and determines the concentration and/or amount of the protein of interest. Therefore, measuring the signature of two or more labelled amino acid types within a sample, and performing the comparison steps of the present invention, uniquely identifies the sample as containing any protein of interest.

This constraint has an effect on the comparison step. During the comparison step, the protein concentration of the protein of interest is identified. When a constraint is applied on the reference, test 1 and/or test 2 is performed as normal, but an additional step is performed. If a solution for the protein concentration, *t,* is found such that the amino acid concentrations for the protein of interest are equal to (test 1) or less than or equal to an error margin (test 2) to the amino acid concentrations measured for the sample, then test 3 is performed. In test 3, the identified solution for the protein concentration is compared to the provided range of protein concentration values. If the protein concentration solution is within the range of protein concentration values provided for the reference, then the presence of the protein of interest is identified in the sample at the protein concentration which provided such a solution. However, if the protein concentration solution is outside of the range of protein concentration values provided for the reference, then the presence of the protein of interest is not identified within the sample.

When constraints are applied, the general form of the parametric equation becomes:

$$p_i(t) = [c_1 t, c_2 t, \cdots, c_n t], \forall t \in \varphi \leq t \leq \omega$$

wherein the general form of the parametric equation is defined for all $t$ contained within the set $\varphi \leq t \leq \omega$, with $\varphi$ the lower bound on the protein concentration $t$ and $\omega$ the upper bound on the protein concentration $t$, which can be provided using publicly available experimental data such as the Protein Atlas.

$p_i$ is the protein, proteome, peptide, oligopeptide, polypeptide, protein complex, or subproteome of interest, $c_1$ the coefficient for amino acid type 1, $c_2$ the coefficient for amino acid type 2, and $c_n$ the coefficient for amino acid type n labelled and measured in the sample, each provided according to (explained for the example of amino acid type *n* but also applying to amino acid type 1 and 2):

- when the measurement for the sample is provided in amino acid concentration and the reference line describes a protein, peptide, oligopeptide, polypeptide, or protein complex of interest, $c_n = a_n$, where $a_n$ is the number of amino acids of amino acid type *n* in the protein, peptide, oligopeptide, polypeptide, or protein complex of interest, with $a_n$ being an integer greater than or equal to 0 ($a_n \in Z^+$)
- when the measurement for the sample is provided in amino acid concentration and the reference line describes a proteome or subproteome of interest, $c_n = w_n$, where $w_n$ is the weighted mean number of amino acids of amino acid type *n* in the proteome or subproteome of interest
- when the measurement for the sample is provided in signal of the label and the reference line describes a protein, peptide, oligopeptide, polypeptide, or protein complex of interest is $c_n = a_n f_n$, where $a_n$ is the number of amino acids of amino acid type *n* in the protein, peptide, oligopeptide, polypeptide, or protein complex of interest, with $a_n$ being an integer greater than or equal to 0 ($a_n \in Z^+$), and where $f_n$ is a calibration factor or calibration function which converts between amino acid concentration and signal of the label for amino acid type *n*
- when the measurement for the sample is the measured label (e.g. signal of the label) and the reference line describes a proteome or subproteome of interest is $c_n = w_n f_n$ where $w_n$ is the number of amino acids of amino acid type *n* in the protein, peptide, oligopeptide, polypeptide, or protein complex of interest, where $w_n$ is the weighted mean number of amino acids of amino acid type *n* in the proteome or subproteome of interest, and where $f_n$ is a calibration factor or calibration function which scales between amino acid concentration and signal of the label for amino acid type *n*.

In Test 3, if $\varphi \leq$ the solution for t identified using test 1 or test 2 $\leq \omega$, then protein of interest $p_i$ is present within the sample at protein concentration *t.*

The amount of the sample is the protein concentration of the sample multiplied by the volume of the sample.

As an alternative or complementary approach, if a reference refers to more than one protein of interest (e.g. two proteins of interest), some information is available to determine which protein of interest is present within the sample, because information is available about how the sample was obtained. For example, within the example of the 20,353-protein human proteome (before subunits are rolled up into protein complexes), the sample can have been obtained from blood plasma, from a biopsy of endometrial tissue, from a potentially cancerous skin growth, from saliva, or from urine. In cases where a sample is identified as a reference, but the reference refers to more than one protein of interest, this information is used to determine which protein of interest the reference refers to and therefore which protein of interest is

present within the sample. This process can be automated with a classifier. For example, although the human proteome contains 20,353 proteins, the proteins are expressed differently in varying tissue types and in varying disease states. Therefore, a classifier to confirm the identity and/or presence and/or concentration of one or more proteins of interest in a sample and is trained on the expression data of proteins of interest within categorical tissue types can be used to identify which protein of interest the reference refers to, based on the reference the sample has been identified as, and an inputted categorical tissue type from which the sample was obtained.

Even when mitigating steps are not applied, non-unique or indistinguishable references do not occur frequently within most clinically relevant proteomes or subproteomes of interest. Figure 10 analyses this behaviour for several clinically relevant proteomes and subproteomes. Figure 10 shows that when two amino acid types are labelled and compared, without application of any bounds or constraints on the protein concentration or other classification, all references are distinguishable and map uniquely to proteins of interest within most of the clinically relevant proteomes and subproteomes considered (SARS-CoV-2, HIV, Epstein-Barr, Glioma) and do not correspond to multiple proteins of interest within the clinically relevant proteomes and subproteomes. In some embodiments, when the methods of the invention are used to identify the presence and concentration of a protein of interest within a sample from a reference set of proteins of interest that contains all proteins present within a proteome or subproteome that contains more than 200 proteins without protein concentration constraints, 3 amino acid types are labelled. Figure 10 shows that when three amino acid types are labelled and compared, all references are distinguishable and map uniquely to proteins of interest within all the clinically relevant proteomes and subproteomes containing fewer than 4000 proteins.

In an alternative embodiment, if the number of two or more amino acid types within one protein of interest is a multiple of the number of two or more amino acid types in another protein of interest, then the reference lines will occupy the same positions within n dimensional space (i.e. they will be on top of each other), but with different parameterizations. Therefore, a sample is identified as a reference, but the reference overlaps with another reference, such that the sample could contain the protein of interest referred to by either reference. In this case, measuring the mass protein concentration of the sample (for example the mass protein concentration of the sample in mg/mL using the A280/A205 ratio and providing the $\varepsilon_{205}$ via the Scopes method, or measuring the mass protein concentration of the sample in mg/mL using the Bradford assay) can identify which of the proteins of interest which overlapping references is present in the sample. The measured protein concentration of the sample in mg/mL is converted to possible molar protein concentrations of the sample, provided on the hypothesis of the sample being each of the proteins of interest with overlapping references (because the protein of interest referred to by the reference line is known, its molecular weight is known, so the mass protein concentration of the sample can be converted to the molar protein concentration of the sample). Then, the measured amino acid concentrations for the sample are divided by the hypothetical molar protein concentration of the sample if the sample were either of the proteins of interest with overlapping reference lines. This reveals the number of amino acids of each labelled amino acid type in the sample. This is compared to the number of amino acids of each amino acid type for each protein of interest with overlapping references. The sample is identified as the protein of interest whose number of amino acids is identical to, within an error margin, $\varepsilon$, to, or closest to the number of amino acids for each of the proteins of interest with overlapping references. To identify the protein of interest whose number of amino acids is closest to the number of amino acids of the sample, the number of amino acids of each amino acid type of each protein of interest described by the reference is subtracted from the number of each amino acid type of the sample, the value is taken for each amino acid type, and the results are summed. This is the number difference between each protein of interest described by the reference and the sample. The sample is identified as the protein of interest described by the reference whose number difference is the smallest, with the protein concentration and/or amount of the sample already having been identified.

In some embodiments, the amino acid types chosen for labelling can be amino acid types which eliminate or minimize any references not being distinguishable or corresponding to more than one protein of interest within a proteome or subproteome. The information content provided by every possible combination of two, three, four, and five amino acid types has been analysed and the percentage of references which are distinguishable and uniquely correspond to proteins of interest shown in representative Figure 11. In Figure 11, the amino acid type combinations have been ranked by the percentage of unique mappings to proteins of interest and the amino acid type combinations with the highest percentage of unique mappings are shown; the size of the amino acid type combination indicates the relative percentage of unique mappings. For example, in general, for two amino acid types, the KW, CW, KC, KY, CY, WY, LS, LK, EL, GL, AL, DL, LS, LP, LV, KS, EL, AR, AE, AG, and AI amino acid type combinations eliminate or minimize any references which are not distinguishable or map to more than one protein of interest within a proteome or subproteome.

Interestingly, when the mean values are taken across a mixture such as a proteome or subproteome, multiples do not occur. Even if a proteome or subproteome contains pure proteins whose numbers of amino acids are multiples of each other, the mean number of amino acids for the entire proteome or subproteome is not a multiple of the mean number of amino acids for any other proteome or subproteome. For example, individual pure proteins within the E. Coli proteome contain numbers of amino acids which are multiples of one another, but the E. Coli proteome contains a mean number of amino acids which is distinct from any other bacterial proteome. Figure 12 shows that all of the over 7000 reference bacterial proteomes available from the UniProt database have mean numbers of amino acids which are distinct from the

mean number of amino acids of every other bacterial proteome, even when only two amino acid types are labelled. The same behaviour is found for the over 9000 viral proteomes available from the UniProt database.

Surprisingly, it has been discovered that by labelling and comparing the number of amino acids, amino acid concentrations, or signal of the label for two or more amino acid types for all proteins within a complex mixture such as a proteome or subproteome, no multiples are observed. Therefore, even if a proteome could not be identified from all of its component proteins because there are multiples in the number of amino acids observed for some component proteins, making their identification challenging, the entire proteome can be identified based on the weighted mean number of amino acids for a representative protein sequence within the proteome. This has been rationalized with a discovery that the mean number of amino acids across bacterial and viral proteomes do not follow Benford's law. This means that this set of numbers is particularly information rich, and diverse relative to other naturally occurring sets of numbers, as shown in Figures 4, 5, and 6.

It has been discovered that even for the labelling of only two amino acid types within a proteome of interest, bacterial and viral proteomes are distinct from one another, allowing for rapid identification of a bacterial or a viral infection based on labelling only two amino acid types, as shown in Figure 3.

It has been discovered that even for the labelling of only two amino acid types within a proteome of interest, bacterial and viral proteomes cluster together according to their lineage, as shown in Figure 13.

For example, for the labelling of K and W amino acid types, Corynebacteriaceae, Legionellales, Bacillales, Streptomycetaceae, and Mycoplasmataceae orders of bacteria form distinct clusters and provide non-overlapping signatures. K and W labelling can both proceed rapidly, within approximately 5 minutes. Therefore, the methods of the invention can be used for rapid diagnosis of any type of bacterial infection, and selection of the appropriate antibiotic treatment to which the bacteria would be susceptible. Traditional methods of diagnosing bacterial infections involve growing out culture plates which takes around 2-5 days, so the methods of the invention provide a method for diagnosis of bacterial infection 500 to 1500 times more rapid than the current state of the art.

## Mixtures

The following aspects are discussed in relation to a mixture of protein. However, these aspects of the invention can equally be applied to any mixture of proteins, peptides, oligopeptides, protein complexes, polypeptides, subproteomes or proteomes and any combination thereof.

In some embodiments, when the sample contains, or is suspected to contain a mixture of proteins, the number of amino acids of each labelled amino acid type is the average number of amino acids of each labelled amino acid type in all of the proteins in the mixture of proteins within the sample. Preferably, the average is the mean number of amino acids of each labelled amino acid type in all of the proteins across the mixture of proteins within the sample. In some embodiments, the mean is the weighted mean number of amino acids of each labelled amino acid type in all of the proteins across the mixture of proteins within the sample. The number of amino acids of each labelled amino acid type in each protein within the mixture or proteome is weighted by the fraction of that protein across the mixture of proteins or proteome. These weights are considered when calculating the mean number of amino acids of each labelled amino acid type in all of the proteins across the proteome or mixture of proteins within the sample, and can also be used to provide the reference for a mixture of proteins, such as a simple mixture of proteins.

In preferred embodiments, the methods of the invention have identified more than one protein, peptide, oligopeptide, polypeptide, protein complex, subproteome, or proteome of interest present within the sample. Therefore, the methods of the invention have identified a mixture of proteins, peptides, oligopeptides, polypeptides, protein complexes, subproteomes, or proteomes of interest present within the sample. The identities of the proteins, peptides, oligopeptides, polypeptides, protein complexes, subproteomes, or proteomes of interest present within the sample have already been determined via the methods of the invention, via performing test 2 and identifying a protein concentration for each protein, peptide, oligopeptide, polypeptide, protein complex, subproteome, or proteome of interest such that the perpendicular (minimum) distance between the reference line and the sample point is less than or equal to an error margin, $\varepsilon$. If the number of components within the mixture was known or suspected to be c, then the error margin can have been defined as the $c^{th}$ order statistic of the set of distances ($\beta$) measured for each protein of interest $\beta_c$. If the number of components within the mixture was not known or suspected, than the error margin can have been defined as a user-specified tolerance value multiplied by the square root of the sum of the squared values of the sample point, as was described by equation 10: $\varepsilon = \varphi\sqrt{S_1^2 + S_2^2 + \cdots + S_n^2}$

It was discovered that these distances can be further used to identify the proportional composition of the mixture.

In some embodiments, the proportion of each component in the mixture is identified. For example, if the sample contains a mixture of two proteins, the proportion of each protein in the mixture in the sample is determined. For example, it can be determined that the sample contains a mixture of Proteins A and B, wherein the mixture contains 90% protein A and 10% protein B. This is because the measurement for the sample is an average between the reference values for the pure

components (e.g. pure protein components, e.g. Protein A and Protein B) comprising the mixture. If the sample contains a mixture of two proteins; Proteins A and B, the measurement for the sample is the mean between the expected values for Protein A and Protein B. The closer the (perpendicular) distance to the reference value for a pure protein, the larger the proportion of the pure protein within the mixture, therefore the distances are inverted.

In some embodiments of the invention, the identity of the mixture is unknown, and the protein concentration of the mixture is also unknown. This means that all the following are unknown: the identities of the proteins comprising the mixture, the relative proportions of each protein within the mixture, the total protein concentration of the mixture, and the relative concentrations of each mixture component within the mixture. Identifying and quantifying the mixture involves comparing the amino acid concentrations or value of the labels measured for the unknown mixture to amino acid concentrations or value of the labels that would be measured for a series of reference proteins which could be contained within the unknown mixture. In some embodiments, the number of proteins present within the mixture is provided. In other embodiments, the number of proteins present within the mixture is not provided. Each case is described with an extended example.

*Number of Proteins Present Within the Mixture is Known or Suspected*

In some embodiments, the number of proteins present within the mixture is provided. For example, the sample is an unknown mixture of proteins. The raw signals measured for each dimension of information for the sample have been converted to amino acid concentrations. There are 3 dimensions of information because 3 types of amino acids are labelled. The expected amino acid concentrations that would be measured for protein of interest 1, protein of interest 2, protein of interest 3, protein of interest 4, protein of interest 5, and protein of interest 6, provided by set of parametric equations 1, are shown in Figure 14.

The sample point is not on the reference line for any of the proteins of interest, so test 2 is performed as described herein, and the value of t for each protein of interest is found such that the perpendicular distance between the sample point and the reference line for each protein of interest is minimized. The perpendicular distance between the sample point and each reference line at protein concentration t is computed as described herein, resulting in 6 distance values, $D_1$,$D_2$,$D_3$,$D_4$, $D_5$ and $D_6$.$\beta$ is the set of $D$ values. The number of proteins in the mixture is known or suspected to be 3, therefore the third order statistic of $\beta$ is set as the distance threshold, $D = \beta_3$.

The 3 smallest distances are $D_1$, $D_5$, and $D_3$,. Therefore, the unknown mixture is identified as comprising protein of interest 1, protein of interest 5, and protein of interest 3. It is also possible to determine the proportion of the amino acid concentration provided by protein of interest 1, protein of interest 5, and protein of interest 3 to the sample. The principle is that the measurement for the sample is the mean of the expected values for the pure components (e.g. pure proteins) comprising the mixture. The closer the distance, the larger the proportion of the pure component (e.g. pure protein) within the mixture, therefore the distances are inverted. The distances must also be normalized so that the proportion of all components within the mixture sum to 1. Therefore, to calculate the proportion of each component within the mixture:

- The distances are inverse normalized by dividing the largest distance by every other distance.
- Then the inverse normalized distances are summed.
- Finally, the fraction of each component within the mixture is calculated by dividing its inverse normalized distance by the sum of all the inverse normalized distances.

This is stated formally as, from the set of proteins of interest $p_{i=1:a}$, where $a$ is the total number of proteins of interest in the reference set, $b$ proteins of interest are identified within the mixture, so the proteins of interest identified within the mixture are $p_{i=1:b}$. The distance of each protein of interest $p_i$=$_{1:b}$ is measured as $D_{i=1:b}$. Find max($D_{i=1:b}$). For each protein of interest $p_{i=1:b}$, find $\frac{\max(D_{i=1:b})}{D_i}$. For each protein of interest $p_{i=1:b}$, the fraction of this protein of interest within the mixture is stated formally with equation 18:

$$\frac{\frac{\max(D_{i=1:b})}{D_i}}{\sum_{i=1}^{b}\frac{\max(D_{i=1:b})}{D_i}}, \forall p_{i=1:b}$$

Applying this approach to the example, the researcher has determined that $D_1$ = 14.1, $D_3$ = 32.1 and $D_5$ = 19.7. The maximum distance is 32.1.

Therefore the percentage of protein of interest 1 in the mixture based on amino acid concentrations is $\frac{32.1}{14.1}$ /(

$$\frac{32.1}{14.1} + \frac{32.1}{32.1} + \frac{32.1}{19.7}) = 0.46$$

The percentage of protein of interest 3 in the mixture based on amino acid concentrations is

$$\frac{32.1}{32.1} /( \frac{32.1}{14.1} + \frac{32.1}{32.1} + \frac{32.1}{19.7}) = 0.20$$

The percentage of protein of interest 5 in the mixture based on amino acid concentrations is

$$\frac{32.1}{19.7} /( \frac{32.1}{14.1} + \frac{32.1}{32.1} + \frac{32.1}{19.7}) = 0.33$$

In some embodiments, if the measured label (e.g. signal of the label) is provided for the sample and the reference proteins of interest, then this step is performed using the measured label (e.g. signal of the label) rather than amino acid concentrations. Because the measured label (e.g. signal of the label) is a constant multiplied by the amino acid concentration, the constants cancel in the division steps above and equivalent proportional results are obtained showing the proportion of each component within the mixture based on amino acid concentrations.

The apparent protein concentration of each pure component (e.g. pure protein) has already been identified, this is the solution for *t* for each pure component for which test 2 was satisfied such that the protein of interest was identified within the sample. However, the pure components (e.g. pure proteins) are contained within a mixture, so each identified solution for *t* is multiplied by the proportion of each component within the mixture to ascertain the protein concentration of each component within the sample. The solution of *t* for protein of interest 1 is $t_1$, the solution of *t* for protein of interest 3 is $t_3$, and the solution of *t* for protein of interest 5 is $t_5$. The protein concentration of protein of interest 1 is $0.46 \times t_1$, the protein concentration of protein of interest 3 is $0.20 \times t_3$, and the protein concentration of protein of interest 5 is $0.33 \times t_5$. The total protein concentration of the sample is then $0.46 \times t_1 + 0.20 \times t_3 + 0.33 \times t_5$.

*Number of Proteins Present Within the Mixture is Not Provided*

In other embodiments, the number of proteins present within the mixture is not known or suspected. In this embodiment, the first step is the same as described in the *Number of Proteins Present Within the Mixture is Known or Suspected* section and in Figure 14 for the case where this is known or suspected. The sample point is not on the reference line for any of the proteins of interest, so test 2 is performed as described herein, and the value of *t* for each protein of interest is found such that the distance between the sample point and the reference line for each protein of interest is minimized. The distance between the sample point and each reference line at protein concentration *t* is computed as described herein, resulting in 6 distance values, $D_1$,$D_2$,$D_3$,$D_4$,$D_5$ and $D_6$. Each distance value is compared to an error margin, $\varepsilon$. Where the number of proteins present within the mixture is not provided, $\varepsilon$ is preferably defined using the measured sample values, for example as provided in equation 10: $\varepsilon = \varphi\sqrt{S_1^2 + S_2^2 + \cdots + S_n^2}$ . In this case, the user-specified tolerance value impacts the number of for example proteins of interest identified as being present within the mixture. The presence protein of interest $p_i$ is identified within the mixture if the *D* value for this protein of interest, $D_i$, is less than or equal to the error margin ($D_i \leq \varepsilon$). When plotted in n dimensional space, $\varepsilon$ defines the radius of a sphere in *n* dimensional space. If 2 types of amino acids are labelled, then 2 dimensions of information are considered and $\varepsilon$ defines a 1-sphere (a circle). If 4 types of amino acids are labelled, then 4 dimensions of information are considered and $\varepsilon$ defines a 3-sphere. A 3-sphere is the name for a sphere in 4-dimensional Euclidean space. In alternative embodiments, an ellipse or hyperellipsoid can be provided by specifying a cutoff distance ($Ø_{i=1:n}$) for each amino acid type labelled (dimension of information); this can be useful if it is expected that one of the labelling reactions would produce a larger amount of experimental error than other labelling reactions.

In this example, $\varepsilon$ defines a sphere in 3-dimensional space (also known as a 2-sphere). $\varepsilon$ is 20. $D_1$ = 14.1, $D_2$ = 42.0, $D_3$ = 32.1, $D_4$ = 37.4, $D_5$ = 19.7 and $D_6$ = 50.0.

$D_1 < \varepsilon$, and $D_5 < \varepsilon$. Therefore, the protein of interest 1 and protein of interest 5 have been identified in the sample.

The same approach described in the *Number of Proteins Present Within the Mixture is Known or Suspected* section is then applied to determine the proportion of protein of interest 1 and protein of interest 5 within the sample. The principle is that the measurement for the sample is the mean of the expected values for the pure components (e.g. pure protein components) comprising the mixture. The closer the distance, the larger the proportion of the pure component (e.g. pure protein) within the mixture, therefore the distances are inverted. The distances must also be normalized so that the

proportion of all components within the mixture sum to 1. Therefore, to calculate the proportion of each component within the mixture:

- The distances are inverse normalized by dividing the largest distance by every other distance.
- Then the inverse normalized distances are summed.
- Finally, the fraction of each component within the mixture based on amino acid concentrations is calculated by dividing its inverse normalized distance by the sum of all the inverse normalized distances.

Which can be stated formally as:

$$\frac{\frac{\max(D_{i=1:b})}{D_i}}{\sum_{i=1}^{b}\frac{\max(D_{i=1:b})}{D_i}}, \forall p_{i=1:b}$$

Applying this approach to the example, the researcher has determined that $D_1$ = 14.1 and $D_5$ = 19.7. The maximum distance is 19.7.

Therefore the percentage of protein of interest 1 in the mixture based on amino acid concentrations is $\frac{19.7}{14.1} / (\frac{19.7}{14.1} + \frac{19.7}{19.7}) =$ 0.58

The percentage of protein of interest 5 in the mixture based on amino acid concentrations is $\frac{19.7}{19.7} / (\frac{19.7}{14.1} + \frac{19.7}{19.7}) = 0.42$

In some embodiments, if the measured label (e.g. signal of the label) is provided for the sample and the reference proteins of interest, then this step is performed using measured label (e.g. signal of the label) rather than amino acid concentrations. Because the signal of the label is a constant multiplied by the amino acid concentration, the constants cancel in the division steps above and equivalent proportional results are obtained regarding the proportion of each component within the mixture based on amino acid concentrations.

The apparent protein concentration of each pure component has already been identified, this is the solution for *t* for each pure component for which test 2 was satisfied such that the protein of interest was identified within the sample. However, the pure components are contained within a mixture, so each identified solution for t is multiplied by the proportion of each component within the mixture to ascertain the protein concentration of each component within the sample. The solution of *t* for protein of interest 1 is $t_1$ and the solution of *t* for protein of interest 5 is $t_5$.The protein concentration of protein of interest 1 is $0.58 \times t_1$ and the protein concentration of protein of interest 5 is $0.42 \times t_5$.

The total protein concentration of the sample is then $0.58 \times t_1 + 0.42 \times t_5$.

The approach for determining the relative proportion of each component within the mixture and the protein concentration of each component within the mixture is the same regardless of whether the number of proteins within the mixture is suspected or not; the difference is simply whether, as an input to the process, an error margin has been supplied based on the values measured for the sample or based on a suspected number of proteins present within the mixture.

In special case 1, the total molar protein concentration of the sample is known, so it is possible to calculate the number of amino acids of two or more amino acid types for the sample, and provide as a reference the number of two or more amino acid types in a protein, peptide, oligopeptide, polypeptide, protein complex, subproteome, or proteome of interest. Alternatively, the amino acid concentration or value of the label of the same two or more amino acid types can be provided at the protein concentration of the sample. There is no need to calculate the minimum distance between the sample point and the reference line for each protein of interest, because the reference for each protein of interest is simply a point in *n* dimensional space, rather than a line. Therefore the distance between the sample point and the reference point for each protein of interest is calculated using the methods as described herein, the calculated distances are compared to an error margin, $\varepsilon$, which can be either the $c^{th}$ order statistic of the calculated distances if it is suspected that the mixture contains *c* proteins of interest, or provided based on the values (measured value of the label, amino acid concentration, or number of amino acids) measured for the sample such as using equation 10, $\varepsilon = \varphi\sqrt{S_1^2 + S_2^2 + \cdots + S_n^2}$.

A protein of interest $p_i$ is identified within the mixture if $D_i < \varepsilon$. Then, the same approach as described above is applied to determine the relative proportion of each component within the mixture.

$$\frac{\frac{\max(D_{i=1:b})}{D_i}}{\sum_{i=1}^{b}\frac{\max(D_{i=1:b})}{D_i}}, \forall p_{i=1:b}$$

For example, a sample is suspected of containing 3 proteins but the components of the mixture and their proportions within the mixture are unknown. The amino acid types W and C are labelled in the sample, and the number of amino acids of W and C is determined. The number of amino acids of W and C in the sample is compared to the number of W and C amino acids in each protein of interest (see Figure 14).

Protein of interest 1, protein of interest 3, and protein of interest 4 fall within the error margin and therefore a positive identification of the mixture as comprising protein of interest 1, protein of interest 3, and protein of interest 4 is made. This is confirmed by formally calculating the distance (e.g. the Euclidean distance) between the sample point and the points corresponding to all the reference proteins.

The Euclidean distances are calculated for each protein of interest:

Sample to protein of interest 1: $D_1$= 2.24
Sample to protein of interest 2: $D_2$ = 5.10
Sample to protein of interest 3: $D_3$= 2.24
Sample to protein of interest 4: $D_4$ = 2.83
Sample to protein of interest 5: $D_5$ = 7.07
Sample to protein of interest 6: $D_6$ = 500

The set of all distances measured is $\beta$. Because the sample is suspected of containing 3 proteins, the 3$^{rd}$ order statistic of $\beta$ is set as the distance threshold, $D = \beta_3$ = 2.83. The mixture is confirmed as comprising protein of interest 1, protein of interest 3, and protein of interest 4 because these are the 3 smallest Euclidean distances.

The percentage of each of the components of the mixture can be determined based on comparing $D_1$, $D_3$, and $D_4$ by inverse normalising the Euclidian distance measurements, summing the inverse normalised distances and calculating the fraction of each component within the mixture by dividing its inverse normalized distance by the sum of all the inverse normalized distances.

The maximum distance of $D_1$ = 2.24, $D_3$ = 2.24 and $D_4$ = 2.83, is 2.83.

Therefore, the percentage of protein of interest 1 in the mixture is

$$\frac{2.83}{2.24} /( \frac{2.83}{2.24} + \frac{2.83}{2.24} + \frac{2.83}{2.83}) = 0.36$$

The percentage of protein of interest 3 in the mixture is

$$\frac{2.83}{2.24} /( \frac{2.83}{2.24} + \frac{2.83}{2.24} + \frac{2.83}{2.83}) = 0.36$$

The percentage of protein of interest 4 in the mixture is

$$\frac{2.83}{2.83} /( \frac{2.83}{2.24} + \frac{2.83}{2.24} + \frac{2.83}{2.83}) = 0.28$$

Therefore, the sample is confirmed to contain 36% protein 1, 36% protein 3 and 28% protein 4.

The treatment is the same regardless of whether this is a mixture of proteins, peptides, oligopeptides, polypeptides, protein complexes, subproteomes or proteomes. As described in the methods herein, the appropriate parametric Equation provides the amino acid concentrations or signals for the reference protein to match the values measured for the sample in each case.

**Classifier**

In some embodiments, the comparison step is carried out in a classifier. In some embodiments, the method further comprises confirming the identification of the presence and/or concentration and/or the amount of a protein of interest in a classifier.

In some embodiments, the positive identification of the presence and/or concentration and/or amount of a protein of interest in the sample is confirmed by inputting the measured label, concentration of each labelled amino acid type, or

number of amino acids of each labelled amino acid type of the sample into a classifier. This is an alternative to solving the reference functions analytically, as described herein. Instead, the reference lines can be discretized to provide the signals at multiple specific protein concentrations of the protein, peptide, oligopeptide, polypeptide, subproteome, or proteome of interest. For example, there are 50 proteins of interest, 3 amino acid types labelled and measured in the sample, and 100 protein concentrations for each protein of interest. Set of parametric equations 1 is used for each of 50 proteins of interest, to multiply the number of amino acids of each of the 3 amino acid types by 100 protein concentrations. The discretized reference lines can also be visualized in *n* dimensional space, if desired. In this embodiment, *t* is a 1 x 1 x 100 matrix of protein concentrations. The results are stored in a database, which has size of 50 x 3 x 100 (the matrix can also be transposed to a 3 x 50 x 100 matrix, 50 x 100 x 3 matrix, 3 x 100 x 50 matrix, 100 x 50 x 3 matrix, or 100 x 3 x 50 matrix). There are 3 indices for the 50 x 3 x 100 matrix, *i* is the index of the matrix row which is the protein of interest, *n* is the index of the amino acid type, and *k* is the index of the protein concentration, *t*. Set of parametric equations 1 populates this database by generating the amino acid concentrations measured for each protein concentration of each protein of interest, by operating for every *k* = 1: 100 and then for every *i* = 1: 50. Each protein concentration of each protein of interest provides a point in n dimensional space, here 3-dimensional space. The point corresponding to each protein of interest at each protein concentration is stored in the database, with an index of (*i,:, k).*

A machine learning classifier, such as a K-Nearest Neighbour (KNN) classifier with a K of 1, can be used to identify the single reference point (protein of interest at a specific protein concentration) which is nearest to the sample point. A KNN classifier can use a variety of distance metrics, such as a Euclidean distance function. The KNN classifier determines the reference point the sample is closest to; the protein of interest is identified as the index, *i,* of this reference point, and the protein identification is identified as the index, *k,* of this reference point. For example, the sample is closest to a reference point with indices *i* = 20, *k* = 40, then the sample is identified as the $20^{th}$ protein of interest at the $40^{th}$ protein concentration. However, this method generates larger amounts of data than the analytical solution method already described and does not provide an exact solution for the protein concentration of the protein of interest.

In some embodiments, the measured label of each labelled amino acid type in the sample, the number of amino acids of each labelled amino acid type and/or the concentration of each labelled amino acid type is inputted into a classifier to implement protein identification and quantification. Preferably, the classifier also contains the label, number of amino acids and/or concentration of the same two or more amino acid types in the one or more proteins, peptides, oligopeptides, polypeptides, protein complexes, subproteomes or proteomes of interest, or, a mixture of proteins, peptides, polypeptides, oligopeptides, subproteomes or proteomes of interest, thereby identifying the presence and/or concentration or amount of the one or more proteins, peptides, oligopeptides, polypeptides, protein complexes, subproteomes or proteomes of interest, or, a mixture of proteins, peptides, polypeptides, oligopeptides, subprotomes or proteomes of interest. The classifier compares the measured label of each labelled amino acid type in the sample, the number of amino acids of each labelled amino acid type and/or the concentration of each labelled amino acid type to the known label value, number of amino acids and/or concentration of the same two or more amino acid types in the one or more proteins, peptides, oligopeptides, polypeptides, protein complexes, subproteomes or proteomes of interest, or, a mixture of proteins, peptides, polypeptides, oligopeptides, subproteomes or proteomes of interest, thereby identifying the presence and/or amount of the one or more proteins, peptides, oligopeptides, polypeptides, protein complexes, subproteomes or proteomes of interest, or, a mixture of proteins, peptides, polypeptides, oligopeptides, subproteomes or proteomes of interest. In some embodiments, the classifier considers the experimental error encountered along each dimension of information such that dimensions with greater experimental error can be given less weight in the identification than dimensions with less experimental error. In some embodiments, 10% random noise is inserted into the classifier.

In some embodiments, the classifier is a Support Vector Machine classifier. A Support Vector Machine classifier finds a hyperplane that separates the datapoints of different classes while achieving maximum separation between the datapoints of different classes. In some embodiments, the classifier is a machine learning classifier. In some embodiments, the classifier is a K-Nearest neighbor classifier. A K-nearest neighbour classifier determines the class of a point based on its K nearest neighbours. In some embodiments, the classifier is an Ensemble classifier. An ensemble combines the results for different subspaces in a way which enhances predictions. In some embodiments, the ensemble classifier is an Ensemble Subspace K-Nearest neighbour classifier. Preferably, the classifier can incorporate Not a Number (NaN) values.

In some embodiments, the classifier has been trained on numbers of amino acids, amino acid concentrations, or signals from the known amino acid sequences of the proteins, peptides, oligopeptides, polypeptides, protein complexes, or mixture of proteins, peptides, polypeptides, oligopeptides, subproteomes, or proteomes of interest, thereby identifying the presence and/or amount of the one or more proteins, peptides, oligopeptides, polypeptides, protein complexes, subproteomes or proteomes of interest, or, a mixture of proteins, peptides, polypeptides or oligopeptides, subproteomes or proteomes of interest. In some embodiments, the classifier has been trained on a subset of discrete outputs from set of parametric equations 1, 2, 3 or 4. In some embodiments, the classifier has known values calculated from the known amino acid sequences found within UniProt. In some embodiments, the classifier has been trained on 75% of the data and the remaining 25% of the data is held out for validation.

*Methods of detecting cancer*

The methods disclosed herein can be used to identify the presence and absence of multiple types of cancer in a sample, for example; pancreatic cancer, human glioma, head and neck cancer, thyroid gland cancer, lung cancer, liver cancer, testisticular cancer, prostate cancer, stomach cancer, colon/rectal cancer, breast cancer, endometrial cancer, ovarian cancer, cervical cancer, kidney cancer, urinary and bladder cancer, human melanoma, brain cancer, endometrial cancer, leukemia, urothelial cancer and any combinations thereof. In preferred embodiments, the methods disclosed herein can be used to identify the presence and absence of one or more of: ovarian cancer, pancreatic cancer, colorectal cancer or prostate cancer; and any combination thereof. This is because different types of cancer have a unique proteomic signature based on the label values, amino acid concentrations or number of amino acids of each of two or more amino acid types. Therefore, the label value, amino acid concentration or number of amino acids of each of two or more amino acid types in a sample can be compared to the known label value, amino acid concentration or number of amino acids of the same two or more amino acid types of a known cancer to determine the presence and amount of such a cancer in the sample. For example, the methods disclosed herein can be used to determine the presence of ovarian cancer, pancreatic cancer, and colorectal cancer in blood plasma with 100% sensitivity and 100% specificity. Additionally, the methods disclosed herein can be used to determine the presence of bladder cancer, prostate cancer, and renal cancer in urine with 100% sensitivity and 100% specificity. The methods disclosed herein can additionally be used to determine the stage and location of colorectal cancer with up to 78% sensitivity for detection of the stage of colorectal cancer and up to 100% positive predictive value for determination of the location of colorectal cancer.

Where identification of a disease subproteome of interest is discussed, this also refers to identification of a disease associated proteome of interest and vice versa.

In preferred embodiments, the amount of cancer in the sample refers to the stage of cancer in the sample. The stage of cancer can be described numerically, for example stage 0, stage I, stage II, stage III, stage IV. Stage 0 describes carcinoma in situ, a precancerous condition in which abnormal cells are present. Stage I cancer describes cancer that has spread to other tissue in a small area, for example in an area less than 20 mm. Stage II cancer describes the localized spread of cancer; the cancer has spread more significantly, for example to an area between 20-50 mm with lymph node involvement or to an area greater than 50 mm with no lymph node involvement. Stage III cancer describes the regional spread of cancer; the cancer has spread to an area larger than 50 mm with diffuse lymph node involvement. Stage IV cancer describes the distant spread of cancer; the cancer has spread to at least one other organ of the body. Alternatively, the stage can be determined using the TNM staging system which provides values for Tumor, Node, and Metastasis, such as T1, T2, T3, T4, N0, N1, N2, N3, M0 or M1. As used herein, the term "early-stage" refers to stages I and II of cancer, and "late-stage" refers to stages III and IV of cancer. The term "early-stage" additionally refers to T values of 1 and 2, N values of 0 and 1, and M values of 0 within the TNM staging system. If one of the TNM values is above the cut off point provided, then this is considered late-stage cancer in the methods disclosed herein. For example, T2N1M0 is early-stage cancer. As another example, T1N0M0 is early stage cancer. As another example, T3N3M1 is late-stage cancer. As an additional example, T2N3M1 is late-stage cancer.

In preferred embodiments, the amount of cancer in a sample refers to whether the cancer is early-stage (stage I or II) or late- stage (stage III or IV).

In preferred embodiments, the amount of cancer in a sample refers to the grade of cancer. There are three grades of cancer: Grade 1, Grade 2, and Grade 3. Grade 1 cancer is comprised of cells that are well differentiated similar to normal tissues and are likely to grow and spread slowly. Grade 3 cancer is comprised of cells that are poorly differentiated, lacking normal structure and tissue patterns and are likely to grow and spread aggressively. Grade 2 cancer is an intermediate grade between Grade 1 and Grade 3 cancer. Although the grade of cancer is generally determined qualitatively, in some embodiments the amount of cancer in a sample refers to the grade of cancer that has been determined semi-quantitively or quantitatively. In the methods of the invention, Grade 1 cancer is considered early-stage cancer(https://www.cancerresearchuk.org/about-cancer/what-is-cancer/cancer-grading and https://www.cancerresearchuk.org/about-cancer/what-is-cancer/stages-of-cancer).

The methods of the invention can be used to detect the presence and absence of one or more types of cancer whenever two more more amino acid types are labeled within a patient sample, the values of the label are measured and optionally converted into the amino acid concentration of each labelled amino acid type from the measured label and/or optionally calculating the number of amino acids of each labelled amino acid type within the patient sample, and the presence and/or absence of one or more cancer subproteomes or proteomes of interest is identified in the sample by comparing the measured label and/or amino acid concentration of each labeled amino acid type in the sample to the known label values and/or amino acid concentrations of the same two or more amino acid types that have been labeled in the sample of each of the one or more healthy or cancer subproteomes or protoemes of interest at one or more concentrations, or comparing the number of amino acids of each labelled amino acid type in the sample to the known number of amino acids of the same two or more amino acid types that have been labelled in the sample in the one or more healthy or cancer subproteomes or proteomes of interest.

In some embodiments, the known label values and/or amino acid concentrations of the same two or more amino acid types that have been labelled in the sample of each of the one or more proteins, peptides, oligopeptides, polypeptides, protein complexes, subproteomes, or proteomes of interest related to cancer is determined experimentally using the methods as disclosed herein, or, the known number of amino acids of the same two or more amino acid types that have been labelled in the sample in the one or more proteins, peptides, oligopeptides, polypeptides, protein complexes, subproteomes, or proteomes of interest related to cancer is determined experimentally using the methods dislcosed herein.

In some embodiments, in the comparison step, information indicating the known label values, and/or amino acid concentrations, and/or number of amino acids of the same two or more amino acid types as the amino acid types that have been labelled in the sample which identifies the presence and/or concentration and/or amount of each of the one or more healthy or cancer proteomes or subproteomes of interest is a reference.

In some embodiments, the known label values and/or amino acid concentrations and/or number of amino acids of the same two or more amino acid types that have been labelled in the sample of each of the one or more proteins, peptides, oligopeptides, polypeptides, protein complexes, subproteomes, or proteomes of interest related to cancer is a reference, and the reference is determined experimentally using the methods disclosed herein. In some embodiments, the known label values and/or amino acid concentrations and/or number of amino acids of the same two or more amino acid types that have been labelled in the sample of each of the one or more proteins, peptides, oligopeptides, polypeptides, protein complexes, subproteomes, or proteomes of interest related to cancer is a single reference and is determined experimentally using the methods disclosed herein.

The reference can be provided as a database, or as operations performed on a database, or the reference can be provided analytically using a function, equation, set of functions, or set of equations that provides the values that would be measured for one or more healthy or cancer subproteomes or proteomes of interest at one or more total protein concentrations. In some embodiments, these functions are vector functions or sets of parametric equations such as the examples provided in vector functions 1-4 or sets of parametric equations 1-4, or any of their variants that include bounding ranges for the total protein concentrations of any cancer or healthy subproteome or proteome of interest, and that pass through various points in N-dimensional space. Alternatively, in other embodiments, these functions, equations, or sets of equations can be nonlinear equations of any form including but not limited to power law equations, exponential equations, polynomial equations, step function equations. In other embodiments, these functions equations or sets of functions or equations can describe a surface or a volume which identifies the values which are or would be measured for any cancer or healthy subproteome or proteome of interest.

The comparison step can be performed analytically with reference to these functions, equations, sets of functions, or sets of equations, for example by finding the minimum distance between the sample point or sample points and the functions, equations, sets of functions, or sets of equations which define the reference for a cancer or healthy proteome or subproteome of interest measured in one or more body fluids of interest, such as urine, saliva, or serum. The comparison step can incorporate any hypothesis test.

Alternatively or additionally, the comparison step can incorporate a machine learning approach such as a machine learning classifier as disclosed herein in order to identify the measured samples as corresponding to a cancer or healthy subproteome or proteome of interest. Any machine learning classifier, optimized or unoptimized, alone, in combination, or refined by for example via any hypothesis updating approach known in the art such as Bayesian updating or predictor corrector approach such as the Predict-Evaluate-Correct-Evaluate (PECE) can be used.

The methods of the invention disclosed herein can be used to detect the presence, stage, and location of multiple (i.e. more 2, more than 3, more than 4, more than 5, more than 6, more than 7, more than 8, more than 9 or more than 10, more than 11, more than 12, more than 13, more than 14, more than 15, more than 16, more than 17, more than 18, more than 19, more than 20, more than 21, more than 22, more than 23, more than 24, more than 25, more than 26, more than 27, more than 28, more than 29, more than 30, or, 2, 3, 4, 5, 6, 7, 8, 9 or 10, 11, 12, 13, 14, 15, 16, 17, 18, 19, 20, 21, 22, 23, 24, 25, 26, 27, 28, 29 or 30) types of cancer via measurements of two or more labeled amino acid types within patient body fluids. In preferred embodiments, the presence and/or absence and/or stage of cancer types selected from ovarian cancer, pancreatic cancer, colorectal cancer, bladder cancer, prostate cancer, renal cacner, brain cancer, glioma, head and neck cancer, thyroid gland cancer, lung cancer, liver cancer, testis cancer, stomach cancer, breast cancer, endometrial cancer, cervical cancer, kidney cancer, melanoma, leukemia, childhood leukemia, and lyphoma is determined. In preferred embodiments, the methods of the invention are used as a liquid biopsy test that detects the presence and/or absence and/or stage of two more more types of cancer from patient blood plasma or patient urine. In some embodiments, samples from multiple patient body fluids can be measured and identified according to the methods of the invention to provide a cancer diagnosis and staging.

In some embodiments, the proteome and/or subproteome of interest is from a sample of a human subject having ovarian cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma

(PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human ovarian cancer proteome and/or subproteome of interest, each of these different proteomic and/or subproteomic sample types can be measured for a given patient, and the presence or absence of the ovarian cancer proteome and/or subproteome of interest can be identified based on comparison of the measured label values, amino acid concentrations, or numbers of amino acids to the reference label values, amino acid concentrations, or numbers of amino acids for the ovarian cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having pancreatic cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human pancreatic cancer proteome and/or subproteome of interest, each of these different proteomic and/or subproteomic sample types can be measured for a given patient, and the presence or absence of the pancreatic cancer proteome and/or subproteome of interest can be identified based on comparison of the measured label values, amino acid concentrations, or numbers of amino acids to the reference label values, amino acid concentrations, or numbers of amino acids for the pancreatic cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having colorectal cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human colorectal cancer proteome and/or subproteome of interest, each of these different proteomic and/or subproteomic

sample types can be measured for a given patient, and the presence or absence of the colorectal cancer proteome and/or subproteome of interest can be identified based on comparison of the measured label values, amino acid concentrations, or numbers of amino acids to the reference label values, amino acid concentrations, or numbers of amino acids for the colorectal cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of healthy human subject. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human healthy proteome and/or subproteome of interest, each of these different proteomic and/or subproteomic sample types can be measured for a given patient, and the presence or absence of the healthy proteome and/or subproteome of interest can be identified based on comparison of the measured label values, amino acid concentrations, or numbers of amino acids to the reference label values, amino acid concentrations, or numbers of amino acids for the healthy proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having prostate cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human prostate cancer proteome and/or subproteome of interest, each of these different proteomic and/or subproteomic sample types can be measured for a given patient, and the presence or absence of the prostate cancer proteome and/or subproteome of interest can be identified based on comparison of the measured label values, amino acid concentrations, or numbers of amino acids to the reference label values, amino acid concentrations, or numbers of amino acids for the prostate cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having bladder cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some

embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human bladder cancer proteome and/or subproteome of interest, each of these different proteomic and/or subproteomic sample types can be measured for a given patient, and the presence or absence of the bladder cancer proteome and/or subproteome of interest can be identified based on comparison of the measured label values, amino acid concentrations, or numbers of amino acids to the reference label values, amino acid concentrations, or numbers of amino acids for the bladder cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having renal cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human renal cancer proteome and/or subproteome of interest, each of these different proteomic and/or subproteomic sample types can be measured for a given patient, and the presence or absence of the renal cancer proteome and/or subproteome of interest can be identified based on comparison of the measured label values, amino acid concentrations, or numbers of amino acids to the reference label values, amino acid concentrations, or numbers of amino acids for the renal cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having leukemia. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human leukemia proteome and/or subproteome of interest, each of these different proteomic and/or subproteomic sample types can be measured for a given patient, and the presence or absence of the leukemia proteome and/or subproteome of

interest can be identified based on comparison of the measured label values, amino acid concentrations, or numbers of amino acids to the reference label values, amino acid concentrations, or numbers of amino acids for the leukemia proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having melanoma. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human melanoma proteome and/or subproteome of interest, each of these different proteomic and/or subproteomic sample types can be measured for a given patient, and the presence or absence of the melanoma proteome and/or subproteome of interest can be identified based on comparison of the measured label values, amino acid concentrations, or numbers of amino acids to the reference label values, amino acid concentrations, or numbers of amino acids for the melanoma proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having glioma. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human glioma proteome and/or subproteome of interest, each of these different proteomic and/or subproteomic sample types can be measured for a given patient, and the presence or absence of the glioma proteome and/or subproteome of interest can be identified based on comparison of the measured label values, amino acid concentrations, or numbers of amino acids to the reference label values, amino acid concentrations, or numbers of amino acids for the glioma proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having lymphoma. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some

embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human lymphoma proteome and/or subproteome of interest, each of these different proteomic and/or subproteomic sample types can be measured for a given patient, and the presence or absence of the lymphoma proteome and/or subproteome of interest can be identified based on comparison of the measured label values, amino acid concentrations, or numbers of amino acids to the reference label values, amino acid concentrations, or numbers of amino acids for the lymphoma proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having stomach cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human stomach cancer proteome and/or subproteome of interest, each of these different proteomic and/or subproteomic sample types can be measured for a given patient, and the presence or absence of the stomach cancer proteome and/or subproteome of interest can be identified based on comparison of the measured label values, amino acid concentrations, or numbers of amino acids to the reference label values, amino acid concentrations, or numbers of amino acids for the stomach cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having lung cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human lung cancer proteome and/or subproteome of interest, each of these different proteomic and/or subproteomic sample types can be measured for a given patient, and the presence or absence of the lung cancer proteome and/or subproteome of interest can be identified based on comparison of the measured label values, amino acid concentrations, or numbers of amino

acids to the reference label values, amino acid concentrations, or numbers of amino acids for the lung cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having brain cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human brain cancer proteome and/or subproteome of interest, each of these different proteomic and/or subproteomic sample types can be measured for a given patient, and the presence or absence of the brain cancer proteome and/or subproteome of interest can be identified based on comparison of the measured label values, amino acid concentrations, or numbers of amino acids to the reference label values, amino acid concentrations, or numbers of amino acids for the brain cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having breast cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human breast cancer proteome and/or subproteome of interest, each of these different proteomic and/or subproteomic sample types can be measured for a given patient, and the presence or absence of the breast cancer proteome and/or subproteome of interest can be identified based on comparison of the measured label values, amino acid concentrations, or numbers of amino acids to the reference label values, amino acid concentrations, or numbers of amino acids for the breast cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having head and neck cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be

utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human head and neck cancer proteome and/or subproteome of interest, each of these different proteomic and/or subproteomic sample types can be measured for a given patient, and the presence or absence of the head and neck cancer proteome and/or subproteome of interest can be identified based on comparison of the measured label values, amino acid concentrations, or numbers of amino acids to the reference label values, amino acid concentrations, or numbers of amino acids for the head and neck cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having thyroid cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human thyroid cancer proteome and/or subproteome of interest, each of these different proteomic and/or subproteomic sample types can be measured for a given patient, and the presence or absence of the thyroid cancer proteome and/or subproteome of interest can be identified based on comparison of the measured label values, amino acid concentrations, or numbers of amino acids to the reference label values, amino acid concentrations, or numbers of amino acids for the thyroid cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having endometrial cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human endometrial cancer proteome and/or subproteome of interest, each of these different proteomic and/or subproteomic sample types can be measured for a given patient, and the presence or absence of the endometrial cancer proteome and/or subproteome of interest can be identified based on comparison of the measured label values, amino acid concentrations, or numbers of amino acids to the reference label values, amino acid concentrations, or numbers of

amino acids for the endometrial cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having urothelial cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human urothelial cancer proteome and/or subproteome of interest, each of these different proteomic and/or subproteomic sample types can be measured for a given patient, and the presence or absence of the urothelial cancer proteome and/or subproteome of interest can be identified based on comparison of the measured label values, amino acid concentrations, or numbers of amino acids to the reference label values, amino acid concentrations, or numbers of amino acids for the urothelial cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage ovarian cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human early-stage ovarian cancer proteome and/or subproteome of interest, each of these different proteomic and/or subproteomic sample types can be measured for a given patient, and the presence or absence of the early-stage ovarian cancer proteome and/or subproteome of interest can be identified based on comparison of the measured label values, amino acid concentrations, or numbers of amino acids to the reference label values, amino acid concentrations, or numbers of amino acids for the early-stage ovarian cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage pancreatic cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome

thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human early-stage pancreatic cancer proteome and/or subproteome of interest, each of these different proteomic and/or subproteomic sample types can be measured for a given patient, and the presence or absence of the early-stage pancreatic cancer proteome and/or subproteome of interest can be identified based on comparison of the measured label values, amino acid concentrations, or numbers of amino acids to the reference label values, amino acid concentrations, or numbers of amino acids for the early-stage pancreatic cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage colorectal cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human early-stage colorectal cancer proteome and/or subproteome of interest, each of these different proteomic and/or subproteomic sample types can be measured for a given patient, and the presence or absence of the early-stage colorectal cancer proteome and/or subproteome of interest can be identified based on comparison of the measured label values, amino acid concentrations, or numbers of amino acids to the reference label values, amino acid concentrations, or numbers of amino acids for the early-stage colorectal cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage prostate cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human early-stage prostate cancer proteome and/or subproteome of interest, each of these different proteomic and/or subproteomic sample types can be measured for a given patient, and the presence or absence of the early-stage prostate cancer proteome and/or subproteome of interest can be identified based on comparison of the measured label values, amino acid concentrations, or numbers of amino acids to the reference label values, amino acid concentrations, or numbers of amino acids for the early-stage prostate cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage bladder cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human early-stage bladder cancer proteome and/or subproteome of interest, each of these different proteomic and/or subproteomic sample types can be measured for a given patient, and the presence or absence of the early-stage bladder cancer proteome and/or subproteome of interest can be identified based on comparison of the measured label values, amino acid concentrations, or numbers of amino acids to the reference label values, amino acid concentrations, or numbers of amino acids for the early-stage bladder cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage renal cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human early-stage renal cancer proteome and/or subproteome of interest, each of these different proteomic and/or subproteomic sample types can be measured for a given patient, and the presence or absence of the early-stage renal cancer proteome and/or subproteome of interest can be identified based on comparison of the measured label values, amino acid concentrations, or numbers of amino acids to the reference label values, amino acid concentrations, or numbers of amino acids for the early-stage renal cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage leukemia. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or

subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human early-stage leukemia proteome and/or subproteome of interest, each of these different proteomic and/or subproteomic sample types can be measured for a given patient, and the presence or absence of the early-stage leukemia proteome and/or subproteome of interest can be identified based on comparison of the measured label values, amino acid concentrations, or numbers of amino acids to the reference label values, amino acid concentrations, or numbers of amino acids for the early-stage leukemia proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage melanoma. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human early-stage melanoma proteome and/or subproteome of interest, each of these different proteomic and/or subproteomic sample types can be measured for a given patient, and the presence or absence of the early-stage melanoma proteome and/or subproteome of interest can be identified based on comparison of the measured label values, amino acid concentrations, or numbers of amino acids to the reference label values, amino acid concentrations, or numbers of amino acids for the early-stage melanoma proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage glioma. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human early-stage glioma proteome and/or subproteome of interest, each of these different proteomic and/or subproteomic sample types can be measured for a given patient, and the presence or absence of the early-stage glioma proteome and/or subproteome of interest can be identified based on comparison of the measured label values, amino acid concentrations, or numbers of amino acids to the reference label values, amino acid concentrations, or numbers of amino acids for the early-stage glioma proteome and/or subproteome of interest. In some embodiments, the sample utilised is human cervical mucus or human menses, and the human cervical mucus proteome or subproteome thereof, or

human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage lymphoma. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human early-stage lymphoma proteome and/or subproteome of interest, each of these different proteomic and/or subproteomic sample types can be measured for a given patient, and the presence or absence of the early-stage lymphoma proteome and/or subproteome of interest can be identified based on comparison of the measured label values, amino acid concentrations, or numbers of amino acids to the reference label values, amino acid concentrations, or numbers of amino acids for the early-stage lymphoma proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage stomach cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human early-stage stomach cancer proteome and/or subproteome of interest, each of these different proteomic and/or subproteomic sample types can be measured for a given patient, and the presence or absence of the early-stage stomach cancer proteome and/or subproteome of interest can be identified based on comparison of the measured label values, amino acid concentrations, or numbers of amino acids to the reference label values, amino acid concentrations, or numbers of amino acids for the early-stage stomach cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage lung cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the

human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human early-stage lung cancer proteome and/or subproteome of interest, each of these different proteomic and/or subproteomic sample types can be measured for a given patient, and the presence or absence of the early-stage lung cancer proteome and/or subproteome of interest can be identified based on comparison of the measured label values, amino acid concentrations, or numbers of amino acids to the reference label values, amino acid concentrations, or numbers of amino acids for the early-stage lung cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage brain cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human early-stage brain cancer proteome and/or subproteome of interest, each of these different proteomic and/or subproteomic sample types can be measured for a given patient, and the presence or absence of the early-stage brain cancer proteome and/or subproteome of interest can be identified based on comparison of the measured label values, amino acid concentrations, or numbers of amino acids to the reference label values, amino acid concentrations, or numbers of amino acids for the early-stage brain cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage breast cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human early-stage breast cancer proteome and/or subproteome of interest, each of these different proteomic and/or subproteomic sample types can be measured for a given patient, and the presence or absence of the early-stage breast cancer proteome and/or subproteome of interest can be identified based on comparison of the measured label values, amino acid concentrations, or numbers of amino acids to the reference label values, amino acid concentrations, or numbers of amino acids for the early-stage breast cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human

amniotic fluid, and the human amniotic fluid proteome or subproteome can be utilised.

In some embodiments, the proteome and/or subproteome of interest is from a sample of a human subject having early-stage head and neck cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human early-stage head and neck cancer proteome and/or subproteome of interest, each of these different proteomic and/or subproteomic sample types can be measured for a given patient, and the presence or absence of the early-stage head and neck cancer proteome and/or subproteome of interest can be identified based on comparison of the measured label values, amino acid concentrations, or numbers of amino acids to the reference label values, amino acid concentrations, or numbers of amino acids for the early-stage head and neck cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage thyroid cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human early-stage thyroid cancer proteome and/or subproteome of interest, each of these different proteomic and/or subproteomic sample types can be measured for a given patient, and the presence or absence of the early-stage thyroid cancer proteome and/or subproteome of interest can be identified based on comparison of the measured label values, amino acid concentrations, or numbers of amino acids to the reference label values, amino acid concentrations, or numbers of amino acids for the early-stage thyroid cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage endometrial cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the

human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human early-stage endometrial cancer proteome and/or subproteome of interest, each of these different proteomic and/or subproteomic sample types can be measured for a given patient, and the presence or absence of the early-stage endometrial cancer proteome and/or subproteome of interest can be identified based on comparison of the measured label values, amino acid concentrations, or numbers of amino acids to the reference label values, amino acid concentrations, or numbers of amino acids for the early-stage endometrial cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having early-stage urothelial cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human early-stage urothelial cancer proteome and/or subproteome of interest, each of these different proteomic and/or subproteomic sample types can be measured for a given patient, and the presence or absence of the early-stage urothelial cancer proteome and/or subproteome of interest can be identified based on comparison of the measured label values, amino acid concentrations, or numbers of amino acids to the reference label values, amino acid concentrations, or numbers of amino acids for the early-stage urothelial cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage ovarian cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human late-stage ovarian cancer proteome and/or subproteome of interest, each of these different proteomic and/or subproteomic sample types can be measured for a given patient, and the presence or absence of the late-stage ovarian cancer proteome and/or subproteome of interest can be identified based on comparison of the measured label values, amino acid concentrations, or numbers of amino acids to the reference label values, amino acid concentrations, or numbers of amino acids for the late-stage ovarian cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the

sample utilised is human amniotic fluid, and the human amniotic fluid proteome or subproteome can be utilised.

In some embodiments, the proteome and/or subproteome of interest is from a sample of a human subject having late-stage pancreatic cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human late-stage pancreatic cancer proteome and/or subproteome of interest, each of these different proteomic and/or subproteomic sample types can be measured for a given patient, and the presence or absence of the late-stage pancreatic cancer proteome and/or subproteome of interest can be identified based on comparison of the measured label values, amino acid concentrations, or numbers of amino acids to the reference label values, amino acid concentrations, or numbers of amino acids for the late-stage pancreatic cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage colorectal cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human late-stage colorectal cancer proteome and/or subproteome of interest, each of these different proteomic and/or subproteomic sample types can be measured for a given patient, and the presence or absence of the late-stage colorectal cancer proteome and/or subproteome of interest can be identified based on comparison of the measured label values, amino acid concentrations, or numbers of amino acids to the reference label values, amino acid concentrations, or numbers of amino acids for the late-stage colorectal cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage prostate cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is

human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human late-stage prostate cancer proteome and/or subproteome of interest, each of these different proteomic and/or subproteomic sample types can be measured for a given patient, and the presence or absence of the late-stage prostate cancer proteome and/or subproteome of interest can be identified based on comparison of the measured label values, amino acid concentrations, or numbers of amino acids to the reference label values, amino acid concentrations, or numbers of amino acids for the late-stage prostate cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage bladder cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human late-stage bladder cancer proteome and/or subproteome of interest, each of these different proteomic and/or subproteomic sample types can be measured for a given patient, and the presence or absence of the late-stage bladder cancer proteome and/or subproteome of interest can be identified based on comparison of the measured label values, amino acid concentrations, or numbers of amino acids to the reference label values, amino acid concentrations, or numbers of amino acids for the late-stage bladder cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage renal cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human late-stage renal cancer proteome and/or subproteome of interest, each of these different proteomic and/or subproteomic sample types can be measured for a given patient, and the presence or absence of the late-stage renal cancer proteome and/or subproteome of interest can be identified based on comparison of the measured label values, amino acid concentrations, or numbers of amino acids to the reference label values, amino acid concentrations, or numbers of amino acids for the late-stage renal cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage leukemia. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human late-stage leukemia proteome and/or subproteome of interest, each of these different proteomic and/or subproteomic sample types can be measured for a given patient, and the presence or absence of the late-stage leukemia proteome and/or subproteome of interest can be identified based on comparison of the measured label values, amino acid concentrations, or numbers of amino acids to the reference label values, amino acid concentrations, or numbers of amino acids for the late-stage leukemia proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage melanoma. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human late-stage melanoma proteome and/or subproteome of interest, each of these different proteomic and/or subproteomic sample types can be measured for a given patient, and the presence or absence of the late-stage melanoma proteome and/or subproteome of interest can be identified based on comparison of the measured label values, amino acid concentrations, or numbers of amino acids to the reference label values, amino acid concentrations, or numbers of amino acids for the late-stage melanoma proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage glioma. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments,

the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human late-stage glioma proteome and/or subproteome of interest, each of these different proteomic and/or subproteomic sample types can be measured for a given patient, and the presence or absence of the late-stage glioma proteome and/or subproteome of interest can be identified based on comparison of the measured label values, amino acid concentrations, or numbers of amino acids to the reference label values, amino acid concentrations, or numbers of amino acids for the late-stage glioma proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage lymphoma. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can be utilised. In some embodiments, the sample utilised is human saliva, and the human saliva proteome or subproteome thereof can be utilised. In some embodiments, multiple different proteomic and/or subproteomic sample types can provide a reference for the human late-stage lymphoma proteome and/or subproteome of interest, each of these different proteomic and/or subproteomic sample types can be measured for a given patient, and the presence or absence of the late-stage lymphoma proteome and/or subproteome of interest can be identified based on comparison of the measured label values, amino acid concentrations, or numbers of amino acids to the reference label values, amino acid concentrations, or numbers of amino acids for the late-stage lymphoma proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage stomach cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human late-stage stomach cancer proteome and/or subproteome of interest, each of these different proteomic and/or subproteomic sample types can be measured for a given patient, and the presence or absence of the late-stage stomach cancer proteome and/or subproteome of interest can be identified based on comparison of the measured label values, amino acid concentrations, or numbers of amino acids to the reference label values, amino acid concentrations, or numbers of amino acids for the late-stage stomach cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having

late-stage lung cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human late-stage lung cancer proteome and/or subproteome of interest, each of these different proteomic and/or subproteomic sample types can be measured for a given patient, and the presence or absence of the late-stage lung cancer proteome and/or subproteome of interest can be identified based on comparison of the measured label values, amino acid concentrations, or numbers of amino acids to the reference label values, amino acid concentrations, or numbers of amino acids for the late-stage lung cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage brain cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human late-stage brain cancer proteome and/or subproteome of interest, each of these different proteomic and/or subproteomic sample types can be measured for a given patient, and the presence or absence of the late-stage brain cancer proteome and/or subproteome of interest can be identified based on comparison of the measured label values, amino acid concentrations, or numbers of amino acids to the reference label values, amino acid concentrations, or numbers of amino acids for the late-stage brain cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage breast cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the

sample utilised is human saliva, and the human saliva proteome or subproteome thereof can be utilised. In some embodiments, multiple different proteomic and/or subproteomic sample types can provide a reference for the human late-stage breast cancer proteome and/or subproteome of interest, each of these different proteomic and/or subproteomic sample types can be measured for a given patient, and the presence or absence of the late-stage breast cancer proteome and/or subproteome of interest can be identified based on comparison of the measured label values, amino acid concentrations, or numbers of amino acids to the reference label values, amino acid concentrations, or numbers of amino acids for the late-stage breast cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage head and neck cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human late-stage head and neck cancer proteome and/or subproteome of interest, each of these different proteomic and/or subproteomic sample types can be measured for a given patient, and the presence or absence of the late-stage head and neck cancer proteome and/or subproteome of interest can be identified based on comparison of the measured label values, amino acid concentrations, or numbers of amino acids to the reference label values, amino acid concentrations, or numbers of amino acids for the late-stage head and neck cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage thyroid cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human late-stage thyroid cancer proteome and/or subproteome of interest, each of these different proteomic and/or subproteomic sample types can be measured for a given patient, and the presence or absence of the late-stage thyroid cancer proteome and/or subproteome of interest can be identified based on comparison of the measured label values, amino acid concentrations, or numbers of amino acids to the reference label values, amino acid concentrations, or numbers of amino acids for the late-stage thyroid cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having

late-stage endometrial cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human late-stage endometrial cancer proteome and/or subproteome of interest, each of these different proteomic and/or subproteomic sample types can be measured for a given patient, and the presence or absence of the late-stage endometrial cancer proteome and/or subproteome of interest can be identified based on comparison of the measured label values, amino acid concentrations, or numbers of amino acids to the reference label values, amino acid concentrations, or numbers of amino acids for the late-stage endometrial cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In some embodiments, the proteome and/or subproteome of interest is from a sample of a human subject having late-stage urothelial cancer. In some embodiments, the sample utilised is a human blood plasma, and the human blood plasma proteome, or a subproteome thereof can be utilised. In some embodiments, the proteome of interest is human platelet poor plasma (PPP) proteome. In some embodiments, the subproteome of interest is human filtered platelet poor plasma (PPP_50) subproteome. In some embodiments, the proteome of interest is platelet rich plasma (PRP) proteome. In some embodiments, the subproteome of interest is human filtered platelet rich plasma (PRP_50) subproteome. In some embodiments, the sample utilised is human serum, and the human serum proteome or subproteome thereof can be utilised. In some embodiments, the sample utilised is human sputum, and the human sputum proteome or subproteome thereof can be utilised. In some embodiments, the sample utilised is human faeces, and the human faecal proteome or subproteome thereof can be utilised. In some embodiments, the sample utilised is human cerebrospinal fluid, and the human cerebrospinal fluid proteome or subproteome thereof can be utilised. In some embodiments, the sample utilised is human perspiration, and the human perspiration proteome or subproteome thereof can be utilised. In some embodiments, the sample utilised is human urine, and the human urine proteome or subproteome thereof can. In some embodiments, the sample utilised is human saliva, and the human saliva proteome or subproteome thereof can be utilised. In some embodiments, multiple different proteomic and/or subproteomic sample types can provide a reference for the human late-stage urothelial cancer proteome and/or subproteome of interest, each of these different proteomic and/or subproteomic sample types can be measured for a given patient, and the presence or absence of the late-stage urothelial cancer proteome and/or subproteome of interest can be identified based on comparison of the measured label values, amino acid concentrations, or numbers of amino acids to the reference label values, amino acid concentrations, or numbers of amino acids for the late-stage urothelial cancer proteome and/or subproteome of interest. In some embodiments, the sample utilised is human cervical mucus or human menses, and the human cervical mucus proteome or subproteome thereof, or human menses proteome or subproteome thereof can be utilised. In some embodiments, the sample utilised is human amniotic fluid, and the human amniotic fluid proteome or subproteome can be utilised.

In preferred embodiments, the sample is a blood plasma sample. In preferred embodiments, the sample is a urine sample.

In some embodiments, the methods disclosed herein, can be used to identify the stage of a cancer in a sample. For example, the methods disclosed herein can be used to identify whether a cancer present in a sample is Stage I, Stage II, Stage III or Stage IV. This is because each stage of cancer has a unique proteomic signature.

In some embodiments, the methods disclosed herein can be used to identify both the type and stage of a cancer. For example, the methods of the invention can be used to identify the presence of colorectal cancer and whether it is early stage (stage II) or late stage (stage III).

In some embodiments, the methods disclosed herein can be used to detect the location of the cancer. For example, in some embodiments, the methods can be used to detect the location of colorectal cancer. For example, the methods disclosed herein can be used to identify that the tumor is specifically located in a patient's right colon rather than their left colon or rectum based on the plasma proteomic signature. This is because different types of cancer have a unique proteomic signature based on the label values, amino acid concentrations or number of amino acids of each of two or more

amino acid types. This is because the location of a cancer has a unique proteomic signature.

In some embodiments, the reference is each proteome of interest (e.g. a healthy patient platelet poor plasma (PPP) sample, a ovarian cancer blood plasma sample, a pancreatic cancer blood plasma sample, a colorectal cancer blood plasma sample). In some embodiments, the reference is experimentally determined using data from the Human Protein Atlas, Human Peptide Atlas and/or Proteome Xchange.

In some embodiments, the comparison step of the methods disclosed herein utilizes a machine learning classifier. The machine learning classifier confirms the type, stage and/or location of the cancer. In some embodiments, the machine learning classifier is a linear support vector machine (SVM).

In some embodiments, the methods disclosed herein can be used to detect the presence, type, stage and location of cancer in a liquid biopsy sample.

When used in this specification and claims, the terms "comprises" and "comprising" and variations thereof mean that the specified features, steps or integers are included. The terms are not to be interpreted to exclude the presence of other features, steps or components.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

Although certain example embodiments of the invention have been described, the scope of the appended claims is not intended to be limited solely to these embodiments. The claims are to be construed literally, purposively, and/or to encompass equivalents.

## Worked examples

The invention is further described in the following examples, which do not limit the scope of the invention described in the claims.

### Example 1

*Labelling two or more amino acid types*

Amino acids of the amino acid type cysteine (C) were labelled with 10 mM tris(2-carboxyethyl)phosphine (TCEP) in 5 mM HEPES buffer at pH 7, followed by 5 mM 4-Fluoro-7-sulfamoylbenzofurazan (ABD-F), 4% sodium dodecyl sulfate (SDS) in 80 mM sodium carbonate buffer at pH 10.5.

Amino acids of the amino acid type reduced cysteine ($C_R$) were labelled with 5 mM 4-Fluoro-7-sulfamoylbenzofurazan (ABD-F), 4% sodium dodecyl sulfate (SDS) in 80 mM sodium carbonate buffer at pH 10.5.

Amino acids of the amino acid type tryptophan (W) were labelled with 0.2 M trichloroethanol (TCE), 10 mM TCEP, 4% SDS in 5 mM HEPES at pH 7.

Amino acids of the amino acid type lysine (K) were labelled with 12 mM ortho-phthalaldehyde (OPA), 18 mM betamercaptoethanol (BME), 4% SDS in 200 mM sodium carbonate buffer at pH 10.5.

The chemical mechanism of each of the fluorogenic labelling reactions is shown in Figure 16.

The labelling solutions were protected from light and stored at 4°C except for the ABD-F and TCEP solutions which were stored at - 20°C.

*Protein solutions*

The following protein solutions were prepared:

Bovine serum albumin (BSA): 1 BSA in 5 mM HEPES buffer at pH.7

Ovalbumin (OVA): 2 μM OVA in 5 mM HEPES buffer at pH 7

Beta-lactoglobulin (β-Lac, Beta-Lac): 4 μM β-Lac in 5 mM HEPES buffer at pH 7

Lysozyme (LYZ): 5 μM LYZ in 5 mM HEPES buffer at pH 7

The following mixture protein solutions were prepared in a 1:1 ratio :
50 μL 1 μM BSA + 50 μL 2 μM beta-lactoglobulin

*Measuring fluorescence intensity*

The reactions were performed using a BMG plate reader. UV transmissive (Corning 3370) 96well plates were used, with 100 μL protein solutions per well. In all cases, 100 μL dye solution was added to the protein solutions for a 1:1 dilution (to the final protein concentrations listed above). Because the $C_R$ amino acid type generally provides a significantly lower signal than the C amino acid type, the gain setting for measurement of the $C_R$ amino acid type was set at twice the gain setting for measurement of the C amino acid type.

For the tryptophan modification reaction, 100 μL of the tryptophan labelling solution was added to each protein solution (also 100 μL). The reaction was photocatalyzed by irradiating with UV light for 30 minutes. Broad spectrum UV light, centered at around 320 nm, was used. After 30 minutes of irradiation, the fluorescence of the newly formed fluorophores was determined by measuring the fluorescence using 350-10 and 480-10 excitation and emission filters.

For the lysine reaction, the fluorescence was measured rapidly after mixing the sample and dye solutions. The fluorescence was measured 3 seconds after mixing the reagents to provide optimal results.

For the cysteine reaction, 1 μL of the 1 mM TCEP stock solution was added to each 100 μL protein well, for a total TCEP concentration of 10 mM. The reaction was allowed to progress, protected from light, for 30 minutes to allow the TCEP reduction of amino acids of the cysteine amino acid type which are disulphide bonded to have reached completion before those amino acids are modified by a fluorogenic dye. 100 μL of the cysteine dye solution (identical to the reduced cysteine dye solution) was added to each well, along with 3 control wells where the dye was added to buffer. The reactions within the wells were protected from light for a 45-minute incubation time, and then the fluorescence was read using 350-10 and 480-10 excitation and emission filters.

For the reduced cysteine reaction, 100 μL of the reduced cysteine dye solution was added to each well, along with 3 control wells where the dye was added to buffer. The reactions within the wells were protected from light for a 45-minute incubation time, and then the fluorescence was read using 350-10 and 480-10 excitation and emission filters.

*Background Correction*

Optionally, the fluorescence background can be subtracted from the fluorescence intensity of the sample. To calculate the fluorescence background, the dye solution was combined with an equal amount of buffer rather than protein, and the fluorescence intensity measured using the parameters provided under the *Measuring Fluorescence Intensity* subsection in order to calculate a fluorescence background. For example, the following fluorescence backgrounds were measured:

C = 2177.5; W = 20632; $C_R$ = 9899

The fluorescence background can be subtracted from the measured fluorescence of the sample to produce a "background corrected" fluorescence intensity.

*Calibration Curve or Standard*

A calibration curve or standard is a general method for determining the concentration of a substance in an unknown sample by comparing the unknown to a set of standard samples, or one standard sample, of known concentration. A calibration curve was calculated for each labelled amino acid type, by measuring the fluorescence intensity of proteins of known amino acid concentration. This establishes the relationship between fluorescence intensity, which was measured in arbitrary units (AU), and amino acid concentration. A standard was also calculated for each labelled amino acid type, by measuring the fluorescence intensity of a protein of known amino acid concentration. The calibration curve or standard was measured once for each amino acid type, covering all experiments performed, rather than comprising part of each experiment in which a sample is measured.

In each case, the measured fluorescence intensity was plotted as a function of the known amino acid concentration of each amino acid type.

The measured data was linear. A linear least squares fit was performed, resulting in the following best fit equations for the calibration curves:

Eq. 1: $C\ Flourescence\ Intensity\ (AU) = 705.1\ {}^{AU}/_{\mu M} \times C\ A.A.concentration\ (\mu M)$

Eq. 2: $C_R\ Flourescence\ Intensity\ (AU) = 2831\ {}^{AU}/_{\mu M} \times C_R\ A.A.concentration\ (\mu M)$

Eq. 3: $W\ Flourescence\ Intensity\ (AU) = 2362\ {}^{AU}/_{\mu M} \times W\ A.A. concentration\ (\mu M) + 9859\ AU$

Eq. 1-3 allowed conversion of the amino acid (A.A.) concentration of each amino acid type for a protein, peptide, oligopeptide, polypeptide, protein complex, subproteome or proteome of interest into the known value of the label of each amino acid type for a protein, peptide, oligopeptide, polypeptide, protein complex, subproteome or proteome of interest.

For the C amino acid type, the calibration factor was determined to be the slope of the best fit line,

$$f_C = 705.1\ {}^{AU}/_{\mu M}$$

For the $C_R$ amino acid type, the calibration factor was determined to be the slope of the best fit line,

$$f_{C_R} = 2831\ {}^{AU}/_{\mu M}$$

For the W amino acid type, the calibration factor was determined to be the slope of the best fit line, plus the y-intercept of the best fit line.

$$f_W = 2362\ {}^{AU}/_{\mu M} + 9859\ AU$$

This means that when the amino acid concentration of the W amino acid type in a protein, peptide, oligopeptide, polypeptide, protein complex, subproteome, or proteome of interest was converted into the corresponding known value of the label of the W amino acid type for the protein, peptide, oligopeptide, polypeptide, protein complex, subproteome, or proteome of interest, the W amino acid concentration was multiplied by 2362, and then 9859 was added. The addition of 9859 was independent of the W amino acid concentration.

The inverse of the calibration curve provided the following inverse best fit equations:

Eq. 4: $C\ A.A. concentration\ (\mu M) = \frac{C\ Flourescence\ Intensity\ (AU)}{705.1\ {}^{AU}/_{\mu M}}$

Eq. 5: $C_R\ A.A. concentration\ (\mu M) = \frac{C_R\ Flourescence\ Intensity\ (AU)}{2831\ {}^{AU}/_{\mu M}}$

Eq. 6: $W\ A.A. concentration\ (\mu M) = \frac{W\ Flourescence\ Intensity\ (AU) - 9859\ AU}{2362\ {}^{AU}/_{\mu M}}$

Eq. 4-6 allowed conversion of the measured signal of the label (Fluorescence Intensity, AU) for each amino acid type to the amino acid (A.A.) concentration of the corresponding amino acid type in the sample.

For the C amino acid type, the calibration factor was determined to be the slope of the line,

$$f_c = 705.1\ {}^{AU}/_{\mu M}$$

For the $C_R$ amino acid type, the calibration factor was determined to be the slope of the line,

$$f_{C_R} = 2831\ {}^{AU}/_{\mu M}$$

For the W amino acid type, the calibration factor was determined to be the slope of the line, plus the y-intercept of the line,

$$f_W = 2362\ {}^{AU}/_{\mu M} + 9859\ AU$$

This means that the amino acid concentration of the W amino acid type is multiplied by 2362, and then 9859 is added to provide the known value of the label for the W amino acid type.

A standard can alternatively be calculated for each amino acid type. In this case, the fluorescence intensity of a single protein of known amino acid concentration was used to establish the relationship between amino acid concentration and fluorescence intensity for that amino acid type. For example, a standard was measured for the K amino acid type.

A standard solution containing 120 μM K amino acid concentration (calmodulin) had a fluorescence-intensity of 86390 AU. Therefore, the known value of the label for the amino acid type K in a protein, peptide, oligoeptpide, polypeptide, protein complex, subproteome, or proteome of interest is $86390\,AU/_{120\,uM}$ which provides the calibration factor $f_k = 719.9\ ^{AU}/_{uM}$ , and the inverse calibration factor $f_k^{-1} = 1.40 \times 10^{-3}\ ^{uM}/_{AU}$

*Reference*

As disclosed herein, the reference for each protein, peptide, oligopeptide, polypeptide, protein complex, subproteome, or proteome of interest was provided as a function of its protein concentration via a vector function (or a set of parametric equations). Vector function 1 was used for a protein of interest wherein the value of the sample was the amino acid concentration of two or more labelled amino acid types, and vector function 2 was used for a proteome of interest wherein the value of the sample was the amino acid concentration of two or more labelled amino acid types. Vector function 3 was used for a protein of interest when the value of the sample was the measured value of the label of two or more labelled amino acid types, and vector function 4 was used for a proteome of interest when the value of the sample was the measured value of the label of two or more labelled amino acid types. Each vector function (equivalent to a set of parametric equations) is described fully herein, and vector function is reproduced below:

Vector function 1 is:

$$p_i(t) = \langle 0, 0, \cdots 0\rangle + \langle a_1 t, a_2 t, \cdots a_n t\rangle, \forall t \geq 0$$

where $p_i$ are the amino acid concentrations provided for protein, peptide, oligopeptide, polypeptide, or protein complex of interest *i* as a function of concentration *t*, $\langle 0, 0, \cdots 0\rangle$ is the origin, $a_1$ is the number of amino acids of amino acid type 1 in the protein, peptide, polypeptide, oligopeptide, or protein complex of interest, $a_2$ is the number of amino acids of amino acid type 2 in the protein, peptide, polypeptide, oligopeptide, or protein complex of interest, $a_n$ is the number of amino acids of amino acid type *n* in the protein, peptide, polypeptide, oligopeptide, or protein complex of interest, t is the total molar concentration of the protein, peptide, polypeptide, oligopeptide, or protein complex of interest which is defined for all values of *t* greater than or equal to 0 ($\forall t \geq 0$). In alternative embodiments, *t* is provided between a lower ($c_1$) and upper ($c_2$) limit of a concentration range ($\forall t \in c_1 \leq t \geq c_2$), and the vector begins at the amino concentrations of the lower bound of the concentration range, $\langle a_1c_1, a_2c_1, \cdots a_nc_1\rangle$.

Vector function 2 is:

$$p_i(t) = \langle 0, 0, \cdots 0\rangle + \langle w_1 t, w_2 t, \cdots w_n t\rangle, \forall t \geq 0$$

where $p_i$ are the amino acid concentrations provided for proteome or subproteome of interest *i* as a function of the concentration, *t*, of the proteome or subproteome, $\langle 0, 0, \cdots 0\rangle$ is the origin, $w_1$ is the weighted mean number of amino acids of amino acid type 1 in the proteome or subproteome of interest, $w_2$ is the weighted mean number of amino acids of amino acid type 2 in the proteome or subproteome of interest, $w_n$ is the number of amino acids of amino acid type n in the proteome or subproteome of interest, and t is the proteome or subproteome concentration (wherein the proteome or subproteome concentration is the total molar or mass concentration of all proteins, peptides, oligopeptides, polypeptides, and protein complexes comprising proteome or subproteome of interest, $p_i$). In some embodiments, the proteome or subproteome concentration *t* is defined for all values of *t* greater than or equal to 0.

Vector function 3 is:

$$p_i(t) = \langle b_1, b_2, \cdots b_n\rangle + \langle a_1 f_1 t, a_2 f_2 t, \cdots a_n f_n t\rangle, \forall t \geq 0$$

Where $p_i$ are the known values of the label provided for protein, peptide, oligopeptide, polypeptide, or protein complex of interest *i* as a function of its concentration *t*, $b_1$ is the background value for amino acid type 1 which is 0 if the measured values of the label in the sample are background-corrected, $b_2$ is the background value for amino acid type 2 which is 0 if the measured values of the label in the sample are background-corrected, $b_n$ is the background value for amino acid type *n*, which is 0 if the measured values of the label in the sample are background-corrected, $a_1$ is the number of

amino acids of acid type 1 in the protein, peptide, polypeptide, oligopeptide, or protein complex of interest, $a_2$ is the number of amino acids of amino acid type 2 in the protein, peptide, polypeptide, oligopeptide, or protein complex of interest, $a_n$ is the number of amino acids of amino acid type n in the protein, peptide, polypeptide, oligopeptide, or protein complex of interest, $f_1$ is the calibration function or calibration factor for amino acid type 1, $f_2$ is the calibration function or calibration factor for amino acid type 2, $f_n$ is the calibration function or calibration factor for amino acid type *n,* and *t* is the molar protein concentration of the protein, peptide, polypeptide, oligopeptide, or protein complex of interest. In some embodiments, *t* is defined for all values of t greater than or equal to 0 ($\forall t \geq 0$). In alternative embodiments, *t* is provided between a lower ($c_1$) and upper ($c_2$) limit of a concentration range ( $\forall t$ E $c_1 \leq t \geq c_2$), and the vector begins at the values of the label of the lower bound of the concentration range, $\langle a_1 f_1 c_1,\ a_2 f_2 c_1, \cdots a_n f_n c_1 \rangle$.

Vector function 4 is:

$$p_i(t) = \langle b_1, b_2, \cdots b_n \rangle + \langle w_1 f_1 t, w_2 f_2 t, \cdots w_n f_n t \rangle, \forall t \geq 0$$

where $p_i$ are the known values of the label provided for proteome or subproteome of interest *i* as a function of its concentration *t,* $b_1$ is the background value for amino acid type 1 which is 0 if the measured values of the label in the sample are background-corrected, $b_2$ is the background value for amino acid type 2 which is 0 if the measured values of the label in the sample are background-corrected, $b_n$ is the background value for amino acid type n which is 0 if the measured values of the label in the sample are background-corrected, $w_1$ is the weighted mean number of amino acids of amino acid type 1 in the proteome or subproteome of interest, $w_2$ is the weighted mean number of amino acids of amino acid type 2 in the proteome or subproteome of interest, $w_n$ is the weighted mean number of amino acids of amino acid type *n* in the proteome or subproteome of interest, $f_1$ is the calibration function or calibration factor for amino acid type 1, $f_2$ is the calibration function or calibration factor for amino acid type 2, $f_n$ is the calibration function or calibration factor for amino acid type *n,* and *t* is the molar concentration of the proteome or subproteome of interest. In some embodiments, *t* is defined for all values of *t* greater than or equal to 0.

The number of amino acids of an amino acid type within a protein of interest was calculated as the number of occurrences of that amino acid type within amino acid sequence or sequences of the protein of interest minus the number of post-translational modifications of that amino acid type that would prevent the amino acid type from reacting with the label. For example, for the C amino acid type, the number of amino acids is the number of unmodified ($C_R$) amino acids, because disulphide bonded amino acids of the C amino acid type do not react with the label. However, modified amino acids can be converted to unmodified amino acids, available for reaction with the label, such as conversion of cysteine disulphide ($C_D$) amino acids to reduced ($C_R$) amino acids with TCEP prior to reaction with the label. Therefore, for the C amino acid type labelling reaction including TCEP, the number of occurrences of C amino acids engaged in disulphide bonds was not subtracted from the number of occurrences of C amino acids within protein sequences. The protein sequences were downloaded in FASTA format from the UniProt database and bioinformatics tools such as the Python ProtParam module were used to determine the number of occurrences of each amino acid type.

For a proteome of interest, the weighted mean number of amino acids across all proteins, peptides, oligopeptides, polypeptides, and/or protein complexes comprising the proteome of interest was calculated. The weighted mean number of amino acids was calculated as a linear combination of the number of amino acids per protein, peptide, oligopeptide, poypeptide, or protein complex times the proportion of that protein, peptide, oligopeptide, polypeptide, or protein complex within the proteome of interest.

It is not generally possible to measure the number of amino acids per protein of an unknown sample, because the molar protein concentration of the unknown sample is unknown (as standard methods in the art for determining this, such via A280, rely on a protein-sequence specific extinction coefficient which is not available when the protein identity is unknown). However if the molar protein concentration of the sample is known, the reference can alternatively be provided as the number/weighted mean number of amino acids of two or more amino acid types of a protein, peptide, oligopeptide, polypeptide, protein complex, subproteome, or proteome of interest.

*Comparison of the Measured Values of the Sample to the Values Provided by the Reference*

The measured values of the sample (values of the label, amino acid concentration, number of amino acids of two or more amino acid types) always provide a point, which can be optionally visualized in n dimensional space for the *n* amino acid types labelled and measured in the sample. The comparison step involved comparing the point measured for the sample to the reference line provided for one or more proteins, peptides, oligopeptides, polypeptides, protein complexes, subproteomes, or proteomes of interest and evaluating whether the shortest distance between the sample point and the reference line is zero, or is less than or equal to an error margin. If the shortest distance between the sample point and the reference line is less than or equal to an error margin, the presence of the protein, oligopeptide, polypeptide, protein complex, subproteome, or proteome of interest is identified within the sample, and it's concentration is determined by the exact point (concentration) on the reference line which provided the shortest distance to the sample point.

The shortest distance between a line and a point is always the perpendicular distance between a line and a point. This is because any other angle between the point and the line other than 90 degrees would form a hypotenuse, whose length will always be greater than the perpendicular distance.

This was achieved by finding the dot product of the direction of the reference line with the vector between the sample point and any point on the reference line, setting the dot product equal to 0, and solving for the concentration of the reference line which provides a perpendicular line between the sample point and the reference line. A dot product is a scalar value that represents the angular relationship between two vectors A and B i.e. $A \cdot B = |A| * |B| * \cos\theta$ where the values $|A|$ and $|B|$ represent the lengths of vectors A and B respectively, and $\theta$ is the angle between the two vectors. If A and B are perpendicular (i.e. at 90 degrees to each other) then the dot product is zero, because cos 90° is zero. This distance between the sample point and the reference line was calculated, and if this distance was less than or equal to an error margin, then the protein, peptide, oligopeptide, polypeptide, protein complex, proteome, or subproteome of interest was identified as being present at the protein concentration on the reference line which provided the perpendicular (minimum) distance.

The comparison approach which was used herein is summarized in the following approach:

1. Let R be the reference line for protein, peptide, oligopeptide, polypeptide, protein complex, proteome, or subproteome of interest $p_i$, and let S be the sample point to find the shortest distance from

2. Find the equation of reference line R in vector format

3. Find the general equation of a point P on the reference line R

4. Find the exact location of the point P on reference line R, called Q, such that the vector from S to P is perpendicular to R. This means find the point Q on reference line R such that the vector between S and Q gives the perpendicular. This is achieved by finding the dot product (•) of the vector from S to P with the direction of R, setting this equal to 0, and solving for *t* to provide the value of *t* which, when substituted into the general equation for a point P on R, yields the perpendicular vector. If protein, peptide, oligopeptide, polypeptide, protein complex, proteome, or subproteome of interest $p_i$ is contained within the sample, then this solution for *t* is its protein concentration.

5. Find the distance between Q and S using the distance formula, called D.

6. Evaluate whether D is less than the error margin, $\varepsilon$.

7. If $D > \varepsilon$, then protein, peptide, oligopeptide, polypeptide, protein complex, proteome, or subproteome of interest $p_i$ is not present within the sample.

8. If $D \leq \varepsilon$, then protein, peptide, oligopeptide, polypeptide, protein complex, proteome, or subproteome of interest $p_i$ is present within the sample at protein concentration t.

The following calculations were performed, explained and outlined for the general vector function of the reference line:

For example, the general parametric form of the reference line ($R$) for protein, peptide, oligopeptide, polypeptide, protein complex, proteome, or subproteome of interest $p_i$ is

$$R = \langle 0,0,\ldots,0\rangle + \langle c_1 t, c_2 t, \ldots, c_n t\rangle,\ \forall t \geq 0$$

which can alternatively be written as $R = \langle 0,0, \ldots , 0\rangle + t\langle c_1, c_2, \ldots , c_n\rangle$

The general equation of a point ($P$) on the reference line is

$$P = \langle 0 + c_1 t, 0 + c_2 t, \cdots, 0 + c_n t\rangle$$

The measured sample point ($S$) has coordinates $S = (S_1, S_2, \ldots, S_n)$

The vector from the measured sample point (S) to any point on the reference line ($P$) is $P - S$

$$P - S = \langle c_1 t - S_1,\ c_2 t - S_2, \ldots, c_n t - S_n \rangle.$$

For this vector to be perpendicular, the dot product (·) of this vector with the direction of the reference line $\langle c_1, c_2, \ldots, c_n \rangle$ must be 0. Therefore, the dot product was set equal to 0

$$\langle c_1 t \quad S_1,\ c_2 t \quad S_2, \ldots, c_n t \quad S_n \rangle \cdot \langle c_1, c_2, \ldots, c_n \rangle = 0$$

$$c_1\langle c_1 t - S_1\rangle + c_2\langle c_2 t - S_2\rangle + \cdots + c_n\langle c_n t - S_n\rangle = 0$$

$$c_1{}^2 t - c_1 S_1 + c_2{}^2 t - c_2 S_2 + \cdots + c_n{}^2 t - c_n S_n = 0$$

$$c_1{}^2 t + c_2{}^2 t + \cdots + c_n{}^2 t = c_1 S_1 + c_2 S_2 + \cdots + c_n S_n$$

$$t(c_1{}^2 + c_2{}^2 + \cdots + c_n{}^2) = c_1 S_1 + c_2 S_2 + \cdots + c_n S_n$$

$$t = \frac{c_1 S_1 + c_2 S_2 + \cdots + c_n S_n}{c_1{}^2 + c_2{}^2 + \cdots + c_n{}^2}$$

and the equation solved for *t.* This solution for t is the protein concentration of the protein, peptide, oligopeptide, polypeptide, protein complex, proteome, or subproteome of interest $p_i$ for which the distance between the sample and the reference line *R* is shortest. Therefore, if protein, peptide, oligopeptide, polypeptide, protein complex, proteome, or subproteome of interest $p_i$ is present within the sample, then the protein, peptide, oligopeptide, polypeptide, protein complex, proteome, or subproteome of interest $p_i$ is present within the sample at protein concentration *t.*

To determine whether the protein, peptide, oligopeptide, polypeptide, protein complex, proteome, or subproteome of interest $p_i$ is present within the sample, the point on the reference line, *Q*, which gave the perpendicular distance was found

*Q = P(t).*

$$P = \langle 0 + c_1 t, 0 + c_2 t, \ldots, 0 + c_n t \rangle = \langle c_1 t, c_2 t, \cdots, c_n t \rangle$$

$$Q = \left( c_1\left(\frac{c_1 S_1 + c_2 S_2 + \cdots + c_n S_n}{c_1{}^2 + c_2{}^2 + \cdots + c_n{}^2}\right), \quad c_2\left(\frac{c_1 S_1 + c_2 S_2 + \cdots + c_n S_n}{c_1{}^2 + c_2{}^2 + \cdots + c_n{}^2}\right), \cdots, c_n\left(\frac{c_1 S_1 + c_2 S_2 + \cdots + c_n S_n}{c_1{}^2 + c_2{}^2 + \cdots + c_n{}^2}\right)\right)$$

*Q* is a point, which is the set of values for the reference which correspond to the solution for *t. S* is also a point. The distance, *D,* between *S* and *Q* was determined using the distance formula.

The Euclidean distance formula between point *S* and point *Q* was used in the worked examples provided here.

$$distance = \sqrt{\sum_{i=1}^{n} (S_i - Q_i)^2}$$

The equation becomes

$$D = \sqrt{\left(S_1 - c_1\left(\frac{c_1 S_1 + c_2 S_2 + \cdots + c_n S_n}{c_1{}^2 + c_2{}^2 + \cdots + c_n{}^2}\right)\right)^2 + \left(S_2 - c_2\left(\frac{c_1 S_1 + c_2 S_2 + \cdots + c_n S_n}{c_1{}^2 + c_2{}^2 + \cdots + c_n{}^2}\right)\right)^2 + \cdots + \left(S_n - c_n\left(\frac{c_1 S_1 + c_2 S_2 + \cdots + c_n S_n}{c_1{}^2 + c_2{}^2 + \cdots + c_n{}^2}\right)\right)^2}$$

$\varepsilon$ is the error margin. The error margin was provided according to one of two approaches. In the first approach, if the number of one or more proteins, peptides, oligopeptides, polypeptides, protein complex, subproteomes, or proteomes of interest the sample contained was not suspected, then the error margin was provided using a tolerance multiplied by the square root of the sample values squared, reflecting the distance calculation. This is provided by Eq 7:

$$\varepsilon = \varphi\sqrt{S_1^2 + S_2^2 + \cdots + S_n^2}$$

**wherein** $\varepsilon$ is the error margin, $\varphi$ is a tolerance value, $S_1$ is the value measured for the sample for amino acid type 1, $S_2$ is the value measured for the sample for amino acid type 1, and $S_n$ is the value measured for the sample for amino acid type n.

In the second approach, the number of one or more proteins, peptides, oligopetpides, polypeptipdes, protein complexes, polypeptides, protein complex, subproteomes, or proteomes of interest the sample contained was suspected. It was suspected that the sample contained k proteins, peptides, oligopetpides, polypeptipdes, protein complexes, polypeptides, protein complex, subproteomes, or proteomes of interest. In this approach, the calculated perpendicular distances are ranked, and the *kth* smallest value ($k^{th}$ order statistic) is the set as the error margin such that *k* proteins, peptides, oligopetpides, polypeptipdes, protein complexes, polypeptides, protein complex, subproteomes, or proteomes of interest the sample have perpendicular distances less than or equal to the error margin. Formally, the $k^{th}$ order statistic is the $k^{th}$ smallest ($k^{th}$ minimum) value of the calculated perpendicular distances. For example, the set of all perpendicular distances between the sample point and each reference line is $\beta$, and the $k^{th}$ order statistic of $\beta$ is $\beta$. $\varepsilon = \beta_k$.

Therefore, $D \leq \varepsilon$ for *c* proteins, peptides, oligopeptides, polypeptides, protein complexes, proteomes, or subproteomes of interest in the reference database, and so *c* proteins, peptides, oligopeptides, polypeptides, protein complexes, proteomes, or subproteomes of interest are identified in the sample

If $D > \varepsilon$, then protein, peptide, oligopeptide, polypeptide, protein complex, proteome, or subproteome of interest $p_i$ is not present within the sample. The hypothesis test was negative.

If $D \leq \varepsilon$, then protein, peptide, oligopeptide, polypeptide, protein complex, proteome, or subproteome of interest $p_i$ is present within the sample at the protein concentration

$$t = \frac{c_1S_1 + c_2S_2 + \cdots + c_nS_n}{{c_1}^2 + {c_2}^2 + \cdots + {c_n}^2}$$

The amount of the protein, peptide, oligopeptide, polypeptide, protein complex, proteome, or subproteome of interest $p_i$ is present within the sample was calculated as the protein concentration of the protein, peptide, oligopeptide, polypeptide, protein complex, proteome, or subproteome of interest $p_i$ multiplied by the volume of the sample.

The methods described in example 1 are now applied in additional examples.

**Example 2** - **Determining the presence and/or concentration and/or amount of a protein, peptide, oligopeptide, polypeptide, or protein complex in a sample based on amino acid concentration**

A sample for testing was obtained. It was suspected that the sample may contain an unknown protein, peptide, oligopeptide, polypeptide, or protein complex. The concentration of the sample was unknown. The sample was measured one time.

The C and W amino acid types were fluorescently labelled using a fluorogenic dye outlined in Example 1.

The fluorescence intensity of each fluorogenic dye which has labelled each amino acid type was measured using a BMG plate reader.

The raw fluorescence intensity values obtained were:

C = 31191 AU; W = 34576 AU

The raw fluorescence intensity values obtained were background corrected as outlined in Example 1. The background corrected fluorescence intensity values obtained were:

C = 2177.5 AU; W = 20632 AU

The background corrected fluorescence intensity values were converted into amino acid concentrations as outlined in Example 1 using

$$C\ A.A.concentration\ (\mu M) = \frac{C\ Flourescence\ Intensity\ (AU)}{705.1\ {AU}/{\mu M}}$$

$$C\ A.A.concentration\ (\mu M) = \frac{2177.5\ AU}{705.1\ {AU}/{\mu M}}$$

$$W\ A.A.concentration\ (\mu M) - \frac{W\ Flourescence\ Intensity\ (AU) - 9859\ AU}{2362\ {}^{AU}/_{\mu M}}$$

$$W\ A.A.concentration\ (\mu M) - \frac{20632\ (AU) - 9859\ AU}{2362\ {}^{AU}/_{\mu M}}$$

This provided the following amino acid concentrations measured for the sample:

| Sample Reading (S) | |
|---|---|
| [C(μM)] | [W(μM)] |
| 41.15 | 1.73 |

The amino acid type C was amino acid type 1, $a_1$, and the amino acid type W was amino acid type 2, $a_2$.

The amino acid concentrations measured for the sample, which are a point in 2-dimensional space, were compared to the reference lines provided for the following selection of peptides, proteins, oligopeptides, polypeptides, and protein complexes of interest. The results of the approach carried out in Example 1 are provided in the following table.

**Table 5: Results of Example 1**

| | | | P | | | | | | | | |
|---|---|---|---|---|---|---|---|---|---|---|---|
| Identifier | $a_1$ | $a_2$ | $a_1$t (uM) | $a_2$t (uM) | P-S (uM) | | Solution t (uM) | [C (uM)] | [W (uM)] | Distance (uM) | Minimum Distance |
| APOL2 | 1 | 3 | 1t | 3t | 1t-41.15 | 3t-1.73 | 4.63 | 4.63 | 13.9 | 38.49 | 0.62 |
| HADHB | 5 | 3 | 5t | 3t | 5t-41.15 | 3t-1.73 | 6.2 | 31.02 | 18.61 | 19.69 | 0.62 |
| CISD2 | 4 | 2 | 4t | 2t | 4t-41.15 | 2t-1.73 | 8.4 | 33.61 | 16.81 | 16.86 | 0.62 |
| BCLAF1 | 2 | 6 | 2t | 6t | 2t-41.15 | 6t-1.73 | 2.32 | 4.63 | 13.9 | 38.49 | 0.62 |
| NAGA | 9 | 13 | 9t | 13t | 9t-41.15 | 13t-1.73 | 1.57 | 14.14 | 20.43 | 32.85 | 0.62 |
| CAPZA2 | 4 | 5 | 4t | 5t | 4t-41.15 | 5t-1.73 | 4.23 | 16.9 | 21.13 | 31.05 | 0.62 |
| SSC5D | 43 | 40 | 43t | 40t | 43t-41.15 | 40t-1.73 | 0.53 | 22.92 | 21.32 | 26.76 | 0.62 |
| ADSS2 | 7 | 5 | 7t | 5t | 7t-41.15 | 5t-1.73 | 4.01 | 28.07 | 20.05 | 22.51 | 0.62 |
| AOC3 | 10 | 14 | 10t | 14t | 10t-41.15 | 14t-1.73 | 1.47 | 14.72 | 20.61 | 32.48 | 0.62 |
| ANGPT2 | 9 | 10 | 9t | 10t | 9t-41.15 | 10t-1.73 | 2.14 | 19.28 | 21.42 | 29.43 | 0.62 |
| BSA | 35 | 2 | 35t | 2t | 35t-41.15 | 2t-1.73 | 1.17 | 41.11 | 2.35 | 0.62 | 0.62 |
| COPS3 | 10 | 0 | 10t | 0t | 10t-41.15 | 0t-1.73 | 4.12 | 41.15 | 0 | 1.73 | 0.62 |
| KLB | 13 | 30 | 13t | 30t | 13t-41.15 | 30t-1.73 | 0.55 | 7.14 | 16.47 | 37.07 | 0.62 |
| UBA1 | 19 | 10 | 19t | 10t | 19t-41.15 | 10t-1.73 | 1.73 | 32.94 | 17.34 | 17.63 | 0.62 |
| DNER | 65 | 6 | 65t | 6t | 65t-41.15 | 6t-1.73 | 0.63 | 40.96 | 3.78 | 2.06 | 0.62 |
| CALD1 | 1 | 5 | 1t | 5t | 1t-41.15 | 5t-1.73 | 1.92 | 1.92 | 9.58 | 40.01 | 0.62 |
| SUMF1 | 11 | 12 | 11t | 12t | 11t-41.15 | 12t-1.73 | 1.79 | 19.65 | 21.44 | 29.16 | 0.62 |
| GM2A | 8 | 3 | 8t | 3t | 8t-41.15 | 3t-1.73 | 4.58 | 36.65 | 13.74 | 12.83 | 0.62 |
| XPNPEP2 | 9 | 21 | 9t | 21t | 9t-41.15 | 21t-1.73 | 0.78 | 7.01 | 16.36 | 37.14 | 0.62 |
| HACD3 | 4 | 9 | 4t | 9t | 4t-41.15 | 9t-1.73 | 1.86 | 7.43 | 16.72 | 36.9 | 0.62 |

Test 2 was performed It was suspected that the sample contained 1 protein, peptide, oligopeptide, polypeptide, or protein complex of interest, so the minimum values of the calculated Euclidean distance (D) was computed as 0.62.

The error margin was set equal to this, $\varepsilon = 0.62$. A protein, peptide, oligopeptide, polypeptide, or protein complex of interest is identified as being present within the sample if $D \leq \varepsilon$. $D \leq \varepsilon$ for BSA. Therefore, the presence of BSA was identified in the sample. The concentration of BSA in the sample was the concentration of BSA which provided the solution for t which satisfied the hypothesis test. This is 1.17 μM. This concentration is multiplied by the volume of the sample (200

μL) to obtain the amount of BSA present within the sample. The results are summarized in the following table:

| | |
|---|---|
| Presence | BSA |
| Concentration | 1.17 μM |
| Amount | 234 pmol |

**Example 3 - Determining the presence and/or concentration and/or amount of a protein, peptide, oligopeptide, polypeptide, or protein complex in a sample based on amino acid concentration**

A sample for testing is obtained. It is suspected that the sample may contain a laboratory protein. The identity and concentration of the sample are unknown.

The K and W amino acid types were fluorescently labelled using a fluorogenic dye outlined in Example 1.

The fluorescence intensity of each fluorogenic dye which has labelled each amino acid type was measured using a plate reader.

The raw fluorescence intensity values obtained were background corrected as outlined in Example 1. The background corrected fluorescence intensity values obtained were:
K = 41804 AU; W = 29865 AU

The background corrected fluorescence intensity values are converted to amino acid concentrations as outlined in Example 1, which provided the following amino acid concentrations:

| | |
|---|---|
| K (μM) | 58.07 |
| W (μM) | 8.47 |

A tolerance value of 0.03 was chosen ($\varphi$ = 0.03). The error threshold was therefore defined as

$$\varepsilon = 0.03 \times \sqrt{(58.07\ \mu M)^2 + (8.47\ \mu M)^2} = 1.76\ \mu M$$

The amino acid concentrations measured for the sample, which are a point in 2-dimensional space, were compared to the reference lines provided for the following selection of laboratory proteins using the approach described (Test 2).

Table 6: Results of Example 3

| | $a_1$ | $a_2$ | $a_1$t (uM) | $a_2$t (uM) | P-S (uM) | | sol. t (uM) | $a_1$t (uM) | $a_2$t (uM) | D (uM) | ε (uM) |
|---|---|---|---|---|---|---|---|---|---|---|---|
| Lysozyme | 6 | 6 | 6t | 6t | 6t-58.07 | 6t-8.47 | 5.55 | 33.27 | 33.27 | 35.07 | 1.761 |
| Alkaline Phosphatase | 21 | 4 | 21t | 4t | 21t-58.0-7 | 4t-8.47 | 2.74 | 57.5938 | 10.97 | 2.545 | 1.761 |
| Aprotinin | 4 | 0 | 4t | 0t | 4t-58.07 | 0t-8.47 | 14.5 | 58.07 | 0 | 8.47 | 1.761 |
| Avidin | 36 | 16 | 36t | 16t | 36t-58.0-7 | 16t-8.4-7 | 1.43 | 51.6349 | 22.949 | 15.84 | 1.761 |
| BSA | 59 | 2 | 59t | 2t | 59t-58.0-7 | 2t-8.47 | 0.99 | 58.2901 | 1.9759 | 6.498 | 1.761 |
| β-Casein | 11 | 1 | 11t | 1t | 11t-58.0-7 | 1t-8.47 | 5.31 | 58.3577 | 5.3052 | 3.178 | 1.761 |
| Enterokinase | 41 | 19 | 41t | 19t | 41t-58.0-7 | 19t-8.4-7 | 1.24 | 51.0352 | 23.65 | 16.73 | 1.761 |
| G6P DH | 27 | 13 | 27t | 13t | 27t-58.0-7 | 13t-8.4-7 | 1.87 | 50.4521 | 24.292 | 17.56 | 1.761 |
| Lysostaphin | 16 | 8 | 16t | 8t | 16t-58.0-7 | 8t-8.47 | 3.12 | 49.844 | 24.922 | 18.39 | 1.761 |

(continued)

| | $a_1$ | $a_2$ | $a_1t$ (uM) | $a_2t$ (uM) | P-S (uM) | | sol. t (uM) | $a_1t$ (uM) | $a_2t$ (uM) | D (uM) | ε (uM) |
|---|---|---|---|---|---|---|---|---|---|---|---|
| Blac | 15 | 2 | 15t | 2t | 15t-58.0-7 | 2t-8.47 | 3.88 | 58.1653 | 7.7554 | 0.721 | 1.761 |
| Asyn | 15 | 0 | 15t | 0t | 15t-58.0-7 | 0t-8.47 | 3.87 | 58.07 | 0 | 8.47 | 1.761 |
| Renin | 15 | 3 | 15t | 3t | 15t-58.0-7 | 3t-8.47 | 3.83 | 57.4654 | 11.493 | 3.083 | 1.761 |
| Superoxide Dismutase | 11 | 1 | 11t | 1t | 11t-58.0-7 | 1t-8.47 | 5.31 | 58.3577 | 5.3052 | 3.178 | 1.761 |
| Kunitz Trypsin Inhibitor | 10 | 2 | 10t | 2t | 10t-58.0-7 | 2t-8.47 | 5.75 | 57.4654 | 11.493 | 3.083 | 1.761 |

The error margin was provided as 1.76 μM. A protein, peptide, oligopeptide, polypeptide, or protein complex of interest is identified as being present within the sample if $D \leq \varepsilon$.

$D \leq \varepsilon$ only for β-Lac. Therefore, the presence of β-Lac was identified in the sample. The concentration of β-Lac in the sample wasthe concentration of β-Lac which provided the solution for t which satisfied Test 2. This was 3.88 μM. This concentration is multiplied by the volume of the sample (200 μL) to obtain the amount of BSA present within the sample. The results are summarized in the following table:

| | |
|---|---|
| Presence | β-Lac |
| Concentration | 3.88 μM |
| Amount | 730 pmol |

**Example 4 - Determining the presence and/or concentration and/or amount of a protein, peptide, oligopeptide, polypeptide, or protein complex in a sample based on values of the label**

A sample for testing is obtained. It is suspected that the sample may contain a laboratory protein. The identity and concentration of the sample are unknown.

The K and C amino acid types were fluorescently labelled using a fluorogenic dye outlined in Example 1.

The fluorescence intensity of each fluorogenic dye which has labelled each amino acid type was measured using a plate reader.

The raw fluorescence intensity values obtained were background corrected as outlined in Example 1.

| | | |
|---|---|---|
| K FI | 24448 | $a_1$ |
| C FI | 28413 | $a_2$ |

However, the background corrected fluorescence intensity values were not converted into amino acid concentrations. Instead, the reference for each protein of interest was provided in terms of the known value of the label of the K ($a_1$) and W ($a_2$) amino acid types based on vector function 3.

Table 7: Results of Example 4

| ID | $a_1$ | $a_2$ | $a_1f_1t$ | $a_2f_2t$ | $a_1f_1t-S_1$ | $a_2f_2t-S_2$ | t sol. | $a_1f_1$ | $a_2f_2$ | $Q_1$ | $Q_2$ | D |
|---|---|---|---|---|---|---|---|---|---|---|---|---|
| ALP | 21 | 5 | 15117.9t | 3525.5t | 15117.9t - 24448 | 3525.5t - 28413 | 1.85 | 15838 | 3526 | 29320 | 6527 | 22422 |
| Avidin | 36 | 8 | 25916.4t | 5640.8t | 25916.4t - 24448 | 5640.8t - 28413 | 1 | 28796 | 5641 | 28905 | 5662 | 23183 |
| BSA | 59 | 35 | 42474.1t | 24678.5t | 42474.1t - 24448 | 24678.5t - 28413 | 0.71 | 43194 | 24679 | 30670 | 17523 | 12542 |
| Enterokinase | 41 | 35 | 29515.9t | 24678.5t | 29515.9t - 24448 | 24678.5t - 28413 | 0.93 | 30956 | 24679 | 28797 | 22958 | 6976.8 |
| G6P DH | 27 | 8 | 19437.3t | 5640.8t | 19437.3t - 24448 | 5640.8t - 28413 | 1.49 | 20157 | 5641 | 30046 | 8408 | 20773 |
| KTI | 10 | 4 | 7199t | 2820.4t | 7199t - 24448 | 2820.4t - 28413 | 3.87 | 7918.9 | 2820 | 30676 | 10926 | 18563 |
| Lysostaphin | 16 | 0 | 11518.4t | 0t | 11518.4t - 24448 | 0t - 28413 | 2 | 12238 | 0 | 24448 | 0 | 28413 |
| LYZ | 6 | 8 | 4319.4t | 5640.8t | 4319.4t - 24448 | 5640.8t - 28413 | 4.95 | 5039.3 | 5641 | 24968 | 27948 | 697.43 |
| Renin | 15 | 6 | 10798.5t | 4230.6t | 10798.5t - 24448 | 4230.6t - 28413 | 2.67 | 11518 | 4231 | 30737 | 11290 | 18242 |
| SOD | 11 | 4 | 7918.9t | 2820.4t | 7918.9t - 24448 | 2820.4t - 28413 | 3.53 | 8638.8 | 2820 | 30476 | 9950 | 19422 |
| β-Casein | 11 | 0 | 7918.9t | 0t | 7918.9t - 24448 | 0t - 28413 | 2.83 | 8638.8 | 0 | 24448 | 0 | 28413 |
| α2M | 88 | 25 | 63351.2t | 17627.5t | 63351.2t - 24448 | 17627.5t - 28413 | 0.47 | 63351 | 17628 | 30029 | 8356 | 20819 |
| αsyn | 15 | 0 | 10798.5t | 0t | 10798.5t - 24448 | 0t - 28413 | 2.12 | 11518 | 0 | 24448 | 0 | 28413 |
| βlac | 15 | 5 | 10798.5t | 3525.5t | 10798.5t - 24448 | 3525.5t - 28413 | 2.63 | 11518 | 3526 | 30305 | 9276 | 20014 |

It was suspected that the sample contained a single protein of interest. Therefore, the presence of the protein of interest in the sample was identified which gave the smallest perpendicular distance. This is LYZ. The concentration of LYZ identified in the sample is the concentration, sol t, which provided this lowest value of the perpendicular distance. This was 4.95 μM.

**Example 5** - **Determining the presence and/or concentration and/or amount of a protein, peptide, oligopeptide, polypeptide, or protein complex in a sample based on values of the label**

A sample for testing is obtained. It is suspected that the sample may contain a protein, peptide, oligopeptide, polypeptide, or protein complex of interest. The identity and concentration of the sample are unknown.
The K and C amino acid types were fluorescently labelled using a fluorogenic dye outlined in Example 1.
The fluorescence intensity of each fluorogenic dye which has labelled each amino acid type was measured using a plate reader.
The raw fluorescence intensity values obtained were background corrected as outlined in Example 1.
The following background corrected fluorescence intensity values were obtained:

| $S_1$ | $S_2$ | $S_3$ |
|---|---|---|
| K | C | W |
| 40278 | 29013.5 | 13944 |

These background corrected fluorescence intensity values were not converted into amino acid concentrations or number of amino acids; instead the known value of the label for the K, C, and W amino acid types of a large database of proteins, peptides, oligopeptides, polypeptides, and protein complexes of interest were generated using vector function 3, and hypothesis test 2 was applied.

Table 8: Results of Example 5

| ID | $a_1f_1$ | $a_2f_2$ | $a_3f_3$ | $a_1f_1t-S_1$ | $a_2f_2t-S_2$ | $a_3f_3t-S_3$ | sol t | $Q_1$ | $Q_2$ | $Q_3$ | D |
|---|---|---|---|---|---|---|---|---|---|---|---|
| ALDOB | 17997.5 | 6345.9 | 16945 | 17997.5t - 40278 | 6345.9t - 2177.5 | 16945t - 13944 | 1.758448 | 31648 | 11159 | 29797 | 25389 |
| CD226 | 7199 | 4935.7 | 26393 | 7199t - 40278 | 4935.7t - 2177.5 | 26393t - 13944 | 1.036764 | 7464 | 5117 | 27363 | 42754 |
| AOX1 | 60471.6 | 29614.2 | 33479 | 60471.6t - 40278 | 29614.2t - 2177.5 | 33479t - 13944 | 0.665242 | 40228 | 19701 | 22272 | 12493 |
| OSCAR | 2159.7 | 4230.6 | 28755 | 2159.7t - 40278 | 4230.6t - 2177.5 | 28755t - 13944 | 0.718959 | 1553 | 3042 | 20674 | 47111 |
| FAP | 31675.6 | 8461.2 | 57099 | 31675.6t - 40278 | 8461.2t - 2177.5 | 57099t - 13944 | 0.534575 | 16933 | 4523 | 30524 | 37678 |
| CST6 | 5039.3 | 3525.5 | 12221 | 5039.3t - 40278 | 3525.5t - 2177.5 | 12221t - 13944 | 2.541289 | 12806 | 8959 | 31057 | 38075 |
| DCXR | 6479.1 | 3525.5 | 16945 | 6479.1t - 40278 | 3525.5t - 2177.5 | 16945t - 13944 | 1.755378 | 11373 | 6189 | 29745 | 40077 |
| PLPBP | 14398 | 3525.5 | 16945 | 14398t - 40278 | 3525.5t - 2177.5 | 16945t - 13944 | 1.812103 | 26091 | 6389 | 30706 | 31530 |
| MYL9 | 10798.5 | 705.1 | 9859 | 10798.5t - 40278 | 705.1t - 2177.5 | 9859t - 13944 | 2.766498 | 29874 | 1951 | 27275 | 31912 |
| TEK | 44633.8 | 26793.8 | 52375 | 44633.8t - 40278 | 26793.8t - 2177.5 | 52375t - 13944 | 0.606148 | 27055 | 16241 | 31747 | 25592 |
| INRNPA2B | 14398 | 705.1 | 12221 | 14398t - 40278 | 705.1t - 2177.5 | 12221t - 13944 | 2.158153 | 31073 | 1522 | 26375 | 31544 |
| ALDH1A1 | 28076.1 | 7756.1 | 26393 | 28076.1t - 40278 | 7756.1t - 2177.5 | 26393t - 13944 | 1.115785 | 31327 | 8654 | 29449 | 27111 |
| EEF1A2 | 33835.3 | 4230.6 | 21669 | 33835.3t - 40278 | 4230.6t - 2177.5 | 21669t - 13944 | 1.095232 | 37057 | 4633 | 23733 | 26468 |
| HEPHL1 | 50393 | 12691.8 | 57099 | 50393t - 40278 | 12691.8t - 2177.5 | 57099t - 13944 | 0.535857 | 27003 | 6801 | 30597 | 30772 |
| SLC3A2 | 21597 | 2820.4 | 40565 | 21597t - 40278 | 2820.4t - 2177.5 | 40565t - 13944 | 0.715764 | 15458 | 2019 | 29035 | 39654 |
| ATP2A2 | 41754.2 | 20447.9 | 47651 | 41754.2t - 40278 | 20447.9t - 2177.5 | 47651t - 13944 | 0.663219 | 27692 | 13561 | 31603 | 26627 |
| FLRT3 | 24476.6 | 8461.2 | 31117 | 24476.6t - 40278 | 8461.2t - 2177.5 | 31117t - 13944 | 1.01604 | 24869 | 8597 | 31616 | 31090 |
| KRT77 | 15117.9 | 3525.5 | 14583 | 15117.9t - 40278 | 3525.5t - 2177.5 | 14583t - 13944 | 2.016011 | 30478 | 7107 | 29399 | 28545 |
| FLNA | 115903.9 | 33844.8 | 50013 | 115903.9t - 40278 | 33844.8t - 2177.5 | 50013t - 13944 | 0.371636 | 43074 | 12578 | 18587 | 17306 |
| KRT39 | 20157.2 | 19037.7 | 19307 | 20157.2t - 40278 | 19037.7t - 2177.5 | 19307t - 13944 | 1.430967 | 28844 | 27242 | 27628 | 17920 |
| SRSF10 | 15837.8 | 705.1 | 14583 | 15837.8t - 40278 | 705.1t - 2177.5 | 14583t - 13944 | 1.857162 | 29413 | 1309 | 27083 | 32530 |
| PTPN11 | 33115.4 | 7051 | 26393 | 33115.4t - 40278 | 7051t - 2177.5 | 26393t - 13944 | 1.034447 | 34256 | 7294 | 27302 | 26200 |
| KLKB1 | 30235.8 | 26088.7 | 35841 | 30235.8t - 40278 | 26088.7t - 2177.5 | 35841t - 13944 | 0.85939 | 25984 | 22420 | 30801 | 23064 |
| SECTM1 | 5039.3 | 3525.5 | 26393 | 5039.3t - 40278 | 3525.5t - 2177.5 | 26393t - 13944 | 0.916763 | 4620 | 3232 | 24196 | 45181 |
| CHGA | 23036.8 | 2115.3 | 19307 | 23036.8t - 40278 | 2115.3t - 2177.5 | 19307t - 13944 | 1.386084 | 31931 | 2932 | 26761 | 30236 |
| PIK3IP1 | 7199 | 5640.8 | 19307 | 7199t - 40278 | 5640.8t - 2177.5 | 19307t - 13944 | 1.583765 | 11402 | 8934 | 30578 | 38907 |
| SHMT2 | 16557.7 | 5640.8 | 19307 | 16557.7t - 40278 | 5640.8t - 2177.5 | 19307t - 13944 | 1.620345 | 26829 | 9140 | 31284 | 29606 |
| CHST10 | 19437.3 | 4230.6 | 24031 | 19437.3t - 40278 | 4230.6t - 2177.5 | 24031t - 13944 | 1.274901 | 24781 | 5394 | 30637 | 32814 |
| FGGY | 23036.8 | 8461.2 | 38203 | 23036.8t - 40278 | 8461.2t - 2177.5 | 38203t - 13944 | 0.827483 | 19063 | 7002 | 31612 | 35310 |
| FUOM | 5759.2 | 2115.3 | 12221 | 5759.2t - 40278 | 2115.3t - 2177.5 | 12221t - 13944 | 2.480008 | 14283 | 5246 | 30308 | 38839 |
| ALP | 15837.8 | 3525.5 | 19307 | 15837.8t - 40278 | 3525.5t - 2177.5 | 19307t - 13944 | 1.587073 | 25136 | 5595 | 30642 | 32504 |
| Avidin | 28796 | 5640.8 | 47651 | 28796t - 40278 | 5640.8t - 2177.5 | 47651t - 13944 | 0.634794 | 18280 | 3581 | 30249 | 37371 |
| BSA | 43194 | 24678.5 | 14583 | 43194t - 40278 | 24678.5t - 2177.5 | 14583t - 13944 | 0.989473 | 42739 | 24419 | 14429 | 5235 |
| nterokinas | 30955.7 | 24678.5 | 54737 | 30955.7t - 40278 | 24678.5t - 2177.5 | 54737t - 13944 | 0.59738 | 18492 | 14742 | 32699 | 32094 |
| G6P DH | 20157.2 | 5640.8 | 40565 | 20157.2t - 40278 | 5640.8t - 2177.5 | 40565t - 13944 | 0.739658 | 14909 | 4172 | 30004 | 38969 |
| KTI | 7918.9 | 2820.4 | 14583 | 7918.9t - 40278 | 2820.4t - 2177.5 | 14583t - 13944 | 2.132276 | 16885 | 6014 | 31095 | 37018 |
| ysostaphi | 12238.3 | 0 | 28755 | 12238.3t - 40278 | 0t - 2177.5 | 28755t - 13944 | 0.915288 | 11202 | 0 | 26319 | 42899 |
| LYZ | 5039.3 | 5640.8 | 24031 | 5039.3t - 40278 | 5640.8t - 2177.5 | 24031t - 13944 | 1.10559 | 5571 | 6236 | 26568 | 43390 |
| Renin | 11518.4 | 4230.6 | 16945 | 11518.4t - 40278 | 4230.6t - 2177.5 | 16945t - 13944 | 1.880181 | 21657 | 7954 | 31860 | 33335 |
| SOD | 8638.8 | 2820.4 | 12221 | 8638.8t - 40278 | 2820.4t - 2177.5 | 12221t - 13944 | 2.587748 | 22355 | 7298 | 31625 | 33247 |
| ß-Casein | 8638.8 | 0 | 12221 | 8638.8t - 40278 | 0t - 2177.5 | 12221t - 13944 | 2.31431 | 19993 | 0 | 28283 | 38195 |
| α2M | 63351.2 | 17627.5 | 35841 | 63351.2t - 40278 | 17627.5t - 2177.5 | 35841t - 13944 | 0.635241 | 40243 | 11198 | 22768 | 19881 |
| αsyn | 11518.4 | 0 | 9859 | 11518.4t - 40278 | 0t - 2177.5 | 9859t - 13944 | 2.616275 | 30135 | 0 | 25794 | 32941 |
| βlac | 11518.4 | 3525.5 | 14583 | 11518.4t - 40278 | 3525.5t - 2177.5 | 14583t - 13944 | 2.151041 | 24777 | 7583 | 31369 | 31673 |

It was suspected that the sample contained one protein, peptide, oligopeptide, polypeptide, or protein complex of interest, so the error margin was defined as the minimum value of the perpendicular distance, such that only the protein, peptide, oligopeptide, polypeptide, or protein complex of interest closest to the sample point was identified as being present in the sample. This minimum value of the perpendicular distance was 5235. Therefore, the presence of BSA in the sample was identified at the protein concentration which provided this perpendicular distance, which was 0.99 μM.

## Example 6 - Determining the presence and/or concentration and/or amount of more than one protein of interest in a sample

A sample for testing is obtained. It is suspected that the sample may contain one or more proteins, peptides, oligopeptides, polypeptides, or protein complexes of interest. The identity and concentration of the sample are unknown.

The C and W amino acid types were fluorescently labelled using a fluorogenic dye outlined in Example 1.

The fluorescence intensity of each fluorogenic dye which has labelled each amino acid type was measured using a plate reader. The raw fluorescence intensities were:

C = 24130 AU; W = 36947 AU

The raw fluorescence intensity values obtained were background corrected as outlined in Example 1 to provide

the following background corrected fluorescence intensities:

C = 21679 AU; W = 16891 AU

A standard of known amino acid concentration was used to convert between the measured signal of the label of the C amino acid type and the amino acid concentration of the C amino acid type, and a standard of known amino acid concentration was used to convert between the measured signal of the label of the C amino acid type and the amino acid concentration of the C amino acid type. A standard solution containing 6 μM C amino acid concentration (ovalbumin) had a fluorescence-intensity of 1322 AU, so the conversion between fluorescence intensity (AU) and μM amino acid concentration is given by $6\,uM/_{1322.4\,AU} = 4.54 \times 10^{-3}\,uM/_{AU}$. A standard solution containing 3 μM W amino acid concentration (ovalbumin) had a fluorescence-intensity of 6888.7 AU, so the conversion between fluorescence intensity (AU) and μM amino acid concentration is given by $3\,uM/_{6888.7\,\mathrm{AU}} = 4.35 \times 10^{-4}\,uM/_{AU}$.

The fluorescence intensities measured for the sample were converted into the following amino acid concentrations:

16.39 μM C; 2.45 μM W.

The sample point is (16.39, 2.45).

The C amino acid type is amino acid type 1 ($a_1$). The W amino acid type is amino acid type 2 ($a_2$).

Vector equation 1 was used to provide the amino acid concentrations, defined as a function of the protein concentration, t, for a variety of proteins of interest, each with a reference line, R.

**Table 9: Results of Example 6**

| ID | $a_1$ | $a_2$ | $a_1$t-$S_1$ | $a_2$t-$S_2$ | sol t | $Q_1$ | $Q_2$ | D |
|---|---|---|---|---|---|---|---|---|
| ALP | 5 | 4 | 5t - 16.39 | 4t - 2.45 | 2.24 | 11.2 | 9 | 8.3 |
| Avidin | 8 | 16 | 8t - 16.39 | 16t - 2.45 | 0.53 | 4.26 | 8.5 | 14 |
| BSA | 35 | 2 | 35t - 16.39 | 2t - 2.45 | 0.47 | 16.5 | 0.9 | 1.5 |
| Enterokinase | 35 | 19 | 35t - 16.39 | 19t - 2.45 | 0.39 | 13.7 | 7.4 | 5.7 |
| G6P DH | 8 | 13 | 8t - 16.39 | 13t - 2.45 | 0.7 | 5.6 | 9.1 | 13 |
| KTI | 4 | 2 | 4t - 16.39 | 2t - 2.45 | 3.52 | 14.1 | 7 | 5.1 |
| Lysostaphin | 0 | 8 | 0t - 16.39 | 8t - 2.45 | 0.31 | 0 | 2.5 | 16 |
| LYZ | 8 | 6 | 8t - 16.39 | 6t - 2.45 | 1.46 | 11.7 | 8.8 | 7.9 |
| Renin | 6 | 3 | 6t - 16.39 | 3t - 2.45 | 2.35 | 14.1 | 7 | 5.1 |
| ß-Casein | 0 | 1 | 0t - 16.39 | 1t - 2.45 | 2.45 | 0 | 2.5 | 16 |
| α2M | 25 | 11 | 25t - 16.39 | 11t - 2.45 | 0.59 | 14.6 | 6.4 | 4.4 |
| αsyn | 0 | 0 | 0t - 16.39 | 0t - 2.45 | 0 | 0 | 0 | 17 |
| βlac | 5 | 2 | 5t - 16.39 | 2t - 2.45 | 3 | 15 | 6 | 3.8 |

The comparison steps described in Example 1 were performed, resulting in the provided perpendicular distance values (D).

It was suspected that the sample contained 2 proteins of interest, so the error margin was set as the second smallest value of the perpendicular distances, 3.8, such that only for BSA and βLac is $D \leq \varepsilon$.

2 proteins of interest were identified as being present in the mixture, and a comparison of their perpendicular distance values identified the proportions of the components within the mixture. Specifically, the principle is that the measurement for the sample is the mean of the expected values for the pure components (e.g. pure proteins) comprising the mixture. The closer the distance, the larger the proportion of the pure component (e.g. pure protein) within the mixture, therefore the perpendicular distances are inverted. The perpendicular distances must also be normalized so that the proportion of all components within the mixture sum to 1. Therefore, to calculate the proportion of each component within the mixture:

- The distances are inverse normalized by dividing the largest distance by every other distance.
- Then the inverse normalized distances are summed.

- Finally, the fraction of each component within the mixture is calculated by dividing its inverse normalized distance by the sum of all the inverse normalized distances.

This is applied below:

| ID | sol t | D | $D_{max}$ | $D_{max}/D$ | Proportion | t mix |
|---|---|---|---|---|---|---|
| BSA | 0.47 | 1.51 | 3.81 | 2.52 | 0.72 | 0.34 |
| βlac | 3 | 3.81 | 3.81 | 1 | 0.28 | 0.84 |

Then, the proportion of each component within the mixture was multiplied by the determined protein concentration (sol t) of the pure component, to find the concentration of that component within the mixture.
The sample was identified as containing a mixture of BSA and βLac, comprising 0.34 μM BSA and 0.84 μM βLac.

**Example 7 - Determining the presence and/or concentration and/or amount of a protein of interest within the entire human plasma proteome (> 3000 proteins of interest) using two amino acid types**

A sample was obtained from human plasma. The identity and concentration of the sample were unknown. The C ($a_1$) and K ($a_2$) amino acid types were labelled as outlined in Example 1.
The raw fluorescence intensities for the C and K amino acid types were background corrected as described in Example 1. The background corrected fluorescence intensities measured were:
$C_T$: 227 AU; K: 1563 AU
The background corrected fluorescence intensities were converted to amino acid concentrations as described in Example 1. The amino acid concentrations calculated were:
$C_T$: 160 nM; K: 1125 nM
Using the methods described herein, a reference database was generated for all proteins within the 3263-protein human plasma proteome.
It was not practical to include the entire database in the worked example, but representative rows, every $50^{th}$ row, are shown below in Table 10.
The distance between the sample point and the reference line for each protein of interest was calculated, and its corresponding protein concentration found. Each protein concentration was compared to the upper (UB) and lower (LB) concentration range publicly available for the protein of interest. This concentration range was accessed from the publicly available Protein Atlas database. The lower bound of the concentration range was set at 0.9 times the provided concentration, and the upper bound of the concentration range was set at 1.1 times the provided concentration. As before, the methods of the invention were applied, and the protein concentration, t, of each protein of interest (if present in the sample) was determined using the perpendicular distance approach described. If LB < t < UB, the row of the reference database received a 1 as a logical output. The LB, t, and UB columns within the matrix are highlighted. In the representative row representation, none of the proteins of interest were within their concentration bounds (so there was no 1 logical output).

Table 10: Results of Example 7

| ID | $a_1$ | $a_2$ | $a_1t$ | $a_2t$ | $a_1t-S_1$ | $a_2t-S_2$ | sol. t | Conc (ug/L) | MW | Conc (uM) | Conc (nM) | Conc LB (nM) | Conc UB (nM) | Q1 | Q2 | D | Is sol. t within bounds? |
|---|---|---|---|---|---|---|---|---|---|---|---|---|---|---|---|---|---|
| SERPINA6 | 3 | 17 | 3t | 17t | 3t - 165 | 17t - 1287 | 65.78859 | 59000 | 45081 | 1.309 | 1308.7577 | 1177.8819 | 1439.6334 | 197 | 1118 | 38 | 0 |
| LGALS3BP | 16 | 19 | 16t | 19t | 16t - 165 | 19t - 1287 | 38.79254 | 11000 | 65245 | 0.169 | 168.5945 | 151.7350 | 185.4539 | 621 | 737 | 602 | 0 |
| LYVE1 | 10 | 25 | 10t | 25t | 10t - 165 | 25t - 1287 | 41 | 3000 | 35167 | 0.085 | 85.3083 | 76.7774 | 93.8391 | 410 | 1025 | 269 | 0 |
| ALDOA | 8 | 26 | 8t | 26t | 8t - 165 | 26t - 1287 | 41.25676 | 1200 | 39368 | 0.030 | 30.4818 | 27.4336 | 33.5300 | 330 | 1073 | 178 | 0 |
| PKM | 10 | 37 | 10t | 37t | 10t - 165 | 37t - 1287 | 29.42478 | 540 | 57859 | 0.009 | 9.3330 | 8.3997 | 10.2663 | 294 | 1089 | 139 | 0 |
| KRT7 | 1 | 25 | 1t | 25t | 1t - 165 | 25t - 1287 | 45.18371 | 350 | 51319 | 0.007 | 6.8201 | 6.1381 | 7.5021 | 45 | 1130 | 115 | 0 |
| RSU1 | 0 | 22 | 0t | 22t | 0t - 165 | 22t - 1287 | 51.13636 | 210 | 31498 | 0.007 | 6.6670 | 6.0003 | 7.3337 | 0 | 1125 | 160 | 0 |
| LAMB1 | 127 | 85 | 127t | 85t | 127t - 165 | 85t - 1287 | 4.964674 | 140 | 197780 | 0.001 | 0.7079 | 0.6371 | 0.7786 | 631 | 422 | 846 | 0 |
| PTPRF | 21 | 77 | 21t | 77t | 21t - 165 | 77t - 1287 | 14.12637 | 95 | 212599 | 0.000 | 0.4469 | 0.4022 | 0.4915 | 297 | 1088 | 142 | 0 |
| SFTPB | 25 | 11 | 25t | 11t | 25t - 165 | 11t - 1287 | 21.9504 | 71 | 42060 | 0.002 | 1.6880 | 1.5192 | 1.8569 | 549 | 241 | 965 | 0 |
| CHIT1 | 10 | 26 | 10t | 26t | 10t - 165 | 26t - 1287 | 39.75515 | 58 | 51614 | 0.001 | 1.1237 | 1.0114 | 1.2361 | 398 | 1034 | 255 | 0 |
| RAC1 | 7 | 17 | 7t | 17t | 7t - 165 | 17t - 1287 | 59.89645 | 44 | 21421 | 0.002 | 2.0540 | 1.8486 | 2.2594 | 419 | 1018 | 280 | 0 |
| A2ML1 | 25 | 78 | 25t | 78t | 25t - 165 | 78t - 1287 | 13.67566 | 34 | 160894 | 0.000 | 0.2113 | 0.1902 | 0.2325 | 342 | 1067 | 191 | 0 |
| HLA-F | 4 | 7 | 4t | 7t | 4t - 165 | 7t - 1287 | 131 | 27 | 39011 | 0.001 | 0.6921 | 0.6229 | 0.7613 | 524 | 917 | 419 | 0 |

(continued)

| ID | $a_1$ | $a_2$ | $a_1t$ | $a_2t$ | $a_1t-S_1$ | $a_2t-S_2$ | sol. t | Conc (ug/L) | MW | Conc (uM) | Conc (nM) | Conc LB (nM) | Conc UB (nM) | Q1 | Q2 | D | Is sol. t within bounds? |
|---|---|---|---|---|---|---|---|---|---|---|---|---|---|---|---|---|---|
| COL1A2 | 9 | 50 | 9t | 50t | 9t - 165 | 50t - 1287 | 22.3518 | 21 | 129144 | 0.000 | 0.1626 | 0.1463 | 0.1789 | 201 | 1118 | 42 | 0 |
| DBN1 | 8 | 22 | 8t | 22t | 8t - 165 | 22t - 1287 | 47.5 | 17 | 71338 | 0.000 | 0.2383 | 0.2145 | 0.2621 | 380 | 1045 | 234 | 0 |
| RAB10 | 4 | 21 | 4t | 21t | 4t - 165 | 21t - 1287 | 53.09628 | 14 | 22511 | 0.001 | 0.6219 | 0.5597 | 0.6841 | 212 | 1115 | 53 | 0 |
| CILP | 40 | 59 | 40t | 59t | 40t - 165 | 59t - 1287 | 14.32297 | 11 | 132390 | 0.000 | 0.0831 | 0.0748 | 0.0914 | 573 | 845 | 499 | 0 |
| PHGDH | 13 | 26 | 13t | 26t | 13t - 165 | 26t - 1287 | 37.07692 | 9 | 56574 | 0.000 | 0.1591 | 0.1432 | 0.1750 | 482 | 964 | 360 | 0 |
| CES2 | 6 | 19 | 6t | 19t | 6t - 165 | 19t - 1287 | 56.25945 | 7.5 | 61725 | 0.000 | 0.1215 | 0.1094 | 0.1337 | 338 | 1069 | 186 | 0 |
| ETFA | 6 | 26 | 6t | 26t | 6t - 165 | 26t - 1287 | 42.42978 | 6.3 | 35032 | 0.000 | 0.1798 | 0.1619 | 0.1978 | 255 | 1103 | 97 | 0 |
| MGAT2 | 12 | 27 | 12t | 27t | 12t - 165 | 27t - 1287 | 36.99313 | 5 | 51482 | 0.000 | 0.0971 | 0.0874 | 0.1068 | 444 | 999 | 311 | 0 |
| IL27RA | 16 | 18 | 16t | 18t | 16t - 165 | 18t - 1287 | 39.32759 | 4.1 | 69381 | 0.000 | 0.0591 | 0.0532 | 0.0650 | 629 | 708 | 628 | 0 |
| RABSC | 4 | 13 | 4t | 13t | 4t - 165 | 13t - 1287 | 82.51351 | 3.6 | 23452 | 0.000 | 0.1535 | 0.1382 | 0.1689 | 330 | 1073 | 178 | 0 |
| NUTF2 | 3 | 6 | 3t | 6t | 3t - 165 | 6t - 1287 | 160.6667 | 3 | 14459 | 0.000 | 0.2075 | 0.1867 | 0.2282 | 482 | 964 | 360 | 0 |
| CYFIP1 | 29 | 71 | 29t | 71t | 29t - 165 | 71t - 1287 | 14.36841 | 2.6 | 144990 | 0.000 | 0.0179 | 0.0161 | 0.0197 | 417 | 1020 | 277 | 0 |
| PI3 | 8 | 11 | 8t | 11t | 8t - 165 | 11t - 1287 | 73.81081 | 2.1 | 12253 | 0.000 | 0.1714 | 0.1543 | 0.1885 | 590 | 812 | 532 | 0 |
| ATP5IF1 | 0 | 10 | 0t | 10t | 0t - 165 | 10t - 1287 | 112.5 | 1.8 | 12233 | 0.000 | 0.1471 | 0.1324 | 0.1619 | 0 | 1125 | 160 | 0 |

(continued)

| ID | $a_1$ | $a_2$ | $a_1t$ | $a_2t$ | $a_1t$-$S_1$ | $a_2t$-$S_2$ | sol. t | Conc (ug/L) | MW | Conc (uM) | Conc (nM) | Conc LB (nM) | Conc UB (nM) | Q1 | Q2 | D | Is sol. t within bounds? |
|---|---|---|---|---|---|---|---|---|---|---|---|---|---|---|---|---|---|
| ECHS1 | 8 | 24 | 8t | 24t | 8t - 165 | 24t - 1287 | 44.1875 | 1.5 | 31345 | 0.000 | 0.0479 | 0.0431 | 0.0526 | 354 | 1061 | 204 | 0 |
| P2RX1 | 11 | 26 | 11t | 26t | 11t - 165 | 26t - 1287 | 38.90841 | 1.3 | 44920 | 0.000 | 0.0289 | 0.0260 | 0.0318 | 428 | 1012 | 291 | 0 |
| TRAP1 | 5 | 44 | 5t | 44t | 5t - 165 | 44t - 1287 | 25.65018 | 1.1 | 80004 | 0.000 | 0.0137 | 0.0124 | 0.0151 | 128 | 1129 | 32 | 0 |
| SEC22B | 4 | 13 | 4t | 13t | 4t - 165 | 13t - 1287 | 82.51351 | 0.98 | 24561 | 0.000 | 0.0399 | 0.0359 | 0.0439 | 330 | 1073 | 178 | 0 |
| ACAT1 | 5 | 30 | 5t | 30t | 5t - 165 | 30t - 1287 | 37.35135 | 0.87 | 45138 | 0.000 | 0.0193 | 0.0173 | 0.0212 | 187 | 1121 | 27 | 0 |
| DOK3 | 13 | 17 | 13t | 17t | 13t - 165 | 17t - 1287 | 46.29913 | 0.75 | 53218 | 0.000 | 0.0141 | 0.0127 | 0.0155 | 602 | 787 | 556 | 0 |
| ADA | 5 | 24 | 5t | 24t | 5t - 165 | 24t - 1287 | 46.25624 | 0.65 | 40711 | 0.000 | 0.0160 | 0.0144 | 0.0176 | 231 | 1110 | 73 | 0 |
| SERPINA12 | 2 | 40 | 2t | 40t | 2t - 165 | 40t - 1287 | 28.25436 | 0.57 | 47112 | 0.000 | 0.0121 | 0.0109 | 0.0133 | 57 | 1130 | 104 | 0 |
| SNRPD1 | 0 | 10 | 0t | 10t | 0t - 165 | 10t - 1287 | 112.5 | 0.49 | 13264 | 0.000 | 0.0369 | 0.0332 | 0.0406 | 0 | 1125 | 160 | 0 |
| NAMPT | 5 | 47 | 5t | 47t | 5t - 165 | 47t - 1287 | 24.02641 | 0.41 | 55448 | 0.000 | 0.0074 | 0.0067 | 0.0081 | 120 | 1129 | 40 | 0 |
| CMIP | 25 | 44 | 25t | 44t | 25t - 165 | 44t - 1287 | 20.89028 | 0.36 | 86216 | 0.000 | 0.0042 | 0.0038 | 0.0046 | 522 | 919 | 417 | 0 |
| HDGF | 2 | 27 | 2t | 27t | 2t - 165 | 27t - 1287 | 41.87585 | 0.3 | 26754 | 0.000 | 0.0112 | 0.0101 | 0.0123 | 84 | 1131 | 76 | 0 |
| CRELD2 | 38 | 15 | 38t | 15t | 38t - 165 | 15t - 1287 | 13.75374 | 0.27 | 38142 | 0.000 | 0.0071 | 0.0064 | 0.0078 | 523 | 206 | 988 | 0 |
| MAVS | 10 | 14 | 10t | 14t | 10t - 165 | 14t - 1287 | 58.61486 | 0.23 | 56454 | 0.000 | 0.0041 | 0.0037 | 0.0045 | 586 | 821 | 524 | 0 |

(continued)

| ID | $a_1$ | $a_2$ | $a_1t$ | $a_2t$ | $a_1t-S_1$ | $a_2t-S_2$ | sol. t | Conc (ug/L) | MW | Conc (uM) | Conc (nM) | Conc LB (nM) | Conc UB (nM) | Q1 | Q2 | D | Is sol. t within bounds? |
|---|---|---|---|---|---|---|---|---|---|---|---|---|---|---|---|---|---|
| SERPINI1 | 0 | 22 | 0t | 22t | 0t - 165 | 22t - 1287 | 51.13636 | 0.2 | 46366 | 0.000 | 0.0043 | 0.0039 | 0.0047 | 0 | 1125 | 160 | 0 |
| GPC4 | 19 | 37 | 19t | 37t | 19t - 165 | 37t - 1287 | 25.81792 | 0.17 | 62330 | 0.000 | 0.0027 | 0.0025 | 0.0030 | 491 | 955 | 372 | 0 |
| PNPO | 6 | 14 | 6t | 14t | 6t - 165 | 14t - 1287 | 72.02586 | 0.15 | 29949 | 0.000 | 0.0050 | 0.0045 | 0.0055 | 432 | 1008 | 296 | 0 |
| HEBP1 | 2 | 16 | 2t | 16t | 2t - 165 | 16t - 1287 | 70.46154 | 0.13 | 21069 | 0.000 | 0.0062 | 0.0056 | 0.0068 | 141 | 1127 | 19 | 0 |
| TANGO2 | 5 | 13 | 5t | 13t | 5t - 165 | 13t - 1287 | 79.51031 | 0.12 | 30897 | 0.000 | 0.0039 | 0.0035 | 0.0043 | 398 | 1034 | 255 | 0 |
| ITGA4 | 25 | 57 | 25t | 57t | 25t - 165 | 57t - 1287 | 17.58518 | 0.1 | 114748 | 0.000 | 0.0009 | 0.0008 | 0.0010 | 440 | 1002 | 305 | 0 |
| HEPACAM | 9 | 24 | 9t | 24t | 9t - 165 | 24t - 1287 | 43.28767 | 0.089 | 51339 | 0.000 | 0.0017 | 0.0016 | 0.0019 | 390 | 1039 | 245 | 0 |
| ERO1A | 15 | 29 | 15t | 29t | 15t - 165 | 29t - 1287 | 32.85647 | 0.075 | 54322 | 0.000 | 0.0014 | 0.0012 | 0.0015 | 493 | 953 | 375 | 0 |
| RPS3A | 4 | 38 | 4t | 38t | 4t - 165 | 38t - 1287 | 29.71918 | 0.063 | 29905 | 0.000 | 0.0021 | 0.0019 | 0.0023 | 119 | 1129 | 41 | 0 |
| CPNE3 | 15 | 37 | 15t | 37t | 15t - 165 | 37t - 1287 | 27.6192 | 0.052 | 60051 | 0.000 | 0.0009 | 0.0008 | 0.0010 | 414 | 1022 | 274 | 0 |
| IST1 | 3 | 25 | 3t | 25t | 3t - 165 | 25t - 1287 | 45.1183 | 0.045 | 39698 | 0.000 | 0.0011 | 0.0010 | 0.0012 | 135 | 1128 | 25 | 0 |
| KPNA2 | 6 | 28 | 6t | 28t | 6t - 165 | 28t - 1287 | 39.58537 | 0.038 | 57785 | 0.000 | 0.0007 | 0.0006 | 0.0007 | 238 | 1108 | 79 | 0 |
| XYLT1 | 15 | 51 | 15t | 51t | 15t - 165 | 51t - 1287 | 21.1518 | 0.03 | 107428 | 0.000 | 0.0003 | 0.0003 | 0.0003 | 317 | 1079 | 164 | 0 |
| FRZB | 18 | 22 | 18t | 22t | 18t - 165 | 22t - 1287 | 34.19554 | 0.027 | 36206 | 0.000 | 0.0007 | 0.0007 | 0.0008 | 616 | 752 | 589 | 0 |

(continued)

| ID | $a_1$ | $a_2$ | $a_1t$ | $a_2t$ | $a_1t-S_1$ | $a_2t-S_2$ | sol. t | Conc (ug/L) | MW | Conc (uM) | Conc (nM) | Conc LB (nM) | Conc UB (nM) | Q1 | Q2 | D | Is sol. t within bounds? |
|---|---|---|---|---|---|---|---|---|---|---|---|---|---|---|---|---|---|
| SNRPA | 0 | 22 | 0t | 22t | 0t - 165 | 22t - 1287 | 51.13636 | 0.023 | 31237 | 0.000 | 0.0007 | 0.0007 | 0.0008 | 0 | 1125 | 160 | 0 |
| SF3A1 | 1 | 59 | 1t | 59t | 1t - 165 | 59t - 1287 | 19.10827 | 0.019 | 88769 | 0.000 | 0.0002 | 0.0002 | 0.0002 | 19 | 1127 | 141 | 0 |
| PPCS | 0 | 12 | 0t | 12t | 0t - 165 | 12t - 1287 | 93.75 | 0.016 | 33960 | 0.000 | 0.0005 | 0.0004 | 0.0005 | 0 | 1125 | 160 | 0 |
| SLAIN2 | 4 | 26 | 4t | 26t | 4t - 165 | 26t - 1287 | 43.19364 | 0.013 | 62462 | 0.000 | 0.0002 | 0.0002 | 0.0002 | 173 | 1123 | 13 | 0 |
| METAP1 | 16 | 22 | 16t | 22t | 16t - 165 | 22t - 1287 | 36.90541 | 0.011 | 43158 | 0.000 | 0.0003 | 0.0002 | 0.0003 | 590 | 812 | 532 | 0 |
| MYO1F | 13 | 68 | 13t | 68t | 13t - 165 | 68t - 1287 | 16.39474 | 0.0088 | 124680 | 0.000 | 0.0001 | 0.0001 | 0.0001 | 213 | 1115 | 54 | 0 |
| PLAA | 27 | 44 | 27t | 44t | 27t - 165 | 44t - 1287 | 20.19512 | 0.0053 | 87042 | 0.000 | 0.0001 | 0.0001 | 0.0001 | 545 | 889 | 452 | 0 |

Then, the matrix was filtered to show only the rows for which LB < t < UB. The calculated distances were compared to the error margin.

Table 11: Continued results of Example 7

| ID | $a_1$ | $a_2$ | $a_1t$ | $a_2t$ | $a_1t-S_1$ | $a_2t-S_2$ | sol. t | Conc (ug/L) | MW | Conc (uM) | Conc (nM) | Conc LB (nM) | Conc UB (nM) | $Q_1$ | $Q_2$ | D | Is sol. t within bounds? | D within bounds |
|---|---|---|---|---|---|---|---|---|---|---|---|---|---|---|---|---|---|---|
| ACTBL2 | 6 | 18 | 6t | 18t | 6t - 165 | 18t - 1287 | 58.92 | 2600 | 41948 | 0.062 | 61.98 | 55.78 | 68.18 | 353.5 | 1060.5 | 204.0 | 1 | 203.97 |
| SERPINA5 | 2 | 24 | 2t | 24t | 2t - 165 | 24t - 1287 | 47.10 | 2300 | 45614 | 0.050 | 50.42 | 45.38 | 55.47 | 94.2 | 1130.5 | 66.0 | 1 | 66.02 |
| PIGR | 21 | 42 | 21t | 42t | 21t - 165 | 42t - 1287 | 22.95 | 1900 | 83175 | 0.023 | 22.84 | 20.56 | 25.13 | 482.0 | 964.0 | 360.0 | 1 | 360.01 |
| VCL | 10 | 78 | 10t | 78t | 10t - 165 | 78t - 1287 | 14.45 | 1800 | 123635 | 0.015 | 14.56 | 13.10 | 16.01 | 144.5 | 1127.0 | 15.6 | 1 | 15.64 |
| MASP1 | 29 | 37 | 29t | 37t | 29t - 165 | 37t - 1287 | 20.93 | 1800 | 79143 | 0.023 | 22.74 | 20.47 | 25.02 | 607.1 | 774.6 | 568.1 | 1 | 568.06 |
| IGF2 | 8 | 7 | 8t | 7t | 8t - 165 | 7t - 1287 | 81.02 | 1700 | 20113 | 0.085 | 84.52 | 76.07 | 92.97 | 648.1 | 567.1 | 741.3 | 1 | 741.29 |
| POTEJ | 25 | 81 | 25t | 81t | 25t - 165 | 81t - 1287 | 13.24 | 1700 | 117236 | 0.015 | 14.50 | 13.05 | 15.95 | 330.9 | 1072.2 | 178.9 | 1 | 178.90 |
| RNASE1 | 8 | 9 | 8t | 9t | 8t - 165 | 9t - 1287 | 78.66 | 1500 | 17621 | 0.085 | 85.13 | 76.61 | 93.64 | 629.2 | 707.9 | 627.8 | 1 | 627.82 |
| APOC4 | 6 | 8 | 6t | 8t | 6t - 165 | 8t - 1287 | 99.60 | 1500 | 14533 | 0.103 | 103.21 | 92.89 | 113.53 | 597.6 | 796.8 | 547.0 | 1 | 547.00 |
| PF4V1 | 4 | 7 | 4t | 7t | 4t - 165 | 7t - 1287 | 131.00 | 1400 | 11537 | 0.121 | 121.35 | 109.21 | 133.48 | 524.0 | 917.0 | 419.2 | 1 | 419.24 |
| S100A9 | 1 | 11 | 1t | 11t | 1t - 165 | 11t - 1287 | 102.75 | 1400 | 13225 | 0.106 | 105.86 | 95.28 | 116.45 | 102.7 | 1130.2 | 57.5 | 1 | 57.49 |
| SELP | 67 | 31 | 67t | 31t | 67t - 165 | 31t - 1287 | 8.37 | 830 | 90714 | 0.009 | 9.15 | 8.23 | 10.06 | 560.5 | 259.3 | 953.8 | 1 | 953.82 |

The number of proteins of interest contained within the sample was not suspected, so the error margin was supplied according to Eq. 7 as 38.93 based on a tolerance value of 0.03. Only VCL had $D \leq \varepsilon$, therefore the protein of interest VCL was identified within the sample (from a reference set of 3263 proteins, peptides, oligopeptides, polypeptides, and protein complexes of interest) at the protein concentration of 14.45 nM.

This strategy eliminates any examples of a reference line (R) referring to more than one protein of interest, by comparing the protein concentration determined by the methods of the invention (if the protein of interest is present in the sample) to a rich data set varying by over 10 orders of magnitude.

**Example 8 - Determining the presence and/or concentration and/or amount of a proteome of interest (SARS-CoV-2 or Influenza A) in a sample**

Three patients present with a dry cough, tiredness, muscle aches, and a fever. There symptoms are suggestive of either common influenza or the early stages of coronavirus. The goal is to rapidly determine what infection, if any, each patient has, and to determine the abundance of the pathogen in each individual to permit an assessment of how aggressively each individual patient may spread an infection and/or to predict the host's response to the virus so that appropriate treatment can be prescribed.

A sample from each patient is collected via methods known in the art. This can include a blood sample, nasal swab, nasopharyngeal aspirate, or lower respiratory mucus aspirate sample. If appropriate, the sample is diluted into a buffer or carrier solution, and is separated by methods known in the art like centrifugation, ultracentrifugation, agarose gel electrophoresis, or column chromatography to isolate the fraction corresponding to particles the size of viruses (around 80-350 nm in diameter). The sample can also be concentrated using methods known in the art such as lyophilization or cellulose membrane concentrators. The sample's identity is unknown. The sample's (protein) concentration is unknown and the (protein) concentration contained within any of the dilutions is unknown.

This viral fraction is lysed via methods known in the art, such as with the use of sodium dodecyl sulphate (SDS) +/-EDTA.

2 amino acid types; K and C, or, C and W, or, K and W are fluorescently labelled using a fluorogenic dye outlined in Example 1.

All of the amino acids of each of the 2 amino acids types; K and C, or, C and W, or, K and W are fluorescently labelled using a fluorogenic dye outlined in Example 1. The diagnostic result of each of these combinations of amino acid types is shown in this example.

The fluorescence intensity of each fluorogenic dye which has labelled each amino acid type is measured using a plate reader and background corrected as outlined in Example 1. The background corrected fluorescence intensity (FI) values are:

C and K

| | FI (AU) C | FI (AU) K |
|---|---|---|
| Patient 1 | 203.4 | 392.4 |
| Patient 2 | 116.1 | 222.4 |

(continued)

| | FI (AU) C | FI (AU) K |
|---|---|---|
| Patient 3 | 118.8 | 381.5 |

<u>C and W</u>

| | FI (AU) C | FI (AU) W |
|---|---|---|
| Patient 1 | 203.4 | 10109.7 |
| Patient 2 | 116.1 | 9980.3 |
| Patient 3 | 118.8 | 10215.6 |

<u>K and W</u>

| | FI (AU) K | FI (AU) W |
|---|---|---|
| Patient 1 | 384.4 | 10109.7 |
| Patient 2 | 217.8 | 9980.3 |
| Patient 3 | 373.6 | 10215.6 |

The fluorescence intensity of the two amino acid types (K and C, C and W, or K and W) in each patient's sample is determined from the fluorescence intensity using a linear fit. The linear fit is calculated based on a calibration curve which relates known concentrations of the labelled amino acid to fluorescence intensity, as outlined in Example 1.

For example, as outlined in Example 1 and specifically Eq. 4, the C amino acid concentration for Patient 1 is determined by dividing the measured C FI for Patient 1 by $705.1\ ^{AU}/_{\mu M}$.

This provides a C amino acid concentration for Patient 1 of 0.288 μM which is 288 nM.

As another example, Patient 1's K amino acid concentration is determined by multiplying Patient 1's background corrected fluorescence intensity by the inverse of the calibration factor provided in example 1,

$$f_k^{-1} = 1.40 \times 10^{-3}\ ^{uM}/_{AU}$$

Patient 1's K amino acid concentration is therefore 0.545 μM which is 545 nM.

As another example, patient 1's W amino acid concentration is determined by subtracting 9859 AU from the measured fluorescence intensity, and dividing by 2362 $^{AU}/_{\mu M}$, as provided by Eq. 6,

$$\text{Eq. 6: } W\ A.A.\,concentration\ (\mu M) = \frac{W\ Flaurescence\ Intensity\ (AU) - 9859\ AU}{2362\ ^{AU}/_{\mu M}}$$

This gives .106 μM W amino acid concentration which is 106 nM W amino acid concentration.

The amino acid concentrations for Patient 1, 2, and 3 determined in this way for each of the three combinations of two labelled amino acid types are shown below.

<u>C and K</u>

| | C amino acid concentration (nM) | K amino acid concentration (nM) |
|---|---|---|
| Patient 1 | 288.512 | 545.13 |
| Patient 2 | 164.64 | 308.907 |
| Patient 3 | 168.4428 | 529.92 |

C and W

| | C amino acid concentration (nM) | W amino acid concentration (nM) |
|---|---|---|
| Patient 1 | 288.512 | 106.126 |
| Patient 2 | 164.64 | 51.3695 |
| Patient 3 | 168.4428 | 150.9835 |

K and W

| | K amino acid concentration (nM) | W amino acid concentration (nM) |
|---|---|---|
| Patient 1 | 545.13 | 106.126 |
| Patient 2 | 308.907 | 51.3695 |
| Patient 3 | 529.92 | 150.9835 |

Based on the publicly available protein sequences which make up the Influenza A H1N1 and SARS-CoV-2 (a.k.a. 2019-nCoV) proteomes, it is determined that the weighted mean number of K and C amino acids in the Influenza A proteome is 24.00 K and 7.18 C, whereas the weighted mean number of K and C amino acids in the SARS-CoV-2 proteome is 60.57 K and 31.36 C. The weights were provided using equation 12:

$$w_n = \sum_{i=1}^{c}\left(a_{n,i} \times \frac{1}{c}\right)$$

wherein $w_n$ is the weighted mean number of amino acids of amino acid type n in the proteome of interest, c is the number of proteins in the proteome of interest, and $a_{n,i}$ is the number of amino acids of amino acid type n in protein i in the proteome of interest. A linear combination of the results is taken for proteins i through c of the proteome of interest. In this embodiment, all proteins within the proteome of interest are taken as having equivalent expression or proportion within the proteome of interest, so the weights for each protein of interest within the proteome of interest are equal.

In an alternative embodiment it is determined that the average number of C and W amino acids in the Influenza A proteome is 7.18 C and 6.91 W, whereas the average number of C and W amino acids in the SARS-CoV-2 proteome is 31.36 C and 11.29 W. In an alternative embodiment, it is determined that the average number of K and W amino acids in the Influenza A proteome is 24.00 K and 6.91 W, whereas the average number of K and W amino acids in the SARS-CoV-2 proteome is 60.57 K and 11.29 W.

These values provide w1 and w2 in vector function 2 for both proteomes of interest, and for all combinations of 2 amino acid types.

For example, vector function 2 for the SARS-CoV-2 proteome of interest, where the K amino acid type is w1 and the W amino acid type is w2, is

$$p_{SARS-CoV-2}(t) = \langle 0, 0\rangle + \langle 60.57t, 11.29t\rangle, \forall t \geq 0$$

Whereas vector function 2 for the Influenza A proteome of interest is

$$p_{InfluenzaA}(t) = \langle 0, 0\rangle + \langle 24.00t, 6.91t\rangle, \forall t \geq 0$$

These vector functions define the reference (vector) lines for the SARS-CoV-2 and Influenza A H1N1 proteomes of interest. The approach described in Test 2 of Example 1 is performed, wherein the perpendicular distance from the sample point to the reference line is determined via the dot product, revealing a closest point on the reference line and a concentration on the reference line. The SARS-CoV-2 and/or Influenza A H1N1 proteomes of interest are identified as being present in the patient sample if the perpendicular distance is less than or equal to an error margin.

The results for each patient and each embodiment (C and K, C and W, or K and W) are provided below.

C and K

| | | | | | Patient 1 | | | | | |
|---|---|---|---|---|---|---|---|---|---|---|
| **Respiratory Virus** | $w_1$ | $w_2$ | $w_1t$ | $w_2t$ | $w_1t - S_1$ | $w_2t - S_2$ | t | $Q_1$ | $Q_2$ | D |
| SARS-CoV-2 | 31.36 | 60.57 | 31.36t | 60.57t | 31.36t - 288.51 | 60.57t - 545.13 | 9.04 | 283.57 | 547.69 | 5.57 |
| Influenza A | 7.18 | 24.00 | 7.18t | 24t | 7.18t - 288.51 | 24t - 545.13 | 24.15 | 173.39 | 579.57 | 120.17 |

| | | | | | Patient 2 | | | | | |
|---|---|---|---|---|---|---|---|---|---|---|
| **Respiratory Virus** | $w_1$ | $w_2$ | $w_1t$ | $w_2t$ | $w_1t - S_1$ | $w_2t - S_2$ | t | $Q_1$ | $Q_2$ | D |
| SARS-CoV-2 | 31.36 | 60.57 | 31.36t | 60.57t | 31.36t - 164.64 | 60.57t - 308.91 | 5.13 | 160.93 | 310.83 | 4.18 |
| Influenza A | 7.18 | 24.00 | 7.18t | 24t | 7.18t - 164.64 | 24t - 308.91 | 13.70 | 98.35 | 328.74 | 69.20 |

| | | | | | Patient 3 | | | | | |
|---|---|---|---|---|---|---|---|---|---|---|
| **Respiratory Virus** | $w_1$ | $w_2$ | $w_1t$ | $w_2t$ | $w_1t$ - S1 | $w_2t - S_2$ | t | $Q_1$ | $Q_2$ | D |
| SARS-CoV-2 | 31.36 | 60.57 | 31.36t | 60.57t | 31.36t - 168.44 | 60.57t - 529.92 | 8.03 | 251.97 | 486.67 | 94.06 |
| Influenza A | 7.18 | 24.00 | 7.18t | 24t | 7.18t - 168.44 | 24t - 529.92 | 22.19 | 159.35 | 532.64 | 9.49 |

C and W

| | | | | | Patient 1 | | | | | |
|---|---|---|---|---|---|---|---|---|---|---|
| **Respiratory Virus** | $w_1$ | $w_2$ | $w_1t$ | $w_2t$ | $w_1t - S_1$ | $w_2t - S_2$ | t | $Q_1$ | $Q_2$ | D |
| SARS-CoV-2 | 31.36 | 11.29 | 31.36t | 11.29t | 31.36t - 288.51 | 11.29t - 106.13 | 9.22 | 289.23 | 104.13 | 2.12 |
| Influenza A | 7.18 | 6.91 | 7.18t | 6.91t | 7.18t - 288.51 | 6.91t - 106.13 | 28.25 | 202.81 | 195.18 | 123.60 |

| | | | | | Patient 2 | | | | | |
|---|---|---|---|---|---|---|---|---|---|---|
| **Respiratory Virus** | $w_1$ | $w_2$ | $w_1t$ | $w_2t$ | $w_1t - S_1$ | $w_2t - S_2$ | t | $Q_1$ | $Q_2$ | D |
| SARS-CoV-2 | 31.36 | 11.29 | 31.36t | 11.29t | 31.36t - 164.64 | 11.29t - 51.37 | 5.17 | 162.12 | 58.37 | 7.44 |
| Influenza A | 7.18 | 6.91 | 7.18t | 6.91t | 7.18t - 164.64 | 6.91t - 51.37 | 15.48 | 111.14 | 106.96 | 77.15 |

| | | | | | **Patient 3** | | | | | |
|---|---|---|---|---|---|---|---|---|---|---|
| **Respiratory Virus** | $w_1$ | $w_2$ | $w_1t$ | $w_2t$ | $w_1t - S_1$ | $w_2t - S_2$ | **t** | $Q_1$ | $Q_2$ | **D** |
| SARS-CoV-2 | 31.36 | 11.29 | 31.36t | 11.29t | 31.36t - 168.44 | 11.29t - 150.98 | 6.29 | 197.23 | 71.01 | 85.00 |
| Influenza A | 7.18 | 6.91 | 7.18t | 6.91t | 7.18t - 168.44 | 6.91t - 150.98 | 22.69 | 162.88 | 156.76 | 8.02 |

K and W

| | | | | | **Patient 1** | | | | | |
|---|---|---|---|---|---|---|---|---|---|---|
| **Respiratory Virus** | $w_1$ | $w_2$ | $w_1t$ | $w_2t$ | $w_1t - S_1$ | $w_2t - S_2$ | **t** | $Q_1$ | $Q_2$ | **D** |
| SARS-CoV-2 | 60.57 | 11.29 | 60.57t | 11.29t | 60.57t - 545.13 | 11.29t - 106.13 | 9.01 | 545.94 | 101.76 | 4.44 |
| Influenza A | 24.00 | 6.91 | 24t | 6.91t | 24t - 545.13 | 6.91t - 106.13 | 22.15 | 531.62 | 153.06 | 48.84 |

| | | | | | **Patient 2** | | | | | |
|---|---|---|---|---|---|---|---|---|---|---|
| **Respiratory Virus** | $w_1$ | $w_2$ | $w_1t$ | $w_2t$ | $w_1t - s_1$ | $w_2t - s_2$ | **t** | $Q_1$ | $Q_2$ | D |
| SARS-CoV-2 | 60.57 | 11.29 | 60.57t | 11.29t | 60.57t - 308.91 | 11.29t - 51.37 | 5.08 | 307.79 | 57.37 | 6.10 |
| Influenza A | 24.00 | 6.91 | 24t | 6.91t | 24t - 308.91 | 6.91t - 51.37 | 12.45 | 298.92 | 86.06 | 36.10 |

| | | | | | Patient 3 | | | | | |
|---|---|---|---|---|---|---|---|---|---|---|
| **Respiratory Virus** | $w_1$ | $w_2$ | $w_1t$ | $w_2t$ | $w_1t - s_1$ | $w_2t - s_2$ | **t** | $Q_1$ | $Q_2$ | **D** |
| SARS-CoV-2 | 60.57 | 11.29 | 60.57t | 11.29t | 60.57t - 529.92 | 11.29t - 150.98 | 8.90 | 539.32 | 100.53 | 51.32 |
| Influenza A | 24.00 | 6.91 | 24t | 6.91t | 24t - 529.92 | 6.91t - 150.98 | 22.06 | 529.50 | 152.45 | 1.53 |

The results of the reference functions for all embodiments (C and K, C and W, and K and W) are plotted in n-dimensional space (see Figure 17).

Across all embodiments of the present example, an error margin of 10.0 is chosen. This reflects the results of experiments on large patient populations. Alternatively, an error margin can be defined using Equation 10, or the kth minimum value of the distances set as the error margin if it is suspected that k proteomes of interest are present within the sample.

$D \leq \varepsilon$ for

C and K

Patient 1, presence of SARS-CoV-2 proteome, at 9.04 nM concentration
Patient 2, presence of SARS-CoV-2 proteome, at 5.13 nM concentration
Patient 3, presence of Influenza A proteome, at 22.19 nM concentration

C and W

Patient 1, presence of SARS-CoV-2 proteome, at 9.04 nM concentration
Patient 2, presence of SARS-CoV-2 proteome, at 5.13 nM concentration
Patient 3, presence of Influenza A proteome, at 22.19 nM concentration

K and W

Patient 1, presence of SARS-CoV-2 proteome, at 9.01 nM concentration
Patient 2, presence of SARS-CoV-2 proteome, at 5.08 nM concentration
Patient 3, presence of Influenza A proteome, at 22.06 nM concentration

Across all embodiments of this example, Patients 1 and 2 tested positive for SARS-CoV-2, and Patient 3 tested positive for Influenza A. Patient 1 had a significantly higher (protein) concentration of the SARS-CoV-2 proteome than Patient 2. This can be thought of as a measure of viral load, because a higher concentration of viruses results in a higher total concentration of viral protein when the viruses are lysed. It can therefore be concluded that Patient 2 has a higher SARS-CoV-2 viral shedding than Patient 1 and has a higher likelihood of transmitting the disease to more contacts than does Patient 1. This information can be useful in the epidemiological modelling of epidemics such as SARS-CoV-2 or other zoonotic viruses, as well as practical use in guiding containment instructions. Additionally, this information can assist in querying whether patients who have a higher circulating load of virus present with more severe infections and/or a higher likelihood of complications.

**Example 9** - **Special Case 1**

In special case 1, the molar protein concentration of the sample is available. Therefore, it is possible to calculate the number of amino acids of two or more amino acid types by dividing the calculated amino acid concentrations of two or more amino acid types by the known molar protein concentration of the solution.
The following background corrected fluorescence intensity values were measured:
W: 15890 AU; CR = 2371 AU
Eq. 5 was used to convert the measured $C_R$ background corrected fluorescence intensity into amino acid concentration of the $C_R$ amino acid type:

$$C_R\ A.A.concentration\ (\mu M) - \frac{2371\ AU}{2831\ ^{AU}/_{\mu M}} = 0.838\ uM\ C_R$$

Eq. 6 was used to convert the measured W background corrected fluorescence intensity into amino acid concentration of the W amino acid type:

$$W\ A.A.concentration\ (\mu M) = \frac{15980\ AU - 9859\ AU}{2362\ ^{AU}/_{\mu M}} = 2.59\ uM\ W$$

It is known that the protein of interest is present in the sample at 1 μM protein concentration. therefore, it is possible to calculate the number of amino acids of each amino acid type as using

$$Number\ of\ A.A.s\ of\ an\ amino\ acid\ type = \frac{Amino\ acid\ concentration\ of\ A.A.s\ of\ an\ amino\ acid\ type}{Protein\ concentration}$$

Therefore, the number of $C_R$ amino acids in the sample was calculated to be 0.838. The number of W amino acids in the sample was calculated to be 2.59.
In this case, the reference provides a point rather than a line. The comparison step involves calculating the distance between the sample point and each reference point, according to the formula

$$distance = \sqrt{\sum_{i=1}^{n} (S_i - Q_i)^2}$$

This resulted in the following distance calculation

| ID | a1 | a2 | D |
|---|---|---|---|
| BSA | 2 | 1 | 1.969351 |
| OVA | 3 | 4 | 2.581152 |
| LYZ | 6 | 0 | 5.775322 |
| $\alpha$S | 0 | 0 | 2.722195 |

It is suspected that the sample contains one protein of interest. Therefore, the error threshold is set as the minimum distance. The presence of BSA is identified in the sample, as this is the protein of interest which provided the smallest minimum distance to its reference point.

**Example 10: Methods for measurement and identification of proteomes and subproteomes of interest from patient body fluid samples**

In the following three Examples, two or more amino acid types within patient blood samples were labeled and measured as disclosed herein, and the methods of the invention were used to identify the presence and/or concentration and/or amount of one or more proteomes or subproteomes of interest within the patient blood samples.

Depending on how patient blood plasma samples are prepared, two proteomes of interest can be available. The platelet poor plasma proteome describes plasma that has been depleted of platelets. In contrast, the platelet rich plasma proteome describes plasma that has not been depleted of platelets. When platelet rich plasma samples are prepared, the platelets are lysed and release their contents into the plasma, so the proteins that had been contained by the platelets become part of the platelet rich plasma proteome. As a sensitive test of the methods of the invention, the following three worked examples confirm that the methods of the invention can detect small differences in the proteomic signatures of the platelet poor plasma (PPP) and platelet rich plasma (PRP) proteomes and determine whether an unknown patient sample contained the PPP proteome of interest or the PRP proteome of interest. Indeed, the presence and/or concentration and/or amount of the proteomes of interest within patient samples were accurately determined using the methods of the invention. As disclosed herein, in some embodiments, this comparison step involves use of a machine learning classifier, which was able to achieve 100% sensitivity and 100% specificity in identification of the presence or absence of the PPP and PRP proteomes of interest within a sample. As a further test of the methods of the invention, two further proteomes of interest were prepared from these plasma samples. The remaining low molecular weight platelet poor plasma subproteome of interest (PPP_50) and low molecular weight platelet rich plasma subproteome of interest (PRP_50) were distinguished from each other and from the PPP and PRP proteomes of interest using the methods of the invention with 100% sensitivity and 100% specificity.

As disclosed herein, in some embodiments, this comparison step involves calculation of reference lines. Each reference line uniquely describes a proteome of interest, for example a reference line uniquely describes the PPP proteome of interest and another reference line uniquely describes the PRP proteome of interest. The methods of the invention can calculate theoretical and experimental reference lines for a proteome of interest. In the case of the PPP proteome of interest, an experimental reference line was calculated in Example 11, and this experimental reference line was compared to the theoretical reference line which was calculated for the PPP proteome of interest in Example 12, and strong agreement between the experimental and theoretical reference lines was demonstrated. This agreement further demonstrates that that theoretical reference lines calculated using the methods of the invention for a proteome or subproteome of interest accurately predict the amino acid concentrations, values of the label, or number of amino acids of two or more labelled amin acid types that will be measured using the methods of the invention when the proteome or subproteome of interest is present within a sample.

In this Example, the methods of the study will first be described in detail.

N = 25 whole blood samples were obtained from consented healthy adult patients. The patients were undergoing regenerative orthobiologic procedures in which whole blood would normally be collected for further processing, and an additional Vacutainer vial of approximately 15 mL of whole blood was obtained as medical waste for medical research purposes. Because the whole blood was going to be used to prepare plasma, each vial of whole blood contained

approximately 17.5% v/v anticoagulant as standard in the field (acid-citrate-dextrose, ACD-A). ACD-A is standardized to contain the following mass of components per 100 mL water: 20.59-22.75 g citric acid, 23.28-25.73g dextrose, 4.90-5.42g sodium.

Two types of plasma preparations are available: platelet-poor plasma (PPP) and platelet-rich plasma (PRP). PPP is blood plasma with a very low number of platelets (approximately 0.5 x $10^8$ platelets/mL). PRP is blood plasma with a higher number of platelets (approximately 6 x $10^{11}$ platelets/mL). If unspecified, "plasma" in the literature or available in biobanks normally refers to PPP.

Both the PRP and PPP fractions of patient blood are available from a single patient blood sample when a two-step procedure is followed. In the first step, the whole blood was centrifuged at 200 x g for 10 minutes, and the platelet-containing plasma over the leukocyte-rich buffy coat layer was removed via pipette taking care not to disturb the buffy coat. In the second step, this isolate was centrifuged again at 2300 x g for 10 minutes.

Platelet-rich plasma (PRP) was manually separated from other blood components following centrifugation at 200 × g for 10 minutes (a "soft spin"). Care was taken during PRP isolation to avoid disturbing the leucocyte-rich buffy coat, thereby minimizing nucleated cell contamination. Isolated PRP was subjected to a second round of centrifugation at 2300 × g for 10 minutes (a "hard spin") which formed a platelet pellet. The upper 2/3 of the volume was collected as PPP due to its low concentration of platelets. The platelet pellet was resuspended via gentle shaking in the lower 1/3 of the volume, and this fraction was collected as PRP due to its higher concentration of platelets.

All plasma samples (PPP and PRP) were then flash frozen via submersion in liquid nitrogen (N2) prior to storage at - 80°C. This step was additionally used to lyse the platelets within the PRP preparation, so that they would release their contents into the plasma solution.

Plasma is approximately 90% albumin and globulin (immunoglobulins) by weight, and both have high molecular weights above 50 kDa. To further demonstrate the ability of the methods of the invention to detect the presence and/or concentration and/or amount of specifically a low molecular weight plasma subproteome, an isolation step as described herein was carried out to deplete high abundance proteins such as albumin and globulin from the PPP and PRP samples. To achieve this, a centrifugal filtration step as disclosed herein was used, resulting of depletion of albumin, globulin, and other proteins with molecular weights greater than 50 kDa from the PPP and PRP samples. The resulting low molecular weight plasma samples are the low molecular weight platelet poor plasma subproteome of interest (PPP_50) and the low molecular weight platelet rich plasma subproteome of interest (PRP_50). This was achieved by aliquoting each PPP and PRP preparation into two fractions, respectively. One fraction was not subjected to an isolation step as disclosed herein, and the other fraction was subjected to an isolation step as disclosed herein. The sample preparation procedure which was used is described in Table 12 below.

**Table 12: Description of sample preparation for PPP, PPP_50, PRP and PRP_50**

| Plasma preparation | PPP | | PRP | |
|---|---|---|---|---|
| Sample type | PPP | PPP_50 | PRP | PRP_50 |
| Sample description | Platelet-poor plasma without isolation step | Platelet-poor plasma with centrifugal filter isolation step | Platelet-rich plasma without isolation step | Platelet-rich plasma with centrifugal filter isolation step |
| Pelleting | No pelleting | No pelleting | No pelleting | Pellet platelet debris via centrifugation at 4000 x g for 10 minutes |
| Acetonitrile | No acetonitrile added | Add 20% v/v acetonitrile | No acetonitrile added | Add 20% v/v acetonitrile |
| Centrifugal filtration | No centrifugal filtration | Centrifugal filtration through an Amicon 50 kDa filter at 40000 x g for 2 hr | No centrifugal filtration | Centrifugal filtration through an Amicon 50 kDa filter at 40000 x g for 2 hr |
| Aliquoting | Aliquot into 5 low-protein binding Eppendorf tubes | Aliquot into 5 low-protein binding Eppendorf tubes | Aliquot into 5 low-protein binding Eppendorf tubes | Aliquot into 5 low-protein binding Eppendorf tubes |

(continued)

| Plasma preparation | PPP | | PRP | |
|---|---|---|---|---|
| Sample type | PPP | PPP_50 | PRP | PRP_50 |
| Flash freezing | All aliquots flash frozen via submersion in liquid $N_2$ | All aliquots flash frozen via submersion in liquid $N_2$ | All aliquots flash frozen via submersion in liquid $N_2$ | All aliquots flash frozen via submersion in liquid $N_2$ |
| Storage | Stored at -80 °C until use | Stored at -80 °C until use | Stored at -80 °C until use | Stored at -80 °C until use |
| Dilution immediately prior to use in labelling reactions | Dilute 1:60 in PBS | Dilute 1:2 in PBS for lysine (K), tryptophan and tyrosine (WY) labelling reactions and do not dilute in PBS for total cysteine (C) labelling reactions | Dilute 1:60 in PBS | Dilute 1:2 in PBS for lysine (K), tryptophan and tyrosine (WY) labelling reactions and do not dilute in PBS for total cysteine (C) labelling reactions |

Patient information was collected about each patient providing a sample. The gender and age of each patient was additionally recorded and is presented in Table 13. The abbreviations for each sample type provided in the key at left of Table 13 are used later in this example when the data which was measured is provided.

**Table 13: Patient information**

| Key | Sample Number | Gender | Age |
|---|---|---|---|
| A = PPP | 1 A/B/C/D | M | 38 |
| B = PPP_50 | 2 A/B/C/D | F | 70 |
| C = PRP | 3 A/B/C/D | M | 52 |
| D = PRP_50 | 4 A/B/C/D | M | 74 |

(continued)

| Key | Sample Number | Gender | Age |
|---|---|---|---|
| | 5 A/B/C/D | F | 61 |
| | 6 A/B/C/D | M | 56 |
| | 7 A/B/C/D | M | 43 |
| | 8 A/B/C/D | F | 75 |
| | 9 A/B/C/D | M | 42 |
| | 10 A/B/C/D | F | 64 |
| | 11 A/B/C/D | M | 58 |
| | 12 A/B/C/D | M | 61 |
| | 13 A/B/C/D | F | 36 |
| | 14 A/B/C/D | F | 20 |
| | 15 A/B/C/D | F | 57 |
| | 16 A/B | F | 19 |
| | 17 A/B | F | 24 |
| | 18 A/B | M | 44 |
| | 19 A/B | F | 32 |
| | 20 A/B | M | 38 |
| | 21 C/D | M | 73 |
| | 22 C/D | M | 35 |
| | 23 C/D | F | 67 |
| | 24 C/D | M | 34 |
| | 25 C/D | F | 70 |

As a further illustration of the sample types provided in the key, sample 7A is a PPP sample from a 43-year-old biological male, whereas sample 23D is a PRP_50 sample from a 67-year-old biological female.

The sample preparation proceeded as expected but it was noted that Sample 15B had become contaminated with liquid $N_2$ during flash freezing and that the effluent from Sample 8D was yellow indicating a failure of the Amicon filter used in the centrifugal filtration step. Collectively, these two samples will be described as sample preparation outliers. Additionally, it was noted that samples 3B, 8B, 12D, and 15D yielded lower than expected volumes of effluent. When patient samples were identified or used to calculate experimental reference lines, the sample preparation outliers (15B, 8D) were excluded from the analysis. Samples 3B, 8B, 12D, and 15D were not excluded from the analysis.

Preparation of labeling solutions

Throughout these examples, amino acids of the K, C, W, and Y amino acid types were labelled and measured as disclosed herein. Although all of these amino acid types were labelled and measured, selected combinations of these amino acid types were used to identify the presence and/or concentration and/or amount of a proteome of interest in the patient samples, to illustrate the feature of the invention disclosed herein that only two amino acid types need to be labelled and measured in order to uniquely identify a proteome of interest.

The following labelling solutions were prepared as disclosed herein.

To label amino acids within patient plasma samples of the K amino acid type, the K labeling solution was prepared: 12 mM OPA, 18 mM BME and 4% w/v SDS in 200 mM carbonate buffer, pH 10.5. To prepare 50 mL of K labelling solution: 80.5 mg OPA was weighted out and 20 mL of 500 mM carbonate buffer, pH 10.5 was added followed by 63.4 μL BME. Next, 20 mL $H_2O$ and 10 mL of 20% w/v SDS stock solution was added. The solution was protected from light and heated to 65 °C for 10 min. The K labelling solution was allowed to cool to room temperature prior to filtering through a 0.45 μm syringe filter to remove any large particles. The solution was then aliquoted into five 10 mL aliquots. All aliquots were sealed with parafilm and protected from light prior to storage at -20 °C.

To label amino acids within patient plasma samples of the W amino acid type and of the Y amino acid type, the WY labeling solution was prepared: 0.2 M TCE, 10 mM TCEP, 4% w/v SDS in 5 mM HEPES, pH 7. To 38.54 mL 5 mM HEPES buffer 500 μL 1 M TCEP stock solution was added followed by 964 μL TCE and 10 mL 20% w/v SDS stock solution. The solution was then aliquoted into five 10 mL aliquots. All aliquots were sealed with parafilm and protected from light prior to storage at -20 °C. To label amino acids within patient plasma samples of the C amino acid type, the C labelling solution was prepared: 5 mM ABD-F, 4% w/v SDS in 160 mM pH 10.5 carbonate buffer. To 10 mg of ABD-F as supplied, 1 mL 200 mM carbonate buffer, pH 10.5 was added. The vial was closed and vortexed for a few minutes so that all the ABD-F dissolved. The ABD-F containing solution was then combined with 6.36 mL 200 mM carbonate buffer, and the ABD-F vial was washed with the carbonate buffer to ensure all the ABD-F solution was combined. Then, 1.84 mL 20% w/v SDS was added. The same procedure was repeated for an additional 10 mg vial of ABD-F as supplied. The solutions were combined and aliquoted into 5 aliquots which were stored at -20 °C until use.

In this example, the total cysteine amino acid type (C) was being labelled, which included both reduced ($C_R$) and dislphide bonded ($C_D$) amino acids of the cysteine (C) amino acid type. Therefore, as disclosed herein, a cysteine reduction solution was also prepared to reduce the disulphide bonded cysteine amno acids ($C_D$) to reduced cysteine amino acids ($C_R$) prior to labeling all cysteine amino acids (C). The reduction solution prepared was: 20 mM TCEP in 5 mM HEPES pH 7. To prepare 50 mL of the C reduction solution: 1 mL 1 M TCEP stock was added to 49 mL 5 mM HEPES. The resulting solution was aliquoted into 10 mL aliquots, and all aliquots were protected from light and stored at -20 °C prior to use.

Excitation and emission filters

The fluorescence intensity from all amino acids of labelled amino acid type was detected using specific excitation and emission filters, as disclosed herein.

The filter pairs presented in Table 14 were used for all experiments in this example.

**Table 14: Excitation and emissions wavelengths and bandwidths for amino acid types C, K, W and Y**

| Amino acid type | | Excitation (nm) | Emission (nm) | Excitation Half Bandwidth (nm) | Emission Half Bandwidth (nm) |
|---|---|---|---|---|---|
| | Cysteine (C) | 355 | 510 | 38 | 10 |
| | Lysine (K) | 355 | 460 | 38 | 24 |
| | Tryptophan (W) | 355 | 510 | 38 | 10 |
| Tyrosine (Y) | Wavelength pair 1 | 320 | 460 | 40 | 24 |
| | Wavelength pair 2 | 320 | 510 | 40 | 10 |

Throughout the experiments described in this example and the following worked examples, each experiment was performed in triplicate (3 technical replicates). Where error bars are shown, these are the sample standard deviation across the three technical replicates of each experiment. Where error bars are shown, they are shown surrounding the mean of the three technical replicates of each experiment.

All fluorescence measurements in this and the following examples were carried out using a Thermo Fisher Flouroskan Ascent FL plate reader using standard settings. These included automatic gain selection, filter pair validation using Ascent software, quiescent conditions for all measurements and incubations (e.g. no shaking or stirring), room temperature of approximately 22 °C, normal beam, measurement direction from above, and measurement order left to right. 200 ms integration time was used rather than the default 20 ms integration time.

Measurement of calibration curves

In the methods of the invention, the amino acid concentration of each labelled amino acid type in the sample can be calculated from the measured label and the amino acid concentration is calculated from the measured label using a calibration curve or standard which converts between the measured label of the sample and the amino acid concentration of that amino acid type in the sample using the measured labels of one or more known amino acid concentrations of one or more proteins or amino acids. The calibration curves can be linear or nonlinear and calculated using linear or nonlinear regression. Calibration curves determine the relationship between measured values of the label (in arbitrary units, AU) and unknown amino acid concentrations (in molar concentration, such as micromolar, μM) so that the patient samples can be converted from the measured fluorescence intensities in AU into their associated amino acid concentrations in μM. As disclosed herein, a calibration curve was only calculated once for each amino acid type and this curve was then used

throughout the study.

To create the calibration curves, several protein solutions were prepared. Protein solutions were prepared as stock solutions with the steps outlined below, and then prior to performing a labelling reaction, were diluted to the specified concentrations via dilution in PBS. The stock solutions prepared are below, followed by the dilute solutions prepared from each stock solution.

300 μM BSA in PBS: 112 mg bovine serum albumin (BSA, product # A7030-10G from MilliporeSigma) was dissolved in 5 mL PBS. This provided a 339 μM stock solution which was adjusted down to 300 μM via dilution in PBS.

300 μM β-Lac in PBS: 30 mg β-Lactoglobulin (β-Lac, product # L0130-1G from MilliporeSigma) was dissolved in 5 mL PBS. This provided a 329 μM solution which was diluted to 300 μM via addition of PBS.

600 μM LYZ in PBS: 46 mg hen egg white lysozyme (LYZ, product # 10837059001 from MilliporeSigma) was dissolved in 5 mL PBS. This provided a 643 μM solution which was diluted to 600 μM via addition of PBS.

Additionally, a solution of 14 μM parathyroid hormone (PH, product # P7036-50UG from Millipore Sigma) was prepared by dissolving the supplied 50 μg PH with 82% peptide content in 310 μL PBS. From this stock solution, 600 μL of 2.33 μM PH was prepared via dilution in PBS. The PH was used as a deconvolution standard to separate at the detection stage the fluorescence signals read from the tryptophan and tyrosine amino acid type, as disclosed herein.

From the stock solutions provided avove, the following diluted protein solutions were prepared via dilution in PBS buffer: 60 μM BSA, 50 μM BSA, 45 μM BSA, 30 μM BSA, 25 μM BSA, 20 μM BSA, 15 μM BSA, 5 μM BSA, 60 μM β-Lac, 50 μM β-Lac, 45 μM β-Lac, 30 μM β-Lac, 25 μM β-Lac, 20 μM β-Lac, 15 μM β-Lac, 5 μM β-Lac, 120 μM LYZ, 100 μM LYZ, 90 μM LYZ, 60 μM LYZ, 50 μM LYZ, 40 μM LYZ, 30 μM LYZ, 30 μM LYZ, 10 μM LYZ.

*Creation of Calibration Curves, Labeling Reactions*

As disclosed in Example 1, the labeling reactions were able to be conveniently performed in high throughput using microplates which allow a large number of reactions to be performed and measured in parallel. The K labeling reactions were performed on one microplate, the C labeling reactions were performed on another microplate, and the W and Y labeling reactions were performed on a third microplate. A deconvolution standard was used to deconvolute the fluorescence intensities from the W and Y amino acid types, as disclosed herein and explained further later in this example. In all cases, dilute protein solutions (or buffer solution, for background correction as disclosed herein) and labeling solutions were combined in equal volumes. All dilute protein solutions and buffer solutions were sequentially added to a microplate, followed by adding a labeling solution to all wells of the microplate that contained a dilute protein solution or buffer solution. This is standard practice when performing chemical reactions and ensures that all reactions were allowed to progress for approximately the same amount of time.

As disclosed herein, to label amino acids of the K amino acid type, 100 μL of a dilute protein solution above was added to each well of a 96-well microplate (Grenier Bio One Catalogue # 655076 microplate). As discussed the labeling reactions were performed in triplicate, so 3 technical replicate wells per microplate contained the same dilute protein solution. Additionally, 3 wells were filled with only buffer (PBS) because these wells would be used for background correction as disclosed herein. Then, 100 μL of the K-labeling solution was added to all protein and buffer containing wells of the microplate. Immediately after adding the K-labelling solution to all protein or buffer containing wells of the microplate, the microplate was measured using the settings described above and the filter pair provided in Table 14 (355 nm excitation, 460 nm emission), resulting in a series of measured values (raw fluorescence intensities) for the K amino acid type.

As disclosed herein, prior to labelling all amino acids of the cysteine (C) amino acid type, the disulphide bonded subset of the cysteine amino acid type ($C_D$) was reduced to form the reduced subset of the cysteine amino acid type ($C_R$) using a reducing agent (TCEP). To do this, 200 μL of each dilute protein solution was combined with 200 μL C-reduction solution (TCEP) in microtubes and protected from light for 30 minutes. Additionally, 200 μL buffer was combined with 200 μL C-reduction solution in a microtube and protected from light for 30 minutes. After 30 minutes, 40 μL of each protein + C-reduction solution was added to each well of a 96-well microplate (Grenier Bio One Catalogue # 655076 microplate). As discussed the labeling reactions were performed in triplicate, so 3 technical replicate wells per microplate contained the same dilute protein + C-reduction solution. Additionally, 3 wells were filled with only buffer (PBS) + C-reduction solution because these wells would be used for background correction as disclosed herein. Then, 40 μL of the C-labeling solution (ABD-F) was added to each dilute protein + C-reduction solution and each only buffer (PBS) + C-reduction solution well of the microplate. The microplate was protected from light and the reaction was allowed to progress for 45 minutes and protected from light, before measuring the microplate using the settings described above and filter pair provided in Table 14 (355 nm excitation, 510 nm emission).

As disclosed herein, to label all amino acids of the W and Y amino acid types, 120 μL of each dilute protein solution was added to a 96-well microplate (Grenier Bio One UV transmissive plate Catalogue # 655801). As discussed the labeling reactions were performed in triplicate, so 3 technical replicate wells per microplate contained the same dilute protein solution. Additionally, 3 wells were filled with only buffer (PBS) because these wells would be used for background correction as disclosed herein. Then, 120 μL of the WY-labeling solution was added to all protein and buffer containing

wells of the microplate. Next, the microplate was placed at the centre of a transilluminator (Product # NEB-MLB-16), and the microplate was irradiated using the 302 nm UB wavelength setting and 100% (maximum) intensity for 30 minutes. The plate was measured using the settings described and sequentially with the filter pairs described in Table 14; the plate was measured sequentially with the W filter pair (355 nm excitation and 510 nm emission), the Y wavelength pair 1 filter pair (320 nm excitation and 460 nm emission), and the Y wavelength pair 2 filter pair (320 nm excitation and 510 nm emission). Deconvolution of the fluorescence intensities from the W and Y amino acid types using a deconvolution standard (parathyroid hormone, PH) as disclosed herein will be discussed below when the calibration curve for the Y amino acid type is presented.

As disclosed herein, the measured label of each amino acid type can be background corrected. This was achieved by combining, as described above, the fluorescent dye solution with an equal volume of buffer to the volume of protein-containing solution that was supplied during the labelling reaction, and then subtracting the fluorescence intensity detected from the dye and buffer solution from the fluorescence intensity detected for each dye and protein solution to provide a background corrected fluorescence measurement. This step was carried out for all labeling reactions. To provide a representative example of this step as carried out across all labeling reactions, the raw values of the label which were measured for labeling the K amino acid type are provided below in Tables 15 and 16:

Table 15: Flourescence intensity values measured for protein solutions and dye solutions when labeling the K amino acid type

| | | Technical Replicates | | |
|---|---|---|---|---|
| | Solution | Flourescence Intensity (AU) | Flourescence Intensity (AU) | Flourescence Intensity (AU) |
| Protein | BSA 60 μM | 2889 | 2814 | 2791 |
| | BSA 50 μM | 2676 | 2685 | 2675 |
| | BSA 45 μM | 2613 | 2610 | 2601 |
| | BSA 30 μM | 2350 | 2357 | 2355 |
| | BSA 25 μM | 2188 | 2184 | 2183 |
| | BSA 20 μM | 2011 | 2012 | 1999 |
| | BSA 15 μM | 1787 | 1779 | 1772 |
| | BSA 5 μM | 920.6 | 922.4 | 912.7 |
| | β-Lac 60 μM | 1880 | 1849 | 1830 |
| | β-Lac 50 μM | 1692 | 1701 | 1690 |
| | β-Lac 45 μM | 1586 | 1594 | 1581 |
| | β-Lac 30 μM | 1256 | 1247 | 1241 |
| | β-Lac 25 μM | 1087 | 1088 | 1076 |
| | β-Lac 20 μM | 954.5 | 945 | 925.3 |
| | β-Lac 15 μM | 756.2 | 741.7 | 724 |
| | β-Lac 5 μM | 278.2 | 276.9 | 268.9 |
| | LYZ 120 μM | 1769 | 1731 | 1719 |
| | LYZ 100 μM | 1596 | 1598 | 1585 |
| | LYZ 90 μM | 1489 | 1483 | 1476 |
| | LYZ 60 μM | 1161 | 1146 | 1139 |
| | LYZ 50 μM | 986.6 | 982.1 | 974.1 |
| | LYZ 40 μM | 841.2 | 831.5 | 820.9 |
| | LYZ 30 μM | 659 | 653 | 639.9 |
| | LYZ 10 μM | 247.5 | 247.7 | 243.2 |
| Dye | Dye (K-labeling solution) | 3.144 | 3.181 | 3.144 |

The mean of the measured dye fluorescence intensity values was calculated, this was 3.156. This value, providing the fluorescence intensity detected from the dye and buffer solution, was then subtracted from the fluorescence intensity detected from each dye and protein solution to provide a background corrected fluorescence intensity. 3.156 was subtracted from the fluorescence intensity detected from all wells labeleld "Protein" in Table 15, resulting in the following background corrected fluorescence intensity values in Table 16.

Table 16: Background corrected fluorescence intensity values measured for protein solutions when labeling the K amino acid type

| | Technical replicates | | |
|---|---|---|---|
| Solution | Background corrected Flourescence Intensity (AU) | Background corrected Flourescence Intensity (AU) | Background corrected Flourescence Intensity (AU) |
| BSA 60 μM | 2885.844 | 2810.844 | 2787.844 |
| BSA 50 μM | 2672.844 | 2681.844 | 2671.844 |
| BSA 45 μM | 2609.844 | 2606.844 | 2597.844 |
| BSA 30 μM | 2346.844 | 2353.844 | 2351.844 |
| BSA 25 μM | 2184.844 | 2180.844 | 2179.844 |
| BSA 20 μM | 2007.844 | 2008.844 | 1995.844 |
| BSA 15 μM | 1783.844 | 1775.844 | 1768.844 |
| BSA 5 μM | 917.444 | 919.244 | 909.544 |
| β-Lac 60 μM | 1876.844 | 1845.844 | 1826.844 |
| β-Lac 50 μM | 1688.844 | 1697.844 | 1686.844 |
| β-Lac 45 μM | 1582.844 | 1590.844 | 1577.844 |
| β-Lac 30 μM | 1252.844 | 1243.844 | 1237.844 |
| β-Lac 25 μM | 1083.844 | 1084.844 | 1072.844 |
| β-Lac 20 μM | 951.344 | 941.844 | 922.144 |
| β-Lac 15 μM | 753.044 | 738.544 | 720.844 |
| β-Lac 5 μM | 275.044 | 273.744 | 265.744 |
| LYZ 120 μM | 1765.844 | 1727.844 | 1715.844 |
| LYZ 100 μM | 1592.844 | 1594.844 | 1581.844 |
| LYZ 90 μM | 1485.844 | 1479.844 | 1472.844 |
| LYZ 60 μM | 1157.844 | 1142.844 | 1135.844 |
| LYZ 50 μM | 983.444 | 978.944 | 970.944 |
| LYZ 40 μM | 838.044 | 828.344 | 817.744 |
| LYZ 30 μM | 655.844 | 649.844 | 636.744 |
| LYZ 10 μM | 244.344 | 244.544 | 240.044 |

Background correction of all fluorescence intensities measured was carried out as illustrated above.

The amino acid concentration of the labelled protein solutions was plotted against the background-corrected fluorescence intensity of the labelled amino acids of each amino acid type and this calibration plot data was fit to provide a calibration curve. The amino acid concentration of each amino acid type of each labelled protein solution is the protein concentration of the protein solution times the number of amino acids of the indicated amino acid type in one molecule of the protein (provided by counting the number of occurrences of the amino acid in the amino acid sequence of the protein). The relationship between amino acid concentration of each amino acid type and the fluorescence intensity of the labelled amino acids of each amino acid type can be linear, and a linear calibration curve can calculated using linear regression providing a linear best fit. As disclosed herein, the relationship between amino acid concentration of each amino acid type and the fluorescence intensity of the labelled amino acids of each amino acid type can alternatively be nonlinear, and a nonlinear calibration curve can be calculated using non-linear regression providing a nonlinear best fit. The calibration

curves are used to translate values in arbitrary units (AU) into quantitative vales (molar concentration, μM).

The calibration curves were created as disclosed herein. The calibration plots are provided in Figures 18-20, and the equations of the calibration curves additionally provided below.

The calibration curve for the K amino acid type is provided in Figure 18. Nonlinear regression was used to calculate a polynomial fit. A polynomial fit is a type of non-linear fit. Other types of non-linear fits include exponential, power law, and sigmoidal fits. For the K amino acid type, the relationship between [K] amino acid concentration in μM and fluorescence intensity in arbitrary units was well described by a polynomial fit which was calculated using nonlinear regression. The polynomial fit is of the form [K (μM)] = a[K F.I. (AU)]$^4$ + b[K F.I. (AU)]$^3$ + c[K F.I. (AU)]$^2$ + d[K F.I. (AU)] + e, and its coefficients are below

| a | b | c | d | e |
|---|---|---|---|---|
| 7.642E-11 | -1.2E-07 | 5.17E-05 | 0.324 | -19.720 |

Therefore, when the values of the label (fluorescence intensities) were measured for the patient samples later in this example, and a calibration curve was used to convert between the measured value of the label of the K amino acid type and the amino acid concentration of the K amino acid type, the following relationship was used to convert the fluorescence intensities measured for the K amino acid type of the patient samples in arbitrary units to the amino acid concentrations of the K amino acid type.

$$[K(uM)] = 7.642 \times 10^{-11}[K\ F.I.\ (AU)]^4 - 1.2 \times 10^{-7}[K\ F.I.\ (AU)]^3 + 5.17 \times 10^{-5}[K\ F.I.\ (AU)]^2 + 0.324[K\ F.I.(AU)] - 19.720$$

The calibration curve for the C amino acid type is provided in Figure 19. The relationship between [C] amino acid concentration and fluorescence intensity was well described by a polynomial fit which was calculated using nonlinear regression. The polynomial fit was of the form [C (μM)] = a[C F.I. (AU)]$^2$ + b[C F.I. (AU)] + c, and its coefficients are below

| a | b | c |
|---|---|---|
| 0.014742 | 14.7802 | 23.1168 |

Therefore, when the values of the label (fluorescence intensities) were measured for the patient samples later in this example, and a calibration curve was used to convert between the measured value of the label of the C amino acid type and the amino acid concentration of the C amino acid type, the following relationship was used to convert the fluorescence intensities measured for the C amino acid type of the patient samples in arbitrary units to the amino acid concentrations of the C amino acid type.

$$[C\ (uM)] = 1.474 \times 10^{-2}[C\ F.I.(AU)]^2 + 14.780[C\ F.I.(AU)] + 23.117$$

The calibration curve for the W amino acid type is provided in Figure 20. For the W amino acid type, the data could be with a linear fit passing through the origin, of the form [W (μM)] = a[W F.I. (AU)], with the coefficient a = 2.315.

Therefore, when the values of the label (fluorescence intensities) were measured for the patient samples later in this example, and a calibration curve was used to convert between the measured value of the label of the W amino acid type and the amino acid concentration of the W amino acid type, the following relationship was used to convert the fluorescence intensities measured for the W amino acid type of the patient samples in arbitrary units to the amino acid concentrations of the W amino acid type.

$$[W\ (uM)] = 2.315[W\ F.I.(AU)]$$

As disclosed herein, the Y amino acid is quantified using two wavelength pairs and a deconvolution standard. At wavelength pair 1, fluorescence from both the Y and W amino acid types is observed, whereas at wavelength pair 2, fluorescence from the W amino acid type is observed. The wavelength pairs used here take advantage of the W amino acid type having a red shifted emission wavelength relative to the Y amino acid type, hence the W amino acid type can be detected with an emission wavelength of 510 whereas both the W and Y amino acid types can be detected with an emission wavelength of 460. However, use of separate excitation wavelengths and/or separate excitation and emission wavelengths is also possible. The deconvolution standard is human parathyroid hormone (PH), which has 1 tryptophan amino acid and 0 tyrosine amino acids. At wavelength pair 2, the mean fluorescence intensity of 0.83 measured for the 2.33 μM

PH corresponds to 2.33 μM W amino acids. At wavelength pair 1, the mean fluorescence intensity measured for the 2.33 μM PH wells (2.33 μM W amino acids) is 4.13. Therefore, at wavelength pair 1, the same concentration of W amino acids is $\frac{4.13}{0.83} = 4.97$ times more fluorescent than at wavelength pair 2.

4.97 was therefore used as the wavelength signal conversion to convert fluorescence from W amino acids at wavelength pair 2 to fluorescence at wavelength pair 1. Each background corrected fluorescence intensity value at wavelength pair 2 was multiplied by the wavelength signal conversion, and this result was subtracted from each corresponding background corrected fluorescence for W and Y amino acids at wavelength pair 1. This revealed the fluorescence from the Y amino acid type at wavelength pair 1.

As an example of these steps, background corrected fluorescence intensity values were measured for a 60 μM solution of BSA at wavelength pair 1 and wavelength pair 2. The background corrected fluorescence intensity of this solution of BSA at wavelength pair 1 was 297.26 AU. The background corrected fluorescence intensity of the same solution of BSA at wavelength pair 2 was 38.88 AU, and this provided the fluorescence from residues of the W amino acid type within BSA.

This value at wavelength pair 2 (38.88 AU) was multiplied by the wavelength signal conversion (4.97) to provide the fluorescence from residues of the W amino acid type within BSA at wavelength pair 1, at which both fluorescence from the W amino acid type and the Y amino acid type is detected. 38.88 AU x 4.97 = 193.23 AU provided by the W amino acid type of BSA at wavelength pair 1. Then, the fluorescence of the W amino acid type at wavelength pair 1 was subtracted from the fluorescence of the W and Y amino acid types at wavelength pair 1, providing the fluorescence from the Y amino acid type at wavelength pair 1. 297.26 AU - 193.23 AU = 103.90 AU.

The amino acid concentrations of the Y amino acid type were plotted against the fluorescence intensities of the Y amino acid type calculated in this way at wavelength pair 1 and linear regression was performed on the fluorescence intensities from the Y amino acid type at wavelength pair 1, resulting in the following relationship between amino acid concentration of the Y amino acid type and fluorescence intensity of the Y amino acid type:

$$[Y\ (uM)] = 7.381[Y\ F.I.(AU)]$$

Measurement of Patient Samples

Having now calculated the calibration curves used to transform the fluorescence intensities in arbitrary units (AU) measured for the patient samples into the corresponding molar amino acid concentrations (μM) for the patient samples, measurement of patient samples is discussed.

Patient PPP, PRP, PPP_50, and PRP_50 samples were labelled using the K-labelling solution, C-labelling solution following the C-reduction solution, and WY-labelling solution in microplates. The labeling steps are the same as described earlier for the creation of the calibration plate. However for convenience they are also indicated below, along with the specific microplate layout used (although they can be labeled by any means and in any format known in the art).

The plate layout for all patient samples is provided in Table 17

**Table 17: Plate layout for patient samples**

| | 1 | 2 | 3 | 4 | 5 | 6 | 7 | 8 | 9 | 10 | 11 | 12 |
|---|---|---|---|---|---|---|---|---|---|---|---|---|
| A | Pat. 1 | Pat. 1 | Pat. 1 | Pat. 9 | Pat. 9 | Pat. 9 | Pat. 17 | Pat. 17 | Pat. 17 | Dye | Dye | Dye |
| B | Pat. 2 | Pat. 2 | Pat. 2 | Pat. 10 | Pat. 10 | Pat. 10 | Pat. 18 | Pat. 18 | Pat. 18 | Pat. 1 | Pat. 2 | Pat. 3 |
| C | Pat. 3 | Pat. 3 | Pat. 3 | Pat. 11 | Pat. 11 | Pat. 11 | Pat. 19 | Pat. 19 | Pat. 19 | Pat. 4 | Pat. 5 | Pat. 6 |
| D | Pat. 4 | Pat. 4 | Pat. 4 | Pat. 12 | Pat. 12 | Pat. 12 | Pat. 20 | Pat. 20 | Pat. 20 | Pat. 7 | Pat. 8 | Pat. 9 |
| E | Pat. 5 | Pat. 5 | Pat. 5 | Pat. 13 | Pat. 13 | Pat. 13 | 30 uM BSA | 30 uM BSA | 30 uM BSA | Pat. 10 | Pat. 11 | Pat. 12 |
| F | Pat. 6 | Pat. 6 | Pat. 6 | Pat. 14 | Pat. 14 | Pat. 14 | 30 uM β-Lac | 30 uM β-Lac | 30 uM β-Lac | Pat. 13 | Pat. 14 | Pat. 15 |
| G | Pat.7 | Pat. 7 | Pat. 7 | Pat. 15 | Pat. 15 | Pat. 15 | 60 uM LYZ | 60 uM LYZ | 60 uM LYZ | Pat. 16 | Pat. 17 | Pat. 18 |
| H | Pat. 8 | Pat. 8 | Pat. 8 | Pat. 16 | Pat. 16 | Pat. 16 | 0.54 uM PH | 0.54 uM PH | 0.54 uM PH | Pat.19 | Pat.20 | Buffer |

In Table 17, the term "Pat. n", where n = 1:20, refers to the $n^{th}$ patient of a sample type provided in Table 13. For example, for the PPP samples, Pat. 16 is Sample 16A from a 19-year-old female. As another example, for the PRP samples, Pat. 16 is Sample 21C from a 73-year-old male.

The proteins indicated on the plate in light grey are used for scaling because the default automatic gain selection feature was used for all plates measured. They are used to transform the fluorescence from the measured patient samples to the equivalent fluorescence on the calibration plates (which were used to create the calibration curves), correcting to what is observed in a standard constant gain experimental setup. However, these are not required in standard assay

operation when a constant gain feature is used.

As explained in Table 12, the patient plasma samples were diluted immediately prior to carrying out a labeling reaction to bring the range of fluorescence intensities measured into the range covered by the calibration curves for the most accurate conversion between fluorescence intensity and amino acid concentration. This is an optional step, as an alternative a wider ranging calibration curve can be calculated.

The dilution factors used are for each sample type are highlighted in Table 18.

Table 18: Dilutions immediately prior to the indicated labeling reaction

| Sample Type | K | C | W | Y |
|---|---|---|---|---|
| PPP | 60 | 60 | 60 | 60 |
| PRP | 60 | 60 | 60 | 60 |
| PPP_50 | 2 | 1 | 2 | 2 |
| PRP_50 | 2 | 1 | 2 | 2 |

The steps performed to carry out each labelling reaction for all (PPP, PRP, PPP_50, and PRP_50 K labelling) plates:

1. 100 μL of the indicated patient sample was added to each labelled white well of a Grenier Bio One Catalogue # 655076 microplate
2. 100 μL of the indicated protein solution was added to each light grey well
3. 100 μL PBS buffer was added to each black dye well
4. 100 μL of the indicated patient sample or PBS was added to each dark grey well
5. 100 μL of PBS was added to each dark grey well
6. 100 μL of K-labelling solution was added to each black well
7. 100 μL of K-labelling solution was added to each light grey well
8. 100 μL of K-labelling solution was added to each labelled white well
9. Immediately after completing step 8, the microplate was measured using the settings described above and filter pair provided in Table 14 (355 excitation, 460 emission).

For all (PPP, PRP, PPP_50, and PRP_50) C labelling plates:

1. 200 μL of the indicated solution (each patient sample indicated in the white wells, the proteins indicated in the light grey wells, and buffer for the black wells) was combined with 200 μL C-reduction solution in microtubes and protected from light for 30 minutes. 200 μL of the patient samples indicated in the dark grey wells or PBS as indicated was also combined with 200 μL PBS and protected from light for 30 minutes.
2. 40 μL of each patient sample solution (indicated patient sample + C-reduction solution) was added to the white wells of a Grenier Bio One Catalogue # 655076 microplate
3. 40 μL of each protein solution (indicated protein + C-reduction solution) was added to the light grey wells
4. 40 μL of the buffer + C-reduction solution was added to the black wells
5. 40 μL of the indicated patient sample + PBS solution or PBS solution was added to the dark grey wells, followed by another 40 μL PBS in each well
6. 40 μL C-labelling solution was added to the black wells
7. 40 μL C-labelling solution was added to each black well
8. 40 μL C-labelling solution was added to each light grey well
9. 40 μL C-labelling solution was added to each labelled white well
10. The reaction was allowed to progress for 45 minutes under quiescent conditions with the plate protected from light
11. The microplate was measured using the settings described above and filter pair provided in Table 14 (355 nm excitation, 510 nm emission)

For all (PPP, PRP, PPP_50, and PRP_50) WY labelling plates:

1. 120 μL of the indicated patient sample was added to each labelled white well of a Grenier Bio One UV transmissive plate Catalogue # 655801
2. 120 μL of the indicated protein solution was added to each light grey well
3. 120 μL of PBS was added to each black well

4. 120 μL of each indicated patient sample was added to each dark grey well with 120 μL PBS added to the buffer well
5. 120 μL of PBS was added to each dark grey well
6. 120 μL WY-labelling solution was added to each black well
7. 120 μL WY-labelling solution was added to each light grey well
8. 120 μL WY-labelling solution was added to each labelled white well
9. The microplate
10. The microplate was placed at the centre of a NEB-MLB-16 transilluminator, and the microplate was irradiated using the 302 nm UB wavelength setting and 100% (maximum) intensity for 30 minutes under quiescent conditions
11. The plate was measured using the settings described and sequentially with the filter pairs described in Table 14; the plate was measured sequentially with the W filter pair (355 nm excitation and 510 nm emission), the Y wavelength pair 1 filter pair (320 nm excitation and 460 nm emission), and the Y wavelength pair 2 filter pair (320 nm excitation and 510 nm emission)

For all PPP plates (K amino acid type, C amino acid type, W and Y amino acid type), the plate layout taking into account the samples described in Table 13 was below

| | 1 | 2 | 3 | 4 | 5 | 6 | 7 | 8 | 9 | 10 | 11 | 12 |
|---|---|---|---|---|---|---|---|---|---|---|---|---|
| A | 1A | 1A | 1A | 9A | 9A | 9A | 17A | 17A | 17A | DYE | DYE | DYE |
| B | 2A | 2A | 2A | 10A | 10A | 10A | 18A | 18A | 18A | 1A | 2A | 3A |
| C | 3A | 3A | 3A | 11A | 11A | 11A | 19A | 19A | 19A | 4A | 5A | 6A |
| D | 4A | 4A | 4A | 12A | 12A | 12A | 20A | 20A | 20A | 7A | 8A | 9A |
| E | 5A | 5A | 5A | 13A | 13A | 13A | BSA | BSA | BSA | 10A | 11A | 12A |
| F | 6A | 6A | 6A | 14A | 14A | 14A | β-Lac | β-Lac | β-Lac | 13A | 14A | 15A |
| G | 7A | 7A | 7A | 15A | 15A | 15A | LYZ | LYZ | LYZ | 16A | 17A | 18A |
| H | 8A | 8A | 8A | 16A | 16A | 16A | PH | PH | PH | 19A | 20A | Buffer |

For all PRP plates (K amino acid type, C amino acid type, W and Y amino acid type), the plate layout taking into account the samples described in Table 13 was below

| | 1 | 2 | 3 | 4 | 5 | 6 | 7 | 8 | 9 | 10 | 11 | 12 |
|---|---|---|---|---|---|---|---|---|---|---|---|---|
| A | 1C | 1C | 1C | 9C | 9C | 9C | 22C | 22C | 22C | DYE | DYE | DYE |
| B | 2C | 2C | 2C | 10C | 10C | 10C | 23C | 23C | 23C | 1C | 2C | 3C |
| C | 3C | 3C | 3C | 11C | 11C | 11C | 24C | 24C | 24C | 4C | 5C | 6C |
| D | 4C | 4C | 4C | 12C | 12C | 12C | 25C | 25C | 25C | 7C | 8C | 9C |
| E | 5C | 5C | 5C | 13C | 13C | 13C | BSA | BSA | BSA | 10C | 11C | 12C |
| F | 6C | 6C | 6C | 14C | 14C | 14C | β-Lac | β-Lac | β-Lac | 13C | 14C | 15C |
| G | 7C | 7C | 7C | 15C | 15C | 15C | LYZ | LYZ | LYZ | 21C | 22C | 23C |
| H | 8C | 8C | 8C | 21C | 21C | 21C | PH | PH | PH | 24C | 25C | Buffer |

For all PPP_50 plates (K amino acid type, C amino acid type, W and Y amino acid type), the plate layout taking into account the samples described in Table 13 was below

| | 1 | 2 | 3 | 4 | 5 | 6 | 7 | 8 | 9 | 10 | 11 | 12 |
|---|---|---|---|---|---|---|---|---|---|---|---|---|
| A | 1B | 1B | 1B | 9B | 9B | 9B | 17B | 17B | 17B | DYE | DYE | DYE |
| B | 2B | 2B | 2B | 10B | 10B | 10B | 18B | 18B | 18B | 1B | 2B | 3B |
| C | 3B | 3B | 3B | 11B | 11B | 11B | 19B | 19B | 19B | 4B | 5B | 6B |
| D | 4B | 4B | 4B | 12B | 12B | 12B | 20B | 20B | 20B | 7B | 8B | 9B |
| E | 5B | 5B | 5B | 13B | 13B | 13B | BSA | BSA | BSA | 10B | 11B | 12B |
| F | 6B | 6B | 6B | 14B | 14B | 14B | β-Lac | β-Lac | β-Lac | 13B | 14B | 15B |
| G | 7B | 7B | 7B | 15B | 15B | 15B | LYZ | LYZ | LYZ | 16B | 17B | 18B |
| H | 8B | 8B | 8B | 16B | 16B | 16B | PH | PH | PH | 19B | 20B | Buffer |

For all PRP_50 plates (K amino acid type, C amino acid type, W and Y amino acid type), the plate layout taking into account the samples described in Table 13 was below

| | 1 | 2 | 3 | 4 | 5 | 6 | 7 | 8 | 9 | 10 | 11 | 12 |
|---|---|---|---|---|---|---|---|---|---|---|---|---|
| A | 1D | 1D | 1D | 9D | 9D | 9D | 22D | 22D | 22D | DYE | DYE | DYE |
| B | 2D | 2D | 2D | 10D | 10D | 10D | 23D | 23D | 23D | 1D | 2D | 3D |
| C | 3D | 3D | 3D | 11D | 11D | 11D | 24D | 24D | 24D | 4D | 5D | 6D |
| D | 4D | 4D | 4D | 12D | 12D | 12D | 25D | 25D | 25D | 7D | 8D | 9D |
| E | 5D | 5D | 5D | 13D | 13D | 13D | BSA | BSA | BSA | 10D | 11D | 12D |
| F | 6D | 6D | 6D | 14D | 14D | 14D | β-Lac | β-Lac | β-Lac | 13D | 14D | 15D |
| G | 7D | 7D | 7D | 15D | 15D | 15D | LYZ | LYZ | LYZ | 21D | 22D | 23D |
| H | 8D | 8D | 8D | 21D | 21D | 21D | PH | PH | PH | 24D | 25D | Buffer |

The following raw fluorescence intensities were measured for labeling of the K amino acid type

| PPP sample type | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 1874 | 1835 | 1817 | 1801 | 1770 | 1769 | 2031 | 1994 | 1985 | 3.693 | 3.722 | 3.696 |
| B | 1872 | 1844 | 1831 | 1761 | 1702 | 1707 | 1747 | 1715 | 1717 | 1.684 | 1.906 | 1.666 |
| C | 1842 | 1812 | 1812 | 1778 | 1705 | 1711 | 1743 | 1698 | 1704 | 2.155 | 1.992 | 1.801 |
| D | 1731 | 1690 | 1690 | 1733 | 1702 | 1705 | 1821 | 1788 | 1790 | 1.822 | 2.278 | 1.353 |
| E | 1767 | 1718 | 1718 | 1746 | 1683 | 1686 | 2282 | 2247 | 2254 | 1.392 | 1.529 | 1.809 |
| F | 1799 | 1762 | 1743 | 1879 | 1830 | 1830 | 1331 | 1287 | 1281 | 2.044 | 2.178 | 1.852 |
| G | 1771 | 1741 | 1720 | 1745 | 1696 | 1691 | 1098 | 1072 | 1082 | 1.607 | 1.636 | 2.003 |
| H | 1674 | 1632 | 1632 | 1706 | 1668 | 1687 | 20.99 | 19.96 | 20.53 | 1.888 | 1.288 | 0.6641 |

| PRP sample type | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 1834 | 1756 | 1747 | 1766 | 1742 | 1741 | 1887 | 1846 | 1858 | 9.259 | 9.598 | 10.12 |
| B | 1909 | 1880 | 1865 | 1672 | 1649 | 1643 | 1748 | 1730 | 1736 | 2.116 | 2.108 | 1.745 |
| C | 1876 | 1841 | 1839 | 1766 | 1731 | 1728 | 1825 | 1792 | 1791 | 2.378 | 1.911 | 1.833 |
| D | 1835 | 1787 | 1794 | 1687 | 1656 | 1658 | 1665 | 1636 | 1661 | 2.016 | 2.247 | 1.394 |
| E | 1705 | 1653 | 1658 | 1723 | 1702 | 1697 | 2156 | 2123 | 2140 | 1.381 | 1.603 | 1.815 |
| F | 1777 | 1757 | 1737 | 1827 | 1776 | 1767 | 1136 | 1095 | 1114 | 2.229 | 2.215 | 1.968 |

(continued)

| PRP sample type | | | | | | | | | | | |
|---|---|---|---|---|---|---|---|---|---|---|---|
| | 1 | 2 | 3 | 4 | 5 | 6 | 7 | 8 | 9 | 10 | 11 | 12 |
| G | 1786 | 1766 | 1747 | 1728 | 1698 | 1690 | 1046 | 989.9 | 994.6 | 1.456 | 2.052 | 1.711 |
| H | 1634 | 1610 | 1596 | 1693 | 1651 | 1655 | 19.32 | 18.85 | 18.89 | 1.849 | 1.965 | 0.667 |

| PPP_50 sample type | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 3603 | 3518 | 3491 | 3506 | 3526 | 3490 | 3502 | 3438 | 3430 | 3.634 | 3.75 | 3.749 |
| B | 3470 | 3449 | 3483 | 3300 | 3309 | 3315 | 3209 | 3203 | 3214 | 1.644 | 4.709 | 4.067 |
| C | 3271 | 3289 | 3274 | 3266 | 3277 | 3264 | 3078 | 3051 | 3069 | 6.721 | 4.652 | 3.774 |
| D | 3325 | 3323 | 3310 | 3379 | 3381 | 3380 | 3446 | 3442 | 3413 | 3.836 | 4.43 | 2.455 |
| E | 3041 | 3067 | 3062 | 3301 | 3235 | 3235 | 2434 | 2425 | 2410 | 1.726 | 3.598 | 1.692 |
| F | 3306 | 3275 | 3278 | 3263 | 3271 | 3256 | 1311 | 1308 | 1313 | 5.004 | 5.447 | 4.275 |
| G | 3026 | 3039 | 3020 | 3011 | 2991 | 3003 | 1171 | 1175 | 1169 | 3.253 | 1.518 | 3.972 |
| H | 2959 | 2991 | 2970 | 3070 | 3064 | 3065 | 10.74 | 9.625 | 8.466 | 3.778 | 1.077 | 0.7327 |

| PRP_50 sample type | | | | | | | | | | | | | |
|---|---|---|---|---|---|---|---|---|---|---|---|---|---|
| | 1 | 2 | 3 | 4 | 5 | 6 | 7 | 8 | 9 | 10 | 11 | 12 | |
| A | 3348 | 3319 | 3336 | 3526 | 3551 | 3576 | 3217 | 3242 | 3257 | 5.541 | 5.463 | 5.452 | |
| B | 3365 | 3370 | 3405 | 3250 | 3275 | 3314 | 3373 | 3400 | 3383 | 3.838 | 5.009 | 4.33 | |
| C | 3229 | 3242 | 3250 | 3278 | 3271 | 3290 | 3636 | 3652 | 3652 | 6.573 | 4.5 | 4.023 | |
| D | 3248 | 3285 | 3288 | 3333 | 3354 | 3346 | 3267 | 3264 | 3261 | 4.132 | 1.471 | 2.495 | |
| E | 3042 | 3075 | 3061 | 3252 | 3257 | 3272 | 2425 | 2414 | 2419 | 1.679 | 3.659 | 1.682 | |
| F | 3309 | 3332 | 3338 | 3278 | 3322 | 3336 | 1356 | 1334 | 1328 | 5.361 | 5.202 | 4.139 | |
| G | 2977 | 3001 | 3005 | 3029 | 3058 | 3085 | 1228 | 1193 | 1180 | 1.512 | 4.471 | 3.956 | |
| H | 1115 | 1168 | 1168 | 3195 | 3210 | 3215 | 16.2 | 14.56 | 14.48 | 3.485 | 4.759 | 0.7442 | |

The following raw fluorescence intensities were measured for labeling of the C amino acid type

| PPP sample type | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 29.94 | 27.64 | 25.74 | 28.46 | 28.13 | 30.61 | 41.26 | 42.3 | 42.41 | 0.1129 | 0.1134 | 0.1052 |
| B | 32.96 | 32.66 | 32.94 | 29.39 | 29.45 | 29.51 | 31.67 | 31.3 | 31.96 | 0.1397 | 0.1347 | 0.1255 |
| C | 34.04 | 33.72 | 34.25 | 30.64 | 30.26 | 30.77 | 31.04 | 31.35 | 31.07 | 0.139 | 0.1369 | 0.1324 |
| D | 29.21 | 28.21 | 28.16 | 29.63 | 29.43 | 30.1 | 34.46 | 34.87 | 35.1 | 0.1306 | 0.1429 | 0.1262 |
| E | 32.59 | 30.66 | 30.95 | 30.14 | 29.26 | 29.69 | 83.46 | 82.6 | 84.05 | 0.1248 | 0.1268 | 0.1502 |
| F | 34.49 | 31.94 | 31.96 | 37.57 | 37.98 | 38.03 | 11.23 | 10.83 | 11.02 | 0.1357 | 0.1442 | 0.1327 |
| G | 34.85 | 33.6 | 32.93 | 31.43 | 31.8 | 31.92 | 28.95 | 28.83 | 28.96 | 0.1275 | 0.127 | 0.1332 |
| H | 30.18 | 29.29 | 29.6 | 29.59 | 29.81 | 29.92 | 0.113 | 0.1091 | 0.1059 | 0.1416 | 0.1181 | 0.241 |

| PRP sample type | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 30.64 | 33.05 | 31.32 | 33.52 | 33.86 | 33.68 | 41.59 | 41.4 | 40.4 | 0.1206 | 0.1145 | 0.1159 |
| B | 37.27 | 37.23 | 35.76 | 32.17 | 32.14 | 31.98 | 32.71 | 33.61 | 32.19 | 0.1432 | 0.1727 | 0.1409 |
| C | 36.7 | 37.62 | 35.52 | 33.68 | 34.23 | 33.44 | 34.22 | 34.34 | 33.48 | 0.1299 | 0.1417 | 0.1442 |
| D | 23.92 | 18.22 | 26.3 | 31.97 | 32.42 | 31.67 | 32.29 | 33.23 | 32.01 | 0.1393 | 0.1514 | 0.1358 |
| E | 32.86 | 44.72 | 32.02 | 33.49 | 33.59 | 32.65 | 88.22 | 89.83 | 87.94 | 0.1337 | 0.1346 | 0.151 |
| F | 36.36 | 34.98 | 34.11 | 41.7 | 42.87 | 41.86 | 10.67 | 10.75 | 10.58 | 0.1509 | 0.1524 | 0.1397 |
| G | 36.68 | 36.76 | 35.76 | 32.99 | 32.99 | 32.71 | 29.84 | 30.38 | 30.1 | 0.1362 | 0.1496 | 0.1491 |
| H | 31.8 | 32.11 | 31.33 | 32.14 | 33.02 | 32.21 | 0.1137 | 0.1127 | 0.1176 | 0.1397 | 0.1499 | 0.197 |

| PPP_50 sample type | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 15.77 | 15.43 | 14.7 | 17.98 | 17.71 | 17.91 | 16.37 | 15.28 | 17.09 | 0.136 | 0.1345 | 0.1329 |
| B | 11.6 | 11.29 | 10.83 | 25.06 | 24.79 | 20.76 | 11.63 | 11.61 | 10.95 | 0.1651 | 0.2724 | 0.2319 |
| C | 12.93 | 12.79 | 12.12 | 21.53 | 23.36 | 22.3 | 4.579 | 4.612 | 4.804 | 0.3524 | 0.2674 | 0.2311 |
| D | 18.29 | 17.32 | 16.99 | 11.88 | 11.99 | 11.23 | 9.165 | 9.156 | 9.251 | 0.2401 | 0.2461 | 0.1842 |
| E | 6.886 | 6.87 | 6.62 | 8.672 | 8.03 | 7.614 | 115.7 | 115.3 | 119.2 | 0.1534 | 0.2577 | 0.1778 |
| F | 10.61 | 10.42 | 10.09 | 15.6 | 14.9 | 14.89 | 13.64 | 14 | 14.61 | 0.2888 | 0.3171 | 0.2556 |
| G | 6.925 | 6.809 | 6.558 | 23.34 | 21.77 | 22.03 | 40.65 | 39 | 40.45 | 0.2351 | 0.1477 | 0.252 |
| H | 8.016 | 8.062 | 7.855 | 6.234 | 6.22 | 6.114 | 0.1339 | 0.1318 | 0.1373 | 0.2446 | 0.1365 | 0.1906 |

| PRP_50 sample type | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 6.495 | 6.344 | 5.996 | 15.77 | 15.88 | 15.75 | 12.45 | 11.8 | 11.26 | 0.1174 | 0.1145 | 0.1169 |
| B | 12.63 | 12.3 | 11.9 | 15.71 | 15.91 | 15.59 | 6.298 | 6.178 | 6.099 | 0.235 | 0.2995 | 0.2606 |
| C | 11.51 | 11.21 | 10.96 | 9.331 | 9.556 | 9.193 | 7.053 | 7.024 | 6.83 | 0.3488 | 0.2825 | 0.2707 |
| D | 10.23 | 9.962 | 9.51 | 11.48 | 11.49 | 11.13 | 6.924 | 6.717 | 6.422 | 0.2633 | 0.3357 | 0.2028 |
| E | 9.02 | 8.72 | 8.359 | 19.26 | 18.92 | 18.04 | 89.88 | 89.94 | 91.28 | 0.16 | 0.2624 | 0.18 |
| F | 19.74 | 19.42 | 18.5 | 26.94 | 23.25 | 23.13 | 11.02 | 10.91 | 11.06 | 0.3228 | 0.341 | 0.2834 |
| G | 4.152 | 4.15 | 3.954 | 12.48 | 12.87 | 12.57 | 30.52 | 31.13 | 31.66 | 0.164 | 0.3047 | 0.2515 |
| H | 15.02 | 14.47 | 14.26 | 20.04 | 20.73 | 19.81 | 0.1171 | 0.1171 | 0.1145 | 0.2314 | 0.3136 | 0.1912 |

The following raw fluorescence intensities were measured for labelling the W amino acid type

| PPP sample type | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 40.64 | 36.78 | 37.94 | 58.17 | 49.85 | 50.36 | 64.49 | 68.5 | 67.31 | 0.1444 | 0.1556 | 0.1552 |
| B | 43.66 | 39.2 | 40.07 | 42.89 | 41.74 | 39.66 | 41.95 | 42.52 | 47.26 | 0.5089 | 0.6656 | 0.6234 |
| C | 45.73 | 40.65 | 42.53 | 48.96 | 48.74 | 45.34 | 40.07 | 44.3 | 43.35 | 0.5254 | 0.5395 | 0.6314 |

(continued)

| PPP sample type | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| D | 36.83 | 32.71 | 34.1 | 45.6 | 41.54 | 35.21 | 40.49 | 42.64 | 40.72 | 0.5354 | 0.6217 | 0.5598 |
| E | 36.44 | 33.06 | 33.83 | 45.22 | 42.25 | 42.48 | 30.34 | 34.1 | 31.38 | 0.4834 | 0.5322 | 0.5848 |
| F | 43.92 | 39.34 | 40.14 | 46.7 | 44.63 | 42.9 | 23.51 | 22.92 | 24.17 | 0.5149 | 0.5978 | 0.5757 |
| G | 42.53 | 39.18 | 39.66 | 43.15 | 44.38 | 43.3 | 75.19 | 86.22 | 84.44 | 0.4861 | 0.5733 | 0.5806 |
| H | 46.97 | 41.67 | 41.92 | 48.98 | 45.9 | 41.85 | 0.4925 | 0.4489 | 0.5554 | 0.4759 | 0.4447 | 0.0959 |

| PRP sample type | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 39.27 | 41.04 | 40.38 | 44.92 | 44.97 | 46.55 | 45.02 | 43.71 | 44.81 | 0.1687 | 0.1651 | 0.1645 |
| B | 36.16 | 46.25 | 43.19 | 36.69 | 36.14 | 38.8 | 46.84 | 46.11 | 46.13 | 0.6357 | 0.7342 | 0.7123 |
| C | 51.39 | 49.1 | 48.02 | 43.35 | 42.36 | 43.39 | 43.88 | 42.53 | 42.24 | 0.6831 | 0.5982 | 0.6587 |
| D | 43.32 | 42.44 | 43.36 | 36.31 | 35.72 | 37.67 | 37.74 | 34.91 | 36.78 | 0.7105 | 0.6158 | 0.5774 |
| E | 38.23 | 35.42 | 36.66 | 41.67 | 37.65 | 39.53 | 27.13 | 26.85 | 27.05 | 0.5842 | 0.5892 | 0.6217 |
| F | 44.88 | 46.22 | 44.29 | 40.12 | 39.45 | 41.72 | 20.61 | 19.44 | 20.2 | 0.646 | 0.6733 | 0.6628 |
| G | 50.22 | 46.88 | 45.48 | 42.04 | 41.66 | 42.98 | 72.13 | 69.83 | 79.21 | 0.5634 | 0.6537 | 0.6427 |
| H | 42.94 | 43.96 | 44.33 | 41.84 | 41.44 | 46.14 | 0.4154 | 0.4138 | 0.5106 | 0.6252 | 0.5748 | 0.203 |

| PPP_50 sample type | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 16.07 | 14 | 14.05 | 13.27 | 12.57 | 12.25 | 13.63 | 12.53 | 12.36 | 0.1564 | 0.1608 | 0.1605 |
| B | 16 | 14.06 | 14.2 | 13.86 | 12.99 | 13.11 | 13.63 | 12.22 | 12.29 | 0.3797 | 0.6725 | 0.6468 |
| C | 14.61 | 12.62 | 12.46 | 14.74 | 13.86 | 13.64 | 10.69 | 9.641 | 9.7 | 0.8465 | 0.633 | 0.6264 |
| D | 12.15 | 11.09 | 11.15 | 14.53 | 13.72 | 13.49 | 13.43 | 11.84 | 11.79 | 0.5616 | 0.5961 | 0.4925 |
| E | 10.12 | 9.308 | 9.16 | 11.66 | 10.7 | 10.62 | 34.2 | 30.45 | 30.38 | 0.4286 | 0.6031 | 0.4368 |
| F | 14.12 | 12.4 | 12.26 | 15.67 | 14.59 | 14.46 | 24.93 | 21.95 | 21.85 | 0.6686 | 0.7816 | 0.6529 |
| G | 11.83 | 10.42 | 10.24 | 14.65 | 14.22 | 14.02 | 82.95 | 74.87 | 73.76 | 0.4674 | 0.4369 | 0.6844 |
| H | 11.99 | 10.43 | 10.37 | 10.52 | 9.575 | 9.572 | 0.2941 | 0.2665 | 0.2781 | 0.5387 | 0.407 | 0.1992 |
| PRP_50 sample type | | | | | | | | | | | | |
| | 1 | 2 | 3 | 4 | 5 | 6 | 7 | 8 | 9 | 10 | 11 | 12 |
| A | 12.51 | 11.47 | 11.15 | 14.22 | 13.34 | 12.85 | 11.98 | 11.46 | 11.3 | 0.1503 | 0.1642 | 0.1547 |
| B | 15.16 | 14.13 | 13.85 | 11.68 | 11.45 | 11.1 | 11.25 | 10.94 | 10.57 | 0.5681 | 0.7334 | 0.6525 |
| C | 13.71 | 13.08 | 12.93 | 10.11 | 9.665 | 9.232 | 9.491 | 9.029 | 8.844 | 0.8842 | 0.6689 | 0.6514 |
| D | 12.04 | 11.04 | 11.07 | 12.9 | 13 | 12.37 | 10.69 | 10.07 | 10.11 | 0.5693 | 0.4004 | 0.49 |
| E | 10.22 | 9.436 | 9.297 | 12.29 | 10.98 | 10.93 | 31.2 | 28.89 | 28.94 | 0.4111 | 0.5882 | 0.4208 |
| F | 14.85 | 13.69 | 13.46 | 12.56 | 12.15 | 11.87 | 22.71 | 21.44 | 21.16 | 0.7211 | 0.718 | 0.5965 |
| G | 9.097 | 8.439 | 8.524 | 9.08 | 9.072 | 8.546 | 75.92 | 71.71 | 71.87 | 0.305 | 0.6751 | 0.6534 |
| H | 18.91 | 17.42 | 17.08 | 7.561 | 7.455 | 7.199 | 0.2276 | 0.2193 | 0.2188 | 0.5693 | 0.7097 | 0.1141 |

The following raw fluorescence intensities were measured for the Y amino acid type

| PPP sample type, wavelength pair 1 | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 207.9 | 188 | 194 | 293.7 | 252.9 | 257.5 | 328.7 | 344 | 333.7 | 2.221 | 2.225 | 2.22 |
| B | 224.5 | 201 | 204.5 | 227.2 | 216.6 | 206.3 | 223 | 230.7 | 252.4 | 3.671 | 4.368 | 4.133 |
| C | 232.9 | 205 | 214 | 237.4 | 248.3 | 232.3 | 214.1 | 228.9 | 221.7 | 3.753 | 3.865 | 4.223 |
| D | 188.6 | 167.1 | 173.7 | 237.4 | 216.2 | 180.6 | 203.7 | 222.3 | 214.1 | 3.764 | 4.201 | 3.944 |
| E | 187.2 | 169.4 | 173.1 | 233.6 | 220.4 | 218.7 | 173.6 | 193.4 | 186.5 | 3.614 | 3.75 | 3.983 |
| F | 220.8 | 196.3 | 201 | 236.6 | 233.7 | 223.8 | 147.1 | 135.3 | 136 | 3.698 | 4.046 | 3.973 |
| G | 219.1 | 197.7 | 199.3 | 237.7 | 232.9 | 222.1 | 324.2 | 394.8 | 398.8 | 3.638 | 3.999 | 3.851 |
| H | 243 | 208.9 | 210.1 | 251.8 | 245.7 | 209.9 | 3.565 | 3.366 | 3.964 | 3.558 | 3.433 | 1.981 |

| PPP sample type, wavelength pair 2 | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 32.03 | 28.83 | 29.59 | 45.83 | 39.61 | 39.84 | 51.38 | 52.77 | 52.36 | 0.6728 | 0.6811 | 0.6796 |
| B | 34.24 | 30.63 | 31.37 | 35.13 | 33.3 | 31.76 | 33.24 | 33.79 | 37.18 | 0.854 | 0.9283 | 0.8953 |
| C | 35.95 | 31.69 | 33.12 | 36.28 | 38.16 | 35.8 | 30.96 | 35.05 | 34 | 0.8789 | 0.8891 | 0.9146 |
| D | 28.99 | 25.83 | 26.88 | 36.44 | 32.46 | 27.65 | 30.52 | 34.44 | 32.48 | 0.8705 | 0.9066 | 0.8821 |
| E | 28.96 | 26.04 | 26.67 | 36.47 | 33.57 | 33.51 | 23.76 | 25.79 | 24.54 | 0.8557 | 0.8731 | 0.9007 |
| F | 34.29 | 30.67 | 31.38 | 36.5 | 35.54 | 34.07 | 20.83 | 18.7 | 17.81 | 0.8635 | 0.9092 | 0.8985 |
| G | 33.51 | 30.61 | 30.92 | 34.69 | 35.67 | 34.14 | 56.1 | 64.15 | 62.21 | 0.8387 | 0.9021 | 0.8895 |
| H | 36.86 | 32.74 | 32.85 | 40.68 | 39.47 | 32.78 | 0.9487 | 0.9062 | 1.014 | 0.8438 | 0.8313 | 0.6315 |

| PRP sample type, wavelength pair 1 | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 201.1 | 210 | 206.8 | 227.2 | 225.6 | 234.7 | 230.9 | 224.2 | 230.8 | 2.314 | 2.347 | 2.278 |
| B | 187 | 238.1 | 221.3 | 188.2 | 186 | 202.5 | 236.5 | 232.6 | 232.5 | 4.277 | 4.666 | 4.634 |
| C | 260.5 | 250.1 | 243.3 | 219.6 | 214.8 | 220.6 | 223.4 | 216.7 | 214.9 | 4.527 | 4.115 | 4.341 |
| D | 221.2 | 215.7 | 221.9 | 186.5 | 182.8 | 193.9 | 193.9 | 182.4 | 189 | 4.72 | 4.223 | 4.078 |
| E | 197.8 | 182.4 | 188.8 | 210.8 | 194 201.6 | 155.9 | 154.7 | | 155.7 | 4.125 | 4.106 | 4.242 |
| F | 227 | 234.2 | 223.9 | 208 | 203.9 | 216.3 | 116.5 | 111.4 | 114.1 | 4.279 | 4.496 | 4.419 |
| G | 257.9 | 249.1 | 240.9 | 212.9 | 212.8 | 219.8 | 332.8 | 356.4 | 367.8 | 4.042 | 4.349 | 4.371 |
| H | 219.6 | 223.7 | 225.2 | 212.7 | 211.5 | 232 | 3.322 | 3.232 | 3.771 | 4.196 | 4.016 | 2.258 |

| PRP sample type, wavelength pair 2 | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 30.41 | 32.67 | 31.79 | 35.14 | 35.15 | 36.14 | 35.01 | 34.06 | 34.8 | 0.6845 | 0.6852 | 0.6792 |
| B | 28.11 | 36.03 | 33.65 | 28.89 | 28.48 | 30.92 | 36.48 | 35.98 | 35.93 | 0.9388 | 0.985 | 0.9728 |
| C | 39.58 | 37.96 | 37.57 | 33.72 | 33.09 | 33.92 | 34.17 | 33.41 | 32.86 | 0.968 | 0.9234 | 0.9454 |
| D | 33.62 | 33.1 | 33.75 | 28.51 | 28.12 | 29.73 | 29.63 | 27.86 | 29.04 | 0.9894 | 0.9181 | 0.899 |

(continued)

| PRP sample type, wavelength pair 2 | | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| E | 29.65 | 27.95 | 28.86 | 32.66 | 29.58 | 31 | 20.42 | 20.33 | 20.29 | 0.9175 | 0.92 | 0.9384 |
| F | 34.8 | 36.06 | 34.6 | 31.46 | 30.89 | 32.51 | 16.21 | 15.27 | 15.89 | 0.9412 | 0.9785 | 0.9624 |
| G | 38.93 | 36.53 | 35.31 | 32.72 | 32.65 | 33.68 | 53.38 | 56.73 | 58.95 | 0.9224 | 0.957 | 0.9307 |
| H | 33.36 | 34.72 | 34.75 | 32.71 | 32.51 | 35.52 | 0.8893 | 0.8707 | 0.9728 | 0.929 | 0.9309 | 0.6824 |

**Example 11: Determination of Experimental Reference Lines for Patient Proteomes and Subproteomes of Interest**

*Calculation of the amino acid concentration of the labeled amino acid types*

For all patient sample types and all types of labelling reactions, the raw clinical data provided in Example 10 was background corrected by subtracting from each dye-containing well the mean fluorescence intensity which was measured for the dye alone without mixing with patient samples, and then subtracting the fluorescence intensity which was measured for the particular patient sample without dye (which provided the autofluorescence that can be removed during the background correction step as disclosed herein). This removed both fluorescence intensity due to the unreacted dye and fluorescence intensity due to any autofluorescence of the patient samples at the indicated wavelengths. For example, the raw fluorescence intensity data which was provided for labeling the K amino acid type in PPP samples in Example 10 is reproduced below, along with the plate layout

| | 1 | 2 | 3 | 4 | 5 | 6 | 7 | 8 | 9 | 10 | 11 | 12 |
|---|---|---|---|---|---|---|---|---|---|---|---|---|
| A | 1874 | 1835 | 1817 | 1801 | 1770 | 1769 | 2031 | 1994 | 1985 | 3.693 | 3.722 | 3.696 |
| B | 1872 | 1844 | 1831 | 1761 | 1702 | 1707 | 1747 | 1715 | 1717 | 1.684 | 1.906 | 1.666 |
| C | 1842 | 1812 | 1812 | 1778 | 1705 | 1711 | 1743 | 1698 | 1704 | 2.155 | 1.992 | 1.801 |
| D | 1731 | 1690 | 1690 | 1733 | 1702 | 1705 | 1821 | 1788 | 1790 | 1.822 | 2.278 | 1.353 |
| E | 1767 | 1718 | 1718 | 1746 | 1683 | 1686 | 2282 | 2247 | 2254 | 1.392 | 1.529 | 1.809 |
| F | 1799 | 1762 | 1743 | 1879 | 1830 | 1830 | 1331 | 1287 | 1281 | 2.044 | 2.178 | 1.852 |
| G | 1771 | 1741 | 1720 | 1745 | 1696 | 1691 | 1098 | 1072 | 1082 | 1.607 | 1.636 | 2.003 |
| H | 1674 | 1632 | 1632 | 1706 | 1668 | 1687 | 20.99 | 19.96 | 20.53 | 1.888 | 1.288 | 0.6641 |

| | 1 | 2 | 3 | 4 | 5 | 6 | 7 | 8 | 9 | 10 | 11 | 12 |
|---|---|---|---|---|---|---|---|---|---|---|---|---|
| A | 1A | 1A | 1A | 9A | 9A | 9A | 17A | 17A | 17A | DYE | DYE | DYE |
| B | 2A | 2A | 2A | 10A | 10A | 10A | 18A | 18A | 18A | 1A | 2A | 3A |
| C | 3A | 3A | 3A | 11A | 11A | 11A | 19A | 19A | 19A | 4A | 5A | 6A |
| D | 4A | 4A | 4A | 12A | 12A | 12A | 20A | 20A | 20A | 7A | 8A | 9A |
| E | 5A | 5A | 5A | 13A | 13A | 13A | BSA | BSA | BSA | 10A | 11A | 12A |
| F | 6A | 6A | 6A | 14A | 14A | 14A | β-Lac | β-Lac | β-Lac | 13A | 14A | 15A |
| G | 7A | 7A | 7A | 15A | 15A | 15A | LYZ | LYZ | LYZ | 16A | 17A | 18A |
| H | 8A | 8A | 8A | 16A | 16A | 16A | PH | PH | PH | 19A | 20A | Buffer |

To background correct the raw fluorescence intensity data, the mean of the "dye" wells was calculated, this was 3.704. First, 3.704 was subtracted from each well which received the dye, in this case the K-labelling solution. The fluorescence of the unreacted dye was not subtracted from wells that did not receive dye (dark grey wells). The results are shown below.

| | 1 | 2 | 3 | 4 | 5 | 6 | 7 | 8 | 9 | 10 | 11 | 12 |
|---|---|---|---|---|---|---|---|---|---|---|---|---|
| A | 1870.3 | 1831.3 | 1813.3 | 1797.3 | 1766.3 | 1765.3 | 2027.3 | 1990.3 | 1981.3 | | | |
| B | 1868.3 | 1840.3 | 1827.3 | 1757.3 | 1698.3 | 1703.3 | 1743.3 | 1711.3 | 1713.3 | 1.68 | 1.91 | 1.67 |
| C | 1838.3 | 1808.3 | 1808.3 | 1774.3 | 1701.3 | 1707.3 | 1739.3 | 1694.3 | 1700.3 | 2.16 | 1.99 | 1.8 |
| D | 1727.3 | 1686.3 | 1686.3 | 1729.3 | 1698.3 | 1701.3 | 1817.3 | 1784.3 | 1786.3 | 1.82 | 2.28 | 1.35 |
| E | 1763.3 | 1714.3 | 1714.3 | 1742.3 | 1679.3 | 1682.3 | 2278.3 | 2243.3 | 2250.3 | 1.39 | 1.53 | 1.81 |
| F | 1795.3 | 1758.3 | 1739.3 | 1875.3 | 1826.3 | 1826.3 | 1327.3 | 1283.3 | 1277.3 | 2.04 | 2.18 | 1.85 |
| G | 1767.3 | 1737.3 | 1716.3 | 1741.3 | 1692.3 | 1687.3 | 1094.3 | 1068.3 | 1078.3 | 1.61 | 1.64 | 2 |
| H | 1670.3 | 1628.3 | 1628.3 | 1702.3 | 1664.3 | 1683.3 | 17.29 | 16.26 | 16.83 | 1.89 | 1.29 | 0.66 |

Next, to remove any autofluorescence from each unlabeled plasma sample, the fluorescence from the indicated plasma sample which had received buffer (PBS) rather than dye in Example 10 (dark grey wells) was subtracted from all wells of the labelled patient sample above. For example, Sample 1A which did not receive K-labelling solution had a raw fluorescence intensity of 1.68 (well B10); this was subtracted from the fluorescence intensity of the Sample 1A wells which did receive K-labelling solution (wells A1-3). The results were 1868.61, 1829.61, and 1811.61 for the three technical replicates for Patient 1A provided in wells A1-3. Because the protein-containing wells in light grey (E7-H9) did not receive any plasma samples, no plasma value was subtracted from these wells. As a further example, this approach was carried out for all patient PPP K-labelling samples, and the results are reproduced in Table 19.

Table 19: Background corrected fluorescence intensities for the K amino acid type of the PPP samples

| | 1 | 2 | 3 | 4 | 5 | 6 | 7 | 8 | 9 |
|---|---|---|---|---|---|---|---|---|---|
| A | 1868.6 | 1829.6 | 1811.6 | 1795.9 | 1764.9 | 1763.9 | 2025.7 | 1988.7 | 1979.7 |
| B | 1866.4 | 1838.4 | 1825.4 | 1755.9 | 1696.9 | 1701.9 | 1741.3 | 1709.3 | 1711.3 |
| C | 1836.6 | 1806.6 | 1806.6 | 1772.8 | 1699.8 | 1705.8 | 1737.4 | 1692.4 | 1698.4 |
| D | 1725.1 | 1684.1 | 1684.1 | 1727.5 | 1696.5 | 1699.5 | 1816 | 1783 | 1785 |
| E | 1761.3 | 1712.3 | 1712.3 | 1740.3 | 1677.3 | 1680.3 | 2278.3 | 2243.3 | 2250.3 |
| F | 1793.5 | 1756.5 | 1737.5 | 1873.1 | 1824.1 | 1824.1 | 1327.3 | 1283.3 | 1277.3 |
| G | 1765.5 | 1735.5 | 1714.5 | 1739.4 | 1690.4 | 1685.4 | 1094.3 | 1068.3 | 1078.3 |
| H | 1668 | 1626 | 1626 | 1700.7 | 1662.7 | 1681.7 | 17.3 | 16.3 | 16.8 |

The background correction steps illustrated above were performed for all raw labelling fluorescence intensity data which was provided in Example 10. This includes the PPP, PRP, PPP_50, and PRP_50 sample types and for each sample type, the K-labelling microplate, C-labelling microplate, W-labelling microplate, and both wavelength pairs (wavelength pair 1 and wavelength pair 2) of the Y-labelling microplate.

Because an automatic gain adjustment had been selected, the protein-containing wells were used to scale the fluorescence intensity values which were measured for protein solutions on this microplate to the fluorescence intensity values which had been measured for the same protein solutions on the calibration plate for the K amino acid type. This provided a scaling factor which was used to equalize the variable gains between this plate and the calibration plate for which the calibration curve had been calculated. This step is not normally necessary and was performed in this example to compensate for a variable gain setting. The results are illustrated below for the K-labelling plate of the PPP sample type.

| | Background corrected K F.I. this plate | Background corrected K F.I. K Protein Standard Plate | Protein Standard Plate / This Plate | Scaling Factor = Average Protein Standard Plate / This Plate |
|---|---|---|---|---|
| 30 μM BSA | 2257.30 | 2350.34 | 1.04 | 1.02 |
| 30 μM β-Lac | 1295.96 | 1244.34 | 0.96 | |

(continued)

| | Background corrected K F.I. this plate | Background corrected K F.I. K Protein Standard Plate | Protein Standard Plate / This Plate | Scaling Factor = Average Protein Standard Plate / This Plate |
|---|---|---|---|---|
| 60 μM LYZ | 1080.30 | 1145.01 | 1.06 | |

The scaling factor of 1.02 indicates that the values measured on the calibration plate used to calculate the calibration curve were 2% brighter than the values measured for the same solutions on this plate, so the values measured on this plate were multiplied by 1.02 to scale to the calibration curve plate. The results are reproduced below.

| | 1 | 2 | 3 | 4 | 5 | 6 | 7 | 8 | 9 |
|---|---|---|---|---|---|---|---|---|---|
| A | 1906.8 | 1867 | 1848.6 | 1832.6 | 1801 | 1800 | 2067 | 2029.3 | 2020.1 |
| B | 1904.5 | 1876 | 1862.7 | 1791.8 | 1731.6 | 1736.7 | 1776.9 | 1744.2 | 1746.3 |
| C | 1874.2 | 1843.5 | 1843.5 | 1809 | 1734.5 | 1740.6 | 1772.9 | 1727 | 1733.1 |
| D | 1760.4 | 1718.5 | 1718.5 | 1762.8 | 1731.1 | 1734.2 | 1853.1 | 1819.4 | 1821.5 |
| E | 1797.3 | 1747.3 | 1747.3 | 1775.8 | 1711.5 | 1714.6 | 2324.8 | 2289.1 | 2296.3 |
| F | 1830.1 | 1792.4 | 1773 | 1911.4 | 1861.4 | 1861.4 | 1354.4 | 1309.5 | 1303.4 |
| G | 1801.5 | 1770.9 | 1749.5 | 1775 | 1725 | 1719.9 | 1116.7 | 1090.1 | 1100.3 |
| H | 1702.1 | 1659.2 | 1659.2 | 1735.4 | 1696.7 | 1716 | 17.6 | 16.6 | 17.2 |

The scaling step illustrated above was performed for all background corrected labelling fluorescence intensity data which was calculated from the raw labelling fluorescence intensity data which was provided in Example 10. This includes the PPP, PRP, PPP_50, and PRP_50 sample types. For each sample type, this includes the K-labelling fluorescence intensity data, the C-labelling fluorescence intensity data, the W-labelling fluorescence intensity data, and the Y-labelling fluorescence intensity data.

Regarding the Y-labeling fluorescence intensity data, the approach disclosed herein and illustrated in Example 10 was performed where the background corrected fluorescence intensity values for the Y amino acid type at wavelength pair 2 were multiplied by the signal conversion factor (4.97 as calculated in Example 10) and the result was subtracted from the background corrected fluorescence intensity values for the Y amino acid type at wavelength pair 1. At wavelength pair 1, both fluorescence from the Y and W amino acid types can be observed, and this deconvolution at the detection stage step removed any contribution to the fluorescence from the W amino acid type, revealing exclusively the Y-labelling fluorescence intensity data. As a further example, the background corrected fluorescence intensity values for the PRP sample type at Y-labelling wavelength pair 1 and wavelength pair 2 are provided below.

Wavelength pair 1

| | 1 | 2 | 3 | 4 | 5 | 6 | 7 | 8 | 9 |
|---|---|---|---|---|---|---|---|---|---|
| A | 194.51 | 203.41 | 200.21 | 220.809 | 219.209 | 228.309 | 224.238 | 217.538 | 224.138 |
| B | 180.021 | 231.121 | 214.321 | 181.762 | 179.562 | 196.062 | 229.816 | 225.916 | 225.816 |
| C | 253.553 | 243.153 | 236.353 | 213.181 | 208.381 | 214.181 | 216.891 | 210.191 | 208.391 |
| D | 214.36 | 208.86 | 215.06 | 179.945 | 176.245 | 187.245 | 187.571 | 176.071 | 182.671 |
| E | 191.372 | 175.972 | 182.372 | 204.208 | 187.408 | 195.008 | 153.587 | 152.387 | 153.387 |
| F | 220.346 | 227.546 | 217.246 | 201.191 | 197.091 | 209.491 | 114.187 | 109.087 | 111.787 |
| G | 250.867 | 242.067 | 233.867 | 206.168 | 206.068 | 213.068 | 330.487 | 354.087 | 365.487 |
| H | 213.064 | 217.164 | 218.664 | 206.345 | 205.145 | 225.645 | 1.009 | 0.919 | 1.458 |

Wavelength pair 2

| | 1 | 2 | 3 | 4 | 5 | 6 | 7 | 8 | 9 |
|---|---|---|---|---|---|---|---|---|---|
| A | 29.4712 | 31.7312 | 30.8512 | 34.241 | 34.251 | 35.241 | 34.053 | 33.103 | 33.843 |
| B | 27.125 | 35.045 | 32.665 | 27.9725 | 27.5625 | 30.0025 | 34.0793 | 33.1293 | 33.8693 |
| C | 38.6072 | 36.9872 | 36.5972 | 32.8 | 32.17 | 33 | 33.241 | 32.481 | 31.931 |
| D | 32.652 | 32.132 | 32.782 | 27.5716 | 27.1816 | 28.7916 | 28.6991 | 26.9291 | 28.1091 |
| E | 28.7266 | 27.0266 | 27.9366 | 31.7188 | 28.6388 | 30.0588 | 19.73703 | 19.64703 | 19.60703 |
| F | 33.8546 | 35.1146 | 33.6546 | 30.4815 | 29.9115 | 31.5315 | 15.52703 | 14.58703 | 15.20703 |
| G | 37.9406 | 35.5406 | 34.3206 | 31.7576 | 31.6876 | 32.7176 | 52.69703 | 56.04703 | 58.26703 |
| H | 32.4419 | 33.8019 | 33.8319 | 31.7876 | 31.5876 | 34.5976 | 0.206333 | 0.187733 | 0.289833 |

The wavelength pair 2 fluorescence intensities were multiplied by the scaling factor, 4.97, to reveal the fluorescence intensity from amino acids of the W amino acid type at wavelength pair 1

| | 1 | 2 | 3 | 4 | 5 | 6 | 7 | 8 | 9 |
|---|---|---|---|---|---|---|---|---|---|
| A | 146.565586 | 157.805 | 153.4286 | 170.2867 | 170.3364 | 175.2598 | 169.3517 | 164.6272 | 168.3073 |
| B | 134.897511 | 174.2851 | 162.4489 | 139.1123 | 137.0733 | 149.2078 | 169.4825 | 164.758 | 168.4381 |
| C | 192.000559 | 183.944 | 182.0045 | 163.1203 | 159.9872 | 164.1149 | 165.3135 | 161.5339 | 158.7986 |
| D | 162.384277 | 159.7982 | 163.0308 | 137.1185 | 135.179 | 143.1858 | 142.7258 | 133.9233 | 139.7916 |
| E | 142.862556 | 134.4081 | 138.9337 | 157.7433 | 142.4259 | 149.4878 | 98.15582 | 97.70824 | 97.50931 |
| F | 168.365023 | 174.6312 | 167.3704 | 151.59 | 148.7553 | 156.8118 | 77.21873 | 72.54394 | 75.62732 |
| G | 188.685437 | 176.7498 | 170.6825 | 157.9363 | 157.5881 | 162.7105 | 262.0718 | 278.732 | 289.7725 |
| H | 161.339412 | 168.1029 | 168.2521 | 158.0855 | 157.0908 | 172.0601 | 1.026133 | 0.933632 | 1.441393 |

These values were then subtracted from the provided Wavelength pair 1 values (which included contributions from both the W and Y amino acid types), revealing the fluorescence intensity exclusively from the Y amino acid type at wavelength pair 1 and thus the Y-labelling fluorescence intensity data.

| | 1 | 2 | 3 | 4 | 5 | 6 | 7 | 8 | 9 |
|---|---|---|---|---|---|---|---|---|---|
| A | 47.9444143 | 45.60503 | 46.78143 | 50.52234 | 48.87261 | 53.04916 | 54.8863 | 52.91082 | 55.83067 |
| B | 45.1234895 | 56.8359 | 51.87207 | 42.64972 | 42.48872 | 46.85416 | 60.3335 | 61.15802 | 57.37787 |
| C | 61.5524408 | 59.20899 | 54.34853 | 50.06069 | 48.3938 | 50.06606 | 51.57752 | 48.65714 | 49.59239 |
| D | 51.975723 | 49.06178 | 52.02921 | 42.82647 | 41.06601 | 44.05919 | 44.84521 | 42.14774 | 42.87938 |
| E | 48.5094442 | 41.56385 | 43.43826 | 46.46469 | 44.98209 | 45.52017 | 55.43118 | 54.67876 | 55.87769 |
| F | 51.9809766 | 52.91477 | 49.87561 | 49.60101 | 48.33572 | 52.67917 | 36.96827 | 36.54306 | 36.15968 |
| G | 62.1815626 | 65.31719 | 63.18447 | 48.23174 | 48.47986 | 50.35748 | 68.41516 | 75.35501 | 75.71455 |
| H | 51.7245881 | 49.06106 | 50.41187 | 48.25954 | 48.05418 | 53.5849 | -0.01713 | -0.01463 | 0.016607 |

This Y-labelling fluorescence intensity data was then scaled in the same manner as the K-labelling, C-labelling, and W-labelling fluorescence intensity data.

The relationship between fluorescence intensity (C F.I., K F.I., W F.I., and Y F.I.) and amino acid concentration ([C (μM)], [K (μM)], [W (μM)], and [Y (μM)]) was established using a calibration curve for each amino acid type (K, C, W, and Y) in Example 10. The transformations are summarized in Table 20.

Table 20: Calibration curves to transform between fluorescence intensity in AU and amin acid concentration in μM

| Amino Acid Type | Transformation |
|---|---|
| Cysteine, total (C) | $[C(uM)] = 1.474 \times 10^{-2}[C\ F.I.\ (AU)]^2 + 14.780[C\ F.I.\ (AU)] + 23.117$ |
| Lysine (K) | $[K(uM)] = 7.642 \times 10^{-11}[K\ F.I.\ (AU)]^4 - 1.2 \times 10^{-7}[K\ F.I.\ (AU)]^3 + 5.17 \times 10^{-5}[K\ F.I.\ (AU)]^2 + 0.324[K\ F.I.\ (AU)] - 19.720$ |
| Tryptophan (W) | $[W\ (uM)] = 2.315[W\ F.I.\ (AU)]$ |
| Tyrosine (Y) | $[Y\ (uM)] = 7.381[Y\ F.I.\ (AU)]$ |

The C amino acid type transformation provided in table 20 was applied to the scaled C fluorescence intensity data from all sample types (PPP, PRP, PPP_50, PRP_50). The K amino acid type transformation provided in table 20 was applied to the scaled K fluorescence intensity data from all sample types (PPP, PRP, PPP_50, PRP_50). The W amino acid type transformation provided in table 20 was applied to the scaled W fluorescence intensity data from all sample types (PPP, PRP, PPP_50, PRP_50). The Y amino acid type transformation provided in table 20 was applied to the scaled Y fluorescence intensity data from all sample types (PPP, PRP, PPP_50, PRP_50).

For example, the scaled PPP data for the C amino acid type is below

| | 1 | 2 | 3 | 4 | 5 | 6 | 7 | 8 | 9 |
|---|---|---|---|---|---|---|---|---|---|
| A | 26.2 | 24.2 | 22.5 | 24.9 | 24.6 | 26.8 | 36.2 | 37.1 | 37.2 |
| B | 28.9 | 28.6 | 28.9 | 25.7 | 25.8 | 25.8 | 27.7 | 27.4 | 28.0 |
| C | 29.8 | 29.6 | 30.0 | 26.8 | 26.5 | 26.9 | 27.2 | 27.4 | 27.2 |
| D | 25.6 | 24.7 | 24.6 | 25.9 | 25.7 | 26.3 | 30.2 | 30.6 | 30.8 |
| E | 28.5 | 26.8 | 27.1 | 26.4 | 25.6 | 26.0 | 73.6 | 72.8 | 74.1 |
| F | 30.2 | 28.0 | 28.0 | 32.9 | 33.3 | 33.3 | 9.8 | 9.5 | 9.6 |
| G | 30.5 | 29.4 | 28.9 | 27.5 | 27.9 | 28.0 | 25.5 | 25.3 | 25.5 |
| H | 26.4 | 25.6 | 25.9 | 25.9 | 26.1 | 26.2 | 0.0 | 0.0 | 0.0 |

The C amino acid type transformation provided in Table 20 was carried out yielding the micromolar C amino acid concentrations, [C (μM)], below

| | 1 | 2 | 3 | 4 | 5 | 6 | 7 | 8 | 9 |
|---|---|---|---|---|---|---|---|---|---|
| A | 420.54 | 389 | 363.1 | 400.4 | 396 | 429.9 | 577.6 | 592 | 594 |
| B | 462.17 | 458 | 461.9 | 413.2 | 414 | 414.8 | 444.4 | 439 | 448 |
| C | 477.21 | 472.8 | 480.1 | 430.3 | 425 | 432.1 | 435.6 | 440 | 436 |
| D | 410.53 | 396.8 | 396.2 | 416.1 | 413 | 422.6 | 483.1 | 489 | 492 |
| E | 457.03 | 430.5 | 434.5 | 423.3 | 411 | 417.2 | 1190 | 1177 | 1199 |
| F | 483.33 | 448.1 | 448.4 | 525.9 | 532 | 532.3 | 169.6 | 164 | 167 |
| G | 488.34 | 471.1 | 461.8 | 441.1 | 446 | 447.9 | 408.9 | 407 | 409 |
| H | 423.79 | 411.6 | 415.8 | 415.9 | 419 | 420.4 | 23.15 | 23.1 | 23.1 |

Finally, the amino acid concentrations calculated for each amino acid type were multiplied by the dilution factors provided in Table 18. This provided the amino acid concentrations of undiluted plasma solutions. As an example, the

diluted amino acid concentration measured for the C labeling reaction for the PPP sample type, Patient 1 (Sample 1A), technical replicate 2 was 389. The dilution provided in Tables 24 and 30 was 60, so the amino acid concentration (of undiluted PPP plasma) for this technical replicate of this sample was 389 μM x 60 = 23,340 μM.

*Amino acid concentrations (μM) for each patient sample*

Thus far, all of the steps explained in detail were been used to calculate the amino acid concentrations in μM for the patient (undiluted) plasma plasma samples.

When all of these steps were carried out as explained disclosed herein and explained for each patient, technical replicate, amino acid type, and sample type, the amino acid concentrations in μM were as presented below in Tables 21.1 and 21.2 for PPP, tables 22.1 and 22.2 for PRP, tables 23.1 and 23.2 for PPP_50, tables 24.1 and 24.2 for PRP_50

Table 21.1

| PPP Samples | [K (μM)] | | | [C (μM)] | | |
|---|---|---|---|---|---|---|
| | Replicate 1 | Replicate 2 | Replicate 3 | Replicate 1 | Replicate 2 | Replicate 3 |
| 1A | 59,429.60 | 56,252.16 | 54,850.28 | 25,232.31 | 23,341.67 | 21,785.33 |
| 2A | 59,243.29 | 56,950.41 | 55,919.75 | 27,730.02 | 27,481.75 | 27,713.46 |
| 3A | 56,809.64 | 54,469.28 | 54,469.28 | 28,632.44 | 28,367.15 | 28,806.62 |
| 4A | 48,647.74 | 45,993.62 | 45,993.62 | 24,632.02 | 23,810.11 | 23,769.05 |
| 5A | 51,138.04 | 47,797.88 | 47,797.88 | 27,422.02 | 25,827.97 | 26,067.16 |
| 6A | 53,479.08 | 50,798.52 | 49,482.32 | 29,000.03 | 26,888.31 | 26,904.83 |
| 7A | 51,434.57 | 49,344.66 | 47,940.31 | 29,300.37 | 28,263.47 | 27,708.58 |
| 8A | 44,996.89 | 42,518.72 | 42,518.72 | 25,427.38 | 24,694.63 | 24,949.73 |
| 9A | 53,662.06 | 51,396.70 | 51,325.47 | 24,025.98 | 23,754.93 | 25,795.57 |
| 10A | 50,756.97 | 46,801.17 | 47,122.08 | 24,791.80 | 24,841.17 | 24,890.55 |
| 11A | 51,957.94 | 46,984.60 | 47,371.78 | 25,819.81 | 25,506.56 | 25,927.02 |
| 12A | 48,804.96 | 46,774.52 | 46,966.63 | 24,968.42 | 24,803.81 | 25,355.45 |
| 13A | 49,670.83 | 45,564.57 | 45,750.83 | 25,400.35 | 24,675.87 | 25,029.75 |
| 14A | 59,809.42 | 55,820.04 | 55,820.04 | 31,552.77 | 31,894.87 | 31,936.60 |
| 15A | 49,615.50 | 46,390.35 | 46,075.30 | 26,466.79 | 26,772.38 | 26,871.53 |
| 16A | 47,043.86 | 44,673.09 | 45,840.36 | 24,954.18 | 25,135.30 | 25,225.89 |
| 17A | 74,362.19 | 70,513.91 | 69,610.08 | 34,654.46 | 35,527.99 | 35,620.47 |
| 18A | 49,742.23 | 47,601.06 | 47,731.68 | 26,664.58 | 26,359.05 | 26,904.17 |
| 19A | 49,476.38 | 46,514.80 | 46,897.43 | 26,137.54 | 26,393.39 | 26,162.30 |
| 20A | 55,188.97 | 52,703.21 | 52,850.18 | 28,987.00 | 29,327.36 | 29,518.40 |

Table 21.2

| PPP Samples | [W (μM)] | | | [Y (μM)] | | |
|---|---|---|---|---|---|---|
| | Replicate 1 | Replicate 2 | Replicate 3 | Replicate 1 | Replicate 2 | Replicate 3 |
| 1A | 4,856.31 | 4,387.44 | 4,528.34 | 18,404.32 | 16,842.32 | 17,712.47 |
| 2A | 5,204.12 | 4,662.36 | 4,768.04 | 20,474.19 | 18,300.46 | 18,229.86 |
| 3A | 5,460.69 | 4,843.62 | 5,071.98 | 20,461.16 | 17,829.92 | 18,569.95 |
| 4A | 4,391.51 | 3,891.05 | 4,059.89 | 16,782.02 | 14,515.04 | 15,055.13 |
| 5A | 4,342.42 | 3,931.85 | 4,025.38 | 16,267.83 | 14,983.10 | 15,205.26 |
| 6A | 5,239.86 | 4,683.52 | 4,780.70 | 18,956.88 | 16,410.75 | 16,868.88 |
| 7A | 5,082.67 | 4,675.75 | 4,734.05 | 19,904.77 | 17,170.26 | 17,193.11 |
| 8A | 5,611.52 | 4,967.73 | 4,998.09 | 22,641.08 | 17,307.28 | 17,563.17 |
| 9A | 6,979.51 | 5,968.87 | 6,030.82 | 25,080.88 | 21,214.18 | 22,568.62 |
| 10A | 5,132.72 | 4,993.03 | 4,740.37 | 19,951.73 | 19,364.26 | 18,329.17 |
| 11A | 5,864.12 | 5,837.39 | 5,424.39 | 21,688.34 | 22,295.93 | 20,625.20 |
| 12A | 5,449.59 | 4,956.42 | 4,187.51 | 21,338.99 | 20,787.71 | 16,210.84 |
| 13A | 5,411.92 | 5,051.15 | 5,079.09 | 19,830.53 | 20,309.51 | 19,760.24 |
| 14A | 5,581.63 | 5,330.18 | 5,120.04 | 20,900.42 | 21,634.92 | 20,620.15 |
| 15A | 5,153.09 | 5,302.50 | 5,171.31 | 24,866.82 | 21,075.80 | 19,825.27 |
| 16A | 5,872.15 | 5,498.02 | 5,006.06 | 18,733.08 | 18,700.77 | 17,709.52 |
| 17A | 7,745.56 | 8,232.66 | 8,088.11 | 27,997.83 | 31,284.71 | 28,047.32 |
| 18A | 5,006.73 | 5,075.97 | 5,651.74 | (13,391.37) | (13,082.84) | (3,779.79) |
| 19A | 4,791.08 | 5,304.90 | 5,189.51 | 22,943.80 | 20,772.61 | 19,997.39 |
| 20A | 4,845.89 | 5,107.05 | 4,873.83 | 19,750.31 | 19,399.62 | 20,006.04 |

Table 22.1

| PRP Samples | [K (μM)] | | | [C (μM)] | | |
|---|---|---|---|---|---|---|
| | Replicate 1 | Replicate 2 | Replicate 3 | Replicate 1 | Replicate 2 | Replicate 3 |
| 1C | 72,513.19 | 64,122.74 | 63,224.08 | 25,236.34 | 27,180.30 | 25,784.07 |
| 2C | 81,679.71 | 78,000.81 | 76,165.57 | 30,578.66 | 30,546.11 | 29,351.43 |
| 3C | 77,551.65 | 73,364.43 | 73,132.43 | 30,140.90 | 30,889.44 | 29,182.46 |
| 4C | 72,597.95 | 67,297.91 | 68,044.43 | 19,866.80 | 15,349.81 | 21,765.51 |
| 5C | 59,227.84 | 54,642.50 | 55,065.59 | 27,027.97 | 36,702.99 | 26,349.75 |
| 6C | 66,303.52 | 64,251.98 | 62,269.23 | 29,861.87 | 28,741.78 | 28,036.93 |
| 7C | 67,230.31 | 65,147.80 | 63,233.99 | 30,125.94 | 30,190.99 | 29,378.55 |
| 8C | 53,040.70 | 51,126.60 | 50,046.78 | 26,164.45 | 26,414.55 | 25,785.52 |
| 9C | 65,211.51 | 62,801.74 | 62,703.46 | 27,566.29 | 27,841.37 | 27,695.72 |
| 10C | 56,316.23 | 54,351.04 | 53,851.23 | 26,477.25 | 26,453.04 | 26,323.93 |
| 11C | 65,190.10 | 61,709.71 | 61,420.99 | 27,696.69 | 28,141.87 | 27,502.56 |
| 12C | 57,602.12 | 54,904.04 | 55,073.75 | 26,301.91 | 26,665.09 | 26,059.93 |
| 13C | 60,883.52 | 58,922.74 | 58,466.28 | 27,529.81 | 27,610.69 | 26,850.90 |
| 14C | 71,703.20 | 66,159.51 | 65,229.86 | 34,213.03 | 35,172.84 | 34,344.18 |
| 15C | 61,385.96 | 58,581.02 | 57,857.27 | 27,134.64 | 27,134.64 | 26,908.41 |
| 21C | 58,173.76 | 54,512.51 | 54,849.77 | 26,451.02 | 27,161.71 | 26,507.52 |
| 22C | 78,879.90 | 73,911.87 | 75,331.24 | 34,125.17 | 33,969.50 | 33,150.97 |
| 23C | 63,363.46 | 61,602.90 | 62,183.73 | 26,900.82 | 27,628.33 | 26,480.96 |
| 24C | 71,518.11 | 67,886.82 | 67,779.90 | 28,129.64 | 28,226.82 | 27,530.78 |
| 25C | 55,659.50 | 53,226.93 | 55,316.60 | 26,561.03 | 27,320.38 | 26,335.07 |

Table 22.2

| PRP Samples | [W (μM)] | | | [Y (μM)] | | |
|---|---|---|---|---|---|---|
| | Replicate 1 | Replicate 2 | Replicate 3 | Replicate 1 | Replicate 2 | Replicate 3 |
| 1C | 5,402.22 | 5,650.78 | 5,558.10 | 20,832.54 | 19,816.05 | 20,327.21 |
| 2C | 4,951.64 | 6,368.61 | 5,938.88 | 19,606.81 | 24,696.02 | 22,539.17 |
| 3C | 7,093.51 | 6,771.92 | 6,620.25 | 26,745.43 | 25,727.17 | 23,615.23 |
| 4C | 5,964.31 | 5,840.73 | 5,969.93 | 22,584.21 | 21,318.05 | 22,607.45 |
| 5C | 5,261.43 | 4,866.81 | 5,040.95 | 21,078.06 | 18,060.10 | 18,874.55 |
| 6C | 6,186.82 | 6,375.00 | 6,103.96 | 22,586.49 | 22,992.23 | 21,671.68 |
| 7C | 6,929.46 | 6,460.41 | 6,263.80 | 27,018.79 | 28,381.27 | 27,454.57 |
| 8C | 5,920.40 | 6,063.64 | 6,115.60 | 22,475.08 | 21,317.74 | 21,904.69 |
| 9C | 6,203.85 | 6,210.87 | 6,432.76 | 21,952.69 | 21,235.86 | 23,050.63 |
| 10C | 5,047.13 | 4,969.89 | 5,343.44 | 18,531.92 | 18,461.97 | 20,358.81 |
| 11C | 5,981.71 | 5,842.68 | 5,987.33 | 21,752.10 | 21,027.81 | 21,754.43 |
| 12C | 4,988.50 | 4,905.64 | 5,179.49 | 18,608.72 | 17,843.78 | 19,144.36 |
| 13C | 5,737.81 | 5,173.27 | 5,437.28 | 20,189.58 | 19,545.37 | 19,779.18 |
| 14C | 5,516.30 | 5,422.21 | 5,741.00 | 21,552.36 | 21,002.57 | 22,889.86 |
| 15C | 5,787.41 | 5,734.05 | 5,919.42 | 20,957.39 | 21,065.20 | 21,881.06 |
| 21C | 5,773.28 | 5,717.11 | 6,377.15 | 20,969.47 | 20,880.24 | 23,283.42 |
| 22C | 6,207.18 | 6,023.21 | 6,177.69 | 23,848.89 | 22,990.52 | 24,259.23 |
| 23C | 6,464.31 | 6,361.80 | 6,364.60 | 26,215.78 | 26,574.05 | 24,931.52 |
| 24C | 6,051.09 | 5,861.50 | 5,820.78 | 22,411.18 | 21,142.23 | 21,548.61 |
| 25C | 5,195.90 | 4,798.48 | 5,061.09 | 19,485.89 | 18,313.80 | 18,631.71 |

Table 23.1

| PPP 50kDa Samples | [K (µM)] | | | [C (µM)] | | |
|---|---|---|---|---|---|---|
| | Replicate 1 | Replicate 2 | Replicate 3 | Replicate 1 | Replicate 2 | Replicate 3 |
| 1B | 16,127.04 | 14,644.92 | 14,198.03 | 173.65 | 170.31 | 163.14 |
| 2B | 13,809.16 | 13,476.97 | 14,018.14 | 131.73 | 128.70 | 124.21 |
| 3B | 10,924.61 | 11,163.83 | 10,964.18 | 145.14 | 143.77 | 137.21 |
| 4B | 11,618.48 | 11,590.79 | 11,412.10 | 196.61 | 187.06 | 183.81 |
| 5B | 8,220.36 | 8,493.61 | 8,440.47 | 85.85 | 85.69 | 83.26 |
| 6B | 11,397.73 | 10,981.27 | 11,021.01 | 122.46 | 120.61 | 117.39 |
| 7B | 8,074.48 | 8,208.09 | 8,013.44 | 86.49 | 85.36 | 82.93 |
| 8B | 7,409.39 | 7,718.13 | 7,514.29 | 97.04 | 97.49 | 95.47 |
| 9B | 14,431.47 | 14,765.82 | 14,168.46 | 195.21 | 192.55 | 194.52 |
| 10B | 11,343.92 | 11,466.77 | 11,549.28 | 265.61 | 262.92 | 222.96 |
| 11B | 10,865.07 | 11,010.09 | 10,838.88 | 229.55 | 247.68 | 237.17 |
| 12B | 12,460.85 | 12,490.25 | 12,475.54 | 135.39 | 136.47 | 129.04 |
| 13B | 11,313.00 | 10,447.07 | 10,447.07 | 103.01 | 96.76 | 92.71 |
| 14B | 10,801.65 | 10,906.45 | 10,710.64 | 170.49 | 163.61 | 163.52 |
| 15B* | 7,918.21 | 7,719.65 | 7,838.27 | 247.51 | 231.95 | 234.52 |
| 16B | 8,540.60 | 8,476.57 | 8,487.21 | 79.83 | 79.69 | 78.66 |
| 17B | 14,380.82 | 13,355.12 | 13,231.23 | 179.72 | 169.01 | 186.81 |
| 18B | 10,134.24 | 10,060.25 | 10,196.25 | 132.22 | 132.03 | 125.58 |
| 19B | 8,620.96 | 8,333.72 | 8,524.29 | 63.69 | 64.01 | 65.87 |
| 20B | 13,486.87 | 13,424.31 | 12,977.79 | 109.29 | 109.21 | 110.13 |

* Outlier

Table 23.2

| PPP 50kDa Samples | [W (µM)] | | |
|---|---|---|---|
| | Replicate 1 | Replicate 2 | Replicate 3 |
| 1B | 64.96 | 56.31 | 56.51 |
| 2B | 63.45 | 55.33 | 55.92 |
| 3B | 57.74 | 49.42 | 48.75 |
| 4B | 46.61 | 42.18 | 42.43 |
| 5B | 39.02 | 35.62 | 35.00 |
| 6B | 55.78 | 48.58 | 48.00 |
| 7B | 46.47 | 40.57 | 39.82 |
| 8B | 46.99 | 40.47 | 40.22 |
| 9B | 52.78 | 49.85 | 48.51 |
| 10B | 55.52 | 51.88 | 52.38 |
| 11B | 58.47 | 54.79 | 53.86 |
| 12B | 58.28 | 54.90 | 53.93 |
| 13B | 45.31 | 41.29 | 40.96 |
| 14B | 61.61 | 57.09 | 56.55 |

| | | | |
|---|---|---|---|
| 15B* | 57.88 | 56.08 | 55.25 |
| 16B | 41.38 | 37.43 | 37.42 |
| 17B | 54.52 | 49.92 | 49.21 |
| 18B | 53.48 | 47.59 | 47.88 |
| 19B | 41.80 | 37.41 | 37.65 |
| 20B | 53.81 | 47.16 | 46.95 |

* Outlier

Table 24.1

| PRP 50kDa Samples | [K (µM)] | | | [C (µM)] | | |
|---|---|---|---|---|---|---|
| | Replicate 1 | Replicate 2 | Replicate 3 | Replicate 1 | Replicate 2 | Replicate 3 |
| 1D | 11,332.97 | 10,949.99 | 11,173.17 | 99.68 | 97.79 | 93.44 |
| 2D | 11,546.66 | 11,614.85 | 12,101.55 | 176.11 | 171.94 | 166.88 |
| 3D | 9,823.56 | 9,979.14 | 10,075.91 | 162.44 | 158.65 | 155.50 |
| 4D | 10,024.49 | 10,480.38 | 10,518.09 | 145.18 | 141.81 | 136.11 |
| 5D | 7,800.61 | 8,128.97 | 7,988.25 | 130.78 | 127.01 | 122.47 |
| 6D | 10,818.07 | 11,117.88 | 11,197.22 | 266.96 | 262.87 | 251.10 |
| 7D | 7,190.43 | 7,412.01 | 7,449.51 | 70.04 | 70.01 | 67.57 |
| 8D* | 678.76 | 720.83 | 720.83 | 205.95 | 198.97 | 196.30 |
| 9D | 13,955.29 | 14,357.38 | 14,768.87 | 217.17 | 218.57 | 216.92 |
| 10D | 10,108.15 | 10,416.45 | 10,913.06 | 216.95 | 219.50 | 215.43 |
| 11D | 10,429.20 | 10,342.00 | 10,580.10 | 134.95 | 137.78 | 133.21 |
| 12D | 11,162.00 | 11,442.66 | 11,335.06 | 163.08 | 163.21 | 158.66 |
| 13D | 10,087.69 | 10,148.62 | 10,333.29 | 260.15 | 255.81 | 244.57 |
| 14D | 10,409.92 | 10,971.31 | 11,155.13 | 358.75 | 311.11 | 309.57 |
| 15D | 7,677.89 | 7,961.96 | 8,234.48 | 174.42 | 179.35 | 175.55 |
| 21D | 9,458.65 | 9,632.74 | 9,691.36 | 272.17 | 281.01 | 269.22 |
| 22D | 9,680.10 | 9,977.45 | 10,159.50 | 173.77 | 165.55 | 158.73 |
| 23D | 11,670.37 | 12,045.84 | 11,808.29 | 97.01 | 95.51 | 94.52 |
| 24D | 15,778.25 | 16,061.36 | 16,061.36 | 106.72 | 106.36 | 103.93 |
| 25D | 10,278.86 | 10,241.87 | 10,204.98 | 104.08 | 101.48 | 97.79 |

* Outlier

Table 24.2

| PRP 50kDa Samples | [W (μM)] | | |
|---|---|---|---|
| | Replicate 1 | Replicate 2 | Replicate 3 |
| 1D | 52.18 | 47.57 | 46.15 |
| 2D | 63.18 | 58.62 | 57.38 |
| 3D | 57.11 | 54.33 | 53.66 |
| 4D | 48.70 | 44.27 | 44.40 |
| 5D | 41.59 | 38.12 | 37.51 |
| 6D | 62.17 | 57.03 | 56.01 |
| 7D | 37.06 | 34.15 | 34.52 |
| 8D* | 81.25 | 74.66 | 73.15 |
| 9D | 60.09 | 56.20 | 54.03 |
| 10D | 49.20 | 48.18 | 46.63 |
| 11D | 41.46 | 39.49 | 37.57 |
| 12D | 54.55 | 55.00 | 52.21 |
| 13D | 50.52 | 44.72 | 44.50 |
| 14D | 51.73 | 49.92 | 48.68 |
| 15D | 36.87 | 36.83 | 34.50 |
| 21D | 31.43 | 30.96 | 29.83 |
| 22D | 49.36 | 47.05 | 46.35 |
| 23D | 46.22 | 44.85 | 43.21 |
| 24D | 38.80 | 36.76 | 35.94 |
| 25D | 43.49 | 40.75 | 40.92 |

* Outlier

*Plotting the amino acid concentration results in N-dimensional space reveals the reference lines disclosed by the methods of the invention*

The invention is based on the discovery that the reference for a proteome or subproteome of interest, provided by amino acid concentrations or values of the label of two or more labeled amino acid types, is a function of the common parameter of the total protein concentration of the proteome or subproteome of interest. Therefore, as the total protein concentration of the proteome or subproteome of interest changes, a line is formed in N-dimensional space. This concept was explained in Figure 1 and Examples 1-9, and was further illustrated in Figure 17.

The concept of the line in N-dimensional space, taught by the methods of the invention, is provided for the PPP proteome of interest in Figure 21. The mean of the amino acid concentrations provided for each technical replicate in Tables 21.1 and 21.2 is shown, with the fourth dimension indicated colorimetrically in grayscale. In Figure 21, the concept of the line in N-dimensional space (N=4, 4-dimensional space) is illustrated with a line drawn through the dataset.

To calculate the exact position of the line in N-dimensional space for the PPP proteome of interest and the other proteomes of interest (e.g. PRP, PPP_50, and PRP_50), the equation of the line in N-dimensional space was calculated experimentally. Later, the equation of this experimental line in N-dimensional space was compared to the equation of the theoretical line in N-dimensional space calculated using protein expression data which was publicly available for the PPP proteome of interest.

*Calculation of experimental reference lines for the PPP, PRP, PPP_50, and PRP_50 proteomes and subproteomes* of *interest*

In the methods of the invention disclosed herein, the vector functions which define the reference lines of a proteome or subproteome of interest can be calculated experimentally, for example by dividing the measured amino acid

concentrations by the total protein concentration (e.g. the mass protein concentration) calculated using standard methods in the art. To do this, the total protein concentration of each patient sample was measured in μg/mL using a bicinchoninic acid protein determination kit (Product # BCA1-1KT, Millipore Sigma) using the manufacturer's provided instructions. The bicinchoninic acid (BCA) standard assay was used for determination of total protein concentration. Briefly, peptide bonds within proteins reduce $Cu^{2+}$ to $Cu^{1+}$, with the amount of reduction proportional to the amount of protein present. BCA then forms a purple-blue complex with $Cu^{1+}$ in alkaline environments, and this complex is quantified via measuring absorption at 562 nm with the protein concentration (by mass, such as in μg/mL) determined by comparison to a standard curve generated for a protein of known concentration, here 1.00 mg/mL BSA.

The results for the total protein concentration of each patient sample measured in this way in accordance with the manufacturer's instructions are provided in Table 25. Three total protein concentrations are provided for each sample, covering a 1:60 dilution, 1:2 dilution, and 1:1 dilution (neat, undiluted). As explained in Table 12 and Table 18, the 1:60 dilution was used for the PPP and PRP labelling microplates, the 1:2 dilution was used for the PPP_50 and PRP_50 K and WY-labeling microplates (the WY labeling microplate revealing the amino acid concentrations of the W and Y amino acid types), and the 1:1 dilution was used for the PPP_50 and PRP_50 C labelling microplate. When calculating the experimental reference lines, the amino acid concentration of each amino acid type was plotted against the total protein concentration at the dilution at which the amino acid concentrations for that amino acid type had been measured. The indicated dilutions are bolded in the table 25 for ease of reference.

Table 25: Total protein concentration of each protein of each patient sample measured with the BCA assay for the calculation of experimental reference lines

| | | Mean Total Concentration (μg/mL) | | | Std Total Concentration (μg/mL) | | |
|---|---|---|---|---|---|---|---|
| Sample Type | Sample | 1:60 dilution | 1:2 dilution | 1:1 dilution | 1:60 dilution | 1:2 dilution | 1:1 dilution |
| PPP | 1A | **1142.0** | 34259.3 | 68518.7 | **58.3** | 1748.7 | 3497.3 |
| | 2A | **1153.9** | 34617.3 | 69234.7 | **63.9** | 1917.6 | 3835.1 |
| | 3A | **1207.4** | 36220.6 | 72441.2 | **22.7** | 680.5 | 1360.9 |
| | 4A | **1054.3** | 31628.8 | 63257.6 | **47.7** | 1431.9 | 2863.8 |
| | 5A | **1000.9** | 30025.6 | 60051.2 | **43.7** | 1310.0 | 2620.0 |
| | 6A | **1101.0** | 33029.7 | 66059.4 | **50.8** | 1524.9 | 3049.7 |
| | 7A | **1174.7** | 35240.0 | 70479.9 | **51.6** | 1546.9 | 3093.7 |
| | 8A | **1056.9** | 31706.6 | 63413.3 | **46.7** | 1400.9 | 2801.8 |
| | 9A | **1174.7** | 35240.0 | 70479.9 | **25.0** | 750.6 | 1501.1 |
| | 10A | **1011.7** | 30352.5 | 60704.9 | **42.4** | 1271.1 | 2542.2 |
| | 11A | **1111.4** | 33341.0 | 66682.0 | **42.0** | 1260.8 | 2521.6 |
| | 12A | **1001.4** | 30041.2 | 60082.3 | **44.5** | 1335.0 | 2670.0 |
| | 13A | **1030.4** | 30912.8 | 61825.6 | **34.4** | 1030.5 | 2061.0 |
| | 14A | **1185.0** | 35551.3 | 71102.5 | **39.4** | 1182.6 | 2365.1 |
| | 15A | **1105.7** | 33169.8 | 66339.6 | **40.5** | 1214.4 | 2428.8 |
| | 16A | **1146.6** | 34399.4 | 68798.9 | **51.6** | 1549.0 | 3097.9 |
| | 17A | **1369.8** | 41092.5 | 82185.0 | **52.3** | 1569.0 | 3138.0 |
| | 18A | **1046.5** | 31395.3 | 62790.7 | **35.8** | 1075.4 | 2150.7 |
| | 19A | **1090.1** | 32702.8 | 65405.6 | **20.3** | 609.5 | 1218.9 |
| | 20A | **1112.4** | 33372.1 | 66744.3 | **24.4** | 732.9 | 1465.8 |

(continued)

| | | Mean Total Concentration (μg/mL) | | | Std Total Concentration (μg/mL) | | |
|---|---|---|---|---|---|---|---|
| Sample Type | Sample | 1:60 dilution | 1:2 dilution | 1:1 dilution | 1:60 dilution | 1:2 dilution | 1:1 dilution |
| PPP_50 | 1B | 17.1 | **513.0** | **1026.0** | 0.5 | **14.4** | **28.8** |
| | 2B | 21.8 | **653.9** | **1307.9** | 0.9 | **26.7** | **53.4** |
| | 3B | 20.7 | **621.7** | **1243.4** | 0.8 | **22.7** | **45.3** |
| | 4B | 27.1 | **814.2** | **1628.3** | 0.8 | **22.8** | **45.5** |
| | 5B | 22.3 | **668.4** | **1336.8** | 1.5 | **45.8** | **91.6** |
| | 6B | 22.1 | **663.1** | **1326.1** | 0.8 | **23.4** | **46.7** |
| | 7B | 20.0 | **600.6** | **1201.2** | 1.2 | **34.6** | **69.3** |
| | 8B | 20.9 | **626.0** | **1252.0** | 1.0 | **29.0** | **57.9** |
| | 9B | 17.4 | **523.1** | **1046.3** | 0.2 | **4.8** | **9.5** |
| | 10B | 19.2 | **574.7** | **1149.4** | 0.4 | **10.7** | **21.4** |
| | 11B | 22.1 | **663.6** | **1327.2** | 0.5 | **13.8** | **27.7** |
| | 12B | 17.7 | **530.8** | **1061.5** | 1.3 | **39.9** | **79.8** |
| | 13B | 21.9 | **656.2** | **1312.4** | 0.6 | **18.7** | **37.5** |
| | 14B | 25.5 | **766.2** | **1532.4** | 0.5 | **15.5** | **30.9** |
| | 15B | 43.6 | **1306.8** | **2613.7** | 1.0 | **30.6** | **61.3** |
| | 16B | 21.3 | **639.2** | **1278.4** | 0.5 | **14.9** | **29.7** |
| | 17B | 13.8 | **415.5** | **830.9** | 0.7 | **19.6** | **39.3** |
| | 18B | 22.9 | **688.5** | **1376.9** | 0.2 | **6.5** | **13.0** |
| | 19B | 18.5 | **555.1** | **1110.3** | 0.6 | **18.5** | **37.0** |
| | 20B | 15.1 | **454.3** | **908.6** | 0.5 | **14.1** | **28.2** |

(continued)

| | | Mean Total Concentration (μg/mL) | | | Std Total Concentration (μg/mL) | | |
|---|---|---|---|---|---|---|---|
| Sample Type | Sample | 1:60 dilution | 1:2 dilution | 1:1 dilution | 1:60 dilution | 1:2 dilution | 1:1 dilution |
| PRP | 1C | **1012.9** | 30387.6 | 60775.3 | **8.2** | 246.5 | 493.0 |
| | 2C | **1082.4** | 32471.2 | 64942.4 | **41.2** | 1235.9 | 2471.8 |
| | 3C | **1121.9** | 33658.2 | 67316.3 | **6.0** | 179.1 | 358.1 |
| | 4C | **1015.4** | 30463.4 | 60926.8 | **46.2** | 1384.9 | 2769.7 |
| | 5C | **927.5** | 27824.3 | 55648.5 | **36.0** | 1081.1 | 2162.1 |
| | 6C | **976.7** | 29301.7 | 58603.4 | **20.8** | 622.9 | 1245.7 |
| | 7C | **1113.9** | 33418.2 | 66836.5 | **45.5** | 1364.0 | 2727.9 |
| | 8C | **1039.4** | 31183.2 | 62366.4 | **13.9** | 415.6 | 831.1 |
| | 9C | **1183.8** | 35514.4 | 71028.8 | **35.4** | 1061.0 | 2121.9 |
| | 10C | **1071.0** | 32130.2 | 64260.5 | **16.2** | 487.1 | 974.2 |
| | 11C | **1100.5** | 33014.2 | 66028.3 | **8.2** | 246.5 | 493.0 |
| | 12C | **936.7** | 28102.1 | 56204.1 | **5.8** | 173.6 | 347.2 |
| | 13C | **1023.4** | 30703.3 | 61406.7 | **13.9** | 417.3 | 834.6 |
| | 14C | **1173.7** | 35211.3 | 70422.7 | **18.4** | 552.0 | 1104.0 |
| | 15C | **1089.5** | 32685.8 | 65371.7 | **2.2** | 65.6 | 131.2 |
| | 21C | **1003.2** | 30097.2 | 60194.4 | **7.4** | 222.0 | 443.9 |
| | 22C | **1228.4** | 36852.9 | 73705.8 | **17.7** | 530.4 | 1060.7 |
| | 23C | **1072.7** | 32180.7 | 64361.5 | **10.9** | 326.6 | 653.2 |
| | 24C | **1102.6** | 33077.3 | 66154.6 | **13.1** | 394.3 | 788.6 |
| | 25C | **1051.2** | 31536.7 | 63073.5 | **19.0** | 568.7 | 1137.3 |

(continued)

| | | Mean Total Concentration (μg/mL) | | | Std Total Concentration (μg/mL) | | |
|---|---|---|---|---|---|---|---|
| Sample Type | Sample | 1:60 dilution | 1:2 dilution | 1:1 dilution | 1:60 dilution | 1:2 dilution | 1:1 dilution |
| PRP_50 | 1D | 24.1 | **722.2** | **1444.4** | 0.8 | **25.0** | **49.9** |
| | 2D | 24.2 | **725.8** | **1451.6** | 0.8 | **23.2** | **46.4** |
| | 3D | 21.2 | **635.8** | **1271.5** | 1.2 | **34.7** | **69.4** |
| | 4D | 31.4 | **942.5** | **1885.0** | 1.3 | **37.8** | **75.7** |
| | 5D | 23.4 | **702.4** | **1404.9** | 0.3 | **8.9** | **17.8** |
| | 6D | 26.3 | **789.4** | **1578.8** | 1.4 | **41.6** | **83.2** |
| | 7D | 20.1 | **603.4** | **1206.9** | 0.6 | **19.0** | **38.0** |
| | 8D | **489.8** | 14693.9 | 29387.8 | **11.6** | 349.1 | 698.1 |
| | 9D | 20.5 | **614.0** | **1227.9** | 0.3 | **9.4** | **18.8** |
| | 10D | 19.8 | **594.0** | **1187.9** | 0.6 | **19.4** | **38.7** |
| | 11D | 19.8 | **595.0** | **1190.0** | 0.2 | **5.4** | **10.8** |
| | 12D | 18.4 | **551.6** | **1103.3** | 0.8 | **24.6** | **49.3** |
| | 13D | 23.9 | **717.1** | **1434.1** | 0.3 | **8.1** | **16.2** |
| | 14D | 27.2 | **817.1** | **1634.2** | 0.9 | **27.2** | **54.5** |
| | 15D | 22.6 | **676.8** | **1353.6** | 0.7 | **20.0** | **40.1** |
| | 21D | 20.7 | **619.9** | **1239.7** | 0.8 | **23.5** | **47.0** |
| | 22D | 26.8 | **803.5** | **1607.0** | 0.8 | **23.6** | **47.2** |
| | 23D | 21.4 | **642.2** | **1284.3** | 0.5 | **13.9** | **27.8** |
| | 24D | 17.9 | **538.3** | **1076.6** | 0.8 | **22.6** | **45.1** |
| | 25D | 23.5 | **703.7** | **1407.4** | 0.5 | **14.7** | **29.3** |

In the methods of the invention disclosed herein, the reference lines for the PPP, PRP, PPP_50 and PRP_50 proteomes of interest can be calculated experimentally using the methods of the invention. To achieve this, the measured amino acid concentrations of the amino acid types (K, C, W, and Y) which were calculated from the measured values of the K, C, W, and Y labels in this example were plotted against the measured total protein concentrations in μg/mL for each sample which were provided in Example 10, and a linear regression was performed for each sample type and each amino acid type. For all sample types except the PRP_50 sample type, the linear regression was constrained to pass through the origin (0,0). The outputs of this linear regression were used to provide the experimental reference for each proteome and subproteome of interest in the form of vector functions of the total protein concentration in μg/mL.

The results were plotted in Figures 22-28. Figure 22 provides the K component of the experimental reference lines for the PPP and PRP proteomes of interest, Figure 23 provides the C component of the experimental reference lines for the PPP and PRP proteomes of interest, Figure 24 provides the W component of the experimental reference lines for the PPP and PRP proteomes of interest, Figure 25 provides the Y component of the experimental reference lines for the PPP and PRP proteomes of interest. Additionally, Figure 26 provides the K component of the experimental reference lines for the PPP_50 and PRP_50 subproteomes of interest, Figure 27 provides the C component of the experimental reference lines for the PPP_50 and PRP_50 subproteomes of interest, and Figure 28 provides the W component of the experimental reference lines for the PPP_50 and PRP_50 subproteomes of interest. In all cases, the mean of the three technical replicates is shown, with the standard deviation across three technical replicates also provided both in the calculation of the amino acid concentrations and in the calculation of the total protein concentration in μg/mL.

On each graph, the equation of the best-fit-line is included. Across all sample types, all amino acid types are functions of the common parameter of protein concentration, t, here expressed in μg/mL. Each best-fit-line provides one parametric equation (as a function of t) within the set of parametric equations which defines each proteome or subproteome of interest. Equivalently, each best-fit line provides one component of the vector function (as a function of t), which describes each proteome or subproteome of interest.

Additionally, the vector functions can be provided in terms of total molar concentration, rather than total mass

concentration, of the proteome or subproteome of interest. To do this, a transformation between mass protein concentration and molar protein concentration was established for each proteome and subproteome of interest. First, the publicly available Human Protein Atlas was accessed, and a table was downloaded providing the reference plasma concentrations for 419 proteins actively secreted to blood based on published research articles where the target proteins had been measured using immunoassays such as ELISA (https://www.proteinatlas.org/humanproteome/blood/proteins+detected+by+immunoassay). The protein concentrations by mass were provided in varying units (g/L to ng/L) and each provided mass protein concentration was converted to a standard unit of μg/mL. To calculate the molar protein concentration of each protein, the protein concentration in μg/mL was converted to molar protein concentration (in μM) by dividing the μg/mL protein concentration by the protein molecular weight and then multiplying by 1000. These total molar protein concentration values were summed across all proteins in the proteome or subproteome. For the PPP and PRP proteomes of interest, the molar concentrations of all proteins in the dataset were summed. For the PPP_50 and PRP_50 low molecular weight subproteomes of interest, which did not contain proteins with molecular weights less than 50 kDa due to a centrifugal filtration step, proteins with molecular weights greater than 50 kDa were not included in the summed protein concentrations. This was done via a logical flag in Microsoft Excel.

The total plasma proteome concentrations, in μg/mL and μM, are provided below in Table 26.

Table 26

| PPP, PRP | |
|---|---|
| Total concentration μM | 1201.46 |
| Total μg/mL | 77453.24 |
| μM / μg/mL | 0.01551 |

| PPP_50, PRP_50 | |
|---|---|
| Total concentration (μM) | 286.26 |
| Total μg/mL | 9140.67 |
| μM / μg/mL | 0.03132 |

Table 26 additionally provides the conversion factors used to calculate the measured total protein concentrations in μg/mL for the patient samples into molar protein concentrations: 0.01551 μM / (μg/mL) for the PPP and PRP proteomes of interest, and 0.03132 μM / (μg/mL) for the filtered PPP_50 and PRP_50 subproteomes of interest.

When these conversion factors were used, the measured amino acid concentrations for each sample type could be plotted against the total molar protein concentrations of the corresponding proteome of interest, with the results shown in Figures 29-35. Figure 29 provides the K component of the experimental reference lines for the PPP and PRP proteomes of interest, Figure 30 provides the C component of the experimental reference lines for the PPP and PRP proteomes of interest, Figure 31 provides the W component of the experimental reference lines for the PPP and PRP proteomes of interest, Figure 32 provides the Y component of the experimental reference lines for the PPP and PRP proteomes of interest. Additionally, Figure 33 provides the K component of the experimental reference lines for the PPP_50 and PRP_50 subproteomes of interest, Figure 34 provides the C component of the experimental reference lines for the PPP_50 and PRP_50 subproteomes of interest, and Figure 35 provides the W component of the experimental reference lines for the PPP_50 and PRP_50 subproteomes of interest. Whereas in Figures 22-28 the diluted amino acid concentrations and protein concentrations were provided, in Figures 29-35 the undiluted amino acid concentrations and protein concentrations are provided. Because an equal dilution was carried out prior to the labeling reactions an prior to measuring the protein concentration in μg/mL, this factors out of the slope of the experimental reference line provided because both the numerator and denominator are multiplied by the same constant. In all cases, the mean of the three technical replicates is shown, with the standard deviation across three technical replicates also provided for the calculation of the amino acid concentrations.

On each graph, the equation of the best-fit-line is included. Across all sample types, all amino acid types are functions of the common parameter of total protein concentration, *t*, here expressed in μM. Each best-fit-line provides one parametric equation (as a function of *t*) within the set of parametric equations which defines each proteome or subproteome of interest. Equivalently, each best-fit line provides one component of the vector function (as a function of *t*), which describes each proteome or subproteome of interest.

The linear fit to the amino acid concentration / total protein concentration data for each amino acid type and sample type provides a coefficient for that amino acid type of the vector function, calculated experimentally, that describes

each proteome or subproteome of interest. The experimental vector function coefficients for each amino acid type, and each proteome or subproteome of interest, are provided below in Tables 39-42. Table 27 provides the coefficients, for total protein concentration *t* in μg/mL, for the PPP and PRP proteomes of interest ($w_K$, $w_C$, $w_W$, and $w_Y$ values). Table 40 provides the coefficients, for total protein concentration *t* in μg/mL, for the PPP_50 and PRP_50 subproteomes of interest ($w_C$ and $w_W$ values). Table 41 provides the coefficients, for total protein concentration *t* in μM, for the PPP and PRP proteomes of interest ($w_K$, $w_C$, $w_W$, and $w_Y$ values). Table 42 provides the coefficients, for total protein concentration *t* in μM, for the PPP_50 and PRP_50 subproteomes of interest ($w_C$ and $w_W$ values).

Table 27

| | [K (μM)/[total protein (μg/mL)]*t | [C (μM)/[total protein (μg/mL)]*t | [W (μM)/[total protein (μg/mL)]*t | [Y (μM)/[total protein (μg/mL)]*t |
|---|---|---|---|---|
| PPP | 0.7677 | 0.402 | 0.0781 | 0.2755 |
| PRP | 0.9902 | 0.4368 | 0.0909 | 0.3414 |

Table 28

| | [C (μM)/[total protein (μg/mL)]* | [W (μM)/[total protein (μg/mL)]*t |
|---|---|---|
| PPP_50 | 0.1115 | 0.0389 |
| PRP_50 | 0.1244 | 0.0332 |

Table 29

| | [K (μM)/[total protein (μM)]*t | [C (μM)/[total protein (μM)]*t | [W (μM)/[total protein (μM)]*t | [Y (μM)/[total protein (μM)]*t |
|---|---|---|---|---|
| PPP | 49.492 | 25.919 | 5.0343 | 17.758 |
| PRP | 63.835 | 28.16 | 5.8573 | 22.006 |

Table 30

| | [C (μM)/[total protein (μM)]*t | [W (μM)/[total protein (μM)]*t |
|---|---|---|
| PPP_50 | 3.5605 | 1.2412 |
| PRP_50 | 3.9716 | 1.0593 |

As disclosed herein, these values provide the coefficients of vector functions which describe and uniquely identify each proteome and subproteome of interest. The vector functions for the amino acid concentrations of four labeled amino labeled amino acid types (K, C, W, Y) are provided below for the PPP and PRP proteomes of interest, where the total protein concentration of the proteome of interest is provided in molar concentration units.

$$p_{PPP}(t) = \langle 49.492t, 25.919t, 5.0343t, 17.758t \rangle$$

$$p_{PRP}(t) = \langle 63.835t, 28.16t, 5.8573t, 22.006t \rangle$$

As another example, the vector functions for the amino acid concentrations of two labeled amino labeled amino acid types (C, W) are provided below for the PPP_50 and PRP_50 subproteomes of interest, where the total protein concentration of the subproteome of interest is provided in molar concentration units.

$$p_{PPP_50}(t) = \langle 3.5605t, 1.2412t \rangle$$

$$p_{PRP_50}(t) = \langle 3.9716t, 1.0593t \rangle$$

These results indicated that the average number of K, C, W, and Y amino acids per protein sequence within the PRP proteome of interest were consistently slightly higher than for the PPP proteome of interest. Additionally, as expected

the average number of amino acids per protein sequence for the low-molecular weight PPP_50 and PRP_50 subproteomes of interest were significantly lower than the average number of amino acids per protein sequence for the PPP and PRP proteomes of interest.

The methods of the invention also provide that reference lines can be calculated theoretically. For the PPP proteome of interest, publicly available data was available enabling calculation of the theoretical reference line for the PPP proteome of interest, and comparison of the theoretical reference line to the experimental reference line (using the data measured for the PPP proteome of interest). The theoretical reference line was calculated for the PPP proteome of interest using the methods of the invention disclosed herein on publicly available data.

Publicly available Human Protein Atlas data was accessed and processed as described above. Additionally, the provided human gene names were mapped to UniProtKB identifiers using the UniProt database, and the amino acid sequence (using single letter abbreviation) and molecular weight of each mapped UniProtKB identifier was downloaded. 415 gene names were successfully mapped to proteins and these proteins were taken forward for further calculations. To count the number of times that the C, K, W, and Y amino acid types occurred within the protein amino acid sequence, the number of occurrences of the indicated character "C", "K", "W", or "Y" within the amino acid sequence string was counted using Microsoft Excel. This can alternatively be calculated using the Python ProtParam count_amino_acids function which simply counts the number of times an amino acid is repeated in the protein sequence. To calculate the molar protein concentration of each protein, the protein concentration in μg/mL was converted to molar protein concentration (in μM) by dividing the μg/mL protein concentration by the protein molecular weight and then multiplying by 1000. Then, the total concentration of each labelled amino acid type (C, K, W, and Y) in μM was calculated by multiplying the number of C, K, W, or Y amino acids within the amino acid sequence, respectively, by the molar protein concentration in μM. This provided the C, K, W, and Y amino acid concentration in μM of each individual protein within platelet poor plasma (PPP), and to access the total amino acid concentration of the C, K, W, or Y amino acid type in μM of all proteins within platelet poor plasma, the individual amino acid concentrations of the C, K, W, or Y amino acid type were summed. Similarly, to access the total protein concentration in μg/mL for all proteins within platelet poor plasma, the individual protein concentrations in μg/mL were summed. This resulted in a total concentration of the C amino acid type in μM ([C (μM)]), total concentration of the K amino acid type in μM ([K (μM)]), total concentration of the W amino acid type in μM ([W (μM)]), total concentration of the Y amino acid type in μM ([Y (μM)]), and a single value for total protein concentration in μg/mL ([total protein (μg/mL)]). Additionally, to access the total protein concentration in μM for all proteins within platelet poor plasma, the individual protein concentrations in μM were summed, providing a single value for total protein concentration in μM.

Each value, $\frac{[C\ (uM)]}{[total\ protein\ (uM)]}$, $\frac{[K\ (uM)]}{[total\ protein\ (uM)]}$, $\frac{[W\ (uM)]}{[total\ protein\ (uM)]}$, and $\frac{[Y\ (uM)]}{[total\ protein\ (uM)]}$ provided the theoretical coefficient of the C, K, W, and Y component of the vector function defining the PPP proteome. These theoretical coefficients were compared to the experimental coefficients $\frac{[C\ (uM)]}{[total\ protein\ (uM)]}$, $\frac{[K\ (uM)]}{[total\ protein\ (uM)]}$, $\frac{[W\ (uM)]}{[total\ protein\ (uM)]}$, and $\frac{[Y\ (uM)]}{[total\ protein\ (uM)]}$ which had been calculated in Example 11 using linear regression. The results are shown in Table 31.

Table 31: Comparison of theoretical and experimental vector function coefficients for the PPP proteome of interest (a plasma proteome of interest)

| | | Theoretical | Experimental | % Difference | Average |
|---|---|---|---|---|---|
| $w_K$ | [K (μM)]/[total protein (μM)] | 50.088 | 49.492 | -1% | 0% |
| $w_C$ | [C (μM)]/[total protein (μM)] | 28.130 | 28.160 | 0% | |
| $w_W$ | [W (μM)]/[total protein (μM)] | 4.504 | 5.034 | 11% | |
| $w_Y$ | [Y (μM)]/[total protein (μM)] | 19.169 | 17.758 | -8% | |

Additionally, each value, $\frac{[C\ (uM)]}{[total\ protein\ \left(\frac{ug}{mL}\right)]}$, $\frac{[K\ (uM)]}{[total\ protein\ \left(\frac{ug}{mL}\right)]}$, $\frac{[W\ (uM)]}{[total\ protein\ \left(\frac{ug}{mL}\right)]}$, and $\frac{[Y\ (uM)]}{[total\ protein\ \left(\frac{ug}{mL}\right)]}$ provided the theoretical coefficient of the C, K, W, and Y component of the vector function defining the PPP proteome.

These theoretical coefficients were compared to the experimental coefficients $\frac{[C\ (uM)]}{[total\ protein\ (\frac{ug}{mL})]}$, $\frac{[K\ (uM)]}{[total\ protein\ (\frac{ug}{mL})]}$, $\frac{[W\ (uM)]}{[total\ protein\ (\frac{ug}{mL})]}$, and $\frac{[Y\ (uM)]}{[total\ protein\ (\frac{ug}{mL})]}$ which had been calculated in Example 11 using linear regression. The results are shown in Table 32.

Table 32

| | | Theoretical | Experimental | % Difference | Average |
|---|---|---|---|---|---|
| $w_K$ | [K (μM)/[total protein (μg/mL)] | 0.777 | 0.768 | -1% | -2% |
| $w_C$ | [C (μM)/[total protein (μg/mL)] | 0.436 | 0.402 | -9% | |
| $w_W$ | [W (μM)/[total protein (μg/mL)] | 0.070 | 0.078 | 11% | |
| $w_Y$ | [Y (μM)/[total protein (μg/mL)] | 0.297 | 0.276 | -8% | |

In all cases, there was close agreement between the theoretical vector function coefficients and the experimental vector function coefficients. The percentage difference between the theoretical vector function coefficients and the experimental vector function coefficients was calculated. The theoretical vector function coefficients were calculated using the methods of the invention to determine, from publicly available data, the vector function that would describe the reference that would be measured for the PPP proteome of interest. This was calculated using equation 11, which as disclosed herein, is used to calculate the coefficients of the vector functions that would describe the proteome or subproteome of interest via an average signature. To do this, equation 11 transforms publicly available data which was collected when each protein within a proteome or subproteome of interest was measured individually (which can include being individually separated or targeted) into an average identifying proteomic or subproteomic signature that can be measured without separation and without targeting individual proteins within a proteome or subproteome of interest or the need to develop tests to find and measure specifically certain biomarkers within a proteome or subproteome of interest. All experimentally measured vector function coefficients are within 11% of their theoretically predicted values, and the average percentage difference across all included vector function coefficients is 0% for the molar coefficients and 2% for the mass coefficients.

This close agreement between experimental and theoretical reference lines confirms that measuring an average proteomic or subproteomic signature (via the values of the label, the total amino acid concentrations calculated from the measured label, or the number of amino acids calculated from the total amino acid concentrations) provides equivalent information to individually measuring each protein within the proteome or subproteome - such as via immunoassay or mass spectrometry - and aggregating the results of these hundreds, if not thousands, of experiments in which proteins were laboriously identified and quantified. As disclosed herein, it is not necessary to separate a proteome or subproteome, or to separately detect individual proteins, peptides, oligopeptides, and polypeptides within a proteome or subproteome, to identify and quantify the proteome or subproteome. Furthermore, even when the methods of the invention are carried out in complex human biofluids such as plasma, which contains cell debris, small molecules, ions, autofluorescent species, and high degrees of molecular crowding, there was no significant deviation from the predicted results.

The close agreement between the predicted vector function and experimental data is plotted in Figure 36 which shows the mean measured amino acid concentrations across the three technical replicates of each patient sample (stars) and the predicted reference line (solid line) whose coefficients were provided in Table 31.

Identification and quantification using the vector function approach disclosed herein is provided in a subsequent Example.

*Identification using a classifier (optional step)*

As disclosed herein, the comparison step can alternatively, or additionally involve a machine learning classifier. The complete data set of three technical replicates of all 25 patient PPP and PRP samples (excluding sample preparation outliers) was randomly partitioned into two sets. The first set included 25% of the data points as the sample points and provided the amino acid concentration of two or more amino acid types (four amino acid types) that had been calculated from the measured label of the same two or more amino acid types (four amino acid types). The second set included 75% of the data points and provided the known amino acid concentrations of the same two or more amino acid types that had been labelled in the sample of the PPP and PRP proteomes of interest. The second set (the known amino acid concentrations of the same two or more amino acid types that had been labelled in the sample in the PPP and PRP proteomes of interest) was used to train a machine learning classifier, specifically a decision tree model (fine K-nearest neighbor). A Euclidean

distance metric was used with equal distance weighting and data standardization and a default cost matrix. No classifier optimization or hyperparameter tuning was required for accurate identification.

The dataset used, including both the training set and the testing set, is shown in Figure 37 and the predictions for the testing set are shown in Figure 38. 100% of predictions were correct.

This resulted in a 100% true positive rate and a 0% false negative rate for identification of a proteome of interest within a patient sample type using the methods of the invention disclosed and 2 labeled amino acid types, as shown in Figure 39.

The results were robust to the type of classifier used. An ensemble bagged decision tree classifier was also trained, using 119 splits of 30 learners and a default cost matrix, and provided equivalent results. As shown in Figure 40, 100% true positive and 0% false negative rates were achieved for the identification of a proteome of interest within a patient sample type using the methods of the invention disclosed and 2 labeled amino acid types. As shown in Figure 41, 100% positive predictive value and 0% false discovery rate was also achieved.

Excellent results were achieved for the identification of one or more proteomes of interest in patient samples.

*Confirmation of generality of patient proteomic and subproteomic signatures*

The invention can provide a diagnostic screening test, and therefore when collecting the clinical data, a patient population representative of the intended use environment for a diagnostic screening test at the primary care level was chosen. As was summarized in Table 13, the patient population included males and females varying by over 50 years in age. To test whether these variations in patient population had any effect on the measured experimental reference

lines, a detailed analysis was carried out. In Figures 42-48, the coefficient of the experimental reference line for every individual male and female patient was plotted as a function of patient age. For all proteomic and subproteomic sample types measured and all amino acid types labelled, in all cases there was no impact of patient gender or age on the coefficient of the experimental proteomic reference lines calculated for each patient. This confirms that the proteomic and subproteomic signatures measured using the methods of the present invention describe any patient population and are specifically not affected by gender or age. This result confirms that the methods of the invention are robust to individual patient variations and that healthy patients exhibt a single identifying proteomic and subproteomic signature.

In the following worked examples, the proteomic signature for healthy patients is compared to proteomic signatures for patients with various types of cancer.

Example 13: Establishing the mechanism by which experimentally measured mass spectrometry data is converted into theoretical reference lines

In Worked Examples 10-12, it was demonstrated that the experimental values which were measured for the PPP proteome of interest matched the values predicted by the PPP proteome of interest theoretical reference line. The theoretical reference line was defined by and calculated using the vector functions disclosed herein, operating on a dataset of publicly available protein quantification values for individual proteins measured within plasma. The PPP plasma proteomic samples were experimentally measured from healthy patients, and so provide the healthy patient plasma (PPP) proteomic signature.

Having established the reference line for the healthy patient plasma (PPP) proteome, in the following worked examples, theoretical reference lines are defined by and calculated using the vector functions disclosed herein, operating on a dataset of publicly available protein quantification values for individual proteins measured within the plasma of cancer patients. These proteins were measured from plasma that had been depleted of platelets, equivalent to the protein poor plasma (PPP) in the experimental data already presented.

Therefore, the PPP data which was experimentally measured is then compared to the theoretical reference lines calculated for patients with varying types and stages of cancer. The machine learning and the minimum perpendicular distance approaches disclosed herein are then used to identify patient samples as corresponding to healthy patients (the healthy patient samples were experimentally measured in Worked Examples 10-12), or to patients with varying types and stages of cancer. In this way, it is established that the proteomic signature, subproteomic signature, reference line, vector function, and identification approaches disclosed herein can be used to identify samples as corresponding to healthy patients or to patients with an indicated cancer type and stage.

The publicly available protein quantification values for individual proteins measured within the plasma of cancer patients were provided by the Proteome Xchange database.

First, an ovarian cancer dataset was examined because this additionally provided healthy controls which allowed validation of the approach disclosed herein for operating the vector functions on quantitative mass spectrometry data.

Using ProteomeXchange, the PXD020557 original dataset was downloaded at http://proteomecentral.proteo mexchange.org/cgi/GetDataset?ID=PXD020557. The goal of this study from which the PXD020557 original dataset is derived, had been to find individual metabolic and protein biomarkers for high-grade serous ovarian cancer within plasma

by examining plasma samples from 10 patients with stage III or IV serous ovarian cancer (ovarian cancer patients) and 10 patients without ovarian cancer (healthy control patients) using mass spectrometry. Liquid chromatography with tandem mass spectrometry (LC-MS/MS) was used for the identification of proteins within the plasma samples. In the LC-MS/MS approach, a complex sample is separated into individual protein components, and these individual protein components are sequentially ionized and directed into two mass analysers. The first mass analyser separates the ions for each protein component by their mass-to-charge ratio (m/z) and then ions of a particular m/z ratio are selected, split into smaller fragment ions, separated by m/z ratio of the fragment ions in the second mass analyser, and then detected. Prior to the analysis of patient plasma samples, an isolation step had been performed to remove 14 high abundance proteins in plasma, specifically using a MARS14 affinity column. This isolation step depleted albumin, a1-antitrypsin, a1-acid glycoprotein, apolipoprotein A1, apolipoprotein A2, a2-marcoglobulin, complement C3, fibrinogen, haptoglobin, IgA, IgG, IgM, transferrin, and transthyretin within the plasma samples and is common in the art of mass spectrometery analysis of patient proteomic samples due to limited instrument dynamic range. The MS/MS step was performed using a Q Exactive Plus Hybrid Quadrupole-Orbitrap mass spectrometer with a nano-ESI source. Label-free quantification (LFQ) was used to calculate the abundance within each plasma sample of each protein that had been identified as being present within the plasma sample and was performed using peak intensity for unique and razor peptides of each protein.

Within the files deposited in ProteomeXChange for PXD020557, accession numbers were provided for each protein detected within each plasma sample. There were 1385 accession numbers and 1382 of these were provided as UniProt IDs. The intensity (abundance) of each protein detected within each plasma sample was also provided as an intensity which had been calculated using label free quantification (LFQ). First, the 1382 acession numbers provided as UniProt IDs were mapped to 1359 UniProt KB IDs for Swiss-Protein reviewed Human Proteins. Each UniProt KB ID describes a protein. These 1359 UniProt KB IDs were downloaded together with the sequence and molecular weight of the protein described by each Uniprot KB ID.

Because the theoretical reference line data was going to be compared to the experimental data reported in Examples 10-12 in which the C, K, W, and Y amino acid types were labelled and measured, the $w_1$, $w_2$, and $w_n$ values were calculated for the C, K, W, and Y amino acid types using equation 11 disclosed herein. Equation 11 is reproduced here for convenience:

$$w_n = \sum_{i=1}^{c}\left(a_{n,i} \times \frac{q_i}{q}\right)$$

wherein $w_n$ is the weighted mean number of amino acids of amino acid type *n* in the proteome of interest, *c* is the number of proteins in the proteome of interest, $a_{n,i}$ is the number of amino acids of amino acid type *n* in protein *i* in the proteome of interest, $q_i$ is a measure of the quantity of protein *i* in the proteome or subproteome of interest, and *q* is an equivalent measure of the total quantity of all proteins (proteins *i* through *c*) in the proteome of interest, such that $^{q_i}/_q$ gives the proportion of protein *i* within the proteome of interest. A linear combination of the results is taken for proteins *i* through *c* of the proteome of interest.

$q_i$ can be the expression level of protein of interest *i* within the proteome or subproteome of interest, preferably determined by publicly available data from mass spectrometry databases, and *q* can be the total concentration of the proteome or subproteome of interest or the total predicted expression level of all proteins (proteins *i* through *c*) contained within the proteome or subproteome of interest each assessed using publicly available protein expression data.

Label free quantification data in mass spectrometry databases is provided as intensitiy vales, *int*, which are proportional to the amount (mass) of protein present. These provided *int* values can be converted to be proportional to the molar amount of protein present by dividing the intensity for an individual protein by its molecular mass.

$$q_i = int_m = \frac{int}{m_r}$$

$int_m$ is the molar intensity of an individual protein, *int* is the provided intensity of the individual protein, and $m_r$ is the molecular mass of the individual protein. As required by equation 11, $int_m$ is calculated for each protein of interest within the reference proteome or subproteome of interest.

Within equation 11, *q* is the sum of the $q_i$ values across all proteins of interest *i* within the proteome or subproteome of interest containing *c* proteins, meaning

$$q = \sum_{i=1}^{c} q_i$$

It follows that just as $int_m$ is one possible form of $q_i$ within equation 11, $\sum int_m$ is one possible form of $q$ for the proteome of subproteome of interest,

$$q = \sum int_m$$

Therefore,

$$\frac{int_m}{\sum int_m}$$

is one possible form of $^{q_i}/_q$ in equation 11 as disclosed herein.

For ease of reference, $\frac{int_m}{\sum int_m}$ is called mass spectrometry molar intensity fraction, which is abbreviated $MSIF_m$.

$$MSIF_m = \frac{int_m}{\sum int_m}$$

Using $MSIF_m$ values for $^{q_i}/_q$ in equation 11, $w_K$,$w_C$,$w_W$, and $w_Y$ values were calculated for the PXD020557 dataset, providing the results below in Table 33.

Table 33

| Patient Sample | Proteome | $w_K$ | $w_W$ | $w_Y$ | $w_C$ |
|---|---|---|---|---|---|
| F21 | Ovarian cancer | 32.58 | 8.83 | 19.04 | 13.97 |
| F22 | Ovarian cancer | 35.26 | 9.99 | 21.64 | 16.28 |
| F23 | Ovarian cancer | 36.27 | 10.03 | 21.98 | 15.97 |
| F24 | Ovarian cancer | 32.41 | 9.05 | 20.00 | 14.43 |
| F25 | Ovarian cancer | 34.70 | 9.39 | 20.91 | 15.56 |
| F26 | Ovarian cancer | 31.19 | 8.18 | 17.86 | 11.99 |
| F27 | Ovarian cancer | 26.80 | 7.79 | 16.23 | 13.48 |
| F28 | Ovarian cancer | 34.44 | 9.18 | 20.36 | 14.35 |
| F29 | Ovarian cancer | 33.72 | 9.40 | 20.44 | 16.62 |
| F30 | Ovarian cancer | 35.73 | 10.07 | 21.29 | 17.52 |
| F31 | Healthy | 29.03 | 7.97 | 16.34 | 13.36 |
| F32 | Healthy | 29.84 | 8.28 | 16.93 | 12.64 |
| F33 | Healthy | 30.77 | 8.10 | 17.33 | 13.76 |
| F34 | Healthy | 22.14 | 5.22 | 10.62 | 5.43 |
| F35 | Healthy | 31.98 | 7.80 | 16.90 | 13.80 |
| F36 | Healthy | 26.33 | 6.54 | 13.76 | 7.38 |
| F37 | Healthy | 33.28 | 8.82 | 19.10 | 14.88 |
| F38 | Healthy | 29.59 | 8.01 | 16.83 | 14.24 |
| F39 | Healthy | 32.23 | 8.26 | 18.68 | 13.92 |
| F40 | Healthy | 30.92 | 8.19 | 17.70 | 14.27 |

To further validate using the $MSIF_m$ as $^{q_i}/_q$ within equation 11, the results for healthy patient samples calculated in this way were compared to the results calculated using equation 11 in which $^{q_i}/_q$ values were calculated using protein concentrations measured using immunoassay deposited in the Human Peptide Atlas. The approach already described in Example 12 was applied again, but the proteins which had been depleted in the mass spectrometry experiments via a MARS column (to remove 14 high abundance proteins from plasma so that the high abundance species would not negatively impact the mass spectrometry results) were also removed from the Human Peptide Atlas dataset. The Human Protein Atlas dataset was processed as already described in Example 12, including counting the number of times that the C, K, W, and Y amino acid types occurred within the protein amino acid sequence, the number of occurrences of the indicated character "C", "K", "W", or "Y" within the amino acid sequence string was counted using Microsoft Excel. This can alternatively be calculated using the Python ProtParam count_amino_acids function which simply counts the number of times an amino acid is repeated in the protein sequence. Because albumin, a1-antitrypsin, a1-acid glycoprotein, apolipoprotein A1, apolipoprotein A2, a2-marcoglobulin, complement C3, fibrinogen, haptoglobin, IgA, IgG, IgM, transferrin, and transthyretin had been removed from the plasma samples before the mass spectrometry experiments, these were also removed from the Human Peptide Atlas dataset prior to further calculations. The protein concentrations by mass had been provided in varying units (g/L to ng/L) in the Human Peptide Atlas database, and each provided mass protein concentration was converted to a standard unit of g/L, and then to molar protein concentration by dividing the g/L concentration by the protein molecular weight. Then, $^{q_i}/_q$ was calculated for each protein within the plasma dataset by dividing its molar concentration by the sum of the molar concentrations of all proteins within the plasma dataset. As defined by equation 11, $^{q_i}/_q$ was then multiplied by $a_{n,i}$ to provide the $w_n$ values.

The $w_n$ values which had been calculated for the healthy patient mass spectrometry dataset which had been depleted of the MARS 14 column proteins were then compared to the $w_n$ values which were calculated for the healthy patient Human Peptide Atlas immunoassay dataset. The results are provided in Table 34. In all cases, the vector function coefficients calculated for the mass spectrometry data match the vector function coefficients calculated for the immunoassay data, which validates the described approach of using $MSIF_m$ as $^{q_i}/_q$ within equation 11. The agreement of the immunoassay and mass spectrometry results is also shown graphically in Figure 49.

Equation 11 robustly performs on abundance data generated from both mass spectrometry and immunoassay, providing a means to build a congruent/unified set of references (vector functions), even though different experimental techniques were employed to generate the underlying data. The provides a framework to build upon existing sources of data.

Table 34

| $w_n$ values for the healthy patient mass spectrometry dataset compared to the $w_n$ values for the healthy patient Human Peptide Atlas immunoassay dataset. | $w_K$ | $w_W$ | $w_Y$ | $w_C$ |
|---|---|---|---|---|
| Mass spectrometry halthy paient mean | 29.61 | 7.72 | 16.42 | 12.37 |
| Mass spectrometry healthy patient STD | 3.27 | 1.05 | 2.50 | 3.23 |
| Immunoassay | 28.62 | 7.35 | 16.64 | 13.49 |

The mass spectrometry and immunoassay data on healthy patient plasma samples (which had been depleted of the MARS14 column proteins) can be defined by the following vector functions.

$$healthy\ plasma_{mass\ spectrometry} = \langle 29.61t, 7.72t, 16.42t, 12.37t \rangle$$

$$healthy\ plasma_{immunoassay} = \langle 28.62t, 7.35t, 16.64t, 13.49t \rangle$$

Prior to the methods of this invention disclosed herein, measuring a proteome or subproteome of interest required individually measuring each protein within the proteome or subproteome of interest. This could be labor, cost, and time intensive because it required either separating a proteome or subproteome of interest into many fractions before analysis could be suitable (such as is required for mass spectrometry experiments), or using binding agents such as antibodies in immunoassays targeted to each individual protein within the proteome or subproteome of interest. If a proteome or subproteome of interest contains 400 proteins, with methods known the art prior to the invention disclosed

herein, measuring the proteome or subproteome of interest would require making 400 measurements of each individual proteins contained within the proteome or subproteome of interest (this was the case in the analyzed mass spectrometry and immunoassay data).

In contrast, in the methods of the invention disclosed herein, it is possible to identify a proteome or subproteome of interest via making only one measurement of two or more labelled amino acid types within the proteome or subproteome of interest. The approach disclosed herein is complementary to the current state of the art methods and provides a way in which disease states can be identified with simple, mean fluorescence intensity measurements without having to individually measure proteins within body fluids or seek to identify biomarkers of disease. Because an average signature is measured which by definition covers all biomarkers, no prior knowledge of relevant biomarkers is required.

Further, amino acid concentrations of two or more amino acid types can be calculated from mass spectrometry databases by recognizing that mass spectrometry molar intensity fraction, $MSIF_m$, is a relative quantity, giving the proportion of molar concentration each protein contributes to the proteome or subproteome of interest. For example, if a protein contributes 1% of the molar concentration of the total molar protein concentration of the proteome of interest, then its $MSIF_m$ value would be the unitless value of 0.01. However, $\sum int_m$ is not a relative quantity and it can be related to the molar protein concentration of the proteome of interest. To achieve this, $\sum int_m$ values accessible via mass spectrometry are related to molar or mass concentration values accessible via immunoassay or peptide microarray experiments on the same proteome of interest.

Furthermore, having established that the using the $MSIF_m$ values $(\frac{int_m}{\sum int_m})$ and the immunoassay molar concentration values $\frac{molar\ protein\ concentration}{\sum molar\ protein\ concentration}$ give equivalent results when used as $q_i/q$ in equation 11, it follows that $\frac{int_m}{\sum int_m} = \frac{a}{a} \times \frac{molar\ protein\ concentration}{\sum molar\ protein\ concentration}$ and that $\sum int_m = a \sum$ molar *protein concentration*

Therefore, total molar intensity ($\sum int_m$) calculated for an individual patient in a mass spectrometry database can be transformed into total molar protein concentration by simply dividing by *a*. Preferably, the value of *a* is calculated for the particular mass spectrometry dataset being transformed, and the value of $\sum int_m$ considered is a mean value ($mean(\sum int_m)$ across the entire healthy patient (or cancer negative) portion of the dataset. Preferably, the value of *a* is calculated with reference to the subset of the immunoassay concentration data (Human Peptide Atlas concentration data) present in the mass spectrometry experiment. For example, if a MARS column was used to deplete highly abundant proteins within the mass spectrometry dataset, then those proteins should be removed from the immunoassay concentration data (Human Peptide Atlas concentration data) before calculating the total molar protein concentration.

It was already established in Example 12 that vector function coefficients calculated from the experimental data which was measured for healthy patient plasma samples (PPP samples) matched the vector function coefficients which were calculated from immunoassay measurements of proteins within healthy patient platelet poor plasma samples in the Human Peptide Atlas.

It was established in this example that the mass spectrometry vector functions from healthy patients matched the vector functions calculated using the immunoassay mass spectrometry data. Having established that the vector functions calculated from the experimental data which was measured from healthy patient plasma samples, the vector functions calculated from the immunoassay data from healthy patient plasma samples, and the vector functions calculated from the mass spectrometry data from healthy patient plasma samples can all be quantitatively compared, in the next worked examples, the experimental data which was measured in Examples 10-12 for healthy patient plasma samples is compared to the mass spectrometry data calculated from plasma samples from patients with varying types and stages of cancer.

Example 14: Identification of patient samples as corresponding to the healthy, ovarian cancer, or pancreatic cancer plasma proteomes

Dataset PXD020557 providing protein quantification data for individual proteins measured within the platelet poor plasma of ovarian cancer patients, and dataset PXD005144 providing protein quantification data for individual proteins measured within the platelet poor plasma of pancreatic cancer patients, were each processed as explained in Example 13. For example, the total molar concentration was calculated by recognizing that $\sum int_m = a \sum$ *molar protein concentration*, so total molar protein concentration is equal to total molar intensity divided by *a*. *a* was calculated by taking the mean total molar abundance value for healthy patients, and dividing this by the total molar protein concentration in the Human Peptide Atlas immunoassay dataset excluding the proteins that had been depleted in the mass spectrometry experiment using a MARS14 column. For example, for the ovarian cancer dataset, *a* was calculated to be 4.41 x $10^5$ AU / $\mu$M, and so each total molar intensity data value was divided by *a* to provide the total molar concentration value. Note that the intensity value provided here in AU is a mass spectrometry total molar intensity value as disclosed herein, not a fluorescence intensity value.

Table 35

| Patient Sample | Subproteome | $w_K$ | $w_W$ | $w_Y$ | $w_C$ | Total Molar Intensity | Total molar concentration |
|---|---|---|---|---|---|---|---|
| F21 | Ovarian cancer | 32.58 | 8.83 | 19.04 | 13.97 | 8.42E+07 | 191.20 |
| F22 | Ovarian cancer | 35.26 | 9.99 | 21.64 | 16.28 | 5.06E+07 | 114.92 |
| F23 | Ovarian cancer | 36.27 | 10.03 | 21.98 | 15.97 | 5.96E+07 | 135.28 |
| F24 | Ovarian cancer | 32.41 | 9.05 | 20.00 | 14.43 | 5.18E+07 | 117.48 |
| F25 | Ovarian cancer | 34.70 | 9.39 | 20.91 | 15.56 | 5.63E+07 | 127.80 |
| F26 | Ovarian cancer | 31.19 | 8.18 | 17.86 | 11.99 | 6.20E+07 | 140.68 |
| F27 | Ovarian cancer | 26.80 | 7.79 | 16.23 | 13.48 | 5.00E+07 | 113.47 |
| F28 | Ovarian cancer | 34.44 | 9.18 | 20.36 | 14.35 | 6.83E+07 | 155.01 |
| F29 | Ovarian cancer | 33.72 | 9.40 | 20.44 | 16.62 | 4.63E+07 | 105.04 |
| F30 | Ovarian cancer | 35.73 | 10.07 | 21.29 | 17.52 | 5.36E+07 | 121.58 |

Then, as specified by vector function 2, the total molar concentration value was multiplied by each of the vector function coefficients $w_K$,$w_W$,$w_Y$, and $w_C$ to provide the total amino acid concentration of the K, W, Y, and C amino acid types that would be measured at the indicated total molar protein concentration.

The same approach used to analyse the ovarian cancer dataset was replicated to analyse a pancreatic cancer dataset (dataset PXD005144), also available from Proteome Xchange at http://proteomecentral.proteomexchange.org/cgi/GetDataset?ID=PXD005144 .

An illustrative subset of the replicated steps is provided below

Table 36

| Patient Sample | Subproteome | $w_K$ | $w_W$ | $w_Y$ | $w_C$ | Total Molar Itensity | Total molar concentration |
|---|---|---|---|---|---|---|---|
| Cancer_01a | Pancreatic Cancer | 29.98 | 8.49 | 16.25 | 12.33 | 482.74 | 264.00 |
| Cancer_01b | Pancreatic Cancer | 30.62 | 8.76 | 16.67 | 12.60 | 529.21 | 289.42 |
| Cancer_01c | Pancreatic Cancer | 30.42 | 8.80 | 16.52 | 12.70 | 660.91 | 361.44 |
| Cancer_02a | Pancreatic Cancer | 33.24 | 9.21 | 17.59 | 13.62 | 315.62 | 172.61 |
| Cancer_02b | Pancreatic Cancer | 31.91 | 9.12 | 17.10 | 13.27 | 401.04 | 219.32 |
| Cancer_02c | Pancreatic Cancer | 32.53 | 9.08 | 17.33 | 13.52 | 320.52 | 175.29 |
| Cancer_03a | Pancreatic Cancer | 32.16 | 8.46 | 16.56 | 12.39 | 266.28 | 145.62 |
| Cancer_03b | Pancreatic Cancer | 32.01 | 8.44 | 16.49 | 12.31 | 267.14 | 146.09 |
| Cancer_03c | Pancreatic Cancer | 31.95 | 8.56 | 16.50 | 12.52 | 244.59 | 133.76 |
| Cancer_04a | Pancreatic Cancer | 32.86 | 8.13 | 16.79 | 12.88 | 370.96 | 202.87 |
| Cancer_04b | Pancreatic Cancer | 32.60 | 8.17 | 16.74 | 12.93 | 370.17 | 202.44 |
| Cancer_04c | Pancreatic Cancer | 32.52 | 8.13 | 16.65 | 13.01 | 460.82 | 252.01 |
| Cancer_05a | Pancreatic Cancer | 33.32 | 8.09 | 17.12 | 12.97 | 287.51 | 157.23 |
| Normal_11b | Chronic Pancreatitis | 29.6 | 7.9 | 15.3 | 11.6 | 290.8 | 159.0 |
| Normal_11c | Chronic Pancreatitis | 29.4 | 7.8 | 15.1 | 11.5 | 282.9 | 154.7 |
| Normal_12a | Chronic Pancreatitis | 31.5 | 7.9 | 16.3 | 13.1 | 178.2 | 97.5 |
| Normal_12b | Chronic Pancreatitis | 31.8 | 7.8 | 16.4 | 13.1 | 237.2 | 129.7 |
| Normal_12c | Chronic Pancreatitis | 31.6 | 7.9 | 16.2 | 13.0 | 246.0 | 134.5 |
| Normal_13a | Chronic Pancreatitis | 32.0 | 7.8 | 16.6 | 12.6 | 291.1 | 159.2 |

(continued)

| Patient Sample | Subproteome | $w_K$ | $w_W$ | $w_Y$ | $w_C$ | Total Molar Itensity | Total molar concentration |
|---|---|---|---|---|---|---|---|
| Normal_13b | Chronic Pancreatitis | 32.1 | 7.9 | 16.5 | 12.6 | 291.9 | 159.6 |
| Normal_13c | Chronic Pancreatitis | 32.1 | 7.9 | 16.6 | 12.7 | 327.1 | 178.9 |
| Normal_14a | Chronic Pancreatitis | 33.2 | 8.1 | 17.1 | 13.0 | 252.7 | 138.2 |
| Normal_14b | Chronic Pancreatitis | 33.0 | 8.0 | 16.9 | 12.8 | 272.4 | 149.0 |
| Normal_14c | Chronic Pancreatitis | 33.2 | 8.0 | 16.9 | 13.1 | 246.7 | 134.9 |

For this dataset, the average total molar intensity for patients without cancer (Chronic Pancreatitis) was 281.303 resulting in an *a* value of 0.547 which was then multiplied by total molar intensity to calculate total molar concentration in μM.

The results for both sets of amino acid concentrations of the K, C, W, and Y amino acid types are shown below in Table 37

Table 37

| Cancer Subproteome | Total molar concentration (μM) | [K] | [C] | [W] | [Y] |
|---|---|---|---|---|---|
| Ovarian | 191.2 | 6229.9 | 2671.5 | 1688.2 | 3640.2 |
| Ovarian | 114.9 | 4052.5 | 1870.3 | 1148.4 | 2486.5 |
| Ovarian | 135.3 | 4906.8 | 2160.7 | 1357.3 | 2973.3 |
| Ovarian | 117.5 | 3807.4 | 1695.1 | 1063.7 | 2349.1 |
| Ovarian | 127.8 | 4434.9 | 1988.6 | 1200.4 | 2671.9 |
| Ovarian | 140.7 | 4387.8 | 1686.8 | 1151.4 | 2513.1 |
| Ovarian | 113.5 | 3041.4 | 1529.6 | 883.5 | 1841.8 |
| Ovarian | 155.0 | 5338.5 | 2224.0 | 1422.3 | 3155.3 |
| Ovarian | 105.0 | 3541.8 | 1746.3 | 987.2 | 2147.0 |
| Ovarian | 121.6 | 4343.7 | 2130.5 | 1224.1 | 2588.9 |
| Pancreatic | 264.0 | 7914.3 | 3254.6 | 2240.5 | 4289.8 |
| Pancreatic | 289.4 | 8861.9 | 3646.9 | 2536.0 | 4824.8 |
| Pancreatic | 361.4 | 10994.9 | 4589.9 | 3180.6 | 5972.3 |
| Pancreatic | 172.6 | 5736.6 | 2350.2 | 1589.6 | 3036.8 |
| Pancreatic | 219.3 | 6998.7 | 2911.0 | 2000.8 | 3750.4 |
| Pancreatic | 175.3 | 5701.9 | 2369.3 | 1591.1 | 3037.8 |
| Pancreatic | 145.6 | 4683.8 | 1804.0 | 1231.9 | 2411.9 |
| Pancreatic | 146.1 | 4676.1 | 1798.5 | 1233.6 | 2408.9 |
| Pancreatic | 133.8 | 4273.2 | 1674.9 | 1145.4 | 2206.5 |
| Pancreatic | 202.9 | 6665.9 | 2613.1 | 1649.9 | 3406.5 |
| Pancreatic | 202.4 | 6599.3 | 2617.5 | 1653.9 | 3388.8 |
| Pancreatic | 252.0 | 8195.1 | 3279.1 | 2049.6 | 4194.9 |
| Pancreatic | 157.2 | 5239.7 | 2039.3 | 1272.7 | 2691.3 |
| Pancreatic | 151.5 | 5065.5 | 1966.8 | 1230.3 | 2614.5 |
| Pancreatic | 166.2 | 5549.0 | 2181.2 | 1361.0 | 2875.4 |
| Pancreatic | 246.1 | 7850.9 | 3175.8 | 2025.3 | 4150.4 |

(continued)

| Cancer Subproteome | Total molar concentration (μM) | [K] | [C] | [W] | [Y] |
|---|---|---|---|---|---|
| Pancreatic | 199.0 | 6557.9 | 2617.3 | 1633.3 | 3428.8 |
| Pancreatic | 186.2 | 6085.0 | 2463.9 | 1534.5 | 3196.6 |
| Pancreatic | 176.4 | 5873.4 | 2332.1 | 1481.3 | 3063.5 |
| Pancreatic | 222.3 | 7407.5 | 2927.2 | 1877.1 | 3841.6 |
| Pancreatic | 197.7 | 6624.0 | 2626.7 | 1680.7 | 3448.1 |
| Pancreatic | 225.4 | 6583.2 | 2745.4 | 2015.8 | 3626.8 |
| Pancreatic | 222.0 | 6630.5 | 2755.5 | 2011.6 | 3609.0 |
| Pancreatic | 317.1 | 9503.4 | 3928.7 | 2820.2 | 5153.3 |
| Pancreatic | 271.8 | 8460.3 | 3423.2 | 2240.1 | 4437.2 |
| Pancreatic | 187.6 | 5885.2 | 2368.9 | 1549.2 | 3082.8 |
| Pancreatic | 165.5 | 5227.5 | 2127.5 | 1390.7 | 2733.5 |
| Pancreatic | 230.8 | 8416.4 | 3234.7 | 1972.7 | 4333.5 |
| Pancreatic | 396.1 | 14426.0 | 5546.3 | 3362.8 | 7429.9 |
| Pancreatic | 239.4 | 8946.3 | 3389.4 | 2114.3 | 4643.7 |
| Pancreatic | 138.7 | 4923.8 | 1802.5 | 1147.8 | 2512.1 |
| Pancreatic | 161.0 | 5785.7 | 2097.0 | 1324.8 | 2929.7 |
| Pancreatic | 189.4 | 6953.2 | 2529.6 | 1617.5 | 3532.9 |
| Pancreatic | 165.2 | 5476.8 | 2123.8 | 1377.1 | 2826.7 |
| Pancreatic | 233.7 | 7935.5 | 3089.7 | 1956.7 | 4085.0 |
| Pancreatic | 278.8 | 9373.0 | 3663.6 | 2398.2 | 4876.2 |
| Pancreatic | 219.7 | 7755.1 | 2960.1 | 1858.8 | 3989.7 |
| Pancreatic | 229.0 | 8262.5 | 3177.9 | 1951.7 | 4220.6 |
| Pancreatic | 228.3 | 8191.0 | 3189.3 | 1965.7 | 4220.4 |
| Pancreatic | 225.1 | 6754.7 | 2945.4 | 1959.6 | 3672.6 |
| Pancreatic | 241.7 | 7370.6 | 3118.5 | 2137.0 | 4039.8 |
| Pancreatic | 248.3 | 7686.2 | 3231.8 | 2236.5 | 4156.5 |
| Pancreatic | 202.0 | 6250.5 | 2475.7 | 1687.6 | 3327.7 |
| Pancreatic | 265.1 | 8271.4 | 3284.9 | 2295.0 | 4429.6 |
| Pancreatic | 198.6 | 6281.5 | 2524.1 | 1703.2 | 3342.1 |
| Pancreatic | 240.1 | 7792.3 | 3061.3 | 2024.8 | 4082.2 |
| Pancreatic | 202.9 | 6727.0 | 2641.1 | 1702.1 | 3516.6 |
| Pancreatic | 196.2 | 6377.9 | 2539.3 | 1655.3 | 3361.3 |
| Pancreatic | 219.2 | 7419.8 | 2759.2 | 1780.7 | 3803.8 |
| Pancreatic | 264.1 | 8532.5 | 3152.6 | 2110.3 | 4378.0 |
| Pancreatic | 207.8 | 6997.3 | 2554.8 | 1662.7 | 3565.2 |
| Pancreatic | 197.0 | 6545.7 | 2451.3 | 1605.1 | 3341.9 |
| Pancreatic | 140.4 | 4851.2 | 1877.7 | 1222.2 | 2477.0 |
| Pancreatic | 228.1 | 7823.0 | 3052.6 | 1956.5 | 3984.2 |
| Pancreatic | 248.5 | 7335.9 | 3043.5 | 1950.1 | 3826.5 |

(continued)

| Cancer Subproteome | Total molar concentration (μM) | [K] | [C] | [W] | [Y] |
|---|---|---|---|---|---|
| Pancreatic | 258.0 | 7998.5 | 3298.7 | 2199.0 | 4226.8 |
| Pancreatic | 278.9 | 8372.9 | 3501.1 | 2378.5 | 4412.1 |
| Pancreatic | 463.8 | 16881.5 | 6293.7 | 4177.7 | 8670.2 |
| Pancreatic | 293.5 | 10686.8 | 3983.6 | 2650.7 | 5480.4 |
| Pancreatic | 332.2 | 12028.0 | 4576.0 | 2986.4 | 6185.9 |
| Pancreatic | 236.0 | 8332.8 | 3160.0 | 2138.7 | 4322.4 |
| Pancreatic | 239.7 | 8434.6 | 3246.0 | 2067.2 | 4340.2 |
| Pancreatic | 396.4 | 14117.1 | 5489.9 | 3544.3 | 7334.7 |
| Pancreatic | 202.6 | 7661.8 | 2914.9 | 1838.4 | 3951.5 |
| Pancreatic | 224.5 | 8437.7 | 3282.0 | 2000.6 | 4358.5 |
| Pancreatic | 196.4 | 7271.3 | 2901.7 | 1802.5 | 3801.2 |

Towards the aim of testing the ability of the methods of the invention to identify three cancer signatures from blood plasma, an additional dataset was added. The PXD013150 dataset was accessed at http://proteomecentral.proteomexchange.org/cgi/GetDataset?ID=PXD013150 and downloaded from Proteome Xchange. This dataset provided quantitative mass spectrometry data on proteins measured within the plasma samples of patients with colorectal cancer, prior to clinical intervention.

Although many steps are replicated, the processing of this dataset is explained because it is slightly different. In this study from which the PXD013150 dataset was derived, the authors had measured the total protein concentration by mass of each plasma sample using the BCA assay, and standardized the sample such that each had 100 μg total protein by mass. This feature allowed an alternative normalization to be performed when applying the methods disclosed herein to this dataset. The mass spectrometry intensity values are proportional to mass (https://www.ncbi.nlm.nih.gov/pmc/articles/PMC3946283/). It was known that the total mass concentration for all proteins in each was 100 μg, because this had been calculated using the BCA assay. Because the mass spectrometry intensity values are proportional to mass, for each sample the sum of the intensity values is equal to the 100 μg total protein. Therefore, calculating the intensity of each protein divided by the sum of the intensities across all proteins in a sample $\frac{int}{\Sigma int}$ provides the fractional mass amount of the individual protein within the sample. This was then multiplied by the provided total protein mass amount per sample of 100 μg to provide an mass amount in μg of each individual protein within each sample. Then, for each protein within each sample, the mass amount in μg was divided by the protein molecular weight (which had been downloaded from Uniprot as disclosed herein) in g/mol, providing the molar amount in μmol. The molar amounts of all proteins within each sample were summed, providing a total molar amount of all proteins within each sample, reported in Table 38.

In equation 11, $q_i$ is a measure of the quantity of protein *i* in the proteome or subproteome of interest, and q is an equivalent measure of the total quantity of all proteins in the proteome or subproteome of interest. Hence, the total molar amount of all proteins within each sample also provides *q* in Equation 11. ${q_i}/{q}$ in Equation 11 was then calculated by dividing the absolute molar amount of each protein within each sample by the total molar amount of all proteins within each sample. Then, Equation 11 was used as disclosed herein to calculate the $w_K$,$w_W$,$w_Y$, and $w_c$ values for each patient plasma sample. The $w_K$,$w_W$,$w_Y$, and $w_c$ were then each multiplied by the value of *q* for each sample, the absolute total molar amount of all proteins within each sample, to calculate the amount of amino acids of the K, C, W, and Y amino acid types within each sample.

The steps are additionally summarized below and the results are reported in Table 39

$\frac{Int}{\Sigma Int}$ = Normalized intensity calculated from a mass spectrometry database such as Proteome Xchange (this is a fraction, units are AU) $\frac{Int}{\Sigma Int}$ (fraction, AU) x 100 μg total protein = Mass amount of each protein (μg)

Mass amount (μg) / mass of the protein (g/mol) = Molar amount (μmol)

Sum of molar amount (μmol) gives total molar amount (μmol)

Molar amount (μmol) / total molar amount (μmol) gives $q_i/q$ in Equation 11.

Table 38: Total molar amount (nmol), K amount (nmol), C amount (nmol), W amount (nmol) and Y amount (nmol) for the colorectal cancer dataset.

| Cancer | Sample.ID | Total molar amount (nmol) | K nmol | C nmol | W nmol | Y nmol |
|---|---|---|---|---|---|---|
| Colorectal | PC1090 | 1.19 | 33.72 | 15.24 | 6.64 | 16.14 |
| Colorectal | PC1188 | 1.11 | 32.73 | 14.93 | 6.46 | 15.95 |
| Colorectal | PC1293 | 1.16 | 33.95 | 15.69 | 6.59 | 16.37 |
| Colorectal | PC1652 | 1.06 | 30.46 | 13.92 | 6.14 | 14.90 |
| Colorectal | PC1777 | 1.19 | 34.00 | 15.78 | 6.59 | 16.43 |
| Colorectal | PC1978 | 1.17 | 33.25 | 14.96 | 6.68 | 16.11 |
| Colorectal | PC2209 | 1.08 | 31.55 | 14.85 | 6.40 | 15.56 |
| Colorectal | PC2355 | 1.12 | 32.91 | 15.18 | 6.38 | 15.92 |
| Colorectal | PC707 | 1.10 | 32.47 | 15.23 | 6.32 | 15.77 |
| Colorectal | PC775 | 1.14 | 31.36 | 16.56 | 7.08 | 16.30 |
| Colorectal | PC1057 | 1.20 | 32.66 | 16.78 | 7.21 | 16.75 |
| Colorectal | PC1481 | 1.22 | 34.69 | 15.89 | 6.47 | 16.40 |
| Colorectal | PC1700 | 1.16 | 33.28 | 16.11 | 6.25 | 16.02 |
| Colorectal | PC2016 | 1.21 | 35.33 | 15.94 | 6.54 | 16.61 |
| Colorectal | PC2045 | 1.22 | 34.55 | 15.78 | 6.59 | 16.47 |
| Colorectal | PC2086 | 1.08 | 31.13 | 14.59 | 5.96 | 15.06 |
| Colorectal | PC2218 | 1.16 | 33.59 | 15.02 | 6.27 | 15.84 |
| Colorectal | PC2371 | 1.10 | 32.08 | 14.93 | 6.05 | 15.32 |
| Colorectal | PC428 | 1.29 | 34.62 | 14.25 | 6.97 | 16.17 |
| Colorectal | PC741 | 1.13 | 32.73 | 14.54 | 6.25 | 15.54 |
| Colorectal | PC792 | 1.23 | 35.57 | 16.44 | 6.69 | 16.90 |
| Colorectal | PC813 | 1.16 | 32.95 | 15.79 | 6.30 | 15.81 |
| Colorectal | PC842 | 1.22 | 33.43 | 14.98 | 6.75 | 16.11 |
| Colorectal | PC1074 | 1.15 | 33.21 | 15.20 | 6.48 | 15.98 |
| Colorectal | PC1197 | 1.06 | 31.27 | 14.75 | 6.01 | 15.11 |
| Colorectal | PC1256 | 1.12 | 32.01 | 14.37 | 6.36 | 15.52 |
| Colorectal | PC1346 | 1.08 | 31.91 | 14.59 | 6.02 | 15.17 |
| Colorectal | PC1385 | 1.18 | 34.26 | 15.51 | 6.70 | 16.46 |
| Colorectal | PC2067 | 1.12 | 32.48 | 14.78 | 6.33 | 15.76 |
| Colorectal | PC2234 | 1.14 | 32.88 | 15.14 | 6.41 | 15.75 |
| Colorectal | PC2313 | 1.16 | 33.95 | 15.38 | 6.33 | 16.04 |
| Colorectal | PC2978 | 1.25 | 35.15 | 17.18 | 7.06 | 17.25 |
| Colorectal | PC874 | 1.01 | 30.07 | 13.91 | 5.69 | 14.41 |
| Colorectal | PC977 | 1.13 | 31.97 | 14.95 | 6.25 | 15.49 |
| Colorectal | PC1189 | 1.27 | 34.84 | 16.26 | 7.22 | 16.94 |

(continued)

| Cancer | Sample.ID | Total molar amount (nmol) | K nmol | C nmol | W nmol | Y nmol |
|---|---|---|---|---|---|---|
| Colorectal | PC1222 | 1.17 | 33.17 | 15.69 | 6.56 | 16.05 |
| Colorectal | PC1336 | 1.13 | 33.08 | 15.13 | 6.53 | 15.93 |
| Colorectal | PC1437 | 1.10 | 32.17 | 15.09 | 6.27 | 15.55 |
| Colorectal | PC1452 | 1.13 | 32.94 | 15.00 | 6.45 | 15.78 |
| Colorectal | PC1521 | 1.20 | 34.63 | 15.36 | 6.67 | 16.41 |
| Colorectal | PC1605 | 1.18 | 32.98 | 15.40 | 6.49 | 15.82 |
| Colorectal | PC1805 | 1.24 | 35.29 | 16.62 | 6.99 | 17.13 |
| Colorectal | PC2095 | 1.13 | 33.56 | 15.57 | 6.50 | 16.20 |
| Colorectal | PC2112 | 1.18 | 33.86 | 15.21 | 6.50 | 16.03 |
| Colorectal | PC2366 | 1.13 | 32.05 | 14.67 | 6.31 | 15.37 |
| Colorectal | PC2519 | 1.13 | 32.80 | 15.14 | 6.40 | 15.83 |
| Colorectal | PC452 | 1.07 | 31.71 | 14.56 | 6.31 | 15.42 |
| Colorectal | PC458 | 1.15 | 32.30 | 15.10 | 6.43 | 15.68 |
| Colorectal | PC465 | 1.23 | 34.68 | 15.64 | 6.97 | 16.72 |
| Colorectal | PC639 | 1.13 | 32.58 | 15.40 | 6.40 | 15.69 |
| Colorectal | PC1079 | 1.16 | 32.88 | 15.00 | 6.42 | 15.78 |
| Colorectal | PC1096 | 1.27 | 36.35 | 16.23 | 6.85 | 17.02 |
| Colorectal | PC1136 | 1.17 | 34.32 | 16.17 | 6.65 | 16.56 |
| Colorectal | PC1506 | 1.27 | 35.82 | 17.67 | 6.86 | 17.17 |
| Colorectal | PC1608 | 1.15 | 33.55 | 15.65 | 6.24 | 15.95 |
| Colorectal | PC1703 | 1.17 | 33.19 | 16.46 | 6.61 | 16.19 |
| Colorectal | PC1728 | 1.19 | 33.23 | 16.23 | 6.71 | 16.29 |
| Colorectal | PC1800 | 1.12 | 33.97 | 15.69 | 6.22 | 15.95 |
| Colorectal | PC1982 | 1.11 | 32.45 | 15.30 | 6.15 | 15.42 |
| Colorectal | PC2030 | 1.13 | 33.40 | 15.35 | 6.49 | 16.06 |
| Colorectal | PC2204 | 1.15 | 33.52 | 15.28 | 6.33 | 15.96 |
| Colorectal | PC2208 | 1.14 | 33.40 | 15.37 | 6.17 | 15.75 |
| Colorectal | PC2337 | 1.13 | 33.29 | 16.08 | 6.31 | 16.04 |
| Colorectal | PC565 | 1.17 | 34.13 | 15.88 | 6.39 | 16.41 |
| Colorectal | PC596 | 1.17 | 33.01 | 15.48 | 6.54 | 15.93 |
| Colorectal | PC711 | 1.19 | 33.96 | 15.43 | 6.48 | 16.04 |
| Colorectal | PC796 | 1.21 | 34.31 | 16.56 | 6.59 | 16.43 |
| Colorectal | PC1081 | 1.20 | 33.46 | 16.69 | 7.17 | 16.94 |
| Colorectal | PC1084 | 1.13 | 31.81 | 14.80 | 6.41 | 15.50 |
| Colorectal | PC1247 | 1.02 | 30.16 | 14.43 | 5.90 | 14.71 |
| Colorectal | PC1595 | 1.06 | 31.19 | 14.11 | 6.12 | 14.99 |
| Colorectal | PC1613 | 1.10 | 31.84 | 14.30 | 6.16 | 15.29 |
| Colorectal | PC1789 | 1.10 | 32.36 | 14.20 | 6.30 | 15.49 |
| Colorectal | PC1794 | 1.09 | 30.82 | 14.13 | 6.25 | 15.19 |

(continued)

| Cancer | Sample.ID | Total molar amount (nmol) | K nmol | C nmol | W nmol | Y nmol |
|---|---|---|---|---|---|---|
| Colorectal | PC2070 | 1.13 | 32.23 | 15.59 | 6.50 | 15.84 |
| Colorectal | PC2099 | 1.11 | 32.16 | 14.69 | 6.23 | 15.54 |
| Colorectal | PC2170 | 1.15 | 33.31 | 14.91 | 6.60 | 15.95 |
| Colorectal | PC2797 | 1.07 | 30.90 | 14.62 | 6.20 | 15.20 |
| Colorectal | PC490 | 1.15 | 33.45 | 15.50 | 6.57 | 16.19 |
| Colorectal | PC515 | 1.21 | 34.26 | 16.06 | 6.69 | 16.51 |
| Colorectal | PC556 | 1.16 | 32.90 | 16.02 | 6.78 | 16.41 |
| Colorectal | PC890 | 1.21 | 32.61 | 16.88 | 7.13 | 16.86 |
| Colorectal | PC962 | 1.07 | 30.96 | 14.68 | 6.02 | 15.07 |

The total molar amount values which were calculated above for colorectal cancer above were transformed into total molar concentration values by assuming a standard volume of 5 μL for all samples. As explained previously, to convert an amount to a concentration, the amount is divided by a volume. This transformed the colorectal cancer total molar amount vales to total molar concentration values which were comparable to the total molar concentration values which had been calculated for ovarian cancer and pancreatic cancer, allowing identification of more than one disease state proteome of interest from patient plasma samples to be assessed in the most rigorous and robust circumstances.

The colorectal cancer total molar concentration and total molar amino acid concentration values calculated as described above are presented in Table 39. Their total molar concentration values in μM, which were calculated as explained above, are also provided in this table. Additionally, the amino acid concentrations for the colorectal cancer subproteome of interest were plotted as a function of total molar protein concentration to calculate the reference line for the colorectal cancer subproteome of interest, as shown in Figures 59-62.

Table 39: Colorectal cancer total molar concentration and total molar amino acid concentration values

| Cancer | Sample.ID | Total molar concentration (μM) | [K (μM)] | [C (μM)] | [W (μM)] | [Y (μM)] |
|---|---|---|---|---|---|---|
| Colorectal | PC1090 | 238.79 | 6744.73 | 3047.85 | 1328.31 | 3227.02 |
| Colorectal | PC1188 | 222.26 | 6545.32 | 2985.85 | 1292.27 | 3189.68 |
| Colorectal | PC1293 | 232.84 | 6789.73 | 3138.35 | 1317.68 | 3274.47 |
| Colorectal | PC1652 | 211.38 | 6091.50 | 2784.69 | 1228.34 | 2979.94 |
| Colorectal | PC1777 | 238.51 | 6799.28 | 3156.04 | 1317.65 | 3285.52 |
| Colorectal | PC1978 | 233.06 | 6650.61 | 2991.14 | 1335.73 | 3222.99 |
| Colorectal | PC2209 | 216.32 | 6310.05 | 2970.60 | 1279.39 | 3111.53 |
| Colorectal | PC2355 | 224.46 | 6582.87 | 3036.09 | 1276.07 | 3183.53 |
| Colorectal | PC707 | 219.37 | 6493.92 | 3046.55 | 1264.73 | 3154.41 |
| Colorectal | PC775 | 228.16 | 6272.63 | 3311.92 | 1415.01 | 3260.85 |
| Colorectal | PC1057 | 239.82 | 6532.92 | 3356.83 | 1442.12 | 3349.29 |
| Colorectal | PC1481 | 243.53 | 6937.41 | 3178.61 | 1294.74 | 3279.01 |
| Colorectal | PC1700 | 232.55 | 6655.27 | 3222.27 | 1250.84 | 3204.07 |
| Colorectal | PC2016 | 241.60 | 7066.28 | 3187.62 | 1309.00 | 3322.09 |
| Colorectal | PC2045 | 243.78 | 6910.74 | 3155.80 | 1318.25 | 3294.00 |
| Colorectal | PC2086 | 215.16 | 6226.63 | 2917.78 | 1191.68 | 3012.12 |
| Colorectal | PC2218 | 231.52 | 6718.92 | 3003.08 | 1253.80 | 3168.20 |
| Colorectal | PC2371 | 220.65 | 6415.90 | 2986.70 | 1209.20 | 3063.80 |
| Colorectal | PC428 | 257.07 | 6924.98 | 2850.65 | 1394.61 | 3234.47 |

(continued)

| Cancer | Sample.ID | Total molar concentration (μM) | [K (μM)] | [C (μM)] | [W (μM)] | [Y (μM)] |
|---|---|---|---|---|---|---|
| Colorectal | PC741 | 226.24 | 6546.09 | 2908.77 | 1250.01 | 3107.00 |
| Colorectal | PC792 | 245.29 | 7114.21 | 3287.18 | 1337.78 | 3380.62 |
| Colorectal | PC813 | 232.79 | 6589.81 | 3157.68 | 1259.56 | 3161.13 |
| Colorectal | PC842 | 243.27 | 6685.07 | 2996.86 | 1350.16 | 3222.18 |
| Colorectal | PC1074 | 230.49 | 6641.75 | 3039.44 | 1296.21 | 3196.17 |
| Colorectal | PC1197 | 211.73 | 6253.88 | 2949.93 | 1201.84 | 3022.39 |
| Colorectal | PC1256 | 223.94 | 6402.52 | 2873.57 | 1272.60 | 3104.08 |
| Colorectal | PC1346 | 215.19 | 6381.52 | 2918.86 | 1203.17 | 3034.62 |
| Colorectal | PC1385 | 236.87 | 6852.47 | 3102.92 | 1340.07 | 3291.85 |
| Colorectal | PC2067 | 224.10 | 6496.51 | 2956.15 | 1266.12 | 3152.08 |
| Colorectal | PC2234 | 228.49 | 6575.83 | 3027.81 | 1282.43 | 3149.37 |
| Colorectal | PC2313 | 231.93 | 6789.84 | 3076.43 | 1265.46 | 3208.41 |
| Colorectal | PC2978 | 249.23 | 7029.48 | 3435.16 | 1411.07 | 3450.26 |
| Colorectal | PC874 | 202.20 | 6013.30 | 2782.83 | 1137.44 | 2881.28 |
| Colorectal | PC977 | 225.99 | 6394.37 | 2990.76 | 1250.99 | 3098.16 |
| Colorectal | PC1189 | 254.63 | 6968.53 | 3251.04 | 1443.61 | 3388.15 |
| Colorectal | PC1222 | 233.55 | 6633.32 | 3138.78 | 1311.65 | 3209.78 |
| Colorectal | PC1336 | 226.04 | 6615.58 | 3025.89 | 1306.06 | 3186.03 |
| Colorectal | PC1437 | 220.79 | 6433.05 | 3018.52 | 1254.24 | 3109.71 |
| Colorectal | PC1452 | 225.55 | 6587.78 | 3000.66 | 1290.36 | 3156.58 |
| Colorectal | PC1521 | 240.03 | 6926.56 | 3071.28 | 1334.57 | 3281.84 |
| Colorectal | PC1605 | 235.52 | 6596.40 | 3079.05 | 1298.55 | 3164.86 |
| Colorectal | PC1805 | 247.95 | 7058.47 | 3324.61 | 1398.81 | 3426.58 |
| Colorectal | PC2095 | 226.49 | 6712.27 | 3114.40 | 1299.46 | 3239.75 |
| Colorectal | PC2112 | 235.71 | 6771.51 | 3041.05 | 1299.62 | 3205.90 |
| Colorectal | PC2366 | 225.92 | 6409.06 | 2934.36 | 1262.16 | 3073.22 |
| Colorectal | PC2519 | 226.95 | 6560.32 | 3027.16 | 1279.42 | 3165.19 |
| Colorectal | PC452 | 214.06 | 6341.22 | 2912.85 | 1262.28 | 3083.92 |
| Colorectal | PC458 | 230.42 | 6459.91 | 3019.27 | 1285.74 | 3135.18 |
| Colorectal | PC465 | 246.13 | 6937.00 | 3128.16 | 1393.59 | 3344.44 |
| Colorectal | PC639 | 226.98 | 6516.11 | 3079.57 | 1280.69 | 3138.90 |
| Colorectal | PC1079 | 232.72 | 6575.15 | 2999.22 | 1284.15 | 3155.47 |
| Colorectal | PC1096 | 253.74 | 7269.41 | 3245.80 | 1369.06 | 3404.48 |
| Colorectal | PC1136 | 233.82 | 6863.43 | 3233.96 | 1330.22 | 3311.25 |
| Colorectal | PC1506 | 253.14 | 7164.81 | 3534.13 | 1372.87 | 3434.39 |
| Colorectal | PC1608 | 230.43 | 6710.37 | 3130.65 | 1247.29 | 3190.91 |
| Colorectal | PC1703 | 234.44 | 6638.67 | 3291.08 | 1322.02 | 3238.27 |
| Colorectal | PC1728 | 238.84 | 6645.12 | 3246.47 | 1342.19 | 3258.91 |
| Colorectal | PC1800 | 223.76 | 6793.91 | 3138.16 | 1243.80 | 3189.49 |

(continued)

| Cancer | Sample.ID | Total molar concentration (μM) | [K (μM)] | [C (μM)] | [W (μM)] | [Y (μM)] |
|---|---|---|---|---|---|---|
| Colorectal | PC1982 | 222.21 | 6489.94 | 3060.33 | 1230.06 | 3083.43 |
| Colorectal | PC2030 | 226.22 | 6680.30 | 3069.90 | 1297.58 | 3211.99 |
| Colorectal | PC2204 | 229.73 | 6703.65 | 3055.24 | 1265.53 | 3192.17 |
| Colorectal | PC2208 | 228.31 | 6679.95 | 3073.75 | 1234.11 | 3150.82 |
| Colorectal | PC2337 | 226.97 | 6658.06 | 3216.27 | 1261.70 | 3208.12 |
| Colorectal | PC565 | 233.00 | 6826.83 | 3175.05 | 1277.28 | 3282.73 |
| Colorectal | PC596 | 234.73 | 6602.16 | 3096.31 | 1308.28 | 3186.43 |
| Colorectal | PC711 | 238.71 | 6791.71 | 3086.12 | 1295.13 | 3208.25 |
| Colorectal | PC796 | 242.63 | 6861.08 | 3312.55 | 1318.83 | 3285.89 |
| Colorectal | PC1081 | 240.25 | 6691.94 | 3338.42 | 1434.17 | 3388.33 |
| Colorectal | PC1084 | 226.81 | 6362.18 | 2960.64 | 1281.96 | 3100.80 |
| Colorectal | PC1247 | 203.94 | 6031.29 | 2886.53 | 1180.54 | 2942.11 |
| Colorectal | PC1595 | 212.92 | 6237.39 | 2821.65 | 1223.91 | 2998.17 |
| Colorectal | PC1613 | 220.93 | 6367.53 | 2860.75 | 1231.81 | 3058.06 |
| Colorectal | PC1789 | 219.51 | 6472.01 | 2839.71 | 1260.11 | 3097.56 |
| Colorectal | PC1794 | 217.29 | 6164.99 | 2825.13 | 1249.20 | 3037.43 |
| Colorectal | PC2070 | 225.36 | 6446.94 | 3117.50 | 1300.11 | 3167.33 |
| Colorectal | PC2099 | 221.11 | 6431.56 | 2937.57 | 1245.95 | 3107.02 |
| Colorectal | PC2170 | 230.07 | 6661.32 | 2982.95 | 1319.58 | 3190.16 |
| Colorectal | PC2797 | 214.95 | 6180.55 | 2923.88 | 1239.68 | 3039.12 |
| Colorectal | PC490 | 230.24 | 6690.38 | 3100.24 | 1313.42 | 3237.94 |
| Colorectal | PC515 | 242.65 | 6851.33 | 3212.03 | 1338.22 | 3302.63 |
| Colorectal | PC556 | 232.05 | 6579.84 | 3204.90 | 1355.08 | 3282.20 |
| Colorectal | PC890 | 241.56 | 6521.72 | 3376.33 | 1425.05 | 3372.76 |
| Colorectal | PC962 | 213.72 | 6191.46 | 2935.30 | 1204.71 | 3014.26 |

Finally, all of these datasets were plotted in N-dimensional space, along with the experimental values measured in Examples 10-12 for the healthy patient subproteome of interest (PPP sample type). The results are shown in Figure 50. The linear nature of the data allows convenient visualization of the reference lines.

When these results were plotted in 4-dimensional space for the 4 types of amino acids labelled and measured (N-dimensional space), a striking differentiation of the positions of the healthy patient (PPP, Worked Examples 10-12), ovarian cancer, pancreatic cancer, and colorectal cancer samples in 4-dimensional space is observed in Figure 50. Additionally, each data set takes the form of a line as disclosed herein and is well described by a reference line. The equation of each reference line is provided by vector function.

To calculate the direction/coefficient of each vector function component for vector function 2 using the provided data from many patient samples, each amino acid concentration for each proteome of interest was plotted as a function of the total molar protein concentration of each proteome of interest because the total protein concentration of each proteome of interest is the common (parametric/underlying) via which the amino acid concentration references for each proteome of interest are expressed algebraically via vector function 2. Then, a linear regression was performed and this slope provided the vector direction. The linear regressions are shown in Figures 51-62. As an alternative approach, the mean of the $w_K$, $w_C$, $w_W$ and $w_Y$ values across all patient samples for each proteome of interest (healthy PPP, ovarian cancer, pancreatic cancer, colorectal cancer) could have been calculated but the approach presents here illustrates the strong linearity of all datasets.

The equation of each reference line is provided by vector function 2, and is included below for the K, C, W and Y amino acid types respectively. Because the references lines for colorectal cancer, pancreatic cancer, and ovarian cancer

were calculated theoretically, the reference line calculated theoretically for the healthy patient PPP dataset is also provided for a robust test of the method.

$$p_{colorectal\ cancer}(t) = \langle 28.695t, 13.349t, 5.619t, 13.850t \rangle$$

$$p_{pancreatic\ cancer}(t) = \langle 33.378t, 13.138t, 8.611t, 17.440t \rangle$$

$$p_{ovarian\ cancer}(t) = \langle 33.340t, 14.772t, 9.1417t, 19.885t \rangle$$

$$p_{healthy\ PPP}(t) = \langle 50.088t, 28.130t, 4.504t, 19.169t \rangle$$

The patient samples were compared to a reference for each proteome of interest (healthy PPP, ovarian cancer blood plasma, pancreatic cancer blood plasma, colorectal cancer blood plasma). As disclosed herein, each reference was provided algebraically at any total protein concentration/amount by a vector function. As disclosed herein, each reference was provided by vector function 2. Alternatively, each reference could have been provided by set of parametric equations 2 yielding equivalent results. Then, Test 2 was carried out to determine whether each sample was within an error margin of the vector function reference for each proteome of interest. Because it was known that in each study, patients were excluded that had more than one cancer type, it was known that only one proteome of interest was present within each patient sample. Therefore, as disclosed herein, the error margin was set equal to the minimum value of the perpendicular distance between the sample point and each reference line. As disclosed herein, the perpendicular distance between the sample point and each reference line was calculated using the dot product (Examples 1-9). Because the error margin was set equal to the minimum value of the perpendicular distance, each sample point was identified as the proteome of interest defined by the reference line which provided the minimum value of the perpendicular distance. When a proteome of interest was identified within the sample, the total concentration of that proteome of interest within the sample was also identified via finding the specific point on the reference line which had provided the minimum value of the perpendicular distance, as disclosed herein. The results are shown below in Table 40 and summarized in Figure 63.

Table 40

| Patient Cancer Status | sol. T Healthy (µM) | sol. T Ovarian (µM) | sol. T Pancreatic (µM) | sol. T Colorectal (µM) | Minimum Distance of Amino Acid Concentrations (Test 2) | Predicted Status | Correct ? | Identified Total protein concentration |
|---|---|---|---|---|---|---|---|---|
| Healthy | 1101.2 | 1528.4 | 1608.4 | 1897.4 | 7653.8 | Healthy | Y | 1101.2 |
| Healthy | 1128.9 | 1569.9 | 1647.8 | 1945.3 | 4986.5 | Healthy | Y | 1128.9 |
| Healthy | 1103.0 | 1533.5 | 1607.3 | 1899.1 | 2980.9 | Healthy | Y | 1103.0 |
| Healthy | 941.2 | 1304.6 | 1368.1 | 1617.9 | 2697.3 | Healthy | Y | 941.2 |
| Healthy | 993.6 | 1367.4 | 1434.4 | 1700.6 | 3145.6 | Healthy | Y | 993.6 |
| Healthy | 1052.5 | 1457.5 | 1526.6 | 1807.1 | 1637.7 | Healthy | Y | 1052.5 |
| Healthy | 1031.7 | 1431.9 | 1497.1 | 1772.5 | 585.0 | Healthy | Y | 1031.7 |
| Healthy | 929.6 | 1314.3 | 1368.9 | 1612.8 | 5314.9 | Healthy | Y | 929.6 |
| Healthy | 1051.0 | 1496.4 | 1564.1 | 1834.4 | 3527.4 | Colorectal Cancer | N | 1834.4 |
| Healthy | 988.5 | 1383.3 | 1448.7 | 1708.2 | 3479.6 | Healthy | Y | 988.5 |
| Healthy | 1022.5 | 1436.7 | 1502.8 | 1770.6 | 3893.4 | Healthy | Y | 1022.5 |
| Healthy | 970.9 | 1365.7 | 1427.1 | 1681.6 | 3757.7 | Healthy | Y | 970.9 |
| Healthy | 978.1 | 1368.4 | 1431.9 | 1689.3 | 2666.8 | Healthy | Y | 978.1 |
| Healthy | 1168.5 | 1618.1 | 1695.8 | 2006.8 | 2392.6 | Healthy | Y | 1168.5 |
| Healthy | 1011.3 | 1430.1 | 1490.6 | 1755.4 | 5680.9 | Colorectal Cancer | N | 1755.4 |
| Healthy | 933.8 | 1306.6 | 1366.6 | 1612.6 | 2295.6 | Healthy | Y | 933.8 |
| Healthy | 1429.3 | 2000.7 | 2097.5 | 2471.6 | 6369.7 | Healthy | Y | 1429.3 |
| Healthy | 815.5 | 1012.7 | 1093.2 | 1327.3 | 30613.0 | Healthy | Y | 815.5 |
| Healthy | 996.5 | 1401.9 | 1463.2 | 1725.2 | 4316.2 | Healthy | Y | 996.5 |
| Healthy | 1079.3 | 1495.6 | 1567.0 | 1854.2 | 2010.8 | Healthy | Y | 1079.3 |
| Healthy | 1035.0 | 1434.8 | 1511.0 | 1782.6 | 7864.1 | Healthy | Y | 1035.0 |
| Healthy | 1083.9 | 1498.9 | 1574.3 | 1861.8 | 4723.2 | Healthy | Y | 1083.9 |
| Healthy | 1054.8 | 1456.2 | 1527.6 | 1808.8 | 3177.7 | Healthy | Y | 1054.8 |
| Healthy | 886.5 | 1221.5 | 1282.1 | 1518.8 | 3154.8 | Healthy | Y | 886.5 |
| Healthy | 928.9 | 1276.6 | 1339.3 | 1588.5 | 3120.8 | Healthy | Y | 928.9 |
| Healthy | 986.1 | 1360.0 | 1426.6 | 1690.0 | 2995.0 | Healthy | Y | 986.1 |

| | | | | | | | | |
|---|---|---|---|---|---|---|---|---|
| Healthy | 980.7 | 1352.8 | 1416.3 | 1679.4 | 1796.4 | Healthy | Y | 980.7 |
| Healthy | 861.9 | 1200.8 | 1254.2 | 1483.6 | 1556.6 | Healthy | Y | 861.9 |
| Healthy | 996.8 | 1404.8 | 1470.9 | 1730.0 | 5013.4 | Colorectal Cancer | N | 1730.0 |
| Healthy | 931.9 | 1303.7 | 1362.9 | 1608.8 | 2187.8 | Healthy | Y | 931.9 |
| Healthy | 955.7 | 1349.0 | 1406.9 | 1657.4 | 4564.9 | Healthy | Y | 955.7 |
| Healthy | 938.6 | 1318.3 | 1376.8 | 1623.7 | 3310.7 | Healthy | Y | 938.6 |
| Healthy | 918.8 | 1290.2 | 1347.0 | 1589.0 | 3112.0 | Healthy | Y | 918.8 |
| Healthy | 1120.4 | 1554.2 | 1624.9 | 1924.2 | 581.0 | Healthy | Y | 1120.4 |
| Healthy | 950.3 | 1332.2 | 1389.4 | 1641.0 | 3268.6 | Healthy | Y | 950.3 |
| Healthy | 902.4 | 1262.1 | 1318.2 | 1557.0 | 2088.5 | Healthy | Y | 902.4 |
| Healthy | 1401.4 | 1975.5 | 2064.2 | 2430.4 | 6204.7 | Healthy | Y | 1401.4 |
| Healthy | 785.8 | 974.5 | 1051.5 | 1277.6 | 29674.6 | Healthy | Y | 785.8 |
| Healthy | 947.5 | 1328.1 | 1385.8 | 1636.4 | 2974.2 | Healthy | Y | 947.5 |
| Healthy | 1046.6 | 1450.1 | 1517.6 | 1796.9 | 828.0 | Healthy | Y | 1046.6 |
| Healthy | 1008.7 | 1406.6 | 1480.4 | 1743.3 | 8048.2 | Healthy | Y | 1008.7 |
| Healthy | 1071.5 | 1481.6 | 1555.3 | 1839.9 | 4037.0 | Healthy | Y | 1071.5 |
| Healthy | 1062.2 | 1469.1 | 1540.0 | 1823.1 | 2458.8 | Healthy | Y | 1062.2 |
| Healthy | 889.2 | 1227.9 | 1288.3 | 1525.2 | 2762.0 | Healthy | Y | 889.2 |
| Healthy | 932.0 | 1281.4 | 1344.0 | 1594.1 | 2893.8 | Healthy | Y | 932.0 |
| Healthy | 970.8 | 1341.4 | 1405.7 | 1664.9 | 2023.1 | Healthy | Y | 970.8 |
| Healthy | 957.6 | 1322.9 | 1384.3 | 1641.0 | 1457.9 | Healthy | Y | 957.6 |
| Healthy | 865.2 | 1205.8 | 1259.1 | 1489.4 | 1792.1 | Healthy | Y | 865.2 |
| Healthy | 1018.5 | 1435.3 | 1500.0 | 1766.2 | 4427.5 | Healthy | Y | 1018.5 |
| Healthy | 930.9 | 1297.3 | 1357.6 | 1604.0 | 1568.0 | Healthy | Y | 930.9 |
| Healthy | 955.0 | 1339.1 | 1398.3 | 1650.3 | 2780.3 | Healthy | Y | 955.0 |
| Healthy | 920.7 | 1272.2 | 1333.1 | 1579.0 | 1757.5 | Healthy | Y | 920.7 |
| Healthy | 921.2 | 1290.6 | 1347.9 | 1591.2 | 2493.1 | Healthy | Y | 921.2 |
| Healthy | 1115.2 | 1542.3 | 1613.5 | 1912.2 | 951.1 | Healthy | Y | 1115.2 |
| Healthy | 940.1 | 1312.8 | 1370.1 | 1620.0 | 2311.1 | Healthy | Y | 940.1 |
| Healthy | 913.2 | 1271.0 | 1329.4 | 1571.9 | 1031.1 | Healthy | Y | 913.2 |
| Healthy | 1372.8 | 1923.2 | 2012.1 | 2373.0 | 4040.9 | Healthy | Y | 1372.8 |
| Healthy | 840.8 | 1086.6 | 1158.8 | 1394.5 | 21012.1 | Healthy | Y | 840.8 |
| Healthy | 946.8 | 1324.2 | 1382.9 | 1633.5 | 2184.1 | Healthy | Y | 946.8 |
| Healthy | 1052.9 | 1459.8 | 1527.2 | 1808.2 | 266.9 | Healthy | Y | 1052.9 |
| Ovarian Cancer | 126.0 | 185.2 | 192.8 | 223.9 | 95.0 | Ovarian Cancer | Y | 185.2 |
| Ovarian Cancer | 83.6 | 123.1 | 128.0 | 148.7 | 86.0 | Ovarian Cancer | Y | 123.1 |
| Ovarian Cancer | 100.2 | 147.6 | 153.6 | 178.3 | 46.0 | Ovarian Cancer | Y | 147.6 |
| Ovarian Cancer | 78.2 | 115.2 | 119.8 | 139.1 | 68.4 | Ovarian Cancer | Y | 115.2 |
| Ovarian Cancer | 90.8 | 133.4 | 138.8 | 161.3 | 34.9 | Ovarian Cancer | Y | 133.4 |
| Ovarian Cancer | 86.9 | 128.1 | 133.5 | 154.9 | 208.1 | Pancreatic Cancer | N | 133.5 |
| Ovarian Cancer | 63.6 | 93.3 | 96.9 | 112.8 | 169.8 | Ovarian Cancer | Y | 93.3 |
| Ovarian Cancer | 107.6 | 158.4 | 165.0 | 191.5 | 132.0 | Ovarian Cancer | Y | 158.4 |
| Ovarian Cancer | 73.8 | 108.1 | 112.4 | 130.8 | 161.9 | Ovarian Cancer | Y | 108.1 |
| Ovarian Cancer | 90.2 | 132.1 | 137.3 | 159.9 | 193.5 | Ovarian Cancer | Y | 132.1 |
| Pancreatic Cancer | 157.4 | 230.9 | 240.9 | 279.6 | 243.8 | Pancreatic Cancer | Y | 240.9 |

| | | | | | | | | |
|---|---|---|---|---|---|---|---|---|
| Pancreatic Cancer | 176.4 | 259.0 | 270.1 | 313.5 | 300.7 | Pancreatic Cancer | Y | 270.1 |
| Pancreatic Cancer | 219.3 | 321.9 | 335.6 | 389.7 | 417.4 | Pancreatic Cancer | Y | 335.6 |
| Pancreatic Cancer | 113.6 | 166.4 | 173.6 | 201.6 | 131.2 | Pancreatic Cancer | Y | 173.6 |
| Pancreatic Cancer | 139.2 | 204.1 | 212.9 | 247.2 | 232.4 | Pancreatic Cancer | Y | 212.9 |
| Pancreatic Cancer | 113.2 | 165.9 | 173.0 | 201.0 | 158.9 | Pancreatic Cancer | Y | 173.0 |
| Pancreatic Cancer | 91.4 | 133.8 | 139.8 | 162.3 | 53.4 | Pancreatic Cancer | Y | 139.8 |
| Pancreatic Cancer | 91.3 | 133.6 | 139.5 | 162.0 | 56.5 | Pancreatic Cancer | Y | 139.5 |
| Pancreatic Cancer | 83.7 | 122.5 | 127.9 | 148.6 | 50.6 | Pancreatic Cancer | Y | 127.9 |
| Pancreatic Cancer | 130.2 | 190.0 | 198.5 | 230.7 | 90.8 | Pancreatic Cancer | Y | 198.5 |
| Pancreatic Cancer | 129.2 | 188.6 | 197.0 | 229.0 | 73.7 | Pancreatic Cancer | Y | 197.0 |
| Pancreatic Cancer | 160.6 | 234.3 | 244.7 | 284.5 | 116.1 | Pancreatic Cancer | Y | 244.7 |
| Pancreatic Cancer | 102.3 | 149.3 | 155.9 | 181.2 | 83.7 | Pancreatic Cancer | Y | 155.9 |
| Pancreatic Cancer | 98.9 | 144.4 | 150.8 | 175.3 | 78.3 | Pancreatic Cancer | Y | 150.8 |
| Pancreatic Cancer | 108.6 | 158.6 | 165.6 | 192.5 | 69.8 | Pancreatic Cancer | Y | 165.6 |
| Pancreatic Cancer | 154.9 | 226.5 | 236.4 | 274.7 | 85.7 | Pancreatic Cancer | Y | 236.4 |
| Pancreatic Cancer | 128.9 | 188.2 | 196.5 | 228.4 | 68.8 | Pancreatic Cancer | Y | 196.5 |
| Pancreatic Cancer | 119.9 | 175.2 | 182.8 | 212.6 | 76.1 | Pancreatic Cancer | Y | 182.8 |
| Pancreatic Cancer | 115.3 | 168.5 | 175.9 | 204.4 | 39.8 | Pancreatic Cancer | Y | 175.9 |
| Pancreatic Cancer | 145.2 | 212.2 | 221.5 | 257.5 | 43.2 | Pancreatic Cancer | Y | 221.5 |
| Pancreatic Cancer | 130.0 | 190.0 | 198.3 | 230.5 | 36.2 | Pancreatic Cancer | Y | 198.3 |
| Pancreatic Cancer | 131.7 | 193.8 | 202.0 | 234.4 | 347.7 | Pancreatic Cancer | Y | 202.0 |
| Pancreatic Cancer | 132.3 | 194.6 | 202.9 | 235.4 | 321.1 | Pancreatic Cancer | Y | 202.9 |
| Pancreatic Cancer | 189.3 | 278.2 | 290.1 | 336.6 | 398.3 | Pancreatic Cancer | Y | 290.1 |
| Pancreatic Cancer | 166.8 | 244.0 | 254.7 | 295.9 | 99.1 | Pancreatic Cancer | Y | 254.7 |
| Pancreatic Cancer | 115.9 | 169.5 | 177.0 | 205.6 | 55.2 | Pancreatic Cancer | Y | 177.0 |
| Pancreatic Cancer | 103.1 | 150.8 | 157.4 | 182.9 | 75.0 | Pancreatic Cancer | Y | 157.4 |
| Pancreatic Cancer | 163.9 | 239.2 | 249.8 | 290.4 | 201.8 | Pancreatic Cancer | Y | 249.8 |
| Pancreatic Cancer | 281.0 | 409.9 | 428.2 | 497.8 | 361.8 | Pancreatic Cancer | Y | 428.2 |
| Pancreatic Cancer | 174.1 | 254.3 | 265.7 | 308.7 | 215.8 | Pancreatic Cancer | Y | 265.7 |
| Pancreatic Cancer | 95.1 | 138.9 | 145.2 | 168.6 | 167.2 | Pancreatic Cancer | Y | 145.2 |
| Pancreatic Cancer | 111.4 | 162.7 | 170.1 | 197.6 | 227.1 | Pancreatic Cancer | Y | 170.1 |
| Pancreatic Cancer | 134.1 | 195.8 | 204.7 | 237.8 | 250.1 | Pancreatic Cancer | Y | 204.7 |

| | | | | | | | | |
|---|---|---|---|---|---|---|---|---|
| Pancreatic Cancer | 107.0 | 156.3 | 163.3 | 189.7 | 49.5 | Pancreatic Cancer | Y | 163.3 |
| Pancreatic Cancer | 155.0 | 226.3 | 236.4 | 274.7 | 99.7 | Pancreatic Cancer | Y | 236.4 |
| Pancreatic Cancer | 183.5 | 268.4 | 280.3 | 325.6 | 32.3 | Pancreatic Cancer | Y | 280.3 |
| Pancreatic Cancer | 150.9 | 220.4 | 230.2 | 267.6 | 158.4 | Pancreatic Cancer | Y | 230.2 |
| Pancreatic Cancer | 160.8 | 234.5 | 245.0 | 284.8 | 191.3 | Pancreatic Cancer | Y | 245.0 |
| Pancreatic Cancer | 159.9 | 233.4 | 243.7 | 283.4 | 148.0 | Pancreatic Cancer | Y | 243.7 |
| Pancreatic Cancer | 135.7 | 198.8 | 207.3 | 240.8 | 331.7 | Pancreatic Cancer | Y | 207.3 |
| Pancreatic Cancer | 147.5 | 216.5 | 225.7 | 262.1 | 313.3 | Pancreatic Cancer | Y | 225.7 |
| Pancreatic Cancer | 153.4 | 225.1 | 234.7 | 272.5 | 307.0 | Pancreatic Cancer | Y | 234.7 |
| Pancreatic Cancer | 123.1 | 180.5 | 188.4 | 218.8 | 86.4 | Pancreatic Cancer | Y | 188.4 |
| Pancreatic Cancer | 163.2 | 239.6 | 250.0 | 290.2 | 174.4 | Pancreatic Cancer | Y | 250.0 |
| Pancreatic Cancer | 124.0 | 181.7 | 189.7 | 220.2 | 97.7 | Pancreatic Cancer | Y | 189.7 |
| Pancreatic Cancer | 152.9 | 223.7 | 233.6 | 271.3 | 18.0 | Pancreatic Cancer | Y | 233.6 |
| Pancreatic Cancer | 131.9 | 192.8 | 201.3 | 233.9 | 33.1 | Pancreatic Cancer | Y | 201.3 |
| Pancreatic Cancer | 125.5 | 183.6 | 191.7 | 222.7 | 34.7 | Pancreatic Cancer | Y | 191.7 |
| Pancreatic Cancer | 143.8 | 210.1 | 219.6 | 255.0 | 191.8 | Pancreatic Cancer | Y | 219.6 |
| Pancreatic Cancer | 165.3 | 241.8 | 252.7 | 293.4 | 206.8 | Pancreatic Cancer | Y | 252.7 |
| Pancreatic Cancer | 135.1 | 197.4 | 206.4 | 239.7 | 224.9 | Pancreatic Cancer | Y | 206.4 |
| Pancreatic Cancer | 126.9 | 185.5 | 193.9 | 225.2 | 142.9 | Pancreatic Cancer | Y | 193.9 |
| Pancreatic Cancer | 94.6 | 138.2 | 144.3 | 167.7 | 59.3 | Pancreatic Cancer | Y | 144.3 |
| Pancreatic Cancer | 152.6 | 222.8 | 232.8 | 270.5 | 104.3 | Pancreatic Cancer | Y | 232.8 |
| Pancreatic Cancer | 145.1 | 212.0 | 221.2 | 257.2 | 155.4 | Pancreatic Cancer | Y | 221.2 |
| Pancreatic Cancer | 158.5 | 232.0 | 242.0 | 281.2 | 183.7 | Pancreatic Cancer | Y | 242.0 |
| Pancreatic Cancer | 166.3 | 243.5 | 254.0 | 295.1 | 273.6 | Pancreatic Cancer | Y | 254.0 |
| Pancreatic Cancer | 327.5 | 479.0 | 500.5 | 581.2 | 362.2 | Pancreatic Cancer | Y | 500.5 |
| Pancreatic Cancer | 207.3 | 303.2 | 316.8 | 367.9 | 229.1 | Pancreatic Cancer | Y | 316.8 |
| Pancreatic Cancer | 234.1 | 342.2 | 357.5 | 415.3 | 185.9 | Pancreatic Cancer | Y | 357.5 |
| Pancreatic Cancer | 162.4 | 237.7 | 248.3 | 288.3 | 112.0 | Pancreatic Cancer | Y | 248.3 |
| Pancreatic Cancer | 164.4 | 240.1 | 250.9 | 291.5 | 126.9 | Pancreatic Cancer | Y | 250.9 |
| Pancreatic Cancer | 276.1 | 403.6 | 421.5 | 489.7 | 110.2 | Pancreatic Cancer | Y | 421.5 |
| Pancreatic Cancer | 149.1 | 217.8 | 227.5 | 264.3 | 157.9 | Pancreatic Cancer | Y | 227.5 |
| Pancreatic Cancer | 164.7 | 240.4 | 251.0 | 291.9 | 173.3 | Pancreatic Cancer | Y | 251.0 |

| | | | | | | | | |
|---|---|---|---|---|---|---|---|---|
| Pancreatic Cancer | 142.9 | 208.6 | 217.8 | 253.2 | 83.4 | Pancreatic Cancer | Y | 217.8 |
| Colorectal Cancer | 133.3 | 191.4 | 199.9 | 233.8 | 83.5 | Colorectal Cancer | Y | 233.8 |
| Colorectal Cancer | 129.8 | 186.6 | 194.9 | 227.8 | 66.7 | Colorectal Cancer | Y | 227.8 |
| Colorectal Cancer | 134.8 | 193.4 | 202.0 | 236.3 | 21.1 | Colorectal Cancer | Y | 236.3 |
| Colorectal Cancer | 121.0 | 174.0 | 181.7 | 212.4 | 72.4 | Colorectal Cancer | Y | 212.4 |
| Colorectal Cancer | 135.1 | 193.9 | 202.4 | 236.9 | 15.5 | Colorectal Cancer | Y | 236.9 |
| Colorectal Cancer | 131.5 | 189.2 | 197.6 | 230.9 | 105.0 | Colorectal Cancer | Y | 230.9 |
| Colorectal Cancer | 126.1 | 181.2 | 189.1 | 221.2 | 73.0 | Colorectal Cancer | Y | 221.2 |
| Colorectal Cancer | 130.7 | 187.6 | 195.9 | 229.1 | 28.4 | Colorectal Cancer | Y | 229.1 |
| Colorectal Cancer | 129.4 | 185.7 | 193.8 | 226.8 | 28.8 | Colorectal Cancer | Y | 226.8 |
| Colorectal Cancer | 129.1 | 185.7 | 193.4 | 226.4 | 412.5 | Colorectal Cancer | Y | 226.4 |
| Colorectal Cancer | 133.5 | 191.9 | 200.0 | 234.1 | 339.6 | Colorectal Cancer | Y | 234.1 |
| Colorectal Cancer | 137.1 | 196.4 | 205.2 | 240.2 | 89.9 | Colorectal Cancer | Y | 240.2 |
| Colorectal Cancer | 133.2 | 190.5 | 198.9 | 233.0 | 132.0 | Colorectal Cancer | Y | 233.0 |
| Colorectal Cancer | 139.2 | 199.4 | 208.4 | 243.8 | 127.0 | Colorectal Cancer | Y | 243.8 |
| Colorectal Cancer | 136.7 | 196.0 | 204.8 | 239.6 | 66.7 | Colorectal Cancer | Y | 239.6 |
| Colorectal Cancer | 123.9 | 177.7 | 185.6 | 217.2 | 34.9 | Colorectal Cancer | Y | 217.2 |
| Colorectal Cancer | 132.2 | 189.5 | 198.1 | 231.7 | 130.4 | Colorectal Cancer | Y | 231.7 |
| Colorectal Cancer | 127.3 | 182.4 | 190.5 | 223.0 | 54.0 | Colorectal Cancer | Y | 223.0 |
| Colorectal Cancer | 134.3 | 193.5 | 202.4 | 236.2 | 345.0 | Colorectal Cancer | Y | 236.2 |
| Colorectal Cancer | 128.8 | 184.9 | 193.2 | 225.9 | 127.8 | Colorectal Cancer | Y | 225.9 |
| Colorectal Cancer | 140.9 | 201.9 | 210.9 | 246.8 | 70.0 | Colorectal Cancer | Y | 246.8 |
| Colorectal Cancer | 131.6 | 188.4 | 196.6 | 230.3 | 96.5 | Colorectal Cancer | Y | 230.3 |
| Colorectal Cancer | 132.1 | 189.9 | 198.4 | 231.9 | 114.1 | Colorectal Cancer | Y | 231.9 |
| Colorectal Cancer | 131.6 | 188.9 | 197.3 | 230.8 | 45.6 | Colorectal Cancer | Y | 230.8 |
| Colorectal Cancer | 124.6 | 178.7 | 186.5 | 218.3 | 44.8 | Colorectal Cancer | Y | 218.3 |
| Colorectal Cancer | 126.6 | 182.0 | 190.1 | 222.2 | 102.8 | Colorectal Cancer | Y | 222.2 |
| Colorectal Cancer | 126.2 | 180.9 | 189.0 | 221.1 | 68.8 | Colorectal Cancer | Y | 221.1 |
| Colorectal Cancer | 135.5 | 194.6 | 203.3 | 237.7 | 77.1 | Colorectal Cancer | Y | 237.7 |
| Colorectal Cancer | 128.7 | 184.9 | 193.1 | 225.8 | 65.4 | Colorectal Cancer | Y | 225.8 |
| Colorectal Cancer | 130.3 | 187.0 | 195.3 | 228.5 | 33.2 | Colorectal Cancer | Y | 228.5 |
| Colorectal Cancer | 133.9 | 191.9 | 200.5 | 234.7 | 104.2 | Colorectal Cancer | Y | 234.7 |

| | | | | | | | | |
|---|---|---|---|---|---|---|---|---|
| Colorectal Cancer | 141.3 | 202.7 | 211.5 | 247.6 | 153.0 | Colorectal Cancer | Y | 247.6 |
| Colorectal Cancer | 119.3 | 171.0 | 178.6 | 209.0 | 43.0 | Colorectal Cancer | Y | 209.0 |
| Colorectal Cancer | 127.3 | 182.7 | 190.7 | 223.1 | 16.9 | Colorectal Cancer | Y | 223.1 |
| Colorectal Cancer | 138.8 | 199.5 | 208.3 | 243.6 | 79.2 | Colorectal Cancer | Y | 243.6 |
| Colorectal Cancer | 132.3 | 189.8 | 198.1 | 231.9 | 48.8 | Colorectal Cancer | Y | 231.9 |
| Colorectal Cancer | 131.1 | 188.3 | 196.6 | 230.0 | 49.0 | Colorectal Cancer | Y | 230.0 |
| Colorectal Cancer | 128.1 | 183.8 | 191.8 | 224.5 | 24.5 | Colorectal Cancer | Y | 224.5 |
| Colorectal Cancer | 130.3 | 187.2 | 195.5 | 228.6 | 59.2 | Colorectal Cancer | Y | 228.6 |
| Colorectal Cancer | 136.2 | 195.6 | 204.4 | 238.9 | 140.5 | Colorectal Cancer | Y | 238.9 |
| Colorectal Cancer | 131.1 | 188.1 | 196.4 | 229.8 | 22.6 | Colorectal Cancer | Y | 229.8 |
| Colorectal Cancer | 140.7 | 202.0 | 210.9 | 246.7 | 40.8 | Colorectal Cancer | Y | 246.7 |
| Colorectal Cancer | 133.3 | 191.3 | 199.8 | 233.8 | 16.1 | Colorectal Cancer | Y | 233.8 |
| Colorectal Cancer | 133.4 | 191.5 | 200.0 | 234.0 | 107.3 | Colorectal Cancer | Y | 234.0 |
| Colorectal Cancer | 126.9 | 182.3 | 190.4 | 222.6 | 45.4 | Colorectal Cancer | Y | 222.6 |
| Colorectal Cancer | 130.2 | 186.9 | 195.2 | 228.3 | 22.9 | Colorectal Cancer | Y | 228.3 |
| Colorectal Cancer | 125.9 | 181.0 | 188.9 | 220.9 | 48.3 | Colorectal Cancer | Y | 220.9 |
| Colorectal Cancer | 128.6 | 184.7 | 192.8 | 225.6 | 26.2 | Colorectal Cancer | Y | 225.6 |
| Colorectal Cancer | 137.2 | 197.2 | 206.0 | 240.8 | 99.6 | Colorectal Cancer | Y | 240.8 |
| Colorectal Cancer | 129.9 | 186.2 | 194.4 | 227.6 | 45.9 | Colorectal Cancer | Y | 227.6 |
| Colorectal Cancer | 130.2 | 186.9 | 195.2 | 228.3 | 54.4 | Colorectal Cancer | Y | 228.3 |
| Colorectal Cancer | 142.9 | 204.8 | 214.1 | 250.4 | 148.1 | Colorectal Cancer | Y | 250.4 |
| Colorectal Cancer | 136.7 | 196.0 | 204.7 | 239.6 | 41.4 | Colorectal Cancer | Y | 239.6 |
| Colorectal Cancer | 143.8 | 205.6 | 214.6 | 251.5 | 194.9 | Colorectal Cancer | Y | 251.5 |
| Colorectal Cancer | 133.1 | 190.6 | 199.1 | 233.1 | 78.4 | Colorectal Cancer | Y | 233.1 |
| Colorectal Cancer | 133.7 | 191.5 | 199.8 | 234.0 | 184.0 | Colorectal Cancer | Y | 234.0 |
| Colorectal Cancer | 133.6 | 191.6 | 199.9 | 234.0 | 144.9 | Colorectal Cancer | Y | 234.0 |
| Colorectal Cancer | 134.3 | 192.2 | 200.8 | 235.1 | 112.6 | Colorectal Cancer | Y | 235.1 |
| Colorectal Cancer | 129.0 | 184.7 | 192.9 | 225.9 | 75.2 | Colorectal Cancer | Y | 225.9 |
| Colorectal Cancer | 132.4 | 190.1 | 198.5 | 232.2 | 35.4 | Colorectal Cancer | Y | 232.2 |
| Colorectal Cancer | 132.5 | 190.0 | 198.5 | 232.2 | 75.7 | Colorectal Cancer | Y | 232.2 |
| Colorectal Cancer | 132.1 | 189.1 | 197.6 | 231.3 | 95.4 | Colorectal Cancer | Y | 231.3 |
| Colorectal Cancer | 133.2 | 190.6 | 199.0 | 233.1 | 120.9 | Colorectal Cancer | Y | 233.1 |

| | | | | | | | | |
|---|---|---|---|---|---|---|---|---|
| Colorectal Cancer | 135.6 | 194.3 | 202.9 | 237.5 | 59.0 | Colorectal Cancer | Y | 237.5 |
| Colorectal Cancer | 131.4 | 188.6 | 196.9 | 230.4 | 26.8 | Colorectal Cancer | Y | 230.4 |
| Colorectal Cancer | 134.0 | 192.2 | 200.8 | 234.9 | 87.9 | Colorectal Cancer | Y | 234.9 |
| Colorectal Cancer | 137.1 | 196.3 | 204.9 | 240.0 | 121.8 | Colorectal Cancer | Y | 240.0 |
| Colorectal Cancer | 135.7 | 195.1 | 203.4 | 238.0 | 250.6 | Colorectal Cancer | Y | 238.0 |
| Colorectal Cancer | 126.7 | 182.0 | 190.0 | 222.2 | 43.4 | Colorectal Cancer | Y | 222.2 |
| Colorectal Cancer | 120.7 | 173.1 | 180.6 | 211.4 | 75.1 | Colorectal Cancer | Y | 211.4 |
| Colorectal Cancer | 123.3 | 177.2 | 185.0 | 216.4 | 73.0 | Colorectal Cancer | Y | 216.4 |
| Colorectal Cancer | 125.7 | 180.6 | 188.6 | 220.6 | 92.3 | Colorectal Cancer | Y | 220.6 |
| Colorectal Cancer | 127.2 | 182.9 | 191.1 | 223.4 | 155.3 | Colorectal Cancer | Y | 223.4 |
| Colorectal Cancer | 122.6 | 176.4 | 184.2 | 215.3 | 84.8 | Colorectal Cancer | Y | 215.3 |
| Colorectal Cancer | 129.4 | 185.7 | 193.7 | 226.8 | 114.7 | Colorectal Cancer | Y | 226.8 |
| Colorectal Cancer | 127.4 | 183.0 | 191.1 | 223.5 | 51.7 | Colorectal Cancer | Y | 223.5 |
| Colorectal Cancer | 131.4 | 188.9 | 197.3 | 230.7 | 107.8 | Colorectal Cancer | Y | 230.7 |
| Colorectal Cancer | 123.6 | 177.5 | 185.2 | 216.7 | 65.6 | Colorectal Cancer | Y | 216.7 |
| Colorectal Cancer | 133.0 | 190.9 | 199.3 | 233.1 | 15.3 | Colorectal Cancer | Y | 233.1 |
| Colorectal Cancer | 136.4 | 195.6 | 204.2 | 239.0 | 24.3 | Colorectal Cancer | Y | 239.0 |
| Colorectal Cancer | 132.5 | 190.4 | 198.6 | 232.4 | 157.7 | Colorectal Cancer | Y | 232.4 |
| Colorectal Cancer | 133.6 | 192.1 | 200.1 | 234.2 | 361.3 | Colorectal Cancer | Y | 234.2 |
| Colorectal Cancer | 123.6 | 177.3 | 185.1 | 216.6 | 53.4 | Colorectal Cancer | Y | 216.6 |

The vector function approach described herein has 100% accuracy (sensitivity) for the identification of colorectal cancer from blood plasma, 90% accuracy (sensitivity) for the identification of ovarian cancer from blood plasma, 100% accuracy (sensitivity) for the identification of pancreatic cancer from blood plasma, and 95% accuracy (specificity) for the identification of healthy samples (absence of cancer) from blood plasma.

Additionally, using the vector function approach as described herein, not only the presence or absence of the proteome of interest can be identified, but also the amount of the proteome of interest. Illustrating the robust information available with the methods of the invention, the predicted vs true values for the total protein concentration of the proteome of interest are shown below in Figure 64.

All patient samples fall along the line y = x, meaning that the amount of the cancer or healthy proteome of interest identified is accurate. In preferred embodiments, this amount is correlated with cancer stage.

As an alternative approach, as disclosed herein the proteomes of interest were identified within the sample using a machine learning classifier. Specifically, the dataset was divided randomly into two portions, 75% of the data provided the reference for each proteome of interest which was used to train the classifier, and 25% of the data was held back and used to test the performance of the classifier once trained. A linear support vector machine (SVM) classifier was trained, with box constraint level 1, one-to-one multiclass method, a linear kernel function, automatic kernel scale, and data standardization. The results for identification using the K, C, W and Y amino acid type are shown in Figure 65. A 100% positive predictive value and 0% false discovery rate was obtained for each cancer proteome of interest (all cancer patient samples) as well as the healthy proteome of interest (all healthy patient samples).

However, the methods of the invention provide equivalent results when only three or two amino acid types are

labeled and measured. Illustrating this, Figure 66 provides the results when a linear SVM classifier was trained using the same settings as described above, but only had access to the amino acid concentrations of three amino acid types (K, C, and W). As in the case of four amino acid types, 100% sensitivity and specificity was obtained across all cancer proteomes of interest.

Finally, a linear SVM classifier was trained but this time given access only to the amino acid concentrations of two amino acid types (K and C). As in the case of 3 and 4 amino acid types, 100% sensitivity and 100% specificity was obtained as shown in Figure 67.

Machine learning is a robust approach in which a computer program is trained on a reference training set, and learns to classify correctly. The analytical solution method presented herein in which a hypothesis test is performed to identify a sample as a reference provided by a reference function involves no training or learning steps, and the fact that the sensitivity and specificity for the identification of multiple cancer types from blood plasma are comparable with the analytical method and machine learning (average sensitivity of 96.67% and specificity of 95%) speaks to the robustness of the ability to describe a proteome or subproteome of interest as a reference line, and perform an identification against this reference. Additionally, this method allows simultaneous highly accurate (within 2% error) determination of the concentration or amount of the cancer associated proteome or subproteome of interest.

Example 15: Identifying cancer stage and location from plasma

In this dataset, proteins within platelet poor plasma samples from patients with stage II and stage III colorectal cancer prior to clinical intervention were quantified using mass spectrometry. Patients were additionally grouped by the type of colorectal cancer (colon cancer, rectal cancer) and by the location of the cancer (left and right colon).The results are provided in Table 41.

Table 41: Amino acid amounts of colorectal cancer patients, grouped by cancer stage and location.

| Cancer | Stage | Location | Sample.ID | K nmol | C nmol | W nmol | Y nmol |
|---|---|---|---|---|---|---|---|
| Colorectal | 2 | Left Colon | PC1090 | 33.72 | 15.24 | 6.64 | 16.14 |
| Colorectal | 2 | Left Colon | PC1188 | 32.73 | 14.93 | 6.46 | 15.95 |
| Colorectal | 2 | Left Colon | PC1293 | 33.95 | 15.69 | 6.59 | 16.37 |
| Colorectal | 2 | Left Colon | PC1652 | 30.46 | 13.92 | 6.14 | 14.90 |
| Colorectal | 2 | Left Colon | PC1777 | 34.00 | 15.78 | 6.59 | 16.43 |
| Colorectal | 2 | Left Colon | PC1978 | 33.25 | 14.96 | 6.68 | 16.11 |
| Colorectal | 2 | Left Colon | PC2209 | 31.55 | 14.85 | 6.40 | 15.56 |
| Colorectal | 2 | Left Colon | PC2355 | 32.91 | 15.18 | 6.38 | 15.92 |
| Colorectal | 2 | Left Colon | PC707 | 32.47 | 15.23 | 6.32 | 15.77 |
| Colorectal | 2 | Left Colon | PC775 | 31.36 | 16.56 | 7.08 | 16.30 |
| Colorectal | 2 | Rectum | PC1057 | 32.66 | 16.78 | 7.21 | 16.75 |
| Colorectal | 2 | Rectum | PC1481 | 34.69 | 15.89 | 6.47 | 16.40 |
| Colorectal | 2 | Rectum | PC1700 | 33.28 | 16.11 | 6.25 | 16.02 |
| Colorectal | 2 | Rectum | PC2016 | 35.33 | 15.94 | 6.54 | 16.61 |
| Colorectal | 2 | Rectum | PC2045 | 34.55 | 15.78 | 6.59 | 16.47 |
| Colorectal | 2 | Rectum | PC2086 | 31.13 | 14.59 | 5.96 | 15.06 |
| Colorectal | 2 | Rectum | PC2218 | 33.59 | 15.02 | 6.27 | 15.84 |
| Colorectal | 2 | Rectum | PC2371 | 32.08 | 14.93 | 6.05 | 15.32 |
| Colorectal | 2 | Rectum | PC428 | 34.62 | 14.25 | 6.97 | 16.17 |
| Colorectal | 2 | Rectum | PC741 | 32.73 | 14.54 | 6.25 | 15.54 |
| Colorectal | 2 | Rectum | PC792 | 35.57 | 16.44 | 6.69 | 16.90 |
| Colorectal | 2 | Rectum | PC813 | 32.95 | 15.79 | 6.30 | 15.81 |
| Colorectal | 2 | Rectum | PC842 | 33.43 | 14.98 | 6.75 | 16.11 |

(continued)

| Cancer | Stage | Location | Sample.ID | K nmol | C nmol | W nmol | Y nmol |
|---|---|---|---|---|---|---|---|
| Colorectal | 2 | Right Colon | PC1074 | 33.21 | 15.20 | 6.48 | 15.98 |
| Colorectal | 2 | Right Colon | PC1197 | 31.27 | 14.75 | 6.01 | 15.11 |
| Colorectal | 2 | Right Colon | PC1256 | 32.01 | 14.37 | 6.36 | 15.52 |
| Colorectal | 2 | Right Colon | PC1346 | 31.91 | 14.59 | 6.02 | 15.17 |
| Colorectal | 2 | Right Colon | PC1385 | 34.26 | 15.51 | 6.70 | 16.46 |
| Colorectal | 2 | Right Colon | PC2067 | 32.48 | 14.78 | 6.33 | 15.76 |
| Colorectal | 2 | Right Colon | PC2234 | 32.88 | 15.14 | 6.41 | 15.75 |
| Colorectal | 2 | Right Colon | PC2313 | 33.95 | 15.38 | 6.33 | 16.04 |
| Colorectal | 2 | Right Colon | PC2978 | 35.15 | 17.18 | 7.06 | 17.25 |
| Colorectal | 2 | Right Colon | PC874 | 30.07 | 13.91 | 5.69 | 14.41 |
| Colorectal | 2 | Right Colon | PC977 | 31.97 | 14.95 | 6.25 | 15.49 |
| Colorectal | 3 | Left Colon | PC1189 | 34.84 | 16.26 | 7.22 | 16.94 |
| Colorectal | 3 | Left Colon | PC1222 | 33.17 | 15.69 | 6.56 | 16.05 |
| Colorectal | 3 | Left Colon | PC1336 | 33.08 | 15.13 | 6.53 | 15.93 |
| Colorectal | 3 | Left Colon | PC1437 | 32.17 | 15.09 | 6.27 | 15.55 |
| Colorectal | 3 | Left Colon | PC1452 | 32.94 | 15.00 | 6.45 | 15.78 |
| Colorectal | 3 | Left Colon | PC1521 | 34.63 | 15.36 | 6.67 | 16.41 |
| Colorectal | 3 | Left Colon | PC1605 | 32.98 | 15.40 | 6.49 | 15.82 |
| Colorectal | 3 | Left Colon | PC1805 | 35.29 | 16.62 | 6.99 | 17.13 |
| Colorectal | 3 | Left Colon | PC2095 | 33.56 | 15.57 | 6.50 | 16.20 |
| Colorectal | 3 | Left Colon | PC2112 | 33.86 | 15.21 | 6.50 | 16.03 |
| Colorectal | 3 | Left Colon | PC2366 | 32.05 | 14.67 | 6.31 | 15.37 |
| Colorectal | 3 | Left Colon | PC2519 | 32.80 | 15.14 | 6.40 | 15.83 |
| Colorectal | 3 | Left Colon | PC452 | 31.71 | 14.56 | 6.31 | 15.42 |
| Colorectal | 3 | Left Colon | PC458 | 32.30 | 15.10 | 6.43 | 15.68 |
| Colorectal | 3 | Left Colon | PC465 | 34.68 | 15.64 | 6.97 | 16.72 |
| Colorectal | 3 | Left Colon | PC639 | 32.58 | 15.40 | 6.40 | 15.69 |
| Colorectal | 3 | Rectum | PC1079 | 32.88 | 15.00 | 6.42 | 15.78 |
| Colorectal | 3 | Rectum | PC1096 | 36.35 | 16.23 | 6.85 | 17.02 |
| Colorectal | 3 | Rectum | PC1136 | 34.32 | 16.17 | 6.65 | 16.56 |
| Colorectal | 3 | Rectum | PC1506 | 35.82 | 17.67 | 6.86 | 17.17 |
| Colorectal | 3 | Rectum | PC1608 | 33.55 | 15.65 | 6.24 | 15.95 |
| Colorectal | 3 | Rectum | PC1703 | 33.19 | 16.46 | 6.61 | 16.19 |
| Colorectal | 3 | Rectum | PC1728 | 33.23 | 16.23 | 6.71 | 16.29 |
| Colorectal | 3 | Rectum | PC1800 | 33.97 | 15.69 | 6.22 | 15.95 |
| Colorectal | 3 | Rectum | PC1982 | 32.45 | 15.30 | 6.15 | 15.42 |
| Colorectal | 3 | Rectum | PC2030 | 33.40 | 15.35 | 6.49 | 16.06 |
| Colorectal | 3 | Rectum | PC2204 | 33.52 | 15.28 | 6.33 | 15.96 |
| Colorectal | 3 | Rectum | PC2208 | 33.40 | 15.37 | 6.17 | 15.75 |

(continued)

| Cancer | Stage | Location | Sample.ID | K nmol | C nmol | W nmol | Y nmol |
|---|---|---|---|---|---|---|---|
| Colorectal | 3 | Rectum | PC2337 | 33.29 | 16.08 | 6.31 | 16.04 |
| Colorectal | 3 | Rectum | PC565 | 34.13 | 15.88 | 6.39 | 16.41 |
| Colorectal | 3 | Rectum | PC596 | 33.01 | 15.48 | 6.54 | 15.93 |
| Colorectal | 3 | Rectum | PC711 | 33.96 | 15.43 | 6.48 | 16.04 |
| Colorectal | 3 | Rectum | PC796 | 34.31 | 16.56 | 6.59 | 16.43 |
| Colorectal | 3 | Right Colon | PC1081 | 33.46 | 16.69 | 7.17 | 16.94 |
| Colorectal | 3 | Right Colon | PC1084 | 31.81 | 14.80 | 6.41 | 15.50 |
| Colorectal | 3 | Right Colon | PC1247 | 30.16 | 14.43 | 5.90 | 14.71 |
| Colorectal | 3 | Right Colon | PC1595 | 31.19 | 14.11 | 6.12 | 14.99 |
| Colorectal | 3 | Right Colon | PC1613 | 31.84 | 14.30 | 6.16 | 15.29 |
| Colorectal | 3 | Right Colon | PC1789 | 32.36 | 14.20 | 6.30 | 15.49 |
| Colorectal | 3 | Right Colon | PC1794 | 30.82 | 14.13 | 6.25 | 15.19 |
| Colorectal | 3 | Right Colon | PC2070 | 32.23 | 15.59 | 6.50 | 15.84 |
| Colorectal | 3 | Right Colon | PC2099 | 32.16 | 14.69 | 6.23 | 15.54 |
| Colorectal | 3 | Right Colon | PC2170 | 33.31 | 14.91 | 6.60 | 15.95 |
| Colorectal | 3 | Right Colon | PC2797 | 30.90 | 14.62 | 6.20 | 15.20 |
| Colorectal | 3 | Right Colon | PC490 | 33.45 | 15.50 | 6.57 | 16.19 |
| Colorectal | 3 | Right Colon | PC515 | 34.26 | 16.06 | 6.69 | 16.51 |
| Colorectal | 3 | Right Colon | PC556 | 32.90 | 16.02 | 6.78 | 16.41 |
| Colorectal | 3 | Right Colon | PC890 | 32.61 | 16.88 | 7.13 | 16.86 |
| Colorectal | 3 | Right Colon | PC962 | 30.96 | 14.68 | 6.02 | 15.07 |

The amino acid amounts in Table 41 provided by the vector functions were randomly portioned into two groups. The first group, containing 80% of the amino acid amounts, provided a reference which was used to train a machine learning classifier, and the performance of the machine learning classifier on identification of the other 20% of the amino acid amounts held back for validation as one of the references was assessed.

First, the amino acid amounts were used to train an ensemble subspace KNN classifier to identify the stage of colorectal cancer from blood plasma. The classifier used a nearest neighbor learner type with 30 learners, a subspace dimension of 2, and a default cost matrix. 78% of the time, stage III colorectal cancer was correctly identified as specifically stage III (see Figure 68).

Additionally, the amino acid amounts were used to train a linear support vector machine (SVM) classifier to identify the location of colorectal cancer from blood plasma. The classifier used data standardization, an automatic kernel function, one-vs-one multiclass method, 1 box constraint level, and default cost matrix. As demonstrated in Figures 68 and 69, where a patient's colorectal cancer was present in the left colon, the classifier correctly identified this based on the patient's amino acid amounts and the reference which defined the amino acid amounts of the left colon. Similarly, when a patient's colorectal cancer was present in the right colon, the classifier correctly identified this based on the patient's amino acid amounts and the reference which defined the amino acid amounts of the right colon. Finally, when the classifier identified a patient's amino acid amounts as corresponding to the reference for the rectum colorectal cancer location, this identification was correct 100% of the time. These results are summarized in a true positive rates, false negative rates confusion matrix and in a positive predictive values, false discovery rates confusion matrix (see Figures 68 and 69).

Example 16: Identifying multiple types of cancer from urine

The PXD008407 dataset was downloaded from Proteome Xchange at http://proteomecentral.proteomex change.org/cgi/GetDataset?ID=PXD008407 . In the PXD008407 dataset, first morning urine samples were collected from patients prior to clinical intervention and the proteins within each sample were quantified using mass spectrometry.

The authors of the PXD008407 dataset had quantified the total protein by mass of each plasma sample using the BCA assay. In this case, the concentrations were standardized such that each protein sample had a total mass concentration of 200 μg/mL. Applying the approach described previously for the colorectal cancer dataset, the mass concentration of each individual protein within each sample was then calculated by multiplying the $\frac{int}{\Sigma int}$ by 200 μg/mL. Then, the molar concentration of each individual protein within each sample was calculated by multiplying the mass concentration of each individual protein within each sample by $\frac{1000}{molecular\ weight\ (\frac{g}{mol})}$, where the molecular weight of each protein was downloaded form Uniport. The molar concentrations of each individual protein within each sample were summed to provide the total molar concentration of each individual protein within each sample and these values were reported in Table 42. The total molar concentration of all proteins within each sample provides q in Equation 11. $q_i/q$ in Equation 11 was then calculated by dividing the molar concentration of each protein within each sample by the total molar concentration of all proteins within each sample. Then, Equation 11 was used as disclosed herein to calculate the $w_K$, $w_W$, $w_Y$, and $w_c$ values for each patient plasma sample. The $w_K$, $w_W$, $w_Y$, and $w_c$ were then each multiplied by the value of q for each sample, the total molar concentration of all proteins within each sample, to calculate the concentration of amino acids of the K, C, W, and Y amino acid types within each sample. The results are provided in Table 42, and graphed in four dimensional space in Figure 70. Each type of cancer has a clear distribution defined by its reference when labelled amino acid concentrations are plotted in 4 dimensional space.

Table 42: Amino acid concentrations for three cancer types measured in urine

| Sample.ID | Subproteome | $w_K$ | $w_C$ | $w_W$ | $w_Y$ | Total protein concentration (μM) | [K (μM)] | [C (μM)] | [W (μM)] | [Y (μM)] |
|---|---|---|---|---|---|---|---|---|---|---|
| Bladder_16_1 | Bladder cancer | 22.6 2 | 8.34 | 3.8 2 | 9.65 | 5.51 | 124.67 | 45.96 | 21.05 | 53.22 |
| Bladder_16_2 | Bladder cancer | 22.3 5 | 8.18 | 3.6 2 | 9.30 | 5.66 | 126.45 | 46.27 | 20.46 | 52.62 |
| Bladder_4_3 | Bladder cancer | 18.7 4 | 8.79 | 4.0 0 | 9.54 | 6.01 | 112.62 | 52.86 | 24.04 | 57.34 |
| Bladder_4_4 | Bladder cancer | 18.3 9 | 8.39 | 3.8 4 | 9.27 | 6.14 | 113.00 | 51.58 | 23.57 | 56.95 |
| Bladder_40_1 | Bladder cancer | 22.2 1 | 10.8 2 | 3.8 0 | 10.3 5 | 5.50 | 122.24 | 59.54 | 20.91 | 56.98 |
| Bladder_40_2 | Bladder cancer | 22.4 8 | 10.8 7 | 3.8 3 | 10.4 8 | 5.42 | 121.90 | 58.96 | 20.78 | 56.85 |
| Bladder_51_1 | Bladder cancer | 21.2 7 | 10.4 5 | 3.8 4 | 10.1 5 | 5.65 | 120.17 | 59.04 | 21.70 | 57.34 |
| Bladder_51_2 | Bladder cancer | 21.9 1 | 10.8 5 | 3.9 7 | 10.4 7 | 5.50 | 120.56 | 59.72 | 21.84 | 57.59 |
| Bladder_66_1 | Bladder cancer | 20.9 0 | 10.2 9 | 3.6 7 | 9.86 | 5.75 | 120.09 | 59.10 | 21.11 | 56.66 |
| Bladder_66_2 | Bladder cancer | 20.6 1 | 9.97 | 3.6 1 | 9.71 | 5.85 | 120.56 | 58.33 | 21.10 | 56.79 |
| Prostate_115_ 1 | Prostate cancer | 20.8 8 | 10.4 0 | 4.0 0 | 10.4 0 | 5.63 | 117.64 | 58.58 | 22.51 | 58.60 |
| Prostate_115_ 2 | Prostate cancer | 20.6 4 | 10.2 6 | 3.9 7 | 10.2 9 | 5.72 | 118.06 | 58.71 | 22.72 | 58.88 |
| Prostate_137_ 3 | Prostate cancer | 15.1 0 | 7.56 | 3.2 0 | 7.97 | 7.16 | 108.02 | 54.11 | 22.93 | 57.04 |
| Prostate_137_ 4 | Prostate cancer | 14.4 3 | 7.25 | 3.2 1 | 7.77 | 7.32 | 105.68 | 53.07 | 23.49 | 56.92 |
| Prostate_30_1 | Prostate cancer | 26.1 6 | 13.7 2 | 4.7 5 | 12.4 3 | 4.76 | 124.68 | 65.38 | 22.62 | 59.25 |
| Prostate_30_2 | Prostate cancer | 25.7 6 | 13.4 3 | 4.7 3 | 12.2 9 | 4.85 | 124.99 | 65.17 | 22.93 | 59.63 |
| Prostate_32_3 | Prostate cancer | 18.1 7 | 9.20 | 4.1 4 | 9.64 | 5.89 | 106.99 | 54.19 | 24.36 | 56.78 |
| Prostate_32_4 | Prostate cancer | 17.9 1 | 8.97 | 4.0 6 | 9.48 | 5.98 | 107.18 | 53.67 | 24.27 | 56.72 |
| Prostate_52_3 | Prostate cancer | 16.2 4 | 8.07 | 3.7 9 | 8.70 | 6.48 | 105.27 | 52.30 | 24.58 | 56.38 |
| Prostate_52_4 | Prostate cancer | 15.9 4 | 8.07 | 3.7 6 | 8.53 | 6.58 | 104.84 | 53.09 | 24.73 | 56.12 |
| Prostate_55_1 | Prostate cancer | 19.3 0 | 9.51 | 3.7 3 | 9.46 | 6.14 | 118.42 | 58.37 | 22.89 | 58.05 |
| Prostate_55_2 | Prostate cancer | 19.5 1 | 9.66 | 3.7 7 | 9.55 | 6.08 | 118.67 | 58.78 | 22.93 | 58.12 |
| Prostate_65_1 | Prostate cancer | 22.1 1 | 10.8 0 | 3.9 8 | 10.3 5 | 5.61 | 124.04 | 60.57 | 22.30 | 58.07 |
| Prostate_65_2 | Prostate cancer | 22.1 7 | 10.8 1 | 3.9 7 | 10.3 9 | 5.57 | 123.59 | 60.25 | 22.11 | 57.93 |
| Prostate_67_3 | Prostate cancer | 19.1 3 | 10.2 3 | 4.2 2 | 9.88 | 5.65 | 107.99 | 57.76 | 23.83 | 55.77 |
| Prostate_67_4 | Prostate cancer | 19.4 2 | 10.4 6 | 4.3 0 | 10.0 5 | 5.56 | 107.91 | 58.12 | 23.86 | 55.82 |

(continued)

| Sample.ID | Subproteome | $w_K$ | $w_C$ | $w_W$ | $w_Y$ | Total protein concentration (μM) | [K (μM)] | [C (μM)] | [W (μM)] | [Y (μM)] |
|---|---|---|---|---|---|---|---|---|---|---|
| Prostate_88_1 | Prostate cancer | 13.8 9 | 6.26 | 3.1 2 | 7.71 | 7.71 | 107.06 | 48.29 | 24.06 | 59.45 |
| Prostate_88_2 | Prostate cancer | 13.8 7 | 6.18 | 3.0 8 | 7.59 | 7.82 | 108.53 | 48.35 | 24.06 | 59.35 |
| Prostate_95_1 | Prostate cancer | 23.2 1 | 11.3 7 | 4.4 3 | 11.4 1 | 5.28 | 122.54 | 60.03 | 23.38 | 60.25 |
| Prostate_95_2 | Prostate cancer | 23.4 9 | 11.6 3 | 4.5 3 | 11.6 8 | 5.17 | 121.49 | 60.16 | 23.40 | 60.38 |
| Renal_1_1 | Renal cancer | 16.6 8 | 8.46 | 3.9 5 | 8.97 | 6.24 | 104.10 | 52.78 | 24.63 | 55.96 |
| Renal_1_2 | Renal cancer | 16.5 3 | 8.38 | 3.8 5 | 8.80 | 6.32 | 104.50 | 52.99 | 24.36 | 55.65 |
| Renal_14_3 | Renal cancer | 20.0 1 | 10.4 8 | 4.1 7 | 10.1 4 | 5.50 | 110.08 | 57.64 | 22.92 | 55.76 |
| Renal_14_4 | Renal cancer | 20.2 4 | 10.9 5 | 4.2 5 | 10.3 0 | 5.39 | 109.15 | 59.05 | 22.91 | 55.52 |
| Renal_18_1 | Renal cancer | 20.0 1 | 10.0 1 | 4.0 3 | 10.0 6 | 5.59 | 111.90 | 55.96 | 22.51 | 56.27 |
| Renal_18_2 | Renal cancer | 18.9 9 | 9.77 | 3.9 1 | 9.64 | 5.85 | 111.07 | 57.17 | 22.88 | 56.39 |
| Renal_7_1 | Renal cancer | 17.1 0 | 8.66 | 3.4 0 | 8.76 | 6.72 | 114.84 | 58.16 | 22.82 | 58.80 |
| Renal_7_2 | Renal cancer | 16.8 4 | 8.40 | 3.3 4 | 8.57 | 6.90 | 116.27 | 57.99 | 23.05 | 59.14 |
| Renal_8_1 | Renal cancer | 18.6 1 | 9.83 | 4.0 3 | 9.76 | 5.82 | 108.28 | 57.23 | 23.48 | 56.81 |
| Renal_8_2 | Renal cancer | 18.8 1 | 9.93 | 4.0 6 | 9.87 | 5.78 | 108.68 | 57.39 | 23.48 | 57.02 |

Applying vector function 2 on this dataset as disclosed herein, the vector functions providing the amino acid concentration of K, C, W and Y amino acid types at any total molar protein concentration of the bladder cancer, prostate cancer, and renal cancer proteomes in urine were calculated to be:

$$p_{Bladder\ Cancer} = \langle 21.15t, 9.70t, 3.80t, 9.88t \rangle$$

$$p_{Prostate\ Cancer} = \langle 19.37t, 9.69t, 3.94t, 9.78t \rangle$$

$$p_{Renal\ Cancer} = \langle 18.38t, 9.49t, 3.90t, 9.49t \rangle$$

Finally, to identify the presence of bladder cancer, prostate cancer, and renal cancer proteomes of interest within urine, a cubic support vector machine (SVM) classifier was trained on 80% of the data points which provided the reference while the trained dataset was tested on the remaining randomly selected 20% of the datapoints which were held back. The cubic SVM used data standardization, an automatic kernel scale with one-vs-one multiclass method, box constraint level 1, and a default cost matrix.

As shown in Figure 71, 100 % correct identification and 0% incorrect identification was achieved, providing 100% true positive rate and 0% false negative rate for the identification of bladder cancer, prostate cancer, and renal cancer from urine samples using the methods disclosed herein.

The methods of the invention can detect the presence and absence of cancer, as well as other diseases of interest, from multiple body fluids.

## Claims

**1.** A method of identifying the presence and/or concentration and/or amount of one or more proteins, peptides, oligopeptides, polypeptides, protein complexes, subproteomes, or proteomes of interest within a sample, the method comprising:

a) labelling two or more amino acid types within the sample, wherein an amino acid type is defined by the R-group of the amino acid;
b) measuring the label of each labelled amino acid type in the sample;
c) optionally calculating the amino acid concentration of each labelled amino acid type from the measured label;
d) optionally calculating the number of amino acids of each labelled amino acid type; and
e) identifying the presence and/or concentration and/or amount of one or more proteins, peptides, oligopeptides, polypeptides, protein complexes, subproteomes, or proteomes of interest in the sample by comparing the measured label and/or amino acid concentration of each labelled amino acid type in the sample to the known label values and/or amino acid concentrations of the same two or more amino acid types that have been labelled in the sample of each of the one or more proteins, peptides, oligopeptides, polypeptides, protein complexes, subproteomes, or proteomes of interest at one or more concentrations, or comparing the number of amino acids of each labelled amino acid type in the sample to the known number of amino acids of the same two or more amino acid types that have been labelled in the sample in the one or more proteins, peptides, oligopeptides, polypeptides, protein complexes, subproteomes, or proteomes of interest;

wherein the one or more peptides, oligopeptides, polypeptides, proteins, protein complexes, or peptides, oligopeptides, polypeptides, proteins, or protein complexes within subproteomes or proteomes are denatured during or prior to the labelling reaction of the amino acid types in the sample.

**2.** The method of claim 1, wherein one or more peptides, oligopeptides, polypeptides, proteins, protein complexes, or peptides, oligopeptides, polypeptides, proteins, or protein complexes within subproteomes or proteomes are denatured during or prior to the labelling reaction of the amino acid types in the sample using any combination of: an organic solvent, surfactant, reducing agent, or high or low pH conditions.

**3.** The method of any preceding claim, wherein one or more polypeptides contained within a sample are denatured by reducing polypeptide disulphide bonds, adding a surfactant, and optionally changing the buffer conditions to high or low pH; and/or
wherein one or more proteins contained within a sample are denatured by reducing protein disulphide bonds, adding a surfactant and optionally changing the buffer conditions to high or low pH.

4. The method of claim 2 or 3, wherein:

(i) the organic solvent is dimethyl sulfoxide, methanol, acetonitrile, ethanol, or isopropanol; and/or
(ii) high or low pH is pH 2, pH 3, pH 4, pH5, pH 7.5, pH 8.5, pH 9, pH 10, or pH 10.5, preferably pH 10.5; and/or
(iii) the reducing agent is TCEP, β-mercapto ethanol, DTBA, or OTT, preferably TCEP or β-mercapto ethanol; and/or
(iv) the surfactant is one or more of sodium dodecyl sulfate (SDS), dodecyltrimethylammonium bromide (DTAB), cetyltrimethylammonium bromide (CTAB), phosphatidylcholine, Triton X-100, Triton X-114, CHAPS, NP-40, sodium 1-undecanesulfonate (SUS) sodium dodecylbenzenesulfonate (SOBS), sodium deoxycholate (DOC), sodium stearate, 4-(5-dodecyl)benzenesulfonate, dioctyl sodium sulfosuccinate, alkyl ether phosphates, benzalkaonium chloride (BAC), and perfluorooctanesulfonate (PFOS), preferably SDS.

5. The method of any preceding claim, wherein one or more proteins, peptides, oligopeptides, polypeptides, and/or protein complexes which comprise a subproteome or a proteome contained within a sample are denatured by reducing the protein, peptide, oligopeptide, polypeptide, and/or protein complex disulphide bonds with TCEP and adding the surfactant SDS,
optionally wherein one or more proteins contained within a sample are denatured by reducing protein disulphide bonds with TCEP and adding the surfactant SDS.

6. The method of any preceding claim, wherein the labelling reactions are performed at pH 10.5 in the presence of 4% w/v SDS and 10 mM TCEP, or wherein the labelling reactions are performed at pH 10 in the presence of 4% w/v SDS and 18 mM β-mercaptoethanol.

7. The method of any preceding claim, wherein both the modified and the unmodified amino acids of an amino acid type are labelled,

optionally wherein both the modified and the unmodified amino acids of the amino acid type cysteine (C) are labelled,
further optionally wherein the modified amino acids of Cysteine are disulphide bonded cysteine ($C_D$) amino acids.

8. The method of any preceding claim, wherein the total cysteine amino acid type (C) is labelled, including both reduced ($C_R$) and disulphide bonded ($C_D$) amino acids of the cysteine (C) amino acid type,

optionally wherein prior to labelling all amino acids of the cysteine (C) amino acid type, the disulphide bonded subset of the cysteine amino acid type (Co) is reduced to form a reduced subset of the cysteine amino acid type ($C_R$) using a reducing agent, optionally wherein the reducing agent is TCEP,
further optionally wherein the combination of Co and $C_R$ are fluorogenically labelled with ABD-F after reduction with TCEP, and denaturation with sodium dodecyl sulfate (SDS) in a buffer.

9. The method of claim 8, wherein the sample is separated into two fractions, optionally wherein $C_R$ is labelled in one fraction and the total cysteine amino acid (C) which is a combination of Co and $C_R$ is labelled in the second fraction.

10. The method of any preceding claim, wherein step (a) comprises labelling three or more amino acid types within a sample.

11. The method of any preceding claim, wherein step (a) comprises labelling four or more amino acid types within a sample.

12. The method of any preceding claim, wherein the two, three, or four or more amino acid types are selected from the group consisting of: tryptophan (W), cysteine (C), tyrosine (Y) and/or lysine (K) and any combination thereof.

13. The method of any preceding claim, wherein step (a) comprises labelling four amino acid types, preferably wherein the four amino acid types are tryptophan (W), tyrosine (Y), lysine (K) and cysteine (C).

14. The method of any preceding claim, wherein step (a) comprises labelling four amino acid types and the four amino acid types are Cysteine (C), Tyrosine (Y) and Lysine (K) and Tryptophan (W), wherein both unmodified Cysteine ($C_R$) amino acids, and the modified and unmodified Cysteine amino acids (C) are labelled.

**15.** The method of any preceding claim, wherein the method is a method of detecting cancer, and wherein step e) comprises identifying the presence and/or concentration and/or amount of one or more proteins, peptides, oligopeptides, polypeptides, protein complexes, subproteomes, or proteomes of interest related to cancer in the sample by comparing the measured label and/or amino acid concentration of each labelled amino acid type in the sample to the known label values and/or amino acid concentrations of the same two or more amino acid types that have been labelled in the sample of each of the one or more proteins, peptides, oligopeptides, polypeptides, protein complexes, subproteomes, or proteomes of interest related to cancer at one or more concentrations, and/or comparing the number of amino acids of each labelled amino acid type in the sample to the known number of amino acids of the same two or more amino acid types that have been labelled in the sample in the one or more proteins, peptides, oligopeptides, polypeptides, protein complexes, subproteomes, or proteomes of interest related to cancer.

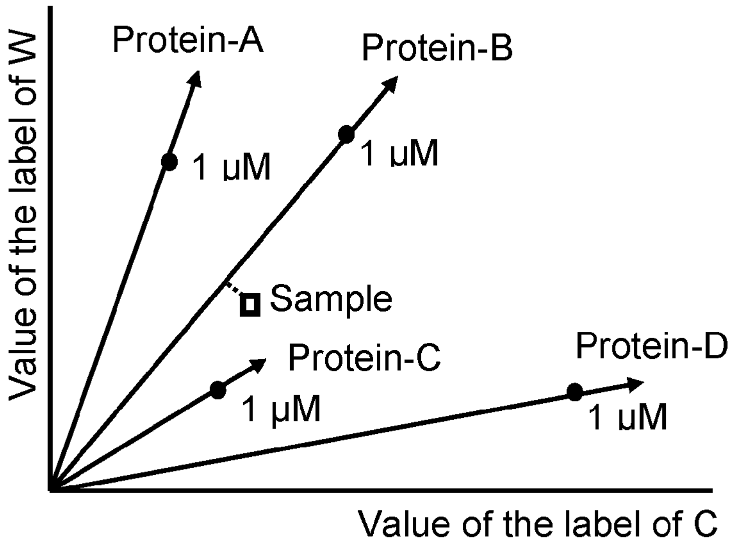


Figure 1

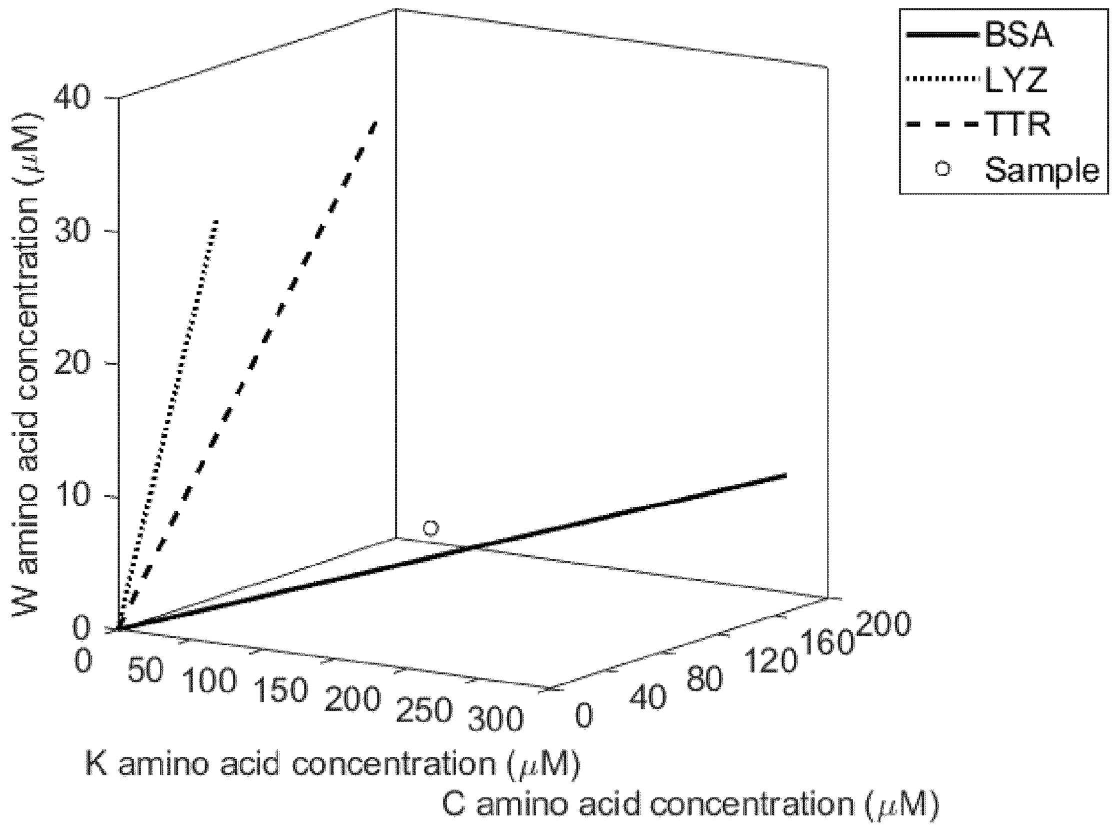


Figure 2

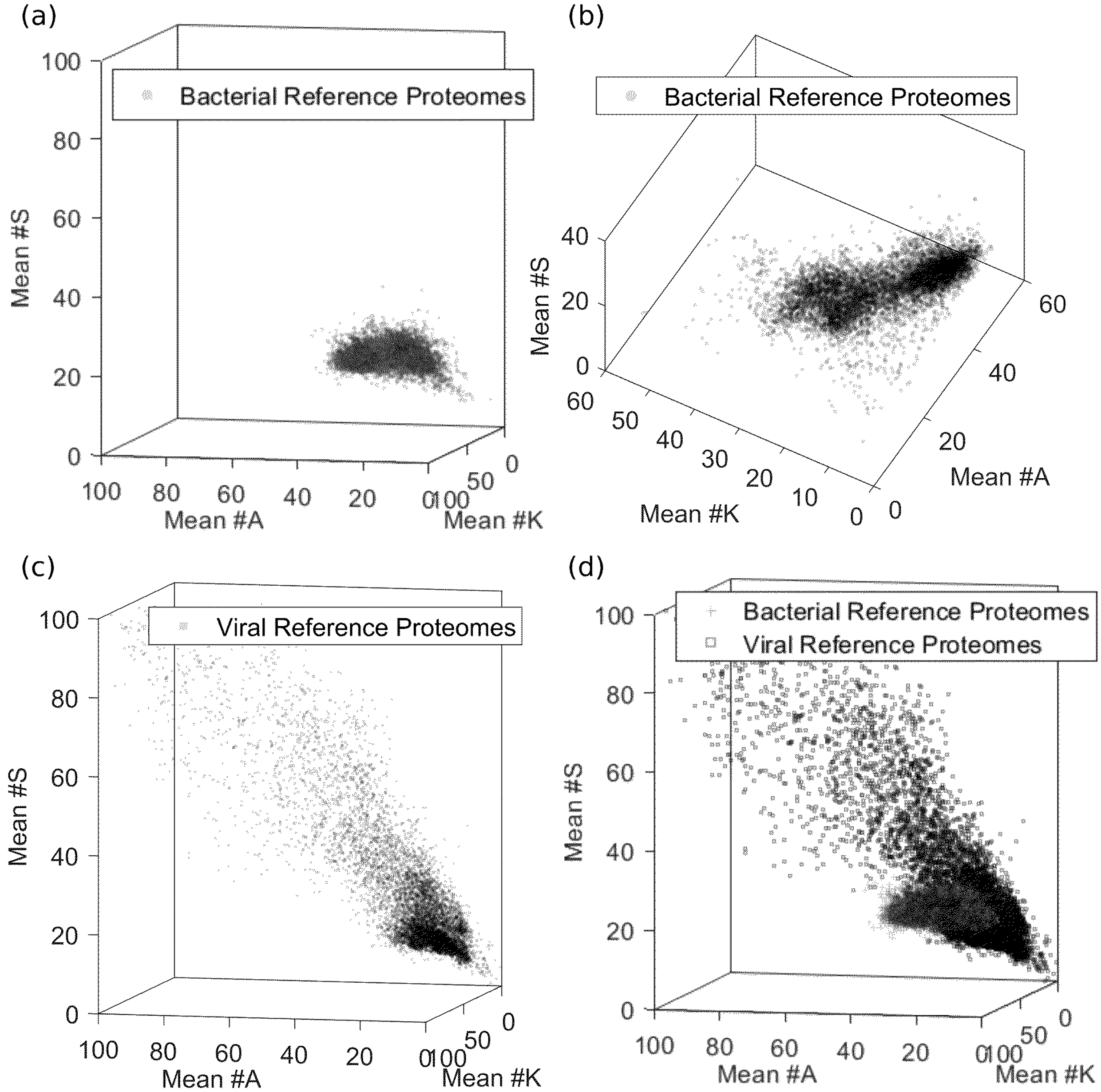
(a)
Bacterial Reference Proteomes
Mean #S
Mean #A
Mean #K
(b)
Bacterial Reference Proteomes
Mean #S
Mean #K
Mean #A
(c)
Viral Reference Proteomes
Mean #S
Mean #A
Mean #K
(d)
Bacterial Reference Proteomes
Viral Reference Proteomes
Mean #S
Mean #A
Mean #K

Figure 3

Benford's Law applied to number of amino acids of proteins within the Human Plasma Proteome

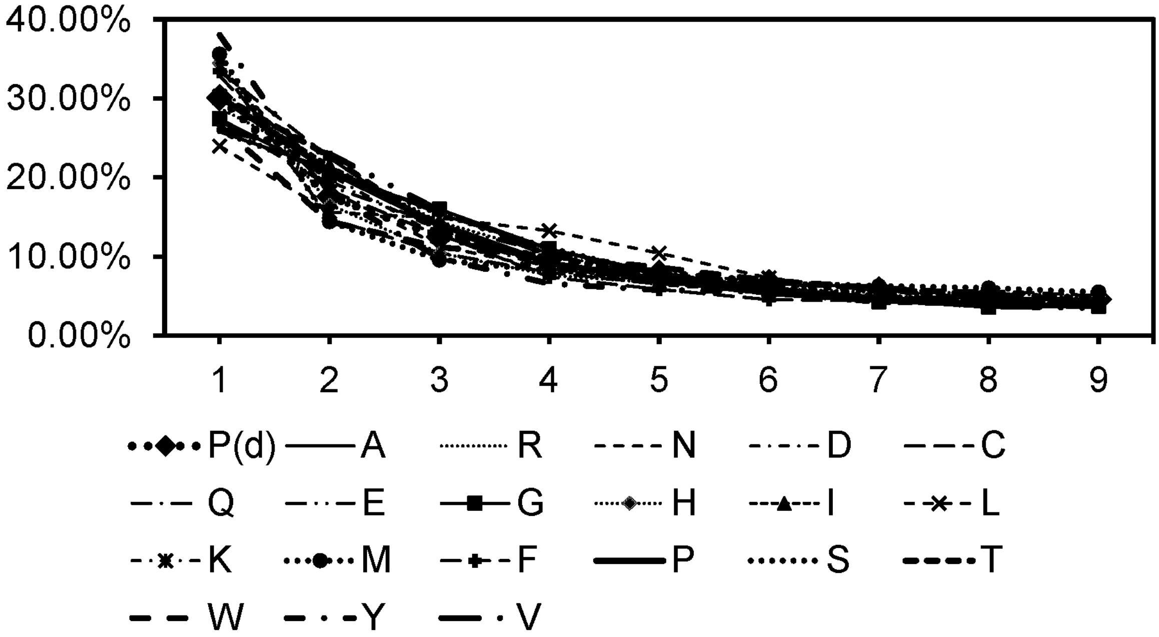


Figure 4

Benford's Law applied to number of amino acids of proteins within Viral Proteomes

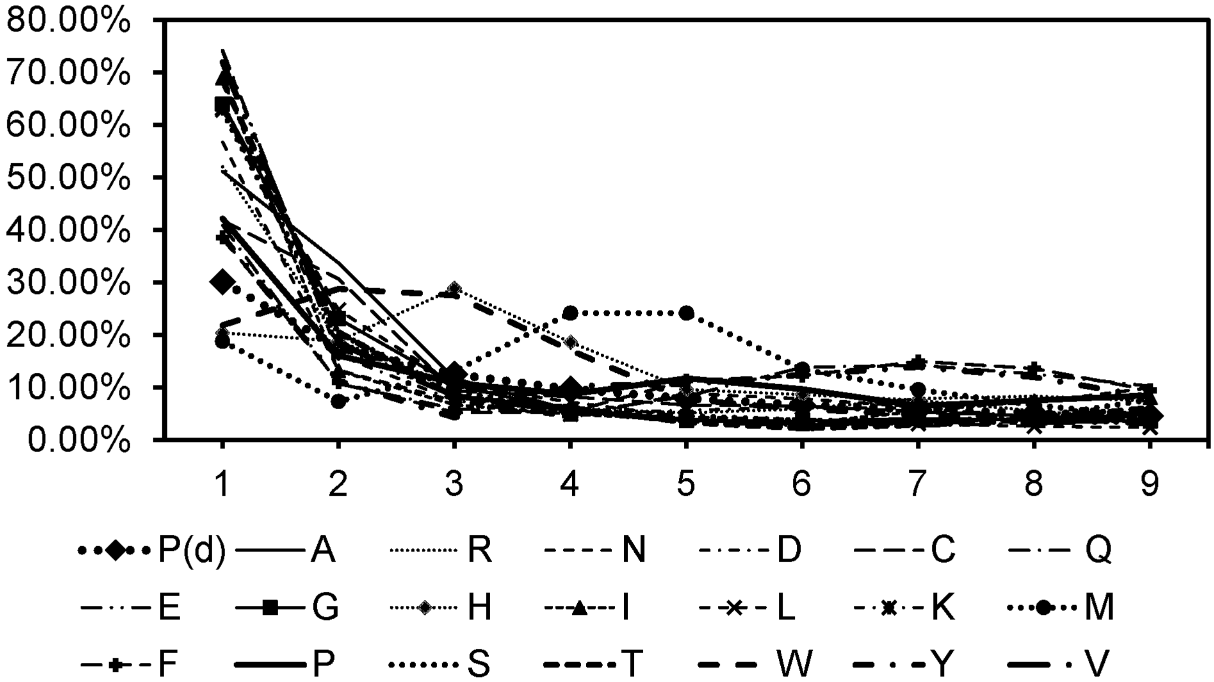


Figure 5

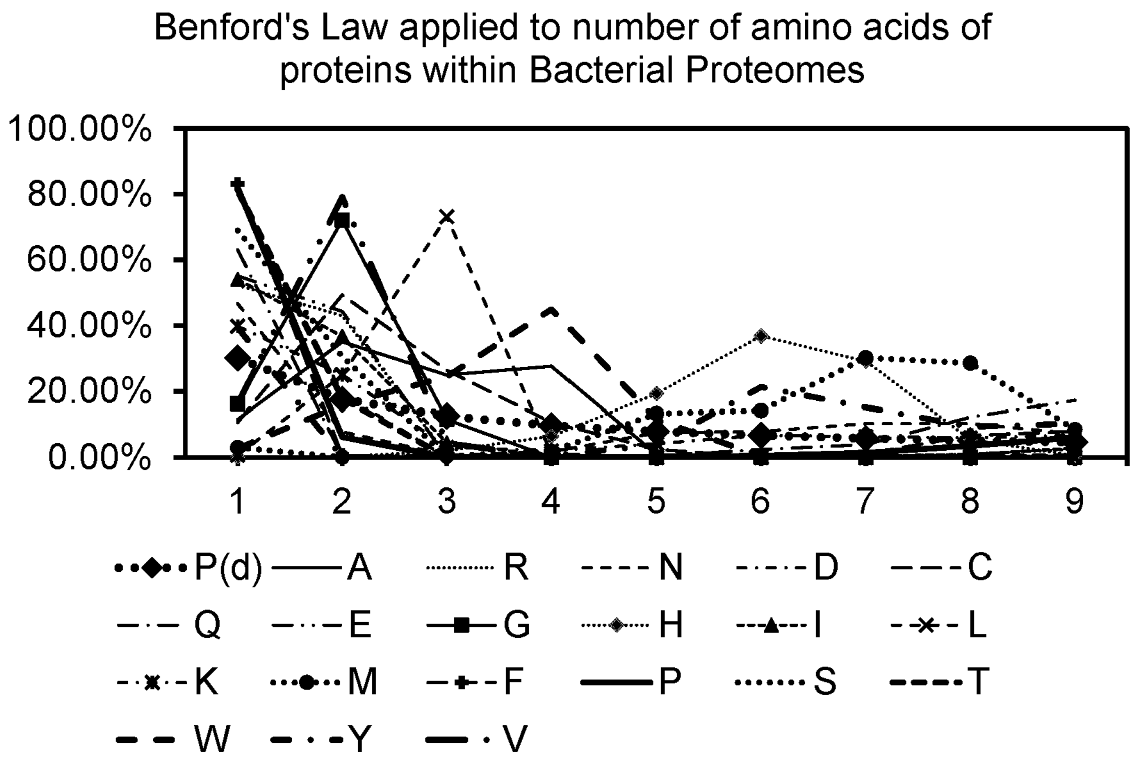


Figure 6

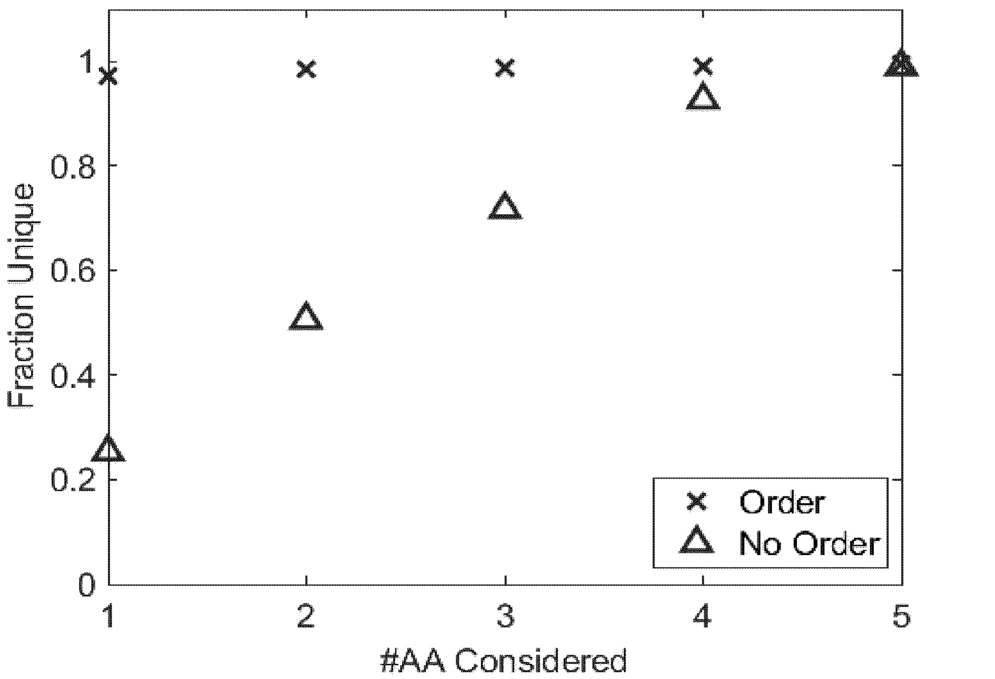


Figure 7

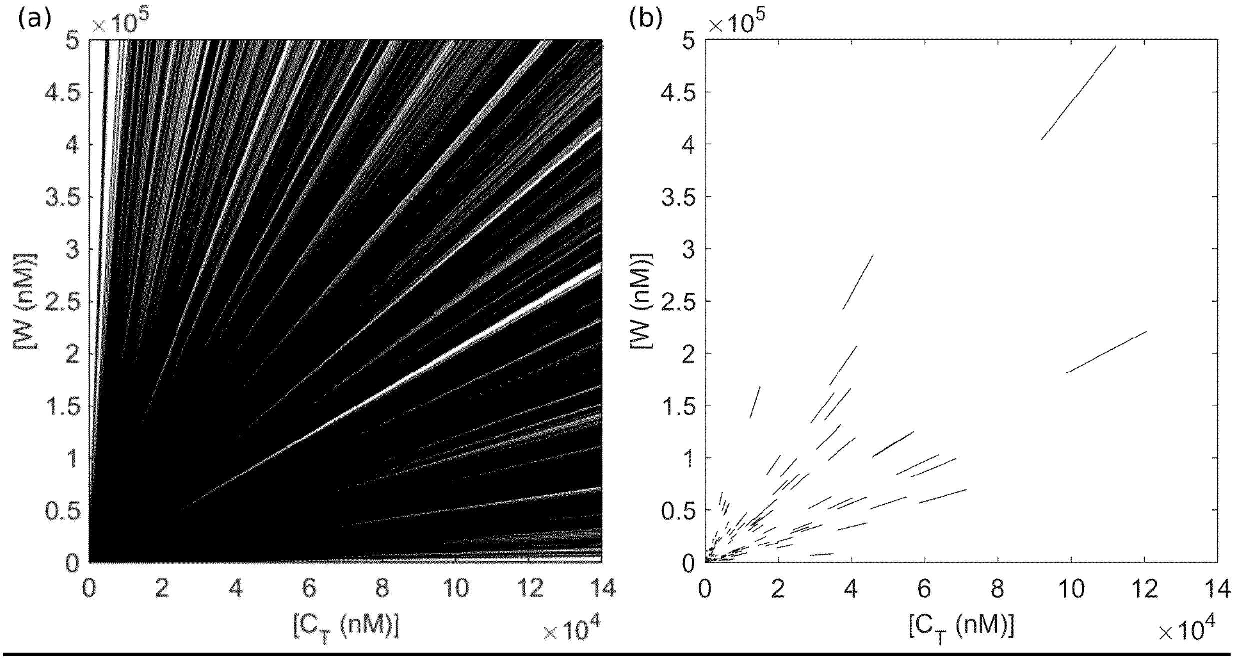


Figure 8

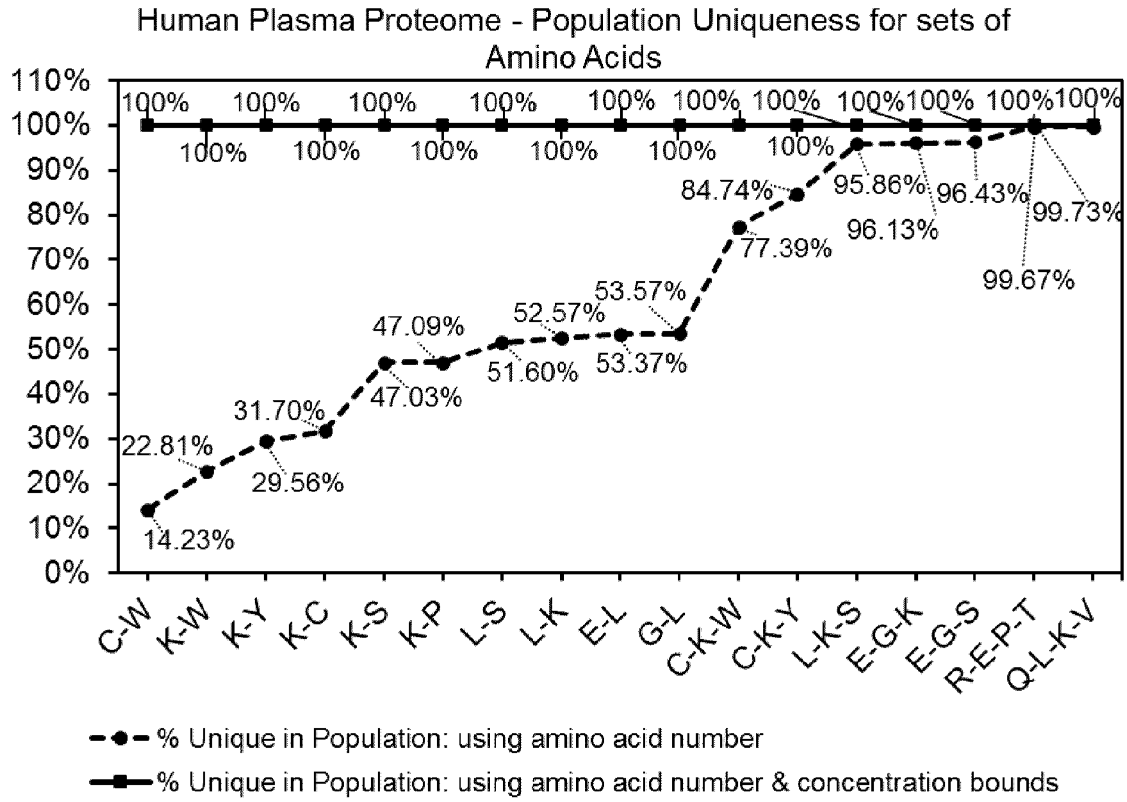


Figure 9

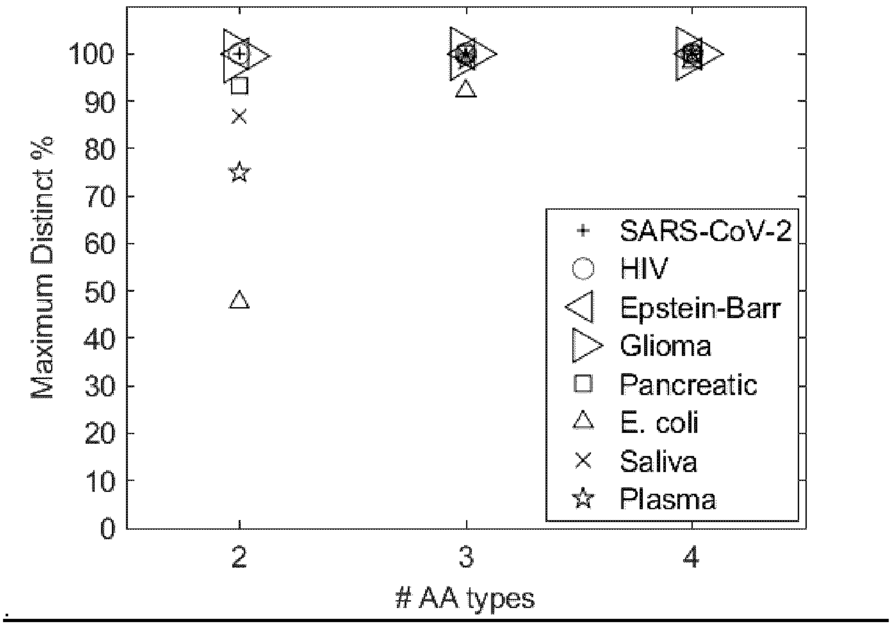
100
90
80
70
60
50
40
30
20
10
0
Maximum Distinct %
2
3
4
# AA types
SARS-CoV-2
HIV
Epstein-Barr
Glioma
Pancreatic
E. coli
Saliva
Plasma

Figure 10

(a)

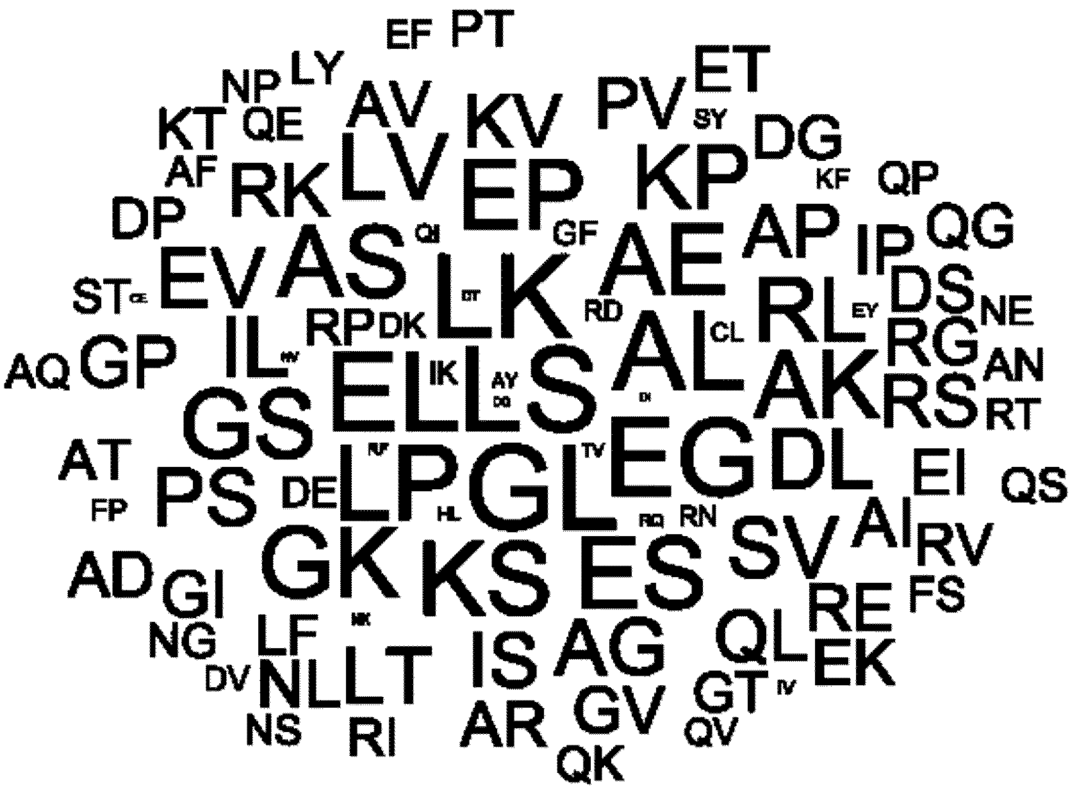
EF PT
NP LY
ET
AV KV PV SY
KT QE DG
AF RK LV EP KP KF QP
DP GF AP IP QG
ST EV AS LK AE RL EY DS NE
RP DK RD CL RG
AQ GP IL EL LS AL AK AN
IK AY DQ
GS RS RT
AT TV EG DL EI QS
PS DE LP GL
FP HL RQ RN SV AI RV
AD GI GK KS ES RE FS
NG LF QL
IS AG EK
DV NL LT GT IV
NS RI AR GV QV
QK

(b)

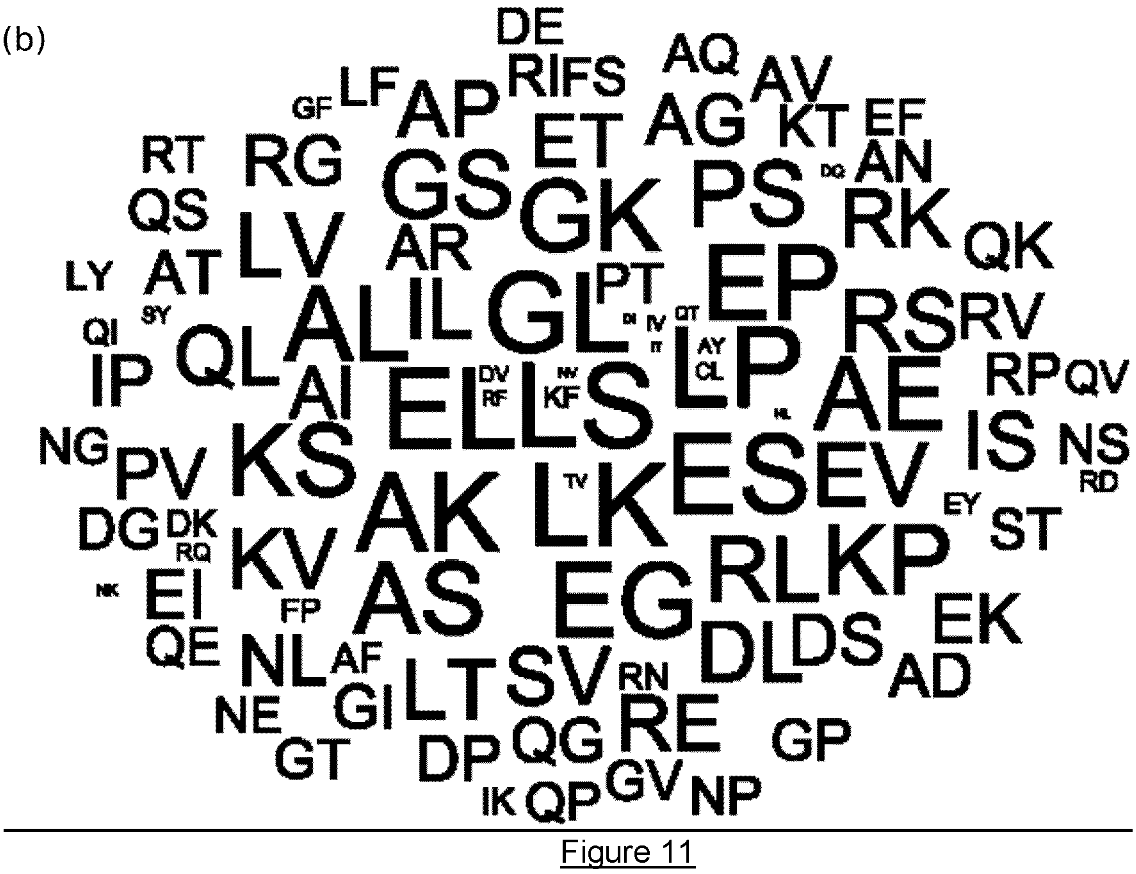
DE
RIFS AQ AV
GF LF AP AG KT EF
ET
RT RG GS PS DQ AN
QS GK RK
LV AR QK
LY AT EP
PT
AL IL GL RS RV
QI SY
IP QL LP
AI EL LS AE RP QV
NG PV KS ES EV IS NS
RD
AK LK EY ST
DG DK RQ KV RL KP
EI AS EG
FP
QE NL AF DL DS EK AD
LT SV RN
NE GI RE GP
GT DP QG
IK QP GV NP

Figure 11

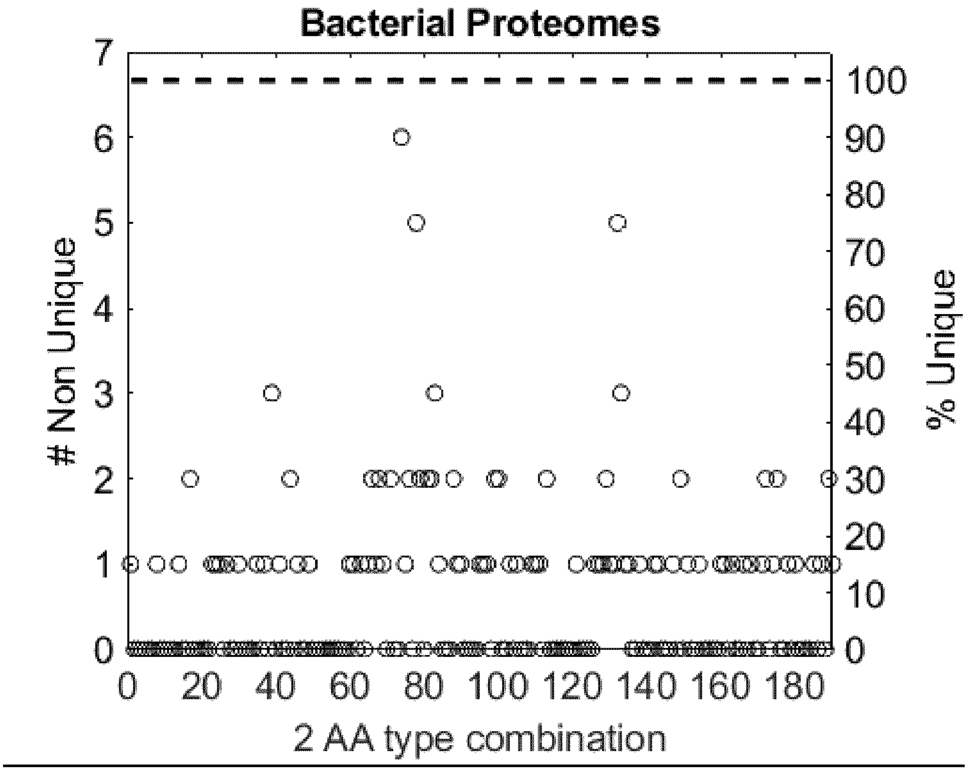


Figure 12

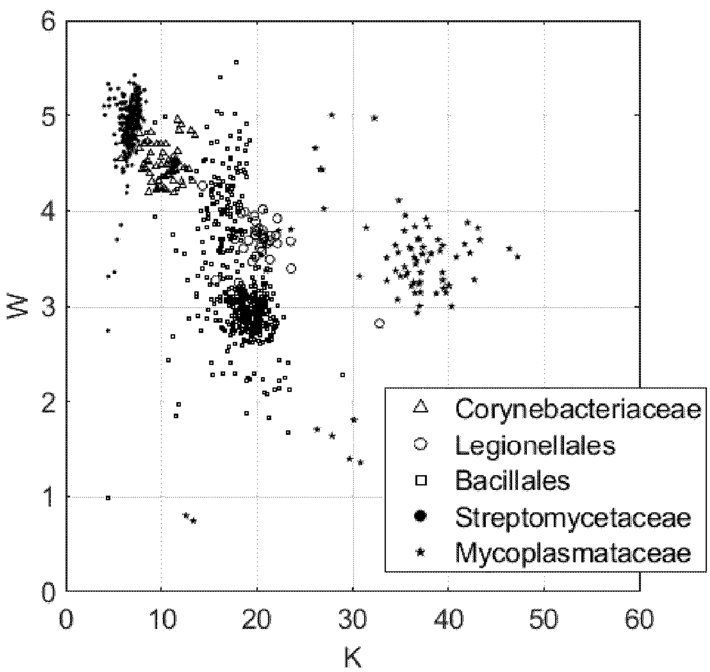


Figure 13

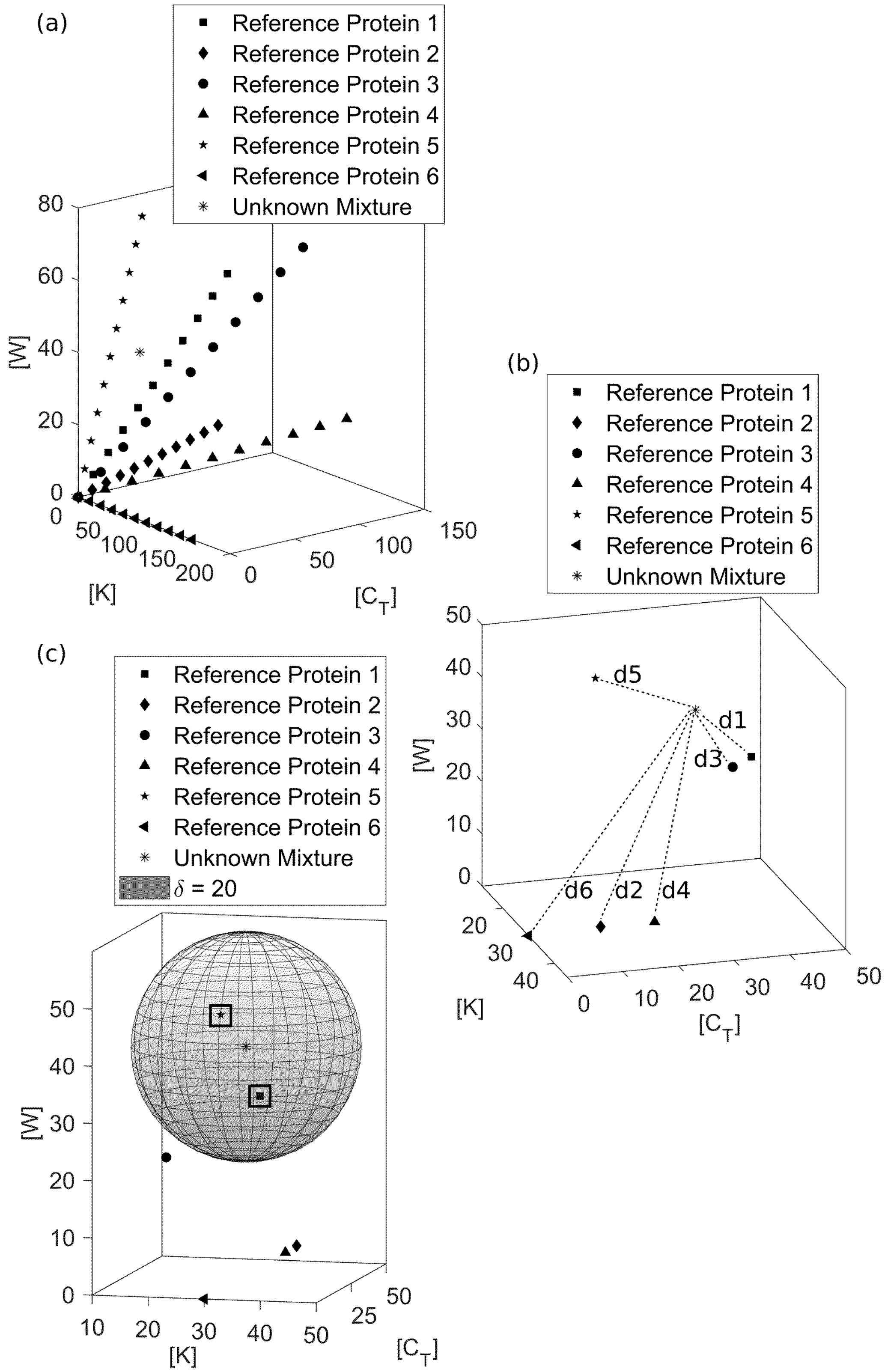
(a)
Reference Protein 1
Reference Protein 2
Reference Protein 3
Reference Protein 4
Reference Protein 5
Reference Protein 6
Unknown Mixture
[W]
[K]
[C_T]
(b)
Reference Protein 1
Reference Protein 2
Reference Protein 3
Reference Protein 4
Reference Protein 5
Reference Protein 6
Unknown Mixture
d5
d1
d3
d6
d2
d4
[W]
[K]
[C_T]
(c)
Reference Protein 1
Reference Protein 2
Reference Protein 3
Reference Protein 4
Reference Protein 5
Reference Protein 6
Unknown Mixture
δ = 20
[W]
[K]
[C_T]

Figure 14

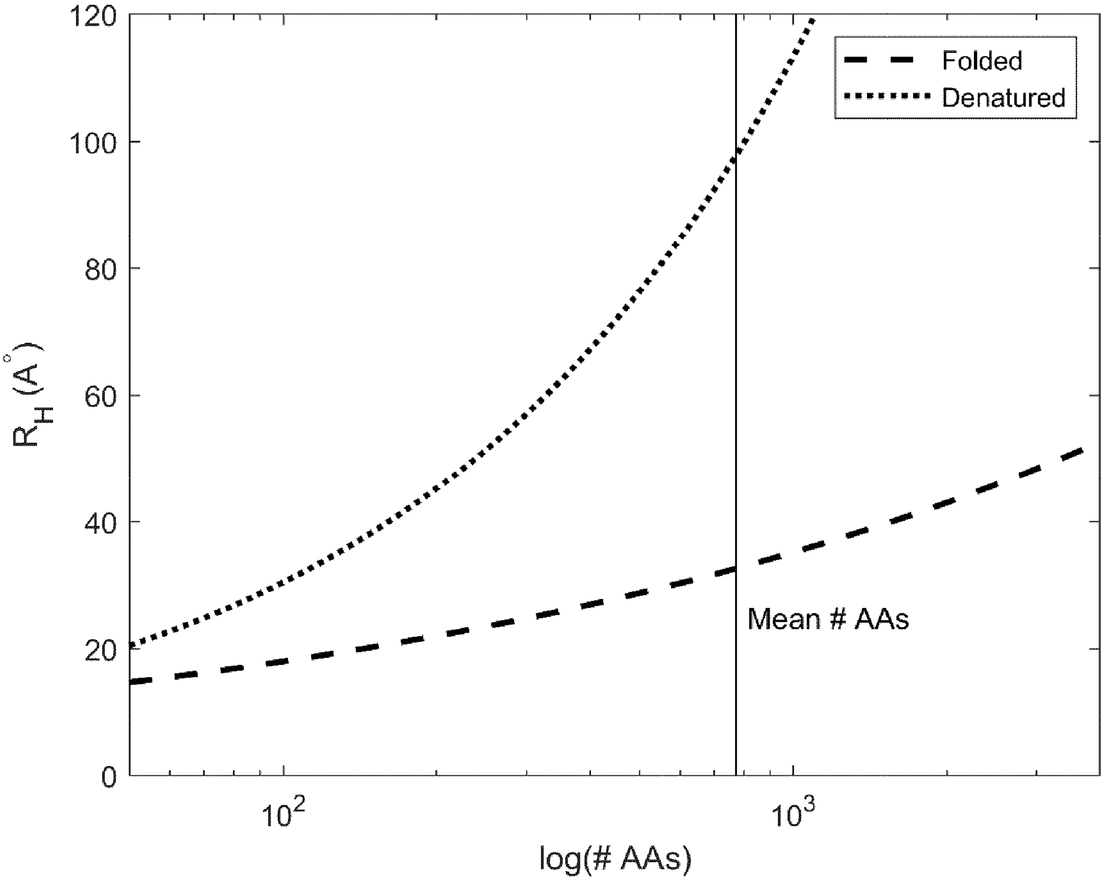
120
100
80
60
40
20
0
$R_H$ (Å)
Folded
Denatured
Mean # AAs
$10^2$
$10^3$
log(# AAs)

Figure 15

(a) Tryptophan

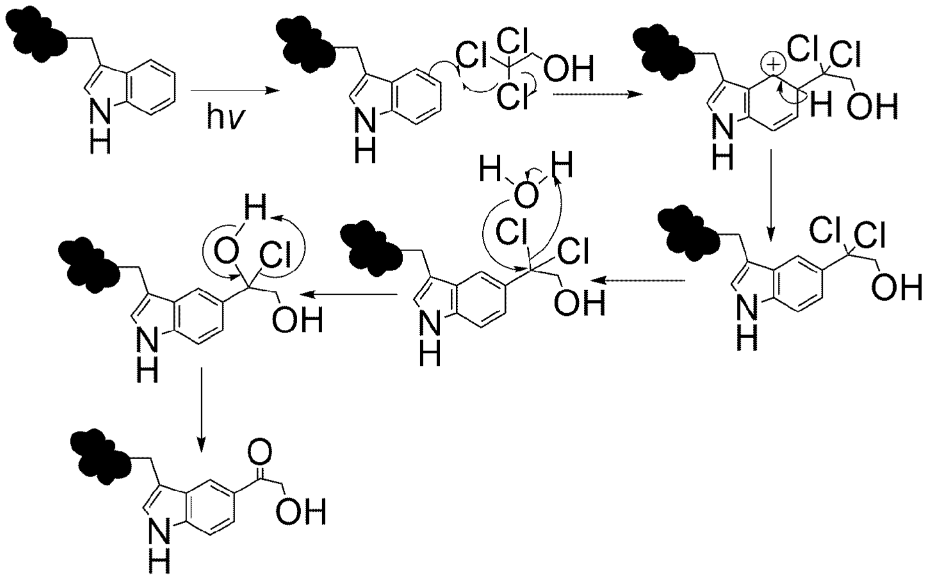


(b) Tyrosine

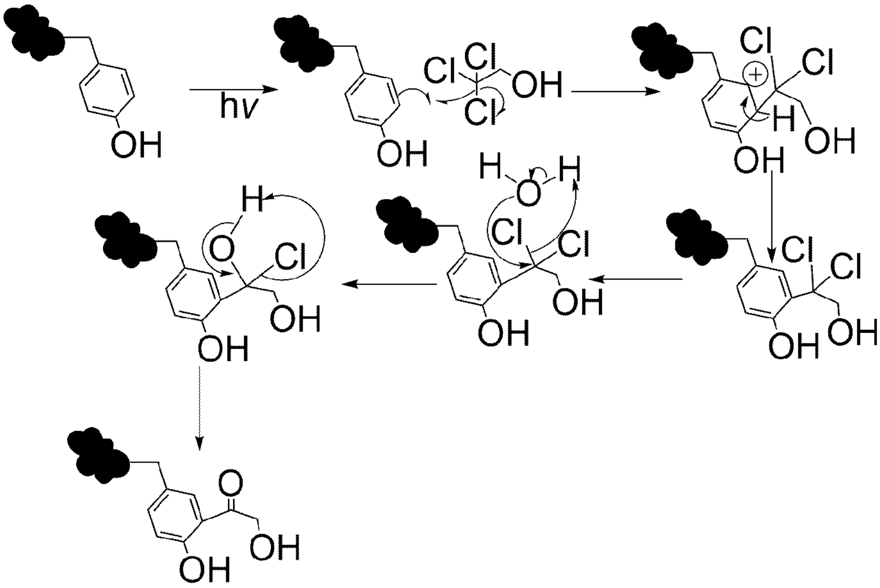


(c) Cysteine reduced ($C_R$)

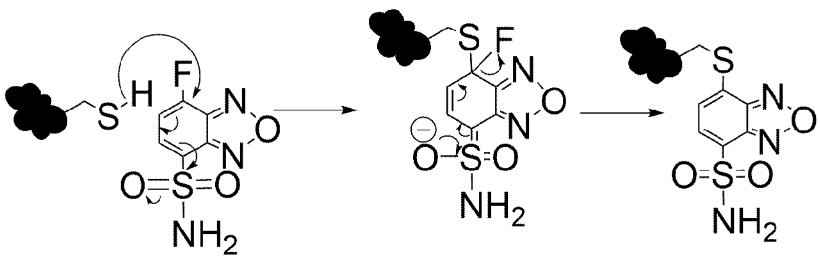

Figure 16a-c

(d) Cysteine

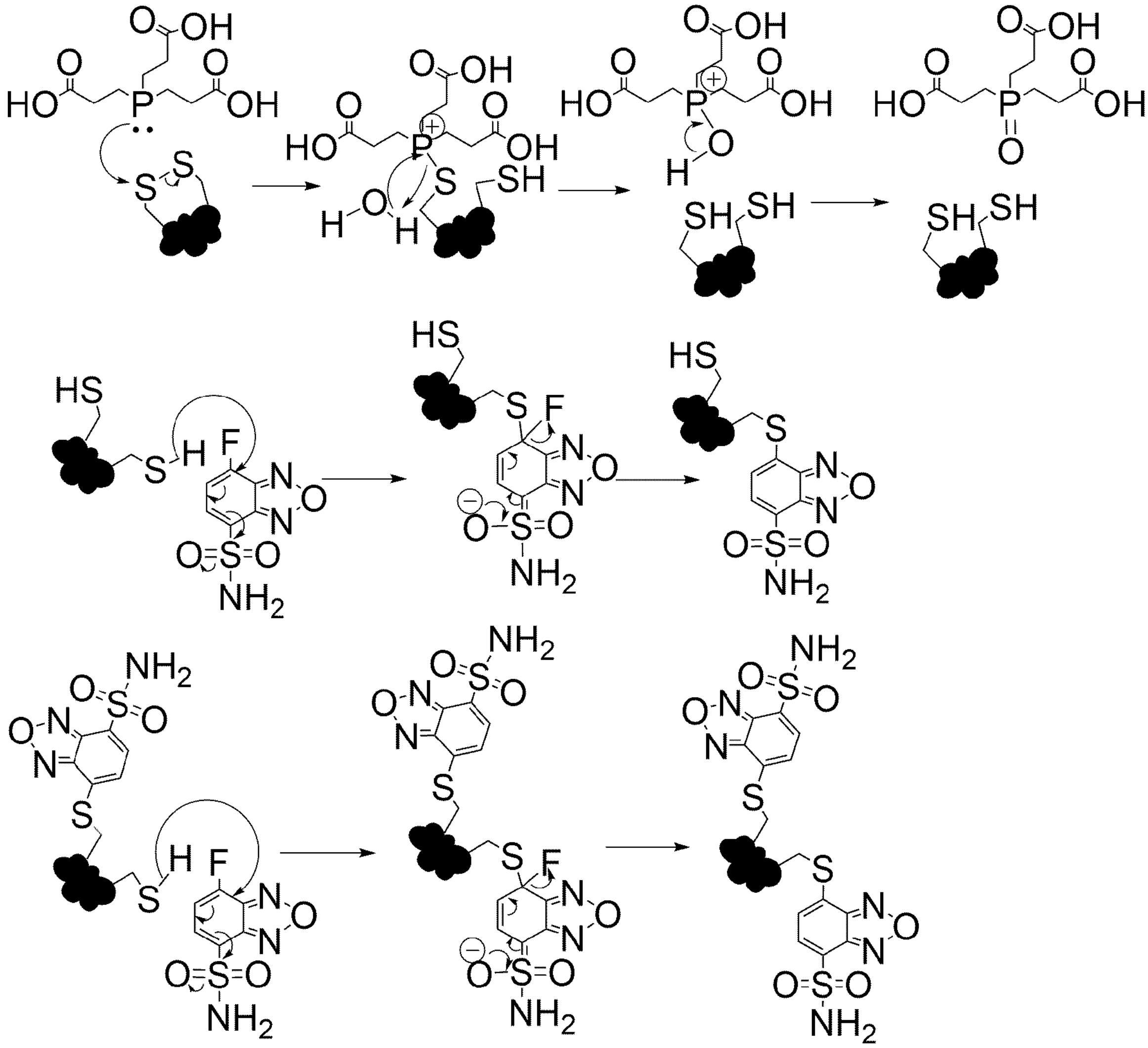

(e) Lysine

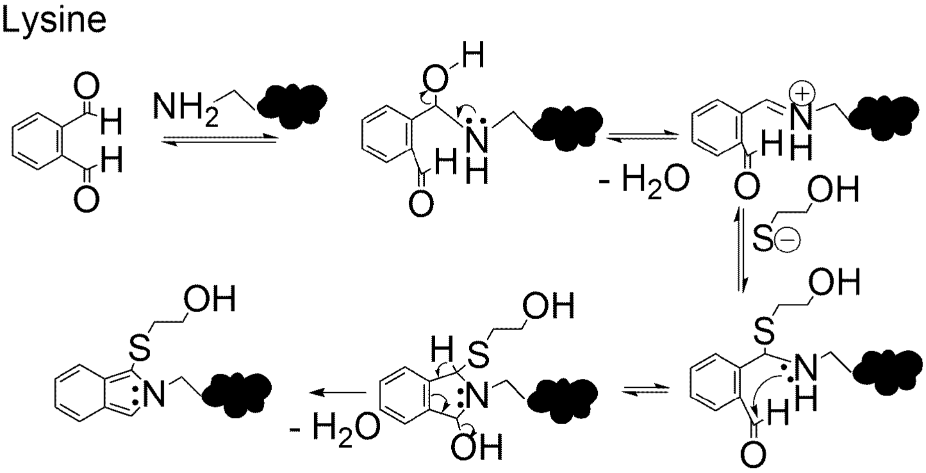

Figure 16d-e

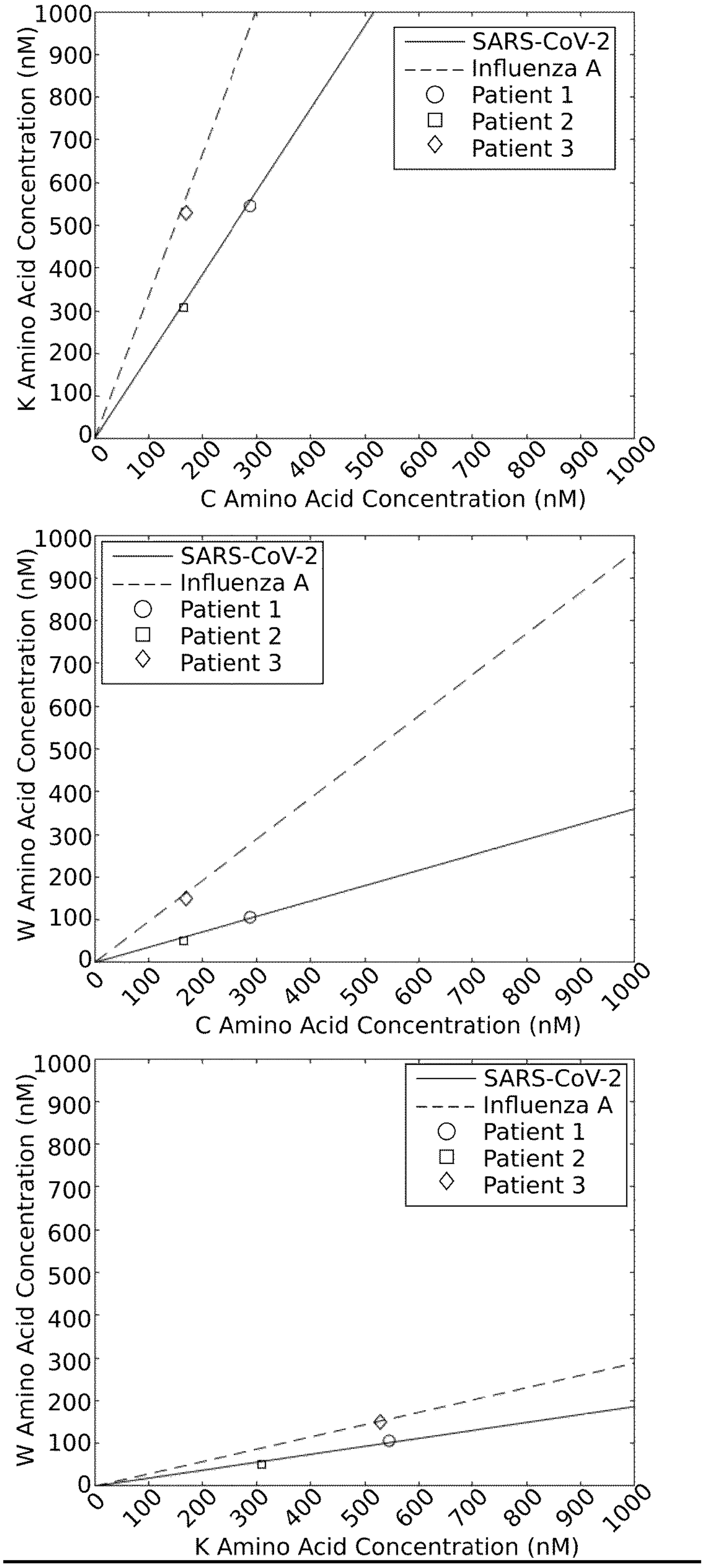

K Amino Acid Concentration (nM)
C Amino Acid Concentration (nM)
SARS-CoV-2
Influenza A
Patient 1
Patient 2
Patient 3
0
100
200
300
400
500
600
700
800
900
1000
W Amino Acid Concentration (nM)
C Amino Acid Concentration (nM)
SARS-CoV-2
Influenza A
Patient 1
Patient 2
Patient 3
W Amino Acid Concentration (nM)
K Amino Acid Concentration (nM)
SARS-CoV-2
Influenza A
Patient 1
Patient 2
Patient 3


Figure 17

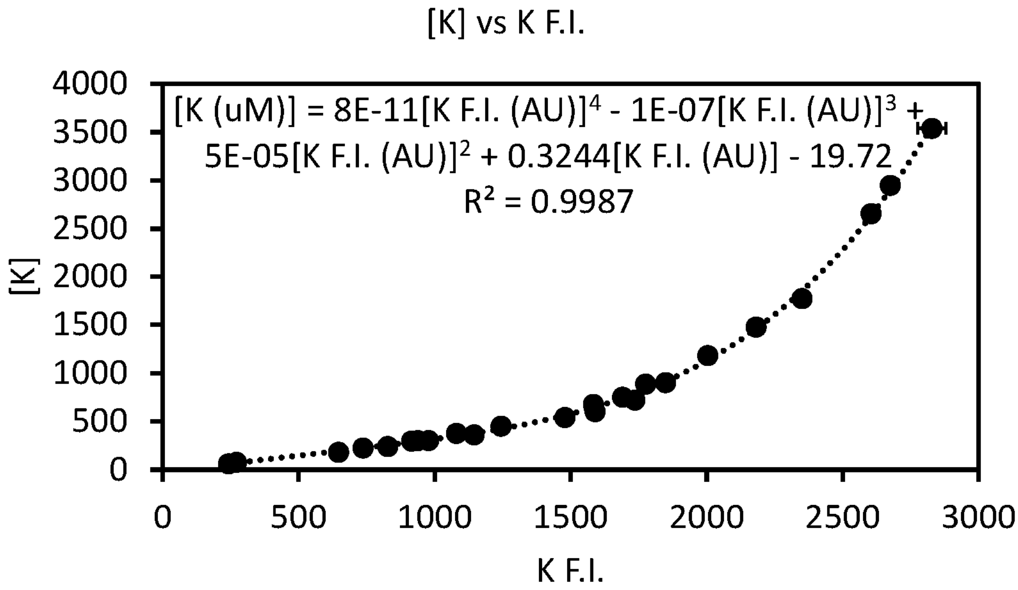


Figure 18

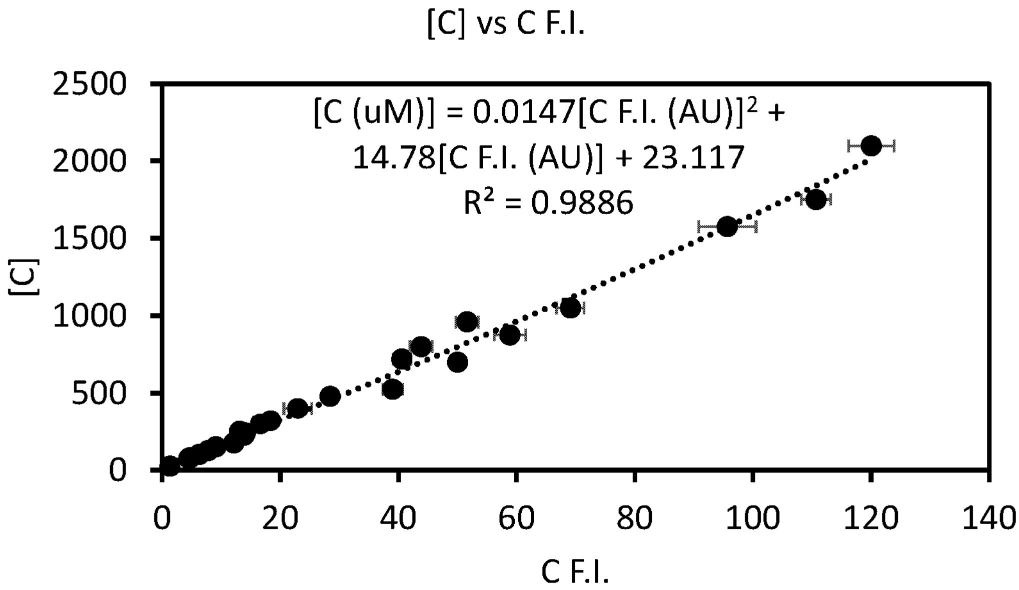


Figure 19

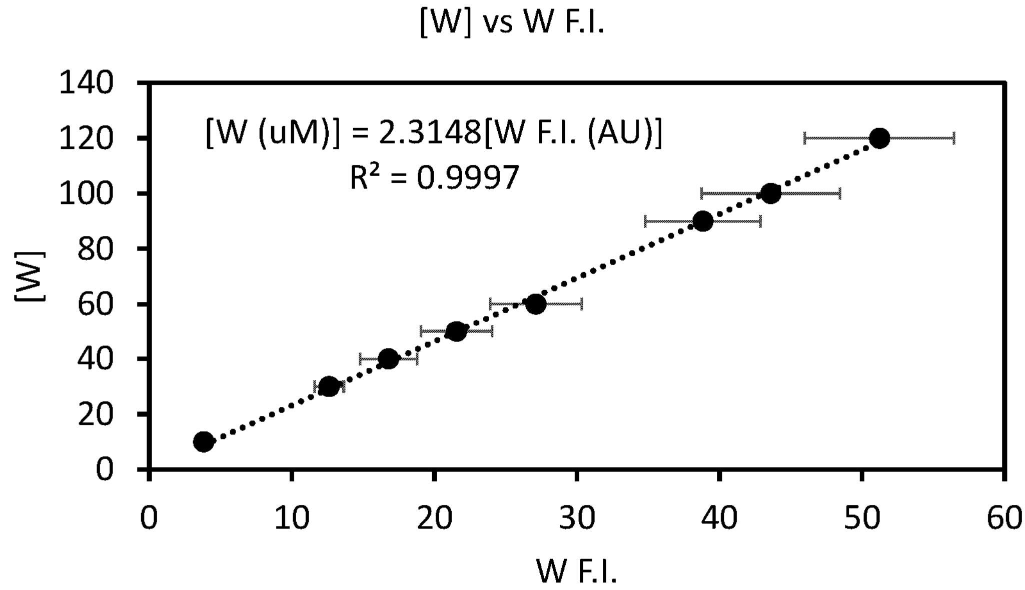


Figure 20

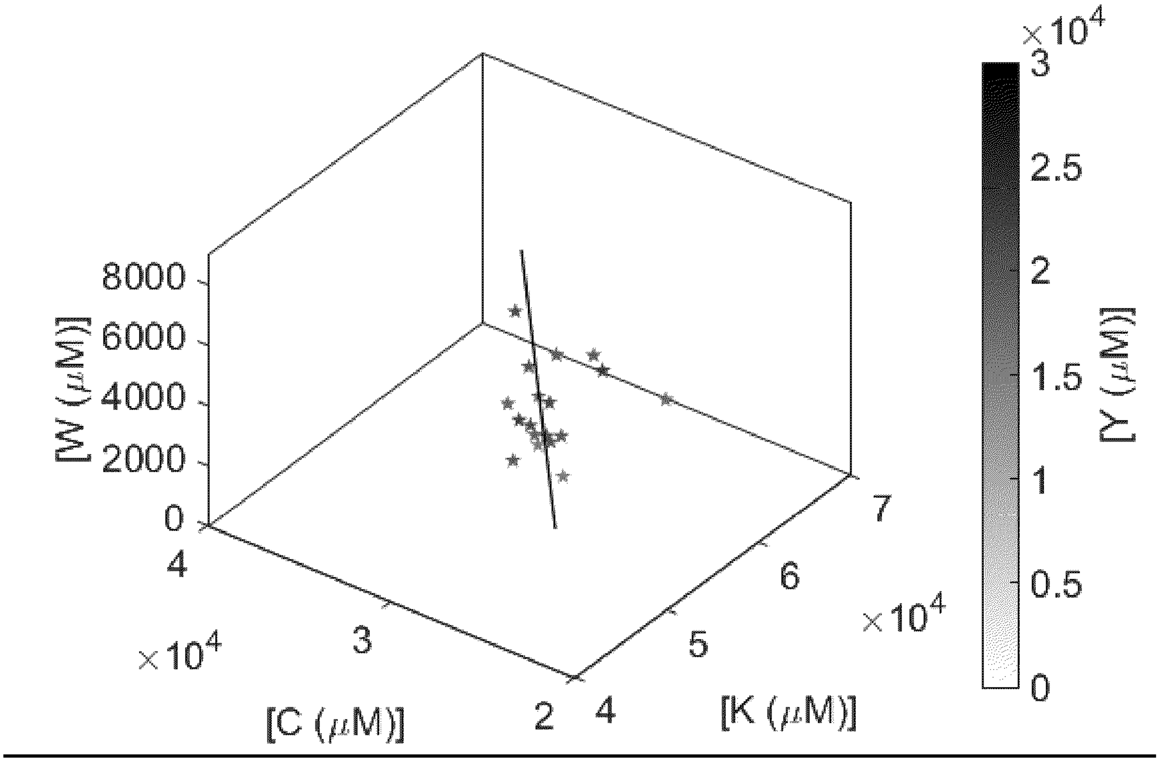


Figure 21

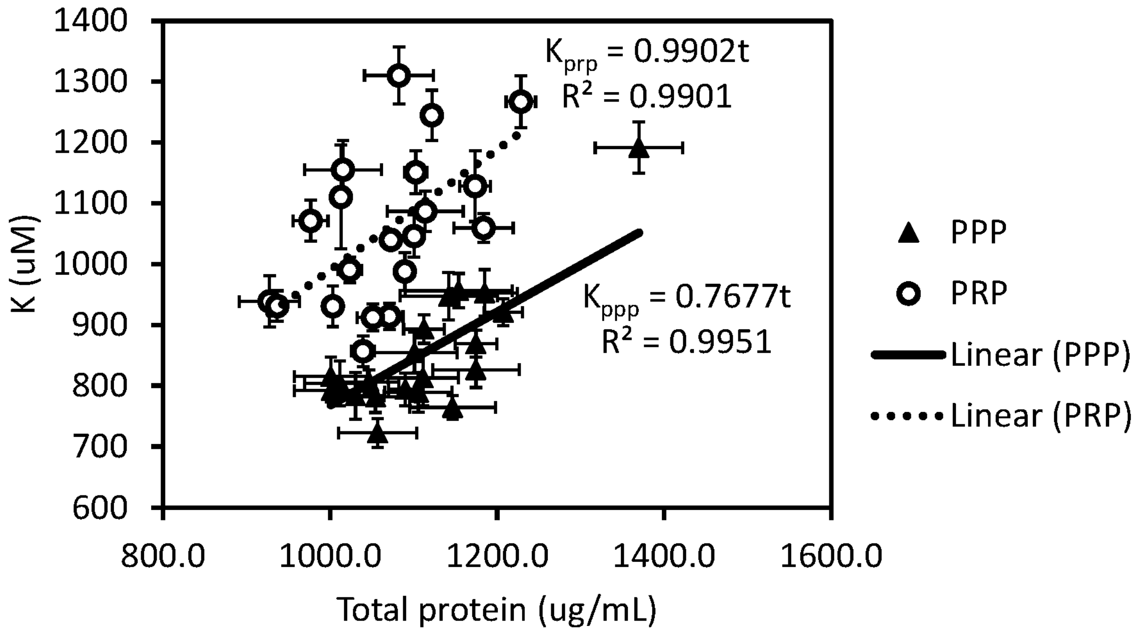


Figure 22

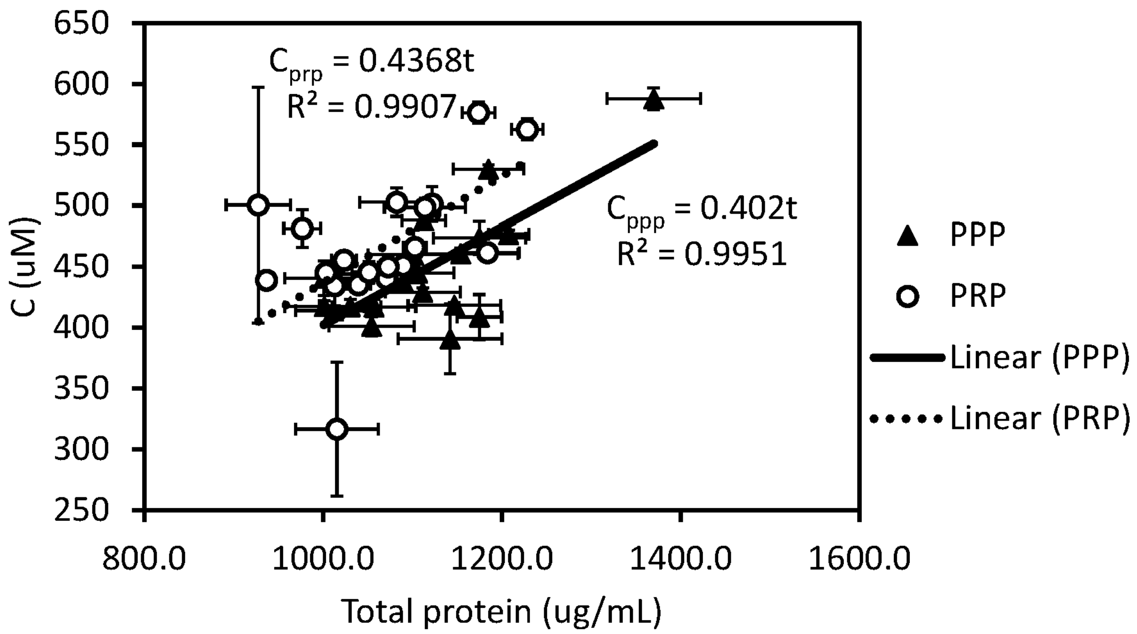


Figure 23

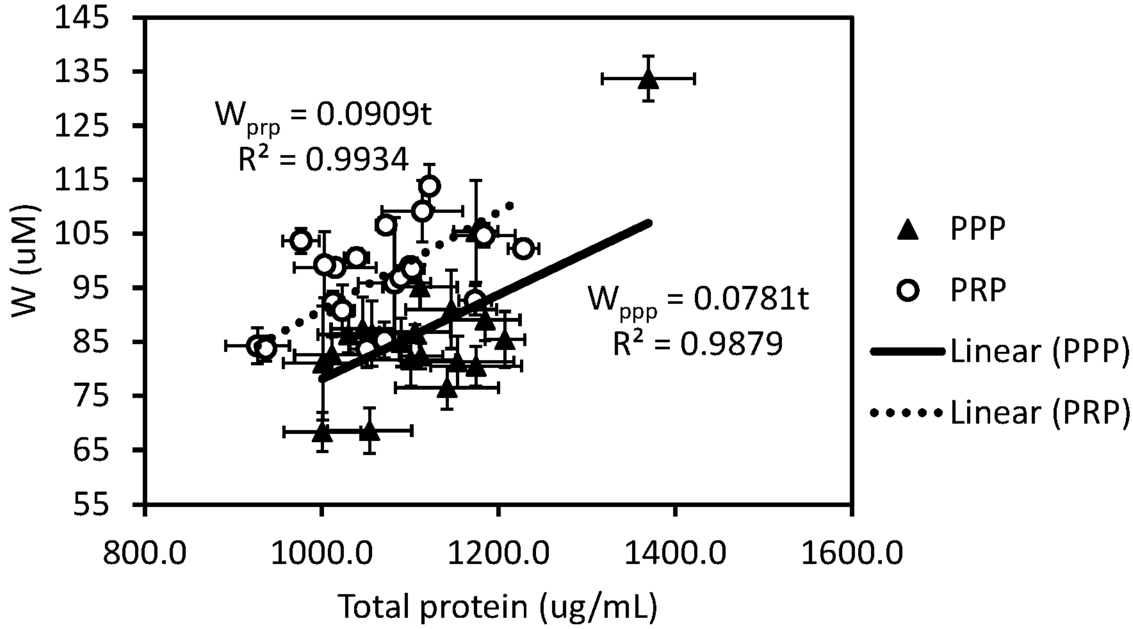


Figure 24

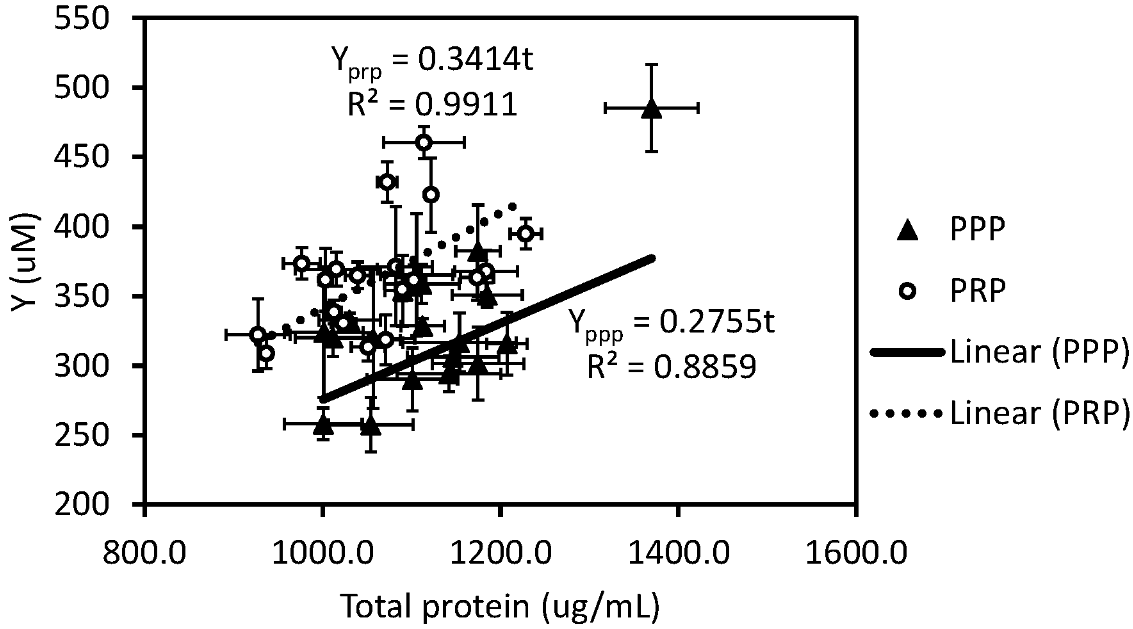


Figure 25

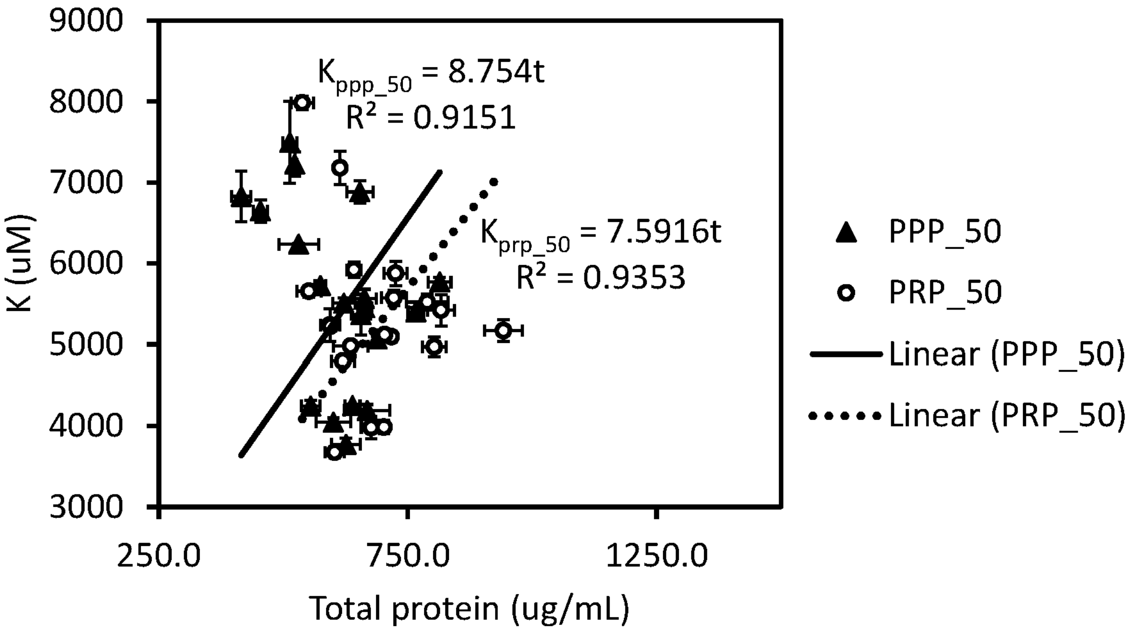


Figure 26

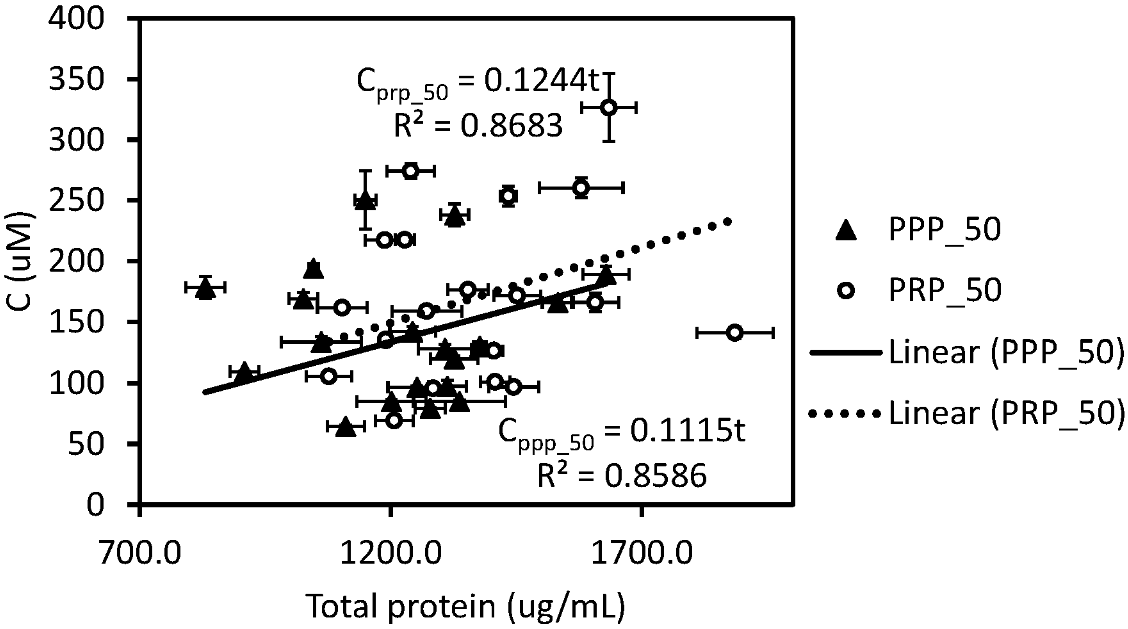


Figure 27

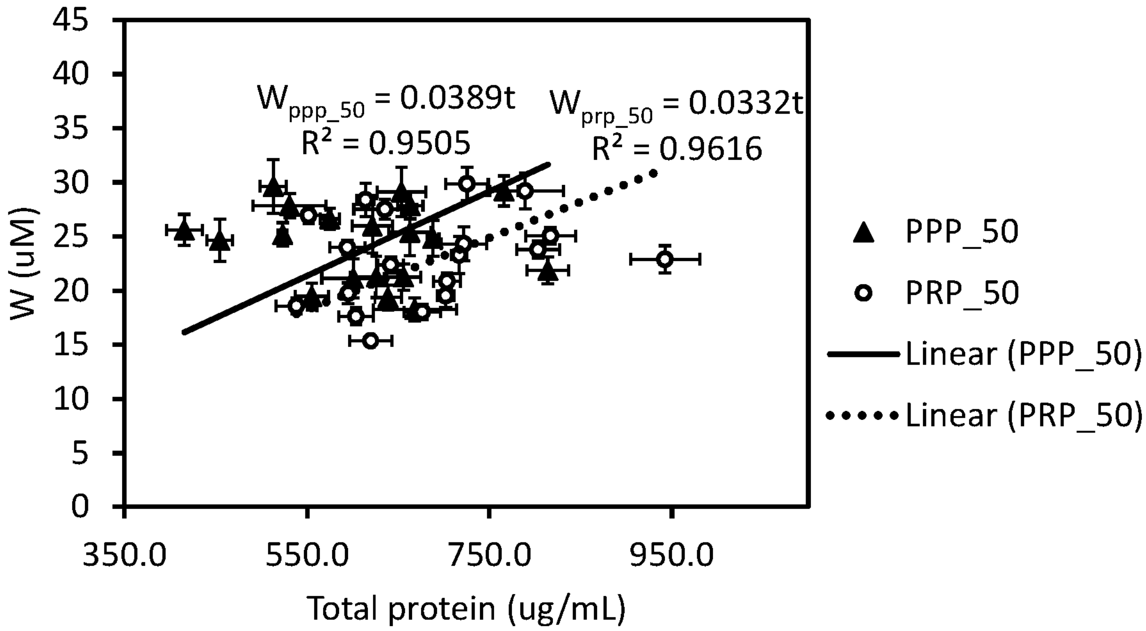


Figure 28

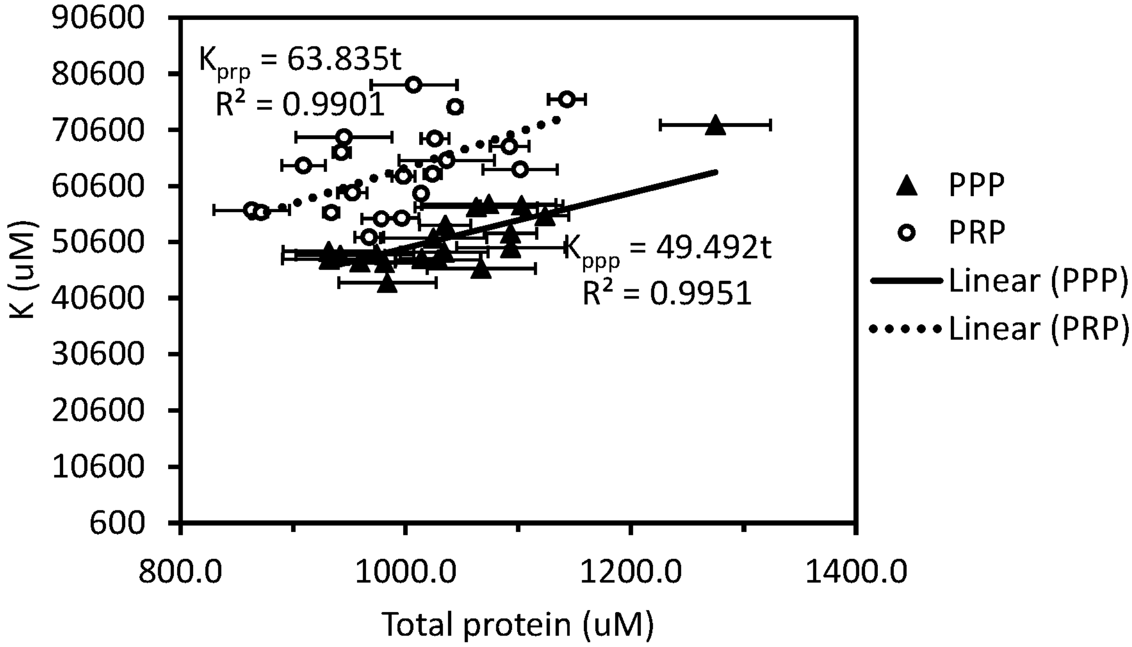


Figure 29

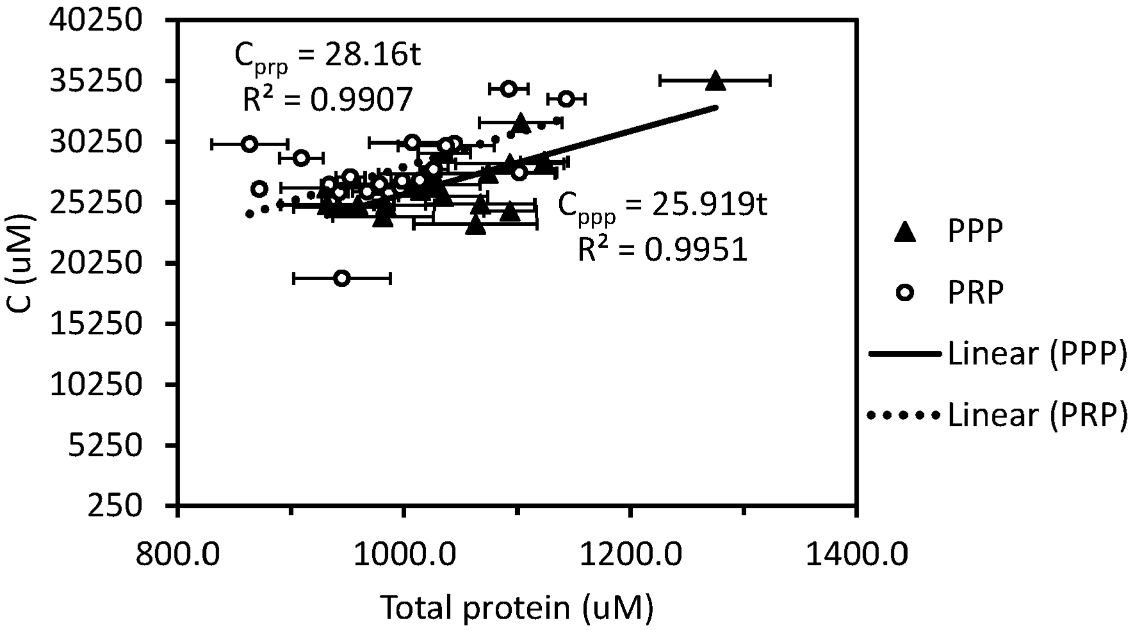


Figure 30

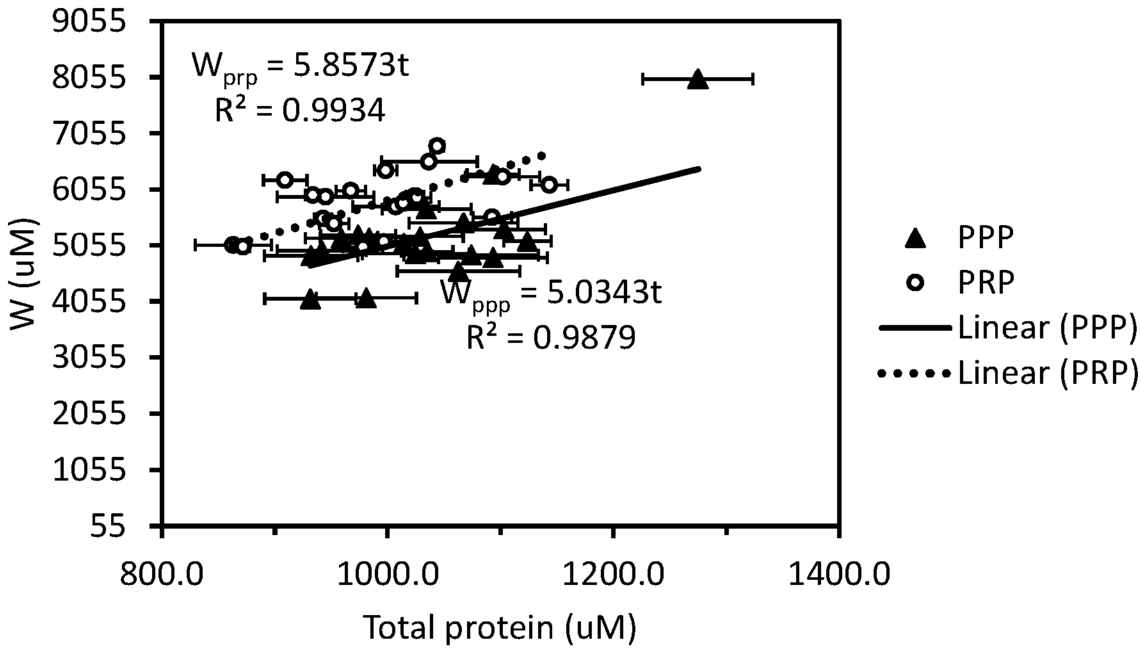


Figure 31

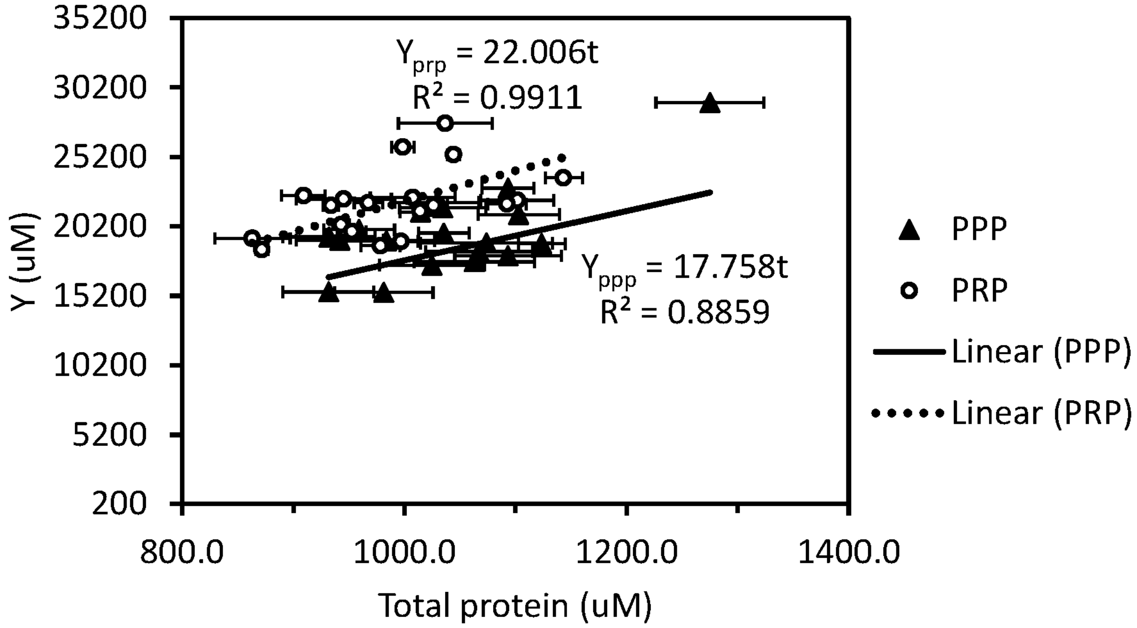


Figure 32

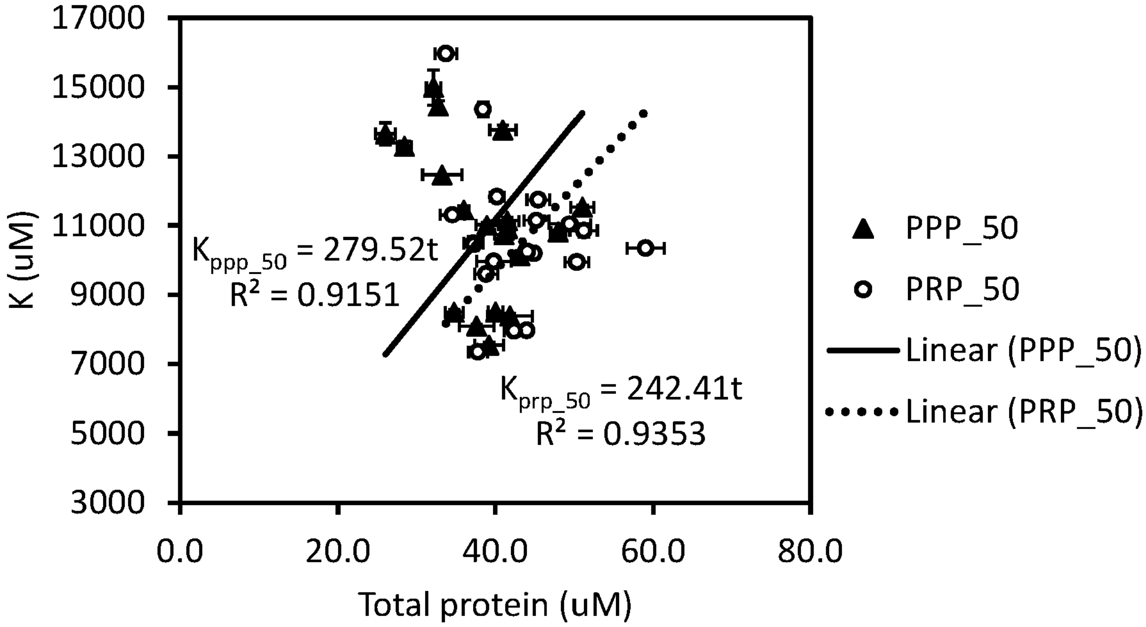


Figure 33

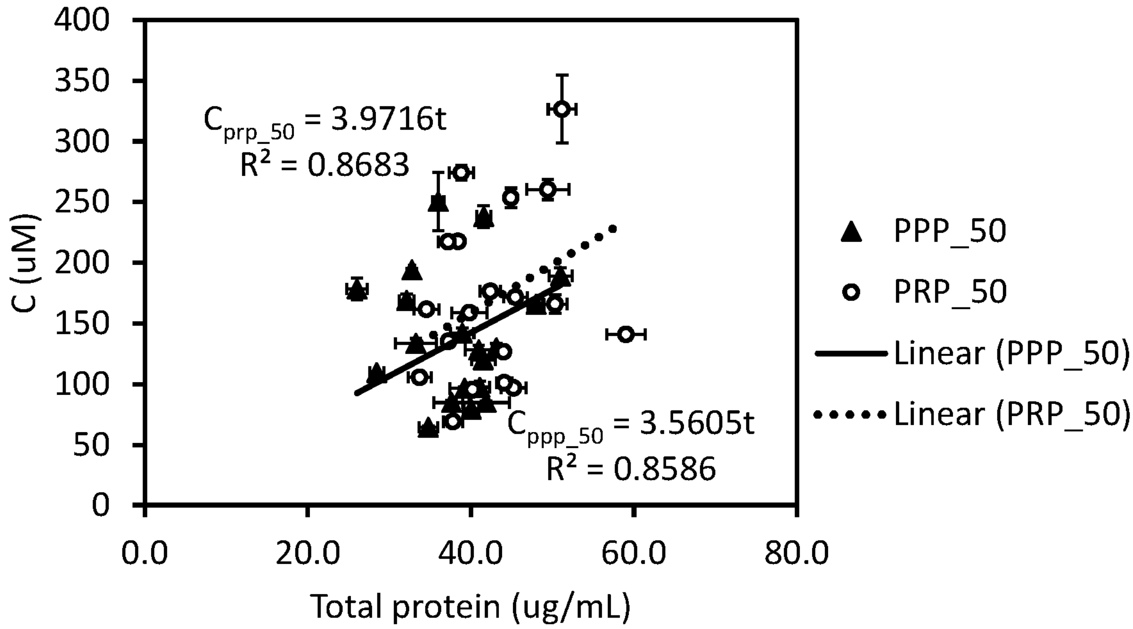


Figure 34

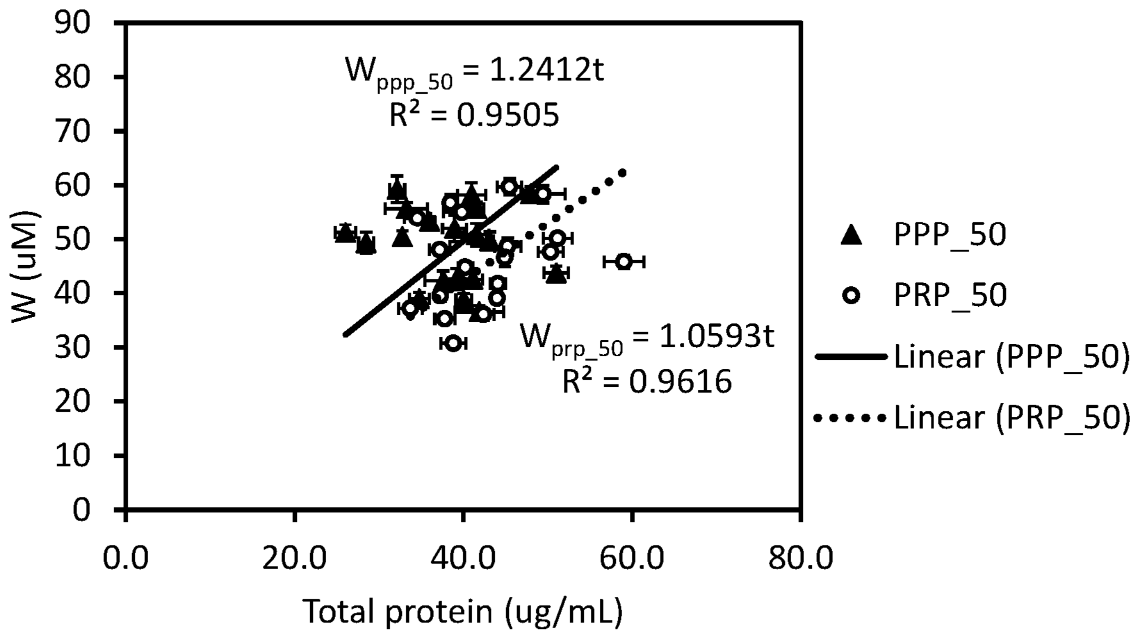


Figure 35

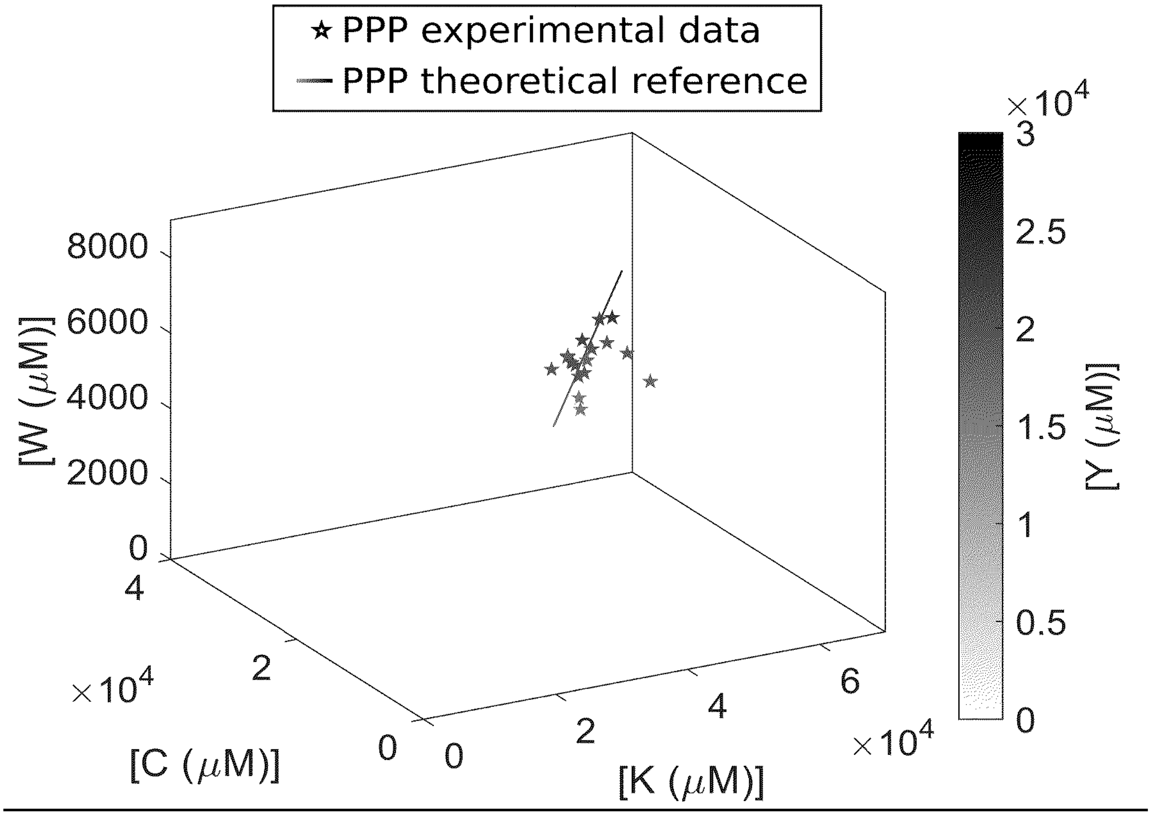


Figure 36

**Identification of a Proteome of Interest Within a Patient Sample using the Methods Disclosed Herein and 2 Labelled Amino Acid Types (Dataset)**

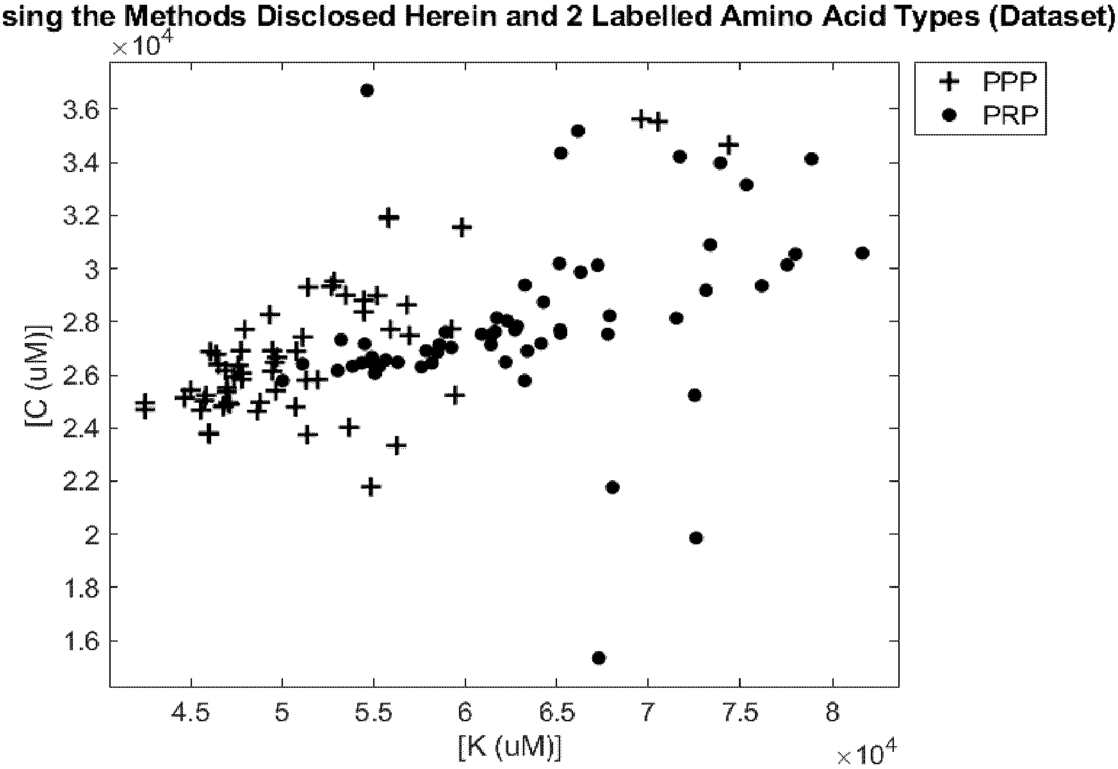


Figure 37

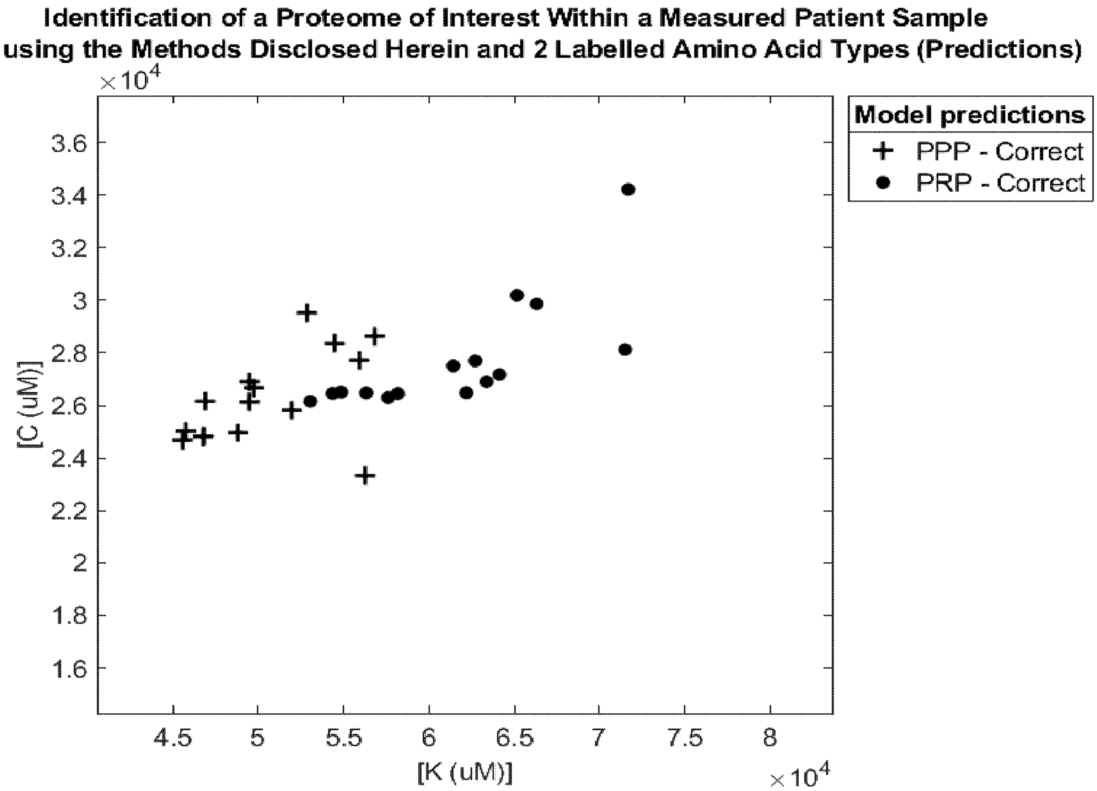


Figure 38

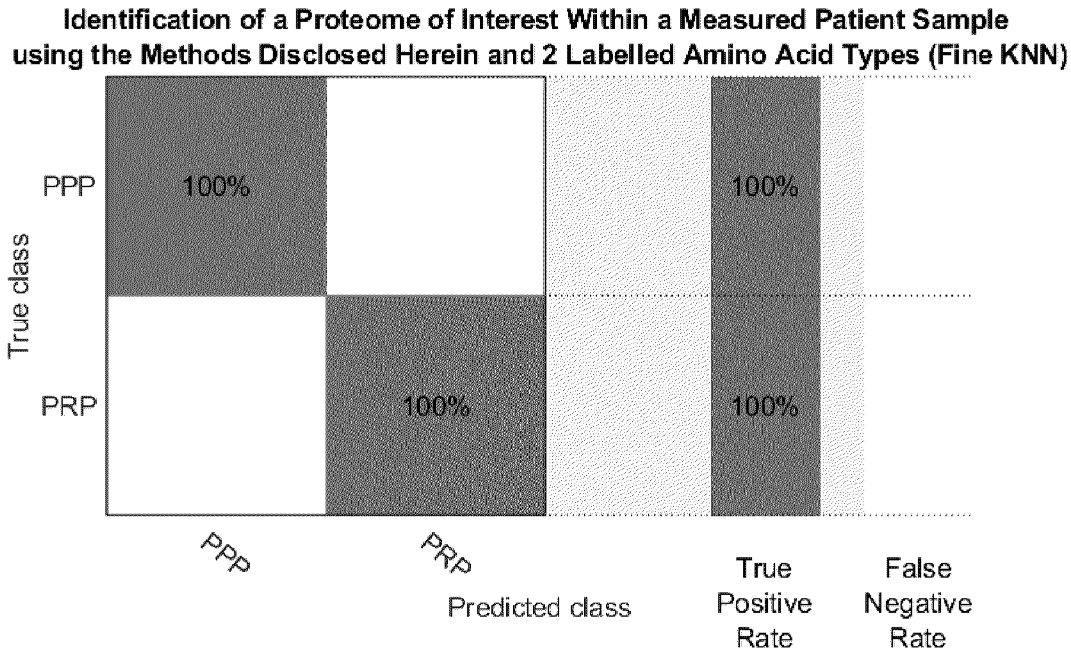


Figure 39

**Identification of a Proteome of Interest Within a Measured Patient Sample using the Methods Disclosed Herein and 2 Labelled Amino Acid Types (Bagged DecisionTrees)**

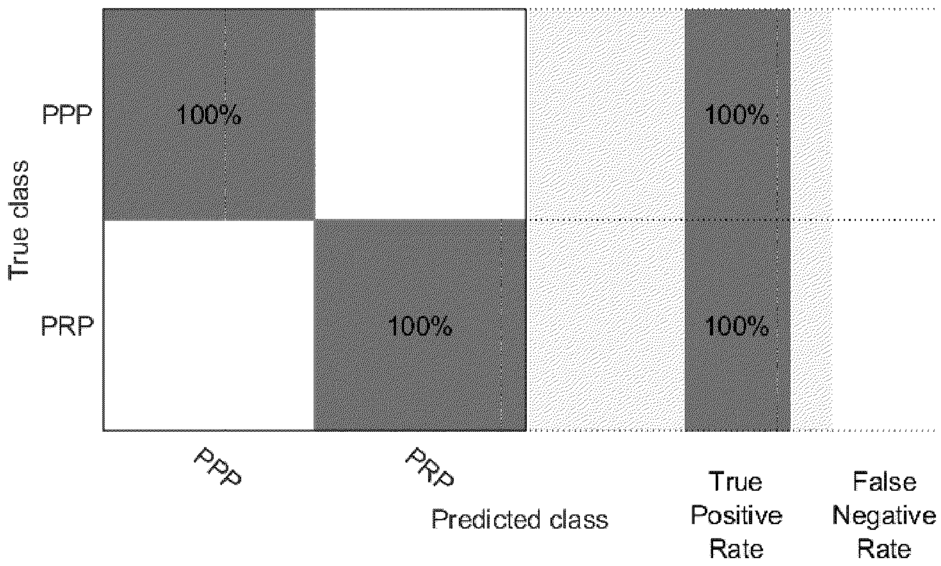


Figure 40

**Identification of a Proteome of Interest Within a Measured Patient Sample using the Methods Disclosed Herein and 2 Labelled Amino Acid Types (Bagged DecisionTrees)**

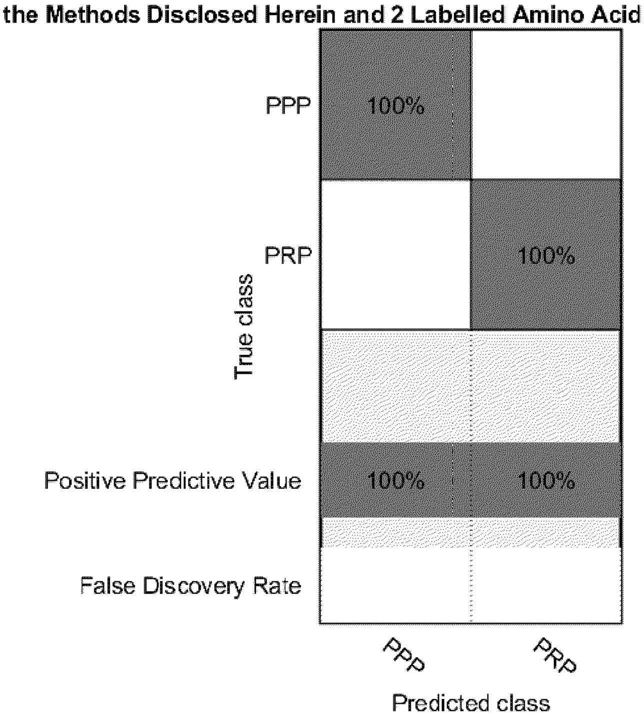


Figure 41

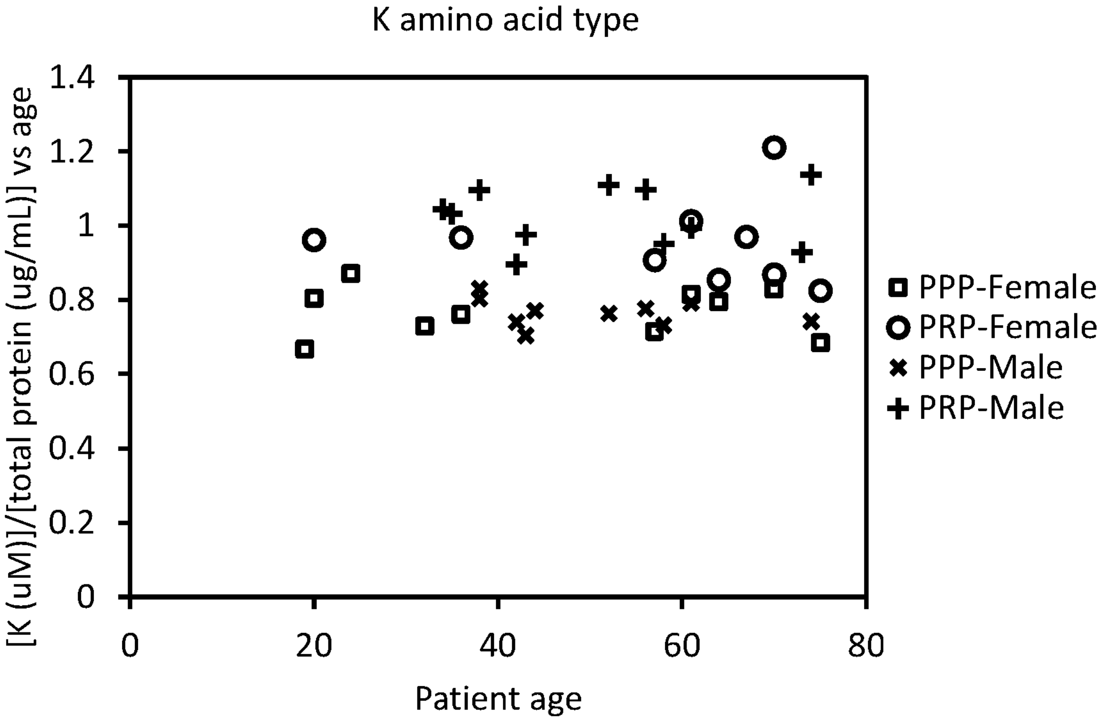


Figure 42

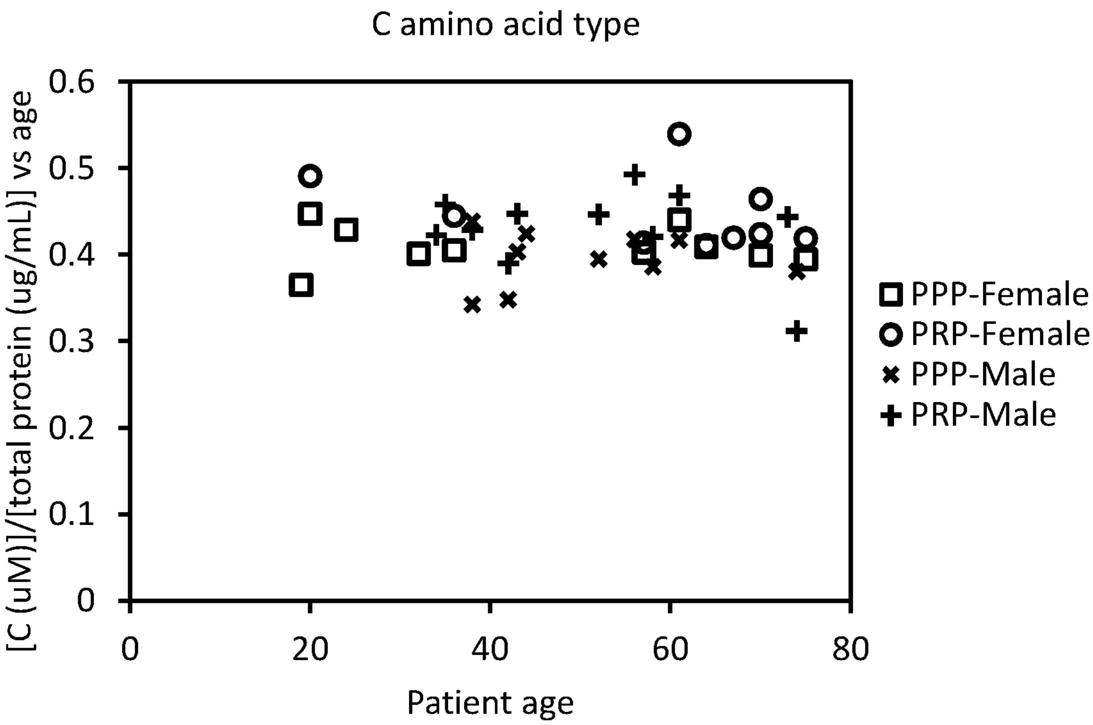


Figure 43

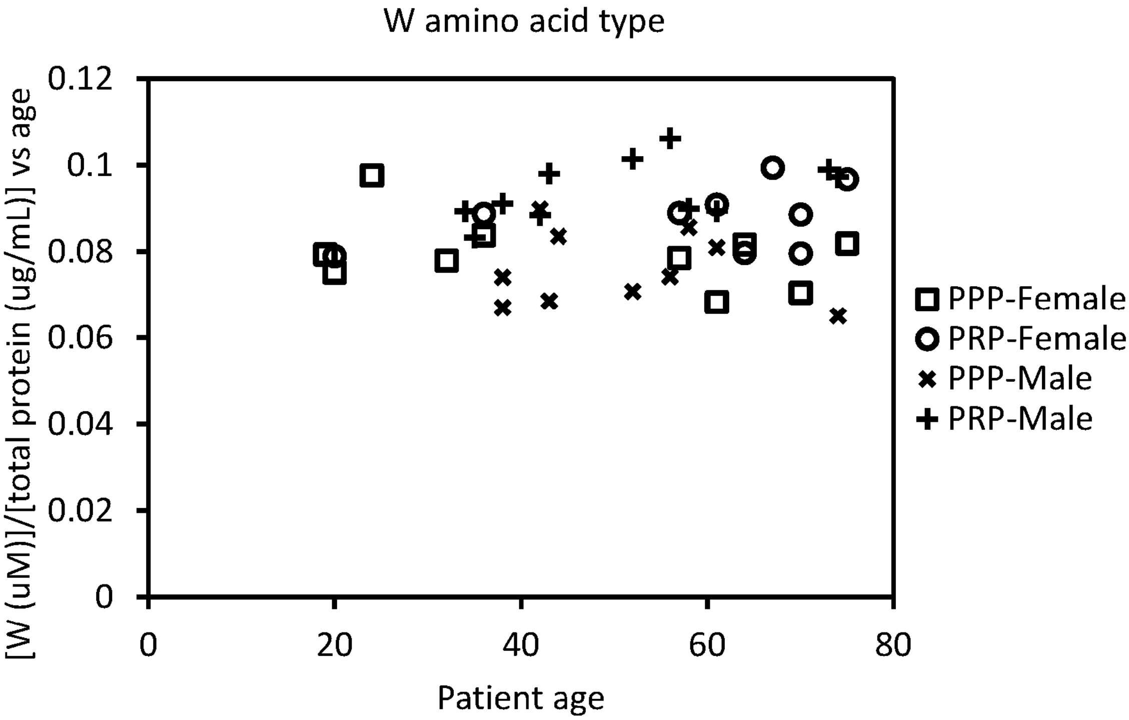


Figure 44

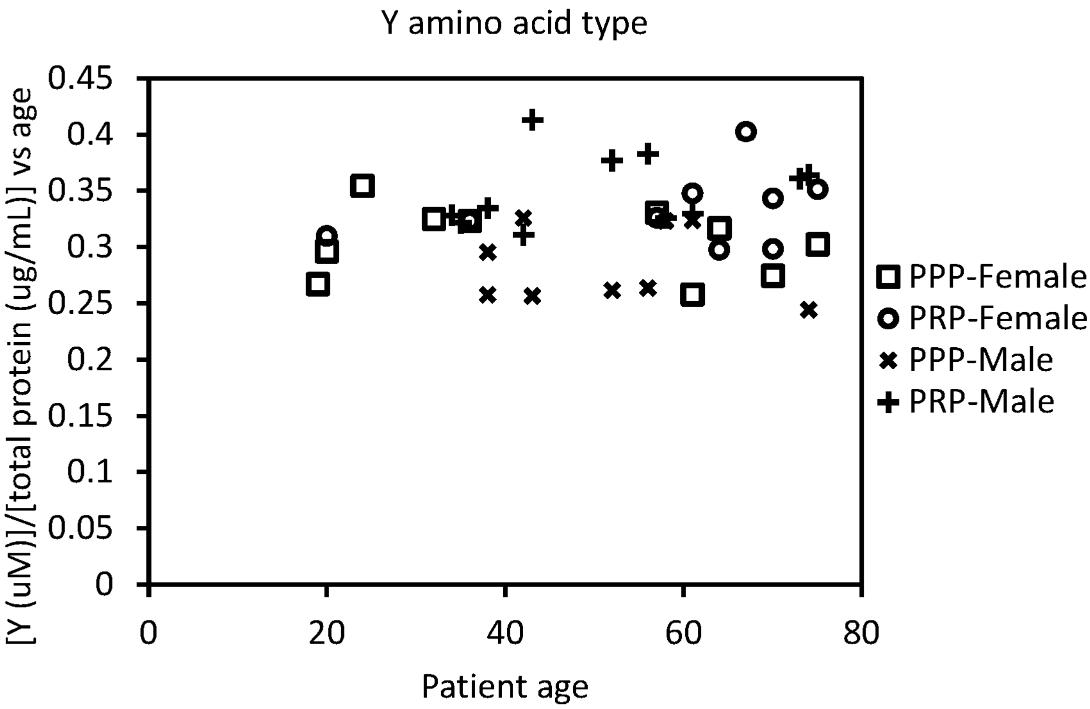


Figure 45

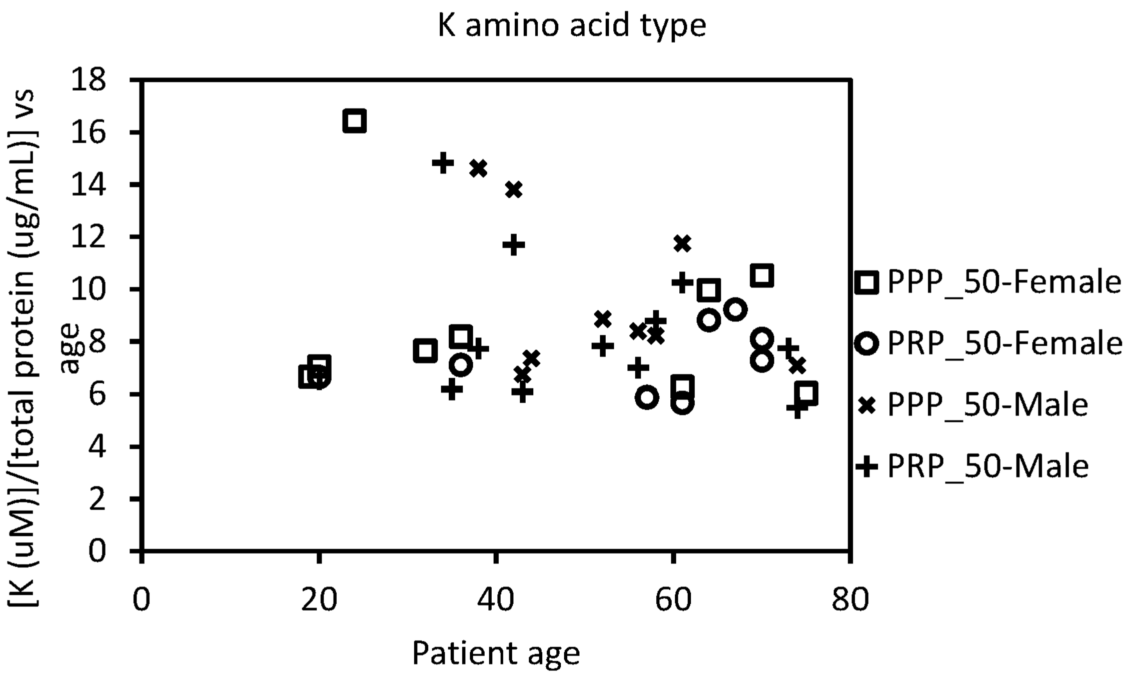


Figure 46

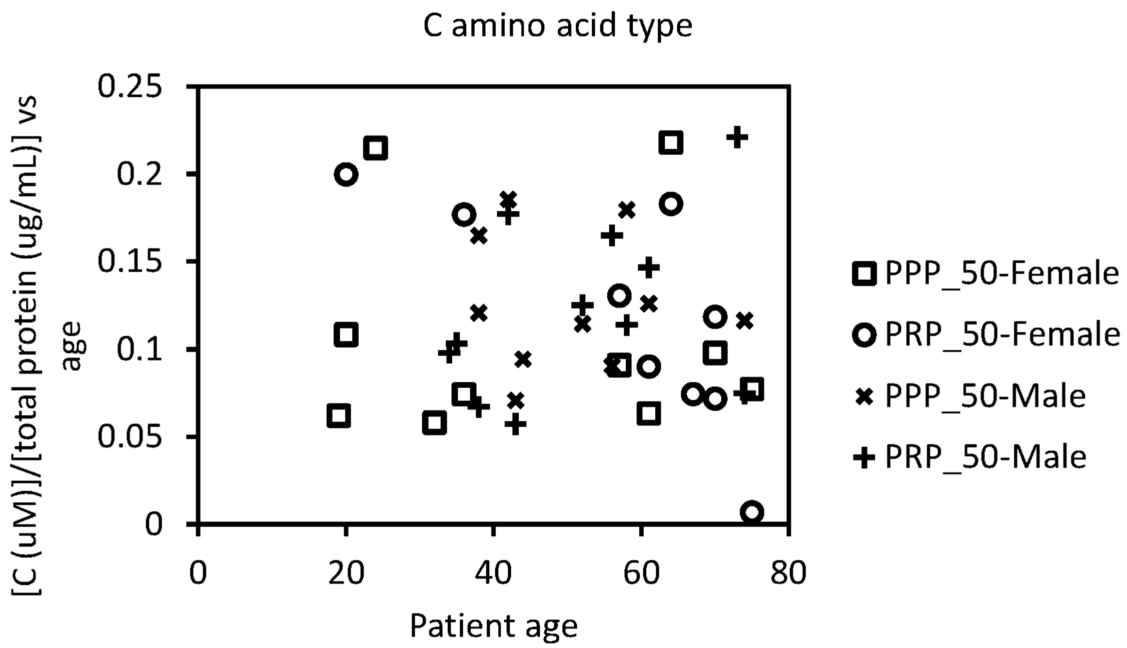


Figure 47

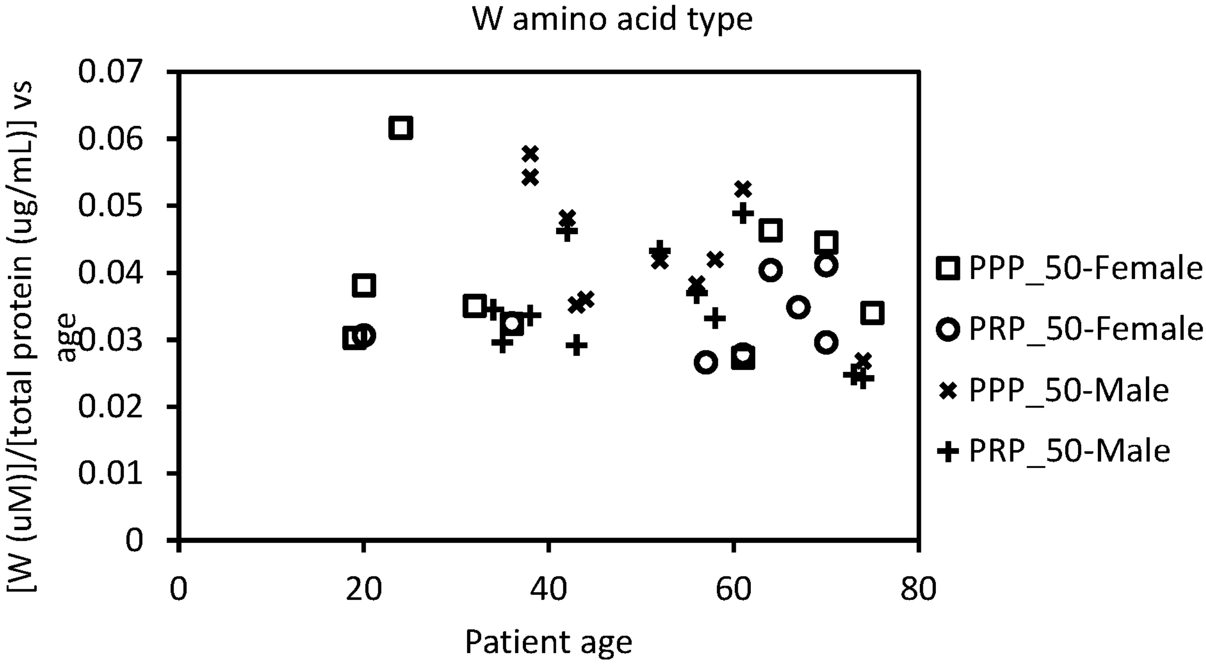


Figure 48

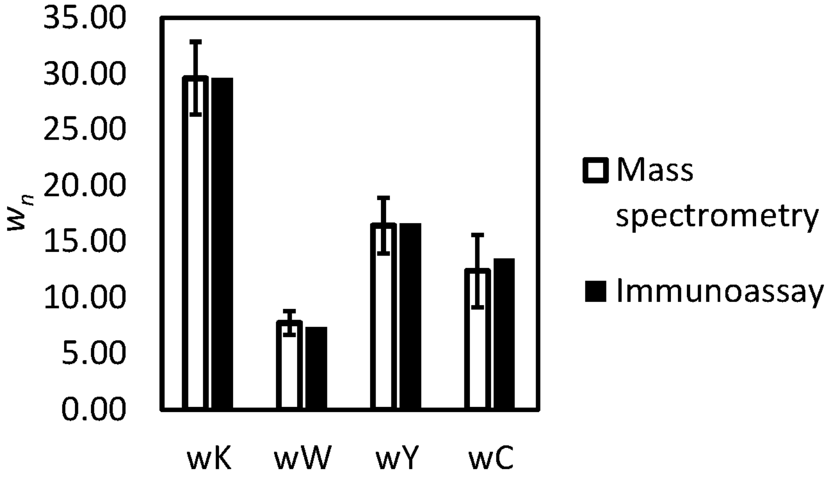


Figure 49

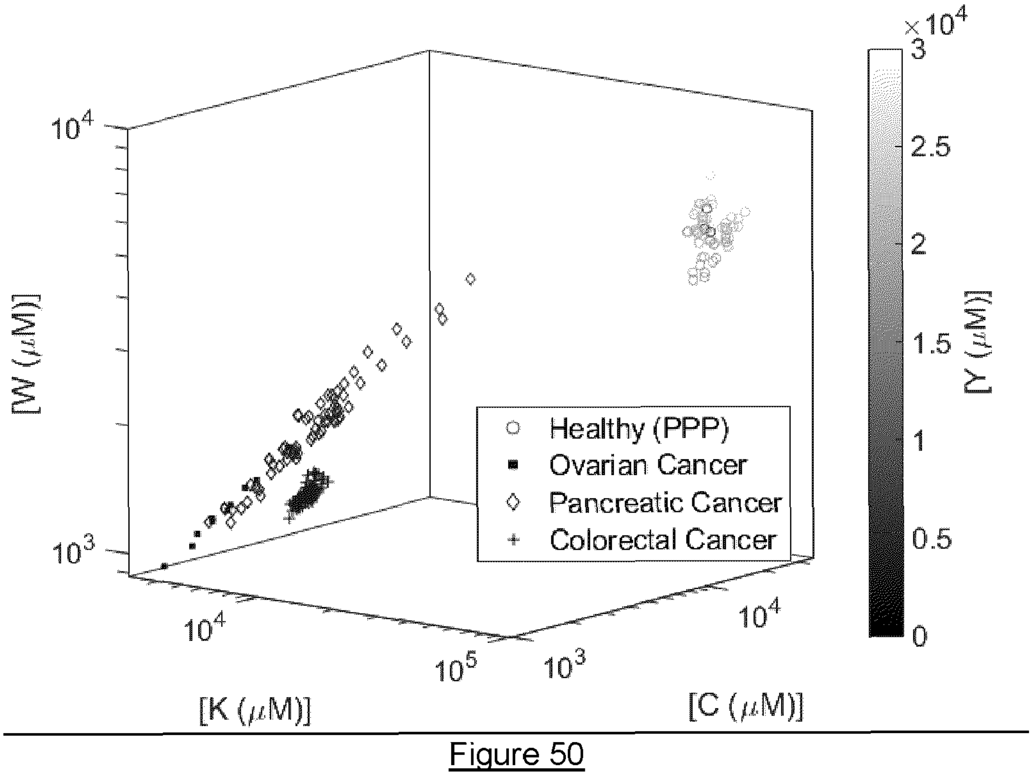


Figure 50

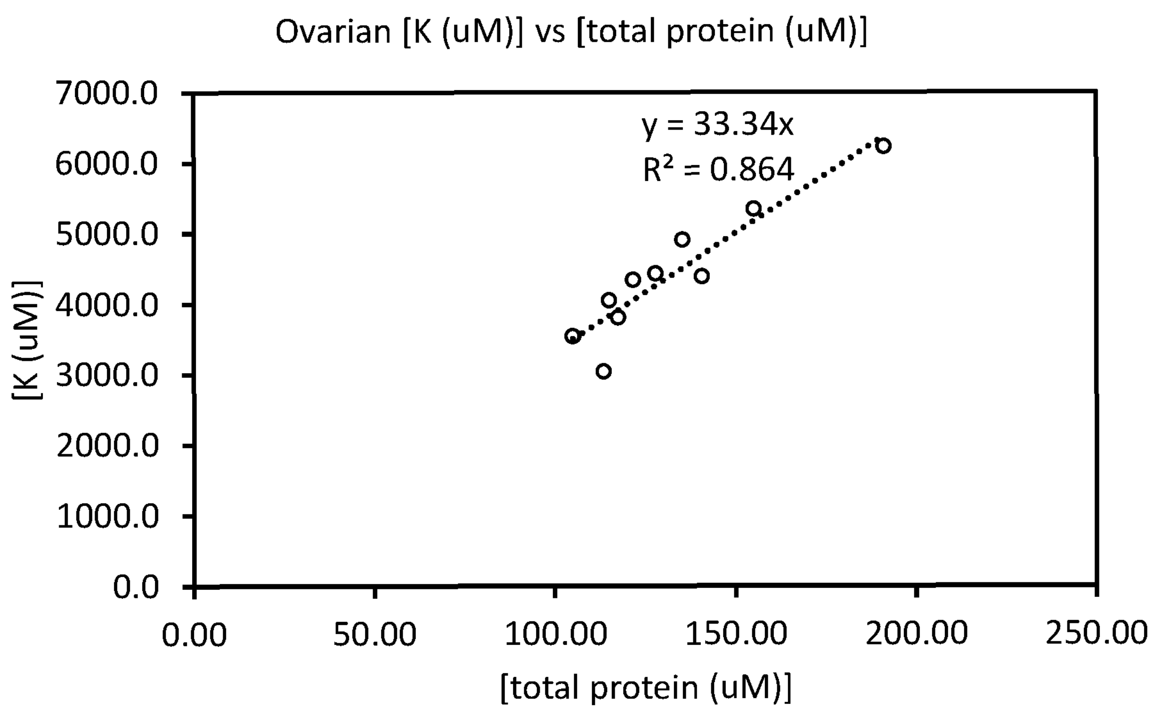


Figure 51

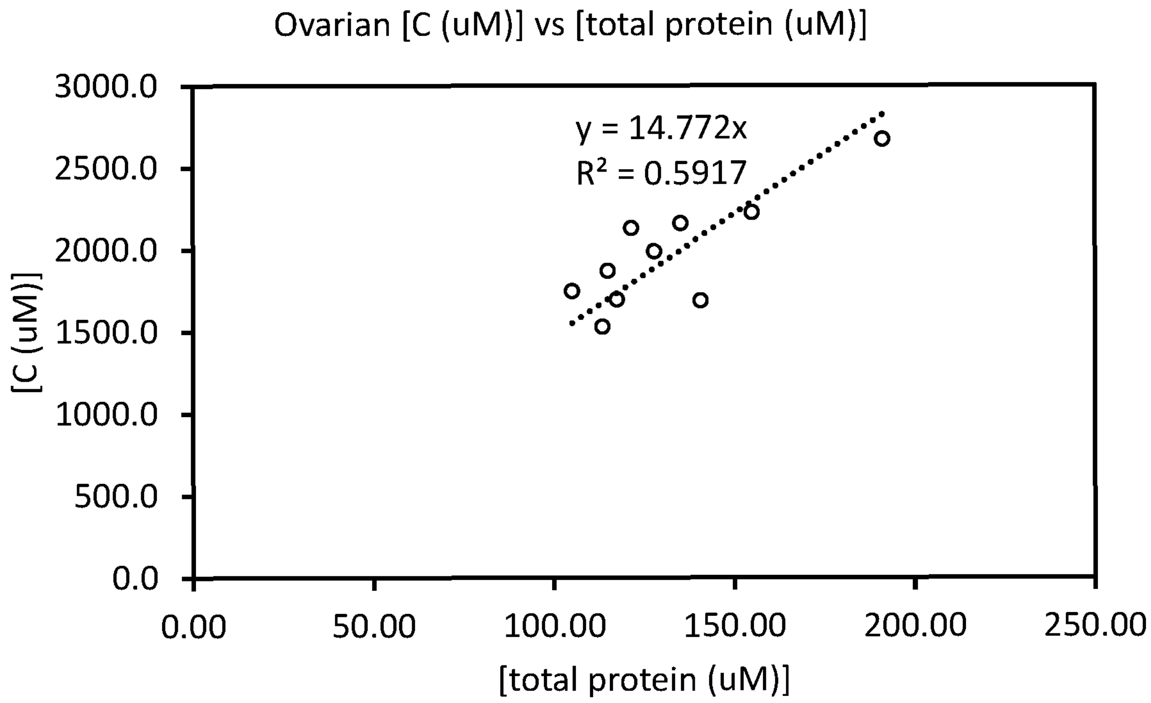


Figure 52

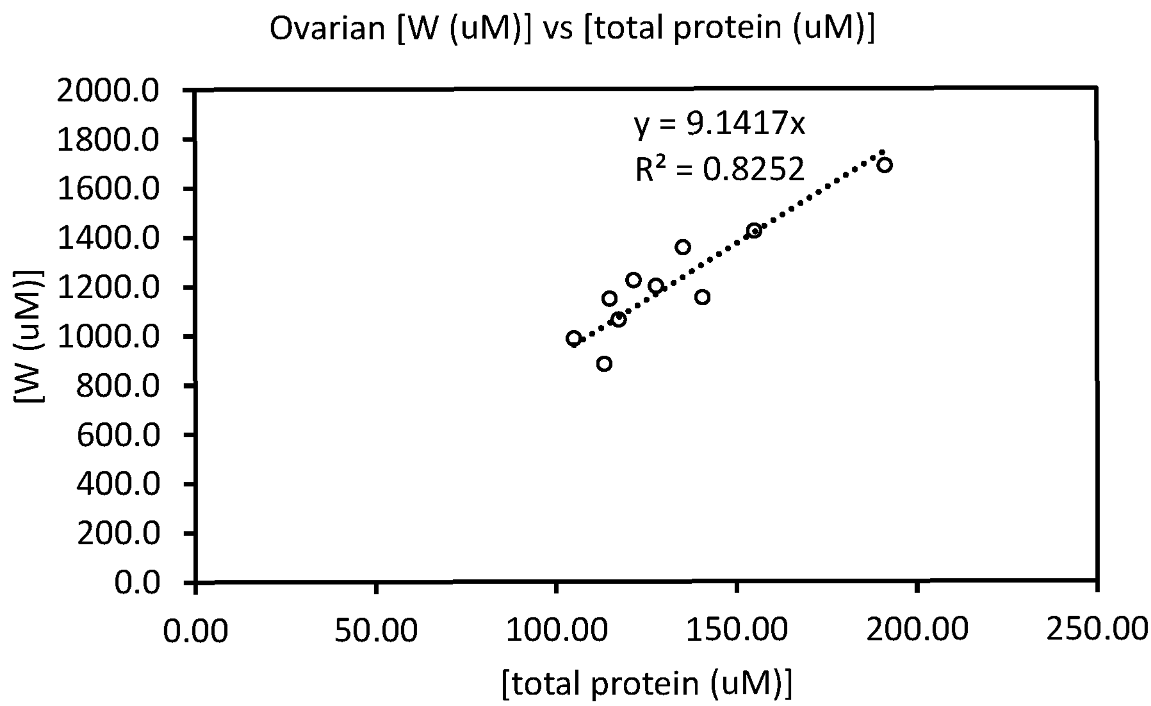


Figure 53

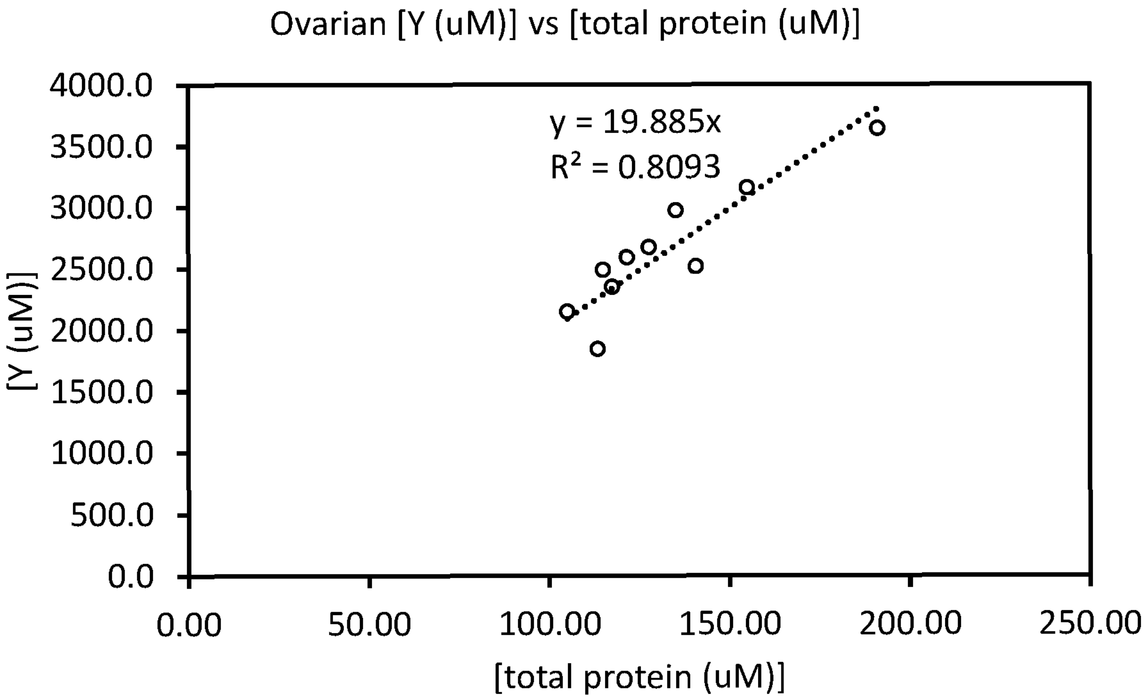


Figure 54

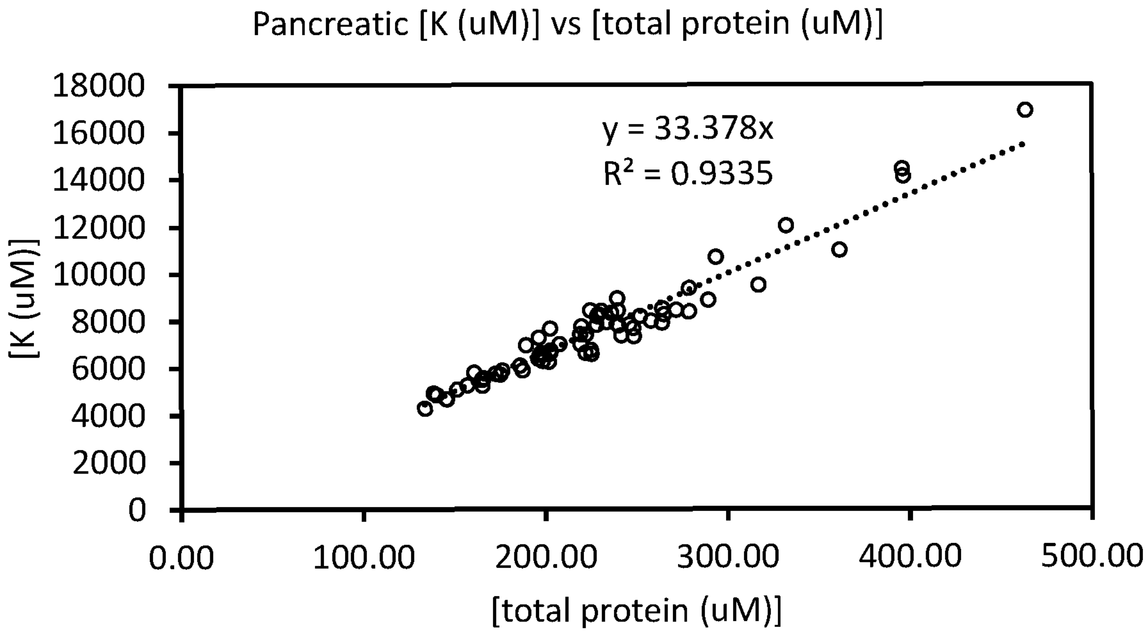


Figure 55

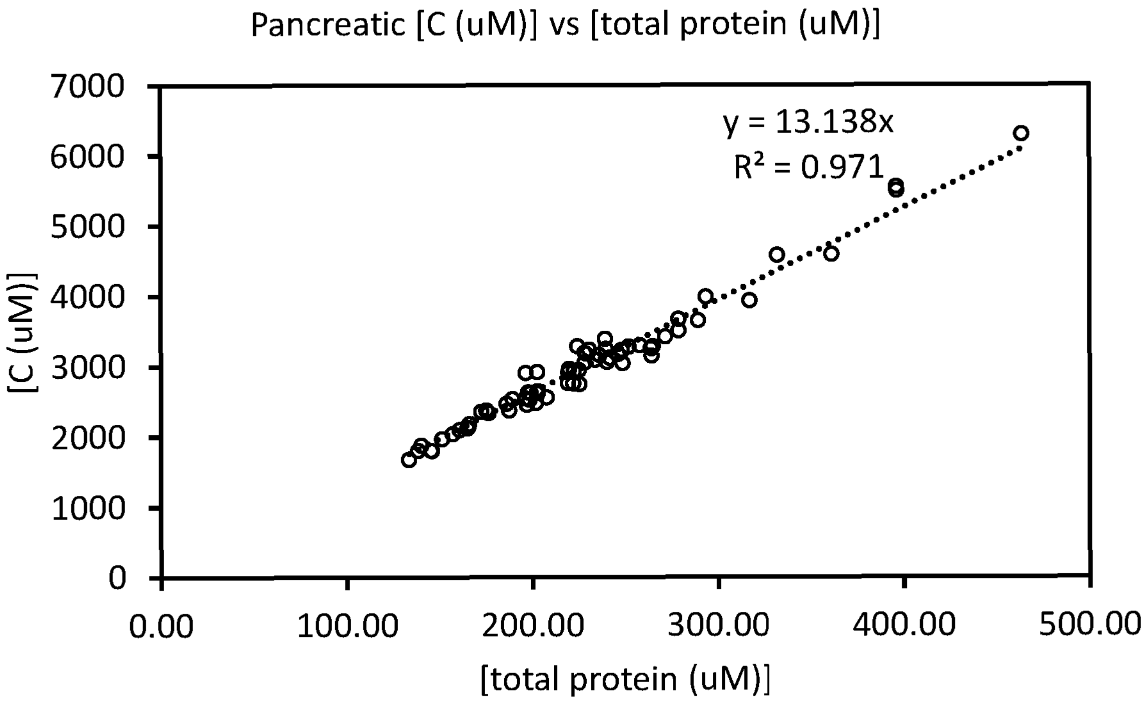


Figure 56

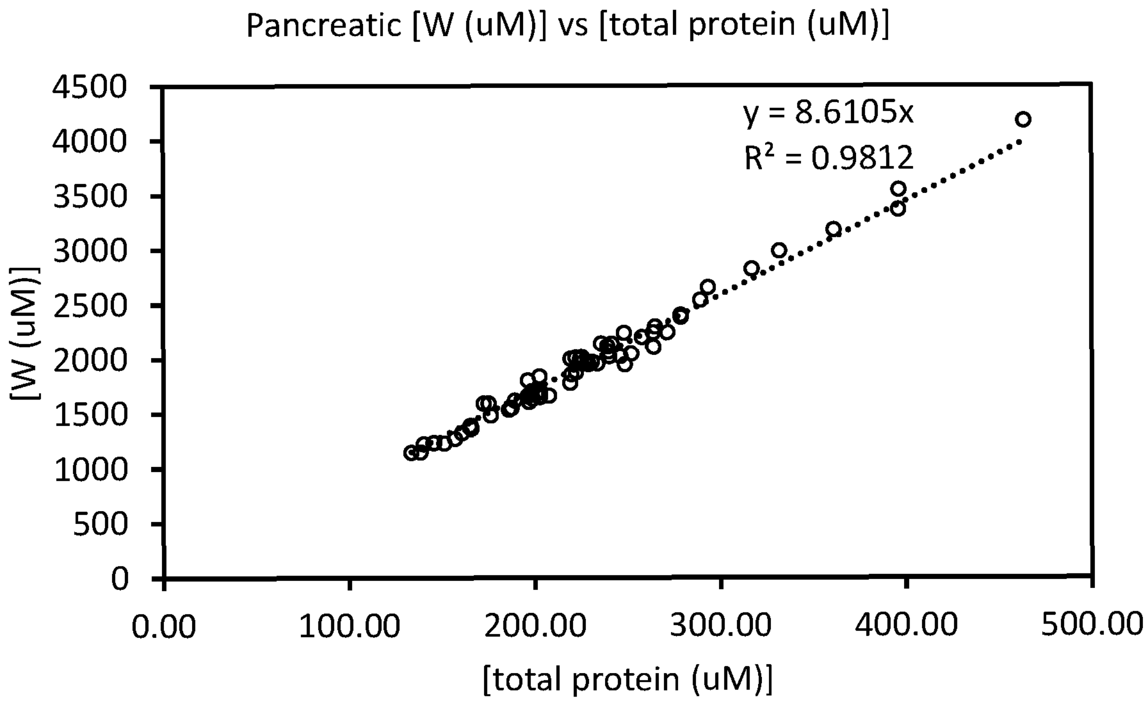


Figure 57

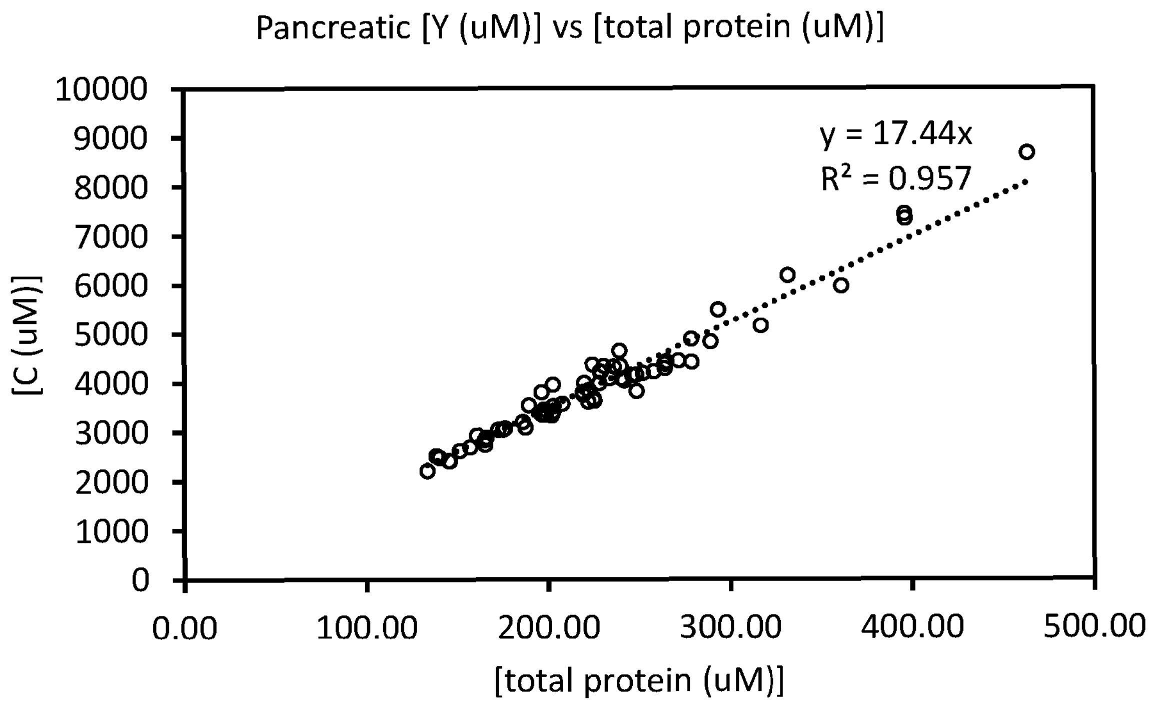


Figure 58

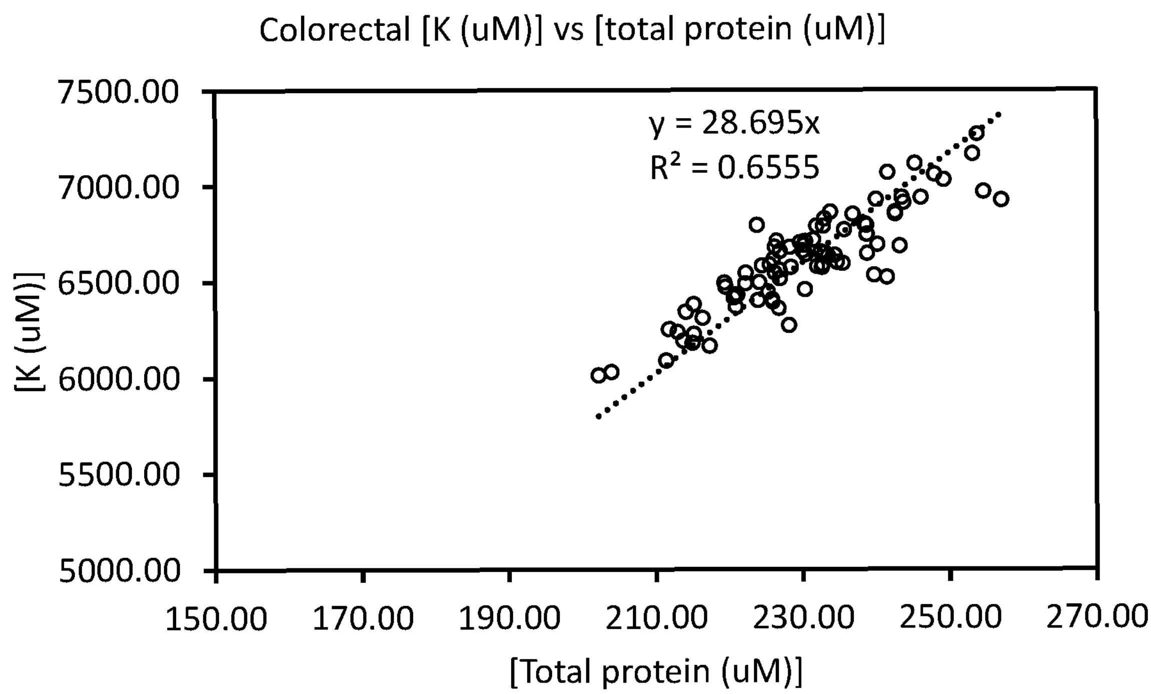


Figure 59

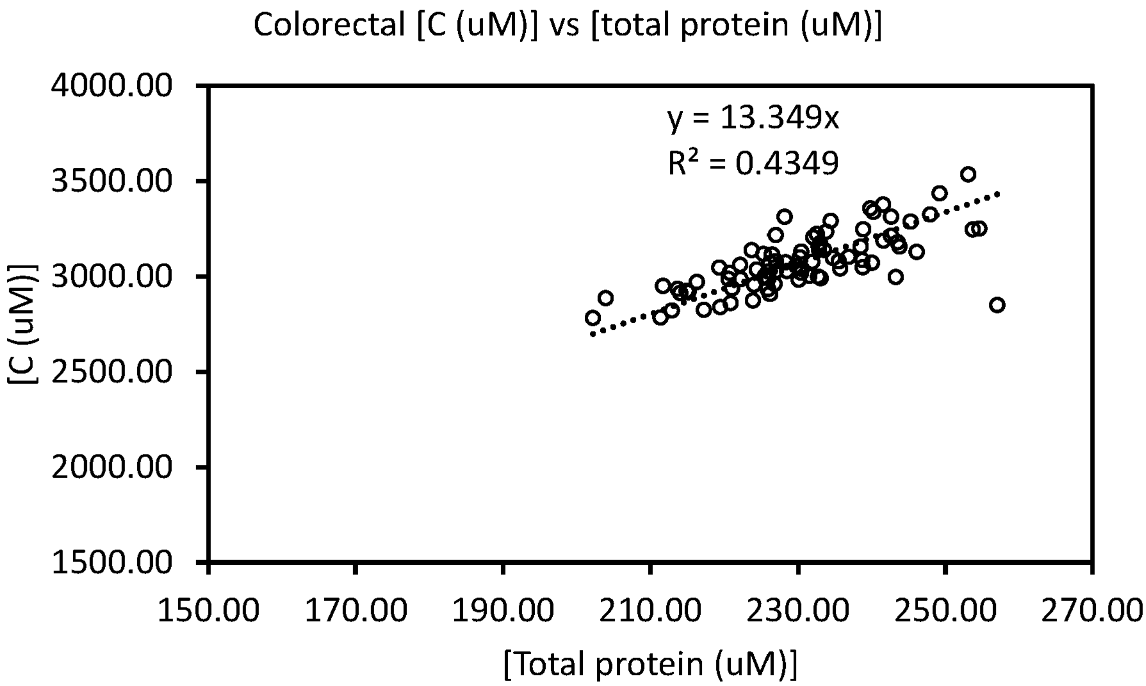


Figure 60

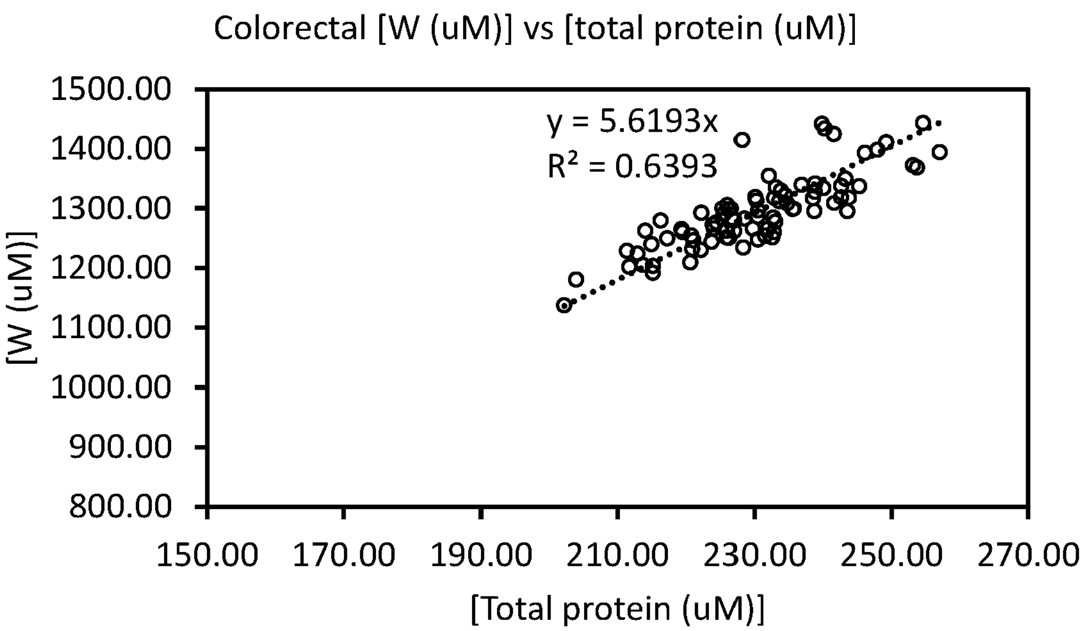


Figure 61

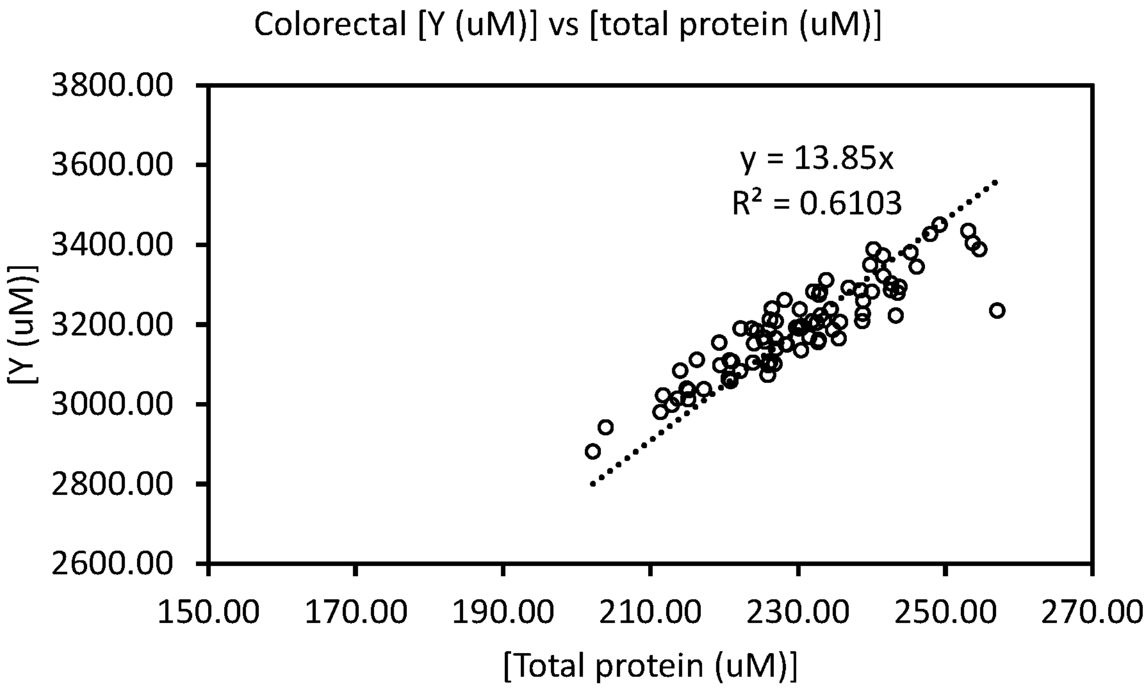


Figure 62

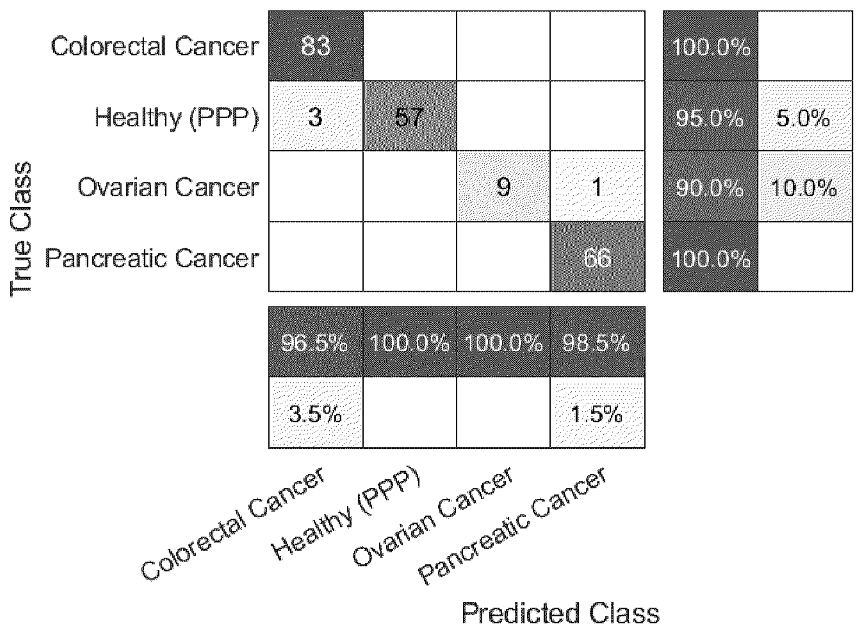


Figure 63

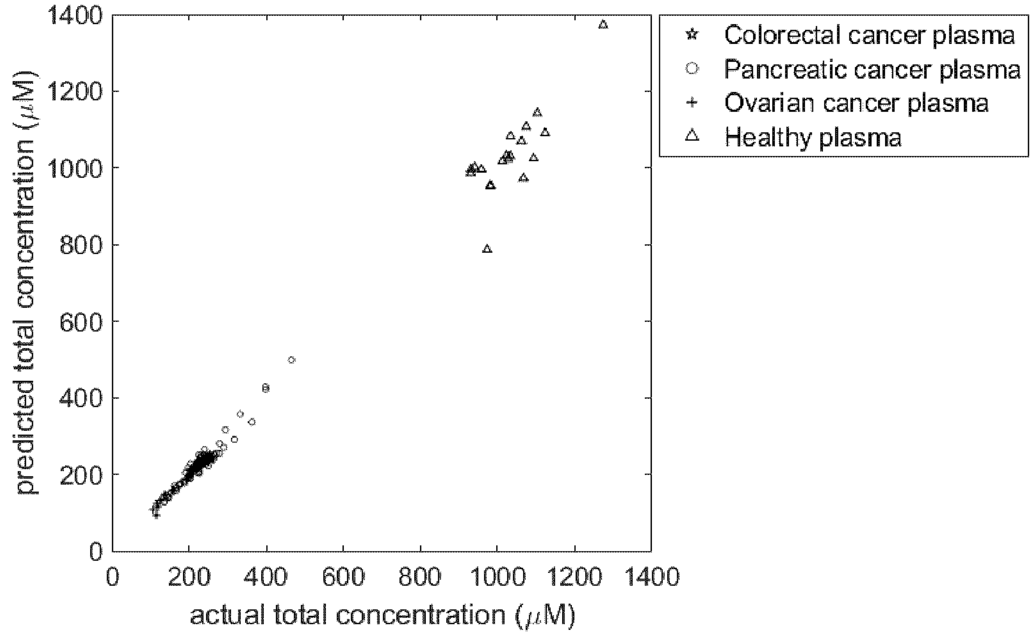


Figure 64

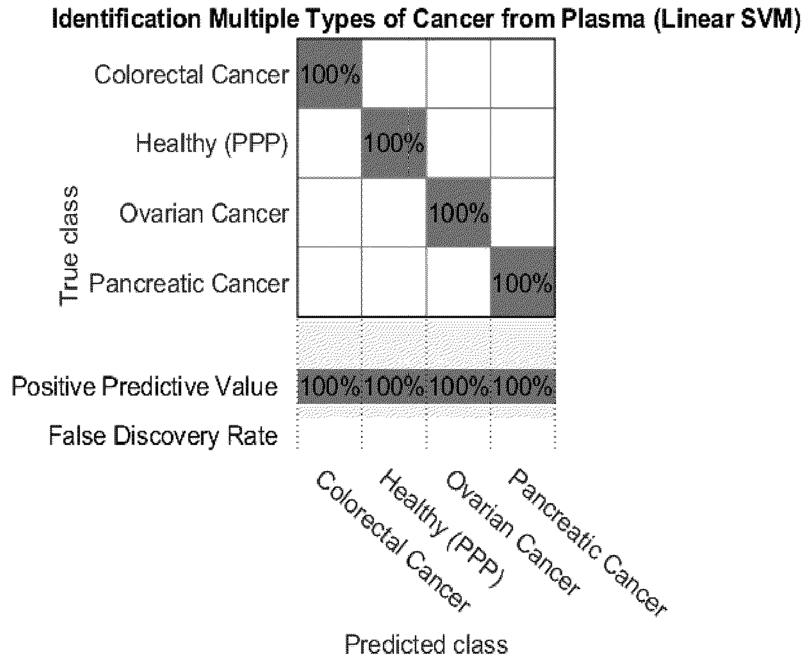


Figure 65

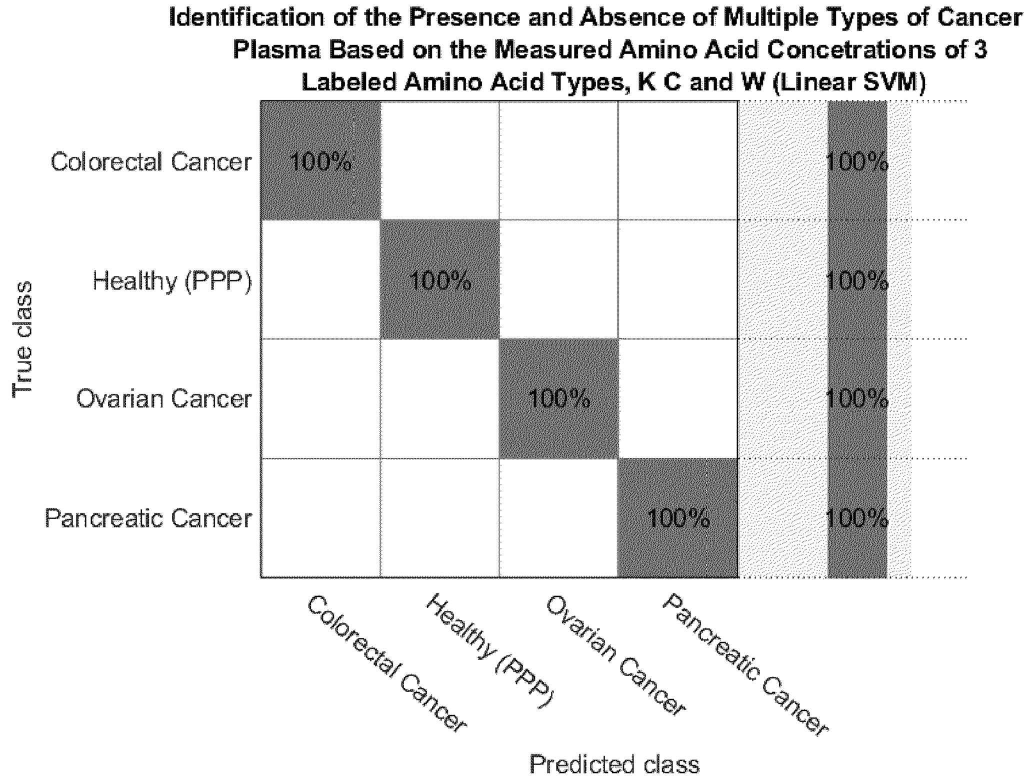


Figure 66

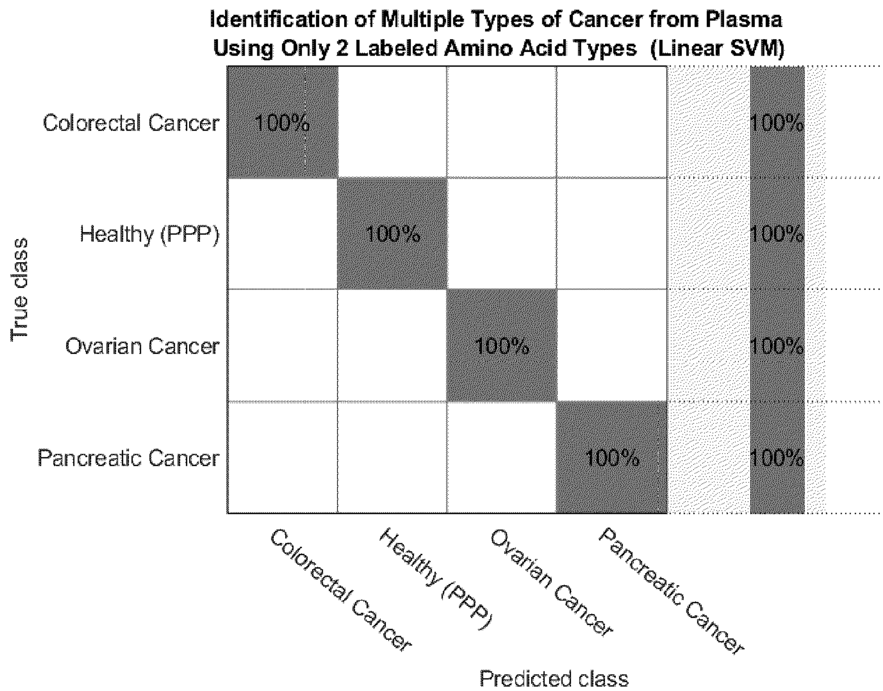


Figure 67

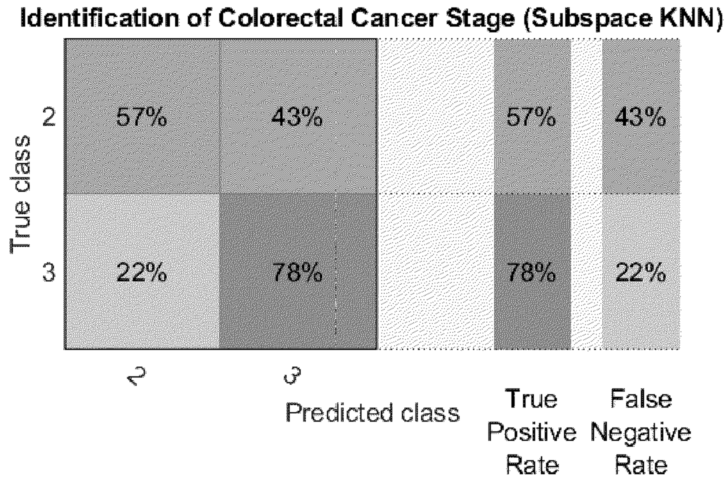


Figure 68

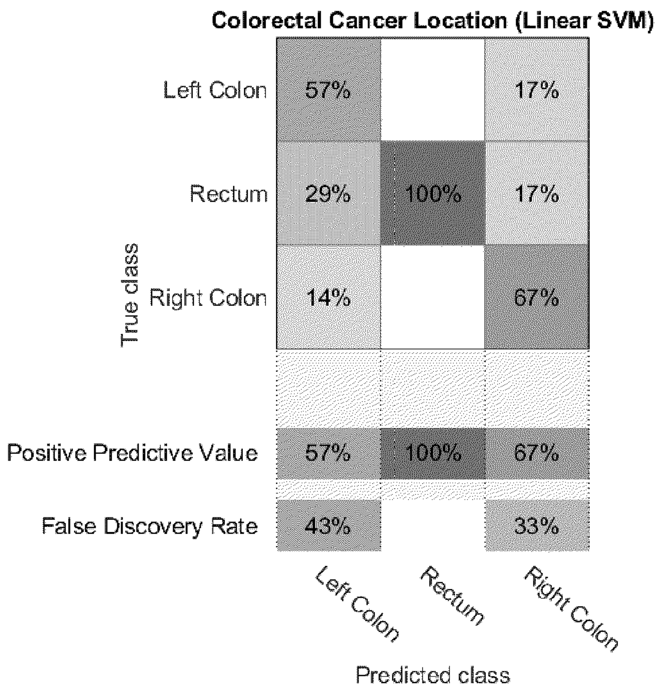


Figure 69

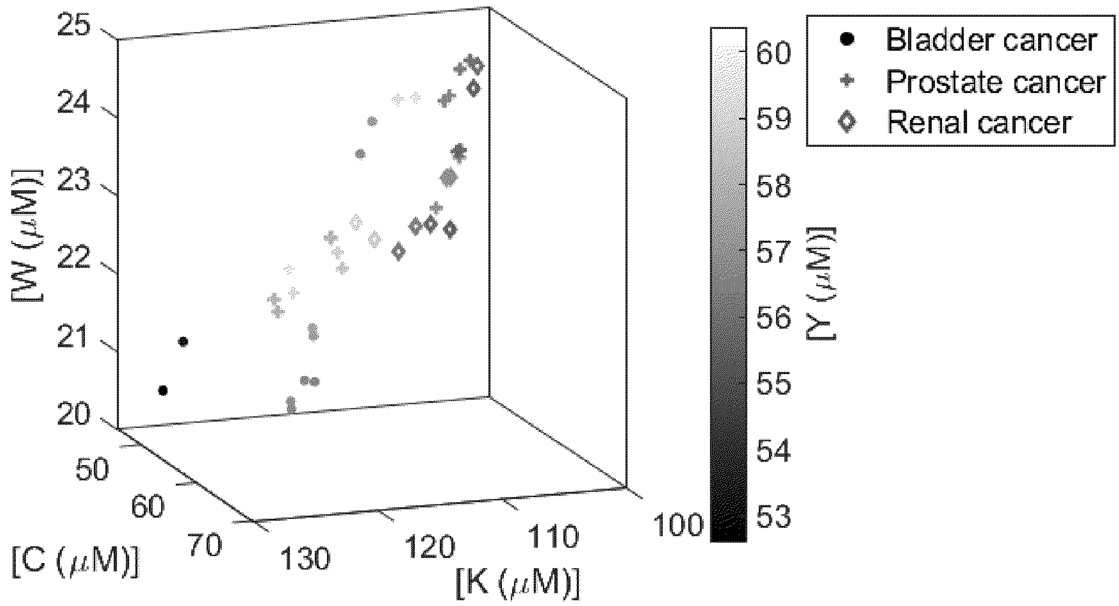


Figure 70

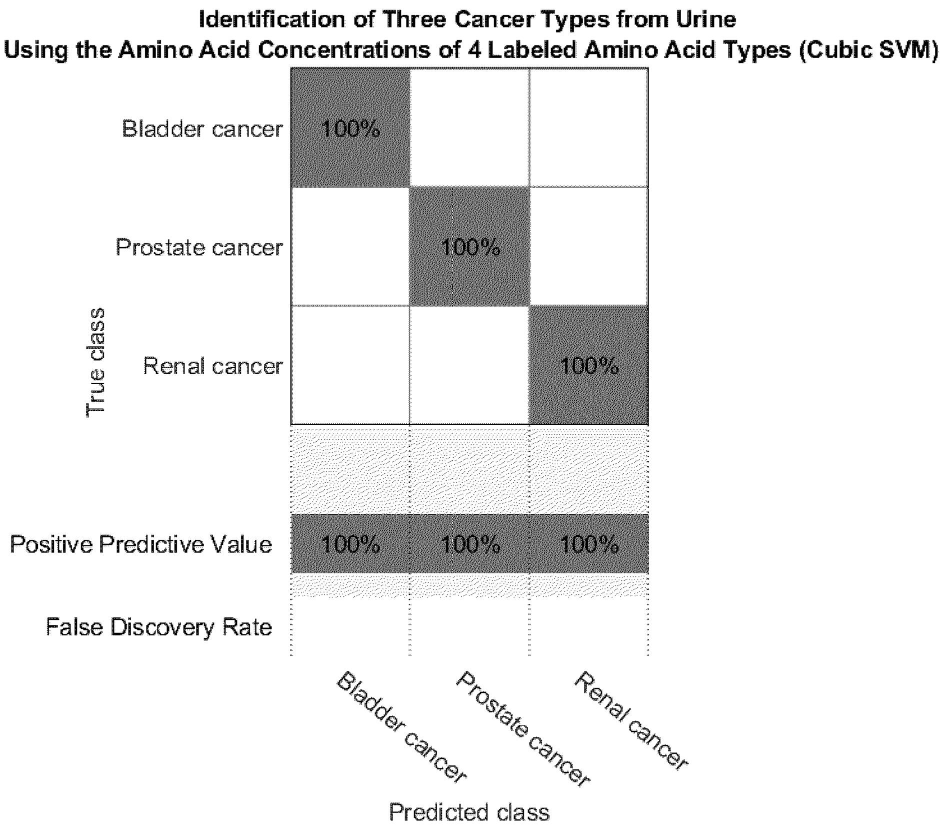


Figure 71

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ZHANG et al.** Top-down proteomics on a microfluidic platform. *eprint 1910.11861 arXiv physics.bio-ph*, 2019
- **SWAMINATHAN, J et al.** *Nat Biotechnology*, 2018, vol. 36, 1076-1082
- Protein Extraction from Solid Tissue. *Methods in molecular biology*, 2011, vol. 675, 307-12